# FASCICULE DE BREVET EUROPEEN

(11) **EP 1 917 365 B1**
(45) Date de publication et mention de la délivrance du brevet: **17.03.2010**
(21) Numéro de dépôt: 06778773.9
(22) Date de dépôt: 05.07.2006
(51) Int. Cl.: C12Q 1/68

(54) **PROCEDE POUR LE DIAGNOSTIC DU CANCER DU SEIN**
BRUSTKREBSDIAGNOSEVERFAHREN
METHOD FOR BREAST CANCER DIAGNOSIS

(30) Priorité: 07.07.2005 FR 0552087
(43) Date de publication de la demande: 07.05.2008
(73) Titulaire: BIOMERIEUX SA, 69280 Marcy L'Etoile (FR)
(72) Inventeur: KRAUSE, Alexander, F-38000 Grenoble (FR); LEISSNER, Philippe, F-38320 Eybens (FR); MOUGIN, Bruno, F-69003 Lyon (FR); PAYE, Malick, F-38100 Grenoble (FR)
(74) Mandataire: Bitaud, Valérie Marie-Odile
(86) Numéro de dépôt international: PCT/FR2006/001593
(87) Numéro de publication internationale: WO 2007/006911

(56) Documents cités:
- WO-A-02/059271
- FR-A- 2 833 968
- US-A1- 2003 099 974
- WANG ZHINING ET AL: "Identification and utilization of inter-species conserved (ISC) probesets on Affymetrix human GeneChip platforms for the optimization of the assessment of expression patterns in non human primate (NHP) samples." BMC BIOINFORMATICS [ELECTRONIC RESOURCE]. 26 OCT 2004, vol. 5, 26 octobre 2004 (2004-10-26), page 165, XP002381760 ISSN: 1471-2105
- YANAGISAWA J ET AL: "A matrix attachment region (MAR)-binding activity due to a p114 kilodalton protein is found only in human breast carcinomas and not in normal and benign breast disease tissues." CANCER RESEARCH. 1 FEB 1996, vol. 56, no. 3, 1 février 1996 (1996-02-01), pages 457-462, XP001246677 ISSN: 0008-5472

### SEQUENCE LISTING

<110> bioMérieux S A
<120> Procédé de diagnostic du cancer du sein
<130> Unknown
<160> 139
<170> PatentIn version 3.3
<210> 1
   <211> 11740
   <212> DNA
   <213> Homo sapiens
<400> 1
   cccggagccg gtgcgaggag ggcacccggt gcgtccccgg agcggggagg ccaggccggg     60
   caaccctggg gccggtcggg gcggcgtcac tgcaccctcc gccaggctcc gcgggatgca    120
   ccgtggtagc cgagggcgga ggcgacactc tcaggtaaat tggcaggatg aggagcagca    180
   aatcaaaaga ggtgccttta ccaaatccaa ggaactctca aagcaaggat actgttcaag    240
   cagatataac cacatcgtgg gatgcacttt ctcaaaccaa ggctgctctg agacacattg    300
   aaaataaatt agaagtagcc cctacaagta cagctgtgtg tgattctgtc atggatacca    360
   agaagtcttc tacaagtgct actcgaaaaa taagtagaaa agatggtaga tacctggatg    420
   attcttgggt taatgctcca atctccaaat ccactaaatc acgaaaagag aaatctcgta    480
   gtcctctcag ggccaccacc ctggagggta atgtgaagaa aaataatcgt gtggaatttc    540
   gtgaaccttt ggtttcttat agggaaatcc atggtgcacc ttccaatttc agttccagcc    600
   atctggaatc aaagcacgta tactgtgtag atgttaatga agaaaagact gagagcggta    660
   actggatgat aggcagtcga gaagaacgga atatacggag ctgtgatttt gagagctccc    720
   aatcatctgt catcaatgat acagttgtta ggtttttaaa tgatcgacca gcaattgatg    780
   cattgcaaaa ttctgaatgt ttgattagga tgggagcttc tatgagaact gaggaagaaa    840
   tgcctaacag aacaaaagga agtgagaata atttgaagct ttctgtgaat aacatggccc    900
   atgatactga tccaaaagcg ttacgactaa ctgactcttc tccatcctct actagtactt    960
   ctaattccca aagattagat attctaaagc ggcgacaaca tgatgtcaaa ctggaaaaac   1020
   ttaaggaacg gattagaaaa cagtgggaac actcagaaga aacaaatggc cggggccaga   1080
   agctgggtca tattgaccat ccagtaatgg ttgttaatgt tgataactca gtaacagcaa   1140
   aagtcagaaa agtggcaaca gcaccacctg ctccagcata taaaggtttc aacccttcag   1200
   agaccaagat tcgaacacct gatgggaaag tgtggcagga ggctgagttt caaaacatga   1260
   gtagagaact gtatcgagat ttagcacttc actttgcaga tgatatttct ataaaggaga   1320
   aacctgctga aaaaagtaaa gagaagaaag tagtcaagcc agtacgaaaa gtccaaaaag   1380
   tagcacagtt atcaagtaca gaatgcagaa caggtagtag tcatcttata agtacatctt   1440
   cttggcgaga tggacaaaaa ttagtaaaga agattctggg acctgctccc agaatggagc   1500
   caaaagagca aagaacagca tcaagtgaca gaggtggaag aagaagaact gctaaatctg   1560
   ggggtcacat tggaagagca gaatctgatc ccaggttgga cgttttacat agacatcttc   1620
   aaagaaactc agaacgttcg agaagtaaat ctcggtctga aaataatata aagaaactag   1680
   cttcatctct tccagataat aagcaggagg aaaatactgc cttaaataag gactttttac   1740
   ctattgaaat tcgtggcatt cttgatgacc tacagctgga ttctacagct cacactgcaa   1800
   agcaagatac tgtagagtta cagaaccaga agtcatcagc accagtacat gctcctagga   1860
   gtcacagccc agtaaaaaga aaacctgaca aaataacagc taatgaagat ccccctgtta   1920
   tttccaaaag gcgccactat gacacagatg aggtacgaca gtacattgtt aggcagcagg   1980
   aggaaaggaa gagaaagcaa aatgaagaga agaaggctca aaaggaggct acagaacaga   2040
   aaaacaaacg attacaagag ctctaccgga agcagaagga agcctttact aaagtaaaaa   2100
   atgtccctcc ttctgagcca tcagcaacta ggcgactaca ggaaacttac tccaaattgc   2160
   tactagaaaa gaccttgctt gaagagccat ctcatcaaca tgttacgcag gaaacacagg   2220
   ccaaaccagg gtatcagcca tctggagaat ctgacaaaga aaacaaagta caggaacgtc   2280
   ccccaagtgc atcttccagt agtgacatgt ctctctcaga acctccacag cctcttgcaa   2340
   gaaaagactt gatggaatct acatggatgc agcctgaaag attgagccca caagttcacc   2400
   attctcaacc acagcctttt gctggaacag ctggaagttt actctcccat ctcttgagtt   2460
   tagagcatgt aggaattttg cataaggatt ttgaatctat tttaccaacc aggaagaatc   2520
   ataatatggc ttcaaggcca ttaactttta cacctcaacc atatgtgacc tcaccagctg   2580
   cttatacaga tgccttgtta aaacctagtg ccagccaata taagagtaaa ctggatcgta   2640
   ttgaagcctt gaaagcaaca gctgcttctt tgtccagcag aattgaaagt gaagccaaga   2700
   aattagctgg ggccagcatt aactatgggt cagcatggaa cactgagtat gatgtgcagc   2760
   aggcacctca agaagatgga ccttggacca aggctgtaac tccacctgtg aaagatgata   2820
   atgaagatgt tttctctgcc agaattcaga agatgctggg aagctgtgta tctcatgcaa    2880
   cttttgatga tgatcttcct ggtgtaggca atcttagtga atttaaaaag cttcctgaga   2940
   tgataagacc acagagtgcc atatcaagct ttagagtgag atcccctggt cccaaaccac   3000
   aagggctact ggcacagtta tgtaaaaggc agactgactc ttctagctct gatatgcaag   3060
   cctgttctca agacaaagcc aaaatatctc ttggttccag catagattca gtcagtgaag   3120
   ggcctcttct tagtgagggg agtctctctg aagaagaggg agaccaggat ggacagcccc   3180
   ttttgaaagt agcagaaatt ttaaaagaaa aggaattttg tcctggagaa agaaatagtt   3240
   atgaacccat caaagagttt cagaaagaag ctgaaaaatt cttgccactt tttgggcaca   3300
   taggtggtac acaaagcaaa ggaccatggg aagaattggc aaagggaagt ccacatagcg   3360
   tcattaatat ttttacaaaa tcctatcagt tatatggaaa agggtttgaa gacaagttgg   3420
   acagaggaac atcaacatca cggcctttga atgccaccgc aactcctcta agtggtgttt 3480
   catatgaaga tgattttgtc tcctctccag ggactgggac ttcgacagaa aaaaaatcaa 3540
   ctcttgaacc tcatagcact ttaagccctc aggaggacca ttctaacaga aagtctgcct 3600
   atgatccttc ctctgtggat gttacctccc agcattcatc aggagcccag tctgctgcat 3660
   cgtctcgttc atctacttct tctaaaggaa agaaaggaaa aaaggaaaag acagaatggt 3720
   tggattcatt cactggaaat gttcagaact cacttcttga tgaggaaaaa gcagaacgtg 3780
   gctcccatca aggaaagaaa tctgggacca gcagcaaact ttctgttaaa gattttgagc 3840
   agactcttga tacagatagc actttggagg atctttctgg acattctgtg agtgtctcat 3900
   cagataaggg aagatctcag aaaactccaa cttctcccct gtcaccaagt tcccagaaat 3960
   cattgcagtt tgacgttgca ggaacttctt cagaaagatc taagtcgtca gtaatgcctc 4020
   caactataac aggatttaag cctaatgcac ctctcactga tctgaacccg gcagccagca 4080
   gaacaacgac agagaacatg gctccaatac caggttctaa gcgcttttct cctgctggcc 4140
   tccatcatcg tatggcagca gaactcagtt atctgaacgc cattgaggag tcggtgcgcc 4200
   aactgtcaga tgtagaaaga gttagaggca tttcacttgc tcagcaggag agtgtgtctc 4260
   tagctcagat aataaaggca caacagcaac gccatgaaag agacttggcc ctcttgaaac 4320
   taaaggctga acaagaggct ctggagagtc agagacaatt agaagaaacc cgaaacaaag 4380
   cagctcaggt ccatgcagaa tcattacagc aggtggttca atcacaacgg gaagtaactg 4440
   aagtcctgca ggaagcaacg tgtaaaatag cagctcagca gtcagaaact gctcgcctca 4500
   ccacagacgc agcacgtcaa atctgtgaga tggcagagtt gactagaact catatctcag 4560
   atgctgtcgt ggcttcagga gctccccttg caatactgta tgaccaccaa cggcagcacc 4620
   ttccagactt tgtgaaacag ctgaggacca gaactgaaac agataggaaa agtccatctg 4680
   tttcactctc tcagagtaaa gaagggaccc ttgactcaaa gcatcagaag tattctgctt 4740
   catatgatag ttattctgag tcttcaggat acaagaatca tgatagaaga agtagcagtg 4800
   gtagcagccg ccaagaaagt ccttcagttc catcttgtaa ggaaaatgag aagaaactta 4860
   atggtgaaaa gatagagagt tccattgatg aacaggttca gactgctgca gatgattctc 4920
   tacgaagtga tagtgttcca tctcttcctg atgaaaaaga ctcaacgtct attgcaacag 4980
   aatattctct gaaatttgat gaatccatga cagaagatga aatagaagaa caatcatttc 5040
   gatcattact accttcagag agtcaccgca gatttaacat ggaaaagaga agaggtcatc 5100
   atgatgactc tgatgaagaa gcttctccag aaaaaactac actgtctact gccaaggaac 5160
   tgaacatgcc attctcagga ggacaagata gcttttctaa atttactatg gagatggttc 5220
   gacagtatat gaaagaggaa gaaatgaggg cagctcacca gtcttcactc ctgcgtctcc 5280
   gtgaaaaggc cttgaaggag aagactaagg ctgaattggc ctggttagag catcaaaaaa 5340
   aacatctacg agacaaagga gaggatgata aaatgccccc gctccggaag aaacagcgtg 5400
   gtttgctttt aaggttgcag caagaaaagg cagaaataaa acgtcttcaa gaagccaata 5460
   aggcagctcg gaaggaaaga cagctgattc ttaaacagca ggaggagata gaaaagatcc 5520
   gacagaccac cataaaacta caggagaaat tgaagtctgc aggggagagt aaattggact 5580
   ctcatagtga tgatgataca aaggataata aggcaaccag tcctggtcca actgacttgg 5640
   agacccgcag tccttctccc atttcaatct ccagcagtga aactagcagc attatgcaga 5700
   aactgaagaa aatgagaagc cgcatggatg aaaagtttct gacaaagcgg gagcaaaaat 5760
   taatgcaacg gcgacaacat gcagaggagc tcctagagtg gaagcgacgt ttagatgcag 5820
   aagaagcaga aattcgtcaa atggaaaaac aagctttggc tgcctgggac aaagaattaa 5880
   taaaacccaa aactcctaag aaagaactgg aggaccagag aacagaacag aaagaaatag 5940
   caagtgaaga ggaatctcca gtacctctgt actctcatct aaacagtgaa agctccattc 6000
   cagaagaatt aggcagccct gctgttgaat atgtaccatc cgagtctata ggacaggagc 6060
   agccagggag tccagatcac agtatactta ctgaagaaat gatttgttca caggaactag 6120
   aatcttctac ctctcctagt aaacattcac ttcccaaaag ctgcacatct gtgtcaaagc 6180
   aggagtctag caaaggaagt cataggactg gaggacaatg tcacctgcct atcaagtccc 6240
   atcagcactg ttatagttgg tcagatgagt cattatctat gacacagtca gaaactacat 6300
   ctgaccagag tgatattgaa ggtaggatca gagctctgaa ggatgagttg cggaaaagaa 6360
   aatcagttgt gaaccagctg aagaaggaac agaaaaaaag gcaaaaggaa agactgaaag 6420
   cccaagaagc cagtctgatc aagcagttag agtcatatga tgaatttatt aagaaaactg 6480
   aagccgagct tagccaagat ttggaaacat caccaacagc caagcctcag attaaaacgc 6540
   tctcctcagc ttctgaaaaa cccaagatca aacccctcac accactacac agatctgaaa 6600
   cggcaaagaa ttggaaatca ctaacagagt cagaacgttc cagaggatcc ctggagccta 6660
   ttgctgaaca tgttgatgct tcactgtctg gttctgagag atcagtatca gaaaggtctt 6720
   tatctgcata tgcaaagaga gtaaatgaat gggacagtcg aacagaagat tttcagaccc 6780
   catctccagt tctcagatca tcaaggaaaa tcagagaaga atctggagat tctctagaaa 6840
   atgtacctgc attacatctt ctcaaagaat taaatgccac tagtagaatt cttgatatgt 6900
   cagatggcaa ggttggagaa tctagtaaaa aatcagaaat aaaagaaata gagtatacaa 6960
   aattgaagaa gagtaagatt gaagatgcct tttctaaaga aggtaaatct gatgtcttac 7020
   tgaaattagt cctagaacag ggagattcat ctgaaattct ttcaaagaaa gatcttcctt 7080
   tagattctga aaatgttcag aaagacctag ttggattagc tattgaaaat ctccataaaa 7140
   gtgaggaaat gttgaaagag agacagtcag atcaagatat gaatcatagt ccaaacatcc 7200
   aatcaggaaa agacattcac gaacaaaaga acacaaagga aaaagatttg tcttggtcag 7260
   aacatctttt tgctcctaaa gagataccat actctgaaga ttttgaagtg tcttctttca 7320
   agaaagaaat ttcagctgaa ttgtacaaag atgattttga ggtgtcatct ttgctgtcac 7380
   tcaggaaaga ctctcagtct tgcagagata agccacagcc aatgaggagc tctacaagtg 7440
   gagccactag ctttggtagt aatgaggaaa tcagtgagtg cctaagtgag aaaagccttt 7500
   ctatccatag caatgttcat tctgacaggc tgttggaact caagtcccct actgagctga 7560
   tgaaaagtaa ggagcgcagt gatgtggagc atgaacagca agttactgaa tccccttcct 7620
   tggcttcagt tcctactgca gacgagttat ttgatttcca cattggtgat agggtgttga 7680
   ttggaaatgt tcagccagga attcttcgat tcaaaggtga gactagtttt gctaaaggat 7740
   tttgggccgg agtggagtta gataaacctg aaggaaataa caatggaaca tatgatggta 7800
   ttgcatattt tgagtgcaaa gaaaagcatg gtatttttgc tcctcctcaa aaaatatctc 7860
   acattccaga aaactttgat gactatgtag acattaatga agatgaagac tgttactcag 7920
   atgaacgata tcagtgctat aatcaagagc aaaatgatac agagggtcca aaagacagag 7980
   aaaaggatgt cagtgaatat ttttatgaga aatccctacc tagtgtgaat gatatagaag 8040
   cctcagttaa tagaagtaga agccttaaaa tagaaacaga caatgtacag gacatttctg 8100
   gggtacttga agcccatgtt caccagcagt cttcagtgga ttcacagatt tcttcaaagg 8160
   aaaacaaaga cctcatttct gatgccacag aaaaggtttc catcgctgca gaagatgaca 8220
   ctttagacaa taccttttcc gaagaattgg agaagcaaca gcagtttaca gaagaggaag 8280
   acaacctata tgctgaagct tcagaaaagc tttgtacacc acttctggat cttttaacaa 8340
   gagaaaaaaa ccaactggaa gcccagctga agtcatcact aaatgaggaa aaaaagtcaa 8400
   aacaacaact ggaaaaaatc agcttactga cagacagttt actaaaagtc tttgtaaagg 8460
   acacagtcaa tcaactacaa caaatcaaaa aaaccaggga tgagaaaatc cagcttagca 8520
   atcaggagct tcttggtgat gaccaaaaga aagtaacacc ccaagaccta tcccaaaatg 8580
   ttgaggaaca gtcgccaagt atttcaggtt gcttcttaag ttctgaattg gaagatgaaa 8640
   aagaagagat ttcctctcca gatatgtgtc ccagaccgga gagcccagta tttggtgcca 8700
   gtgggcagga agaacttgct aagagacttg ctgaacttga actcagccgg gagttcctga 8760
   gcgcgttagg agatgatcaa gactggtttg atgaagactt tggtttgagc tcttctcaca 8820
   agatccaaaa aaataaggca gaagaaacca ttgtacctct aatggcagaa cctaaaagag 8880
   taacccaaca accatgtgaa acattattgg cagtccccca tactgcagaa gaagtagaga 8940
   ttcttgtaca taatgcagca gaagaacttt ggaaatggaa agaattaggc cacgatcttc 9000
   atagcatcag tattcctaca aaactgcttg gctgtgccag taaaggtcta gatatagaaa 9060
   gcactagtaa aagggtctac aaacaggcgg tttttgattt aacaaaagag atttttgagg 9120
   aaatatttgc tgaggatccc aacttaaatc aacctgtctg gatgaagcca tgtagaatca 9180
   actctagtta tttccgacga gtgaaaaatc caaataacct tgatgaaatc aagagcttca 9240
   tagcaagtga agtactcaag ttgttcagtc ttaaaaagga gccaaaccac aaaacagatt 9300
   ggcagaaaat gatgaaattt ggaagaaaga aaagagaccg agtggatcat atcctggttc 9360
   aggagctcca tgaggaggag gcacagtggg tgaactatga tgaggatgag ttgtgtgtga 9420
   aaatgcagct agccgacggg atctttgaga ccctgatcaa agatactatt gatgttctga 9480
   atcagatcag tgaaaagcag gggagaatgc tactagtgtg acatcttgca aataaatcga 9540
   acgctgagtg ctaatgtgag tcctgggcct ttctgcctcc tgatgtacac ccatcgccat 9600
   catagcaaga gtgcttctgg accttgtact tattcttaaa gactaccagt atggagttca 9660
   taggacaatg tggtacacct ggtattacag cctttgcctt tcgagactat ccactggatt 9720
   aatgggttat tttcagtggg cagggttgca cagtgtaatc ctacaccttt tgctaacacc 9780
   cctactaggt cccagagggc cagaaacacc tgacttacct ctgagtttag actagggtat 9840
   cacttctttg agtctgaagt caagtgagag aggtatagat aaatgcatca catcactttt 9900
   gaaatgtaat tctggtctat accatggaag tcataaatgg acattatagt ctctaaacag 9960
   tattaaaccc ttaaccactt ctaaaatagg caagctcaat aatgtctgcc aacttcacat 10020
   tctggagttt atttcatttc tttttgaaga ccattttctt ccattattgt agttgagcag 10080
   caccaagtgg actgtcaggc taacaggaat aagtggtagc cttgctttct gagcaccatc 10140
   taaagaattt taaacctctg cattatgttt agtgttctct gtgtgggcat gaaaacaaag 10200
   aaatgcccct actgaagact gggctcaaag gaccaatgca gggctggttc ttttacccct 10260
   tggtttactc ctgcacttgt cttactcatt ctgaatctct accagctgct cccagaatca 10320
   cagatactca ggaccatctc aggcatccag gcatggcaaa gtggaaataa ttcattttgg 10380
   cctgcaaacc ttcaactccc atctttcccc aagaagtcag agatgctgtt acttgaatga 10440
   tttaggaaat gagtgtgtgc accaaagaca aaaagatatt gtctattgtt tgtgtgcttg 10500
   ttttgcgcta tggaacattt tttaatttat tttaagataa attattaagt tgaaaatgtg 10560
   tgtccctatt cagaagtgaa agattcatct tgtaatagtt aaacctccat cttgaagctt 10620
   ctatggttca tagtctttgc acaggaacct gtggttttaa caaaccaata cacatattga 10680
   agaagtcatt ttaattcagt gaaacgaaga tgggcttttc cagatcacct gcaatagcag 10740
   cagtgggata aaatgattta aaaacactgt acaatttaac tctgcctctc ttgcagcatt 10800
   gcttctcaca actattacct gcatctgaaa aaaaatctat agactccagc tgctacatta 10860
   gagcataaga gatgctctcc tgggacctca gtaccctgcc ttcttgactg gtttccgttc 10920
   atcagtcctg tccctcttca agtaatctag aagaatgtgg atactcttag gcgtgaatgt 10980
   aaatgcctta atattgaagg tcctggttag aagcatgata caagacatct actggattca 11040
   tatttacaaa tatcctggaa tgttatagct tcaaagtata ttagaaaaac cccaaagatg 11100
   gtataatctt taagtgtgca cgttcgttta tttctgcatc ttccctccaa acttgccttt 11160
   gcatcttaaa tatttcacta tgcacactcc cattcctctt gggtttcatc ttgtcgttta 11220
   agaaatgtac tgaaataatc attggaatat ttgcattttg cacaatgact ggtatgatag 11280
   ctcttgacaa ataaggaaag cactgaaatg ttgtgattgg gtctcgggaa atgctcagat 11340
   tgatgtctta ccagcatttc ttctgggctt gtgatgttga gctgtagtct tgtagccata  11400
   atgagcaaat tgactaagag aagcaaaggt ttcttggggt tattaaccag tagtgtggaa  11460
   atactagttt tatgtggcca aggaaaagca aagccttttc ttttcagttt gtgttatttg  11520
   gaagacagaa aaacatcttg tctacatcct ttggctgttt gtaggatcac gttgtcctta  11580
   cgatactgaa actttacagc tgctgtaaat tttttataaa tgaatttcaa aatgttataa  11640
   tgggactgta ggttgttttt ctacatcttc attatttgga cctaaaacca gtttttaata  11700
   agaaagttta tctttactct ttctgaaatt atgactccag                        11740
<210> 2
   <211> 7597
   <212> DNA
   <213> Homo sapiens
<400> 2
   cgctaggaaa gggaactggg ttgcgacggt ccggcgagag agagctgggg tgctggggtg 60
   cggggaagtt ggggagcaga ggccgcttgg tgtccgagta gggtaagacc gcaccgaccc 120
   agtccgttag gaaagaaggg aaacgaggca attgtcgggc ggatccccgg acggagggct 180
   aaggttgtgt ggaaggcgct gctccccgga tggcgaccgc agatactccg gccccggcct 240
   ccagtggcct ctcgccgaag gaagaagggg agcttgaaga tggggaaatc agtgacgacg 300
   ataataacag ccagatacgg agtcggagca gcagcagcag cagcggcggc gggctgttac 360
   cctatccgcg gcgaaggcct cctcactcgg cccggggcgg tggatctggc ggaggcggtg 420
   gctcttcctc gtcatcgtcc tcttctcagc agcagctgag gaatttctca cgctcgcggc 480
   acgcgtctga gcggggccac ctcaggggac ccagcagcta ccgacccaaa gaaccgttcc 540
   ggtctcatcc gccttctgta cggatgcctt cgagctcact gtccgaaagc agtccccggc 600
   cgtctttctg ggagcggagc cacctcgcct tggaccgttt ccgctttcga ggcaggcctt 660
   accggggtgg gagtcgctgg agtcgggggc gaggagtggg tgagcgagga ggcaagccgg 720
   ggtgcagacc tcctctggga ggaggagcag gatccgggtt cagcagcagt cagagctggc 780
   gagagccctc tccacctcgg aagagctcca aaagttttgg aaggtctcca tcaagaaaac 840
   aaaattattc atcaaaaaat gaaaactgtg tggaagaaac ttttgaagat ttgcttttaa 900
   agtataaaca aatacagttg gaactagaat gcatcaataa ggatgaaaaa ctagcattga 960
   gtagcaaaga agagaatgtg caggaagatc ctaaaacatt gaacttcgag gaccaaacta 1020
   gcactgataa tgtcagtatt acaaaggatt caagtaaaga agtagctcct gaggagaaaa 1080
   cacaagtcaa aacttttcag gcatttgaat taaaaccact caggcaaaaa ttgactttac 1140
   caggagataa gaaccgtttg aaaaaagtta aagatggagc aaaaccactt tccctgaaat 1200
   ccgacactac tgattctagt caaggattac aagataaaga acaaaattta acaagaagaa 1260
   ttagtacctc agatattctg tctgaaaaga aacttggtga agatgaagag gaactatctg 1320
   aattacagct tcgccttttg gctcttcagt cagccagtaa aaaatggcaa caaaaagaac 1380
   agcaggtgat gaaagaaagc aaagaaaagt tgactaagac gaaaactgta cagcaaaaag 1440
   ttaaaacaag tacaaaaaca cattcggcca aaaaagttag cactacagct aaacaagcat 1500
   tgaggaagca gcaaacaaag gcatggaaga aactacaaca acaaaaagag caggaaagac 1560
   agaaagaaga ggatcagcgg aaacaagctg aagaagaaga gagaaggaaa agagaggaag 1620
   aaatcagaaa aattcgagat ctctcaaatc aggaagaaca gtacaatcga ttcatgaaat 1680
   tggttggtgg caagaggaga tcaagaagta aatcttcaga tcctgacctg aggcgatcct 1740
   tagataagca acctactgat agtggaggag gcatttatca gtatgataac tatgaagaag 1800
   ttgctatgga tacagatagt gaaaccagtt ctccagctcc ttcaccagtg caaccgccat 1860
   ttttctctga atgttcattg gggtattttt ctccagcacc atctctttct ttgcctccac 1920
   cacctcaggt ttcttctctg ccacctttga gccagcctta tgtggaaggc ttgtgtgttt 1980
   ctcttgaacc tctacctcct ctaccaccat taccacctct cccacctgaa gatccagaac 2040
   agcctccaaa accacctttt gcagatgagg aagaggagga agaaatgctg cttcgagaag 2100
   aactacttaa atctctagca aataaaagag cttttaagcc agaggaaaca tccagtaata 2160
   gtgacccacc ttcacctcca gttctgaaca attcacatcc tgtgccaaga agcaatctat 2220
   caatagtcag tattaacaca gtgtctcagc ctaggataca gaatccaaag tttcacagag 2280
   gaccccgtct tccacgaact gtgatctcgc ttccaaagca taaatcagtg gttgtaacac 2340
   taaatgattc agatgatagt gaatctgatg gagaggcttc caagtcaaca aatagtgttt 2400
   ttggtggatt agagtccatg attaaagaag caagacgaac tgctgagcaa gcttcaaaac 2460
   cgaaagtacc tccaaaatct gaaaaagaaa atgatcctct gcgaacaccg gaggctttgc 2520
   ctgaagaaaa gaagattgaa tatagattgt taaaggaaga gattgccaac cgtgagaaac 2580
   agcgtttgat taaatcagat cagctgaaga caagttcatc atccccagca aactctgatg 2640
   tggaaattga tggtattggc aggatagcaa tggttactaa gcaggttaca gatgcagaat 2700
   caaaactgaa aaaacatagg attctcttga tgaaagatga atctgtttta aagaatttag 2760
   tgcaacaaga agctaagaag aaagaatctg ttagaaatgc tgaagcaaag attacaaaac 2820
   ttacagaaca gcttcaagca actgaaaaaa ttcttaatgt taacagaatg tttttgaaga 2880
   agcttcagga acaaattcac agagttcaac agcgtgttac aattaagaaa gctttgactc 2940
   taaaatatgg agaagagctt gctcgggcaa aggcagtggc cagtaaagaa ataggaaaac 3000
   gtaaactgga acaagatcgc tttgggccaa acaaaatgat gagactggac agttctccag 3060
   tatcaagtcc aagaaagcat tcagcagaac taattgctat ggagaaaaga cggttacaaa 3120
   agctagaata tgaatatgcc ctgaaaattc aaaaattaaa agaagcccgt gcccttaaag 3180
   caaaggaaca acaaaatatc tctccagttg tggaagagga acccgaattt tctttacctc 3240
   aaccctcact tcatgatctg acacaagata aattaaccct ggacactgaa gaaaatgatg 3300
   ttgatgatga aattttgtct ggttcaagca gagagcgaag aagatctttt ttagaatcca 3360
   attattttac taaacctaac cttaagcaca ctgatactgc taacaaagaa tgcataaaca 3420
   aacttaataa aaatactgta gaaaaaccag aactttttct agggttaaaa attggtgaat 3480
   tgcaaaaatt gtattcaaaa gctgacagcc taaaacagct gattttaaaa accaccacag 3540
   gcattacaga gaaggttttg catggtcagg agatttctgt agatgtggat tttgtcacag 3600
   cacaaagtaa aacaatggaa gtgaagccat gtccttttag accctaccat agtcctcttc 3660
   tagtttttaa gtcctacaga tttagtccat attatcgaac caaggaaaaa cttcccctga 3720
   gctcagtatc atacagtaat atgattgaac cggatcagtg tttctgccgt tttgatttaa 3780
   caggaacatg taatgatgat gattgtcaat ggcagcatat acaagactat acacttagcc 3840
   gaaaacagtt attccaggac attctgtcat ataatctgtc tttgattggt tgtgcagaga 3900
   caagtactaa tgaagaaatt actgcttcag cagaaaaata tgttgagaaa ctttttggag 3960
   taaacaaaga tcgaatgtca atggaccaga tggctgttct ccttgttagc aatatcaatg 4020
   aaagtaaagg tcatactcct ccatttacaa cctacaaaga taaaagaaag tggaagccaa 4080
   agttttggag aaaacctatt tcagataata gcttcagtag tgatgaggaa cagtctacag 4140
   gaccaattaa gtatgctttc cagccagaga accaaataaa tgttccagct ctggatacag 4200
   ttgtcactcc agatgatgtc agatacttta caaatgagac tgatgacatc gctaatttag 4260
   aagcaagtgt gcttgaaaat ccttctcatg tacaactttg gctcaagctt gcgtacaagt 4320
   acttgaatca aaatgagggg gagtgctcag aatccttgga ttctgcttta aatgttctgg 4380
   cgcgagcatt ggaaaataac aaagacaatc cagaaatttg gtgccattac ctcagattgt 4440
   tctcaaaaag aggaaccaag gacgaggtgc aggaaatgtg tgaaacagct gttgaatatg 4500
   ctccagatta tcaaagcttt tggacttttc tacacctaga aagtaccttt gaagaaaagg 4560
   attacgtatg tgagagaatg ttggagtttc tgatgggagc agccaagcag gaaacatcca 4620
   atattttgtc ctttcagctt ttagaggctc ttttgtttag agttcagctg cacatattta 4680
   ctggaagatg ccaaagtgca ctggcaattt tacagaatgc attgaaatct gctaatgatg 4740
   gaatagtagc tgaatacctt aaaaccagtg atcgatgttt ggcatggttg gcctacatac 4800
   atcttattga attcaacatt ctcccttcaa aattttatga tccatctaat gataatcctt 4860
   caagaattgt taacactgaa tcatttgtaa tgccatggca agctgttcaa gatgtaaaga 4920
   ctaatcctga catgttgtta gcagtttttg aagatgcagt gaaagcttgc acagatgaga 4980
   gccttgctgt tgaggaaaga atagaggcct gccttccact ttacacaaac atgattgctc 5040
   tgcaccaact cctggagagg tatgaggctg caatggagct ttgtaaatct ttattggaat 5100
   catgtcctat taactgccag ttgctggaag ctcttgttgc attatatttg caaacaaatc 5160
   agcatgacaa agccagagca gtgtggctta ctgcatttga aaaaaatcct cagaatgcag 5220
   aggtttttta tcatatgtgc aaattcttca tcttacagaa tcgaggcgat aatcttcttc 5280
   catttttgcg gaaatttatt gcatccttct ttaaaccggg gtttgagaag tataataact 5340
   tggatctgtt tcggtatctc ttaaatattc caggaccaat tgacattcca tctcgtttat 5400
   gtaaagggaa ttttgatgat gatatgttta accaccaagt tccttatttg tggctgattt 5460
   actgcctttg tcatcctctt caatcaagta ttaaagaaac agtggaggca tatgaggcag 5520
   cattaggggt ggctatgaga tgtgatatag tacagaagat atggatggat tatcttgtct 5580
   ttgcaaataa tagagctgct ggatccagaa acaaagttca agaattcaaa ttttttactg 5640
   atttagtgaa tagatgtttg gttacagtcc ctgcccgata ccccattcct tttagcagtg 5700
   ctgattactg gtccaactat gaatttcata atagggttat tttcttttat ttgagctgtg 5760
   ttccaaagac ccagcattcc aaaaccttgg aacggttttg ttcagttatg ccagctaatt 5820
   ctggacttgc attgaggtta cttcaacatg aatgggaaga aagcaatgtt cagattctga 5880
   aacttcaagc caagatgttt acatataata tcccaacatg cctggccacc tggaaaatgt 5940
   aatgcaacaa tcatgtacat gtttttatag ctattgcttt tatttataag gtttgctcca 6000
   gacaggtaaa tctgaaatga catctatata tatatatata tatatatatt tttttttttt 6060
   ttttcctgga cagattgcta ttcttaattt agatttgttt ttagcattta ctgaggttta 6120
   atgagagtga gtactcattg aatagcctct aaaagcaaaa agcgtagact tttaattttc 6180
   tttgaaggac aacatttagg tgacagtact gttatcaaac aaatctgcta cagacatttt 6240
   aagaccacct cttgaaatgg tggtgtgtca cctgttgtag tctgatttct tctttctttt 6300
   aaaggtgtta gtgctaggta tttttgacta gtaattatga ggtgtttttt ttttgttgtt 6360
   gttgttgttt tttttttctc ctgtgaacgt tatttatgtt caaaacactg gtctgctttt 6420
   ttcttgtaaa gttgataaaa attgttgaat ttttcttttc actataggtt ttcctccttg 6480
   gaagtttagc ctgttaactt tcaggaagtt ttgtactcag attcccacag tcagtattct 6540
   gccagtggtg gtgtttcatt ttgttcaaag atatataaat ttttcttttc ctttttctat 6600
   atttcttttt tttttttttc tcttagagcc attgctgctg agattgttct aaaggacaaa 6660
   agagaggtcc accgtttata tcagagagcc ttacagaagt tacctctttg tgcatcactg 6720
   tggaaagatc aactcttgtt tgaagcatca gaaggaggta aaactgataa cctgagaaaa 6780
   ctagtttcca agtgccaaga gattggagtc agcctaaatg agctcttaaa tttaaacagt 6840
   aacaaaacag aaagcaagaa tcactgaaca ctgggtgcag tcagttctaa gtccttataa 6900
   taattgccaa aattatttga atgattcttc aagattaggc tgatccctgg ctaaggtctg 6960
   tgtaaggcag acaagcgtta ttgatcatat caagttccct acaatatcct gtcctcaaaa 7020
   ccggaagcaa tgaacatgat cctcttcggt tggataaatg aacttcctgt ttggcctgct 7080
   tctaggccct gccagattct cataacatca tatacgtaag tatagttcct caaagtgact 7140
   gacatttatt ttaattttgc tttgtttttt tttattttct cccccattcc tttattttgt 7200
   gttattcctg actcacttga cactctctga tgcctgagag attcctgttt gggatttaat 7260
   atccagggct gtgtttacag taaaaaaagc aggcagtccc ttttagtttt tcctttttaa 7320
   atttttttga gattcttcat ttcaggattt aaaactatag cagtccatct taaggaaagt 7380
   gtaactgcca tggccacaag tctgctagtt gcacttgaat gctctatcag ggttgtttat 7440
   taccctttct acgttctgga ctccttgccg agactgttta acttgaagat taaagaaact 7500
   attgcaaatg ccagtgcatc agaacctaag agtggtcaaa tattatgtgc aatttttttg 7560
   taaagaaatt ttaatttata ataaagttta acagttt 7597
<210> 3
   <211> 14709
   <212> DNA
   <213> Homo sapiens
<400> 3
   agggaattct ctggggcttt ggggaattta gtgcgtgggt gagccaagaa aatactaatt    60
   aataatagta agttgttagt gttggttaag ttgttgcttg gaagtgagaa gttgcttaga   120
   aactttccaa agtgcttaga actttaagtg caaacagaca aactaacaaa caaaaattgt   180
   tttgctttgc tacaaggtgg ggaagactga agaagtgtta actgaaaaca ggtgacacag   240
   agtcaccagt tttccgagaa ccaaagggag gggtgtgtga tgccatctca caggcagggg   300
   aaatgtcttt accagcttcc tcctggtggc caagacagcc tgtttcagag ggttgttttg   360
   tttggggtgt gggtgttatc aagtgaatta gtcacttgaa agatgggcgt cagacttgca   420
   tacgcagcag atcagcatcc ttcgctgccc cttagcaact taggtggttg atttgaaact   480
   gtgaaggtgt gattttttca ggagctggaa gtcttagaaa agccttgtaa atgcctatat   540
   tgtgggcttt taacgtattt aagggaccac ttaagacgag attagatggg ctcttctgga   600
   tttgttcctc atttgtcaca ggtgtcttgt gattgaaaat catgagcgaa gtgaaattgc   660
   attgaatttc aagggaattt agtatgtaaa tcgtgcctta gaaacacatc tgttgtcttt   720
   tctgtgtttg gtcgatatta ataatggcaa aatttttgcc tatctagtat cttcaaattg   780
   tagtctttgt aacaaccaaa taaccttttg tggtcactgt aaaattaata tttggtagac   840
   agaatccatg tacctttgct aaggttagaa tgaataattt attgtatttt taatttgaat   900
   gtttgtgctt tttaaatgag ccaagactag aggggaaact atcacctaaa atcagtttgg   960
   aaaacaagac ctaaaaaggg aaggggatgg ggattgtggg gagagagtgg gcgaggtgcc  1020
   tttactacat gtgtgatctg aaaaccctgc ttggttctga gctgcgtcta ttgaattggt  1080
   aaagtaatac caatggcttt ttatcatttc cttcttccct ttaagtttca cttgaaattt  1140
   taaaaatcat ggttattttt atcgttggga tctttctgtc ttctgggttc cattttttaa   1200
   atgtttaaaa atatgttgac atggtagttc agttcttaac caatgacttg gggatgatgc  1260
   aaacaattac tgtcgttggg atttagagtg tattagtcac gcatgtatgg ggaagtagtc  1320
   tcgggtatgc tgttgtgaaa ttgaaactgt aaaagtagat ggttgaaagt actggtatgt  1380
   tgctctgtat ggtaagaact aattctgtta cgtcatgtac ataattacta atcacttttc  1440
   ttccccttta cagcacaaat aaagtttgag ttctaaactc attagaattg ttgtattgct  1500
   atgttacatt tctcgacccc tatcacattg ccttcataac gactttggat gtatcttcat 1560
   attgtagatt taggtctaga tttgctagct ccaagtaatt aaggccatgt aggagagcat 1620
   ggtaaccaca gatagaactg gtattatccc aagtggtctg cagactgctg agtggggatg 1680
   ggatctgctc tctgttgaga gttggtaatc attggtttga aatgtgatga aaccactcaa 1740
   gccaatgaag gtgggtgtgt aggtggggag tactttgcca taatatttta aaacattacc 1800
   tggttagagt tctaagtggt acttattttt gtttggttag gggaaagcct gaataaaaac 1860
   agaaatggac acataatatg catattccat agtctttggg aggctggaat gtgcctggga 1920
   tttgggtcta agtgtatgcg taattcttac ctcactaaag aatttgcctt gtttttttcc 1980
   ttttggtgag tgactaaaac gtctgggctt ccctgtgtgc gtgctacagt aagcaagcag 2040
   aggctgtgca aaggtgtgag caggatcacg tggaatctgg aggatacatc ttggcttgca 2100
   aactgcctct gtctcctggg tgggactgtt ctgtccttgc actgctgttc tgtgttacct 2160
   cttggggtgt aaggttttgc ttacaggaga caaactttgg gcgtagaatg gaagccactg 2220
   ccagcctctg tgctgagaag gaaggtgctt gtttcaaagg gagcagcaag ggaggcttgt 2280
   tctactcacc tgggcctgtt tgcctgagaa ggggagataa gggctgaact gggactagcc 2340
   agggggacca acacaaatgg tgggggatca tgacctgaag gattctttcc ttcccatgag 2400
   ctgcagggct ggttgccgtc cttgcaactg tgtcttattt gcctgtgccg ttatatcttg 2460
   gtgacccctc cacgtgtaca ctactgacaa acgggtggag tgctggggag aagtcactgt 2520
   gccgcccacc tagtaaacct tctgtctgtg ctcatggcat ctccaagatg gggcactgct 2580
   gtgtgcagaa tccagggtcc tctttctgct tgcaactcct ttccctggat gccccagaaa 2640
   caatccaggc ctcctttcct atcttacccc tttgctttgc tttttacccc agcacctcta 2700
   taaccgcctt ctcttctttt cagaactcct tgtttctcat cctgtttttt atgattacaa 2760
   aactcttgct tccaccctgg aagataactg ctatagatgc ctgtatgtaa atggtgctgt 2820
   ctccagcaac tggcatgctg aagaagaatt gattcacggg gtataaatgt tggggattgg 2880
   aagtggggat gaaatggcac ttgttgatac aggagcagag aggtgaggcc gactgctgaa 2940
   gacagctcgc caccctcctt gcctccactc caatccaggg gctggggcca cattctttgc 3000
   cttcatttat cctcagatca ggtgagatcg acaggaggtg ttgatggcag tgccagcaat 3060
   tattgctaat ccgtttgcat ccttatgcat agatctgaat tcagactttg tgaatttcca 3120
   gaggtgtggg taatataata gaattcagtg agtgggcatg gctgatcttg tgcaaattaa 3180
   aagttatggg gcataagaat agcaaaagtt gaacttcttt taaaaaggaa agtaccctga 3240
   gagccagtat tggttgaggc tcttcagtat gcccaggttg gcagcactga gaaccgcagg 3300
   aacggcctgt tgttacaaaa aggagattga ctcagctgcc cttggtgcat ctgactgact 3360
   atgactgctg agagattcca aggaccctta atgccagggc taacctctcc atgtgcagtg 3420
   agacctctgg aggaagtgtc atcctctggc tttgtgtggt actcattatg gtgcagtgcg 3480
   ggcatgaaat gaagacaccc aaataggctt acagatacga tatgttttaa atgttcgtat 3540
   ttaacaaaaa catactgaca ctgtttggaa atggcaacag gaagatagca aaatgaatac 3600
   taacattacg aaaagatgaa caggtacatg ttccaaggca ggtggctgtg aacttcctct 3660
   gagtgaaggc atcccctcca gcacctttca gcctgctagt taggacgacc cgccgccacc 3720
   ctccaggacc tccagccctg cactgccttt cctctctttt aaataattct tcattgagtt 3780
   ctaatatgta aaaaaaaaaa gtttactgta aagtttgcaa ataaggaaat tttttttaaa 3840
   agtcctcagt aatcttacca gtaacaattg ttatgggcac atttgctttt ggaagatttc 3900
   ttttgtatgc atgggataag tacattttta aacaaaaatg ggattatgcc ataaattcta 3960
   ttttgtgact ttaatatata gtgaacacct tttttaatga tgacaggatg ttcccttgca 4020
   tggctgtatc aatttaaaca atcttgtttc aatgggcata cagggtattt tctagttttt 4080
   ttttcctctt agaaaataat acttgcgatg actttccttg tagctcagac tttttcacgt 4140
   ctgttgttat ctctttggga atgctgaata catacatttc gagaaggaaa tgactgttaa 4200
   actcttaaga cttcaggttc atattgctaa actgcccagc agggagggat tttttcaatt 4260
   agtgttctca ctggtgaggc aaacctgatg ccttcccctc ttcctcagaa ccggctttat 4320
   cacattgaaa acctttgctc ctccgacgga tcgagtctgc tttccctgtg gatgtgagca 4380
   ttgctttgtc tgctggtgac tgaacatctc taccatgtgt caattggcca tttgtggtgt 4440
   gtgtgtgtgt gcgtgtgtgt gtgtgtgtgt gtgtgtatga ttttctaatt cctagtcatt 4500
   tttctattga ttgttttgca aaagccattt acatcttaag gatattgata atcttttgtt 4560
   atatttgatg caaatatttt tttccagttt ataggttgcc ttttaatttt gtgtttcagg 4620
   tagataaaag ttaaacgatt ttcttaggtt agtttatcac tgtggtttct gaacttgtta 4680
   tgtgtagatc ttttccaccc caagagtaca taaatattaa tccatacttt cttatggaac 4740
   ttgtatggtt tcgtttttta catttaaacc ttcttccccg tggtgtgtgt tgtggaatct 4800
   gtgtttgtgt gaggaggggc atggtgctct cagaacccac ctcctgtggc cagagagccc 4860
   tgtcctgtga gggtggttat cacagtggca gggttcaatt cagaagacct tgagggcagg 4920
   ctgatgtttc ctgaatgggc ccctggttgt tgcttgtccc tgactctcca tttccccatc 4980
   tgagtggatt tggacctaat agggcactgg agctggttcg aatcctgact ggactacttg 5040
   gcaactttat gtctgggagc aagttactta acctccccaa gcctgtgtct gtgaaatgcg 5100
   ggtaaatgaa tgtagatgtt tggcagcagc tactccttgt tgagctctca cagtgaactc 5160
   tcctgcctct gccctccttc cccgcctccc ctggtgccta gcgtcaggtc tagccacttc 5220
   ctcctgggcc cctctccctt ttctgtggct ggctgcctgc ccgcctggcg ctggaccttt 5280
   catgtaacgg gaatcagcat gtatattctg gtctggtctg tttctacact taattttgtt 5340
   tccagtagta tttccctgta ccggcagagt tcacaaacac atttgaagag gctttttctc 5400
   aggattctta accttcccaa aggaagtccc atggatgggt ttctagaagt ctataaatgc 5460
   tctgaaattg tatttttctg tggaaagcat aactttcatc tgcttgttcg tgctcaaaaa 5520
   agatcatgaa tgaatgattg catgatttta tgccattgtg cttatactaa aggatatgta 5580
   gcccatctct tgagctgtta aactgttttg actactttaa atcgtgcagc tgtgagcatc 5640
   tctgtaaatt tagtgtacac atgtatcccc tggagtggca ttgcctcggc agtgagcact 5700
   tatggtttta taactctctt cacagactca aatgactcca gaaagctaca cttcctgttg 5760
   tgagtatatg atatccattt ccctacatag ccactaacat caggttttta caattttatt 5820
   tatttcttgc tactttaaga aatttttgtg gtgaaataca tataatagaa gttgactatc 5880
   tgaatcattt ttaagtatac attcagtagt gttaagtatg tcgccattgt tgtacaacca 5940
   atctccagaa ctttttcatc ttgcaaaaca aactctgtac ccattaaata acattaaaca 6000
   ttccattccc tccagcctca gcaaccccat tctactttct gtttctgtga gtttgactat 6060
   tccaagcact tcatatcagt taaatcatga agtatttgtc tgtctgtgac tggcttattt 6120
   ctctgagcac agtgtcctcg agatgcgtct atgttgtagc atatgtcaga atttccttcc 6180
   tttttaaaag atccaaataa tattcttatt ttatatcttt tttttatcca ttcatccatt 6240
   agtggacact tgggttgctt ttggctattg taaataatgg tgctatgtac aaatatctat 6300
   attattgtat ttacaagtat aatgctgtaa tgtacacaca tctttttgag atcctacctt 6360
   cagttctttt gagtatatag ccagaagtgg tattactaaa tcttacgata tttctatttt 6420
   taatttattg aggaaccact gtagtttttc atagcaactg caccatttta cgttctcacc 6480
   aagagtgcac aagggttccg aggttcccac atcctcccca acacttgtta ttttctgctt 6540
   tttttagatt gcagccatca tagtgggtgt gaggtgacat ttcattgtgg ttttgatttg 6600
   catttcccta atgaggagtg atgctgagca tcttttcata tgcttactgg tcatttgtat 6660
   gttgtctttg gaaaaatgtc tattcaagtc ctttgactat tttaaaaatt gggttattag 6720
   agttatcgtt gttgttgact tgtaggagtt tctttctata ttctggatat taatccccta 6780
   tcagatatat gatttgcaaa tatcttctct tattccataa ggttactttt tcactttgtt 6840
   gattgtgttc tttgatgtat agaagttttt agttttgaaa tagtctaatt tatctgtttt 6900
   tacttttgtg gtctgtgctt ttggtgtcat atccaagaaa tccttgccaa atccaacgtt 6960
   ataaggtact tttaaggtat tttagttgtc ttagtctata tttctgtact cacctttctt 7020
   tatccactca tcagttgatg ggcatgtagg ttggttccat atctttgcaa ttctgaattg 7080
   tgctgtgatc aggtgtcttt ttagtataat gatttactct cctttgcgta gatacccagt 7140
   agtgggattg ctggatcgaa tggtttttat aattttctat tttaccacag tttctctctg 7200
   catttttcct ctttgaccac taaccatgtg aaattctcat attgaccttt ataatgatca 7260
   tgaactctta gtatcattgg gaaggccaca tttgccactt atgattgtaa accttatcct 7320
   ccatttttcc tgttattgtt ggtgcaaaaa gcacctatta taccaggact ttaaaaatca 7380
   gtctgataag tctttgataa gtctaataat aataactgat aagtccattg aatttgcttc 7440
   tgattacttt ttctttagta gctaaacatg tatgtactcc tatgattaca atgaacactc 7500
   ctctccattt aaattaatta tttacattga tgaaatagca aaatgttaat gactaaatac 7560
   tgtcttggtt ttttcgttcc aggtcagtca atattaactt cttataattt tctttttttt 7620
   ctttatgtgt gtgtgtgtgt gtattttttt ttttttaatt tcaatggctt ttggggtaca 7680
   aatggctttt ggtcatatag atgaattcta cagtagtgaa gtctgagatt ttactgcacc 7740
   ggtcacctga gtagtgtaca ttgtacccaa tatgtggttt tttatacctt gcccccctct 7800
   taccctcccc actttgagtc tctagtgtcc attatgtcac tctgtatacc tttttgtacc 7860
   cataagttag ctctcactta taagtgagaa cacacagtat ttggttttcc attcctgagt 7920
   tgcttcactt agaataatat cctccagctc catccaaaat tgctgcaaaa aaaaaaaaaa 7980
   ccacaaacat tattttgttc ttttttattg ctaagtcata ttccatggtg tagagatacc 8040
   acattttatt tatccactca ctggttgatg ggttggttcc acatctttgc aattgtgact 8100
   tgtactgcca tcaagtgtct ttctggtata atgacttctt ttcctttggg tagataccca 8160
   ggagtgggat tgctagatca aatggttctt aacattttct ctctggatct atttctggaa 8220
   attttaggct ccagtttttg ttgttgttgt taataaaatg caatggaatg taatgatcat 8280
   cacttttcat tatgctttaa aatctggtaa atggaggcta gaacactcct gtaaggcaag 8340
   aatattctct ctgttggaac tcaaatacac agaactgggt aaatctcaat cttaatcttt 8400
   gattcaggac acaacatggc tctcttttac ttgctttctt taattgtttt ttaataatgt 8460
   ggtaagcatt tctgaatctc ctatccaata caaaaactag gacaatacag acagtaactc 8520
   ctatggttac aatgaacact cctctccact taaattaatt atttacactg atgaaattga 8580
   aatagcaaaa ttttaatgac taaatactgt ctttgatttt ttgttccagg tctgtcaata 8640
   ttaacttctt ataattttct ttttttttct ttatgtgtgt gtgtgtgtgt atatatatat 8700
   atatttaatt tcaatggctt ttggggtaca aatggctttt ggtcatatat atgagttcta 8760
   cagtagtgaa gtctgagatt ttactacacc ttccacttat gtggtcccac accacccgcc 8820
   tcccctgccg cctcctgcca ccccctaggc caaggtaata atcatcctga atcctgggtt 8880
   tatctctcac ttgctttctt ttcatataat tttgcaaaag aatctgatct aaatgtgttt 8940
   ttcagagtat atatttatat tttagctgtt cttagagaaa atttattatt ttgcatgtaa 9000
   tcttatggaa cattctcatt taataccatg gtaagattca gcccttgccc aggggatagt 9060
   tcatttagtt tgtttactgg atagagctca tcatgtgact atacctcagt tagtttatca 9120
   gttctcccat ccatggtgac taggttgcct ctcagcctct caacaacact gtttctcagt 9180
   gtccttgtag aagtgatatg tgggtgtttt ctccttacac agagttgaaa ggtgacgaca 9240
   acaacgttgg cactaccaat cccccaccct ccagaggggt aaccagtgtt accagtttgc 9300
   tgtgtttcct gctacacctc gccttattca cttccatttg tatctgaaaa acgtgttgca 9360
   tggtttcttt tctatagaag tggtaaaatg ctattgtgtc ctgtacatta ttgattactt 9420
   tttttcattt aacagtaggg agatgcctgg gagtacacag agaactgccc tcattgtttt 9480
   caacttctgc actgtatgtc tgtgagttta gccattctgc tgttaatgga aatttacagt 9540
   attctaatct tttgatatta caaacagttc tgtgcgatca tcgtcataca caaccccttg 9600
   tgcacaatgc atgagtgttt ctcagggtag gtaccaagaa gtgaaattcc tgggtcatag 9660
   ggcgtgagtc cgacattttt ctccattctg ccctgttgcc ctccagagtg ggtgtccagc 9720
   tttgcatacc taagtatgag agtatctgtt gttcatatcc tctacgacgc tccatatatg 9780
   aaacttaagt ttctgctagt tgccatcttt gatctatcat gtatgcagtg acctactaag 9840
   actgtaattg gtacagtaga ttcttgtcat ctgtgtgtga atttagcatt catgggctta 9900
   atgctgacaa ggcccccagg gtccaagaca tataatcatg tataattttg tcaaggtata 9960
   attttttaaa ttgcttttgt catgtgtctg ctggtgatgc ccaacccagt gctctgcacc 10020
   caggtcacac tgtggctttg tcctctgctt atgcctgcat tgcagcaact gtcctgaaga 10080
   gaccaaaatt atgcagattt aggtaagtcc atggctaatg ttattatatt atgtgctatt 10140
   gtaatggatg gggctgtgga gtgtatgaat ttataaatca ctggtcttgt aattaaaatt 10200
   caaacactat agaaaaaggc catgtagaag ataaaagttc ctctataatc ccggacccct 10260
   aagataacta ctaatgacaa cttcatttat attccttcag acattttctg gctgtggatg 10320
   tactaaaatg tatcctatta ttctctgccc taaaatggaa tcatacaagg tgtactgtta 10380
   tttttatggc tctataacat gtcatattgt acgtgttggt atggtcattt taaccatttt 10440
   tctagtgatg gctttgaggt tatttgcagt ttcctagcca tctcaaagtg tgctgcgggg 10500
   atctcttttg catccctctg ggtgcagagc tgaggcaccc agaggcagtg tccagaggag 10560
   gcagcatctg taggtgtctt cacctgctct ggctcttggc acatctggtt ggtgacactg 10620
   ttttgtgaga tgggttgaaa gcacgtgctg ccaaaataga ataatgttgg tcctctcctc 10680
   atgtgccgtg gaactggggt aaaactgcgt agtggctgca gctgcctgtc cataccggaa 10740
   tcgagtataa cacggtgcct ggcttagcac aaaacagtag tgggtcctgc aggccccaga 10800
   gtctaattcc tggtattctt tcccctacac agattaaata aaccaaaaac aaactattct 10860
   aggaaagcgt ctgtgacatt tgtaaaaagt ggtatttaat gatcttttat tcacttgtct 10920
   gtttagtttg ttgaaatctt aagtggcatc ctggtctggg aaggagtgct gtctgcgcct 10980
   gccctccgct gggcacagcg tggctgcttc aggggctaag cacacacttt ctgtcttcta 11040
   aagggccgcc acatgccagg agctcaggtg tgagcccggc tctggctctt acctcatagg 11100
   gtcactcata ggggcacagg gagcagaaca ttgtacacag cgaggcacca cccggcttgg 11160
   catctgcctc ggtggactta ctacctctag aaggaaatac ctgagttcct ctggcctcag 11220
   ctcctagagt gactggtgtg ctgtccctgt tactcttctg tcaaggtgac aactgtgtga 11280
   cccatcatct gtgtgtcaaa gcaaggccct gcctgggcct ctgctcctgt gctgacccca 11340
   aaggcaaatg ctttgctagt ttccttccag ttaatttcac ctatgaatag atgtgtgaaa 11400
   actgttcaaa gccatacctg cacatgtttg aacttcaaac cctgtgggtg attcagtggc 11460
   atctttctct aacccccagc ctcccttccc acagaggcca ccgtcatggc cagttgctgc 11520
   agtttctttc cagagaacct gtgtatgtgt aaagctgtac aggcgtgggt acaccacaca 11580
   gcctgtcttg cactgtggac tgttgagtta ctagtacatc taggtaagca ccgcatatct 11640
   gtattcatgt ctgccttggt cttttcaaca tctgtgtggt agccgtgttt gaattaccca 11700
   ttcccttttt ggggaaccat taagttgttt cagcaatttt tactgtagat aaggctatac 11760
   cgcatatctg tgtacatggg tttttatgta catgggcaag tatatctgtg agagaaaagt 11820
   ttcctcagga ggaattctgg gcacagcatg tgtaaatttc taaatatgat ggacaccccc 11880
   agcttccacc tcaaggaggt tggtcccatt gacatttccc cacaccttca cccaggctgt 11940
   gcccttaaac ttggttattt gtcaatgtga gaagtggaaa atagtattta attgtagttt 12000
   ggatttgtat ttctattggg ttgtatactt actgattaat aataagagct ctttacatat 12060
   taaggaaatt aacccttttc aaatacattc ctatttctca ctaatcttta agttttattg 12120
   taatattttg ctctttagtt tatatatata tgtatatata tatatatgta tatatatata 12180
   tatacatata tatatatata tatatatata tatatatata tatatatata tatatatata 12240
   tatatatata tacatatata tatacatata tatatactaa ttttctttta tggttcctgg 12300
   attttgtgag tagtttgaaa aggctaatcc agctgaagat tttgttgttg ttgttaaacc 12360
   ccatgttttc tcctaactct ttttattttt attttggagg actctatcta gacttaattt 12420
   tagcataaca agtgacaggg ttagttagcc tgttgtcctt acaccatttt ctggctaata 12480
   cagctattaa ctattgatct gtctattcac gtgccagttc ctaatggttt tacatagtgt 12540
   aatctgcact tcaaaatagc gaagggaagc cctacctcat tattctactt ttccagaatt 12600
   ctcctggcta ttccaggctg catgtttacc ttaaccttcc ctgtgatgtc ttcatgccgt 12660
   tgtcttctta tgcaagaata aggtacgtct ttccatccac tcacgtctat ttaatttgac 12720
   tttgcattac acagaaagct ggtcttggtc tgtctacctc ggcatctagt tgtcctcact 12780
   gccccctagc cgaccccacc ccatctgact gactacccca tcacagagta cttttattta 12840
   cgttttgctc tgcctaatgg ttacttgata ctgtcacgcc gacagtgtcc agttcagtgg 12900
   tctttgcagt tgaaatgctc ccgtacacac tgtcttgtta aaaatgccag taagttcata 12960
   caaacccagc ttgcacccaa ggtcacattc agagagcgta gggctgggat gggttgtttt 13020
   ccaagcttct gccactgtgt ggctagctct tcccactggg aagttctgtg tacccggaat 13080
   gtcggagtgg agtcctgttc tagtgtccag cacctgaccc tgtgcccaac ccctcaacag 13140
   cctattcctg ctgtccacag cctgctggaa ctttttacaa aatatgttgc catgctggac 13200
   cctgggcact ggacataagc cccctggcag cctttttcat gtcacccaaa ggggtaattg 13260
   tcctactggt ggtctgtaag atgagttagg gtgacttgct aatagacatt gtaaatctta 13320
   atatttatgt atgtatttta ttattaccgg ttttccattt atgatggtaa tattgtttct 13380
   tctaagaata tttatttttc cttctaaata ttgagataaa attcatgctt ttgaaatgtt 13440
   ctattcagtg gcttttagta tatttgctat gttgtgcaac catcgacact atccatttct 13500
   agaacttttt cgtcatccca aacagacgct ctgtattcat aaaaaaataa cttcctacct 13560
   gtctctcccc ctagtctttg gtaacctttg ttatactggt aaactttgtt gtgctctctg 13620
   tctgtgtgaa tttgcctatt ctaggggcct catataagtg taatcataca gtatttgtct 13680
   ttttgggtct gtctgatttc acttagcggg ttttcagggt tcattcatgt tgcagcatat 13740
   aacagtactg cgttcctttt tctggctgaa taatattcca ctgtatggat agaccccatt 13800
   ttgtttattc acacatcatt tggacatttg gattatttct ggtttttggc tattatgaac 13860
   aatggtgcta tgaacagttg cgtacaagtt tttgtgtgaa catatgtttt caattctctc 13920
   attatatacc taggagtaga attactgggt catatggtaa ctgtatattt ttgaggaact 13980
   gccaaactat tttcccacgt ccatgcacca tttcacattc ccaccagtaa gtaagagggt 14040
   tccaatttct gcgcattctt gccaacacta gttattatct gactttctgg ttataatcat 14100
   tctaatgagt gtgaagtagc ctctggtgtc atttggattt gcatttctct gatgagtgat 14160
   gctatcaagc acctttgctg gtgctgttgg ccatatgtgt atgttccctg gagaagtgtc 14220
   tgtgctgagc cttggcccac tttttaatta ggcgtttgtc tttttattac tgagttgtaa 14280
   gagttcttta tatattctgg attctagacc cttatcagat acatggtttg caaatatttt 14340
   ctcccattct gtgggttgtg ttttcacttt atcgataatg tccttagaca tataataaat 14400
   ttgtatttta aaagtgactt gatttggctg tgcaaggtgg ctcacgcttg taatcccagc 14460
   actttgggag actgaggtgg gtggatcata tgaggaggct aggagttcga ggtcagcctg 14520
   gccagcatag cgaaaacttg tctctactaa aaatacaaaa attagtcagg catggtggtg 14580
   cacgtctgta ataccagctt ctcaggaggc tgaggcacga ggatcacttg aacccaggag 14640
   gaggaggttg cagtgagctg agatcatgcc agggcaacag aatgagactt tgtttaaaaa 14700
   aaaaaaaaa 14709
<210> 4
   <211> 6111
   <212> DNA
   <213> Homo sapiens
<400> 4
   aacaggtttg atctgtggat gaaatgaatc atgattttca agctcttgca ttagaatctc      60
   ggggaatggg agagcttttg cctaccaaaa agttttggga acctgatgat tcaacaaaag     120
   atggacaaaa aggcatattt cttggggatg atgaatggag agagactgca tggggagctt     180
   ctcaccattc aatgtcccag cctattatgg tacagagaag atctggacag ggttttcatg     240
   gaaacagtga agtaaatgca atactgtctc cgcgatcaga aagtggaggc cttggtgtga     300
   gcatggtaga atatgtatta agttcttctc ctgctgataa attggattct cgatttagga     360
   agggaaattt tggcactaga gatgctgaaa cagatggacc tgagaaagga gatcaaaaag     420
   gcaaggcttc tccatttgag gaggaccaaa acagagatct taaacaagga gatgatgatg 480
   attctaaaat aaatggcaga ggtttgccaa atggaatgga tgccgattgc aaagatttta 540
   atcgtactcc tggaagtcgt caagcctctc caactgaagt agttgagcgc ttgggcccca 600
   atactaatcc ctcagaagga ctggggcctc ttcctaatcc tacagctaat aaaccacttg 660
   ttgaagaatt ttcaaatcct gaaactcaga atctggatgc catggaacaa gttggtctgg 720
   aatccttaca gtttgactat cctggtaatc aggtaccaat ggactcttca ggagctactg 780
   taggcctttt tgactacaat tcccagcagc agctctttca gaggactaat gcactaacag 840
   ttcaacagtt aactgcagct caacagcagc aatatgcatt agcagcagct cagcagccac 900
   atatagctgg tgtattctca gcaggccttg ctccagctgc atttgtgcca aatccataca 960
   ttattagtgc tgctcctcca gggaccgatc cgtatactgc agcaggattg gctgcagcag 1020
   ctacattagc aggtccagca gtggttccac ctcagtatta cggcgttcca tggggggtgt 1080
   atccagccaa cttatttcag cagcaagctg cagctgcggc aaataacaca gccagtcagc 1140
   aagcagcatc acaagctcag cctggacagc aacaggttct ccgtgctgga gcaggtcagc 1200
   gtcctcttac tcccaatcag ggtcagcaag ggcagcaagc agaatcactt gcggcagctg 1260
   cagcagcaaa tccaacattg gcttttggtc agggtcttgc tactggcatg ccaggctatc 1320
   aagtactagc tccaactgcc tattatgatc agactggtgc cttagtggtt ggccctggag 1380
   caaggactgg ccttggagct ccagttcggt taatggctcc aacacctgtt ttaattagtt 1440
   cagcagcagc acaagctgca gcagcagcag cagctggagg aactgcaagt agccttacag 1500
   gcagcacaaa tggtctgttt cggccaattg gcactcagcc accacagcag cagcaacagc 1560
   agccaagcac taatctgcaa tctaattcat tttatggaag cagttctttg actaatagct 1620
   cccagagtag ttctttattt tctcatggac ctggtcaacc tggaagtaca tctcttggct 1680
   ttggaagtgg taactctttg ggtgctgcta taggctcagc cctcagtgga tttggttcat 1740
   cagttggcag ttctgcaagt agtagtgcca caaggagaga gtctctatct actagctctg 1800
   acttgtacaa aagatctagt agcagcctag cacccatagg gcaaccattt tacaatagtc 1860
   tgggatttcc ctcctctcca agtccaatag gcatgcctct gccaagccaa actccaggac 1920
   attcacttac gccaccgcca tcactttcat cacatggatc ctcatccagt ttgcatttag 1980
   gaggactgac aaatggtagt ggtcgatata tctctgcagc acctggagca gaagcaaaat 2040
   atcgaagtgc ttcaagcact tccagtctat ttagctccag cagccagctc tttcctcctt 2100
   cccggcttcg gtataatagg tctgatatta tgccttctgg ccgcagtaga ttattggaag 2160
   atttcagaaa caaccgcttc ccaaaccttc agcttagaga cttgattgga catatagttg 2220
   agttttctca agaccagcat ggttctagat tcatacagca aaaactagag agagctactc 2280
   cagctgagcg acagatggta tttaatgaaa ttctgcaagc agcctatcaa ttaatgactg 2340
   atgtttttgg caactatgtt atacagaagt tttttgagtt tgggagtctg gatcaaaaat 2400
   tagccctggc tactcgtatt cgtggtcatg ttctaccctt agccttgcag atgtatggct 2460
   gccgcgttat tcagaaagca ttagaatcta tttcttctga ccagcagagt gaaatggtaa 2520
   aggagctgga tggtcatgtg ctcaaatgtg tgaaagatca gaatggaaac catgttgtac 2580
   aaaaatgtat cgaatgtgtt cagccacagt cactacagtt catcattgat gctttcaagg 2640
   gacaagtatt tgtgctttca actcatcctt atggctgcag agtaattcag cgcatcctag 2700
   agcattgcac tgcagaacag accttaccta tcttagaaga actccaccaa catacagagc 2760
   agttggtaca ggatcagtat ggcaattatg ttattcagca tgtactggaa cacggtcgac 2820
   ctgaagacaa gagcaaaatt gtttccgaaa tcaggggaaa ggttttagcc ctgagtcaac 2880
   acaaatttgc cagcaatgta gtagaaaagt gtgttactca tgcctcccgt gctgagagag 2940
   ctttactgat tgacgaggtt tgctgccaga atgatggtcc tcacagtgcc ttatacacca 3000
   tgatgaagga ccagtatgcc aattacgtgg ttcaaaagat gattgatatg gctgaacctg 3060
   ctcagagaaa gataatcatg cacaagattc gacctcacat tactactttg cgcaaataca 3120
   catacgggaa gcatatactg gccaagttgg aaaagtatta tttgaagaat agcccggacc 3180
   taggacctat tggaggacca ccaaatggaa tgctgtaaat tacaggagca agagaaagaa 3240
   gataatttaa ccatgtgaaa agaatttttt tgtgtgtgaa ttatcaaaac acaactcaac 3300
   tatgaatctt caattttttt ttaaagcaaa actatttatt gactttattc atccatttgt 3360
   aaatttttta aggttcttgt gtatatttgg ggggtggggg atgaattata aattatattc 3420
   agccctgagt ggagacctat cagattggat tgctggcaaa gcacagaatg cctgtatatg 3480
   atgtaactgt atcaaaaata aaaagctgtc acatattttg taaattttta ccttgtaaag 3540
   tcacaaaaat agtttttaaa ggaaaaagta cagtattctt ttaataaact ggctcacagt 3600
   ctggtaggtc tacaacccca tagcacaaca ggtttataga gatgtatata gaattatagt 3660
   ccttattttt ttcctttgcg tgaaaccttt tataacagat taacaatcaa ctgcataaat 3720
   attattaata ttttaaaaag agttaagttg tattttgata attcacaaac tatcatgcaa 3780
   ataacgagta agtagacaag aataaagtgg tttgagatga aaagaaccta acattattta 3840
   cagtagatgt ggttttaata caattactgc cctaaaatgt ctctggcaat gtacagaaat 3900
   attgtatata cttacatatg taattgttgt aagagttaaa tacaaaatca tggtgacact 3960
   tccaattaag tgcactaaat gaaaagttaa gtcacttatt aacttttcag tttggtttgc 4020
   aatgagaaag agtggaaatt tgtattttgt tttgcttata gaattacaga catgttgagg 4080
   aagtgttgag ctttattttg ctttttcata gaggcagaaa gtaggaacca gatagagatg 4140
   aaaaggggcc actgaaaagt gaatttgata gctcagcatt taagcatgat tacatattca 4200
   gatagctctt tttgctttct ataaatatat gcattgtgtg tgtagtaata gatgtaagtt 4260
   tacactttga aaggaaatct tgtttcaatg tttattataa aagccttgct aatttagtag 4320
   tgatgctttc cttggttgta caggtgtaca tttgtaaacc ttcatgctgt aaatggaatt 4380
   tgttttatct ctttgggata catttgcatt ttagtgtaca tttacgtccc tgccctcttt 4440
   gacctggcaa tatagtgttg tataatgtaa atttatttct ccaaatcgag agtgattttt 4500
   taaaaatttt ttatctttat atggtttcag aagtatgaac cagctttctt tttattattg 4560
   tgagatcatt ttgttttata acatagttgt tgactgttaa tatggacctg ctagaatttg 4620
   gatcactttc aattgaagtc agggtattgt gcataataga aagtattgga ctgagatatt 4680
   tggttaccat ggaggccaat gcttttttca tcttattaaa tgtgatgtga cttttttctt 4740
   tgtacagaag agtactgtat ttttgaatag cctactccca agtaagagca aatctgtatg 4800
   ataacatttt ttcctctgga cataagacat aacagtaaca cgatgtacat ttacaagcgg 4860
   ccttatgtac atttcccaac aatcttttta aggcaaaatt gtgaccatat gtgtataatt 4920
   aaaatcgttt ttaatccttt gcctatgaaa atattttgga aaaaaacttg ctgtgtatat 4980
   tcagtttctg aaagataaag aaagtgcttt gtattttgtt gaagtcagta ttttgtataa 5040
   acatttatgt tgacccactt atgttcagtg ctgaaaacta aaatgaacat gctattctgt 5100
   cagctgaata tggaagagat ctttttttac tagagatctg cagaagaaac gcaatcttct 5160
   gagcacaata tggaatctaa aggttttatc acttagttgt tcatattatg aacctaaaaa 5220
   taatggcata aagtttgggg atgccaggca tactttttca tgtttggtgt tgagttattt 5280
   tacttttcta acccaacatt ccttggtgag accattaaat ccaaacactt gtcaccgttc 5340
   cttctcatag tcactctggg tcatcagcat gtcccagtca ctgcagcaac gccttgtgtt 5400
   tgtttcattt ttttaaaacc cacacaaagc cgctgtctca ctttttccta ctttaccaac 5460
   ctcagagtat ttcggcccgt atcgaacttt tgttctcagt atcagcccat ggtttcagga 5520
   tcaaagctgt catgttggag attggtaatg gctttcctgt ctttgtacag ttgaattcct 5580
   agtcttcctt catccttgcc ctctgttggc acaggcatta tctctgcaat tttagaaaat 5640
   gacaagtaga gaatactaca ttgagaaact aaaccctctt cttggggtcc tgatactcat 5700
   tcccatttgt cccagtgctg acaacccaat cttcccaata ctttcaggcc tgctctacaa 5760
   aagtacctgt tcttgtagaa attttacagt ctgccatttt gggtgcccac cccaattttt 5820
   accttttagt aagttggcat gaaattttgg taaaatctga aaatcacatt tcagaataaa 5880
   acaattgggc aaaactacct aggctttact cttgagtgtc tccttttgat agggattgtt 5940
   tctggaccag tttgtctaag tcctggctct tattggttca tatgaaataa tgttaacttc 6000
   acttctttgt atattatgta taaattagaa aatgaaaaat gtgtgaataa cattgtatga 6060
   aataaacctg gtcttgtgtt tttctctaga taaaataaaa atctgtacct c 6111
<210> 5
   <211> 549
   <212> DNA
   <213> Homo sapiens
<400> 5
   cctttcctgt taggcgagag ctgcgaaagg cgagagctgc gaagggccag gtgtcgggcg 60
   ctgtttctcg ttttcatcat atagacaaaa cagccctgct gcaaagatgg tcaacgtacc 120
   taaaacccga agaaccttct gtaagaagtg tggcaagcat cagcctcaca aagtgacaca 180
   gtataagaag ggcaaggatt ctttgtatgc ccagggaagg aggcgctatg atcggaagca 240
   gagtggctat ggtgggcaga caaagccaat tttccggaag aaggctaaga ccacaaagaa 300
   gattgtgcta aggctggaat gtgttgagcc taactgcaga tccaagagga tgctggccat 360
   taagagatgc aagcattttg aactgggagg agataagaag agaaagggcc aagtgatcca 420
   gttctaaact ttgggatatt tttcttcaat tttgaagaga aaatggtgaa gccatagaaa 480
   agttacccga gggaaaataa atacagtgat attcttacgc aaaaaaaaaa aaaaaaaaaa 540
   aaaaaaaaa 549
<210> 6
   <211> 676
   <212> DNA
   <213> Homo sapiens
<400> 6
   cggatggggc gtaggtgggc ggtgcgccca cagctgcctg ggtaaggccc aagatggctg 60
   tcttcgcctt agtactcgtg tgaagttggc ggggacggtt cctgtcatct tcttgggctt 120
   atttggtgtg ctgttgaagg ggggagacta gagaaatggc agggaacctc ttatccgggg 180
   caggtaggcg cctgtgggac tgggtgcctc tggcgtgcag aagcttctct cttggtgtgc 240
   ctagattgat cggtataagg ctcactctcc cgccccccaa agtggttgat cgttggaacg 300
   agaaaagggc catgttcgga gtgtatgaca acatcgggat cctgggaaac tttgaaaagc 360
   accccaaaga actgatcagg gggcccatat ggcttcgagg ttggaaaggg aatgaattgc 420
   aacgttgtat ccgaaagagg aaaatggttg gaagtagaat gttcgctgat gacctgcaca 480
   accttaataa acgcatccgc tatctctaca aacactttaa ccgacatggg aagtttcgat 540
   agaagagaaa gctgagaact tcggaaaagg ctcatctgtc accctggaga agggaaactg 600
   tacttttccc tgtgaggaaa cggctttgta ttttctctgt aataaaatgg ggcttctttg 660
   gaaaaaaaaa aaaaaa 676
<210> 7
   <211> 4796
   <212> DNA
   <213> Homo sapiens
<400> 7
   atgcgcctgc gcagaccgct ggggacggga ggggcggggc tcgaggggcg gtcaatgcgc 60
   ctgcgcacac cgcacggcga agtgggggag ggcagtccgt gtgtgtctgt gttgttgttc 120
   ggcggcggcg gcggcggtaa gatggctgcc cacgggggct ccgcggcgtc ctcggcgctg 180
   aaggggttaa ttcaacagtt caccaccatt accggtgcaa gtgaaagtgt aggaaaacat 240
   atgcttgaag cgtgcaacaa taatctggaa atggcagtca ctatgttttt ggatggtgga 300
   ggaatcgctg aagagcccag taccagttca gcaagtgtct ctactgtcag accacacaca 360
   gaagaagaag ttcgtgcccc aattcctcaa aagcaggaaa tactggtgga accagaacca 420
   ttatttggtg ctcctaaaag acgacggcct gcacgttcaa tttttgatgg tttccgggat 480
   tttcagactg aaactattcg gcaagaacaa gaattaagaa atggaggagc tatcgataag 540
   aaattaacta cccttgcaga tctattccgg ccacccattg atttgatgca taaaggcagc 600
   tttgaaacag ccaaagagtg tggccagatg caaaataagt ggctgatgat aaacattcaa 660
   aatgttcaag actttgcatg tcagtgcctc aaccgcgatg tgtggagcaa cgaagctgtg 720
   aagaatatta tccgggaaca tttcattttc tggcaggttt atcatgacag tgaggaaggt 780
   cagagataca tacagtttta taagttaggg gatttcccct atgtttccat attggaccca 840
   cggacaggtc agaagctagt agaatggcac cagttagatg tatcttcttt cttggaccaa 900
   gtgacgggat ttctgggtga acatggacaa ctggatggac tttctagcag tccccccaaa 960
   aaatgtgccc gttcagagag ccttatagat gcaagtgaag acagccagct agaagctgcc 1020
   atcagagcct ccttacaaga aacacatttt gattcaacac agacaaaaca ggatagccgc 1080
   tcagatgaag aatctgaatc tgaacttttt tctggcagtg aggagttcat atccgtttgt 1140
   ggctctgatg aagaagaaga ggtagagaat cttgccaagt ccagaaagtc tccccacaaa 1200
   gatttggggc atagaaaaga ggagaataga aggccgctga ctgagccacc agtcagaact 1260
   gatcctggaa cagccacaaa ccaccaagga ttgccagctg tggattcaga gatactggag 1320
   atgccacctg aaaaagcaga tggagtagtg gaggggatag atgtaaatgg accaaaagca 1380
   cagctgatgt tgcggtatcc agatggaaaa agggaacaga tcactcttcc agagcaagct 1440
   aaactgctag ctttggtgaa gcacgtgcag tctaaaggat acccaaatga acgttttgaa 1500
   cttctcacca actttcctcg aaggaaatta tctcatctgg actatgatat tacattgcaa 1560
   gaggcaggcc tttgtcctca agagactgtc tttgtacagg aaagaaatta acaccatggc 1620
   ttgacctctc tcagcttacc ccttttttcc cttttcctgt gagatacatg tcactaagta 1680
   tgcattttgg ctcataggac cacagagcca aagttgggca agcaagtcac cttccttctc 1740
   ttatttcttg atctctccta taatcagtct ctttctcccc ctttgtttct ttctcctctt 1800
   ctgttttctc gctccccact ccgtaccctt ctgcctcccc ctccccccac acttttcttt 1860
   cttacctaat ctggtgacca aactgaagtg taaggataag acctctccta gtactagcag 1920
   caggaattgt gtgttccagt aagtggtctc ttgcacggca cttttttggg atcaaatgtt 1980
   aacgttactc cccagactct tcgggcaaag gaatggctag attcagagta agaacaaccc 2040
   tccctttttt gtaagtcccc gtgtttagca gggagaagaa attctctaac atgggtttgg 2100
   ttttgttgtg ttcttcatgt ggaaatgtcg cttaacaaaa tatccaggct tttgtttacg 2160
   tggaaaaagc atcccttgta atgattgctc atcatactta aaaacctttt tcaaaggatt 2220
   ttcatgttcc cagctataag gactatttcc atgacatgtg ttattggcag aatgagtgtt 2280
   aaatatggag catatagcat ggggtgactt tcattgtcct aacctgagac agttttcctt 2340
   attactctgt attgatcctg ctagtccaag aatggacatg aagtgaacct atcgtggtga 2400
   ctgggatagg aaggtgcttg ctatttttgc cagcacagca tattagttcc tttggagccc 2460
   tccattgtct gagtctgcag tgatctgtag gaaggcagct ggtcaataat catgtagtac 2520
   aatggcttgg aattgtaacc actatggtta ttgattgtcc tgtgttgttt caggcatact 2580
   taggtatgtc cctggggaaa aagaaaacca ttcagctgag agttgctaac catgttcttt 2640
   tggttagaaa taatggttca ttttttgccc ctggttggaa tagtctctaa aaggctctgg 2700
   tgactgaatt gaacatgagt ccgcatgctg ttttctttca aaaggtatca aaacggaaag 2760
   cctctctaag gggaagacct ttcaactcca ttcagaagac aacatttagt aaggaggatg 2820
   gcggaggtta ctagtaattt tcagatgtct tgggcttttt ctgccaacaa aacccaaaat 2880
   caaattagag ttggtgaaag ctttcttcag tgtttttaga agaggccatc ttgaatctgt 2940
   agaataccat ttacacatca acttcaccct atgctcattt cgtattttga gcttaaatgg 3000
   agtcctctga agggggggta gtcgtgattc tggtggcaaa actagaactt ttaacttgta 3060
   aaatgaaaaa tattaaatgg acctttttgt cagttgagga tttagattga ttcttttatc 3120
   tgaggagcgt atgttcctca gatgttgcgt agagaccttt aggttttcat ctactttaag 3180
   attgctctct tggcaattag gggatttggg aaaagaagaa aaaaagatgc catgtgttgc 3240
   tagtacacag ttataggata tggtttctaa tggttgaatt ttgaggaact ctccctaaag 3300
   aatgagtttt atatctcctc aaggaaatca tggaaaaatc tgtttattct tcagtgagtc 3360
   cttttgaatt aatgttctta aatttttttc taagtctgtg taagtgctta tgtataagta 3420
   tataattgta taaatattta taaatatatt tatataatta cggtttcatt tctaccttga 3480
   gtcaaagttc tgtctttaga ttggtgactg agtaatactt acactttggt gttttttctt 3540
   aggttcttga ggcttactta actagcagct tctgatttat tgagtgaaag atggttttca 3600
   tgttaattcc tcagttgcat ctctgaactt ggataacata cttgccgttt gaaaaataga 3660
   gctgtatact gtcaaaggtg cactggaggg taaaacattt gttggtagta gacaagctca 3720
   gaaatccaaa aattcaggga gggcacaggg taagaagaga ggtttgctca gctttgtgct 3780
   ctgatggcac ccatctgtac tccagcaaag tcatagctga agcccaaatc gctcaaacgt 3840
   gtgaagtcaa tcatttttcc tatagggctc tgtttccttt tctttacctt ctagcttctc 3900
   ttgataaaac agttgggaga actgccaacg tgttcatcag tgagagggtg tggttctttc 3960
   cgatatgttc agcacgtgca tattcattta tgagaaacca aagtaacttc ttcaactcgg 4020
   gttactgtgg gatgattaag tagatataaa gtgatttaaa agacagcatg gtactttcac 4080
   tcagcttaac atgcagctgg gatgtctaca atacaagatg tggtgggttt tattttattt 4140
   tattttgagg caggagtgta attatcttag ggttgatata aaggcccatg ggaagagtta 4200
   cggattgtac atgggaagaa tccataagga gtattgcctt gctcaagaat ttaatccgct 4260
   gacagtatgc ttcttccagt ttgccttact tcctctgcaa gtttctttgc tccttagggc 4320
   agaggtttta ttgaatagat taaatgaggt cagtaagatt agttaaaaat agtcattcct 4380
   tgtgggtgtt ggacacagct tgaaaggagt gtttaaaaaa aaaaagctag ggaggaaaag 4440
   aacaagaaag atagacttcg agagtgataa gagaaaaata cagggagatg gaatggaaac 4500
   taccaggaag gccgggcgcg gtggctcatg cctgtaatcc caacactttg ggaggcggag 4560
   gtgggcagat cacctaaggt caggagatcg aggccagcct gagcaacatg gtgaaacccc 4620
   gtctctacta aaaatacaaa aattacctgg gtgtggtggc gtgcacctgt aatcccagct 4680
   actcagaggc tgaggcaggg gaatggcttg aacctgggag gcagaggttg cagtgagcca 4740
   agatcgcacc attacactcc agcctgggcg acaaagcgac actccatctc aaattt 4796
<210> 8
   <211> 709
   <212> DNA
   <213> Homo sapiens
<400> 8
   ctaaaataca aaaaagggag acaggtatat ttacaaaaat ccatggctgg tacagtacat 60
   aatttttaag acacactaaa tgctacaatc tttcaggtcc tgagattatt acaaacatca 120
   actcaaagct tgtgttcagg tccagttttt aaaggaggaa aaaaaaatcc caaaatatta 180
   agtcacacac ttatttgaaa tacagtatat cccttcaagt tagcaaaaac acacctccct 240
   ataagcaaat gcaatacagt aattcttgtc tttcacctag aggaccatca actctttagg 300
   ctccttcaag attggttttg aaacatgttg gcatctatgt caagtgtgga ggtggctaat 360
   gtatgaggaa gatgtaactg gagggaagtt cctctgcaga gtgaacattc actgtataaa 420
   tacatcctgt ttttccttgg gctaccaaac aagtctttcc cttatcaaaa ttcacaccga 480
   agtatgtgtt tacccagact aaaaatcaga atcattattt ccaatcagtt atagcaaata 540
   ggtctggagt tcggtaatca tttttacaac tgcaaatgaa ctttcttgct tgtaataaaa 600
   gagagaaaac atccattact tagttacctt ggtagacttg gattaatata ttatttgggg 660
   cacaaaattt tcaccagctg gagatctatt ttcttctttt aagtttgag 709
<210> 9
   <211> 1230
   <212> DNA
   <213> Homo sapiens
<400> 9
   aaagacttcc ggtcggcgtg agcgtgaggt gtgggtgttc gtttctcagg taaaacatgg 60
   ctaaaagctt acggagtaag tggaaaagaa agatgcgtgc tgaaaagaga aaaaagaatg 120
   ccccaaagga ggccagcagg cttaaaagta ttctcaaact agacggtgat gttttaatga 180
   aagatgttca agagatagca actgtggtgg tacccaaacc caaacattgc caagagaaaa 240
   tgcaatgtga ggtaaaagat gaaaaagatg acatgaaaat ggagactgat attaagagaa 300
   acaaaaagac tcttctagac cagcatggac agtacccaat atggatgaac caaaggcaaa 360
   gaaaaaggct gaaggcaaag cgagagaaaa gaaaggggaa aagcaaagca aaagcagtga 420
   aagtggcaaa gggtttggcc tggtagactc ttaaaacctt ggaaaatgcc acatgggata 480
   gatgacggat tagaatgtat acacatgtat actttgattt caactgccac caaatgaaaa 540
   ccatttgttt tcctatttta acttggcctt ttttcttcaa ttcaaaccca gcataactcc 600
   tcaagtttgt tttgggaact tgaataaaat attttctttg atacatccaa gctctactga 660
   tactttaact ggatgctacc caatatccag ttataatcag gcctcaacgt tctgtcaggt 720
   attaatatgg ttagaattgt tcttacaact ggtaattata gtcaatagag ataaaacaga 780
   tgatgtactg acaaattttt gtgaacttag attttaaaga aagttgttgt ataatcatat 840
   catttaaaaa tactacatta gggctgggca tggtggctca cacctgtaat cccagcactt 900
   tgggaggccg aggcaggtgg atcacgaggt cagttcgaga ctagcctggc taatacagtg 960
   aaaccccatc tctactaaaa atacaaaatt agccgggcat gggggcgcgc acctgtagtt 1020
   ccagctactc gggaggctga ggcaggataa ttgcttgaac ccaggagatg gaggttgcag 1080
   tgagccaaga ttgccccact gcactccacc ctgggcaatg gagtaggact ctgtctcaaa 1140
   aaaaaaaaaa ataatattaa tggatcttat aaagtacttg tgtggagctt tgattccaca 1200
   gaataaaaga tattttgtgt gtagatcttc 1230
<210> 10
   <211> 2065
   <212> DNA
   <213> Homo sapiens
<400> 10
   aacagcaatg gcggaaggag aagatgtgcc gccgctgcca acgtcgagcg gcgacggctg 60
   ggaaaaagat cttgaagaag ctctggaagc aggaggttgt gatcttgaaa cgttgagaaa 120
   tataattcaa ggaagaccgc tgcctgctga tctgagggcc aaagtttgga agattgctct 180
   gaatgttgca ggaaaaggtg attgtttggc atcatgggat ggtattttag acttgccaga 240
   acagaacact attcacaaag attgcctgca gtttattgac cagctttcag tgccagagga 300
   gaaggcagca gaattacttt tggatattga atctgtaatt accttttatt gtaaatcacg 360
   taacattaaa tatagcacat cccttagctg gatacatcta ctgaaaccat tggtgcatct 420
   tcaactgcca cgcagcgatt tatacaactg cttttatgcc ataatgaata agtacattcc 480
   cagggattgt tcccagaaag ggagaccatt tcatcccttc aggttgctca tccaatacca 540
   tgagcctgag ctttgttctt atcttgatac aaagaaaatt actccagact cctatgcact 600
   caactggctt ggaagtcttt ttgcatgtta ctgttccact gaagtcactc aggcaatatg 660
   ggatggatat ctacaacaag cagatccatt ttttatttat ttcttaatgt taattatcct 720
   tgttaatgca aaagaagtta ttttaacaca agagtcagac agcaaagaag aagttatcaa 780
   gttcttggaa aatactccat ccagtctgaa tatagaagat atagaagacc ttttctctct 840
   ggctcagtat tattgcagca aaacaccggc ttcttttagg aaggataatc accatctctt   900
   tggtagtact ttgttgggaa ttaaggatga tgatgcagat ctgagtcagg ctctttgtct   960
   ggccatctcc gtgtcagaga tccttcaagc gaatcagcta caaggggaag gagtccggtt  1020
   ctttgtggtg gattgccgtc ctgcagaaca atataatgct gggcatttat caactgcttt  1080
   ccactcagat tcagacctga tgcttcagaa tccatctgag tttgcacagt cagtaaaatc  1140
   cttgctggaa gcacagaagc agtccattga gtctggctcc atagctggtg gggagcacct  1200
   ctgttttatg ggcagtggca gggaggaaga agacatgtat atgaacatgg tcctggcaca  1260
   ctttttacag aaaaacaaag aatatgtgag tattgccagt ggaggattta tggcactgca  1320
   gcagcacctg gcagacatta atgtggatgg accagaaaat ggatatggcc attggattgc  1380
   tagtacctca ggctcaagga gcagtatcaa ttctgttgat ggtgaatctc ctaatggctc  1440
   aagtgataga ggaatgaaat cactagtaaa taaaatgact gtggctttga agacaaaatc  1500
   cgttaatgtc agggaaaaag ttatcagttt tatagagaat acatcaactc ctgtggatcg  1560
   gcatgtgagc agcagtgaca gagtgggcaa gccttaccgt ggcgtaaagc ctgttttcag  1620
   cattggggat gaagaagaat acgacacaga tgaaattgac agttcttcaa tgtcagatga  1680
   tgatagaaaa gaggttgtaa acattcagac ttggataaac aaaccagatg tcaaacatca  1740
   ttttccttgt aaagaagtaa aagaaagtgg acacatgttt cccagtcatc tgttggttac  1800
   tgcaacacat atgtactgtt taagggagat tgtttcacgg aaaggattgg cttatataca  1860
   gtctcgacaa gcgctgaatt ctgtagttaa aattacatcc aaaaaaaaac atcctgaact  1920
   cattaccttc aagtatggaa atagcagtgc ttcaggaata gaaatcttgg caatcgaaag  1980
   gtatttgatt ccaaatgcag gggatgcaac taaagccata aaacagcaga tcatgaaagt  2040
   tttggatgct ttggaaagtt aatat                                        2065
<210> 11
   <211> 3221
   <212> DNA
   <213> Homo sapiens
<400> 11
   tgtcaaaagc ctgcacacga gacccccgtg ggccaggact gacttcctcc ccacacaggg     60
   ctgggcgggg caagcaggaa ggagcctgtt acagtcaagg tgacgcatcc ttgccgggcc    120
   agcccgaaca gcagctgggt cggcaagcga caggagcacg ggtcatcttt tccccagagg    180
   cgtcggaatg gacctgtgcc acccagagcc cgcggagctg agcagcgggg agacggaaga    240
   gttacagagg atcaagtggc accgaaagca gctcctggag gacatccaga agctgaagga    300
   tgagattgca gatgtgtttg cccaaatcga ctgcttcgag agtgcggagg agagccggat    360
   ggcccagaag gagaaggagc tgtgtattgg gcgcaagaag ttcaacatgg accccgccaa    420
   gggtatccag tatttcattg agcacaagct gctgacccct gacgtccagg acattgcacg    480
   gttcctgtat aaaggcgagg gcctcaacaa gacagccatt ggtacctacc tgggggagag    540
   ggatcccatc aacctgcagg tcctccaggc cttcgtggac tgccacgagt tcgccaacct    600
   caacctcgtc caggccctca ggcagttcct gtggagcttc cggctgccgg gcgaggccca    660
   gaagatagac cggatgatgg aggcctttgc cactcgatac tgcctctgca acccaggcgt    720
   cttccagtcc acagacacct gctacgtgtt gtccttctcc atcatcatgc tcaacaccag    780
   cctccacaat cccaacgtcc gggacaggcc gcctttcgag cgctttgtgt ccatgaaccg    840
   cggcatcaac aatggtagcg acctgcccga ggaccagctg cggaacctct tcgacagcat    900
   caagagtgag ccattctcca tccctgagga cgacggcaat gacctcactc acaccttctt    960
   caatccagac cgggagggtt ggctgctcaa gctagggggc cgcgtgaaga cgtggaaacg   1020
   gcgctggttc atcctgaccg acaactgcct ctactacttc gagttcacca ctgacaagga   1080
   gccacgggga attatacctc ttgagaacct ctcggtgcag aaggtggatg accccaagaa   1140
   gccattctgc ctggagctct acaaccctag ctgccgaggc cagaaaatca aggcctgcaa   1200
   gaccgatggc gacggcaggg tggtggaggg caagcacgaa tcgtaccgca tctcagccac   1260
   cagtgccgag gaacgtgacc agtggatcga gtccatccga gccagcatca cccgtgtccc   1320
   cttctacgac ctggtctcta ctcggaagaa gaagattgcc agcaagcagt gagattcctg   1380
   gaggtggcac tgggggctgg tcaccctgag agtcccatcg cctgcagcac ctggagaccc  1440
   acctcccacc ccagtgcact cttttgggcc acagacatca ttgctgttcc ccgttacctc   1500
   gagctgactc tagaggggaa ggcagagctc aggagggtgg gtgggagctg cagtgggctc   1560
   agagtccagc aatgaggccc cctggcctgg gcacccagct gcaggcccct gccctacgtg   1620
   cactacagga aggggtgagg agagcagcca gaggaaaacc agccccagaa tgctgcaact   1680
   tctcttctct ctggacttcg gtgtcctcgg ctctgaaggc gctctctgca cttagatgct   1740
   gtcagccctc aacgtaggag gggccgtggg gtccctaagt gattcttctc cctggcaagg   1800
   ctcttccttt tcagggatat ctctgccaac ccttcccctg tcctcgggtt gggcttggcc   1860
   ctctctgcct gagagagctc agcacacaca cagctcagac ccacggacag gaccccggga   1920
   cagaaccccg ggagcactgc cctaggagcc tggaccccac gagctcagtc ccagctctgc   1980
   ctctgactcg ctgtgtaccc tctgccaagt catgcccatt ctctagttct tgcaaaactg   2040
   cagtgtttgg actagaaacg tattggcccc tgctagccct gtgctccagc ttccagctgg   2100
   agatggcagc cgttatttgc ggagcccagg ctgactccgg attcaacgtt gctggggaga   2160
   gaaaagcagt atagactcca ccttccagga tgtccatttc ggggagagga gcaggtggga   2220
   ccctcaagaa aatgacggag aacatcccag acagatggga ctcaagcagg atgggtgcta   2280
   tatccaagaa gccaagaagg gagagtttcg tgcactgtgg ttaacaggag ggccgcctgg   2340
   aggcagtggc tgagccagaa agtaacacag agctcatgct tggagagata gagtcttggc   2400
   catggctttc ctgcaagacc actgacgagc agtcccatgg tgataaaggg cagcccggcg   2460
   ggtcccagca tctcgagacc ctctgcagaa gtattatcag agtagagctg acttccagta 2520
   cccgggcagc cagctctgtc tccagggagg cctggcctgg aggaggagtc cactaacagg 2580
   aaacacttct caccccctca cacaggccca ggggagcccc tggagggaaa ttccttggca 2640
   ggggaacagg aaatgtggtc gcctctgccc cactgccagc ctgagtgggc aaggaagcag 2700
   gtggatcccc cagaaggaac cgcagctcgc gaggcacctc tcctcgtccc cacccacccc 2760
   cacatttgca caccttccac agggtcagga tggagtagct gccttgcgag gtggtgagac 2820
   tcctgtctct ggaggtctgc aagcactggt agtgatattg cagcagacaa ggtctgggtt 2880
   gggcgtctgc aggaggagac cttgtgggac atctgaggac atccgcagat tcttgtcagc 2940
   ctgtgaacta ggccctgcct ctgtcacctc attggtccat gaaggagcag ccaggggtgg 3000
   tggaaggagc actgggctag gggtcagggg tcagagattc tgtcctgctc ctgggatgca 3060
   ctggctactc ccttagtcta ctcccttccc ctctctggtc ctcagcttcc tccatcatgg 3120
   aggagatggg atcatggatt ttctgggcat aagtggctgt tctgggagtc attccaaaga 3180
   gagaataaaa ctcctgatcc aaaaaaaaaa aaaaaaaaaa a 3221
<210> 12
   <211> 1336
   <212> DNA
   <213> Homo sapiens
<400> 12
   ggggcttgca gagccggcgc cggaggagac gcacgcagct gactttgtct tctccgcacg     60
   actgttacag aggtctccag agccttctct ctcctgtgca aaatggcaac tcttaaggaa    120
   aaactcattg caccagttgc ggaagaagag gcaacagttc caaacaataa gatcactgta    180
   gtgggtgttg gacaagttgg tatggcgtgt gctatcagca ttctgggaaa gtctctggct    240
   gatgaacttg ctcttgtgga tgttttggaa gataagctta aaggagaaat gatggatctg    300
   cagcatggga gcttatttct tcagacacct aaaattgtgg cagataaaga ttattctgtg    360
   accgccaatt ctaagattgt agtggtaact gcaggagtcc gtcagcaaga aggggagagt    420
   cggctcaatc tggtgcagag aaatgttaat gtcttcaaat tcattattcc tcagatcgtc    480
   aagtacagtc ctgattgcat cataattgtg gtttccaacc cagtggacat tcttacgtat    540
   gttacctgga aactaagtgg attacccaaa caccgcgtga ttggaagtgg atgtaatctg    600
   gattctgcta gatttcgcta ccttatggct gaaaaacttg gcattcatcc cagcagctgc    660
   catggatgga ttttggggga acatggcgac tcaagtgtgg ctgtgtggag tggtgtgaat    720
   gtggcaggtg tttctctcca ggaattgaat ccagaaatgg gaactgacaa tgatagtgaa    780
   aattggaagg aagtgcataa gatggtggtt gaaagtgcct atgaagtcat caagctaaaa    840
   ggatatacca actgggctat tggattaagt gtggctgatc ttattgaatc catgttgaaa    900
   aatctatcca ggattcatcc cgtgtcaaca atggtaaagg ggatgtatgg cattgagaat    960
   gaagtcttcc tgagccttcc atgtatcctc aatgcccggg gattaaccag cgttatcaac   1020
   cagaagctaa aggatgatga ggttgctcag ctcaagaaaa gtgcagatac cctgtgggac   1080
   atccagaagg acctaaaaga cctgtgacta gtgagctcta ggctgtagaa atttaaaaac   1140
   tacaatgtga ttaactcgag cctttagttt tcatccatgt acatggatca cagtttgctt   1200
   tgatcttctt caatatgtga atttgggctc acagaatcaa agcctatgct tggtttaatg   1260
   cttgcaatct gagctcttga acaaataaaa ttaactattg tagtgcgaaa aaaaaaaaaa   1320
   aaaaaaaaaa aaaaaa                                                   1336
<210> 13
   <211> 479
   <212> DNA
   <213> Homo sapiens
<400> 13
   agattccgtc gcttcttccg gagccgtacg tggcaccgcc ccgcctcgcg ggcgaccgcg 60
   gggcttgctg ggaagagagg cgaagccagg tcacctttca aggacccaga agtagggttt 120
   tggcctaggt aacggggcag agatgtggtt cgagattctc cccggactct ccgtcatggg 180
   cgtgtgcttg ttgattccag gactggctac tgcgtacatc cacaggttca ctaacggggg 240
   caaggaaaaa agggttgctc attttgggta tcactggagt ctgatggaaa gagataggcg 300
   catctctgga gttgatcgtt actatgtgtc aaagggtttg gagaacattg attaaggaag 360
   cattttcctg attgatgaaa aaaataactc agttatggcc atctacccct gctagaaggt 420
   tacagtgtat tatgtagcat gcaaatgtgt ttatgtagtg gcttaataaa aataaaatg 479
<210> 14
   <211> 6404
   <212> DNA
   <213> Homo sapiens
<400> 14
   ttacatctta tagcacccag tgaattatga tatggggctg cggtttattt gctctggcga 60
   caatatggct tacttgccgt ggaatatccc ggtatttcag aagtaacata ttatttttaa 120
   agagggagct ttgctttctc taaatcacat ggtgcaaagt tgggaataca gtggcctgta 180
   aattacaaat attatttaca aactagaagg aaagctgaca tgtgtttgtg cctgtgtgac 240
   agtttcacta aggaattcca aaactgtgga tagcctgtta cttgagaaaa atacacggtg 300
   gcagtcttgc agatgttttt cccgcctgtt agccctccag gttcaaaaac tgtttggtgc 360
   attttaaggc ggcagtctca ccagatcgca ggattctagt ctaattggct tctaaccttc 420
   aatctaacct tgccggcttc cttgcaaagc ccggtgcaag ggcctctttc aaaatgaacc 480
   cactggtgtg cctagcagtc ggtagaagaa gcgggagggc gtccggtctg cacgcccgcc 540
   gcgaggttac aatgctgaac gcatgagatg gaagatacca acgggaggcc gaggggatcc 600
   acggcgcccg cgcgggctcc ggcttcctcc tgctctcggc gccgctgggc gaccgcccat 660
   gacccgctct tgcgggctct gtccggttga caggcgaccc tgtggcccgg ggaagcgcgg 720
   gagggcgccg gcggaaagtt gaagagcgtt tttctcgccg ccgcgtgcat taggagctcg 780
   acgagtccgc cctgggcttc ctggtggggc tgggcgggcg ggggaggggc cgcgcagcag 840
   cagcggaagc cagacctcgg cgataagagg ctgcacagcg acatgcaaca gtcttttcac 900
   tgcagctgaa tgagttgtgg cgcccacaat gctcccatga caaggagctg acaagttcca 960
   ttttccgtcg cgggcatctt ggaatcatga ctcccacaat gccttgggca cttggtcgac 1020
   agtggggccg cctctgaaaa aaaaatgtga gaggttggta ctaagaagtg cctttcctga 1080
   cgtctctgct gcttggaacc gcttctagag cagtctctgc ttttgccttg cttgctgcca 1140
   gctagactgt gacgacagca catccaccct ccacctctag cccagacacc cccatttcta 1200
   cttataatca agagaaaagc tctaagtatc tggcattgcc ctaggctgct ttagtgttaa 1260
   aagaaaagtt tgctgaaaaa gtaagatatc ttctgccagg aaatcaagga ggaaaaaaaa 1320
   aatcattttc tcgattttgc tctaaactgc tgcatctgtc tatgccaaac taatcaatac 1380
   cgattgcacc accaaactcc attgcaaatt cagctgtgag gagattccct ttcagacaac 1440
   tttgctgaaa gcagcttgga aattcggtgt cgaagggtct gccacgtttt catgcttgca 1500
   ttttgggctc caaattggca ctgggaaggg gttactgaga gcacaaggct gataccaggc 1560
   cctactttta aacgttcatc tacttacaat cctagtattt ctctaaaaac caaaacctct 1620
   ttgaattaac agtttcatgc tgtgaatttc tagtgggaga tcttttcctt gatattgacg 1680
   acacaatttt ccatgtactt ttaaagcagg gagtggggaa aagtattttg aggggacatt 1740
   ttcatcatca gttcagcttt ttttttttgg ttgttgctct tttttggggg ggttgggttt 1800
   gttggtttca ctgaaacatt taactacctg taaaatctaa acatggctgt tagtgtcaca 1860
   ccaattcggg acacaaaatg gctaacactg gaagtatgta gagagttcca gagggggact 1920
   tgctcacggc cagacacgga atgtaaattt gcacatcctt cgaaaagctg ccaagttgaa 1980
   aatggacgag taatcgcctg ctttgattca ttgaaaggcc gttgctccag ggagaactgc 2040
   aaatatcttc atccaccccc acatttaaaa acgcagttgg agataaatgg acgcaataac 2100
   ttgattcagc agaagaacat ggccatgttg gcccagcaaa tgcaactagc caatgccatg 2160
   atgcctggtg ccccattaca acccgtgcca atgttttcag ttgcaccaag cttagccacc 2220
   aatgcatcag cagccgcctt taatccctat ctgggacctg tttctccaag cctggtcccg 2280
   gcagagatct tgccgactgc accaatgttg gttacaggga atccgggtgt ccctgtacct 2340
   gcagctgctg cagctgctgc acagaaatta atgcgaacag acagacttga ggtatgtcga 2400
   gagtaccaac gtggcaattg caaccgagga gaaaatgatt gtcggtttgc tcatcctgct 2460
   gacagcacaa tgattgacac caatgacaac acagtcactg tgtgtatgga ttacatcaaa 2520
   gggagatgct ctcgggaaaa gtgcaaatac tttcatcccc ctgcacattt gcaagccaag 2580
   atcaaggctg cccaatacca ggtcaaccag gctgcagctg cacaggctgc agccaccgca 2640
   gctgccatgg gaattcctca agctgtactt cccccattac caaagaggcc tgctcttgaa 2700
   aaaaccaacg gtgccaccgc agtctttaac actggtattt tccaatacca acaggctcta 2760
   gccaacatgc agttacaaca gcatacagca tttctcccac caggctcaat attgtgcatg 2820
   acacccgcta caagtgttgt tcccatggtg cacggtgcta cgccagccac tgtgtccgca 2880
   gcaacaacat ctgccacaag tgttcccttc gctgcaacag ccacagccaa ccagataccc 2940
   ataatatctg ccgaacatct gactagccac aagtatgtta cccagatgta gaattttcat 3000
   cactaaacaa tcatgctaaa gaggaaagga cagtgtgctt ggttagagta aaggacgagg 3060
   tcattagcca tattgtatat atcgtcaagc aacacacaca aaagttcctc agccacaaga 3120
   catccacata ttgcatgtta accagaagaa aagacaacat tttccggaaa tccactgcac 3180
   actgttgcct atacactttg tacatttaat tgatatttgt gctgaggtga tattcctgtc 3240
   taaaagaaca acattgtctt tcttttctag cacagagtta tgcattcaaa gatgcatacc 3300
   tagttagttt cctatatatt catgccatct tgaaaagaca gactatggtg taaccatgat 3360
   tctattatgt attggtacgt ctgtagacca agatataatt ttttaaaaat aagtttattt 3420
   ctttcaaggt ttacaaataa caaaggtgca ccttgtattt aaaattgcca ttatagatga 3480
   gagcgtgcat gcacagtcat ttttgtttaa gagtaatatt tttaatgtaa tagattgtaa 3540
   gacgtggtga gggagggatc tgacagagat gaatgtgcca agcaaaacca caactgtgta 3600
   tattttaaag cacatcatgg ctttaagtac catgttgtta aggattctca tgaagtgcca 3660
   tagactgtac atcaaattag agtattattt cttcagtgtt attgttttca gagccacatt 3720
   ttgttgcata tttgctagta ctaatcagtc aaagggcacc attctttttt tttttttttg 3780
   aaaccaaagc tgtctcagaa atggccaatt taactttaca gtaacaatag acagcacaac 3840
   acaaactctc tcaatacaga taaactcaca catactggag atatatatat aatagatata 3900
   tataaaatta ttttaatgca ttgtagtgta atatttatgc atactatact gtataacatg 3960
   ttattcaaaa gggattgcca tttctgagac acagtaacaa aaaaatgagg aaattatttt 4020
   gcttctattt atagcctctg tcaaaagtca aaagactata aatgctttgc aaaaatggtt 4080
   tcacgtttgc ttaaatgctt catcacagtc acattcaaaa tagtgactct aaacaaagaa 4140
   gaaagcagca ctgtcatcag atgcatgata aaccaaaata tgaaaatggg aaatgtttaa 4200
   ttaacctagt aattgggtgg gttaagtaca tgggtgaatt ttatatgtga tttttgtttt 4260
   gttttgtttt gttcagatta actgcttata gccttagaaa gccttttaca aaattaaaaa 4320
   aaaaatagat gtgcattcag tttttaagaa tggaatcatc caaaggaatt cctttttttg 4380
   aggtttggat gttgcagcta gtaaaggata tttttgctct gttcagcagt tctaaaaatt 4440
   gctgaagtag gggccaggtc actggtagtt atagtatgga atgggagaag tgaaagttca 4500
   gttatagaac tttccatact tccaagttta ctgcaagttt ttatgcttga gagagatgct 4560
   ttctaatata agactgatgt gttgatttta ctgattgtac tgtacatcta ttaaagcctt 4620
   agattattac attacgggtt ggaacccata ccaatgtaat ttcaatcgtg ttaagaaagt 4680
   aatggtgact tcacatgtta ttgtagttag ttacattata gaatattact tatttttctt 4740
   gttaaaatgt agtttttcat ttcctacatt tattagattt tcattttcta ttaacaattg 4800
   aataccattt cagtttatag acttgtttta ttagatttta ccaatgaatt tttcaaaata 4860
   caaaaaaaag tagtttttcc ttcataacat actcagtttt gaattacatg tagtgtcaca 4920
   tgaatattcg tattgttaac taaatgattt atattttact gatttaatat tacagtgtaa 4980
   gaatgtcagt cattgttagt tcttgtctag ttttcattaa aagaacaaag atcttttata 5040
   tggatatctt ataaatatat aatcattgct aagtaagaag ttaagttgtt gctatcgcaa 5100
   caatcctggc agacaattga gtaatatttt gatgatttat tttgtttgta attagttatt 5160
   ataagaagat ctagatccta gatattagaa taaaatttat tttctactgt atccatttca 5220
   aatgttaaaa tattgtttaa tatttttgaa atccctgagt atcaggcctt gttataaata 5280
   agctgcataa tcaataaata gaacaaggga ctttttgttg ataatccaaa tactcaaagt 5340
   ttacgtaatg aaaattatag cgtgtgtgca aactcttgag ggttgattat gctgcaattt 5400
   agcatgttgg aacgtctagg gagaaggttg actttttgca cttctgtata tagtcaaaag 5460
   agagaaacct gtataatagt aagatcttat tttgaataaa aacgtctata attacaagga 5520
   gttttgttaa ggctaataca atgacagact gagcaaaatt gcttgcaaaa gtggcacaga 5580
   gttagcactc catacccctt caaacatgtt gctttgcttt cttgtggaca gcttgtagtt 5640
   tgccaggatt ttttcagctg gaaagatacg ccatcctttc aaaccctcat gactgacaaa 5700
   aactccatgg ggccaaatct gcctgaagat cattaccaaa aatagcaggt acttctacca 5760
   ttaaggtgaa atcatggatc agatattcct tacatttttc aaaactactg catgtttaaa 5820
   acttcaacaa aaaaagagag aaagaactat actaagaaca tatattattc agatcagttt 5880
   ctgccaattt cagtggttta ttgttcacaa aaaaatcttc aaaacaagta ttgactttca 5940
   caaaatttaa atcataaaca ggcaaaccaa acagcacact gtagctatag ttgttatgtg 6000
   attgtttttt aattgctgta ggatcctgtt ctttcagcag gtgaaaaata aaacgcagtt 6060
   caaatttcat ggttttaatt ttcaactcag aagcactcaa aaatgcaaaa tgtgataatg 6120
   ggcacttgtt taaaagaatt agtgtatcca gccttcactc cagctggtta aaaatgttgc 6180
   acttatcagc aaccctacca ctttcatctg ctgaaaggac aaatgtgctt ggttttacta 6240
   ttatgtaatc acaacttact ttctgcttgt agttgcttaa aattatgtat tttgtcttgg 6300
   gctgcaattt gttttatgct tattttatta ttactgcagt agttgacttt gctgtatgga 6360
   aaaataaagt gaaattgccc taataaaact tctctttctt aagt 6404
<210> 15
   <211> 4968
   <212> DNA
   <213> Homo sapiens
<400> 15
   gcggccgccg tggccctcac agtcggcgtt tcgccgcctg cccgcggtgc ccgcgcacgc 60
   cggccgccat cgccttcgcg cctggctggc gggggcgctg tcctcccagg ccgtccgcgc 120
   cgctccctgg agctcggcgg agcgcggcag ccagggccgg cggaggcgcg aggagccggg 180
   cgccaccgcc gccgccgccg ccgccgccgc gggggccatg accgtggagc agaacgtgct 240
   gcagcagagc gcggcgcaga agcaccagca gacgtttttg aatcaactga gagaaattac 300
   ggggattaat gacacccaga tactacagca agccttgaag gatagtaatg gaaacttgga 360
   attagcagtg gctttcctta ctgcgaagaa tgctaagacc cctcagcagg aggagacaac 420
   ttactaccaa acagcacttc ctggcaatga tagatacatc agtgtgggaa gccaagcaga 480
   tacaaatgtg attgatctca ctggagatga taaagatgat cttcagagag caattgcctt 540
   gagtttggcc gaatcaaaca gggcattcag ggagactgga ataactgatg aggaacaagc 600
   cattagcaga gttcttgaag ccagcatagc agagaataaa gcatgtttga agaggacacc 660
   tacagaagtt tggagggatt ctcgaaaccc ttatgataga aaaagacagg acaaagctcc 720
   cgttgggcta aagaatgttg gcaatacttg ttggtttagt gctgttattc agtcattatt 780
   taatcttttg gaatttagaa gattagttct gaattacaag cctccatcaa atgctcaaga 840
   tttaccccga aaccaaaagg aacatcggaa tttgcctttt atgcgtgagc tgaggtatct 900
   atttgcactt cttgttggta ccaaaaggaa gtatgttgat ccatcaagag cagttgaaat 960
   tcttaaggat gctttcaaat caaatgactc acagcagcaa gatgtgagtg agtttacaca 1020
   caaattatta gattggttag aagatgcctt ccaaatgaaa gctgaagagg agacggatga 1080
   agagaagcca aagaacccca tggtagagtt gttctatggc agattcctgg ctgtgggagt 1140
   acttgaaggt aaaaaatttg aaaacactga aatgtttggt cagtacccac ttcaggtcaa 1200
   tgggttcaaa gatctgcatg agtgcctaga agctgcaatg attgaaggag aaattgagtc 1260
   tttacattca gagaattcag gaaaatcagg ccaagagcat tggtttactg aattaccacc 1320
   tgtgttaaca tttgaattgt caagatttga atttaatcag gcattgggaa gaccagaaaa 1380
   aattcacaac aaattagaat ttccccaagt tttatatttg gacagataca tgcacagaaa 1440
   cagagaaata acaagaatta agagggaaga gatcaagaga ctgaaagatt acctcacggt 1500
   attacaacaa aggctagaaa gatatttaag ctatggttcc ggtcccaaac gattcccctt 1560
   ggtagatgtt cttcagtatg cattggaatt tgcctcaagt aaacctgttt gcacttctcc 1620
   tgttgacgat attgacgcta gttccccacc tagtggttcc ataccatcac agacattacc 1680
   aagcacaaca gaacaacagg gagccctatc ttcagaactg ccaagcacat caccttcatc 1740
   agttgctgcc atttcatcga gatcagtaat acacaaacca tttactcagt cccggatacc 1800
   tccagatttg cccatgcatc cggcaccaag gcacataacg gaggaagaac tttctgtgct 1860
   ggaaagttgt ttacatcgct ggaggacaga aatagaaaat gacaccagag atttgcagga 1920
   aagcatatcc agaatccatc gaacaattga attaatgtac tctgacaaat ctatgataca 1980
   agttccttat cgattacatg ccgttttagt tcacgaaggc caagctaatg ctgggcacta 2040
   ctgggcatat atttttgatc atcgtgaaag cagatggatg aagtacaatg atattgctgt 2100
   gacaaaatca tcatgggaag agctagtgag ggactctttt ggtggttata gaaatgccag 2160
   tgcatactgt ttaatgtaca taaatgataa ggcacagttc ctaatacaag aggagtttaa 2220
   taaagaaact gggcagcccc ttgttggtat agaaacatta ccaccggatt tgagagattt 2280
   tgttgaggaa gacaaccaac gatttgaaaa agaactagaa gaatgggatg cacaacttgc 2340
   ccagaaagct ttgcaggaaa agcttttagc gtctcagaaa ttgagagagt cagagacttc 2400
   tgtgacaaca gcacaagcag caggagaccc agaatatcta gagcagccat caagaagtga 2460
   tttctcaaag cacttgaaag aagaaactat tcaaataatt accaaggcat cacatgagca 2520
   tgaagataaa agtcctgaaa cagttttgca gtcggcaatt aagttggaat atgcaaggtt 2580
   ggttaagttg gcccaagaag acaccccacc agaaaccgat tatcgtttac atcatgtagt 2640
   ggtctacttt atccagaacc aggcaccaaa gaaaattatt gagaaaacat tactagaaca 2700
   atttggagat agaaatttga gttttgatga aaggtgtcac aacataatga aagttgctca 2760
   agccaaactg gaaatgataa aacctgaaga agtaaacttg gaggaatatg aggagtggca 2820
   tcaggattat aggaaattca gggaaacaac tatgtatctc ataattgggc tagaaaattt 2880
   tcaaagagaa agttatatag attccttgct gttcctcatc tgtgcttatc agaataacaa 2940
   agaactcttg tctaaaggct tatacagagg acatgatgaa gaattgatat cacattatag 3000
   aagagaatgt ttgctaaaat taaatgagca agccgcagaa ctcttcgaat ctggagagga 3060
   tcgagaagta aacaatggtt tgattatcat gaatgagttt attgtcccat ttttgccatt 3120
   attactggtg gatgaaatgg aagaaaagga tatactagct gtagaagata tgagaaatcg 3180
   atggtgttcc taccttggtc aagaaatgga accacacctc caagaaaagc tgacagattt 3240
   tttgccaaaa ctgcttgatt gttctatgga gattaaaagt ttccatgagc caccgaagtt 3300
   accttcatat tccacgcatg aactctgtga gcgatttgcc cgaatcatgt tgtccctcag 3360
   tcgaactcct gctgatggaa gataaactgc acactttccc tgaacacact gtataaactc 3420
   tttttagttc ttaacccttg ccttcctgtc acagggtttg cttgttgctg ctatagtttt 3480
   taactttttt ttattttaat aactgcaaaa gacaaaatga ctatacagac tttagtcaga 3540
   ctgcagacaa taaagctgaa aatcgcatgg cgctcagaca ttttaaccgg aactgatgta 3600
   taatcacaaa tctaattgat tttattatgg caaaactatg cttttgccac cttcctgttg 3660
   cagtattact ttgcttttat cttttctttc tcaacagctt tccattcagt ctggatcctt 3720
   ccatgactac agccatttaa gtgttcagca ctgtgtacga tacataatat ttggtagctt 3780
   gtaaatgaaa taaagaataa agttttattt atggctacct atgtgtttgt aagcaggtat 3840
   attgtatatt agtgtattag taatactaga taaatgaatt ttgtctgggg attaagattg 3900
   gatagttaat agattaatac aatcttttaa ttctgctcta atgctagcaa attggaaaat 3960
   gtttaagtct ttgacactta aatttatcta tatttttaac aaagttcttg aacttagtat 4020
   ggcaccggaa cctgttttga attcagtcag gtttttactc aagtaagtgg ttgatttttt 4080
   ttaagtcaaa ctacactgaa acttttatcc ttttcttaga ttaatcttac tttttaaatg 4140
   tatttacaat atacagcaag gtgattattt caagagaatc ccaaagtact tgaataaggg 4200
   ctattgtaaa atttaaaaga aatatttata tatacacata tatacacata cacacatgta 4260
   tatatatatt cttcataatg gaggacaatg ttttgcaata tataaatcat tctatttttg 4320
   taaattgtat atcactttaa ttgaaaatgt tctctactaa ttaatactgt gaaacaaaat 4380
   tgatgttgtt taactagaag ttatgagtat cttaactgcc tttattcctt ttcaaaaagg 4440
   aaaaagctgt agaacatttt gtagatgaaa ctactgttta agattaatga attaatattg 4500
   tgaatgaaaa tcaaaatcca tactttaaag gtaatcatgt tactaacaac ctatttttga 4560
   attcataaaa atttctttat aaatgatgtt ttgtgaacat agtaaaatag accattatac 4620
   tatgtgtatg tttgatacag cgtcgccaaa actagtgttc tttattagtg cctctcacaa 4680
   aagatcctgg atggaggagt aagatgaaat attatgctat tatatgatgc tgtttgtaaa 4740
   ggtattaatg tactagtaag gtgttaatga caaggaatta gtactattcc tgttgtaaag 4800
   ttagattttg catattgtat ctatcaaaat atgtttgggt ttagatttta agttgtctac 4860
   tgagcagatt tctgcattgg ttttccagtc ctgttaaaag tttagaaact tcatatgtgt 4920
   catcacagct tttgtaaaga aagtatcctt aatattttat gacattct 4968
<210> 16
   <211> 3282
   <212> DNA
   <213> Homo sapiens
<400> 16
   atgagttggt tcaacgcctc ccagctctcc agcttcgcta agcaggccct gtcccaggcc 60
   cagaagtcta ttgacagggt tctggacatc caggaagagg agccgagcat ctgggccgag 120
   accattccgt atggagagcc gggaataagt tcccctgtca gtggaggatg ggatacttca 180
   acctgggggt tgaaatcaaa cactgaacct cagagtccac caatagcctc tcctaaagca 240
   atcacaaagc cagttcggag gactgtggtc gatgaatctg aaaatttctt cagtgccttt 300
   ctctcgccaa ccgatgtcca gaccattcag aagagtccag tggtatcaaa acctccagca 360
   aaatcacaac gaccagaaga agaagtgaaa agcagcttac atgaatcctt gcacattggc 420
   cagtcaagaa ctcctgaaac aactgaatca caagtaaaag actcttcttt gtgtgtttca 480
   ggggaaactc tggcagcagg tacttcatca cctaaaactg aaggcaagca cgaagaaact 540
   gttaataaag aatcggatat gaaggtgcca actgtaagtt tgaaagtatc tgaaagtgta 600
   attgatgtga aaacaactat ggaaagtata tctaatacgt ctacgcagtc tctcacagca 660
   gaaacaaagg acatagcttt ggaacctaag gaacaaaaac atgaagacag gcagagcaat 720
   acaccttctc ctcctgttag taccttttca tcaggtactt ctaccaccag tgatattgaa 780
   gttttagatc atgaaagtgt aataagtgag agctcagcga gctcgagaca agagactaca 840
   gattcaaaat caagtcttca cttgatgcag acatcttttc agcttctctc tgcatctgct     900
   tgtcctgaat ataatcgttt agatgatttc caaaaactca ctgagagttg ctgttcatct     960
   gatgcttttg aaagaataga ctcatttagt gtacagtcat tagatagccg gagtgtaagt    1020
   gaaatcaatt cagatgatga attgtcaggc aagggatatg ctttagtgcc tattatagtt    1080
   aattcttcaa ctccaaagtc taaaacagtt gaatctgctg aaggaaaatc tgaagaagta    1140
   aatgaaacat tagttatacc cactgaggaa gcagaaatgg aagaaagtgg acgaagtgca    1200
   actcctgtta actgtgaaca gcctgatatc ttggtttctt ctacaccaat aaatgaagga    1260
   cagactgtgt tagacaaggt ggctgagcag tgtgaacctg ctgaaagtca gccagaagca    1320
   ctttctgaga aggaagatgt ttgcaagaca gttgaatttc tgaatgaaaa gctggaaaaa    1380
   agggaggctc agttattatc tcttagtaag gaaaaagcac ttctagaaga agcttttgat    1440
   aacctgaaag atgaaatgtt cagagtgaaa gaagaaagca gtagcatttc ttccttgaaa    1500
   gatgagttta ctcaaagaat tgcagaagca gaaaagaaag ttcaactagc ctgcaaagag    1560
   agagatgctg ctaaaaagga aatcaaaaac ataaaagaag aacttgccac tagattaaat    1620
   agtagtgaaa ctgcagacct tttgaaagag aaagatgagc agatccgagg gttaatggaa    1680
   gaaggagaaa aactttcaaa acagcagctg cacaattcta acatcatcaa gaaattaaga    1740
   gctaaagaca aggagaatga aaatatggtt gcaaagctga acaaaaaagt taaagagcta    1800
   gaagaggagt tgcagcattt gaaacaggtc cttgatggca aagaagaggt tgagaaacaa    1860
   catagagaaa atattaaaaa actaaattcc atggtagaac gccaagagaa agatcttggc    1920
   cgtcttcagg tagacatgga tgaacttgaa gaaaagaacc gaagtattca ggctgccctg    1980
   gatagtgcat acaaagaact tactgatctt cacaaagcca atgctgcaaa ggatagtgag    2040
   gcacaggaag ctgctctgag ccgtgaaatg aaagctaaag aagaactttc tgcagcatta    2100
   gagaaggccc aagaagaagc ccgtcagcag caagaaacat tagccattca agtgggggac    2160
   cttaggcttg cattgcagcg tacagaacaa gcggctgcca gaaaggagga ttatttacgc    2220
   catgagatcg gtgaacttca gcagagactc caggaagcag agaatcgaaa ccaggaactg    2280
   agtcaaagtg tttcatcaac aacaagacca ttgcttcgac aaatagaaaa tttgcaagca    2340
   accctgggat cccagacatc gtcgtgggag aaattagaga agaatctttc tgataggctt    2400
   ggtgaatccc agaccttgct ggcagcagca gttgagagag aacgtgcagc tacagaagaa    2460
   ctccttgcta acaaaattca gatgtcttcc atggagtcac agaattctct tttaagacag    2520
   gaaaacagta gatttcaagc ccagctagaa tcagagaaaa ataggctgtg taaactggag    2580
   gatgagaaca ataggtacca ggttgaattg gaaaacctaa aagatgaata tgtaagaaca    2640
   cttgaagaga cgaggaaaga aaagacattg ttgaatagtc agttagaaat ggaaagaatg    2700
   aaagttgaac aagaaaggaa gaaagccatt tttactcaag aaacaataaa agaaaaggaa    2760
   cgcaagccat tttctgtttc tagcactccc accatgtcac gctcaagttc aataagtggt    2820
   gttgatatgg caggactaca gacatctttt ctgtctcagg atgagtctca tgatcactca 2880
   tttggaccaa tgcctatatc agccaaatgg aagcatcttt atgctgcctg taaggatggg 2940
   agcaggatca agcatattga aaacctacag tctcagctaa agctaaggga aggggaaatc 3000
   actcatttac agctagaaat tggcaatcta gaaaaaactc gatcaataat ggctgaagaa 3060
   ctagttaaat taacaaatca aaatgatgaa cttgaagaga aggtgaagga gatacccaaa 3120
   cttagaactc agctaagaga tttggatcaa aggtacaaca ctattctgca gatgtatgga 3180
   gaaaaagcag aagaggcaga agaacttcga ttagatctcg aagatgtaaa aaatatgtac 3240
   aaaactcaaa tagatgaact tttaagacaa agtctcagtt aa 3282
<210> 17
   <211> 4357
   <212> DNA
   <213> Homo sapiens
<400> 17
   atagtcacca gaagctggaa gagtcaaagg acacattctc ccctcaagcc ccagtgggag     60
   cacggcccag ctggattttg gacttctggc ctccagaact agacagggcc tcactgtgtc    120
   acccagggtg gaatacagtg gtgtgatcat agctcactgc agcctggaat tcctgggctc    180
   aagcaaccct gccacctcag ccttccaagt agctaggact acagaacatc catgatagca    240
   gtcttctgta aatcgaactt ttcaagaatt ctctgaagga accaagtagg atattcttac    300
   atcatgactt aatgtgaatg caagaacaag aaataggttt tatctctaaa tataatgaag    360
   ggctgtgtgt aaacactgac cctgtctcaa ttctaacaag cattttagac atgagtttac    420
   atcggcaaat gggttcagat cgagatcttc agtcctctgc ttcatctgtg agcttgcctt    480
   cagtcaaaaa ggcacccaaa aaaagaagaa tttcaatagg ctccctgttt cggaggaaaa    540
   aagataacaa acgtaaatca agggagctaa atggcggggt ggatggaatt gcaagtattg    600
   aaagtataca ttctgaaatg tgtactgata agaactccat tttctctaca aatacctctt    660
   ctgacaatgg attaacttcc atcagcaaac aaattggaga cttcatagag tgccctttgt    720
   gccttttgcg gcattctaaa gacagatttc ctgatataat gacttgtcat cacagatctt   780
   gtgtggattg cttacgacaa tatttaagga tagaaatctc tgaaagcaga gttaatatta    840
   gttgcccaga atgtactgaa cggtttaatc cccatgatat tcgcttgata ttaagtgatg    900
   atgtcttgat ggaaaaatac gaagaattta tgcttagacg gtggcttgtt gcagatcctg    960
   attgtaggtg gtgtccagct ccagactgtg gatatgctgt gatagcattt ggatgtgcca   1020
   gctgtccaaa attaacttgt gggcgagagg gctgtggaac agagttttgc taccactgta   1080
   aacagatttg gcaccccaac cagacctgtg atgctgctcg acaagagaga gcccagagct   1140
   tacgtttgag aactatacgt tcttcatcca ttagttatag tcaagagtct ggagcagcag   1200
   ctgatgatat aaagccatgt ccacgatgtg ctgcttatat aataaagatg aatgatggga   1260
   gctgcaatca catgacatgt gctgtttgtg gttgtgagtt ttgttggttg tgtatgaaag 1320
   aaatctcaga tttgcattat ctaagtccat caggatgtac tttttggggg aagaaaccct 1380
   ggagccgaaa gaagaaaata ttgtggcaac tgggaacact ggttggtgct cctgtcggaa 1440
   tcgctttaat agctggcatt gctattcctg caatgattat tggcattcct gtgtatgtgg 1500
   gccgcaagat tcacaatcgc tatgaaggca aggatgtttc aaagcacaaa cggaatttgg 1560
   ccatagcagg tggtgtaacg ttgtctgtaa tcgtgtctcc agtagtagct gcagtgactg 1620
   taggtatcgg tgttcctatt atgttagctt atgtctatgg cgtagttcca atttctcttt 1680
   gtcgaagcgg aggttgtgga gtctcagcag gcaatggaaa aggagttagg attgaatttg 1740
   atgatgaaaa tgatataaat gttggtggaa ctaacacagc tgtagacaca acatcagtag 1800
   cagaagcaag acacaaccca agcatagggg agggaagtgt tggtgggctg actggcagtt 1860
   tgagtgcaag tggaagccac atggatcgaa taggagccat ccgagacaac ctgagtgaaa 1920
   cggccagcac catggcacta gctggagcca gtataacggg gagtctgtca ggaagtgcca 1980
   tggtaaactg ttttaacagg ttggaagtac aagcagatgt acagaaagaa cggtacagtc 2040
   taagtggaga atctggcaca gtcagcttgg gaacagttag tgataatgcc agcaccaaag 2100
   caatggcagg atccattctg aattcctaca tcccattgga caaagaaggc aacagtatgg 2160
   aggtgcaagt agatattgag tcaaagccat ccaaattcag gcacaacagt ggaagcagta 2220
   gtgtggatga tggcagtgcc acccgaagtc atgctggcgg ttcatccagt ggcttgcctg 2280
   aaggtaaatc tagtgccacc aagtggtcca aagaagcaac agcagggaaa aaatcaaaaa 2340
   gtggtaaact gaggaaaaag ggtaacatga agataaatga gacgagagag gacatggatg 2400
   cacagttgtt agaacaacaa agcacgaact caagtgaatt tgaggctcca tccctcagtg 2460
   acagtatgcc ttctgtagca gattctcact ctagtcattt ttctgaattt agttgttctg 2520
   acctagaaag catgaaaact tcttgtagtc atggttccag tgattatcac acccgctttg 2580
   ctactgttaa cattcttcct gaggtagaaa atgaccgtct ggaaaattcc ccacatcagt 2640
   gtagcatttc tgtggttacc caaactgctt cctgttcaga agtttcacag ttgaatcata 2700
   ttgctgaaga acatggtaac aatggaataa aacctaatgt tgatttatat tttggcgatg 2760
   cactaaaaga aacaaataac aaccactcac atcagacaat ggaattaaaa gttgcaattc 2820
   agactgaaat ttaggcccat aaatgctgca gaataattac cactgtacaa ccgtgtttgg 2880
   agctggttga actacatgtg actacttaag tttcaggtta ccagcaaaag ccgggtttca 2940
   ttatcataat gcagatacat tttctgtgtt cagcaaggca ttgtgtgtca tgtggatctt 3000
   agttaccaaa ctatgaagtg aaggctttaa aagtgcatta ttttaaggat aataaatttg 3060
   aagagcaaag catgttttgt gtgtttgcca caaaacattg cttgaagcac atacttagat 3120
   agaaattggt cttaatttat ataatcaata taaaatacta atgcaattct acagcattca 3180
   aatgaagaaa acttgaggct ttagggataa gtggttagtg atattttatt gaaaccacta 3240
   aagagataag tttaaaagaa ctgcataggt tactctcagt atatgatact ctgtaacatt 3300
   tctatttata tcggcataaa tttcattttt tttcttcata tgcaatgtgg ttatataaag 3360
   cttaatgcag ctcatttgct accatttgga tacttagaca ctttgagcaa gattgtggca 3420
   gtttttgcac aactttgaaa tagaaatacc tggtactcta tcttgtttat tgttgatgcc 3480
   atcttagagg aaaaaatgta aaggtaagta attaagcata tgacagcaac aaataagata 3540
   cataaaacta caaaataaag tcccattagg ttataagtat tacaaaaaat ccacctttct 3600
   ctaaggggaa gtttgtaccc cattgattct tggtgccttt gggatcgact gggttttaat 3660
   ggcctagtta tttgaggatt ttgctgtgtt gttttccatg tcttctctgg tcaccttgga 3720
   ttatatataa aaatacagga aatagataaa catgaatgtg attaataatg ctgaaaaagt 3780
   attagcctac caaagacaca ctcaggcttt agtgaataac tttacataac ctcagttttt 3840
   aacacatgca tatcttctcc aaccatgaaa tcaaagcacg gtgcagaact tgtaccaagt 3900
   acaaaaggtc catgtatgat tagcattatt ttcttttgct tttgtttatg gacaatgttc 3960
   agctgacata agcagaagtt ggccaaaata ctgcctgtac tgttaatttc ctgtataatt 4020
   cacttaaata aaagcaggtt aacctcaatg atagcagtta aaatgttcta tcttatgtat 4080
   ttcttttaag tattaccatt atggtgctac tgagcgtttt cttttggtaa aaagaaaaat 4140
   gccatgggct gcagtcttct tccatcactt ttccctacca ggtccattaa tatgcttata 4200
   acactagtgc cagttatttt atttgataat gcttatggta tttgtatatt tgtttgcatt 4260
   ccaattttgt ttaataatga gtgtgtaaac tgcatacgtt aaataaatgt aaatactaat 4320
   gtactgctgc aaaaaaaaaa aaaaaaaaaa aaaaaaa 4357
<210> 18
   <211> 1544
   <212> DNA
   <213> Homo sapiens
<400> 18
   gcgctcccgg aacgcgcgca ccgcagacgg cgcggatcgc agggagccgg tccgccgccg 60
   gaacgggagc ctgggtgtgc gtgtggagtc cggactcgtg ggagacgatc gcgatgaaca 120
   cggtgctgtc gcgggcgaac tcactgttcg ccttctcgct gagcgtgatg gcggcgctca 180
   ccttcggctg cttcatcacc accgccttca aagacaggag cgtcccggtg cggctgcacg 240
   tctcgcggat catgctaaaa aatgtagaag atttcactgg acctagagaa agaagtgatc 300
   tgggatttat cacatttgat ataactgctg atctagagaa tatatttgat tggaatgtta 360
   agcagttgtt tctttattta tcagcagaat attcaacaaa aaataatgct ctgaaccaag 420
   ttgtcctatg ggacaagatt gttttgagag gtgataatcc gaagctgctg ctgaaagata 480
   tgaaaacaaa atattttttc tttgacgatg gaaatggtct caagggaaac aggaatgtca 540
   ctttgaccct gtcttggaac gtcgtaccaa atgctggaat tctacctctt gtgacaggat 600
   caggacacgt atctgtccca tttccagata catatgaaat aacgaagagt tattaaatta 660
   ttctgaattt gaaacaacat atttttatac ttaatgaatt gtatctcatt aatctcttcc 720
   cttacatctt catgtattgt tggtttgttt tttggttttg ggtttttttt tttttttttt 780
   ggtataagaa ctaacatcaa aaggcctgtt taaagggaaa ggttaatggg ctacttaata 840
   ttatgaacaa aacaaaaaaa caaggctgcc acagtggaat attatcttac aagaataaga 900
   actacataaa acagatttgt aaaaaataca tatttgaagt attccctgta tttccattat 960
   tctttatgga atataaagta agcatgaaag gtagttaaaa ctttcaggtg cctgtagagt 1020
   cataataact gtattttatg ccttgcattc acgcaaattc acattggatg tgatttaaaa 1080
   gtagacattc tctttttcct cttttaggat atgtttgatt actggaaaat taatatggtt 1140
   atttgttaga agtctggttt ataaaaaagc caaaagtgat ggaatttatt ccatttgtct 1200
   taggaaggcc cataatactt gtttttctta catgtgacta gcaactttct ccacttaaag 1260
   actaaatacc tctttatatg atgtaaatta ttctaattca ttttaaaatc ttttaggtca 1320
   gcaaaatgtg tgtcttcagt gctttctcta aaaacgttcc tttatagctt tgtacttttt 1380
   tttttagcgt tgccattgaa agttaaaatg tttgcatggt ttaccctctg agttatgttt 1440
   cttctagtga gcatgcctgc tgtcactaag tgaattattt actacttttt gtaggtctat 1500
   attttaataa ttattgggat aataataaaa aaaaaaaaaa aaaa 1544
<210> 19
   <211> 2913
   <212> DNA
   <213> Homo sapiens
<400> 19
   gaatggctgg ggttggctga agagcgcaca gtggagtttt aatgtccgcc atgttggcca     60
   tggcgtattg aagagagcga gcgagagagg agcggagcgg cacagcctcc caccctcccg    120
   gctggtgtta gtgcccggac ggcgggctct gcgctccgcc cctcaagtcc ccggcagcgg    180
   ttggcgagtg ggggccgaac ccccggttct ccatgatccc gctggccggg gccgtttccc    240
   cagagcggag aggtatctgc tgcgcctgag atgagtaaac tgtcgtttcg ggcgcgggcg    300
   ctagacgcct cgaagccgct gccggttttc cgctgtgagg atctgcccga cctgcacgaa    360
   tacgcctcga taaacagggc cgtgccgcag atgcccaccg gaatggagaa ggaagaggag    420
   tcggaacatc atcttcagcg ggctatttca gcacagcagg tgtatggcga gaagagggat    480
   aatatggtta taccggtccc agaggcagaa agtaatattg cttactatga gtctatatat    540
   cctggggaat ttaagatgcc aaagcagctc attcacatac agccttttag tttggatgct    600
   gaacagcctg attatgattt ggattctgaa gatgaagtat ttgtgaataa actgaaaaag    660
   aaaatggaca tctgcccatt gcaatttgag gagatgattg accgcctaga aaaaggcagt    720
   ggtcagcagc cagtcagtct gcaggaagcc aaactactgc taaaagaaga tgatgaacta    780
   attagagaag tttatgaata ttggattaaa aagagaaaaa actgtcgagg gccatctctt    840
   attccatcag taaaacaaga gaagcgagat ggttccagca caaatgatcc ttatgtggct 900
   tttagaaggc gtactgaaaa aatgcagact cgaaaaaatc gcaaaaatga tgaagcctct 960
   tacgaaaaaa tgcttaagct gcgacgagat ctaagtcgag ctgttactat tctagagatg 1020
   ataaaaagaa gagaaaaaag taaaagagag ctattgcact taacactgga aattatggaa 1080
   aagaggtata atttgggcga ctacaatgga gagatcatgt ctgaggttat ggcacagaga 1140
   cagccaatga aacctactta tgccatcccc atcatcccta ttactaatag cagtcaattt 1200
   aaacaccagg aagcaatgga tgtgaaggag ttcaaagtta ataagcaaga taaagccgat 1260
   cttatccgac cgaaacggaa atatgaaaag aagcccaaag tcttaccatc gtctgccgct 1320
   gctactcccc aacagacgag tcctgctgca ctgccagtct tcaatgctaa agatctgaat 1380
   cagtatgact ttcccagctc agacgaagaa cctctctccc aggttttgtc tggctcttcg 1440
   gaagctgagg aagacaatga tcctgatggt ccttttgctt tccgtaggaa agcaggctgt 1500
   cagtactatg ctcctcactt agaccaaact ggcaactggc cttggactag tcctaaagat 1560
   ggaggattag gggatgtgcg atatagatac tgcttaacta ctctcaccgt accccaaagg 1620
   tgtattggat ttgcacgaag acgggttggg cgcggtggaa gggtcttact ggacagagct 1680
   cattcagact atgacagtgt gtttcaccat ctggatttgg aaatgctttc ctcaccacaa 1740
   cattctccag tcaatcagtt tgccaatacc tcagaaacaa atacctcgga caaatctttc 1800
   tctaaagacc tcagtcagat actagtcaat atcaaatcat gtagatggcg gcattttagg 1860
   cctcggacac catccctaca tgacagtgac aatgatgaac tctcctgtag aaaattatat 1920
   aggagtataa accgaacagg aacagcacaa cctgggaccc agacatgcag tacctctacg 1980
   caaagtaaaa gtagcagtgg ttcagcacac tttgcattta cagccgaaca ataccagcaa 2040
   catcaacagc aactggcact catgcagaaa cagcagcttg cacaaattca gcaacagcaa 2100
   gcaaatagta attcctccac caacacatca cagaaccttg catctaacca gcagaaaagt 2160
   ggctttcgcc tgaatataca gggtttagaa agaacactac agggttttgt ttctaagact 2220
   ttggattctg ctagtgcaca gtttgctgct tctgctttgg tgacatcaga acaactgatg 2280
   ggattcaaga tgaaggatga tgtggtgctt ggaatcgggg tgaatggcgt ccttccagcc 2340
   tcaggagtat acaagggctt acacctcagt agtactacac caacagcact tgtacataca 2400
   agtccatcaa cggcaggttc agctttgtta cagccttcaa atattacaca gacttcaagt 2460
   tcccacagtg cactgagtca tcaagtaact gctgccaatt ctgcaacaac tcaggttctg 2520
   attgggaaca acattcgatt aactgtacct tcatcagttg ccactgtaaa ctctattgcc 2580
   ccaataaatg cacgacatat acctaggact ttaagtgctg ttccatcatc tgccttaaag 2640
   ctggccgctg cagcaaactg tcaagtttcc aaggtcccat cttcatcctc tgtagattca 2700
   gttccaaggg aaaatcatga atcagaaaag ccagcactga acaacatagc agacaacaca 2760
   gtagcgatgg aggtgacgta gcttcctccg agtggaactg cagcctgggg acttgattag 2820
   gtgctatgca tcagtagcat tttgaatgca agggatgatg gaaagcagca catgcgtttc 2880
   tgtggcagct gtggggactt gacagcaatg ctc 2913
<210> 20
   <211> 1512
   <212> DNA
   <213> Homo sapiens
<400> 20
   acttcgctgt gaagatggcg tcgggcagcg ggacaaaaaa cttggacttt cgccgaaagt 60
   gggacaaaga tgaatatgag aaactcgccg agaagaggct cacggaagag agagaaaaga 120
   aagatggaaa accagtgcag cctgtcaagc gagagctttt acggcatagg gactacaagg 180
   tggacttgga atccaagctt gggaagacaa ttgtcattac caagacaacc cctcaatctg 240
   agatgggagg atattactgc aatgtctgtg actgtgtggt gaaggactcc atcaactttc 300
   tggatcacat taatggaaag aaacatcaga gaaacctggg catgtctatg cgtgtggaac 360
   gttccaccct ggatcaggtg aagaaacgtt ttgaggtcaa caagaagaag atggaagaga 420
   agcagaagga ttatgatttt gaggaaagga tgaaggagct cagagaagag gaggaaaagg 480
   ccaaagcgta caagaaagag aaacagaagg agaagaaaag gagggctgag gaggacttga 540
   catttgagga ggacgatgag atggcagctg tgatgggctt ctctggcttt ggttccacca 600
   agaagagtta ctgaggcttt ctgtgcttgg cctgactttg gcctatgctg gacctaactt 660
   tgcgtgtgtg tgtgtgtagt agggggtcat ttctttttgg gtaatgggaa agttcttaag 720
   agtgtcaatg gggagggata gagggtgggg gctcatggtt tccctctact ttgggagagg 780
   gcacagattg cagaggtaat gctgtggcat attgcttctg cctcagtgta tcactggagt 840
   cacaggaccc tgcccacctg agttcccaat aaagaaaaac ctccccttct gaggctgctt 900
   tcccaaaact ccccctgcat ctttatctct tcatctatcc cacctcttgt ctgaacatcc 960
   cacctttatc ctgtgttctg cctttgtttt aattttaact catgttcatc ctgcaacaga 1020
   agcattctct aggtcccagt ttccagttga ttgcatatcc ttgatcagcc ctttttccca 1080
   tcctgcccta tggttctcta gccacctgtg catgcatgtg tatttctgcc tggttctatg 1140
   gtgtgtggat gtgtgtgcat gaatctgtca tatagagggg gtccgagctg gaatcctaga 1200
   gcattgctgc cctggggcct gatgttcttg gcttcctcag agcatgtaac aggaaattaa 1260
   atgggatgag tgtttggtgt ggtttgtgtc tgatgagttt tttaacattc aggtgtagat 1320
   tgtttcagct tctcttgttt cattttcctg aagatttatg tttttgtcta ccttgtgagc 1380
   aggcttttgg aagaacctgt ttgatgcaaa aaagaaaatg aaaaacaaaa caaaaaatcc 1440
   ccaaaacctt attatggggg cccttcggtc ttagaagctg tttgacatgt ataataaatg 1500
   gcattgactg gg 1512
<210> 21
   <211> 509
   <212> DNA
   <213> Homo sapiens
<400> 21
   tttttttttt tttttttaac accagaatct cttttagtct ttaacaaaag tgtagtcggt 60
   cctgtgcata catgggttct gcgtccctga acccaaccaa ccacagatca aacatatttg 120
   aggaaaaaaa aaaattccac aaagtcctga agagtaaaac ttgaatttgc cacgtgctga 180
   gtactatgtt caatttatgt gaatgaagtg acgtgcaggc attgtattag gtattataaa 240
   taatctacac atacaagtat atgagaggat gtggttaggt tatatgcaaa tactacaata 300
   ttttacataa ggaatttgaa catctgtggt atccactgat acaaagggat gactgtacac 360
   agcaagaaaa aggcaaaaag tgattcatta atccacataa tttttaaaga tctgctttat 420
   ctttaagtta aaaaaatgcc cctttcccca tcccatgcct tgtataaact actcaataac 480
   tgaagaaata aagttgcacc ctcgtgccg 509
<210> 22
   <211> 2109
   <212> DNA
   <213> Homo sapiens
<400> 22
<210> 23
   <211> 666
   <212> DNA
   <213> Homo sapiens
<400> 23
   atctgtacaa attattgagt aataatagta tctcttatag gattggtata aagacttaaa     60
   aagttaatgt catgtaaaat gctttagcat aatacctgac tcagagtaaa ggcttaataa    120
   accctagctg atcttactgt cagaatcaat aatattacta tttgtaatat aattaccaga    180
   cttaacaaat gggagaagta ttaaccatct gcaggaataa gtaccagagc ctgcatcaaa    240
   atagtttata ttcaacatga agtctttaaa ttggcaaaag cactcagttt aatatatacc    300
   tgttaagaaa attttaagag acagtcatgg tttttagctg gatactctgg gaaaccaaat    360
   aaagcagata attgtacagt atgataatca aaaaaaggat gtctttataa atagcttgaa    420
   atagttcccc agatgcaact tgaataaagg aaataaatag ccgacttgct ctaacttgag    480
   gttagagcaa aattcctagt ctgattcact ggatgctgaa aacccttgcc ccacattttc    540
   ccactccctc ttggttagaa aaatttacat tggtttagca ttatactctt ggagctattt    600
   tttttcaaaa aaaaaaaaaa aaacctcgag gggggcccgg tacccaattc gccctttgtg    660
   agtcgt                                                               666
<210> 24
   <211> 4134
   <212> DNA
   <213> Homo sapiens
<400> 24
   cgaagatggc ggcgtcgcgt cgctctcagc atcatcacca ccatcatcaa caacagctcc 60
   agcccgcccc aggggcttca gcgccgccgc cgccacctcc tcccccactc agccctggcc 120
   tggccccggg gaccacccca gcctctccca cggccagcgg cctggccccc ttcgcctccc 180
   cgcggcacgg cctagcgctg ccggaggggg atggcagtcg ggatccgccc gacaggcccc 240
   gatccccgga cccggttgac ggtaccagct gttgcagtac caccagcaca atctgtaccg 300
   tcgccgccgc tcccgtggtc ccagcggttt ctacttcatc tgccgctggg gtcgctccca 360
   acccagccgg cagtggcagt aacaattcac cgtcgtcctc ttcttccccg acttcttcct 420
   catcttcctc tccatcctcc cctggatcga gcttggcgga gagccccgag gcggccggag 480
   ttagcagcac agcaccactg gggcctgggg cagcaggacc tgggacaggg gtcccagcag 540
   tgagcggggc cctacgggaa ctgctggagg cctgtcgcaa tggggacgtg tcccgggtaa 600
   agaggctggt ggacgcggca aacgtaaatg caaaggacat ggccggccgg aagtcttctc 660
   ccctgcactt cgctgcaggt tttggaagga aggatgttgt agaacactta ctacagatgg 720
   gtgctaatgt ccacgctcgt gatgatggag gtctcatccc gcttcataat gcctgttctt 780
   ttggccatgc tgaggttgtg agtctgttat tgtgccaagg agctgatcca aatgccaggg 840
   ataactggaa ctatacacct ctgcatgaag ctgctattaa agggaagatc gatgtgtgca 900
   ttgtgctgct gcagcacgga gctgacccaa acattcggaa cactgatggg aaatcagccc 960
   tggacctggc agatccttca gcaaaagctg tccttacagg tgaatacaag aaagacgaac 1020
   tcctagaagc tgctaggagt ggtaatgaag aaaaactaat ggctttactg actcctctaa 1080
   atgtgaattg ccatgcaagt gatgggcgaa agtcgactcc tttacatcta gcagcgggct 1140
   acaacagagt tcgaatagtt cagcttcttc ttcagcatgg tgctgatgtt catgcaaaag 1200
   acaaaggtgg acttgtgcct cttcataatg catgttcata tggacattat gaagtcacag 1260
   aactgctact aaagcatgga gcttgtgtta atgccatgga tctctggcag tttactccac 1320
   tgcacgaggc tgcttccaag aaccgtgtag aagtctgctc tttgttactt agccatggcg 1380
   ctgatcctac gttagtcaac tgccatggca aaagtgctgt ggatatggct ccaactccgg 1440
   agcttaggga gagattgact tatgaattta aaggtcattc tttactacaa gcagccagag 1500
   aagcagactt agctaaagtt aaaaaaacac tcgctctgga aatcattaat ttcaaacaac 1560
   cgcagtctca tgaaacagca ctgcactgtg ctgtggcctc tctgcatccc aaacgtaaac 1620
   aagtgacaga attgttactt agaaaaggag caaatgttaa tgaaaaaaat aaagatttca 1680
   tgactcccct gcatgttgca gccgaaagag cccataatga tgtcatggaa gttctgcata 1740
   agcatggcgc caagatgaat gcactggaca cccttggtca gactgctttg catagagccg 1800
   ccctagcagg ccacctgcag acctgccgcc tcctgctgag ttacggctct gacccctcca 1860
   tcatctcctt acaaggcttc acagcagcac agatgggcaa tgaagcagtg cagcagattc 1920
   tgagtgagag tacacctata cgtacttctg atgttgatta tcgactctta gaggcatcta 1980
   aagctggaga cttggaaact gtgaagcaac tttgcagctc tcaaaatgtg aattgtagag 2040
   acttagaggg ccggcattcc acgcccttac acttcgcagc aggctacaac cgcgtgtctg 2100
   ttgtagagta cctgctacac cacggtgccg atgtccatgc caaagacaag ggtggcttgg 2160
   tgccccttca taatgcctgt tcatatggac actatgaggt ggctgagctt ttagtaaggc 2220
   atggggcttc tgtcaatgtg gcggacttat ggaaatttac ccctctccat gaagcagcag 2280
   ctaaaggaaa gtatgaaatc tgcaagctcc ttttaaaaca tggagcagat ccaactaaaa 2340
   agaacagaga tggaaataca cctttggatt tggtaaagga aggagacaca gatattcagg 2400
   acttactgaa aggggatgct gctttgttgg atgctgccaa gaagggctgc ctggcaagag 2460
   tgcagaagct ctgtacccca gagaatatca actgcagaga cacccagggc agaaattcaa 2520
   cccctctgca cctggcagca ggctataata acctggaagt agctgaatat cttctagagc 2580
   atggagctga tgttaatgcc caggacaagg gtggtttaat tcctcttcat aatgcggcat 2640
   cttatgggca tgttgacata gcggctttat tgataaaata caacacgtgt gtaaatgcaa 2700
   cagataagtg ggcgtttact cccctccatg aagcagccca gaaaggaagg acgcagctgt 2760
   gcgccctcct cctagcgcat ggtgcagacc ccaccatgaa gaaccaggaa ggccagacgc 2820
   ctctggatct ggcaacagct gacgatatca gagctttgct gatagatgcc atgcccccag 2880
   aggccttacc tacctgtttt aaacctcagg ctactgtagt gagtgcctct ctgatctcac 2940
   cagcatccac cccctcctgc ctctcggctg ccagcagcat agacaacctc actggccctt 3000
   tagcagagtt ggccgtagga ggagcctcca atgcagggga tggcgccgcg ggaacagaaa 3060
   ggaaggaagg agaagttgct ggtcttgaca tgaatatcag ccaatttcta aaaagccttg 3120
   gccttgaaca ccttcgggat atctttgaaa cagaacagat tacactagat gtgttggctg 3180
   atatgggtca tgaagagttg aaagaaatag gcatcaatgc atatgggcac cgccacaaat 3240
   taatcaaagg agtagaaaga ctcttaggtg gacaacaagg caccaatcct tatttgactt 3300
   ttcactgtgt taatcaggga acgattttgc tggatcttgc tccagaagat aaagaatatc 3360
   agtcagtgga agaagagatg caaagtacta ttcgagaaca cagagatggt ggtaatgctg 3420
   gcggcatctt caacagatac aatgtcattc gaattcaaaa agttgtcaac aagaagttga 3480
   gggagcggtt ctgccaccga cagaaggaag tgtctgagga gaatcacaac catcacaatg 3540
   agcgcatgtt gtttcatggt tctcctttca ttaatgccat tattcataaa gggtttgatg 3600
   agcgacatgc atacatagga ggaatgtttg gggccgggat ttattttgct gaaaactcct 3660
   caaaaagcaa ccaatatgtt tatggaattg gaggaggaac aggctgccct acacacaagg 3720
   acaggtcatg ctatatatgt cacagacaaa tgctcttctg tagagtgacc cttgggaaat 3780
   cctttctgca gtttagcacc atgaaaatgg cccacgcgcc tccagggcac cactcagtca 3840
   ttggtagacc gagcgtcaat gggctggcat atgctgaata tgtcatctac agaggagaac 3900
   aggcataccc agagtatctt atcacttacc agatcatgaa gccagaagcc ccttcccaga 3960
   ccgcaacagc cgcagagcag aagacctagt gaatgcctgc tggtgaaggc cagatcagat 4020
   ttcaacctgg gactggatta cagaggattg tttctaataa caacatcaat attctagaag 4080
   tccctgacag cctagaaata agctgtttgt cttctataaa gcattgctat agtg 4134
<210> 25
   <211> 1094
   <212> DNA
   <213> Homo sapiens
<400> 25
   cagattctgg ctcaggaaag tgatgcaaac gcgtcgtttt caaaggagag accccagcct 60
   cgggtcaggc gcggcgcaga cagcggcgcg gggtccttgg ctgggcgggg cttgctcgcg 120
   gtggcttgtg gctccttcct gcggtgcttc tctctttcgc tcaggcccgt ggcgccgaca 180
   ggatgggcaa gtgtcgtgga cttcgtactg ctaggaagct ccgtagtcac cgacgagacc 240
   agaagtggca tgataaacag tataagaaag ctcatttggg cacagcccta aaggccaacc 300
   cttttggagg tgcttctcat gcaaaaggaa tcgtgctgga aaaagtagga gttgaagcca 360
   aacagccaaa ttctgccatt aggaagtgtg taagggtcca gctgatcaag aatggcaaga 420
   aaatcacagc ctttgtaccc aatgacggtt gcttgaactt tattgaggaa aatgatgaag 480
   ttctggttgc tggatttggt cgcaaaggtc atgctgttgg tgatattcct ggagtccgct 540
   ttaaggttgt caaagtagcc aatgtttctc ttttggccct atacaaaggc aagaaggaaa 600
   gaccaagatc ataaatatta atggtgaaaa cactgtagta ataaattttc atatgccaaa 660
   aaatgtttgt atcttactgt cccctgttct caccacgaag atcatgttca ttaccaccac 720
   caccccccct tatttttttt atcctaaacc agcaaacgca ggacctgtac caattttagg 780
   agacaataag acagggttgt ttcaggattc tctagagtta ataacatttg taacctggca 840
   cagtttccct catcctgtgg aataagaaaa tgggatagat ctggaataaa tgtgcagtat 900
   tgtagtatta ctttaagaac tttaagggaa cttcaaaaac tcactgaaat tctagtgaga 960
   tactttcttt tttattcttg gtattttcca tatcgggtgc aacacttcag ttaccaaatt 1020
   tcattgcaca tagattatct taggtaccct tggaaatgca cattcttgta tccatcttac 1080
   aggggcccaa gatg 1094
<210> 26
   <211> 1565
   <212> DNA
   <213> Homo sapiens
<400> 26
   ctctttgctt ttgcatggct tggcttagta tccaaggtat attagggcca cttgaaagca      60
   tgaagaccag ttatataggg aacaggtttc tctcagtggc acattttgct ttttctgagc      120
   cccaaataca ttgcctgggc atgaacattg ttaccgtaaa ttgcacatgg tcatggactg     180
   aattatgtga ctttaaagga tgtaactgcc caacatttgc agattctggg tggtctatgt     240
   gaccatttgt ctcgtatcca aaaaccccgg ggctattgga acccttccaa cactttttcc     300
   tttgtcatag acaagtttat atataactta ccaagatgtt ggctgtcctg gtgtattgcc     360
   agacagctgt cttttggttc ccattccaaa tgtgctgctg tccttctttg catttcacaa     420
   tatcaaagaa accaccaccc ttcttcctaa cagcatttta tgccttttat tccacattaa     480
   atgggaattg tgcctactta ggagtgcccc tccaattaat tacatgtgtc caagaataat     540
   ccaagctaga gacacaaggt gggaaaacat ttcaaaaaaa aaaagtcctc ttaaggccag     600
   taatttatct gaaaaggtat tttatcacac cttgacacct tatatatgag cctattagga     660
   gctgcaggtg gtttcatagg gtaaaatcca agaaaagaga aggatgtgtg gggtttctat     720
   tagaagataa ttttgttctc attttacctt ttcttttatg atccttctct gctagaacag     780
   gttaattctc caaatttgtt ttgttttgtt ttgttatttt ttagggaact cttttgcaaa     840
   agcaatggtc ggatgtaaat aacatttaaa gtatagtgca cataacttcc ccggactgtt     900
   ccaatctgat aatttgtaaa tgctttagag tttttttaat taacacttgt gttgctaaat     960
   tctatttatg taagtctgct aaagtttttt agcccactta aaacttaaga caaccattta    1020
   aaataatgga tgggttacta tgagcaattt cgctttcaga acccccttgt tttagtatat    1080
   gaaaaagcct aatgcgcatt aatgaggttg aagagactat gagaaatatg tatagtgtat   1140
   attttaaaac agctttgctt gtattgtgaa gatttaaaaa caaacttgag atttttaacg   1200
   taactattaa cacagtttta acataagtta tcccactggg tttaagagca tcttgaatgt   1260
   ataatccttt ttgtaaccca ggttggtttc tacttttacc agtcacccaa acatatttat   1320
   gtttttagtt ttatgtactc atttcccttt gttttcctca aacagcatga tttttttgca   1380
   catgtagaaa ttttttaaaa gaaagaaatt agtacatcat tttctctgga ttttcttcac   1440
   ttccctcttc ctttctacta actccttcct taaaggccat atcactccat ttgcattatt    1500
   tgtgcaaatg ccagggttgg tttttatttt tatttttgct atttacctaa aaaaaaaaaa     1560
   aaaaa                                                                1565
<210> 27
   <211> 4877
   <212> DNA
   <213> Homo sapiens
<400> 27
   atggatggag gtgctttaac tgatacaagt ctcacagatt cctattttag caccagcttt 60
   attggagtca atggatttgg aagccctgta gaaacaaaat atcccctgat gcagagaatg 120
   actaatagta gcagctcccc aagccttcta aatgacagtg ccaagccata ttcagcccat 180
   gatcctctaa catcacctgc ttcatccttg tttaatgact ttggtgccct caacatctct    240
   cagagacgaa agacaccaaa tcctactgca agcgagttta ttcctaaagg aggatcaacc    300
   tccaggctga gtaacgtgtc ccagtcaaat atgtctgcct tctctcaagt tttctctcac    360
   ccatccatgg gaagccctgc tactgctgga ttagcgccag gaatgtcgtt gtctgctggg    420
   tcttcccctc ttcattcccc caagattact ccacatactt ctcctgctcc cagaagaaga    480
   agtcacactc caaatccagc aagttacatg gtgccttcta gtgcctctac atctgttaat    540
   aatcctgttt ctcagactcc gtcttctggt caggtgatcc aaaaggaaac tgttggtggg    600
   acgacttact tctatacaga cacaactcca gcacctttga ctggaatggt gtttccaaac    660
   tatcatattt atcctccaac tgcacctcac gttgcttata tgcaaccgaa agcaaacgca    720
   ccttccttct tcatggctga tgaactccga caggagctga tcaacagaca tttaataaca    780
   atggctcaaa ttgatcaagc agatatgcca gcagttccta cagaggttga cagctaccat    840
   agcctattcc ctctagaacc actgccacct cccaaccgga tacagaaatc aagtaatttt    900
   ggatatatta catcttgcta caaagctgta aacagcaaag atgatctgcc atattgcctt    960
   cggaggatac atggttttcg tcttgttaac acaaagtgca tggtgttggt cgacatgtgg   1020
   aagaaaattc aacactcaaa tatcgtaact ttgcgtgaag tatttaccac taaagcattt   1080
   gctgagccct ctcttgtgtt tgcatatgat ttccatgctg gaggagaaac tatgatgagc   1140
   agacacttta atgaccctaa tgctgatgcc tacttcacca agagaaagtg gggtcagcac   1200
   gagggaccat tgcccaggca gcatgctgga ttattgccag aatctcttat ttgggcatat   1260
   attgtccaac taagttctgc attgcgtacc attcatacag caggtttggc atgtcgagtt   1320
   atggatccaa caaagattct gataactggc aaaacaaggt tgcgagtaaa ttgtgttgga   1380
   gtttttgatg ttttaacatt tgataacagt caaaataata atccattggc attaatggcc   1440
   cagtaccagc aagcagatct gatatcatta ggaaaagttg tgttggcttt ggcttgcaac   1500
   tctttggcag gaattcagcg agagaattta cagaaagcca tggaactggt gacaatcaac   1560
   tattcctctg acctgaagaa tctgattttg tatttgttga ctgaccaaaa caggatgcga   1620
   agtgtaaatg acatcatgcc catgattggt gctcgatttt atactcaatt ggatgctgct   1680
   caaatgagaa atgatgtcat agaggaagac cttgcaaagg aggttcaaaa tggaagactg   1740
   tttaggctcc tagcaaaatt gggaacaatc aatgagaggc cggagtttca gaaggatccc   1800
   acttggtcag agactggaga ccgttatctg ttgaaactct ttagggatca tctttttcat   1860
   caggtgacag aagcaggtgc tccctggatt gacctcagtc atataatttc ttgtcttaac   1920
   aagctagatg ctggtgtgcc agaaaaaata agcctgattt ccagagatga gaagagtgta   1980
   cttgtggtga cctacagtga cttaaagcgc tgctttgaaa atacttttca agaactgatt   2040
   gcagctgcaa atggtcagtt gtagtatttg ctaaaaaagc acgcaggaca tggctaaaga   2100
   ccttaaccaa tagcaaattg cactacagct gaacttttca tcatctcatt cacatttggg   2160
   aaacgaacag gagatgagca aagctgcttg cacttcagtc aggtacactg ttacttgaaa 2220
   ggaagaatgt ttcacttacc caagagctat ggctgccatt ggaggctgtt atctgtgaag 2280
   aatttatttt agatttagga gcaccatcag gtgaatgatg ttcctgcttt tgttttttcc 2340
   acttgtatat gcactaattt taatttttta aagacttttt ctgatctttg aacttttgcc 2400
   acattgtata cttataatgg gaaactttgc aaggacattt ttgggaagca atgctgggca 2460
   gcgtttttgc cattgagggt tgcagaattt gctctttttg ggatgggttg ccctgaataa 2520
   cattacggac caggtaaata atttcataga agttcagtat agtacgtaat tcttgtaaga 2580
   gtatcgtatg caagctctct gtatgccacc cactgttagt tcaaattgag atttttgttt 2640
   tgttttgttc ttaagaacat accaagaaaa taccagtgaa ttgttggata tgaattccct 2700
   gttagttgat ttaaatcctt tctgaataaa gatcagataa acagtaactg aaggagcatg 2760
   tatagctctt tccttcaaat tcaactagag cctttttaaa aagacatttg cctgctagtc 2820
   agatacattt acatttatgc aaatttttta aataggagaa aattaagaaa ttgtgttaaa 2880
   ggcctaaagt tcaggagtgt attactttag gttctttcca gtttgctggt tttttttttt 2940
   tttttttttt ttttttttgg gcggggggga ggtttatgaa gttttgctct ttgaaccaca 3000
   gctttattat ggtcctttac actggagaca gacacagaga cactgttcag aagatgaact 3060
   aaactcagca gtggtctggt atcaagagct ttctgatgtt ttacagcctg aatttggagg 3120
   gataacgatc tgctgtgcct ttgcagtcac tgcttagccc aaagtaatag tacttttgat 3180
   aataactcac tctgtgcgat attcctgaat aagtccatct caaaagtttg ggattttcct 3240
   cctcttaact ttcttaatat ttggacatgc agttgtcgcc aaacttgggt attcatggaa 3300
   tttctagtaa atgaaatacc tatactttga tactgaagac tgccaaatac ataggaattt 3360
   tctttcttaa aaaacagtaa tgaagactat atctcctttc ccagcactga atgttttact 3420
   agcactgggt gctcaccatg caactgaaga aaatgtggaa actcaaaagg tcaggacaga 3480
   cttccaagca cttgcaactg atgttactgt cttcaatttt aataattaca catatttgta 3540
   tatttcagag aagtttttaa tatttctctg tccacttttt ataagcttta aaatgatttt 3600
   ctctgccttg agatttgcat caagaaaaag cacctctctt cacctgaaag ctttgaagac 3660
   tagagacacg ctttacacgt tttaacaagt atattgagtc cacgtttggt agcatcagtt 3720
   gttgagttaa aaagaaaatt attgcatttg atctggatgg attttaaaag aatataatgt 3780
   tcaatattaa aaggataatt cacatttgcc accataatgt tcttttttat gtaaaaagaa 3840
   gaacttggag acattaacat gaaaaagtct tttatcatta gctcatgtat ttgaggaaga 3900
   gcagctgtct ttttatatgt tttttgacaa atcatattgt attcttttgt acaaaaaaga 3960
   actacttgta ttctagaaga aatatgaaat gcttaattta taagcgggct ggagattttt   4020
   tccaatattg ttttctttga aaatgaaagg ggatcatcta ttttagtttt ggggtctggg   4080
   aactttttga aaatttaatt tgtggaccaa tgttttgtga aagctaaaga gggcaggggt   4140
   taaaataggg cttgaatttc tcattctgta tagaccagca aacttccctg tgcaaggcaa   4200
   gtttacatca caaatccaag aatgtttgca tcctaaatgc tagtttgctt cagcccctag   4260
   ttaacctcag gacttggttt gcatataaaa ggtagacagc tgatatgttt tcatgaataa   4320
   atattgtcag ccagaaaagg ttggtgtcag gtaatgcata tttttttaag ctttgtttta   4380
   tatttatttt tcatttagtt tttattggga atggttttca aagaactctc agttctgcct   4440
   aggtgttttt gggggagccc tgttttccat agtgtaattc catttaagag gttgtctaaa   4500
   agtcttttta attaatagaa agattttaat atccaagagt agtcaaatta aggatataaa   4560
   ctttccacac cttcctgtcg tgacagataa aagcacagaa aggacaaccc ttgaaatcat   4620
   gtaacgttgg tcatttcaat attttgtacc tgttttaaat tctgttagtg tatttacttc   4680
   attgtaaata tttttgaggg tacctttgta ttttgctttt gaccttggtt ctgtgatttg   4740
   gatgtcaaca acttccctaa aaagcaccag tgtgttaggt tctaatgtca tgacccaatt   4800
   tgtgttattc atctttaatc ctgttttcag tctctatgtg tacagcagta tttttaataa   4860
   agaattacag agataaa                                                  4877
<210> 28
   <211> 5052
   <212> DNA
   <213> Homo sapiens
<400> 28
   agtcccagct acttgggagg ctgaggcagg aggattgctt gagcccagga gttggaggct    60
   acaataagct atgatccagc tgctgcactc tagcttgggt gacagagacc ctgtcttctt   120
   aagacaaatc cagaatttgg gacatcttat aatttagttg ccctggattc ttcaaaagtt   180
   taagctatga agaaggacat ccctcacact ctgaaacaga tctccttcag agacagagtt   240
   ttgcagcctc tcatcagctt cctggatatg ctcccacacc tcagcctact ggtctttctg   300
   gaatatttga tactagtgtg aacagtgcca gcagtaacac taaagagtct tcagtgatga   360
   attttctgtc tactgctgaa tcccgaactg ctcaggctgc tgcttcagga actactctct   420
   taccacaatt cagggctcca tcctggcaga caggcatgca ttcctcagca gcaactgagc   480
   tgtttgctac tggacctttg ccaagcactg gaacacttcc accatctctc tctgcttatc   540
   agcatcccac caccttcagc aatagaaact ttgctaccac ttcacctttg gtgcttcagg   600
   attcaacttt taacactaca tcaaatggaa ttttaagtca tcatgaccct ttgctacaaa   660
   tcaagacttc ccagggaact gttccaactg ctttggcatt tgagcgcctg ggcagttctg   720
   tattaagtaa cagcatacca cctcagtctt caacataccg ctcagctcaa gagtctgcac   780
   cccatctttt acaacctcaa tttagtttgt tgccttcagc acttggggga tcccagcaga    840
   ctcctcaagc ctacagttca actctcttta ctagttctac tgcttccatt gaaagagctc    900
   ttcttcgaga atgtagtgtt attaaacacc atcagcggcc ttcaggtacc cagtcaattc    960
   aggcacaact gactggttca cagcactcct tacatagtta tctatcaaat tcaagtgtag   1020
   ttaattttca ggaaacaacc aggcagtcat ctttatcctg tagcccaatt ggagattcca   1080
   ctcagaattt gccagactct agcccgaccc agaattatat ttctatgcat tcttcccaaa   1140
   atgttcagac tcaagagtca tcatctcccc agtcccagaa gtttttgcct gctgtccagt   1200
   catcatcttt tgcatcctct actcattgtc agacattaca aaataacata acttcccctg   1260
   acccaaagtc ttatgctgaa agaaagcttg actcagatgt gtatccatct tcaaagcaag   1320
   aagatggttt tccaatgcaa gagttacagg tgttgcagcc acaagcatct cttgagtcat   1380
   caacccaaag gctatctgat ggagaaatta atgctcaaga atcaacttat aaggtgtcaa   1440
   aggcagatga cagatattct cagagtgtaa tcagaagtaa ttcccgtctt gaagatcaag   1500
   ttattggggt tgctctgcaa gcatcaaaaa aagaagaaag tgttgttggt tcagtgacac   1560
   aacttaacca acaaattggc caagtcaata atgcagctac ccttgatctt aagaactcaa   1620
   ctaatttaat acagactcca caaataaggt tgaatactaa agacttaaag cagcaacatc   1680
   ctctcatact taaggtgcat gagtccaagg tccaggaaca gcacgatcaa ataattaatg   1740
   cttcatctca gattcaaatt ccaaatcatg ctttagggca tggccatcag gcatctcttc   1800
   ctaatacaca ggtcctttta gattctgcct gtgatttaca aattcttcag cagtcaatac   1860
   tgcaggcagg tttaggtcaa gtaaaggcat ctttacaagc acagcgtgtt caaagccctc   1920
   aacaaatagt acatcccttc cttcagatgg aaggtcatgt tattcaaagc aatggtgatc   1980
   attctcagca gcaactccat cctcaaaatt ctgaagttat gaaaatggac ctctctgagt   2040
   cttcaaaacc attacaacaa catctaacaa caaagggcca ttttagtgaa acaaatcaac   2100
   atgattcaaa gaatcagttt gtttctcttg gatcgatgtg tttcccagag gcagtgcttc   2160
   ttagtgatga aagaaatatt ttatcaaatg tagatgatat cttagcagct acagcagcag   2220
   cttgtggagt tacacctact gatttttcca agtcaacttc aaatgaaacc atgcaggctg   2280
   ttgaagatgg tgattctaaa tctcattttc agcagtcatt agatgtcagg catgtgactt   2340
   cagattttaa ctctatgaca gctacagtag gaaagccaca gaatataaat gatacttcct   2400
   taaatggaaa tcaggttact gtgaaccttt caccagtacc tgcccttcag tcaaaaatga   2460
   ctcttgatca acagcacatt gaaacacctg gtcaaaatat accaactaaa gtaacttcag   2520
   cagtggttgg accaagtcat gaagtccagg agcaaagttc tggcccattc aagaaacagt   2580
   ctgctaccaa tcttgaatct gaagaagaca gtgaagctcc tgttgatagt acattaaata   2640
   ataacagaaa ccaagagttt gtttctagta gtagaagtat aagtggagag agtgctacat   2700
   cagagagtga atttacctta gggggtgacg acagtggtgt gtcaatgaac ccagctagga   2760
   gtgcacttgc actgttggcc atggcccaat ctggggatgc agtcagtgtc aagattgaag   2820
   aagaaaacca agatttaatg cattttaacc ttcaaaagaa aagagctaaa ggaaaagggc 2880
   aagttaaaga ggaagacaac agtaatcaga aacagctgaa aagacctgcc caaggcaaac 2940
   gccagaatcc aaggggaaca gatatttact taccgtatac tcctccttcc tcagaaagct 3000
   gccatgatgg ttatcagcat caagaaaaaa tgagacagaa gatcaaagag gtggaggaaa 3060
   aacaaccgga agtcaaaaca ggatttattg cttctttctt agattttctg aaatccgggc 3120
   ccaagcagca gttttccact cttgctgtac gaatgcctaa caggactaga cggccaggga 3180
   cccagatggt tcgtacattt tgtcccccac cacttcccaa gccttcatct acaacaccca 3240
   cacctttagt gtctgaaact ggcggtaaca gtccatcaga taaagttgat aatgaactta 3300
   aaaacttgga acatttatct tcattttctt ctgatgaaga tgatcctgga tatagtcaag 3360
   atgcttataa aagcgtctct actcccttaa ctactttgga tgctacttct gataaaaaga 3420
   agaaaacaga agccctacag gtggcaacta ctagcccaac tgccaatact actggtactg 3480
   ctactacttc ctcaaccact gtgggtgcag ttaagcaaga acctctccac tctacttcat 3540
   atgcagtaaa tattctggaa aatataagct cttcagaatc ctcaaagccc attgaacttg 3600
   atggtcttcc ttcagaccag tttgcaaaag gacaggacac tgttgccata gaaggtttta 3660
   cagatgagga ggacacagaa agcggaggag aaggccaata cagagagcgt gatgaatttg 3720
   tggtaaagat agaagacata gagactttta aggaggcttt aaaaacagga aaagaacctc 3780
   cagctatttg gaaagtacaa aaagctttat tacagaaatt tgttcctgaa attcgagatg 3840
   gtcaaagaga atttgctgct acaaatagtt atcttggata ttttggagat gcaaagagta 3900
   aatacaaaag aatatatgtg aagttcattg aaaatgcaaa caagaaggaa tatgtcagag 3960
   tgtgttctaa aaagccaaga aataaacctt cacaaactat cagaactgtt caagctaagc 4020
   caagtagtag cagtaaaact tctgatcctc tagcatcaaa aactacaact acaaaagccc 4080
   cttccgtgaa acccaaagtt aaacagccaa aagtaaaggc tgagccacca ccaaagaaac 4140
   ggaaaaaatg gaaagaagaa ttttcatcat cccaatctga ctcatctcct gagatccata 4200
   ctagtagtag tgacgatgag gaatttgaac ctcccgctcc ctttgtcact cgctttttga 4260
   acacaagagc aatgaaggaa acctttaaga gctacatgga atagcttgtt agcattgcct 4320
   tggaccctga cacaatgcaa gccttagaga agagcaatga tgagctactt ttacctcata 4380
   tgaaaaaaat agatggcatg ctaaatgata accgaaagag acttcttttg aatcttcatt 4440
   tggatcaatc attcaagaat gctttggaaa gttttcctga actaacaata attactcgag 4500
   attctaaagc aaagagtgga ggaactgcta tttctaaaat caaaatgaat ggcaaagcct 4560
   ataataagaa aactctaagg acttctaaaa caaccaccaa atctgcacaa gagtttgctg 4620
   tcgatccaga gaaaatacag ttgtattctt tgtatcattc actccatcat tataagtacc 4680
   atgtttatct gatatgtaag gatgagattt cttcggtgca gaaaaaaaat gaagatttag 4740
   gacaggagga aattgttcaa ctttgtatga aaaatgtaaa atgggtggag gacctctttg 4800
   aaaaatttgg agaacttcta aatcataccg gaatgttggg agaaatgcaa agcgtctcag 4860
   ttagcagcac cagaagatca gagcagcaga atattctaga ggctgggaag tccaagatca 4920
   aggtacctgt atctggtgac tgttgagagc cttcttgctg tgccttagaa tgaaagaagg 4980
   tggagggcaa ggtgatatga acaatgtgcc tttacatggc aaaagaatga aagaaagtaa 5040
   acctattccc ac 5052
<210> 29
   <211> 2261
   <212> DNA
   <213> Homo sapiens
<400> 29
   tcacctctgc attgtgttgg cttgctgtgt atcctttcag cttcagtttc ctttgcgaac     60
   tgtgattact tttaatagct cgattccaat tcctgagaag acaaatttac taggcctgtt    120
   gtatcccagt ttagttaaga tatttatata cccacagcaa cagtttgtgg atcttcactt    180
   ttgttttctg ccattaatat gatgattacc ctgttttgct gcttttactt tgcttgtttc    240
   ttatagctta gcttataaag tatccattaa caactgactg ttggacttct ctctctttgt    300
   ttcaaggact gcaattgcaa atgcctgtag gatccagatg ccataaatgt ataagatggt    360
   tctggtgttt tcttcttttc ttgaaacact gtcttgtgtg tgtgaggggg gtgatctttt    420
   gttcagtcat ttttgataga gacattagaa gagcgatttc tttaaaatat aaacaagggt    480
   gcaaatggga tggtaaaggg caactgacgc tactaggagg atagtaggga gtgtttgaca    540
   ttgtgtgtaa ttgggacata catatttcct ccaaaaggga agactttagt acttctgctg    600
   attgttgcct agtggaaatg tctgcctaag attaccagtt gtctacaaaa agcctgattg    660
   gcttcaggtt tttttttttt tttaatttag atatgattta acgtatatga taggatgtgt    720
   ataggttgta tgcaattact agaacatttt ctataaaggt catgaatatc tgtggtggat    780
   cctggaacca atggggttgg gagggcagct tcaattgttt taaaatgttt caggccaaca    840
   gaatatgctt ataaatgctt ttataacctc atagcttctg tttctttata gttttggctt    900
   atttgtaatg tttccttttt ctcatgtggt acaaaacatg gttatgtacc attacctctt    960
   caactcagcc agtggcttgg cagtttccct tgtgcagtgt ttaaaactgg gccacagttt   1020
   tgaatgcagt tgtggatatt aaaatgaaat agattaggaa agaatagatt aagatagaat   1080
   acagataaga aaataagaaa atatcagcct tcatctggca aagaaaatac cagccttcat   1140
   ctggcaaagg gcatcatcag gcatcaccag gaggtgctct gtatccatag gttccctatc   1200
   tgtgggttcc accaccactg ataaaaaata tttggaaaaa aatacaacaa tgcaaaataa   1260
   tacaaattta aaaaaataca atatatttat gtggtgttta cattgtattc gttattataa   1320
   gtaatctaga gatgatttaa agggacggga agatatatgt aggttatatg taaatatacc   1380
   attttatata aggaacgtgt atatccatgg attttagaat ctgtagggat tcctggaacc   1440
   agtcttccat ggataccgag ggatgactgt attgttctgt gaaatttttg tcccagctac   1500
   atatttgtat gtatgtatgt ttttggtccg gtataattgt ataatatttc ttactatggg   1560
   ttaaacaact ttataagaca ttgtgaggaa cctggtatcc tctggactag atcattcccg   1620
   tagacagttc cattgtgcac acatagtatt tcgttaaaat caggtgactg tttaaaaatt   1680
   aaaaagattt tagataaaaa tctagatttt tactttctcc tgaaatatca gaagatgggg   1740
   tctaaattca cactgaggcc aagcagtggt aggctcattt taaagaaggg ctttctgctc   1800
   tctggtttac cccattgtat actaattagc ctggtcttac ttttatatat tacctgaata   1860
   gccacttagg gcattttgaa cacgtggctt gcattgcttt gtctttttga aatccgctgt   1920
   ggcaggccca aaccttgcct ttggtgggct taagtaaggt ctggtttcca gtacaaaggc   1980
   aaaggcaaat gaggattatt agtattccat tttgccctat ggatattctc agtgctttct   2040
   actggaaact cacaaccaat tctgaatatg ccacatactg tgtgatttgc ccttgctgtc   2100
   caaccctgtt cttgctgcca tttaaaaaca atcaggccag gtgtggtggc tcatttctgt   2160
   ggtcccggca ctttgggagg ccggggtggg tggatcattt gagctctgga gttcgagacc   2220
   agccagggcc gcatggcgag acctcgtctc tactaaaaat t                       2261
<210> 30
   <211> 2219
   <212> DNA
   <213> Homo sapiens
<400> 30
   ggcaggtgtt gaggggctcc cggtccggct gccgccgctc ccccgctccg gacccggggc      60
   tccccctagc gccgctgagg agccgcctct gcggctccag gagggcgcag gagcgggact    120
   gagagcgcct ggaggctcga gcggagggta attcatttgc acacctgtta gcaagaaaca    180
   gaagttgaag gactggaaca agtgaactag gaaagaggga acgccaatcc aaggatagaa    240
   ggacaaggac agaatcacca gcactggctg aaggcctcct gtttcctgcg ctttctcctt    300
   ttcctgtgaa atctccgagg agaagaaaga atgatggaca gtttatcctt tcactgccac    360
   aaggcctgtt tacttggcag taggtcctta agttccttgc ttttttgctg ctgtttggtg    420
   actggaagag gcaccagaga ctctcactct ggggaggttt gctggcatgg gtaatctcat    480
   taaggtgcta accagggaca tagaccacaa tgcagcacat tttttcttgg actttgaaag    540
   taccttaaca tggggaatct tcttaaagtt ttgacatgca cagaccttga gcaggggcca    600
   aattttttcc ttgattttga aaatgcccag cctacagagt ctgagaagga aatttataat    660
   caggtgaatg tagtattaaa agatgcagaa ggcatcttgg aggacttgca gtcatacaga    720
   ggagctggcc acgaaatacg agaggcaatc cagcatccag cagatgagaa gttgcaagag    780
   aaggcatggg gtgcagttgt tccactagta ggcaaattaa agaaatttta cgaattttct    840
   cagaggttag aagcagcatt aagaggtctt ctgggagcct taacaagtac cccatattct    900
   cccacccagc atctagagcg agagcaggct cttgctaaac agtttgcaga aattcttcat    960
   ttcacactcc ggtttgatga actcaagatg acaaatcctg ccatacagaa tgatttcagc 1020
   tattatagaa gaacattgag tcgtatgagg attaacaatg taccggcaga aggagaaaat 1080
   gaagtaaata atgaattggc aaatcgaatg tctttgtttt atgctgaggc aactccaatg 1140
   ctgaaaacct tgagtgatgc cacaacaaaa tttgtatcag agaataaaaa tttaccaata 1200
   gaaaatacca cagattgttt aagcacaatg gctagtgtat gcagagtcat gctggaaaca 1260
   ccggaataca gaagcagatt tacaaatgaa gagacagtgt cattctgctt gagggtaatg 1320
   gtgggtgtca taatactcta tgaccacgta catccagtgg gagcatttgc taaaacttcc 1380
   aaaattgata tgaaaggttg tatcaaagtt cttaaggacc aacctcctaa tagtgtggaa 1440
   ggtcttctaa atgctctcag gtacacaaca aaacatttga atgatgagac tacctccaag 1500
   caaattaaat ccatgctgca ataacaattc tggaataagc acctgctgta gacagaagac 1560
   agtattctgc aatgactgag aatgcagttt tttagtgatt gcaattacta tctcatttat 1620
   tcttgctttt atttctttcc tctgttcctc ttccctcttt tttaatcatg ttcttaagac 1680
   ttcttttctg tgccaaaatc agtaaagtta cactctgaag ggatatcatc ctttcaaacg 1740
   ggccatctaa ggcagctaat tatgcattgc attggggtct ctactgagaa aaattctgtg 1800
   acttgaacta aatattttta aatgtggatt tttttgaaaa ctaatattta atattgcttc 1860
   tcctgcatgg caaaactgcc tattctgcta tttaaaaacc ctcaatgact ttattttcta 1920
   ctgccgcctt tttcatgtgc aaccaaaatg aaaatgttta aattaactgt gttgtacaaa 1980
   tggtacccaa cacaaacttt ttttaaatta gtaatacttt tgtttaaagt tttaagtttg 2040
   cattttgact ttttttgtaa ggatgtatgt tgtgtgttta acctttatta actaacgtta 2100
   aaagctgtga tgtgtgcgta gaatattacg tatgcatgtt catgtctaaa gaatggctgt 2160
   tgatgataaa ataaaaatca gctttcattt ttctaaaaaa aaaaaaaaaa aaaaaaaaa 2219
<210> 31
   <211> 10220
   <212> DNA
   <213> Homo sapiens
<400> 31
   ggaaactctg aaagaactta gaatcagcat tttgagagca gaagcttggg catgctgtga     60
   ttttccaata aactgctatc acaatgtcaa aatgcagttc agacaagagc aacacagaga    120
   tctcaaacat taaaacgtaa gctgtgctag aacaaaaatg caatgaaaga aacactggat    180
   gaatgaaaag ccctgctttg caacccctca gcatggcagg cctgcagctc atgacccctg    240
   cttcctcacc aatgggtcct ttctttggac tgccatggca acaagaagca attcatgata    300
   acatttatac gccaagaaaa tatcaggttg aactgcttga agcagctctg gatcataata    360
   ccatcgtctg tttaaacact ggctcaggga agacatttat tgcagtacta ctcactaaag    420
   agctgtccta tcagatcagg ggagacttca gcagaaatgg aaaaaggacg gtgttcttgg    480
   tcaactctgc aaaccaggtt gctcaacaag tgtcagctgt cagaactcat tcagatctca 540
   aggttgggga atactcaaac ctagaagtaa atgcatcttg gacaaaagag agatggaacc 600
   aagagtttac taagcaccag gttctcatta tgacttgcta tgtcgccttg aatgttttga 660
   aaaatggtta cttatcactg tcagacatta accttttggt gtttgatgag tgtcatcttg 720
   caatcctaga ccacccctat cgagaaatta tgaagctctg tgaaaattgt ccatcatgtc 780
   ctcgcatttt gggactaact gcttccattt taaatgggaa atgtgatcca gaggaattgg 840
   aagaaaagat tcagaaacta gagaaaattc ttaagagtaa tgctgaaact gcaactgacc 900
   tggtggtctt agacaggtat acttctcagc catgtgagat tgtggtggat tgtggaccat 960
   ttactgacag aagtgggctt tatgaaagac tgctgatgga attagaagaa gcacttaatt 1020
   ttatcaatga ttgtaatata tctgtacatt caaaagaaag agattctact ttaatttcga 1080
   aacagatact atcagactgt cgtgccgtat tggtagttct gggaccctgg tgtgcagata 1140
   aagtagctgg aatgatggta agagaactac agaaatacat caaacatgag caagaggagc 1200
   tgcacaggaa atttttattg tttacagaca ctttcctaag gaaaatacat gcactatgtg 1260
   aagagcactt ctcacctgcc tcacttgacc tgaaatttgt aactcctaaa gtaatcaaac 1320
   tgctcgaaat cttacgcaaa tataaaccat atgagcgaca gcagtttgaa agcgttgagt 1380
   ggtataataa tagaaatcag gataattatg tgtcatggag tgattctgag gatgatgatg 1440
   aggatgaaga aattgaagaa aaagagaagc cagagacaaa ttttccttct ccttttacca 1500
   acattttgtg cggaattatt tttgtggaaa gaagatacac agcagttgtc ttaaacagat 1560
   tgataaagga agctggcaaa caagatccag agctggctta tatcagtagc aatttcataa 1620
   ctggacatgg cattgggaag aatcagcctc gcaacaaaca gatggaagca gaattcagaa 1680
   aacaggaaga ggtacttagg aaatttcgag cacatgagac caacctgctt attgcaacaa 1740
   gtattgtaga agagggtgtt gatataccaa aatgcaactt ggtggttcgt tttgatttgc 1800
   ccacagaata tcgatcctat gttcaatcta aaggaagagc aagggcaccc atctctaatt 1860
   atataatgtt agcggataca gacaaaataa aaagttttga agaagacctt aaaacctaca 1920
   aagctattga aaagatcttg agaaacaagt gttccaagtc ggttgatact ggtgagactg 1980
   acattgatcc tgtcatggat gatgatgacg ttttcccacc atatgtgttg aggcctgacg 2040
   atggtggtcc acgagtcaca atcaacacgg ccattggaca catcaataga tactgtgcta 2100
   gattaccaag tgatccgttt actcatctag ctcctaaatg cagaacccga gagttgcctg 2160
   atggtacatt ttattcaact ctttatctgc caattaactc acctcttcga gcctccattg 2220
   ttggtccacc aatgagctgt gtacgattgg ctgaaagagt tgtagctctc atttgctgtg 2280
   agaaactgca caaaattggc gaactggatg accatttgat gccagttggg aaagagactg 2340
   ttaaatatga agaggagctt gatttgcatg atgaagaaga gaccagtgtt ccaggaagac 2400
   caggttccac gaaacgaagg cagtgctacc caaaagcaat tccagagtgt ttgagggata 2460
   gttatcccag acctgatcag ccctgttacc tgtatgtgat aggaatggtt ttaactacac 2520
   ctttacctga tgaactcaac tttagaaggc ggaagctcta tcctcctgaa gataccacaa 2580
   gatgctttgg aatactgacg gccaaaccca tacctcagat tccacacttt cctgtgtaca 2640
   cacgctctgg agaggttacc atatccattg agttgaagaa gtctggtttc atgttgtctc 2700
   tacaaatgct tgagttgatt acaagacttc accagtatat attctcacat attcttcggc 2760
   ttgaaaaacc tgcactagaa tttaaaccta cagacgctga ttcagcatac tgtgttctac 2820
   ctcttaatgt tgttaatgac tccagcactt tggatattga ctttaaattc atggaagata 2880
   ttgagaagtc tgaagctcgc ataggcattc ccagtacaaa gtatacaaaa gaaacaccct 2940
   ttgtttttaa attagaagat taccaagatg ccgttatcat tccaagatat cgcaattttg 3000
   atcagcctca tcgattttat gtagctgatg tgtacactga tcttacccca ctcagtaaat 3060
   ttccttcccc tgagtatgaa acttttgcag aatattataa aacaaagtac aaccttgacc 3120
   taaccaatct caaccagcca ctgctggatg tggaccacac atcttcaaga cttaatcttt 3180
   tgacacctcg acatttgaat cagaagggga aagcgcttcc tttaagcagt gctgagaaga 3240
   ggaaagccaa atgggaaagt ctgcagaata aacagatact ggttccagaa ctctgtgcta 3300
   tacatccaat tccagcatca ctgtggagaa aagctgtttg tctccccagc atactttatc 3360
   gccttcactg ccttttgact gcagaggagc taagagccca gactgccagc gatgctggcg 3420
   tgggagtcag atcacttcct gcggatttta gataccctaa cttagacttc gggtggaaaa 3480
   aatctattga cagcaaatct ttcatctcaa tttctaactc ctcttcagct gaaaatgata 3540
   attactgtaa gcacagcaca attgtccctg aaaatgctgc acatcaaggt gctaatagaa 3600
   cctcctctct agaaaatcat gaccaaatgt ctgtgaactg cagaacgttg ctcagcgagt 3660
   cccctggtaa gctccacgtt gaagtttcag cagatcttac agcaattaat ggtctttctt 3720
   acaatcaaaa tctcgccaat ggcagttatg atttagctaa cagagacttt tgccaaggaa 3780
   atcagctaaa ttactacaag caggaaatac ccgtgcaacc aactacctca tattccattc 3840
   agaatttata cagttacgag aaccagcccc agcccagcga tgaatgtact ctcctgagta 3900
   ataaatacct tgatggaaat gctaacaaat ctacctcaga tggaagtcct gtgatggccg 3960
   taatgcctgg tacgacagac actattcaag tgctcaaggg caggatggat tctgagcaga 4020
   gcccttctat tgggtactcc tcaaggactc ttggccccaa tcctggactt attcttcagg 4080
   ctttgactct gtcaaacgct agtgatggat ttaacctgga gcggcttgaa atgcttggcg 4140
   actccttttt aaagcatgcc atcaccacat atctattttg cacttaccct gatgcgcatg 4200
   agggccgcct ttcatatatg agaagcaaaa aggtcagcaa ctgtaatctg tatcgccttg 4260
   gaaaaaagaa gggactaccc agccgcatgg tggtgtcaat atttgatccc cctgtgaatt 4320
   ggcttcctcc tggttatgta gtaaatcaag acaaaagcaa cacagataaa tgggaaaaag 4380
   atgaaatgac aaaagactgc atgctggcga atggcaaact ggatgaggat tacgaggagg 4440
   aggatgagga ggaggagagc ctgatgtgga gggctccgaa ggaagaggct gactatgaag 4500
   atgatttcct ggagtatgat caggaacata tcagatttat agataatatg ttaatggggt 4560
   caggagcttt tgtaaagaaa atctctcttt ctcctttttc aaccactgat tctgcatatg 4620
   aatggaaaat gcccaaaaaa tcctccttag gtagtatgcc attttcatca gattttgagg 4680
   attttgacta cagctcttgg gatgcaatgt gctatctgga tcctagcaaa gctgttgaag 4740
   aagatgactt tgtggtgggg ttctggaatc catcagaaga aaactgtggt gttgacacgg 4800
   gaaagcagtc catttcttac gacttgcaca ctgagcagtg tattgctgac aaaagcatag 4860
   cggactgtgt ggaagccctg ctgggctgct atttaaccag ctgtggggag agggctgctc 4920
   agcttttcct ctgttcactg gggctgaagg tgctcccggt aattaaaagg actgatcggg 4980
   aaaaggccct gtgccctact cgggagaatt tcaacagcca acaaaagaac ctttcagtga 5040
   gctgtgctgc tgcttctgtg gccagttcac gctcttctgt attgaaagac tcggaatatg 5100
   gttgtttgaa gattccacca agatgtatgt ttgatcatcc agatgcagat aaaacactga 5160
   atcaccttat atcggggttt gaaaattttg aaaagaaaat caactacaga ttcaagaata 5220
   aggcttacct tctccaggct tttacacatg cctcctacca ctacaatact atcactgatt 5280
   gttaccagcg cttagaattc ctgggagatg cgattttgga ctacctcata accaagcacc 5340
   tttatgaaga cccgcggcag cactccccgg gggtcctgac agacctgcgg tctgccctgg 5400
   tcaacaacac catctttgca tcgctggctg taaagtacga ctaccacaag tacttcaaag 5460
   ctgtctctcc tgagctcttc catgtcattg atgactttgt gcagtttcag cttgagaaga 5520
   atgaaatgca aggaatggat tctgagctta ggagatctga ggaggatgaa gagaaagaag 5580
   aggatattga agttccaaag gccatggggg atatttttga gtcgcttgct ggtgccattt 5640
   acatggatag tgggatgtca ctggagacag tctggcaggt gtactatccc atgatgcggc 5700
   cactaataga aaagttttct gcaaatgtac cccgttcccc tgtgcgagaa ttgcttgaaa 5760
   tggaaccaga aactgccaaa tttagcccgg ctgagagaac ttacgacggg aaggtcagag 5820
   tcactgtgga agtagtagga aaggggaaat ttaaaggtgt tggtcgaagt tacaggattg 5880
   ccaaatctgc agcagcaaga agagccctcc gaagcctcaa agctaatcaa cctcaggttc 5940
   ccaatagctg aaaccgcttt ttaaaattca aaacaagaaa caaaacaaaa aaaattaagg 6000
   ggaaaattat ttaaatcgga aaggaagact taaagttgtt agtgagtgga atgaattgaa 6060
   ggcagaattt aaagtttggt tgataacagg atagataaca gaataaaaca tttaacatat 6120
   gtataaaatt ttggaactaa ttgtagtttt agttttttgc gcaaacacaa tcttatcttc 6180
   tttcctcact tctgctttgt ttaaatcaca agagtgcttt aatgatgaca tttagcaagt 6240
   gctcaaaata attgacaggt tttgtttttt tttttttgag tttatgtcag ctttgcttag 6300
   tgttagaagg ccatggagct taaacctcca gcagtcccta ggatgatgta gattcttctc 6360
   catctctccg tgtgtgcagt agtgccagtc ctgcagtagt tgataagctg aatagaaaga 6420
   taaggttttc gagaggagaa gtgcgccaat gttgtctttt ctttccacgt tatactgtgt 6480
   aaggtgatgt tcccggtcgc tgttgcacct gatagtaagg gacagatttt taatgaacat 6540
   tggctggcat gttggtgaat cacattttag ttttctgatg ccacatagtc ttgcataaaa 6600
   aagggttctt gccttaaaag tgaaaccttc atggatagtc tttaatctct gatctttttg 6660
   gaacaaactg ttttacattc ctttcatttt attatgcatt agacgttgag acagcgtgat 6720
   acttacaact cactagtata gttgtaactt attacaggat catactaaaa tttctgtcat 6780
   atgtatactg aagacatttt aaaaaccaga atatgtagtc tacggatatt ttttatcata 6840
   aaaatgatct ttggctaaac accccatttt actaaagtcc tcctgccagg tagttcccac 6900
   tgatggaaat gtttatggca aataattttg ccttctaggc tgttgctcta acaaaataaa 6960
   ccttagacat atcacaccta aaatatgctg cagattttat aattgattgg ttacttattt 7020
   aagaagcaaa acacagcacc tttaccctta gtctcctcac ataaatttct tactatactt 7080
   ttcataatgt tgcatgcata tttcacctac caaagctgtg ctgttaatgc cgtgaaagtt 7140
   taacgtttgc gataaactgc cgtaattttg atacatctgt gatttaggtc attaatttag 7200
   ataaactagc tcattatttc catctttgga aaaggaaaaa aaaaaaaact tctttaggca 7260
   tttgcctaag tttctttaat tagacttgta ggcactcttc acttaaatac ctcagttctt 7320
   cttttctttt gcatgcattt ttcccctgtt tggtgctatg tttatgtatt atgcttgaaa 7380
   ttttaatttt tttttttttg cactgtaact ataatacctc ttaatttacc tttttaaaag 7440
   ctgtgggtca gtcttgcact cccatcaaca taccagtaga ggtttgctgc aatttgcccc 7500
   gttaattatg cttgaagttt aagaaagctg agcagaggtg tctcatattt cccagcacat 7560
   gattctgaac ttgatgcttc gtggaatgct gcatttatat gtaagtgaca tttgaatact 7620
   gtccttcctg ctttatctgc atcatccacc cacagagaaa tgcctctgtg cgagtgcacc 7680
   gacagaaaac tgtcagctct gctttctaag gaaccctgag tgaggggggt attaagcttc 7740
   tccagtgttt tttgttgtct ccaatcttaa acttaaattg agatctaaat tattaaacga 7800
   gtttttgagc aaattaggtg acttgtttta aaaatattta attccgattt ggaaccttag 7860
   atgtctattt gattttttaa aaaaccttaa tgtaagatat gaccagttaa aacaaagcaa 7920
   ttcttgaatt atataactgt aaaagtgtgc agttaacaag gctggatgtg aattttattc 7980
   tgagggtgat ttgtgatcaa gtttaatcac aaatctctta atatttataa actacctgat 8040
   gccaggagct tagggctttg cattgtgtct aatacattga tcccagtgtt acgggattct 8100
   cttgattcct ggcaccaaaa tcagattgtt ttcacagtta tgattcccag tgggagaaaa 8160
   atgcctcaat atatttgtaa ccttaagaag agtatttttt tgttaatact aagatgttca 8220
   aacttagaca tgattaggtc atacattctc aggggttcaa atttccttct accattcaaa 8280
   tgttttatca acagcaaact tcagccgttt cactttttgt tggagaaaaa tagtagattt 8340
   taatttgact cacagtttga agcattctgt gatcccctgg ttactgagtt aaaaaataaa 8400
   aaagtacgag ttagacatat gaaatggtta tgaacgcttt tgtgctgctg atttttaatg 8460
   ctgtaaagtt ttcctgtgtt tagcttgttg aaatgttttg catctgtcaa ttaaggaaaa 8520
   aaaaaatcac tctatgttgc cccactttag agccctgtgt gccaccctgt gttcctgtga 8580
   ttgcaatgtg agaccgaatg taatatggaa aacctaccag tggggtgtgg ttgtgccctg 8640
   agcacgtgtg taaaggactg gggaggcgtg tcttgaaaaa gcaactgcag aaattcctta 8700
   tgatgattgt gtgcaagtta gttaacatga accttcattt gtaaattttt taaaatttct 8760
   tttataatat gctttccgca gtcctaacta tgctgcgttt tataatagct ttttcccttc 8820
   tgttctgttc atgtagcaca gataagcatt gcacttggta ccatgcttta cctcatttca 8880
   agaaaatatg cttaacagag aggaaaaaaa tgtggtttgg ccttgctgct gttttgattt 8940
   atggaatttg aaaaagataa ttataatgcc tgcaatgtgt catatactcg cacaacttaa 9000
   ataggtcatt tttgtctgtg gcatttttac tgtttgtgaa agtatgaaac agatttgtta 9060
   actgaactct taattatgtt tttaaaatgt ttgttatatt tcttttcttt tttcttttat 9120
   attacgtgaa gtgatgaaat ttagaatgac ctctaacact cctgtaattg tcttttaaaa 9180
   tactgatatt tttatttgtt aataatactt tgccctcaga aagattctga taccctgcct 9240
   tgacaacatg aaacttgagg ctgctttggt tcatgaatcc aggtgttccc ccggcagtcg 9300
   gcttcttcag tcgctccctg gaggcaggtg ggcactgcag aggatcactg gaatccagat 9360
   cgagcgcagt tcatgcacaa ggccccgttg atttaaaata ttggatcttg ctccgttagg 9420
   gtgcctaatc cctttacaca agattgaagc caccaaactg agaccttgat accttttttt 9480
   aactgcatct gaaattatgt taagagtctt taacccattt gcattatctg cagaagagaa 9540
   actcatgtca tgtttattac ctatatggtt gttttaatta catttgaata attatatttt 9600
   tccaaccact gattactttt caggaattta attatttcca gataaatttc tttattttat 9660
   attgtacatg aaaagtttta aagatatgtt taagaccaag actattaaaa tgatttttaa 9720
   agttgttgga gacgccaata gcaatatcta ggaaatttgc attgagacca ttgtattttc 9780
   cactagcagt gaaaatgatt tttcacaact aacttgtaaa tatattttaa tcattacttc 9840
   tttttttcta gtccattttt atttggacat caaccacaga caatttaaat tttatagatg 9900
   cactaagaat tcactgcagc agcaggttac atagcaaaaa tgcaaaggtg aacaggaagt 9960
   aaatttctgg cttttctgct gtaaatagtg aaggaaaatt actaaaatca agtaaaacta 10020
   atgcatatta tttgattgac aataaaatat ttaccatcac atgctgcagc tgttttttaa 10080
   ggaacatgat gtcattcatt catacagtaa tcatgctgca gaaatttgca gtctgcacct 10140
   tatggatcac aattaccttt agttgttttt tttgtaataa ttgtagccaa gtaaatctcc 10200
   aataaagtta tcgtctgttc 10220
<210> 32
   <211> 7638
   <212> DNA
   <213> Homo sapiens
<400> 32
   gcgccccgca ggaagtgctt ccctgggcgg aagcttctga gcgtgatata gcggaagtgc 60
   cttctcttcc ggtctttctg gtctcggccg cagaagcgag atgacgaagg gaacgtcatc 120
   gtttggaaag cgtcgcaata agacgcacac gttgtgccgc cgctgtggct ctaaggccta 180
   ccaccttcag aagtcgacct gtggcaaatg tggctaccct gccaagcgca agagaaagta 240
   taactggagt gccaaggcta aaagacgaaa taccaccgga actggtcgaa tgaggcacct 300
   aaaaattgta taccgcagat tcaggcatgg attccgtgaa ggaacaacac ctaaacccaa 360
   gagggcagct gttgcagcat ccagttcatc ttaagaatgt caacgattag tcatgcaata 420
   aatgttctgg ttttaaaaaa tacatatctg gttttggtaa ggtattttta atcaattagg 480
   cttgtagtat cagtgaaata ctgtaggttt agggactggg ctagcttcat atcagattta 540
   cttgttaagt gactgttttg gaatgtttac ttttggactg ggtttgtaac acggttaaag 600
   gcaatgagaa acaagcagaa ttccaggagt ccttgaagca gagggcactg gaagacaata 660
   tagcagatta aaatagcaca gctcatgtgg cataggtggg tattttagat gtttgagtaa 720
   atttgaaaga gtatgatgtt taaattacct ttagcaacat gttcatctgc tatgctgtca 780
   tgactagggg gatgattatt agtcacatag agcttgggag taccactgga aacgtatggg 840
   taggagttta ggtggcttct gtttttcaaa agatgatctt atcctagtat ctgtaatgct 900
   cacttggcac acctgacttg tgggctgtgt gtaaggtggc tagctaagtg aaaaaagcct 960
   gctaggtgtg agtcaactta agaatatgta aataggtttg agaaaaagta gggcttgggt 1020
   gcaagtaaag attgagcagg aaataaagga aaatcaagta taatccctga gatttgtaga 1080
   ctaaaggcaa tgatgtggga ctacttggtc gaattttttt agccctcaac ttggtaattg 1140
   ggtgtttctg tgttaaagca ctgaaacttg ctgtcgtgcc ttcctagttt tcgtggttta 1200
   ttgacagggt tgggggtttt ttttgttttt ttaaaatgaa gggacaaagt caactggact 1260
   gctgagtgag agggcagggg cagttgaagg gaacatgaat tgctggaaca gctacataaa 1320
   atagtgatgt agccaagtca tgctatttaa attataattc tccactgtgt ttagaataac 1380
   atctgaggtt cttaacctgg ccttggaagg gtatcacttt tacttgtaac ctggaatggc 1440
   tttataatgt gctagctaat tgctactctc atcttgtatt ttaactccta atttaccctt 1500
   caggtctcag cttcagaaca ttcacttata aagaaaccct gctgattaaa tctctcttgg 1560
   gcttcctccc gaaatgtgag actatacttt aaagatgtat ggttagagtc caattgccat 1620
   tgcctttctt gtttacagat agtgcaatgg cgcaatcctg gctcactgca gcctctaatt 1680
   cctgggctca agtcaagtgg ttctcctgcc tcagccttct gagtagctgg gactacagat 1740
   gcacaccacc acacctggct aatttttgtg ttttttgtag agatggagat tcactcgact 1800
   aattcttttt gtattcttag tagagactgg gtttcaacat gttgaccagg ctggtctcga 1860
   actcgacctc gtgatccgcc gcctcggcct cccaaagtgc taggattaca ggcgtgagcc 1920
   accactcccg gctgtcatca tcaaattttc aagtgaagat agtctgttga agattgaaca 1980
   atgaccttga aagacagctg agttgctgtg ggtataatgt aaagctggtg aagtttggcc 2040
   agtttgggct tcagaagtct aagtctagtg aaggtaccct gacccccata taaacaaccc 2100
   ttgaggccgg tgtggtggct cacggctgta atcctggcac tttgggaggc cgaggtgggc 2160
   ggatcacaag gtcaagagat cgagatcacc ctggcccaca tggtgagacc ccaactctac 2220
   taaaaataca aaaattagct gggtgtggtg gcatgtgcct gtagtcttag ctacttgaga 2280
   ggctgaggca ggagaatcgc ttgaacctgg gaggcagagg ttgcagtgag ctgagatcat 2340
   gccactgtgc tccagcctgg tggcacagtg agactccacc tcaaaaaaaa aaaaaaatcc 2400
   ttgaactaat gactcaaatt ttcaaatgaa acaaaataag cagtggatct tgcattggag 2460
   atggagttaa actatgttgc ccaggctggt cttggactcc tggtttcaag tgatcctctc 2520
   atcttggcct cccaaagtgc tgggattaca gggacgagcc accacacccc acctattgtc 2580
   tatatttcta tctttaacag cacttcagtc ctgccttaag ttatagttat gtatagatac 2640
   ccattatact ttaaattttt cagcagaaat tatgctttta tcttctctgc agtgttatat 2700
   gttggtgtgc aaaaatgtta aatttatttt tcctaagtac cccatctgct tttcaactct 2760
   gtcctctgcc tgaaaagcct ccctccagcc cctacttccc tcccatctta gttcacaaag 2820
   tcaggttgat ttgcccccag ctgtcaaagc agactacctg tttccacatg taactggtta 2880
   tgttctgtaa agttacaaaa atagaaaggt tgaatctgtg gcggccgggt acggtagctc 2940
   acgtcctata gtcgcagcac tttgggaggc caaggtgggc agttcacgtg aggtggggag 3000
   ttcgagacca gcctgaccaa catggagaaa ccccgtctct actaaaaata caaaaatatt 3060
   agccgggcgt ggtggtgcat gcctgtaatc ccagctacta gggaggctga ggcaggagaa 3120
   ttgcttgaac ctgggagacg gaggttgcgg tgagctgcag aggcctcacc tctaattgag 3180
   acacaattat atattgttga tatatatata tatatatata cacacacaca cacacactat 3240
   gatggataaa tgcatgagtt tctgtgagag cattggaaag gagtttgtca ctcaataggt 3300
   gaagccaggc taagatttaa gctgagccag ggaggacttg aaggaatcat gatgagagag 3360
   aaggtaagtg gctttgccag caatgaaaca gctgacataa tggtaaccag tcagaggagg 3420
   gcataactat gaaactggac accttggttg tcaggttaga aggatggggt gtagggttgg 3480
   taagaaaaga attcagggaa gagcagcgat cagattatga agaatttgtc ttgagaaatt 3540
   acagaggatt taaaccagaa tgttaggaat agttattcta gcaagatgaa tgtggaaagt 3600
   gttagtgtgc atgtgatgag tcttgaagct ggaaactagg taacaggttc ttaaatagtt 3660
   catgtgaaaa tcatgacaga ctaaggcagt ggctgtgggg ctgtccggga gttctctaca 3720
   gaaaacatct aaaacttgaa tgtgcaagtg agtagctaac ttccaagctt cccatttctg 3780
   tataatttaa gcatgaaaat gagaacactg agatttgata ggcatgtaga agtcagagta 3840
   agcaagaggg cttgagttca tcgatgaact tcagtaacta tccttgactt agtattggtg 3900
   gaaaccattt gtgaatttac agactccaaa aacaaaaaga taatttagag tctcaaagta 3960
   agatttgggg agatgtccat tggagacaga agtggagaga gggagaagtt catttgttga 4020
   catatttatg tatggtgcac ctactgtgtg ccaagccatg ttagagatac agtgggagca 4080
   aaaccagatg tgttttctgc cctctgactc cagtggttgc agaattagcc caggaactag 4140
   aattggatgg agtcacacaa accaaggaag gggattgttt gaggggggca ctcagtcaca 4200
   tgtggctgag gccaaagaga atgaagatat attttaaaat tctggatttg gcaaaattta 4260
   ggctagtctt tacttttcat aaacctctct catgttagag cagcaaagaa aaaccagttt 4320
   gccaagaggt cacgagattg ggcagtgagg aatggggagt ttagtttaaa agacaacact 4380
   agatactttt ggaagtttac ctattctgtg gttccctttt attgactaaa tggtgccctt 4440
   cattcatgaa gcaagtatct atttagtgac tactatgtgt caaaagctat ctaggcccag 4500
   aaacttttaa gtggaaaaca aaacaaagac cctgccctga cagatttcaa tcatgtatat 4560
   actgtatgta tgtttgtgga aggtgacata atagacaatt gcagatagtg ataagtgtgt 4620
   aagacaagga taccacgagc gtaatagaca gtggggatgg aggaaccttt agaaggttgg 4680
   ttaagttgaa tgataaggag ccattctttt gtctttacct gaatgactta tcattcagcc 4740
   ctttttgagt tttgggttgc ttgcagagtt taaccttgcc tgtaattgaa acttaatttt 4800
   gtaatagcat aacttcatgt aagaaaagca aagcaataca ttacagaatt attttactga 4860
   agaacttgtt tcagagaaag aggctgtttc acatttatta gcacatttgg attatattta 4920
   ggtgttttat ttttttttaa acaaaggagt ttggatcata atacaagaga agcacagggc 4980
   aaagacactg cataacctca agaactaaga atggaaggac tggccaggcc tggtggtgca 5040
   cacctgtaat cccagctact caggaggctg aggcgagaga atcgcttgag cctggggggc 5100
   agaggttgca gtgagctgag agaacggcgg gcggcagagc gagactccat ctcaaaaaaa 5160
   aaaaaggata aaggaaggac ttaagcaaaa tcttccttgt aagtagaagg atgttttgac 5220
   aagaaaagtt gcaatggaaa aatggttctc atgtacacga gtatgtagaa taagcatcgt 5280
   gtgtggattg gattcagatc aaaacattgc ttttatgttt gtgtctttat acggtgggag 5340
   tataccctgg tgccccagga tgaagacttg acctgaccca tgtattttta gattactcac 5400
   agataacaaa aagtattttc atcatgatta gttgcgaaaa cagttttatt tcaataggta 5460
   aaacgtgcag tcctatgtaa tcgtcagaag gtaatcttaa ttatagcttg ggtgtgcttt 5520
   aaactgcaag ctggcagtgg agggcacgat tcctctgatt tcagctttct ccttatactt 5580
   ttctggagct gtgagctgca agttaactca gtgggattaa agtgtagact ggaggtacaa 5640
   aaggtgagga gtgaggagat agggtagttc ttccttggct ggctggcttc ataatccctg 5700
   ggccccgcag ataattaaat cgactttttc tgtctcaggc atttgtatga cctctttgga 5760
   ggttccctgc tgggtagtta tccttgtatc tgatggaccc atctcaattt aaaatactct 5820
   gccaggttcg gaggttcatg cttgtcatcc cagcactttg ggaggctcag aggtgccatt 5880
   ggcttgagcc caagagtttg agaccagcct gggcaacctg gtgaaacctc ttctccatta 5940
   aaaatacaaa aaattagcca ggcatggtgg tatgtgcctg tagtcccagc tactcaggga 6000
   gctgaggtgg aaggattgct tgagcctggg aggtcaaggc tgcagtgaac cttgattgtg 6060
   ccactgcact ccagcctgga tgacaaagac cctgtctcaa gaaaagtctg taattctttc 6120
   ctaaccctta gtatccagcc tcagtcctga ggttttcttt acctctgggg gctattttat 6180
   gccggctctc tcctgagtgt cacacatctg gtcctcagga aacattctca catccctggc 6240
   ctgaaaaaaa caatttcagg gagatcgcat ggcagcagcc ctctctgggc tccccagcta 6300
   aatggttgta cgaaacacat ttcaaagctc tctgaagggc ttccttgaag ttcctttcac 6360
   tgggtttcaa agtagaaggt agtaactcct ttgtccaaga aggctgaatt gagcacttaa 6420
   caattctcca aagaaatttc ttcattggtc tctatcctag accccttctg tatccttgat 6480
   atggctgagg attctaaaaa atgaccagtc ttacatggga gggctgggat ataaaaaata 6540
   aaataataaa taatgataaa aactgaaaac tgaccacgtt cttggatatg ttttctttgg 6600
   gttgtgtgtg tgtgtgagac ttttgatagt tacccaaagt aggaaaaatc ccattctaat 6660
   aaggttatat ttatgtagct ctgcaaataa acatctagca aatgtaaaaa gtattttctt 6720
   tgccttaaaa atgattaaaa ttatttgaac tcctgaggag tgttatatga ataaaattag 6780
   taagttattt ggaggaaagt tattttttaa aaagacaact ggtaaaacag tacaggagaa 6840
   aggccagctt cctcaagtga ggacagttgt ttagaattga ctgaggagcg gccgggtgcg 6900
   gaggctcaca tctgtaatcc cacacgcctt gggaggctct gaggcgggta gatcacctga 6960
   ggtcaggagt ttgagaccag cctggccaac atggcgaaac cccgtctcca ctaaaaatac 7020
   aaaaattagc caggtgcagt ggcacacacc tgtaatccca gctactccga aggctgaggc 7080
   aggaggatca cctgagccca ggaagttgag actgcagtga gctgagattg caccactgca 7140
   ctccagcctc agtgacagcg agactgtctc aaaaaaagaa aaaagtgact gaggaggaag 7200
   aggccaggtg gcaaatggaa cagaatcacc aaagggtgaa caggactaag gcaatgtagt 7260
   gtatggctca gctacgtcag agtggaaaag gtgttattag agcagaaact atggtccctg 7320
   cgtcacaggg aagcaaccta cagagaagca gcagctcccc aagagaggag agataagaag 7380
   ccagaagcct cagagtgaac aattgtccta ttagggattg ctctagagag agaaacctct 7440
   gggaacgtac ccctgtgagg cagcacagca caatgctttt agaattgtat gagagttgat 7500
   atatctccat ttgttttgca aaggcaaaaa ctaagataca gagattatct gttaaagtta 7560
   tgtatttctt tggtaataaa gatgctgaca gtgttgctgg aatgcatttc tttaataaag 7620
   atattgtaca atgaaata                                                7638
<210> 33
   <211> 1637
   <212> DNA
   <213> Homo sapiens
<400> 33
   gaggcgaacc ggagcgcggg gccgcggtcg ccccgaccag agccgggaga ccgcagcacc    60
   cgcagccgcc cgcgagcgcg ccgaagacag cgcgcaggcg agagcgcgcg ggcgggggcg   120
   cgcaggccct gcccgcccct tccgtcccca cccccctccg ccctttcctc tccccacctt   180
   cctctcgcct cccgcgcccc cgcaccgggc gcccaccctg tcctcctcct gcgggagcgt   240
   tgtccgtgtt ggcggccgca gcgggccggg ccggtccggc gggccggggg atggcgctgc   300
   tggacctggc cttggaggga atggccgtct tcgggttcgt cctcttcttg gtgctgtggc   360
   tgatgcattt catggctatc atctacaccc gattacacct caacaagaag gcaactgaca   420
   aacagcctta tagcaagctc ccaggtgtct ctcttctgaa accactgaaa ggggtagatc   480
   ctaacttaat caacaacctg gaaacattct ttgaattgga ttatcccaaa tatgaagtgc   540
   tcctttgtgt acaagatcat gatgatccag ccattgatgt atgtaagaag cttcttggaa   600
   aatatccaaa tgttgatgct agattgttta taggtggtaa aaaagttggc attaatccta   660
   aaattaataa tttaatgcca ggatatgaag ttgcaaagta tgatcttata tggatttgtg   720
   atagtggaat aagagtaatt ccagatacgc ttactgacat ggtgaatcaa atgacagaaa   780
   aagtaggctt ggttcacggg ctgccttacg tagcagacag acagggcttt gctgccacct   840
   tagagcaggt atattttgga acttcacatc caagatacta tatctctgcc aatgtaactg   900
   gtttcaaatg tgtgacagga atgtcttgtt taatgagaaa agatgtgttg gatcaagcag   960
   gaggacttat agcttttgct cagtacattg ccgaagatta ctttatggcc aaagcgatag  1020
   ctgaccgagg ttggaggttt gcaatgtcca ctcaagttgc aatgcaaaac tctggctcat  1080
   attcaatttc tcagtttcaa tccagaatga tcaggtggac caaactacga attaacatgc  1140
   ttcctgctac aataatttgt gagccaattt cagaatgctt tgttgccagt ttaattattg  1200
   gatgggcagc ccaccatgtg ttcagatggg atattatggt atttttcatg tgtcattgcc  1260
   tggcatggtt tatatttgac tacattcaac tcaggggtgt ccagggtggc acactgtgtt  1320
   tttcaaaact tgattatgca gtcgcctggt tcatccgcga atccatgaca atatacattt  1380
   ttttgtctgc attatgggac ccaactataa gctggagaac tggtcgctac agattacgct  1440
   gtgggggtac agcagaggaa atcctagatg tataactaca gctttgtgac tgtatataaa  1500
   ggaaaaaaga gaagtattat aaattatgtt tatataaatg cttttaaaaa tctaccttct  1560
   gtagttttat cacatgtatg ttttggtatc tgttctttaa tttatttttg catggcactt  1620
   gcatctgtga aaaaaaa                                                1637
<210> 34
   <211> 7465
   <212> DNA
   <213> Homo sapiens
<400> 34
   acactctggg cgcggagcac aatgattggt cactcctatt ttcgctgagc ttttcctctt    60
   atttcagttt tcttcgagat caaatctggt ttgtagatgt gcttggggag aatgggggcc 120
   tcttctccaa gaagcccgga gcctgtcggg ccgccggcgc ccggtctccc cttctgctgc 180
   ggaggatccc tgctggcggt tgtggtgctg cttgcgctgc cggtggcctg gggtcaatgc 240
   aatgccccag aatggcttcc atttgccagg cctaccaacc taactgatga gtttgagttt 300
   cccattggga catatctgaa ctatgaatgc cgccctggtt attccggaag accgttttct 360
   atcatctgcc taaaaaactc agtctggact ggtgctaagg acaggtgcag acgtaaatca 420
   tgtcgtaatc ctccagatcc tgtgaatggc atggtgcatg tgatcaaagg catccagttc 480
   ggatcccaaa ttaaatattc ttgtactaaa ggataccgac tcattggttc ctcgtctgcc 540
   acatgcatca tctcaggtga tactgtcatt tgggataatg aaacacctat ttgtgacaga 600
   attccttgtg ggctaccccc caccatcacc aatggagatt tcattagcac caacagagag 660
   aattttcact atggatcagt ggtgacctac cgctgcaatc ctggaagcgg agggagaaag 720
   gtgtttgagc ttgtgggtga gccctccata tactgcacca gcaatgacga tcaagtgggc 780
   atctggagcg gccccgcccc tcagtgcatt atacctaaca aatgcacgcc tccaaatgtg 840
   gaaaatggaa tattggtatc tgacaacaga agcttatttt ccttaaatga agttgtggag 900
   tttaggtgtc agcctggctt tgtcatgaaa ggaccccgcc gtgtgaagtg ccaggccctg 960
   aacaaatggg agccggagct accaagctgc tccagggtat gtcagccacc tccagatgtc 1020
   ctgcatgctg agcgtaccca aagggacaag gacaactttt cacctgggca ggaagtgttc 1080
   tacagctgtg agcccggcta cgacctcaga ggggctgcgt ctatgcgctg cacaccccag 1140
   ggagactgga gccctgcagc ccccacatgt gaagtgaaat cctgtgatga cttcatgggc 1200
   caacttctta atggccgtgt gctatttcca gtaaatctcc agcttggagc aaaagtggat 1260
   tttgtttgtg atgaaggatt tcaattaaaa ggcagctctg ctagttactg tgtcttggct 1320
   ggaatggaaa gcctttggaa tagcagtgtt ccagtgtgtg aacaaatctt ttgtccaagt 1380
   cctccagtta ttcctaatgg gagacacaca ggaaaacctc tggaagtctt tccctttgga 1440
   aaagcagtaa attacacatg cgacccccac ccagacagag ggacgagctt cgacctcatt 1500
   ggagagagca ccatccgctg cacaagtgac cctcaaggga atggggtttg gagcagccct 1560
   gcccctcgct gtggaattct gggtcactgt caagccccag atcattttct gtttgccaag 1620
   ttgaaaaccc aaaccaatgc atctgacttt cccattggga catctttaaa gtacgaatgc 1680
   cgtcctgagt actacgggag gccattctct atcacatgtc tagataacct ggtctggtca 1740
   agtcccaaag atgtctgtaa acgtaaatca tgtaaaactc ctccagatcc agtgaatggc 1800
   atggtgcatg tgatcacaga catccaggtt ggatccagaa tcaactattc ttgtactaca 1860
   gggcaccgac tcattggtca ctcatctgct gaatgtatcc tctcgggcaa tgctgcccat 1920
   tggagcacga agccgccaat ttgtcaacga attccttgtg ggctaccccc caccatcgcc 1980
   aatggagatt tcattagcac caacagagag aattttcact atggatcagt ggtgacctac 2040
   cgctgcaatc ctggaagcgg agggagaaag gtgtttgagc ttgtgggtga gccctccata 2100
   tactgcacca gcaatgacga tcaagtgggc atctggagcg gcccggcccc tcagtgcatt 2160
   atacctaaca aatgcacgcc tccaaatgtg gaaaatggaa tattggtatc tgacaacaga 2220
   agcttatttt ccttaaatga agttgtggag tttaggtgtc agcctggctt tgtcatgaaa 2280
   ggaccccgcc gtgtgaagtg ccaggccctg aacaaatggg agccggagct accaagctgc 2340
   tccagggtat gtcagccacc tccagatgtc ctgcatgctg agcgtaccca aagggacaag 2400
   gacaactttt cacccgggca ggaagtgttc tacagctgtg agcccggcta tgacctcaga 2460
   ggggctgcgt ctatgcgctg cacaccccag ggagactgga gccctgcagc ccccacatgt 2520
   gaagtgaaat cctgtgatga cttcatgggc caacttctta atggccgtgt gctatttcca 2580
   gtaaatctcc agcttggagc aaaagtggat tttgtttgtg atgaaggatt tcaattaaaa 2640
   ggcagctctg ctagttattg tgtcttggct ggaatggaaa gcctttggaa tagcagtgtt 2700
   ccagtgtgtg aacaaatctt ttgtccaagt cctccagtta ttcctaatgg gagacacaca 2760
   ggaaaacctc tggaagtctt tccctttgga aaagcagtaa attacacatg cgacccccac 2820
   ccagacagag ggacgagctt cgacctcatt ggagagagca ccatccgctg cacaagtgac 2880
   cctcaaggga atggggtttg gagcagccct gcccctcgct gtggaattct gggtcactgt 2940
   caagccccag atcattttct gtttgccaag ttgaaaaccc aaaccaatgc atctgacttt 3000
   cccattggga catctttaaa gtacgaatgc cgtcctgagt actacgggag gccattctct 3060
   atcacatgtc tagataacct ggtctggtca agtcccaaag atgtctgtaa acgtaaatca 3120
   tgtaaaactc ctccagatcc agtgaatggc atggtgcatg tgatcacaga catccaggtt 3180
   ggatccagaa tcaactattc ttgtactaca gggcaccgac tcattggtca ctcatctgct 3240
   gaatgtatcc tctcaggcaa tactgcccat tggagcacga agccgccaat ttgtcaacga 3300
   attccttgtg ggctaccccc aaccatcgcc aatggagatt tcattagcac caacagagag 3360
   aattttcact atggatcagt ggtgacctac cgctgcaatc ttggaagcag agggagaaag 3420
   gtgtttgagc ttgtgggtga gccctccata tactgcacca gcaatgacga tcaagtgggc 3480
   atctggagcg gccccgcccc tcagtgcatt atacctaaca aatgcacgcc tccaaatgtg 3540
   gaaaatggaa tattggtatc tgacaacaga agcttatttt ccttaaatga agttgtggag 3600
   tttaggtgtc agcctggctt tgtcatgaaa ggaccccgcc gtgtgaagtg ccaggccctg 3660
   aacaaatggg agccagagtt accaagctgc tccagggtgt gtcagccgcc tccagaaatc 3720
   ctgcatggtg agcatacccc aagccatcag gacaactttt cacctgggca ggaagtgttc 3780
   tacagctgtg agcctggcta tgacctcaga ggggctgcgt ctctgcactg cacaccccag 3840
   ggagactgga gccctgaagc cccgagatgt gcagtgaaat cctgtgatga cttcttgggt 3900
   caactccctc atggccgtgt gctatttcca cttaatctcc agcttggggc aaaggtgtcc 3960
   tttgtctgtg atgaagggtt tcgcttaaag ggcagttccg ttagtcattg tgtcttggtt 4020
   ggaatgagaa gcctttggaa taacagtgtt cctgtgtgtg aacatatctt ttgtccaaat 4080
   cctccagcta tccttaatgg gagacacaca ggaactccct ctggagatat tccctatgga 4140
   aaagaaatat cttacacatg tgacccccac ccagacagag ggatgacctt caacctcatt 4200
   ggggagagca ccatccgctg cacaagtgac cctcatggga atggggtttg gagcagccct 4260
   gcccctcgct gtgaactttc tgttcgtgct ggtcactgta aaaccccaga gcagtttcca 4320
   tttgccagtc ctacgatccc aattaatgac tttgagtttc cagtcgggac atctttgaat 4380
   tatgaatgcc gtcctgggta ttttgggaaa atgttctcta tctcctgcct agaaaacttg 4440
   gtctggtcaa gtgttgaaga caactgtaga cgaaaatcat gtggacctcc accagaaccc 4500
   ttcaatggaa tggtgcatat aaacacagat acacagtttg gatcaacagt taattattct 4560
   tgtaatgaag ggtttcgact cattggttcc ccatctacta cttgtctcgt ctcaggcaat 4620
   aatgtcacat gggataagaa ggcacctatt tgtgagatca tatcttgtga gccacctcca 4680
   accatatcca atggagactt ctacagcaac aatagaacat cttttcacaa tggaacggtg 4740
   gtaacttacc agtgccacac tggaccagat ggagaacagc tgtttgagct tgtgggagaa 4800
   cggtcaatat attgcaccag caaagatgat caagttggtg tttggagcag ccctccccct 4860
   cggtgtattt ctactaataa atgcacagct ccagaagttg aaaatgcaat tagagtacca 4920
   ggaaacagga gtttcttttc cctcactgag atcatcagat ttagatgtca gcccgggttt 4980
   gtcatggtag ggtcccacac tgtgcagtgc cagaccaatg gcagatgggg gcccaagctg 5040
   ccacactgct ccagggtgtg tcagccgcct ccagaaatcc tgcatggtga gcatacccta 5100
   agccatcagg acaacttttc acctgggcag gaagtgttct acagctgtga gcccagctat 5160
   gacctcagag gggctgcgtc tctgcactgc acgccccagg gagactggag ccctgaagcc 5220
   cctagatgta cagtgaaatc ctgtgatgac ttcctgggcc aactccctca tggccgtgtg 5280
   ctacttccac ttaatctcca gcttggggca aaggtgtcct ttgtttgcga tgaagggttc 5340
   cgattaaaag gcaggtctgc tagtcattgt gtcttggctg gaatgaaagc cctttggaat 5400
   agcagtgttc cagtgtgtga acaaatcttt tgtccaaatc ctccagctat ccttaatggg 5460
   agacacacag gaactccctt tggagatatt ccctatggaa aagaaatatc ttacgcatgc 5520
   gacacccacc cagacagagg gatgaccttc aacctcattg gggagagctc catccgctgc 5580
   acaagtgacc ctcaagggaa tggggtttgg agcagccctg cccctcgctg tgaactttct 5640
   gttcctgctg cctgcccaca tccacccaag atccaaaacg ggcattacat tggaggacac 5700
   gtatctctat atcttcctgg gatgacaatc agctacactt gtgaccccgg ctacctgtta 5760
   gtgggaaagg gcttcatttt ctgtacagac cagggaatct ggagccaatt ggatcattat 5820
   tgcaaagaag taaattgtag cttcccactg tttatgaatg gaatctcgaa ggagttagaa 5880
   atgaaaaaag tatatcacta tggagattat gtgactttga agtgtgaaga tgggtatact 5940
   ctggaaggca gtccctggag ccagtgccag gcggatgaca gatgggaccc tcctctggcc 6000
   aaatgtacct ctcgtgcaca tgatgctctc atagttggca ctttatctgg tacgatcttc    6060
   tttattttac tcatcatttt cctctcttgg ataattctaa agcacagaaa aggcaataat    6120
   gcacatgaaa accctaaaga agtggctatc catttacatt ctcaaggagg cagcagcgtt    6180
   catccccgaa ctctgcaaac aaatgaagaa aatagcaggg tccttccttg acaaagtact    6240
   atacagctga agaacatctc gaatacaatt ttggtgggaa aggagccaat tgatttcaac    6300
   agaatcagat ctgagcttca taaagtcttt gaagtgactt cacagagacg cagacatgtg    6360
   cacttgaaga tgctgcccct tccctggtac ctagcaaagc tcctgcctct ttgtgtgcgt    6420
   cactgtgaaa cccccaccct tctgcctcgt gctaaacgca cacagtatct agtcagggga    6480
   aaagactgca tttaggagat agaaaatagt ttggattact taaaggaata aggtgttgcc    6540
   tggaatttct ggtttgtaag gtggtcactg ttctttttta aaatatttgt aatatggaat    6600
   gggctcagta agaagagctt ggaaaatgca gaaagttatg aaaaataagt cacttataat    6660
   tatgctacct actgataacc actcctaata ttttgattca ttttctgcct atcttctttc    6720
   acatatgtgt ttttttacat acgtactttt ccccccttag tttgtttcct tttattttat    6780
   agagcagaac cctagtcttt taaacagttt agagtgaaat atatgctata tcagttttta    6840
   ctttctctag ggagaaaaat taatttacta gaaaggcatg aaatgatcat gggaagagtg    6900
   gttaagacta ctgaagagaa atatttggaa aataagattt cgatatcttc tttttttttg    6960
   agatggagtc tggctctgtc tcccaggctg gagtgcagtg gcgtaatctc ggctcactgc    7020
   aacgtccgcc tcccgggttg acaccatttt cctgcctcag cctcctgagt agttgggact    7080
   accagtagat gggactacag gcacctgcca acacgcccgg ctaatttttt tgtattttta    7140
   gtagagacgg ggtttcacca tgttagccag gatggtctgg atctcctgac ctcgtgatcc    7200
   acccgcctcg gcctcccaaa gtgctgcgat tacaggcatg agccaccgcg cctggccgct    7260
   ttcgatattt tctaaacttt aattcaaaag cactttgtgc tgtgttctat ataaaaaaca    7320
   taataaaaat tgaaatgaaa gaataattgt tattataaaa gtactagctt acttttgtat    7380
   ggattcagaa tatactaaat taacttttta aaacacaact tttaaaaaat gtatcaaaaa    7440
   taataaacgt gttctgatat tttta                                          7465
<210> 35
   <211> 4380
   <212> DNA
   <213> Homo sapiens
<400> 35
   gcatatcttg agtagaactt aacatatctt gaatgggata ttttttcttt ttattcttat 60
   gagtggtctc acacaggacc aaacagcagg ccagcatagc accacgtgat ctgagcttgt 120
   ttttactctc tcctttttca taaacagagt cccagattaa tgccagtagt tgattttggg 180
   tctttggggt agtgaagctt gtggatttga cttctctgat tccttttgct gaccttgagt 240
   ggattgtatc tttcactgtt aattataata atttaaaaat tgagttctac ttactggaca 300
   tgaatctctg cacttgaaag ttaacatatt aaaagcatat tttttttctt ccttggtttt 360
   cttgttcatg tggataaccc agagaatgcc tgaatctaag gtgccatcct ctgagaatca 420
   tcgcccagaa atgtgctcta gctgcaatgt tcctcttccc attggagata gcagctcctt 480
   ctctgggagt tgttccagca gtccagaaag gatagtttct caaacttcct ctgttgagaa 540
   cccattggag aaccagaaaa atgatcaaaa taattcagat actaagatct ctgagacaga 600
   gacccttaaa tcatcacaga attttcagac tctgccttca tctccacttc tggtccccca 660
   agaatctttg gcctcttctg aggtcaaaga gaatttacgt atagattctt cttcagcttc 720
   acagcatgga cgggatgcca tcctctatct ccagacacaa gtagctgaaa tgtcccgagt 780
   gatacgtgat ctgcagtcca ggagctgttt tagatttcat cattctaggc caagtgagaa 840
   ctcctcagtt ccttgggaca tctccacctc taaagaggaa aatttatcca cagttgaaga 900
   agaaactgat tacaaatcac catcagctga tgacaaaggg cagccatctg accccagcca 960
   atctagtttc acaggtcttt tgaagagaat ggaacaaaga ggtgttataa aaagggtgac 1020
   attacaatct gaagcggagt catgtgaagg gaaacctgat tgtgtgactt ctaaaaaacg 1080
   tttggttcct ccattgcatc ctcttctgag aattgccacc actgaggttt ttaaagaccc 1140
   tgctgattgc catccttctt ccttcatggg acacagggta tatcctgtgg ccaaggacac 1200
   ctctcctttc caaccaaacc caccagctga aggccccatt gtagaagcat tagaacacag 1260
   caaaagagga aacacaacat cccctctaga ttctacctca aaagaaatgg aggtcatggg 1320
   ttgtaggttt taccacgctg cctccattgc agcccgagct gctagctaca tggcctatat 1380
   gactcaatat cagcgtaaac tctgggaaga catggaagat ctggttcatg acccagaatt 1440
   tgatcgtgga aaagcaagat gcataatatc tgatggtatg gatgcaggcc tttggcaact 1500
   ttgtactact agggacataa tggactctgt agtcagagtt atggccatgg ccatagacta 1560
   tagacggcag gcctggcttc gacttacatc tctcactaag aaaacccagg agaagatctc 1620
   ccacttgccc tttgatggta cttccctttt tggacaagat gtgaaagctg ttgttgcaga 1680
   agacaacaat ataaaagaaa atgactataa agatcacaaa tactataatc agcatcgata 1740
   cttttatagt catgatcaga aagcacatta tcacaataga ggatactcca aaggggattg 1800
   gtacaaacct cgaaaccacc cctatagata tagaaagaag ggagactctc cagaacgcca 1860
   tgggtacaag aattaataac ctgtttaatg ttcagcagag tagtcattca agatcctaac 1920
   tattttactt gttcctcctt gtacaaatag gatctacagg taggagatca tctcaaaaac 1980
   tggatgcatg tctggaatga tcacagctga catttgaatt ctagactttt gaaatgaagt 2040
   atataataaa gaaactcatg acttggccaa gtgagcagtg gagactttct tgaaagggtt 2100
   ggacaaatgt ttatccttta tttctggtcc aaaaataagt gtggaggagg accactcagg 2160
   aatctgaatc ctcttgtgat taaagaaaaa tataaagatg tgttcctcag gaaaatctta 2220
   tttgtaaact gaggactagc agattttgaa tctagaaata atactagatg tctgtttact 2280
   aagaccagag gtaaatcctt aacttttagg ttatcagtgc ttccttgttc aggcttcagt 2340
   ttgacagatc ttcacaaaat gacacatctg ttaaaatgtc tttatgtcaa gatggtcaac 2400
   tcaccaagag tccgtactac tatcaagctc tgtgagactt cagaatcagc catgaagctt 2460
   tgtgtcaact ggaattcaaa tcaaattttg ccaaatccag tttgcttccc cttggaatgg 2520
   ctagatgtcg caggtctatt atggacacca actggaaaaa gacttctacc tgtaacatct 2580
   ctgtagaaat tgcacagtaa ctatcttcat caagactttg atggccacca taccatcact 2640
   actcataaag ctacatgtga gagaattgtt tgttgttatc aatcctaata gtctttgacc 2700
   tatcaagctc tcgtatcaag ctgtaagact gagtcatggt agtctctcac tgtaagctgg 2760
   gcctttcacc aagatggcct tcttaaacaa cctaccttct aaacatttaa ttcttttgag 2820
   cttgatagtg catcacactc taaatacaaa aaagatacgc acatcagtgc cctggaaata 2880
   aaggatgtta cacgatccag gcatgcaaat ctgaactcct aagaccaaga attccaaaca 2940
   tgcctgacaa tcttgtcccc ttattgtaat aaagagggat accatcagtc tagattttat 3000
   tgagaatcct taagatttaa agcatagctg tggatgtggt gaagagaaca agccacacag 3060
   ctctgtttct ggatatctaa gtgatgatat ccattaatga tttgaaaggc tgaaatcaag 3120
   atttaagtga tacgctcttg gttaattggt tacttaattt agtcagattc ttagtgtaag 3180
   agacagctta tctttttgct cccaattggt cccattaata gtttttcacc atactggaac 3240
   tggcagcaga tacctcaatt atatgtccca cagtatcttc tctttagact caaggtagtg 3300
   gatgtatggt atatcagccc ttagatccat gtcttcacag catgacatca ggaatggaga 3360
   atatgtcaaa tttgaggggt ctgaatacat cacctagtat tatttgcttg gcagccaaaa 3420
   acagttgttt ttattgacag acttaattct gtaacttctg cgttttttca tctgcttgat 3480
   caaggcattt tggatgtact gctaatggaa tatggcactg ttttacggtt tatgtggctt 3540
   ctgttcttgc cacagttgtt gcacagtggt aattgatgtt ttctctggtg ccacactttt 3600
   taataatcta ttggaagctc attcctcctc ccccatcata accatattca gcacccattt 3660
   ttaaatctac ttttcttcct tatttgtgct acagaggttg atggcgtaaa ttttcctact 3720
   tggaagaaat tactttatca gttaatttca gggtactgta ttgatcttgt ttgtcctatt 3780
   tgtagattaa acttgaacta gtcttaataa aggggatatt gttttgcaaa tttagcccag 3840
   tcatatcaag agttaatatt tatcttattt ttctgtattt tactcaaaat tttatttcca 3900
   cttctgtaga aataggtcca tgccagtgtt ctctacaaag tgtctttcaa actaaactct 3960
   agaagaatat cgattacctg aacagaaaca aaggcctaag aaatcataca tttttgtagc 4020
   ttatgtgtat tgcaccaatc aagcaatccc atgtgctctt gatattggca ctgtagtttt 4080
   atcttagagg acttgccact ggagaactca gtggtgagag accttggaaa tgttttatgt 4140
   ggctgcacat aagtgtttct gactagctaa ggctacttca gacttttcac tttccatttg 4200
   tccccttttc cttccctgaa ttagatgcta tgtcataaac ttggtggttt ttgaaatact 4260
   cttatttcaa accttaaact aaaccgaaat aagttatgcc tgagaatgcg tttgtctgaa 4320
   acgattttgg cacttccctt taatatgctt ccccttccct ttgcctttta aattgtggtt 4380
<210> 36
   <211> 2001
   <212> DNA
   <213> Homo sapiens
<400> 36
   gaagggcggc gcgcttaggc aggcggtggc gcggctggag tgccgcgggg agggctgtgc 60
   cggttgcttt ctgcagccgc atctcggcca gctctcctcg ccgtccccgg ggcgctgtgc 120
   gtctccagtc cgggaccgaa gccgcctgcc gtagcgggcg gccagatccg cgtcccgcct 180
   cagcggccgg aggacatgcg ggagagagaa tgagccagag ggacacgctg gtgcatctgt 240
   ttgccggagg atgtggtggt acagtgggag ctattctgac atgtccactg gaagttgtaa 300
   aaacacgact gcagtcatct tctgtgacgc tttatatttc tgaagttcag ctgaacacca 360
   tggctggagc cagtgtcaac cgagtagtgt ctcccggacc tcttcattgc ctaaaggtga 420
   tcttggaaaa agaagggcct cgttccttgt ttagaggact aggccccaat ttagtggggg 480
   tagccccttc cagagcaata tactttgctg cttattcaaa ctgcaaggaa aagttgaatg 540
   atgtatttga tcctgattct acccaagtac atatgatttc agctgcaatg gcaggtttta 600
   ctgcaatcac agcaaccaac cccatttggc ttataaagac tcggttacag cttgatgcaa 660
   ggaaccgcgg ggaaaggcga atgggtgctt ttgaatgtgt tcgtaaagtg tatcagacag 720
   atggactaaa aggattttat aggggcatgt ctgcttcata tgctggtata tcagagactg 780
   ttatccattt tgttatttat gaaagtataa aacaaaaact actggaatat aagactgctt 840
   ctacaatgga aaatggtgaa gagtctgtga aagaagcatc agattttgtg ggaatgatgc 900
   tagctgctgc cacctcaaaa acttgtgcca caactatagc atatccacat gttgtaagaa 960
   caagactacg tgaagaggga acaaaataca gatctttttt tcagactcta tctttgcttg 1020
   ttcaagaaga aggttatggg tctctttatc gtggtctgac aactcatcta gtgagacaga 1080
   ttccaaacac agccattatg atggccacct atgaattggt ggtttaccta ctcaatggat 1140
   agcagcacga ggactgctgt actgcaaaaa aagaagacca aaagattaca gtggaccatg 1200
   ggatacagaa gccagcatgg cagacagaag aaaaatagtt tgggaacatg taactattct 1260
   aagtggaagt tttgttgtag gaattatagt aatcacacca cattacttgg cctttcggta 1320
   atgtgaaaaa aaaaaaaaaa cctcagagcc tccaaggaaa tgcctttaga agcactcctc 1380
   tctcaaaatt gccattttct ctaccatgtc ccccagacac agttgggttt tgttgattta 1440
   tggcagtctt ctaaacaaag ccatccttaa ttttacatac tgtattgtaa ctatccaaag 1500
   atagtattgg cagtcataaa tttataactt ctggcctttg tttaattcca gtaaaataac 1560
   agcaatgaca atgagatcgt cagtattatt ttcacatttc cctgagagga cctggcacaa 1620
   tattttaaat ttaattattt ggcagtagtc actgtttttg acaaccaatg aaatagcgtc 1680
   taaatatctt gtatattttt tagcatcaaa atgttttggt ttccactgct gacaggtgct 1740
   tgttgtttag cctaatgtgg atatttaaat tgttaacata ccaaacacac atggtaatag 1800
   tttgggacaa aataactagt aaaatgttga tttctcggct atttcactga ctgcggcatg 1860
   tcctcgtgtc ttctctgatt ttgtggtaca tgaatgaatg catttatctt tttagtgact 1920
   tactagttac gaacttgaat tatcacctct ttgtcatact taagtatcat ttacataaag 1980
   tgtaaaagat tttttcactt t 2001
<210> 37
   <211> 1377
   <212> DNA
   <213> Homo sapiens
<400> 37
   agcgggactc cgggccccgg cggcggccca tggggcggga ggcgtgaggc cgctgcctgt 60
   ccggggctcg gggggtgggg ggagcggggc ggggagatgg ataaactgac catcatctca 120
   ggatgtctct ttctggccgc cgatatcttc gccatcgcca gcatcgccaa cccggactgg 180
   atcaacaccg gggagtctgc ggtctcacct taatccagct cagttctgtg tacaaaccat 240
   ggaggttatt cattcaggtg aaataaatgg gttacttctg ccagattaat ctgggaagaa 300
   acagagcact cactgtgggc ctcgtgcgac agtgtcaaac aatccatgga cgagaccgga 360
   cgtgcatccc tccccggctt cccccggagt gggtcaccac actgtttttt atcatcatgg 420
   gaatcatttc attgactgtc acatgtggtt tgctggtggc ttcccactgg cgaagagaag 480
   ctacaaaata tgctcgatgg atagcattca ctggaaagtg tgtgcattct tctcatacct 540
   aagaaaatca ctgtaaaatc tgctgaaaac tatttttagg ttttatttgc acaagactga 600
   attagtttga catttttgga agctcctatt gaacataccc aaacatctgt aaacatgaaa 660
   aatcttcaat ttattaaaag caaacatttc agtatgattc tttccaaagt ttgctgctga 720
   ttcttccttg tgtggccttt tgaggagcct cctggggaat tggaggatta agacatgcca 780
   ctatgagaag attataggaa aaacaccaag actagaggac tctgggttct ttttatgcaa 840
   agtcaactct tctgggtcac agttacccag caacaaaaat aaagaatttc cccatggact 900
   ccagatcctt tactactgaa ctcacagggc tgtaacactg ttactgtaca gagaggacca 960
   ggacgatgcc agcaccccgt ttatcctgag tgaactctcc ggaggcctct tcaagcttgt 1020
   gggttctctg ctgtcttgaa gccatccatc catttgatag gttttgcaaa gacttggtcc 1080
   tgccaagatg gttttaatca tttctgctaa aaggaatgga ctcgaggatt tgatctcatt 1140
   ttagatgcag ttgtcctcac ttggccattt tacagcactt tagtaaatat ggccagtgta 1200
   tttggtcact attaaatcaa tccccattca ttatctgtca gggcaactca gtgaactaaa 1260
   tactatgttc tgacctctgg cactctttct catgttgttt aaatatttaa tattgtctaa 1320
   ggcaattcaa gtattttctt aaataaaaaa tatgaaaacc caaaaaaaaa aaaaaaa 1377
<210> 38
   <211> 8811
   <212> DNA
   <213> Homo sapiens
<400> 38
   taatatattt agttttttta aaatcatggt aattgattgg aagatttatt tttaatagct 60
   ttgccttttt tgcagagtgc aaaatttcta aattataact tatgtcataa agcaaaatta 120
   tatttggtat caccataaat ttctattctg aatggtgaga acagataatt tgaatatttc 180
   tcacctgtga gtctatatta gtaagctttt tattttaatg tactgtaatg aagtggtcac 240
   gagtagttac ctcggctcaa aaatgtcatt tagtttgtta aactattttc acaaccatca 300
   taagcataac tagataactg tccattccca gcagttcagt ttagtctcca ttgtgtattt 360
   aaaggttgtc gcagagcagc acattgactt cctcatcagc cctttgacaa ggaagggaaa 420
   gtcgtgggta gtcaatttga actttttctg tctacttgtc ccctcatgtt agctgcttga 480
   aattcttcag ttatgataca aagctgagtt tgcagtaagt tgcacatttt attgtttgca 540
   attttctgtt ttatgtgcat ttaaaagccc ttctctgctt tctagtaagg aggcatgaaa 600
   ttatcaggaa tgttgctagt tacagcttca caccaaaaac tttgagtaaa tacattttcg 660
   tttggatgcc catgatgaaa gctgaggaaa gtggttgagg aattatcaac aacaaacttt 720
   tcctcttatt ttatctagtt caaaacagat atcaaagttt gcaatttgct ttgctcttgc 780
   tgtttcttat gagtcccttt ttggaagaga ggagaacatc tataagccca gagagatctg 840
   tgtttttctt ttacttaata gagccattct cgtctattag taggaacttt gctattaata 900
   tgtatagaaa ggaatgaagt ggcttttact ccagaaatat gaaaattagt atcttttaaa 960
   atagcactta tgctatcaca aggcaatact ggaatgaaaa acaaatctta taaggtccta 1020
   aataggtaga aactagcttt tattctagtg ttgaacccag gagaaatagt ggcaaagaag 1080
   tttcactctg tttctgcagt tctttgggac tatttataaa ggccagttag attgtgttac 1140
   tgtgatttaa tttacaaagt tgctgcagtt aatggataca ttctgtgctc tgagaatctg 1200
   aattccattc taatgtgtga tattcactgg ctatgatcag tagtatctga agaaaagatg 1260
   ggctttttct cctttactct ctctacataa ttattaatat tattttaatt aaagtactta 1320
   agacattgtt ttttcttctg taaaaatggc ttagcaattt gcacatctgg gtaggttatg 1380
   tagctagtaa acaactcaat agtaagtagc aattatactt aacccaccaa gtgacagact 1440
   atgcaatgga aaaattttct aattatcgtc acatagctta gacagcaaag gattattttc 1500
   tctggtgacc taccatgtgt gatataactg ctttataaat gacttcctga gcaatttcat 1560
   gttggcaggc agtgtggtgt gtctagtgcc cctctatgag aagactataa tttaatcaga 1620
   aacaaatagt aggcatgggg agcaagttgg aaagtgaggt tattattttt tttccttaaa 1680
   attggtgtta ctgtctttag tggcttgagg ctaaagaaag ctgatacttt atctttgcca 1740
   aataattatt tataagattt atttttaaat aagcccaatc tctcacctgc agttaagaat 1800
   catcattttc tgtgactgac actaaagctc ttccttatca gagcttgttc tcaatctgtg 1860
   atcacagtcc acagtggcaa agacaactct ttgattagga aggaaaccat gtttgggatg 1920
   cacagaggtg ccctgtccat acggttttct tcttacatga aagaattgac ctttctgttg 1980
   acctatacca ttctgttttt ataaagtggg attagaattt gagcatatct agtaaatgct 2040
   gtgtggtcag tccgctgatg cttgcccgcc gtctttgccg tgccttggca tcctaggggt 2100
   gctggtgatg gtcagtgggc atcatcggtc agcagtgcac gtgtaaggca atctcctctc 2160
   ctccaggtta gttcagaagg tcgctatggg ttggtattgc aaaaagatga gtatatgaac 2220
   actaaactta ttaagaggtt tgaaattcat taaaaattag ttttcagttt cttcatcttt 2280
   cttttcagac ttctttaaaa atactgttct taatctggta tatatagaaa tctaatatta 2340
   tggtctttta aatggtcaac agaactctcc agtttaatga ggcaatttag aggattcctt 2400
   gaaagaaaat ttcatcatac tgaacagtcc tcatttgtat ctcttttatt accagtattt 2460
   acattgttcc aaggattttt ttcctgacaa aaaatatatt tctcgggagt ggagagtttt 2520
   tttatatata tatatgaaag aacatagatg tttccccccg cccaatacat taactgaatc 2580
   ttcactggca ttctgtagaa aagttaaaac agtttaataa tgtcttaaca tctttataca 2640
   ttttcctata ttctggagga aaccagaatc atgtttagta gattaaagta acattttaaa 2700
   tactaccttt ccaataattt gtaataaaat accagtgaag gacatgtttg gatatctgtt 2760
   aagcattttt ccctgactaa tgaattagga aagaaaacat aaaacataaa tgaatagtta 2820
   tgtctcttct aataaacagt gtcattaatg ctgcagttgt gggtcaaact tagggagcct 2880
   tatttctaaa tagcaaaaat tctgttttca cttcagccct tatttttttt gttagttttt 2940
   ctctgcactg tgatacagag ctaataagag gaaatgtgac gtttcagaga gagagcaagc 3000
   aaattgggta tgactttttt accctatgtt ccccaatact ggggatgttt ggctactttg 3060
   aatagcagtt tttagtgatc taaagtttag atcaatactc gtataagaga aaaatctaac 3120
   tctcagtgag ctccataggg cagcacagct ttccatcttc tctttcgcct tcttgcgtct 3180
   gcccaccaca gtcccgaaga atgcacacgc acatatgatc tgtacccatc ccgcccgaag 3240
   agtcatagaa tcctcttctt tcacttgttt ctttttcttt cgtttgtttg tttttgtttg 3300
   tttgtttttt aagctgggga ccactgaagt aagctctata tgcacccaaa gttccccttg 3360
   gaaaaatttt caacacgcag tgcagaaggc tccagtgcag ctaaatgcca caccattggt 3420
   tctacctggg ggaatagact gatttgccct aaaatgactc caacctgcat cattaaggta 3480
   cagttggctc cagtggatta gtgctgaaat gatgggaagt ctgacacttg ttgacattgt 3540
   cttcatctgt ttggaagggt tgaatctgtt cctctcaaca gtgatagttc ttttcactgt 3600
   atagtattga gtatgtctca gggattcctt gtggaaatta gggcagtttt taaaataaga 3660
   aaatagtttt aaaagaaact aaaggtgact catcattgaa gagagcggaa gaaagaggaa 3720
   acatttggtt ttgtagccca cagtgtcttg cataggactt tttcctttct cctccaggct 3780
   ctcatctcag cttcagaata cagaatcctt tcccatcacg cacagcttta aaacttttat 3840
   ttaaaaattt ccttccccaa tgagctttca gaatacttat tgtagatttt aagagaaaag 3900
   gtatattaat ttatgctatt aacatcacct cataaattat aagttttata ctaaagctgt 3960
   atcgagtgta atgaattatg ttgcctatgt gtttaatagc taaatatata tgagcttttt 4020
   tttttttttt ttttgacaaa acaaactagt gaagcacctt tttacactgg atctctgccg 4080
   tgtcaaggtg tatagctatc ctttgctacc ttgcagatat ataaaataaa aggatatcct 4140
   ggggcctcaa aatgtagaac acctttagac catttataac tgttcctaga actgttgaga 4200
   atcatgttct ttagtaaatg atctgtggtt tacacttaag atggctagaa gcttagttac 4260
   agggtacata gaaatacata gatcaagtaa aactccaact actgtagctg gaatttgtaa 4320
   actaagtttg taagacctct tttatttcct atggagttat tctttaattt acagtttatt 4380
   ctgtaagttg ggaagtaaaa tagagaaaat aataaatcta gatgttctca gtgtcttatt 4440
   caggaacttt ttatccctcc tcactaagga attcccactg tgactttaaa atagaactaa 4500
   attacttgtg tgcatgtata tgtctgtatt ttttacacac atgcaaaaat atacattggg 4560
   ggcacgtgtg tgagagatat gaatgtgtgc ttatataaaa atagataaag gtaaccgaat 4620
   gtattgtaga aacatgattt atttagttga gggtattgga gttactttct cattgtctct 4680
   gttgtataaa tacaccaaat taatcattat gttatcttat gttaaaaggc tagctctgat 4740
   atcatgtgca ttttatgtta acctttggat taacttatat tatgctatgt tcataaaatg 4800
   acatgagaaa caggaggttc taagatcagc ctctcacttt cgagcataaa aataagagag 4860
   ggagacagac agccccagac atgagctgtg tttatctatt gtggtgatgg gaccacagat 4920
   ctattgaatc ttttttatgt taattataga tttctttata ttcgagtaag aaaatattgt 4980
   tgatcatctt ttcttggtga agttaagaaa aagtataggc ctttcataaa caatttttgg 5040
   aaggggctgc tttgtttaag gagttggtgg gatcatttca ccgtgcatat ttcaaagctg 5100
   gggtgatttc atttatttcc aaaatttttc aaaaaacttt tactcagttc tgctgttatt 5160
   tattaactta agagtgctcc catccccata ttttagctat aggaaaattg tgctacccct 5220
   gattcatatg gaattaaaaa aaaatacatc cctttatttt gagttttaag ttgttatttt 5280
   gctatacatt tattactgga gtatctggtg gtctgaaata gtcaaaagta gagttggtat 5340
   taaatgttcc aatgacattt atttttaata cttaaaaaat catgtacttt gaaatatgtc 5400
   aaagcaactt ctgataatat acctgaattt gtagttgtct cttgagcatc atttacttca 5460
   tcttagatat agtgaagatc taggaaagct ctatatgctg ttcttttcta cagttgtatt 5520
   tttgcagcat ctcctggttt cattcactct tgttttggga tttttttttt agatctgcat 5580
   atttcttgta catatgcatg caaatgaaag aagggagttt gtactggtgc catttctccc 5640
   ttcagttgct ggttaatggg atttgctaga aaaaattctc ccggttgaag ggtgaaaaca 5700
   gacccttatg tgtatatctg tacagagatg tgtatatggg atgtggtggc actttgctga 5760
   atgtgaactt gccttgtcaa tggaaagatt gaaaagtatt atgtttattt atacatttgt 5820
   ataaatctat atatacacgt atgtatatgt gtgtgtatag ataaagctat atacatatat 5880
   ttcccttaaa aatgtgtgtg tataataggt aaacagcctt tgttaagcaa gattaatgtc 5940
   tatggaaagt tctggattat tctgtaagcc agaggaggtg acagtctaga gtacatcatc 6000
   agaacatact aaaatggaag tcctttggat tatagttttg tttatggata ttacacaatg 6060
   aatgcttgtc tgaacagttc ttacttgcca gttccactat tcttcatctt caccaccttc 6120
   tactggtcag tctttcatca cttaaaaaaa aaaaaaaaaa tcacacatca ttgtggtttt 6180
   tttccccctt aattctgtct cttctagcca gaagcatctg gcttaagcat atttcatcaa 6240
   cttctctgtt atttctttta aagatcttta tctctgaaat tttcccagaa gatacaagtt 6300
   ttggtaatat tatcaatagg aattttgaga cttgggcatt catctttgtc tcaaaaacaa 6360
   acagaaagcc aaccttcaaa taaaacaaat ttgaaagttt tagctcaata atttggggac 6420
   attttactta aattacaaaa gaacataatt atgattttat gatcgatatt agtgttaggt 6480
   ttatttccac acttactgtc attaatttgt catttacata tttttatttg ctttgtagtt 6540
   taatcataac ttcaccattt acattttaat ggaaagcata cgaggtcaca tgttagcctc 6600
   ttgaattctg taactaactc atttttctca tgatgaaact gctttgatga attcctgtgt 6660
   gatgaattct gctatatttc cttttgactt ttctcagttt gtgacattct gtattaactt 6720
   tgctgcagct ctctagaaaa cttgcatccc tatatattgt gcctttaaag ctcttatgca 6780
   cacacagaca aaagtgtata tatacatgtc agagcatgtg tacatatgct ttcgtatgta 6840
   ctcacacagt atgtctctag tatatattgt gggagtagat atatagatat tcaaaggcaa 6900
   tgaaatattg ctctccagat atatatgtat ataaatagtg ttgatataac tgtatataca 6960
   catgtatatg tgtgagtttt aaatggcaat cttttacatt tagcaaaatg ctgcccctac 7020
   tggaatattg tcactgcagt ggcagttgta tgtaatgatt taaaatacat tatcaaaagt 7080
   tatttaaaga acatttaaag tctcatctaa agacatccac acactattta tttatcttcc 7140
   ttttcctttt attgttgtgt ttttcttaac tgaaagggag actgtcattt taaaaatgcc 7200
   atctgtccca tgagagtaag aaagagctgg agattcactt gaggagcttt gtgcattttt 7260
   cctctctctg aagtggagat gttccacaga ccagactgtt tatttcactt ataaagcaat 7320
   cctaatattt ccgttagtgt atgtcagcca ccatctccta gatcaactta attcaccaat 7380
   ttgcctcctc ctttcattaa taaataatga ttattgagat cattttgcct gggggaagtt 7440
   aactactccc atgctgcagg gaaatatatc acctaattgt tattttgcct tgttcaaaat 7500
   tcaaatttga aagaaggcag ctctctcatg aagctgtctt aagcacattt tagttaaggg 7560
   ccctcccaaa ctcttttttc tttctttctt tctttctttt tttttttttt tgcgagttcc 7620
   aagttgcatg tgcttgaaat agatttaatt cttattcccc acagtttagg tatttttcat 7680
   tagtacatca atttgacaca ctgaatgcaa gactattaag gaagaacgat taaatattat 7740
   tttattttgc gaagagttga cagcagatta catctcaaga acttgcagag agaggaaggt 7800
   agatggacaa tcctaaattg taagatgtta caaaaaacag tgaagtaaga gtactcctga 7860
   agactaaaat agagaggctg gggtttgagc cattttactg agtagcttag ctggaacctg 7920
   atatcagaag tagcctttaa caaaaagcct cttggcaatt gtatggtact aacaactaga 7980
   gtactgaagt gtaagttgaa accaagttgc agtgggaaat caaaggtgag gtagcttatt 8040
   tgaaaccagc aaatgagaca ggttggacag ttttaaaatc tcttctaaca aagaaactgc 8100
   acggtagcaa ggactagcgg ttctcaaagc ccttcttttt cagtgttctc attcaccttg 8160
   gcacccaagt atgtttaaca ggccatgcat taaaaataaa tacaaaaata taaaagccgc 8220
   ttaaagggaa cttacaaact gacaatctct cctctgtatt tgtgttcata gtggctggga 8280
   gtttaattat atgcacaaaa gttaggagcc acttgtttct gcacagactg taggagcaag 8340
   atgaggagat gggcaggttt tggtaagagc ccccagttct ggtggacagg catacttgtg 8400
   gcattgggtg cggcattgct gggaggacca cgtcttggga ggcgattgac ttttggtttg 8460
   taatttccct ttaaacaaga agagatggct cacattttcc atatatatct caatgaatgt 8520
   actgtattac tgttttaaaa atttgatgaa ataataatga attggtctcc ttttgttatc 8580
   tggtccttgt ttaatttgtt taagggtttt tgtatacaaa agtttacatt tttatgtata 8640
   tttttcttgt gtaaaaactg atgtaatatg tgtatgaaac actgtatgta ttatctgtat 8700
   atagtgtgac aaaatcattt ttctttcttt cttttggatg tattaataaa tcttgctgtg 8760
   aagtaaaaaa aaaaaaaaaa aaaaaaaaaa aagaaaaaaa aaaaaaaaaa a 8811
<210> 39
   <211> 1967
   <212> DNA
   <213> Homo sapiens
<400> 39
   gtggggtcgc gttgccaccc cacgcggact ccccagctgg cgcgcccctc ccatttgcct     60
   gtcctggtca ggcccccacc ccccttccca cctgaccagc catgggggct gcggtgtttt    120
   tcggctgcac tttcgtcgcg ttcggcccgg ccttcgcgct tttcttgatc actgtggctg  180
   gggacccgct tcgcgttatc atcctggtcg caggggcatt tttctggctg gtctccctgc  240
   tcctggcctc tgtggtctgg ttcatcttgg tccatgtgac cgaccggtca gatgcccggc  300
   tccagtacgg cctcctgatt tttggtgctg ctgtctctgt ccttctacag gaggtgttcc  360
   gctttgccta ctacaagctg cttaagaagg cagatgaggg gttagcatcg ctgagtgagg  420
   acggaagatc acccatctcc atccgccaga tggcctatgt ttctggtctc tccttcggta  480
   tcatcagtgg tgtcttctct gttatcaata ttttggctga tgcacttggg ccaggtgtgg  540
   ttgggatcca tggagactca ccctattact tcctgacttc agcctttctg acagcagcca  600
   ttatcctgct ccataccttt tggggagttg tgttctttga tgcctgtgag aggagacggt  660
   actgggcttt gggcctggtg gttgggagtc acctactgac atcgggactg acattcctga  720
   acccctggta tgaggccagc ctgctgccca tctatgcagt cactgtttcc atggggctct  780
   gggccttcat cacagctgga gggtccctcc gaagtattca gcgcagctct tgtgtaagga  840
   ctgactacct ggactgatcg cctgacagat cccacctgcc tgtccactgc ccatgactga  900
   gcccagcccc agcccggggt ccattgccca cattctctgt ctccttctcg tcggtctacc  960
   ccactacctc cagggttttg ctttgtcctt ttgtgaccgt tagtctctaa gctttaccag  1020
   gagcagcctg ggttcagcca gtcagtgact ggtgggtttg aatctgcact tatccccacc   1080
   acctggggac ccccttgttg tgtccaggac tccccctgtg tcagtgctct gctctcaccc   1140
   tgcccaagac tcacctccct tcccctctgc aggccgacgg caggaggaca gtcgggtgat  1200
   ggtgtattct gccctgcgca tcccacccga ggactgaggg aacctagggg ggacccctgg  1260
   gcctggggtg ccctcctgat gtcctcgccc tgtatttctc catctccagt tctggacagt  1320
   gcaggttgcc aagaaaaggg acctagttta gccattgccc tggagatgaa attaatggag  1380
   gctcaaggat agatgagctc tgagtttctc agtactccct caagactgga catcttggtc  1440
   tttttctcag gcctgagggg gaaccatttt tggtgtgata aataccctaa actgcctttt  1500
   tttctttttt gaggtggggg gagggaggag gtatattgga actcttctaa cctccttggg 1560
   ctatattttc tctcctcgag ttgctcctca tggctgggct catttcggtc cctttctcct   1620
   tggtcccaga ccttggggga aaggaaggaa gtgcatgttt gggaactggc attactggaa  1680
   ctaatggttt taacctcctt aaccaccagc atccctcctc tccccaaggt gaagtggagg  1740
   gtgctgtggt gagctggcca ctccagagct gcagtgccac tggaggagtc agactaccat  1800
   gacatcgtag ggaaggaggg gagatttttt tgtagttttt aattggggtg tgggaggggc  1860
   ggggaggttt tctataaact gtatcatttt ctgctgaggg tggagtgtcc catcctttta  1920
   atcaaggtga ttgtgatttt gactaataaa aaagaatttg taaaaaa                1967
<210> 40
   <211> 6991
   <212> DNA
   <213> Homo sapiens
<400> 40
   atgggagctg ccgaagcggc aagaacagcg tttccgtcca tcgagcgcca ggtcaccatc     60
   gactattcag ccttgcaggc gcctctggcg ggcttcactg agatccgctc tttcggtgct    120
   cgactcgccc gtgctgctgc cgccgccgaa ggaggggcaa agctcaagat ggcggacaaa    180
   acgccaggcg gatctcagaa ggccagttca aagacgagat catcagatgt tcattcatct    240
   ggatcttcag atgcacatat ggatgcatct ggaccctcag atagtgatat gccaagtcgg    300
   acacgaccta agagcccaag aaaacataat tataggaatg aaagtgcccg tgaaagcctt    360
   tgtgattctc ctcatcagaa tctctcaaga cctcttctgg aaaacaaact taaagcattc    420
   agtattggaa aaatgagtac agctaagcga actttaagta aaaaggaaca ggaagaatta 480
   aagaaaaagg aggatgaaaa ggcagctgct gagatttatg aggagtttct tgctgctttt 540
   gaaggaagtg atggtaataa agtgaaaaca tttgtgcgag ggggtgttgt taatgcagct 600
   aaagaagaac atgaaacaga tgaaaaaaga ggtaaaatct ataagccatc ttcaagattt 660
   gcagatcaaa aaaatcctcc aaatcagtct tccaatgaaa gaccaccatc tcttcttgtg 720
   atagaaacca aaaaacctcc acttaaaaaa ggagagaaag aaaagaaaaa aagcaatttg 780
   gaactcttca aagaagaatt aaagcaaatt caagaggaac gtgatgagag acataaaaca 840
   aaaggcagat taagtcgatt tgaacctcct cagtcagatt ctgatggtca gcgtcgttct 900
   atggacgcgc cttcaagaag aaatagatca tctggtgttc ttgatgatta cgcacctggc 960
   tcacatgatg taggagatcc aagcactact aatttatacc ttggaaacat taatccacag 1020
   atgaatgaag aaatgctgtg ccaagaattt ggaagatttg gaccgttagc cagtgtgaaa 1080
   atcatgtggc ctagaactga tgaagaaaga gccagagaga gaaattgcgg ctttgtggcc 1140
   tttatgaata gaagagatgc tgaaagagct ttaaaaaatt tgaatggaaa aatgattatg 1200
   tcttttgaaa tgaagttagg ttggggtaaa gctgtaccta ttcctccaca tccaatatac 1260
   attccgcctt ctatgatgga acatacgctt cccccacctc catccggact gccttttaat 1320
   gcgcagccta gagagcggtt aaaaaaccct aatgctccta tgttaccgcc acctaaaaac 1380
   aaagaggatt ttgagaagac tctgtcgcaa gccatagtca aagtggttat cccaacagaa 1440
   aggaatttgc tcgccctgat acatcgaatg atagagtttg ttgtacgtga agggccaatg 1500
   tttgaagcta tgattatgaa cagagaaatc aacaatccta tgttcaggtt cttatttgaa 1560
   aaccagacac cagcccatgt ttactatagg tggaagcttt attctattct gcagggagat 1620
   tctccaacta aatggcggac ggaagatttt cgtatgttca aaaatggatc tttttggagg 1680
   ccaccaccat taaatccgta cttgcatgga atgtcagaag agcaagaaac agaagctttt 1740
   gtagaggaac ctagtaaaaa gggagcactt aaggaagaac agagggataa attggaagaa 1800
   atcttgcggg gattaactcc aaggaaaaat gatattggag atgcaatggt tttctgtctt 1860
   aataatgctg aagctgctga agaaatagtg gattgcatta ctgagtcgtt gtccatctta 1920
   aagacacccc ttcctaaaaa gattgccaga ttatatttgg tttctgatgt tttgtacaac 1980
   tcttcagcca aagttgctaa tgcttcatat tatagaaaat tttttgaaac aaagttatgt 2040
   cagatatttt cagacctcaa tgccacctat cgtacaattc aaggccattt acaatctgaa 2100
   aactttaagc aacgggtaat gacttgcttc agagcatggg aagattgggc aatttatcca 2160
   gaaccatttt tgatcaaact acaaaatatt ttcttaggac ttgtaaatat tattgaagaa 2220
   aaggaaacag aggatgttcc agatgacctt gatggtgccc ccatcgagga agagcttgat 2280
   ggtgcacctc tggaagatgt agatggaatt cctattgatg ctactcccat cgatgatctt 2340
   gatggagtcc ctataaaaag tcttgatgat gatcttgatg gagtgccttt ggatgcaact 2400
   gaagactcaa aaaagaatga gcctatattt aaagttgccc catcaaaatg ggaagctgtg 2460
   gatgaatctg aattggaagc acaggctgtt acaacttcta aatgggaatt atttgaccag 2520
   catgaagaat cagaagaaga agaaaatcaa aatcaagaag aagaaagtga agatgaagaa 2580
   gatactcaaa gttccaaatc tgaagaacat catttgtact ctaatccaat caaagaagaa 2640
   atgactgagt ctaagttctc taagtactct gaaatgagtg aggaaaaacg agccaaactt 2700
   cgtgaaattg agctcaaagt tatgaagttt caggatgaat tggaatctgg gaaaagacct 2760
   aaaaaaccag gccagagttt tcaggagcaa gtagaacact acagagataa acttcttcaa 2820
   cgagagaaag agaaagagtt agaaagagaa cgagaaagag acaagaaaga taaagaaaaa 2880
   ttggaatctc gctccaaaga caagaaggaa aaagatgagt gtactccgac aaggaaggaa 2940
   aggaagaggc gacacagtac atcccccagc ccatctcgca gtagcagtgg tagacgagtg 3000
   aaatccccat caccaaaatc ggagcgatca gagcgttcag aaagatctca taaagagagc 3060
   tcacggtcca ggtcatctca caaagattct cctagagatg ttagcaaaaa agccaaaaga 3120
   tcaccatctg gttcaaggac acctaaaagg tctaggcgat cacggtctag atctcctaaa 3180
   aaatcaggaa agaagtccag atcccagtcc agatctccac acaggtctca taaaaagtca 3240
   aagaaaaaca aacactgacg taaattttta agatgctgtc acttattgga aatgcgattt 3300
   gttttgtgcc tgaacggtct gttttttaaa aaaacaaaaa atcaaatgaa agagcattcc 3360
   tggggttttt tgtttgtttg tgtatgcatg tgtaaactca tgagcaactg catctgtaga 3420
   tctgtcattg ttttatattg tgtaaattac tttcattgtg gctatttctc aagatgaaat 3480
   ttttattgtt ctaatggatt tcatcagaaa tgtgtataat ggatctgctg acagtagtag 3540
   tattttgttt taggatgttg tgacttagca aaaataatac agatgtcttc cccccttttg 3600
   tagctttgac aatttgaatt agatttcaaa taaaatctga acagaaaact ataatgttgt 3660
   ttttttgccc caccggtgat attaagtccc ttaaagtcct actgagtttc acactactgt 3720
   tgtgcttctt atacctgatg cactttataa gccccagtgt tcaagtagct taagttttat 3780
   atttactaag atgactatcc aaattaaggg acctgagact cctatttggt ggtttgctaa 3840
   ccatttgctt ttgataagtt tctcttgggt aatactaata cccagatatc aaagactagg 3900
   tagatatggc atggcgtttt gttagtggaa tgcctggcta aaacattttt ttcacagaag 3960
   caatatgatt tccatacatc caacccatgt tctgagcaac tacttacttt tagggggaaa 4020
   ttaaatatct tttcatttcc tcttctatta tgaaagaagt ttatttgtaa aacaaatttt 4080
   ctaacaaggt ttggccatag aattctcttg tatgatcgtt gaccttttat aatcttctgt 4140
   aggctatctt tcaaacactg gcatcagaat attttttata agtttgtgtt taaacagctt 4200
   agttggtccc cccccactcc caagagactt gggtttagtt atagctttaa gtaaaattta 4260
   aaaataaaat gtttttcagg aaacttcgta tctaatggtt tgtaaattca aggtgcaaaa 4320
   agttgattta aaccatttgc agagttgaac tctattatga aaataaattt gctacggtat 4380
   gaggaagaaa taaaacttgt gtaatgttgg tcataatact gctataaata taataaaggg 4440
   ttatgtagaa ttgaactgac actattattt gtgaatcttg atttcagttt tttatgtagg 4500
   cacttcatac actggtttga tgggtttttt ttttcctccc taaaagagaa agtagaaaac 4560
   tattctaaca atggattatt ttgatttagc ttgcttttta aaaaaatctt ttcaacttgt 4620
   tttacttaat cttgcctagt cacaaaataa gatgtgcacc catggtttgg agagttccta 4680
   tattagctga gcagtgagat acactatttc caaacggtgc acacctacag tagctttgga 4740
   aatgagccaa tcactgtttt acttaatggt tcttatcagc atgcaaatat tgcttgaaag 4800
   ttatttcctt attcactgtt ttgttagtcc attttgttag gaaacattaa ttcctaaaaa 4860
   tttgttcaga ataattaaaa gtgaacattt ggtgctgata ctcaaaaacc tacaaatgta 4920
   gccatttaaa aagtaacatg tttttctccc ctgctcattg cctgggagaa tggaatttta 4980
   tataactacc tttctttgca aaaataacgg tcgtgtcgag ttggtggtga ttttggcatt 5040
   ccatcttgca ctggtttcta gtataggctt agaaataatt ggtcaggtaa taatctttcc 5100
   agtcaagttg caagggatgc ttatttctct tcaaaaaaag acatcctgcg ggattgagta 5160
   gaaaatttta ggtcagtttt gggtgcttat ttgtaatatt tttcctacta cattggagtt 5220
   tagcagttct ttttttctgg atccagatac aagtgtcatg gtttatctta cagtgggtga 5280
   aactgacttt cttttggttg ggtgggtgag gatttcttag gcctgataga atatatattc 5340
   tgtgaagttt gttaatgtac atattagatt gtattggatt tttttttctt gaattgcaaa 5400
   tggtattatt agataggtta tttccagttt tacttcatga caaattacct agagtaaacc 5460
   tacttaatac tcccatggat tctatgaaag tttaatggga tcagaaattg gtgacttata 5520
   agggggaaga tattctacca tatttttata atagcttatt attcatgttt cttgtctgaa 5580
   ggacactcaa gttacagagc aaaatttcta taggttgact agaatgttca taagcatggt 5640
   cttccagttg caggaaagat catgttctat ctgtggacac ttactgtcct ctaccacagc 5700
   tacgtgccag agttgttttc cacagttctt ataaagggca tgacttaggc tctttaccct 5760
   ccaacttaat gtttatacac agggattgtt tactaggtta atgacattta actcccctct 5820
   cttctgtagg tgagagaaaa taagtaagtc ttgatctgtt tcttaccaaa gagagacaga 5880
   cctatgatgg aaaatgatca cgtctctgaa ttttttcttt aacgttatag ttccttatta 5940
   cagatagtaa gcatatggga atttctgagc tataacatgt tgagaagtta gaaattaaaa 6000
   ctaacacaac aaaaggcgct gaatcaaaag atctttgctt ttatttggct cagaatgttt 6060
   ttggcttttc tgctaaagat ggcagaaatt actctacaca gacctgattt ttctttattg 6120
   cagaccattc ttgtgggctt accctgagac ttttatccca attagtgaat cttggaggga 6180
   atacttgctt atttatgact taggtatttc cccccaaact ttaatattct tgagcacttg 6240
   aaaatacttt tgagaaattt taactgtgat taaatttagg tttattagaa atattctgta 6300
   cacatttgcc tccatggtgg cgtaagttct gaaaaattat atgaccgtga caatagttta 6360
   tcatcatcat tattgttatt caaaataagg gtaaataaat ctctgtattg ccaaagtgac   6420
   ttaaactgtt ctgatgacca cacagtgtga tttctttagc agagaaagtt ggttttaaaa   6480
   ataaatagta ccacttttct aagactgtac agtttacaaa taaggttttt ttctttgttg   6540
   ttttcctctt ctattaagtt ttagtgaaaa gcctaattac agaaaattgt gcagatacta   6600
   gtgaagatac tagtataagt ttaaaggaac acgtgactgt aaaatctcac atttacaaag   6660
   tgcttgatct cttcatattt cacacgcatg ttttagaata gattttaggg agtgtttaat   6720
   tcattatcct tttgacttaa aatttttgtt accaacttcc taggacttag ataatatata   6780
   aataagtaca aatcccaggg gaagtgttgt gatgctagac taaaaggtgg gaatgtgctg   6840
   ctgttccgtg agccttgttc cattgttgaa aatttgatgc ctcagtgttt attcagtacc   6900
   acctcatgga gcttcaatgt aaatggatta tatgtataat tggtaatttg tatagttttg   6960
   tagattgtag attaaatgca ctcatcatgt c                                  6991
<210> 41
   <211> 7444
   <212> DNA
   <213> Homo sapiens
<400> 41
   gctggctgcg gaaggggagg gggggggaga aggcgattgg atgcggcggc ggcggcggat 60
   cccggagagc cccggagtga gcggagtagc gagtcggcaa cccggagggg tagaaatatt 120
   tctgtcatgg ctcattcaaa gactaggacc aatgatggaa aaattacata tccgcctggg 180
   gtcaaggaaa tatcagataa aatatctaaa gaggagatgg tgagacgatt aaagatggtt 240
   gtgaaaactt ttatggatat ggaccaggac tctgaagaag aaaaggagct ttatttaaac 300
   ctagctttac atcttgcttc agattttttt ctcaagcatc ctgataaaga tgttcgctta 360
   ctggtagcct gctgccttgc tgatattttc aggatttatg ctcctgaagc tccttacaca 420
   tcccctgata aactaaagga tatatttatg tttataacaa gacagttgaa ggggctagag 480
   gatacaaaga gcccacaatt caataggtat ttttatttac ttgagaacat tgcttgggtc 540
   aagtcatata acatatgctt tgagttagaa gatagcaatg aaattttcac ccagctatac 600
   agaaccttat tttcagttat aaacaatggc cacaatcaga aagtccatat gcacatggta 660
   gaccttatga gctctattat ttgtgaaggt gatacagtgt ctcaggagct tttggatacg 720
   gttttagtaa atctggtacc tgctcataag aatttaaaca agcaagcata tgatttggca 780
   aaggctttac tgaagaggac agctcaagct attgagccat atattaccaa tttttttaat 840
   caggttctga tgcttgggaa aacatctatc agcgatttgt cagagcatgt ctttgactta 900
   attttggagc tctacaatat tgatagtcat ttgctgctct ctgttttacc ccagcttgaa 960
   tttaaattaa agagcaatga taatgaggag cgcctacaag ttgttaaact actggcaaaa 1020
   atgtttgggg caaaggattc agaattggct tctcaaaaca agccactttg gcagtgctac 1080
   ttgggcaggt ttaatgatat ccatgtacca atccgcctgg aatgtgtgaa atttgctagc 1140
   cattgtctca tgaaccatcc tgatttagca aaagacttaa cagagtatct taaagtgagg 1200
   tcacatgacc ctgaggaagc tattagacat gatgttattg tgtcaatagt tacagctgct 1260
   aaaaaggata ttcttctggt caatgatcac ttacttaatt ttgtgagaga gagaacatta 1320
   gacaaacgat ggagagtacg caaagaagcc atgatgggac ttgcccaaat ttataagaaa 1380
   tatgctttac agtcagcagc tggaaaagat gctgcaaaac agatagcatg gatcaaagac 1440
   aaattgctac atatatatta tcaaaatagt attgatgatc gactacttgt tgaacggatc 1500
   tttgctcaat acatggttcc tcacaattta gaaactacag aacggatgaa atgcttatat 1560
   tacttgtatg ccacactgga tttaaatgct gtgaaagcat tgaatgaaat gtggaaatgt 1620
   caaaatctgc tccgacatca agtaaaggat ttgcttgact tgattaagca acccaaaaca 1680
   gatgccagtg tcaaggccat attttcaaaa gtgatggtta ttacaagaaa tttacctgat 1740
   cctggtaagg ctcaggattt catgaagaaa ttcacacagg tgttagaaga tgatgagaaa 1800
   ataagaaagc agttagaagt acttgttagt ccaacatgct cctgcaagca ggctgaaggt 1860
   tgtgtgcgtg aaataactaa gaagttgggc aaccccaaac agcctacaaa tcctttcctg 1920
   gaaatgatca agtttctctt ggagaggata gcacctgtgc acatagatac cgaatctatc 1980
   agtgctctta ttaaacaagt gaacaaatca atagatggaa cagcagatga tgaagatgag 2040
   ggtgttccaa ctgatcaagc catcagagca ggtcttgaac tgcttaaggt actctcattt 2100
   acacatccca tctcatttca ttctgctgaa acatttgaat cattactggc ttgtctgaaa 2160
   atggatgatg aaaaagtagc agaagctgca ctacaaattt tcaaaaacac aggaagcaaa 2220
   attgaagagg attttccaca catcagatca gccttgcttc ctgttttaca tcacaaatct 2280
   aaaaaaggac ccccccgtca agccaaatat gccattcatt gtatccatgc gatattttct 2340
   agtaaagaga cccagtttgc acagatattt gagcctctgc ataagagcct agatccaagc 2400
   aacctggaac atctcataac accattggtt actattggtc atattgctct ccttgcacct 2460
   gatcaatttg ctgctccttt gaaatctttg gtagctactt tcattgtgaa agatcttctc 2520
   atgaatgatc ggcttccagg gaaaaagaca actaaacttt gggttccaga tgaagaagta 2580
   tctcctgaga caatggtcaa aattcaggct attaaaatga tggttcgatg gctacttgga 2640
   atgaaaaata atcacagtaa atcaggaact tctaccttaa gattgctaac aacaatattg 2700
   catagtgatg gagacttgac agaacagggg aaaattagta aaccagatat gtcacgtctg 2760
   agacttgctg ctgggagtgc tattgtgaag ctggcacaag aaccctgtta ccatgaaatc 2820
   atcacattag aacaatatca gctatgtgca ttagctatca acgatgaatg ctatcaagta 2880
   agacaagtgt ttgcccagaa acttcacaaa ggcctttccc gtttacggct tccacttgag 2940
   tatatggcaa tctgtgccct ttgtgcaaaa gatcctgtaa aggagagaag agctcatgct 3000
   aggcaatgtt tggtgaaaaa tataaatgta aggcgggagt atctgaagca gcatgcagct 3060
   gttagtgaaa aattattgtc tcttctacca gagtatgttg ttccatatac aattcacctt 3120
   ttggcacatg acccagatta tgtcaaagta caggatattg aacaacttaa agatgttaaa 3180
   gaatgtcttt ggtttgttct ggaaatatta atggctaaaa atgaaaataa cagtcacgct 3240
   tttatcagaa agatggtaga aaatattaaa caaacaaaag atgcccaagg accagatgat 3300
   gcaaaaatga atgaaaaact gtacactgtg tgtgatgttg ccatgaatat catcatgtca 3360
   aagagtacta catacagttt ggaatctcct aaagacccgg tactaccagc tcgtttcttc 3420
   actcaacctg acaagaattt cagtaacacc aaaaattatc tgcctcctga aatgaaatca 3480
   tttttcactc ctggaaaacc taaaacaacc aatgttctag gagctgttaa caagccactt 3540
   tcatcagcag gcaagcaatc tcagaccaaa tcatcacgaa tggaaactgt aagcaatgca 3600
   agcagcagct caaatccaag ctctcctgga agaataaagg ggaggcttga tagttctgaa 3660
   atggatcaca gtgaaaatga agattacaca atgtcttcac ctttgccggg gaaaaaaagt 3720
   gacaagagag acgactctga tcttgtaagg tctgaattgg agaagcctag aggcaggaaa 3780
   aaaacgcccg tcacagaaca ggaggagaaa ttaggtatgg atgacttgac taagttggta 3840
   caggaacaga aacctaaagg cagtcagcga agtcggaaaa gaggccatac ggcttcagaa 3900
   tctgatgaac agcagtggcc tgaggaaaag aggctcaaag aagatatatt agaaaatgaa 3960
   gatgaacaga atagtccgcc aaaaaagggt aaaagaggcc gaccaccaaa acctcttggt 4020
   ggaggtacac caaaagaaga gccaacaatg aaaacttcta aaaaaggaag caaaaaaaaa 4080
   tctggacctc cagcaccaga ggaggaggaa gaagaagaaa gacaaagtgg aaatacggaa 4140
   cagaagtcca aaagcaaaca gcaccgagtg tcaaggagag cacagcagag agcagaatct 4200
   cctgaatcta gtgcaattga atccacacag tccacaccac agaaaggacg aggaagacca 4260
   tcaaaaacgc catcaccatc acaaccaaaa aaaaatgtcc gtgtaggacg ctccaaacaa 4320
   gcagctacta aggaaaatga ttcaagtgaa gaagtagatg tgtttcaggg tagctctcct 4380
   gtcgatgata ttccacagga agaaacagag gaggaggaag tttctacagt aaatgtacgg 4440
   cggcgaagtg ctaaaaggga acggcgatga acaaatgtaa ttaataactt tctctgtgaa 4500
   agctttggaa aaatcttttt tttttttttt tttttttggt caagcttgag gctgaataaa 4560
   gcctttgatg cacaaaatgg gactgctgaa gagtggacag ttggacctta ctttggtgac 4620
   cccatacatt tgtggtcaca tgctttagcc atacacatgg taacattgac tatggagtct 4680
   tgtgaaagtg taatgtgcga tggctatgta gacataaaga agaaacttgt aaatatcttt 4740
   tttctttttt ttaatgtttc tgatttctga agtgcttgta tagcttttat ctgcggcttt 4800
   aaactgacag tacccgactg tttattggat ctattgattt gaaaagaatt tgttaggata 4860
   gatcttaagc agtaatctgt cagtgtttgt atttgtattc tctgcaattt tactgtgaaa 4920
   aaaaatttgt tttcaacaat tggtgtcatt ttcttgatgt cactatttgt tggagagtta 4980
   aatggtctct tccctttgtg tatcttacct agtgtttact cctgggcacc cttaatcttc 5040
   agaggtgcta aattgtctgc cattacacca gaaggatgcc tctgatagga ggacaaccat 5100
   gcaaattgtg aaatagtcct gaagttcttg gattacttta cacctcagta ttgatttgtc 5160
   ccagaatttt ctggcctttc atggcaatga aaattttaag aagaaagatt taaagtattt 5220
   taattttaaa gagtgtgtta taaaataatg tactgaattc tttatcccat tttatcatcc 5280
   tttcagtttt tattaatcta ctgtatcaat aaaattctgt aatttgaatg agtttttaat 5340
   agtctagaat gttattgtgt atagatattt cctcttgaac gttatgttca gaaaatgcaa 5400
   attacactat aatataaaac ctgatatata cacattagaa atattccagt tctccgtaac 5460
   agtgtaaagt taatcagaaa gaaaaaattt tattgatgca gtgtgtcttt ataaagctgt 5520
   tcttgaaagc caagtgtttt gtattttata gtgagttgca tgtttttgaa aaaaagtgct 5580
   gaaaatggga tgtgctgttt tctgtaaatt catatttagc catagtatat gatagattgc 5640
   cccataacat agtttttcat caagagttta ttattgtact ttattttgga gccaaaaaat 5700
   tgattctggg gggtgggggc agcgtagaag tggtatatcc aagtatatca gctactgtag 5760
   ttgtcacagt cttgtgaact ttgaatgaaa ttccactttg tccagattgg gagaagtgga 5820
   aatttatttg gatttagagc aggtcttttt ttttcttcat tgtaatctgc aaaatgtaga 5880
   aataaattac ttaaggcagt atcctttata cagttgtata aactgtattt tgaaccaaaa 5940
   catataggtt actacttaat gcttacctaa tttcatttta gtattttagg tgctgttatg 6000
   ttttttcatg gttaacacca gaggggaaaa aatcatattc taacttatta aatttatcac 6060
   attgaatagt ggaacatttt catatgatac accattatgt ttaagatata tttccaagat 6120
   tggttataat agatggggca tataataaag aagctactat tttggaaaat gacattttac 6180
   tatttgccaa tgtatattgc aattgatgtg attatttttc ttttaaagat ttgtttgtgt 6240
   ttatgaaaca gccatttatt ttttaaaaag tgttttgaat tgtgtcttaa tctctccata 6300
   ttcaactatt cttcatttac aacaatagtg acaccagtaa ttatgagagg ctgtcatata 6360
   tcaaagcagc tcaggtggga tcaacataca ttggttttga aatgtttcct tagctcggtt 6420
   ctcccaaaca cataatttct gtttcagcag tacaaaggca tgttttaaaa tctataacat 6480
   aaagaataag gaatagcttt gtatttttgt cattgattaa attgcaccag aaatgtttat 6540
   ggaaatatta agaacatttt ataaaacata cgaaaaagtg attgtgaagg atttttattt 6600
   gcatgattat agttaagcaa atttcatttc acatttttgt aatgctgtac agcagttcac 6660
   aaaaaaatgt tcattgtaga ttttgttatg ttcaatgcca atgagtctgt aattttacga 6720
   cctgtctgtt ctttttttgg gtggattacg atgtaaattt ttcttttctt tctttctttt 6780
   tttttttttt tgtgtagaaa aataggtgca ataatgatca aagttttgat gtcttgagtc 6840
   tccatcttag gggattatct tacgtttaag cttaacattt catgtagtaa gatttggaga 6900
   gccacatact ttacagtaaa attaagtgtg tataaaacat taattgctaa caattgttag 6960
   caaactattt cagtgataat gttcattttg aaaatatgta ctgtataaca ttaagaaaat 7020
   atttaactcc ctgtaacaag ttccattatg aaaatcttat tcctcagtga ggttatcttg 7080
   ctgcactctg tagcaaattt gtttaatcta cattataata aaatttcttg ctgcagtgca 7140
   acaggaggct ttttcagtga tcttcactgt atatgtaaat tacaaatgtg gctgtaaaac 7200
   ttattccagg tattaaaggt taaattgctt tctatatctt cttataactt gtaagtctga 7260
   tttttaagat ttccttttgt ccacttgtaa gaatgtcagg aatttaagaa tttgtttaaa 7320
   ttaggcatat ctctgccatc acatagtatt atgtcaccat aatgaacaat tgctatttaa 7380
   atagataacc aattttcaga cacatttttg gatttctgtg aagttgaata aacataaaag 7440
   ctaa 7444
<210> 42
   <211> 1851
   <212> DNA
   <213> Homo sapiens
<400> 42
   ggccggtcag cgtcgctgcc ggtctccggc ggagacggac tctggagttt gggcggcccg      60
   ggcggccact aggtactctg atattccgta ctaaacacgt ctgcaagtca agatgtcgca     120
   cccgtccccc caagccaagc cctccaaccc cagtaaccct cgagtcttct ttgacgtgga     180
   catcggaggg gagcgagttg gtcgaattgt cttagaattg tttgcagata tcgtacccaa     240
   aactgcggaa aattttcgtg cactgtgtac aggagaaaaa ggcattggac acacgactgg     300
   gaaacctctc catttcaaag gatgcccttt tcatcgaatt attaagaaat ttatgattca     360
   gggtggagac ttctcaaatc agaatgggac aggtggagaa agtatttatg gtgaaaaatt     420
   tgaagatgaa aatttccatt acaagcatga tcgggagggt ttactgagca tggcaaatgc     480
   aggccgcaac acaaacggtt ctcagttttt tatcacaaca gttccaactc ctcatttgga     540
   tgggaaacat gtggtgtttg gccaagtaat taaaggaata ggagtggcaa ggatattgga     600
   aaatgtggaa gtgaaaggtg aaaaacctgc taaattgtgc gttattgcag aatgtggaga     660
   attgaaggaa ggagatgacg ggggaatatt cccaaaagat ggctctggcg acagtcatcc     720
   agatttccct gaggatgcgg atatagattt aaaagatgta gataaaattt tattaataac     780
   agaagactta aaaaacattg gaaatacttt tttcaaatcc cagaactggg agatggctat     840
   taaaaaatat gcagaagttt taagatacgt ggacagttca aaggctgtta ttgagacagc     900
   agatagagcc aagctgcaac ctatagcttt aagctgtgta ctgaatattg gtgcttgtaa     960
   actgaagatg tcaaattggc agggagcaat tgacagttgt ttagaggctc ttgaactaga    1020
   cccatcaaat accaaagcat tgtaccgcag agctcaagga tggcaaggat taaaagaata    1080
   tgatcaagca ttggctgatc ttaagaaagc tcaggggata gcaccagaag ataaagctat    1140
   ccaggcagaa ttgctgaaag tcaaacaaaa gataaaggca cagaaagata aagagaaggc    1200
   agtatatgca aaaatgtttg cttagaaagg attcagtttt gcttattgtg tgttgattgt    1260
   ataaatgcaa taagaaaatg taaaggtttt tgtctatgaa tatgatccct aatgtgtttc    1320
   ttttgacacc ttagttcctt actgtttaca gtttaggagt actgataggg gttcatgctt    1380
   aataaacatg tcacaataca gtaagtaaag tggttttgtt tgtttctttg agatggagtc    1440
   ttgctctgtc acccaggctg gagtgcggtg gcgcaatctc ggctcactgc atcctctgcc    1500
   tcccgggttc aagcaattct cctgcctcag cttcccaagt agctgggatt acaggcacgt    1560
   gccaccacgc ccagctaatt tttgtatttt tagtagagat ggggtttcac catattggtc    1620
   acgtcacgtt ggtcttgaac tcctgacctt gtgatccacc ccgccttggc ctcccaaagt    1680
   gctgggatta caggtgtgag ccaccgtgcc cggccaagta aaatgttttt taaaatggtt    1740
   atgtgcatta ttcataaaaa ataatggtgt ccagtctttt taaacttgta aagacacatc    1800
   ttattgaata aagagatgag agcttaagtt tgtaaaaaaa aaaaaaaaaa a              1851
<210> 43
   <211> 600
   <212> DNA
   <213> Homo sapiens
<400> 43
   agtcttggcg gaggtgacca aagccacgta atgtccgtag ttcgctcatc cgtccatgcc      60
   agatggattg tggggaaggt gattgggaca aaaatgcaaa agactgctaa agtgagagtg     120
   accaggcttg ttctggatcc ctatttatta aagtatttta ataagcggaa aacctacttt     180
   gctcacgatg cccttcagca gtgcacagtt ggggatattg tgcttctcag agctttacct     240
   gttccacgag caaagcatgt gaaacatgaa ctggctgaga tcgttttcaa agttggaaaa     300
   gtcatagatc cagtgacagg aaagccctgt gctggaacta cctacctgga gagtccgttg     360
   agttcggaaa ccacccagct aagcaaaaat ctggaagaac tcaatatctc ttcagcacag     420
   tgaagcggga gtggaagaag gatctaaagg gaaaaactga catgtttatg ttatggaaaa     480
   agaaattttt ctaagtttca tcacaaactg tgtccagttt ctctgtggtg tttatgaaat     540
   agctaaaagc aaatgaagta aagggcatac tatggttttt cacaaaaaaa aaaaaaaaaa     600
<210> 44
   <211> 2283
   <212> DNA
   <213> Homo sapiens
<400> 44
   agcggttacc cgcgctgcct ccggtgccgc tggcgaccgc tgagccgctt cccgcgcgtc      60
   gcctagcccg cccctcagct cgccgcagcc ccggctcctc cctcgtccgg cccccggggc     120
   ggggtctgtc agcttgccct cccctctccg cctaagcctg ccctatgcca gccgggtgtc     180
   ctccccacag caccacggct tctcttcctc agcacggcga caggggcttc cccttcgccg     240
   ccgccgccgc cgccggccaa gctccgccgc gcccgcggcc cgcggccgcc atgcagttta     300
   tgttgctttt tagtcgtcag ggaaagcttc gactgcaaaa atggtatgtc ccactatcag     360
   acaaagagaa gaaaaagatc acaagagaac ttgttcagac cgttttagca cggaaaccta     420
   aaatgtgcag cttccttgag tggcgagatc tgaagattgt ttacaaaaga tatgctagtc 480
   tgtatttttg ctgtgctatt gaggatcagg acaatgaact aattaccctg gaaataattc 540
   atcgttatgt ggaattactt gacaagtatt tcggcagtgt ctgtgaacta gatatcatct 600
   ttaattttga gaaggcttat tttattttgg atgagtttct tttgggaggg gaagttcagg 660
   aaacatccaa gaaaaatgtc cttaaagcaa ttgagcaggc tgatctactg caggaggaag 720
   ctgaaacccc acgtagtgtt cttgaagaaa ttggactgac ataactctcc tcccttgttg 780
   atgacttctt gtggcatttc acacactgta gatggtcact cccttcatgt ccatgttagc 840
   tcatggtgta agatgatgtc ttgtcagtat tactgttttg ctaagccgct tcattcatgc 900
   ctacacaatt tttttttaaa agggaacttt agttaattaa gtgataaggg acttaaatat 960
   gaattagaat ggtgcagaaa gagatacctt ttctggatat tttaaagttt aaaggtcagt 1020
   ttctcttaat ctgattatgt gcacatatga aaatggcaca tcatatacat gtaaaatcag 1080
   gcagtataca tttattaatt actgtatttg acaaaggaaa ctcttaaatt ataatgtgaa 1140
   acctggtttt atgaaaccaa agactagtgc agcatttcag catatgtaaa aaaaaaaaaa 1200
   aagggaattg acatgtcaca tatcaaatga atggaaactt tgttgaaact ttaaaaagca 1260
   aatttactcc aaagacttgt attggaaatt acataccttt tttttttttt tttaaaggac 1320
   tacagattat ttttaatgac taaattggag tgatacttct tacactaaaa attatttctt 1380
   aggcattctg aatctgggat gagaaacagg attgtttcac aatagtaagc acataatttt 1440
   taaggccaag gcacatttga ctcctgagat gaattttttg tggtcataat caaatactta 1500
   gttgtttttg atgccccaaa ataaagtgag aatggtaatt tgccaggaat tcttcataac 1560
   agtatcttac aaaaaacgtg ttgctctctt cacagtatta tgtgtaaagt cattgtttaa 1620
   agcacgaatg ttccctctgg ggtacttgtt aaagctaaat ttattttgct tccctccact 1680
   tagaagtgct gcacacttta cagcagcttc ctttctttcc atggcactgc ctagttaaca 1740
   gaagtcttat aaaaatttaa aaagacacat ttcttacaaa aaagagttga atgaggtaaa 1800
   atggcattag atggctctat attttttaaa gctatgtaat tgttcagcgt cacttttcta 1860
   agtacttata catatctaaa catgtcttca tggtttatat tttcacttat atatgctggg 1920
   ctggattaag ctttgttgtg attgtgacca acattcaggc cacgtgagca ctgtcttatc 1980
   acatcgccaa ttagttgtaa taaacgttca acgtacaaac actggagtgt gtttttatct 2040
   ctttccaaaa gtttgtcaaa ctatgcagag ctgctgaagg aagaatttct catttttttt 2100
   tcagtaaaat gttgaaaatt cccctccatt tgaatatggt ggttgttata agcacacaca 2160
   agatacatgg tggaagatct aaggagtctt catgtacttc attaaaaaat aacatggatt 2220
   ccatactgag agcctatcta cacatttatt ttcaatcaat aaatgctgct tttaaaacct 2280
   tga 2283
<210> 45
   <211> 2912
   <212> DNA
   <213> Homo sapiens
<400> 45
   cagttgcttc agcgtcccgg tgtggctgtg ccgttggtcc tgtgcggtca cttagccaag 60
   atgcctgagg aaacccagac ccaagaccaa ccgatggagg aggaggaggt tgagacgttc 120
   gcctttcagg cagaaattgc ccagttgatg tcattgatca tcaatacttt ctactcgaac 180
   aaagagatct ttctgagaga gctcatttca aattcatcag atgcattgga caaaatccgg 240
   tatgaaactt tgacagatcc cagtaaatta gactctggga aagagctgca tattaacctt 300
   ataccgaaca aacaagatcg aactctcact attgtggata ctggaattgg aatgaccaag 360
   gctgacttga tcaataacct tggtactatc gccaagtctg ggaccaaagc gttcatggaa 420
   gctttgcagg ctggtgcaga tatctctatg attggccagt tcggtgttgg tttttattct 480
   gcttatttgg ttgctgagaa agtaactgtg atcaccaaac ataacgatga tgagcagtac 540
   gcttgggagt cctcagcagg gggatcattc acagtgagga cagacacagg tgaacctatg 600
   ggtcgtggaa caaaagttat cctacacctg aaagaagacc aaactgagta cttggaggaa 660
   cgaagaataa aggagattgt gaagaaacat tctcagttta ttggatatcc cattactctt 720
   tttgtggaga aggaacgtga taaagaagta agcgatgatg aggctgaaga aaaggaagac 780
   aaagaagaag aaaaagaaaa agaagagaaa gagtcggaag acaaacctga aattgaagat 840
   gttggttctg atgaggaaga agaaaagaag gatggtgaca agaagaagaa gaagaagatt 900
   aaggaaaagt acatcgatca agaagagctc aacaaaacaa agcccatctg gaccagaaat 960
   cccgacgata ttactaatga ggagtacgga gaattctata agagcttgac caatgactgg 1020
   gaagatcact tggcagtgaa gcatttttca gttgaaggac agttggaatt cagagccctt 1080
   ctatttgtcc cacgacgtgc tccttttgat ctgtttgaaa acagaaagaa aaagaacaat 1140
   atcaaattgt atgtacgcag agttttcatc atggataact gtgaggagct aatccctgaa 1200
   tatctgaact tcattagagg ggtggtagac tcggaggatc tccctctaaa catatcccgt 1260
   gagatgttgc aacaaagcaa aattttgaaa gttatcagga agaatttggt caaaaaatgc 1320
   ttagaactct ttactgaact ggcggaagat aaagagaact acaagaaatt ctatgagcag 1380
   ttctctaaaa acataaagct tggaatacac gaagactctc aaaatcggaa gaagctttca 1440
   gagctgttaa ggtactacac atctgcctct ggtgatgaga tggtttctct caaggactac 1500
   tgcaccagaa tgaaggagaa ccagaaacat atctattata tcacaggtga gaccaaggac 1560
   caggtagcta actcagcctt tgtggaacgt cttcggaaac atggcttaga agtgatctat 1620
   atgattgagc ccattgatga gtactgtgtc caacagctga aggaatttga ggggaagact 1680
   ttagtgtcag tcaccaaaga aggcctggaa cttccagagg atgaagaaga gaaaaagaag 1740
   caggaagaga aaaaaacaaa gtttgagaac ctctgcaaaa tcatgaaaga catattggag 1800
   aaaaaagttg aaaaggtggt tgtgtcaaac cgattggtga catctccatg ctgtattgtc 1860
   acaagcacat atggctggac agcaaacatg gagagaatca tgaaagctca agccctaaga 1920
   gacaactcaa caatgggtta catggcagca aagaaacacc tggagataaa ccctgaccat 1980
   tccattattg agaccttaag gcaaaaggca gaggctgata agaacgacaa gtctgtgaag 2040
   gatctggtca tcttgcttta tgaaactgcg ctcctgtctt ctggcttcag tctggaagat 2100
   ccccagacac atgctaacag gatctacagg atgatcaaac ttggtctggg tattgatgaa 2160
   gatgacccta ctgctgatga taccagtgct gctgtaactg aagaaatgcc accccttgaa 2220
   ggagatgacg acacatcacg catggaagaa gtagactaat ctctggctga gggatgactt 2280
   acctgttcag tactctacaa ttcctctgat aatatatttt caaggatgtt tttctttatt 2340
   tttgttaata ttaaaaagtc tgtatggcat gacaactact ttaaggggaa gataagattt 2400
   ctgtctacta agtgatgctg tgatacctta ggcactaaag cagagctagt aatgcttttt 2460
   gagtttcatg ttggttcttt cacagatggg gtaacgtgca ctgtaagacg tatgtaacat 2520
   gatgttaact ttgtgtggtc taaagtgttt agctgtcaag ccggatgcct aagtagacca 2580
   aatcttgtta ttgaagtgtt ctgagctgta tcttgatgtt tagaaaagta ttcgttacat 2640
   cttgtaggat ctactttttg aacttttcat tccctgtagt tgacaattct gcatgtacta 2700
   gtcctctaga aataggttaa actgaagcaa cttgatggaa ggatctctcc acagggcttg 2760
   ttttccaaag aaaagtattg tttggaggag caaagttaaa agcctaccta agcatatcgt 2820
   aaagctgttc aaatactcga gcccagtctt gtggatggaa atgtagtgct cgagtcacat 2880
   tctgcttaaa gttgtaacaa atacagatga gt 2912
<210> 46
   <211> 1593
   <212> DNA
   <213> Homo sapiens
<400> 46
   ccgccgccgc cgcagcccgg ccgcgccccg ccgccgccgc cgccgccatg ggctgcctcg 60
   ggaacagtaa gaccgaggac cagcgcaacg aggagaaggc gcagcgtgag gccaacaaaa 120
   agatcgagaa gcagctgcag aaggacaagc aggtctaccg ggccacgcac cgcctgctgc 180
   tgctgggtgc tggagaatct ggtaaaagca ccattgtgaa gcagatgagg atcctgcatg 240
   ttaatgggtt taatggagag ggcggcgaag aggacccgca ggctgcaagg agcaacagcg 300
   atggtgagaa ggcaaccaaa gtgcaggaca tcaaaaacaa cctgaaagag gcgattgaaa 360
   ccattgtggc cgccatgagc aacctggtgc cccccgtgga gctggccaac cccgagaacc 420
   agttcagagt ggactacatc ctgagtgtga tgaacgtgcc tgactttgac ttccctcccg 480
   aattctatga gcatgccaag gctctgtggg aggatgaagg agtgcgtgcc tgctacgaac 540
   gctccaacga gtaccagctg attgactgtg cccagtactt cctggacaag atcgacgtga 600
   tcaagcaggc tgactatgtg ccgagcgatc aggacctgct tcgctgccgt gtcctgactt 660
   ctggaatctt tgagaccaag ttccaggtgg acaaagtcaa cttccacatg tttgacgtgg 720
   gtggccagcg cgatgaacgc cgcaagtgga tccagtgctt caacgatgtg actgccatca 780
   tcttcgtggt ggccagcagc agctacaaca tggtcatccg ggaggacaac cagaccaacc 840
   gcctgcagga ggctctgaac ctcttcaaga gcatctggaa caacagatgg ctgcgcacca 900
   tctctgtgat cctgttcctc aacaagcaag atctgctcgc tgagaaagtc cttgctggga 960
   aatcgaagat tgaggactac tttccagaat ttgctcgcta cactactcct gaggatgcta 1020
   ctcccgagcc cggagaggac ccacgcgtga cccgggccaa gtacttcatt cgagatgagt 1080
   ttctgaggat cagcactgcc agtggagatg ggcgtcacta ctgctaccct catttcacct 1140
   gcgctgtgga cactgagaac atccgccgtg tgttcaacga ctgccgtgac atcattcagc 1200
   gcatgcacct tcgtcagtac gagctgctct aagaagggaa cccccaaatt taattaaagc 1260
   cttaagcaca attaattaaa agtgaaacgt aattgtacaa gcagttaatc acccaccata 1320
   gggcatgatt aacaaagcaa cctttccctt cccccgagtg attttgcgaa accccctttt 1380
   cccttcagct tgcttagatg ttccaaattt agaaagctta aggcggccta cagaaaaagg 1440
   aaaaaaggcc acaaaagttc cctctcactt tcagtaaaaa taaataaaac agcagcagca 1500
   aacaaataaa atgaaataaa agaaacaaat gaaataaata ttgtgttgtg cagcattaaa 1560
   aaaaatcaaa ataaaaatta aatgtgagca aag 1593
<210> 47
   <211> 3113
   <212> DNA
   <213> Homo sapiens
<400>, 47
<210> 48
   <211> 2386
   <212> DNA
   <213> Homo sapiens
<400> 48
   ggaggaggaa gcaagcgagg gggctggttc ctgagcttcg caattcctgt gtcgccttct     60
   gggctcccag cctgccgggt cgcatgatcc ctccggccgg agctggtttt tttgccagcc    120
   accgcgaggc cggctgagtt accggcatcc ccgcagccac ctcctctccc gacctgtgat    180
   acaaaagatc ttccgggggc tgcacctgcc tgcctttgcc taaggcggat ttgaatctct    240
   ttctctccct tcagaatctt atcttggctt tggatcttag aagagaatca ctaaccagag    300
   acgagactca gtgagtgagc aggtgttttg gacaatggac tggttgagcc catccctatt    360
   ataaaaatgt ctcagagcaa ccgggagctg gtggttgact ttctctccta caagctttcc    420
   cagaaaggat acagctggag tcagtttagt gatgtggaag agaacaggac tgaggcccca    480
   gaagggactg aatcggagat ggagaccccc agtgccatca atggcaaccc atcctggcac    540
   ctggcagaca gccccgcggt gaatggagcc actggccaca gcagcagttt ggatgcccgg    600
   gaggtgatcc ccatggcagc agtaaagcaa gcgctgaggg aggcaggcga cgagtttgaa    660
   ctgcggtacc ggcgggcatt cagtgacctg acatcccagc tccacatcac cccagggaca    720
   gcatatcaga gctttgaaca ggatactttt gtggaactct atgggaacaa tgcagcagcc    780
   gagagccgaa agggccagga acgcttcaac cgctggttcc tgacgggcat gactgtggcc    840
   ggcgtggttc tgctgggctc actcttcagt cggaaatgac cagacactga ccatccactc    900
   taccctccca cccccttctc tgctccacca catcctccgt ccagccgcca ttgccaccag    960
   gagaaccact acatgcagcc catgcccacc tgcccatcac agggttgggc ccagatctgg   1020
   tcccttgcag ctagttttct agaatttatc acacttctgt gagaccccca cacctcagtt   1080
   cccttggcct cagaattcac aaaatttcca caaaatctgt ccaaaggagg ctggcaggta   1140
   tggaagggtt tgtggctggg ggcaggaggg ccctacctga ttggtgcaac ccttacccct   1200
   tagcctccct gaaaatgttt ttctgccagg gagcttgaaa gttttcagaa cctcttcccc   1260
   agaaaggaga ctagattgcc tttgttttga tgtttgtggc ctcagaattg atcattttcc   1320
   ccccactctc cccacactaa cctgggttcc ctttccttcc atccctaccc cctaagagcc   1380
   atttaggggc cacttttgac tagggattca ggctgcttgg gataaagatg caaggaccag   1440
   gactccctcc tcacctctgg actggctaga gtcctcactc ccagtccaaa tgtcctccag   1500
   aagcctctgg ctagaggcca gccccaccca ggagggaggg ggctatagct acaggaagca   1560
   ccccatgcca aagctagggt ggcccttgca gttcagcacc accctagtcc cttcccctcc   1620
   ctggctccca tgaccatact gagggaccaa ctgggcccaa gacagatgcc ccagagctgt   1680
   ttatggcctc agctgcctca cttcctacaa gagcagcctg tggcatcttt gccttgggct   1740
   gctcctcatg gtgggttcag gggactcagc cctgaggtga aagggagcta tcaggaacag   1800
   ctatgggagc cccagggtct tccctacctc aggcaggaag ggcaggaagg agagcctgct   1860
   gcatggggtg gggtagggct gactagaagg gccagtcctg cctggccagg cagatctgtg   1920
   ccccatgcct gtccagcctg ggcagccagg ctgccaaggc cagagtggcc tggccaggag   1980
   ctcttcaggc ctccctctct cttctgctcc acccttggcc tgtctcatcc ccaggggtcc   2040
   cagccacccc gggctctctg ctgtacatat ttgagactag tttttattcc ttgtgaagat   2100
   gatatactat ttttgttaag cgtgtctgta tttatgtgtg aggagctgct ggcttgcagt   2160
   gcgcgtgcac gtggagagct ggtgcccgga gattggacgg cctgatgctc cctcccctgc   2220
   cctggtccag ggaagctggc cgagggtcct ggctcctgag gggcatctgc cctcccccca   2280
   acccccaccc cacacttgtt ccagctcttt gaaatagtct gtgtgaaggt gaaagtgcag   2340
   ttcagtaata aactgtgttt actcagtgaa aaaaaaaaaa aaaaaa                   2386
<210> 49
   <211> 3462
   <212> DNA
   <213> Homo sapiens
<400> 49
   gtgttttcag tctctccggg ttgcatgtac tgtatgtgga gcagtgtaca gtgaagcgga 60
   ggcagagcgg ctccgcgagc ttctctccac tttcccatag agaaaccctg actggccgct 120
   gagggctagc tacacacacg ccctcacgcc cggcgagccc gcgaggtcac tatcatatga 180
   caaaggcttt gccgcagttc atcttcctcc ctgtgtactt tccatttgcc ttcctggaat 240
   cctgctgcat cacagaagct ggaagttctg atgttccact gaaatcacaa tggaaagtct 300
   tgacttgact ggtcacagta atgaaaggca gtaatagaaa taaggatcat tcagcagaag 360
   gagaaggggt tggaaaacga ccaaaacgaa agtgtcttca gtggcatcca ttgctagcaa 420
   agaaacttct tgatttttca gaagaggaag aagaggaaga cgaagaggag gatattgata 480
   aggttcaact tcttggggcc gatggcctag agcaagatgt tggtgaaact gaagatgatg 540
   aatcaccaga gcagcgagcc cggagaccaa tgaatgcatt tcttttattt tgcaaacgcc 600
   atcgctctct tgtacgtcag gaacacccca ggcttgataa ccgaggtgct accaagatac 660
   tagctgattg gtgggctgtt cttgatccaa aggaaaagca gaaatacaca gacatggcca 720
   aggagtataa ggatgcattt atgaaagcaa atcctggcta caaatggtgt cctaccacaa 780
   acaagcctgt gaaatcccca acacccactg tcaatccacg aaagaaactt tgggccttcc 840
   catctgactc ttcaagagac ttgccaagcc ccaagaaagc aaagactgaa gaaatgcctc 900
   agcttaactt tggaatggct gatcctactc aaatgggagg cctgagtatg ctgctgttag 960
   ctggagaaca tgctcttggc acaccagagg tatcctctgg cacatgcagg cctgatgttt 1020
   cagaatctcc tgaattacgt cagaagtcac cattgtttca gtttgccgag atatcttcaa 1080
   gtacgtccca ctctgatgct tctacaaagc agtgtcaaac atctgccttg tttcagtttg 1140
   cagagatttc ttcaaacact tcgcagttgg gtggtgctga gcctgtaaaa cgctgtggaa 1200
   agtctgcact ctttcaactg gcagagatgt gcctggcatc agaagggatg aaaatggaag 1260
   aatcaaagct aataaaagca aaagaatccg atggtggaag aattaaagaa ttagagaagg 1320
   gaaaggaaga aaaagaaatt aaaatggaga aaacagatga aactaggtta cagaaggaag 1380
   cagaatttga aaaatcggct aaggaaaatt taagagattc taaggaattg agaaattttg 1440
   aggcattgca aatagatgac ataatggcta taaaaatgga agatcccaaa gaaattagaa 1500
   aggaagagtt agaagaagat cacaaatgta gtcattttcc tgatttttct tattctgcca 1560
   gtagcaagat aataattagt gatgttccca gtagaaagga tcatatgtgc catcctcatg 1620
   gaattatgat cattgaggat cccgcagcat taaacaagcc agaaaagcta aaaaagaaaa 1680
   agaagaaaag caaaatggat cgacatggaa atgataaatc cacacccaag aagacttgca 1740
   aaaagaggca gtcttcggaa tctgacattg agagcgtcat atataccatt gaagccgtcg 1800
   caaaaggaga ctggggcata gagaaacttg gagatacccc tcgcaagaag gtccgcacat 1860
   cctcaagtgg caagggaagc attttggatg ccaagccacc aaagaaaaaa gtgaaatcaa 1920
   gagagaagaa aatgtcaaag gagaaatcct cagacaccac caaagagtca agacctccag 1980
   atttcattag tatttctgct agcaagaaca tttctggtga gacaccagag ggtataaaag 2040
   cagaaccatt gacccctatg gaagatgcac taccacccag cctatcagga caggccaagc 2100
   ctgaggacag tgactgtcac agaaaaatag aaacttgtgg ttccaggaaa tccgagaggt 2160
   cttgcaaagg tgctctttat aaaaccctgg tgtctgaggg catgctcacc tctctgcgag 2220
   ctaatgttga cagaggaaaa cgaagctcag gaaaaggaaa ctcctctgat catgaagggt 2280
   gttggaatga agaaagctgg acatttagtc agagtgggac cagtgggagc aagaagttca 2340
   agaagacaaa gccaaaagaa gactgtctcc ttggctccgc aaagctggat gaagaatttg 2400
   aaaaaaaatt caacagcctc cctcaatata gtcctgttac atttgaccgg aaatgtgtac 2460
   ctgtcccaag aaaaaagaag aagactggaa atgtgtcctc agaaccgact aaaaccagca 2520
   aaggaagtgg ggataaatgg tcaaacaagc aactcttctt ggatgccatt caccctacag 2580
   aagccatatt ttcagaagac agaaacacca tggagcctgt tcataaggtt aaaaatatcc 2640
   catccatttt caacactcca gagccaacaa caacgcaaga acctttggtg ggcagccaaa 2700
   agagaaaagc aaggaaaacc aagattacac accttgtcag gacagcagat ggccgggtat 2760
   caccagcagg aggtactttg gatgacaaac caaaggaaca actgcagagg agtctcccta 2820
   aagcaactga gacagactgc aatgacaaat gctcacacaa caccgaggtc ggggagacgc 2880
   ggagcagtac tccagaaatg cctgccgtgt ctgcgttctt tagcctcgct gcgctggctg 2940
   aagtggcagc catggaaaat gtgcacagag gtcagaggtc aactccgctc acccatgatg 3000
   gacagccaaa agaaatgccg caggctcctg tacttatttc ctgcgctgac cagtgaagcg 3060
   ccctttcatt gtaaaacatt gtgctttacc tactacccta gccttgtctt taccgaggga 3120
   tgctagtgag tccaagtggt ggaaaatata gactgcaaac aagtgcttgt tgccccacac 3180
   ggcccagatt cacttgaagc agaagttagc atcctgggcc agtttgttct ctcagaaccc 3240
   agaatctttg agggtaaggt tatctgtctg atactgagca gaaacagaat gatcctggag 3300
   ctttgctttc tattgaaggc ttttgacggt aataggtggt aacttggtaa aaggctgcct 3360
   ttactgtagc tcacccagca tctcttttac caaccagaga gtgtgaaact agtttcatat 3420
   attacctagt tattctttca aaacaaaaca aaaaaaacaa aa 3462
<210> 50
   <211> 3389
   <212> DNA
   <213> Homo sapiens
<400> 50
   atgctgctgc taggggtggt gggagcagcc gtgggacgcg tggccgggag cgggggtgac      60
   agcctgggat tccgggggct tctcttcctt gtcctcctcc tctcctctct attcccagtg     120
   tggccgtggc tgacactaaa gactttgtag ccatcaaccc gagtgcagtt tcgatggaaa     180
   atgaaggttg cacgttttca aaaaatacct aatggtgaaa atgagacaat gattcctgta     240
   ttgacatcaa aaaaagcaag tgaattacca gtcagtgaag ttgcaagcat tctccaagct     300
   gatcttcaga atggtctaaa caaatgtgaa gttagtcata ggcgagcctt tcatggctgg     360
   aatgagtttg atattagtga agatgagcca ctgtggaaga agtatatttc tcagtttaaa     420
   aatcccctta ttatgctgct tctggcttct gcagtcatca gtgttttaat gcatcagttt     480
   gatgatgccg tcagtatcac tgtggcaata cttatcgttg ttacagttgc ctttgttcag     540
   gaatatcgtt cagaaaaatc tcttgaagaa ttgagtaaac ttgtgccacc agaatgccat     600
   tgtgtgcgtg aaggaaaatt ggagcataca cttgcccgag acttggttcc aggtgataca     660
   gtttgccttt ctgttgggga tagagttcct gctgacttac gcttgtttga ggctgtggat     720
   ctttccattg atgagtccag cttgacaggt gagacaacgc cttgttctaa ggtgacagct     780
   cctcagccag ctgcaactaa tggagatctt gcatcgagaa gtaacattgc ctttatggga     840
   acactggtca gatgtggcaa agcaaagggt gttgtcattg gaacaggaga aaattctgaa     900
   tttggggagg tttttaaaat gatgcaagca gaagaggcac caaaaacccc tctgcagaag     960
   agcatggacc tcttaggaaa acaactttcc ttttactcct ttggtataat aggaatcatc    1020
   atgttggttg gctggttact gggaaaagat atcctggaaa tgtttactat tagtgtaagt    1080
   ttggctgtag cagcaattcc tgaaggtctc cccattgtgg tcacagtgac gctagctctt 1140
   ggtgttatga gaatggtgaa gaaaagggcc attgtgaaaa agctgcctat tgttgaaact 1200
   ctgggctgct gtaatgtgat ttgttcagat aaaactggaa cactgacgaa gaatgaaatg 1260
   actgttactc acatatttac ttcagatggt ctgcatgctg aggttactgg agttggctat 1320
   aatcaatttg gggaagtgat tgttgatggt gatgttgttc atggattcta taacccagct 1380
   gttagcagaa ttgttgaggc gggctgtgtg tgcaatgatg ctgtaattag aaacaatact 1440
   ctaatgggga agccaacaga aggggcctta attgctcttg caatgaagat gggtcttgat 1500
   ggacttcaac aagactacat cagaaaagct gaataccctt ttagctctga gcaaaagtgg 1560
   atggctgtta agtgtgtaca ccgaacacag caggacagac cagagatttg ttttatgaaa 1620
   ggtgcttacg aacaagtaat taagtactgt actacatacc agagcaaagg gcagaccttg 1680
   acacttactc agcagcagag agatgtgtac caacaagaga aggcacgcat gggctcagcg 1740
   ggactcagag ttcttgcttt ggcttctggt cctgaactgg gacagctgac atttcttggc 1800
   ttggtgggaa tcattgatcc acctagaact ggtgtgaaag aagctgttac aacactcatt 1860
   gcctcaggag tatcaataaa aatgattact ggagattcac aggagactgc agttgcaatc 1920
   gccagtcgtc tgggattgta ttccaaaact tcccagtcag tctcaggaga agaaatagat 1980
   gcaatggatg ttcagcagct ttcacaaata gtaccaaagg ttgcagtatt ttacagagct 2040
   agcccaaggc acaagatgaa aattattaag tcgctacaga agaacggttc agttgtagcc 2100
   atgacaggag atggagtaaa tgatgcagtt gctctgaagg ctgcagacat tggagttgcg 2160
   atgggccaga ctggtacaga tgtttgcaaa gaggcagcag acatgatcct agtggatgat 2220
   gattttcaaa ccataatgtc tgcaatcgaa gagggtaaag ggatttataa taacattaaa 2280
   aatttcgtta gattccagct gagcacgagt atagcagcat taactttaat ctcattggct 2340
   acattaatga actttcctaa tcctctcaat gccatgcaga ttttgtggat caatattatt 2400
   atggatggac ccccagctca gagccttgga gtagaaccag tggataaaga tgtcattcgt 2460
   aaacctcctc gcaactggaa agacagcatt ttgactaaaa acttgatact taaaatactt 2520
   gtttcatcaa taatcattgt ttgtgggact ttgtttgtct tctggcgtga gctacgagac 2580
   aatgtgatta cacctcgaga cacaacaatg accttcacat gctttgtgtt ttttgacatg 2640
   ttcaatgcac taagttccag atcccagacc aagtctgtgt ttgagattgg actctgcagt 2700
   aatagaatgt tttgctatgc agttcttgga tccatcatgg gacaattact agttatttac 2760
   tttcctccgc ttcagaaggt ttttcagact gagagcctaa gcatactggg tctggctctg 2820
   ggagaggagt ggacagcagc tggttgagat acatccccat ctggagacag gactgccact 2880
   gacagaagat gtgagctgtg tctaagtcca gtcttgtgcc cagccgtgtc tgcgccttca 2940
   ctctttggaa ctctgcatac aacatcttag caccatcttc ctgcagctct tccttaccta 3000
   aataaagaaa cagcccaagg gcagtatttc taaaagcact gtaacagctt ttcattttct 3060
   ccacatatac tacaaattct ataaagaaag aaattaattt aaaaaaacta agatgttttt 3120
   ctcttctggc ttcataaatg ccttgctgta taaattgaaa tattgatact gaactgtctt 3180
   tttaatgatg acctaacttt attcaaccca tcggaattta ctttttccct gaaataagat 3240
   cttttccact ggtctactac ctgaccataa acatgtctgc atttgaattc tctaaaccct 3300
   aaatctgtgt ctatgaaaaa tacaaatgac tattaaatat tattctcttt actgttctct 3360
   ttcaccgaaa aaaaaaaaaa aaaaaaaaa 3389
<210> 51
   <211> 1501
   <212> DNA
   <213> Homo sapiens
<400> 51
   ggggtcggcc gggtgctagg ccggggccga ggccgaggcc ggggcgggat ccagagcggg 60
   agccggcgcg ggatctggga ctcggagcgg gatccggagc gggacccagg agccggcgcg 120
   gggccatggc gaggcgcggg ccagggtggc ggccgcttct gctgctcgtg ctgctggcgg 180
   gcgcggcgca gggcggcctc tacttccgcc ggggacagac ctgctaccgg cctctgcggg 240
   gggacgggct ggctccgctg gggcgcagca catacccccg gcctcatgag tacctgtccc 300
   cagcggatct gcccaagagc tgggactggc gcaatgtgga tggtgtcaac tatgccagca 360
   tcacccggaa ccagcacatc ccccaatact gcggctcctg ctgggcccac gccagcacca 420
   gcgctatggc ggatcggatc aacatcaaga ggaagggagc gtggccctcc accctcctgt 480
   ccgtgcagaa cgtcatcgac tgcggtaacg ctggctcctg tgaagggggt aatgacctgt 540
   ccgtgtggga ctacgcccac cagcacggca tccctgacga gacctgcaac aactaccagg 600
   ccaaggacca ggagtgtgac aagtttaacc aatgtgggac atgcaatgaa ttcaaagagt 660
   gccacgccat ccggaactac accctctgga gggtgggaga ctacggctcc ctctctggga 720
   gggagaagat gatggcagaa atctacgcaa atggtcccat cagctgtgga ataatggcaa 780
   cagaaagact ggctaactac accggaggca tctatgccga ataccaggac accacatata 840
   taaaccatgt cgtttccgtg gctgggtggg gcatcagtga tgggactgag tactggattg 900
   tccggaattc atggggtgaa ccatggggcg agagaggctg gctgaggatc gtgaccagca 960
   cctataagga tgggaagggc gccagataca accttgccat cgaggagcac tgtacatttg 1020
   gggaccccat cgtttaaggc catgtcacta gaagcgcagt ttaagaaaag gcatggtgac 1080
   ccatgaccag aggggatcct atggttatgt gtgccaggct ggctggcagg aactggggtg 1140
   gctatcaata ttggatggcg aggacagcgt ggtactggct gcgagtgttc ctgagagttg 1200
   aaagtgggat gacttatgac acttgcacag catggctctg cctcacaatg atgcagtcag 1260
   ccacctggtg aagaagtgac ctgcaacaca ggaaacgatg ggacctcagt cttcttcagc 1320
   agaggacttg atattttgta tttggcaact gtgggcaata atatggcatt taagaggtga 1380
   aagagttcag acttatcacc attcttatgt cactttagaa tcaagggtgg gggagggagg 1440
   gagggagttg gcagtttcaa atcgcccaag tgatgaataa agtatctggc tctgcacgag 1500
   a 1501
<210> 52
   <211> 2068
   <212> DNA
   <213> Homo sapiens
<400> 52
   agtcatattt cttgtaaagc agctctttgc agtgtgccag gtttgagata gtgtgcacac 60
   aatgtgacta ctcaacaatt aacgtgtttt cctgtgagcg aaacttggtg aggagggtgg 120
   cacatttctc tttccgcaat gtgaattctt acacatatac acacattctc tctctctctc 180
   tctctctcac acacacacac acacacacac acacactccc cacacccttc aatcatatat 240
   ccccttagag atgttcatta catcagccat gcagaaggag aaagaaaacc cttacccacc 300
   tgtagccacc agacatgccc tctataaggc agctatttac aagactatga agacatgctc 360
   tagaaatgaa gggacatgca aaggcgtaga aagaacaaaa agaaaattac accattctat 420
   aactgccagg gctgagaaga acagaatatt agctgacatt tcttaaaatt gtgttctgtg 480
   tactgagtac tttatatgct ttatcttgtt taatccttac atcaacctct tgaggtacag 540
   attatattta ttactgtttt agagatgaag aaactgaggc tcagagatgt tcaatgactt 600
   atttatgggc acacaagcca ttctaaatca ccatggaatg caagttaaaa tattcccacc 660
   agctgggtgt ggtggctcat gcctgtaatc ctagaacttt gggaggccga ggcaaaagga 720
   tcacttcagc ccatggtttc aagaccagcc tgggtaacat ggtgagaccc ccatctctat 780
   ttttgaaaat aataataatt ttaaaaatac tcccaccttc ttctggtttt tttctttttg 840
   agacagagtc ttgcagtgag ctgagatcat gccagggcaa cagaatgaga ctttgtttaa 900
   aaaaaaaaaa gtgacttgat ttaagggaaa aaatgactgg ctatattcag tcagatatgg 960
   caaaaagtct caaggtgtta atgtgaatga ttaaggtctt ggggggggtg tcccctatca 1020
   gactacaggt gtttagaggc acagaaaaag gtgcagttgg gttcttaatg tgaaatgatg 1080
   agaagcacaa ctccagtgtg tctctttgtg tagaatgtca gcagacaccc cctgctagat 1140
   gtgctggatc atgggaaagc atttccattt gttactagat tgttcagaag ttttaattta 1200
   tgatgggtgt ggtggctcat gcctgtagtc ccagcactgt gggaggctga ggcaggagga 1260
   tcatctgagg ccaagagttc aagatcagcc tgggcaacat agtgataccc tatctcttaa 1320
   aaaagaagaa gtttttaaat ttgaaataat aataggtact ggatttatgc aaatgtcttt 1380
   tctgcgtctt ttgagatgag tatcaggttt tttttttccc ttttatcatc tgatgatgaa 1440
   cttaatgttt ccatttgtat taatggaata ctaagtccct ctgtgatttc tgaaccaagc 1500
   tattcctagg cctgagtttt attttgttga cacagaaata aattagaagg ccgagcgtgg 1560
   tggcatgtgc ctgtagtcct agttgctgag gtaagaggat tgcttgagcc caggagttca 1620
   aggctgcagc aagctttgat tgcgccactg cactccagcc ttggcgacag actaagacgc 1680
   tgtctcaaaa aaaaacaaaa acgacaaaaa aaaaacaaaa cagaaaaaat aaactaaggc 1740
   aatgacagtc cctggcaaat gctgggaggg aggcagcagt ggtcagggaa ggtaaccctg 1800
   aagcaggact tgtaaagcaa ataagattgg gaggccaagg tgggtggatc acgaggtcag 1860
   gagttcgaga ccagcctggc caacatagtg aaaccccgtc tttactaaaa atacaaaaaa 1920
   attagccagg tgtggtggtg ggtgcctgta gtcccagcta cttgggaggc tgaggcagga 1980
   gaatctcgaa cccaggaggc ggaggttaca gtcagctgag accgcaccat cgcactccag 2040
   cctgggtgac agagcaagat tccgtctc 2068
<210> 53
   <211> 461
   <212> DNA
   <213> Homo sapiens
<400> 53
   ctcgagtccg ggccgcaagt cccagacgct gcccatggag gcgtccagcg agccgccgct 60
   ggatgctaag tccgatgtca ccaaccagct tgtagatttt cagtggaaac tgggtatggc 120
   tgtgagctca gacacttgca gatctcttaa gtatccttac gttgcagtga tgctaaaagt 180
   ggcagatcat tcaggccaag taaagaccaa gtgctttgaa atgacgattc cacagtttca 240
   gaatttctac agacagttca aggaaattgc tgcagttatt gaaacggtgt gaagacggat 300
   tctttggttg ataaattgct atcattctaa agtcatggac ttcactttcg gcaacaaaac 360
   taaataagga tggaacattt attgaatgaa aaatgcactt ttgtttttcc atttttttaa 420
   ataataaaaa tcagacaaac aggaaaaaaa aaaaaaaaaa a 461
<210> 54
   <211> 456
   <212> DNA
   <213> Homo sapiens
<400> 54
   ttttgttttt cagctcactt caagggtacc tgaagcgaat tggcaccaaa gcagcagctg 60
   tattgccgca gttctagctt caccttcacg atgtttccct tggtcaaaag cgcactaaat 120
   cgtctccaag ttcgaagcat tcagcaaaca atggcaaggc agagccacca gaaacgtaca 180
   cctgattttc atgacaaata cggtaatgct gtattagcta gtggagccac tttctgtatt 240
   gttacatgga catatgtagc aacacaagtc ggaatagaat ggaacctgtc ccctgttggc 300
   agagttaccc caaaggaatg gaggaatcag taatcatccc agctggtgta ataatgaatt 360
   gtttaaaaaa cagctcataa ttgatgccaa attaaagcac tgtgtaccca ttaagatatg 420
   gcattattga agaaataaag tacatttgaa accttc 456
<210> 55
   <211> 5627
   <212> DNA
   <213> Homo sapiens
<400> 55
   ccccagcagc cgccccagcc gcagccgcag ccgcccggct cgtctgccac caccccgggc 60
   ggcggcagcg gcggctcgct cagcgccatg ccgccgccca gccccgactc agagaacggc 120
   ttctaccccg ggctgccgtc gtccatgaac ccggccttct tccctagctt ctcgcccgtg 180
   tcgccgcacg gctgcactgg gctcagcgtt ccgacgagcg gcggcggcgg cggcggcttc 240
   ggcggcccct tctcggctac cgctgtgccc cctccgccgc cgcccgccat gaatatacct 300
   caacagcagc ccccgccgcc cgcggcgccg cagcagccgc agagccggag gtcgcccgtc 360
   agcccgcagc tccagcagca gcaccaggcg gcggccgccg ccttcctgca gcagaggaac 420
   tcctataacc accaccagcc tcttctgaaa cagtctccct ggagcaacca tcagagcagt 480
   ggctggggca ctggaagtat gtcctgggga gcaatgcatg gcagagatca tcgtagaacc 540
   ggaaacatgg gaatcccagg aactatgaat cagatatctc cattgaagaa accgttttct 600
   ggtaatgtca tagcaccacc gaaatttact cgctcaactc catcactgac tccaaaatct 660
   tggattgaag ataatgtgtt cagaacagac aacaatagta atacactctt acccttacag 720
   gtgagatcta gtttgcagtt gccagcttgg ggctcagatt cactccaaga tagttggtgc 780
   actgcagccg gaacatccag aatagaccag gatcgaagta gaatgtatga cagtttgaat 840
   atgcactctt tggaaaattc ccttatcgat attatgagag cagagcatga tcctcttaag 900
   ggtcgattga gctatccaca tccaggaact gacaatctgt tgatgttaaa tggtcgttct 960
   tccctctttc caatagatga tggcttgctt gatgatggtc acagtgatca agttggagtt 1020
   ttaaattcac ccacctgtta ttcagctcac caaaatggag agcgaataga acgcttctct 1080
   cgaaaagttt ttgttggtgg tcttcctcca gatattgatg aagatgaaat aactgctagc 1140
   ttcagaagat ttgggccttt ggtagtagat tggcctcata aagcagaaag caagtcctat 1200
   tttccaccaa aaggctatgc atttcttctc tttcaagaag agagctcagt tcaggcactc 1260
   attgatgctt gtattgaaga agatggaaaa ctctatctgt gtgtttctag ccctactatc 1320
   aaggacaaac cagttcaaat acgtccttgg aatttaagtg atagtgattt tgtaatggat 1380
   ggttctcagc ctttggatcc ccgaaaaaca atttttgttg gaggtgttcc taggccatta 1440
   agggctgtgg aacttgctat gatcatggac cggctgtatg gtggagtttg ttatgcagga 1500
   attgatacag atcctgagct aaaataccca aaaggtgctg ggcgagttgc tttctccaat 1560
   cagcagagct atattgctgc cattagtgct cggtttgttc agcttcagca tggtgatatt 1620
   gataaacgtg tggaggtaaa gccatatgtg ctagatgacc agatgtgtga tgaatgccag 1680
   ggcgcacgct gtggtggaaa atttgctccc tttttttgtg ccaatgtcac ttgcctgcag 1740
   tattactgtg agttttgttg ggcaaatatc cactctcgtg ctggacgtga gttccataag 1800
   ccattggtaa aggaaggtgc tgatcgccca cgtcagatcc acttccgctg gaactaagaa 1860
   tagcaaactg gcctctgttt aacaaggaaa gaaagggtgc atgtggctta ctgtgtctga 1920
   agatactgac atgcagaaga aataagtgca ttcttctgct tttcacccca gctatcaata 1980
   catgcatctt tatcagcagc caaaacacta caagcctctt gtttttcacc aaaaccctac 2040
   atctcaggct tactaatttt tgtgatattt tcatgttcaa ataaaatgtt tttttgtatt 2100
   ttctccaagt tatttttata tgtaaagtta aaattagata tgagaatgtt ttgcgtaggg 2160
   gcaacacagt ctgctgctat atagtggggg aagcgtgcac ttatttctaa acatgggttt 2220
   ttaacttcaa gatctgcccc agtaatttac caaaggtagc aaaataatag tgaagatgga 2280
   atatgtctgc tacaaatgct tatttttatt gttgctattt tcagtgtata cataaactaa 2340
   aaattagggt tgattttttt gctctccatt ttgacttgca agaaataata cctcaagata 2400
   atctgattta ttagctattt ttaaacattt ttaatcacaa gctgtattag ctttgctgct 2460
   atataggtgt tatgtgtaaa tgccacctat taatacggga ctgaaaacgt tagagacaca 2520
   ctgcattgca gataaaatgc aggcagcaca atatggccta aactgccata gttttagaat 2580
   gtgaaaaaaa tgcatgttta cttacctgta tacaccatat gcatgcacta gaattattaa 2640
   ctaacgaggt gaggtattgc aaatgttcaa aaaagctctc ttgaatctag gtagcatgaa 2700
   caaattataa aatttgttta aaaaagcaaa cttgcatgca ttattgtgac ttcaagttta 2760
   aaaaatcgtc ttacatgtgt acaatatgca aattagtttt agattagaga gtgcagccat 2820
   tttgtgatct ggtcagtagt ggaattcgat tttatgcaga ctggatgtaa tatttgtaat 2880
   ccctgtgcaa ttttgtgacg tgcggttcta attcatgtgc agtgatatag tatagataaa 2940
   agaatgagta aaagaaaata caagaattct aaagaaagtt ggttttagcc cctttgatag 3000
   tccatggtta agacatcctt tataaaccaa agatggccag cacactgcta accagtcacc 3060
   aaatgtaaga cccataagaa acccatattt aaaaatctga attttttaga agaatgcaaa 3120
   actttagtaa accctaagta aagtcaaaat ggagaagggg aaatatacag atggctagtt 3180
   gcataaaatt aaattttacc tttataagac aatggtgaaa tctggcttga acttgcttat 3240
   gtgtttaacc tataaatatt ggggtcttct gtctaaactg gggtcactgt tgcatggaac 3300
   attgttctta atagttgaga attgcttttt tgaaaatttt catgaaggaa ttttggtaat 3360
   gactttgctt gcaggttttt tggggtgttt tgagaaagtg gcatggaaac atgcagtagt 3420
   taatgagttt ctcttggtac tgaacactat tagaatatca ttagtgatat tttttctctt 3480
   tagagcattt ttaatgcaac tagcccctat attttaatgt aagagttact ctgcaatcta 3540
   agcaaagcac ccaacaatgg taaatgtttt ttaaaaatgc agaactaaga tttttgactc 3600
   taaagagaga aaattacaag ggtgttgcct tatagcaaac ccttgggaca atccttcatg 3660
   tgagcaaagt gttgatctta atattggttg tctgtggtgt gcttttttgt actgtaaaaa 3720
   tatgtggttc atgtctaact ctgctgtttt attgtggttg tggttcaagt ttttaatgtt 3780
   taaagttgat gctgttttca gaagagcttt ttactaattt atttgtcagt gttccctatt 3840
   tgttacttaa ccatgatcct ccagattttt tggagtattc ttttctaacc ttaaccctgc 3900
   caaaccttga tccattttga catttgttat gcactatttt tatatctctg tgagagattt 3960
   ttccaacagt cagctatttt atggcacact ttttttgact gatgacatct cctttgctat 4020
   acctcaattt ttggaattta gagaagaaat cagtagtttt gcaatgttaa ttatttagat 4080
   atttaatttc gcagattttt aaactttatt ttcataattt ctgcttaatg tttaaaattg 4140
   aagagccttt tcatgtatta aataatgaac acaaattata taattaaaat aattggagat 4200
   gttgaaaatc attttccctt cttaaacaga aataaatatt tggaatgaag gggaatgtac 4260
   tagaacaccc tttttgccac gggtaaaaat aacagaaatg tatggtttgt tttaccttca 4320
   tttctgtaca agtaaagctt attagtctaa tgttttgttc ctttcccacc tcacccctac 4380
   ctcttttgtt ttgttttgtt tttgcccttt atgtactaca ttcttatttt ctaactttta 4440
   aacactgtat tggaggtttt tttttaattt acagatcata tttattttac tatttttgta 4500
   gaaaattatt aattttgatt gtatttttgt attttaaaag cttcttcact tgtgttccct 4560
   aaatattcat attgctgccc aaaagtatga ctgtggagga aaaaaaaata ctttaaaaat 4620
   ccacactttt tgttaagaag gaaacattta gcatttatat atttgtgtat ggaaaacact 4680
   tgatatttta tccctgttgc atctggctgc acagagcctc tcctcaaaga tgctacaaaa 4740
   cttgaatata acacattttg gaaggctgac taacctcgat tctgtgttgt gatgtgcaat 4800
   actgtttcta atgtttgtat aaaaaaaaac agtgtaaacc tttttaatgc aaatttattt 4860
   ttttcattgc atattttgca gattttatcc acagtgtcat tttttactgt cagaaaagat 4920
   accccttttg tcattgcaac tattttttaa atccagaaat ctttgtactg atgtaaatga 4980
   ttgtagttat tttggatagt gttttgctaa caaaaggaga gacttttttc atgcatattt 5040
   ctattttgtt tttttgggtt ttattttatt ttaatagtag taaaatactt ggaataattt 5100
   ttcatattct tgtcattaat attattttgt atttttatgt ggaaatatat aattttatga 5160
   cactaattgc taaagtttat tttatgttga attatttttg gagctgaaat ctttgtaata 5220
   ttaaagcaac tagtttctaa ttcccagttt ctgtatagaa tcgcacaagt ggtttatgga 5280
   gtgtttggat tgtaattata aatggttctt tgatatgcaa attaatattt tcagttgatt 5340
   ttattttata ttcctaatgg ggtgttaaag ccgtttttta tttttttcta aataaaaaga 5400
   gaacccatgc ttttatggac actaggtaaa caccttcagc ttaaattttt cgttaaatat 5460
   tttagtttat tttattgtta tcttccaggt gtctaaatct ccagtctgtc tgttgtactg 5520
   gtaatttaac tctgtaatgg aatagtttgc tgccaactat ttatattaag taatttttaa 5580
   atatttgtaa tattgttgac tgactaataa actattaagt tattggc 5627
<210> 56
   <211> 3969
   <212> DNA
   <213> Homo sapiens
<400> 56
   gaagcccagg ctctccttag ttgactgtgt gttaatcacc cagcaatttc attactcaac 60
   agctctccag agttgcacat tacagctggg gtagaaattg ggtgctgaag gccaggcaga 120
   gcatttggct gtagggaggc cgatcctcct cgggcctgtt accggcgggt ctttgttctt 180
   agacctgggg ttcttggcct cacggattcc aaggaatgga acgttgggcc atgcgtgtga 240
   acgagctcta tgtcgatgac ccagacaagg acagcggtgg caagatcgac gtcagtctga 300
   acatcagttt acccaatctg cactgcgagt tggttgggct tgacattcag gatgagatgg 360
   gcaggcacga agtgggccac atcgacaact ccatgaagat cccgctgaac aatggggcag 420
   gctgccgctt cgaggggcag ttcagcatca acaaggtatg gaagccctgc ctcagccctt 480
   tctacctgct cccctttcct gctgtctccc cgctccctgg aaactggttg tggaggcact 540
   cactcgacct gaccctgaca cagcccccag caagcgaggg ttcgtgtcca gctgcctggc 600
   cgttcctgct gagaatctgg atgggggtcc aggctccctg gggttttaag cccctgatgg 660
   ctggttcagg aaggagctac tcttctctcc agtgaggggg acaatgatga gaagacctga 720
   ggatttgcag cccccagccc tgggttcaag tcccagctct accccttctt ggcccctaca 780
   agtcacttga cccatcttag gctgagggtg tgatggcgat aatagtatca cgataccacc 840
   cacttcacaa agtttgtgtg gggattaaat gagctaatgc agattcattc attcagaaaa 900
   atttttgaat ggcacgttct gtgttccagg gtcggtgata ggctctgggg cagcgttcct 960
   gggctggtgg ggctcccatt ctggtagagg gagacagtct acaaaccaga aagcatcagg 1020
   gatgctaagt gcagtgatga ggaataaagc caaggggagt gagatgaggt gggcttgaaa 1080
   gtaccttgtc cgctcagaag gaccattcaa ggttcactgt tgttttgtcc tcagaaccag 1140
   gagcttcaga tcctaagtca agtgggtgaa cgcagtgccc ttgggagggc cgaggcaccc 1200
   ggtggcagct ggcagggttt tgctcagcac gtgccggcct tcctcgaagc tcggtactgt 1260
   cacagtggag cctctcaaca acgctgtgag gcagcaccat ttgacaggtt aggatgctgg 1320
   ggcccagaga ggttaagtgt cttgcccgag gtcacacagc tatctgcatg tcccacaact 1380
   ccccttccca gccccagcca aactgagcca ctggccactc ctggcttctc cttgtccctc 1440
   ctgcagcctc tgctcagaac gcccttcctc cagaccctga cacctgagct ggggttgcaa 1500
   agtcactggc cacatccagc ccaaagataa attttgtttg tccagtatag catttaactg 1560
   catcagaacc agtatgaaaa gaccaggaat ccagatttct ggcttttaaa agtcagaggc 1620
   tctcactaca ctgggtccgt gttcccgcta tgacaatgac ctggcaccaa tgggcagtgt 1680
   tcccctttag agagggtgtg tgctgtccct tcccacagtc cctggcaggc ggctggaagg 1740
   ccaggcctgg tcatctgtca agcagggtgg acttcttacg tgacagttca gggctccctt 1800
   aagtgctaaa gcagaagctg caaggctttc ttaaggtttc gagtgttgct gggagaaatc 1860
   tgctgcatgt tgtgggttaa agggagtctc tcaccagccc aggccctcag gaggaggaga 1920
   taccaggagg cagggatgct gggggtcgtg gttcactggg ggctctctct gcccatgagc 1980
   tgccacacag cacctttgcc atgccccgta atttggattt tatggtggtt gtgatggaaa 2040
   gccatttgag ggttttgaac agggaggcaa tgtaatcaga tttatgcctt agaactggac 2100
   tatccaatag gttgccacca gccacataag gctatttaaa ttaattcaaa ttaaatgtac 2160
   aattcagtca ctcattctca tcaaccacat ttcaagtgct caaagccacg tgctggctag 2220
   gggccacagc gttagacagt gcagagagaa agcacttcca tcgctgagga aagttctgct 2280
   ggaccgcaca cccttagaag gatggctctg gtggccgggc gcggtggctc aaacctgtaa 2340
   tcccagcact ttgggaggcc gaggtgggtg gatcacgagg tcaggagatc gagaccatcc 2400
   cggctaacat ggtgaaaccc tgcctctact aaaaatacaa aaaaaaacaa aattagccgg 2460
   gcgtggttgc gggcacctgt agtcccagct actcaggagg ctgaggcggg agaatggcat 2520
   gaacccggga ggtggagctt gcagtgagcc aagatcgtac cactgcactc cagtctgggc 2580
   gacagagtga gactccatct caaaacaaac aaaaaaagga tggggctggg ctggagaggg 2640
   tggcaggcag tggttgtggc agtggagctg gggagatgtg gtcggattag ggaggtagaa 2700
   tcaataagac tcagtgaaga atcggatgtg ggggtaaggg cacatgtgga agcaaagaaa 2760
   cctttgacgt ctttgtcttg acaaccgggt ggtcctgttt ctagacatgg aagcttagaa 2820
   aagcctggag tctgtgggaa gtaggtaggg ctgggcactg gtcattccac tctggtttcc 2880
   tttggggttc ccattaggtg tctacaggga gaggtgaaat tggaagttgg aggtgtggag 2940
   agttcaggag agggttctgg accacagatg ttgaggtggg agtcattagt gaatagatga 3000
   tgttggaagt catgggtcct cagagtgggg gctccttaag cctccaggcc agcagcatca 3060
   gcatcacctg ggagattgtt aggaatgcag attctcaggc ccccctaaga cccaccgact 3120
   ctgtgctaga acaagcgccc ctcagagatt ctgatgccac tgaagtttga ggagcattgg 3180
   tttaagcaag attacctacg gagaggctgt agatccgtgt tctaaacctg gggtccacag 3240
   acacccccaa gaagagcgga ttgaatgcaa gagatctatg aagttggatg ggggaaaaat 3300
   tgacatcttt atttttgcta aactcgatct aaagtttagc atttccatct gcgatgaatg 3360
   taggccacaa accacagtag tattagcagt gcctgggacc tcctcaacaa cagaaattgc 3420
   cggtatttat agcacgttac agttgttgca gataatttcc agagactgtt tatatgcacc 3480
   actgttttaa aattacggtg attggccagg tgcagtggct cacacctgta atcccagcac 3540
   tttgggaggc caaagtgggt ggatcacttg aggagttcaa gaccagcctg gtcaacatgt 3600
   caaaaccctg tatctacaaa aaaatacaaa agttaaccaa gcctatgctt gtagtcacag 3660
   ctactcggga ggccgaggtg ggagggtctt ctgagcccag ggaggtagag gcttcagtga 3720
   gctgagatcg caccaccaca ctccagcctg ggtgacagag tgaaaccctt aatcaatcag 3780
   tcaataaaaa ttacagtaat tattagaccc accactaggt catcttattt gatgcatcag 3840
   taaagcagca tattcaaatg tggattttta aatattttaa ttactattta aatatctctt 3900
   tactttgtaa tcctatgcat tttacgcatt aaaacatttt aagcatttaa aaaaaaaaaa 3960
   aaaaaaaaa 3969
<210> 57
   <211> 2390
   <212> DNA
   <213> Homo sapiens
<400> 57
   agagggagac ggacgttgag agaacgagga ggaaggagag aaaatggcgt ccacggatta      60
   cagtacctat agccaagctg cagcgcagca gggctacagt gcttacaccg cccagcccac     120
   tcaaggatat gcacagacca cccaggcata tgggcaacaa agctatggaa cctatggaca     180
   gcccactgat gtcagctata cccaggctca gaccactgca acctatgggc agaccgccta     240
   tgcaacttct tatggacagc ctcccactgg ttatactact ccaactgccc cccaggcata     300
   cagccagcct gtccaggggt atggcactgg tgcttatgat accaccactg ctacagtcac     360
   caccacccag gcctcctatg cagctcagtc tgcatatggc actcagcctg cttatccagc     420
   ctatgggcag cagccagcag ccactgcacc tacaagaccg caggatggaa acaagcccac     480
   tgagactagt caacctcaat ctagcacagg gggttacaac cagcccagcc taggatatgg     540
   acagagtaac tacagttatc cccaggtacc tgggagctac cccatgcagc cagtcactgc     600
   acctccatcc taccctccta ccagctattc ctctacacag ccgactagtt atgatcagag     660
   cagttactct cagcagaaca cctatgggca accgagcagc tatggacagc agagtagcta     720
   tggtcaacaa agcagctatg ggcagcagcc tcccactagt tacccacccc aaactggatc     780
   ctacagccaa gctccaagtc aatatagcca acagagcagc agctacgggc agcagagttc     840
   attccgacag gaccacccca gtagcatggg tgtttatggg caggagtctg gaggattttc     900
   cggaccagga gagaaccgga gcatgagtgg ccctgataac cggggcaggg gaagaggggg     960
   atttgatcgt ggaggcatga gcagaggtgg gcggggagga ggacgcggtg gaatgggcag    1020
   cgctggagag cgaggtggct tcaataagcc tggtggaccc atggatgaag gaccagatct    1080
   tgatctaggc cctcctgtag atccagatga agactctgac aacagtgcaa tttatgtaca    1140
   aggattaaat gacagtgtga ctctagatga tctggcagac ttctttaagc agtgtggggt    1200
   tgttaagatg aacaagagaa ctgggcaacc catgatccac atctacctgg acaaggaaac    1260
   aggaaagccc aaaggcgatg ccacagtgtc ctatgaagac ccacccactg ccaaggctgc    1320
   cgtggaatgg tttgatggga aagattttca agggagcaaa cttaaagtct cccttgctcg    1380
   gaagaagcct ccaatgaaca gtatgcgggg tggtctgcca ccccgtgagg gcagaggcat    1440
   gccaccacca ctccgtggag gtccaggagg cccaggaggt cctgggggac ccatgggtcg    1500
   catgggaggc cgtggaggag atagaggagg cttccctcca agaggacccc ggggttcccg    1560
   agggaacccc tctggaggag gaaacgtcca gcaccgagct ggagactggc agtgtcccaa    1620
   tccgggttgt ggaaaccaga acttcgcctg gagaacagag tgcaaccagt gtaaggcccc    1680
   aaagcctgaa ggcttcctcc cgccaccctt tccgcccccg ggtggtgatc gtggcagagg 1740
   tggccctggt ggcatgcggg gaggaagagg tggcctcatg gatcgtggtg gtcccggtgg 1800
   aatgttcaga ggtggccgtg gtggagacag aggtggcttc cgtggtggcc ggggcatgga 1860
   ccgaggtggc tttggtggag gaagacgagg tggccctggg gggccccctg gacctttgat 1920
   ggaacagatg ggaggaagaa gaggaggacg tggaggacct ggaaaaatgg ataaaggcga 1980
   gcaccgtcag gagcgcagag atcggcccta ctagatgcag agaccccgca gagctgcatt 2040
   gactaccaga tttatttttt aaaccagaaa atgttttaaa tttataattc catatttata 2100
   atgttggcca caacattatg attattcctt gtctgtactt tagtattttt caccatttgt 2160
   gaagaaacat taaaacaagt taaatggtag tgtgcggagt ttttttttct tccttctttt 2220
   aaaaatggtt gtttaagact ttaacaatgg gaaccccttg tgagcatgct cagtatcatt 2280
   gtggagaacc aagagggcct cttaactgta acaatgttca tggttgtgat gttttttttt 2340
   tttttttaaa ataaaattcc aaatgtttaa taaaaaaaaa aaaaaaaaaa 2390
<210> 58
   <211> 1283
   <212> DNA
   <213> Homo sapiens
<400> 58
   ctctctgctc ctcctgttcg acagtcagcc gcatcttctt ttgcgtcgcc agccgagcca      60
   catcgctcag acaccatggg gaaggtgaag gtcggagtca acggatttgg tcgtattggg     120
   cgcctggtca ccagggctgc ttttaactct ggtaaagtgg atattgttgc catcaatgac     180
   cccttcattg acctcaacta catggtttac atgttccaat atgattccac ccatggcaaa     240
   ttccatggca ccgtcaaggc tgagaacggg aagcttgtca tcaatggaaa tcccatcacc     300
   atcttccagg agcgagatcc ctccaaaatc aagtggggcg atgctggcgc tgagtacgtc     360
   gtggagtcca ctggcgtctt caccaccatg gagaaggctg gggctcattt gcagggggga     420
   gccaaaaggg tcatcatctc tgccccctct gctgatgccc ccatgttcgt catgggtgtg     480
   aaccatgaga agtatgacaa cagcctcaag atcatcagca atgcctcctg caccaccaac     540
   tgcttagcac ccctggccaa ggtcatccat gacaactttg gtatcgtgga aggactcatg     600
   accacagtcc atgccatcac tgccacccag aagactgtgg atggcccctc cgggaaactg     660
   tggcgtgatg gccgcggggc tctccagaac atcatccctg cctctactgg cgctgccaag     720
   gctgtgggca aggtcatccc tgagctgaac gggaagctca ctggcatggc cttccgtgtc     780
   cccactgcca acgtgtcagt ggtggacctg acctgccgtc tagaaaaacc tgccaaatat     840
   gatgacatca agaaggtggt gaagcaggcg tcggagggcc ccctcaaggg catcctgggc     900
   tacactgagc accaggtggt ctcctctgac ttcaacagcg acacccactc ctccaccttt     960
   gacgctgggg ctggcattgc cctcaacgac cactttgtca agctcatttc ctggtatgac    1020
   aacgaatttg gctacagcaa cagggtggtg gacctcatgg cccacatggc ctccaaggag    1080
   taagacccct ggaccaccag ccccagcaag agcacaagag gaagagagag accctcactg    1140
   ctggggagtc cctgccacac tcagtccccc accacactga atctcccctc ctcacagttg    1200
   ccatgtagac cccttgaaga ggggaggggc ctagggagcc gcaccttgtc atgtaccatc    1260
   aataaagtac cctgtgctca acc                                            1283
<210> 59
   <211> 6053
   <212> DNA
   <213> Homo sapiens
<400> 59
   gaaattttga gaagtggtgg gatgaagaag atccctgaag agaggagtaa ctgagacaaa 60
   ggcgaggcta gcaatagaga gacaagtgag ggaattagac ctttggtatc atcatgaaaa 120
   ggcctggaag aaacgctggt ttatcctgcg gagtggccgg atgagcggtg acccagatgt 180
   tctggaatac tacaagaacg atcactccaa gaagcctctg cggatcatca acctgaactt 240
   ctgtgagcag gtagatgcag gcctgacctt taacaagaag gagctgcagg atagttttgt 300
   gtttgacatc aagaccagtg aacgcacctt ttacctggtg gctgagacag aagaggacat 360
   gaataagtgg gtccagagca tctgccagat ctgtggcttc aatcaggctg aggagagcac 420
   agactccctg agaaatgttt cctcagccgg tcatggcccc cgctcttctc cagctgagct 480
   cagcagctct agccagcacc ttctccgaga gcgcaagtcc tcagccccat cacactccag 540
   ccagccaact ctgttcacgt ttgaaccccc tgtgtcaaac cacatgcagc ccaccttgtc 600
   caccagcgca cctcaggagt atctctactt gcaccagtgc ataagccgaa gagcagaaaa 660
   tgcaaggagt gccagcttct ctcagggcac cagagcctct tttctcatga ggagtgacac 720
   agctgtacaa aaacttgccc agggcaatgg acactgtgtc aacgggatca gtggtcaagt 780
   ccatggcttc tatagccttc ccaagccgag ccggcacaat acagaattca gagacagtac 840
   ctacgacctc ccccgcagcc tggcctccca tggccacacc aagggcagcc tcacaggctc 900
   cgagacagat aatgaggatg tgtacacctt caagacgccc agcaacaccc tgtgcaggga 960
   gttcggggac ctcctggtag acaatatgga tgttccggcc accccactct cagcctacca 1020
   gatccctagg acattcactc tggacaaaaa ccacaatgcc atgacagtgg ccactcctgg 1080
   ggactcagcc atagctcccc caccccgccc ccccaagcca agtcaggcag aaacacctcg 1140
   atggggcagt cctcagcaga gaccgccaat cagtgaaaat agcagatctg tcgctgccac 1200
   catccccaga cgcaacaccc tccctgcaat ggacaacagc cgacttcacc gagcttcttc 1260
   ctgtgagacc tacgagtacc cacagcgtgg tggagagagt gcaggccggt ctgctgaatc 1320
   catgagtgat ggagttggct ctttcctgcc agggaaaatg attgtgggcc gatcggacag 1380
   caccaattct gaagacaact atgtgcccat gaatccaggt tcttccaccc tgttggccat 1440
   ggaacgagca ggtgataatt cccagagcgt ctacatccca atgagcccag gggcccatca 1500
   ctttgactca cttggctacc catcaacaac ccttcctgtg caccgaggcc ccagcagagg 1560
   aagtgagatt cagccacccc ctgtcaaccg caacctcaaa cctgatcgga aagcaaagcc   1620
   aacaccactt gacctgagga acaacaccgt catcgatgaa ctccccttca agtcacctat   1680
   caccaagtct tggtctaggg ccaaccacac cttcaactcc agctcctccc agtactgccg 1740
   ccccatctcc acccagagca tcaccagcac agactcagga gacagcgaag agaactatgt 1800
   ccctatgcaa aacccagtgt ctgcatctcc cgttcccagt ggcacgaaca gtcctgcccc 1860
   taagaagagc accggcagcg ttgattatct ggccctggac ttccagccga gctccccaag 1920
   cccccaccgc aagccatcta cttcatccgt cacctctgat gagaaggtgg actacgttca 1980
   ggtggacaag gagaagaccc aggccctgca gaacaccatg caggagtgga cagacgtgcg 2040
   gcagtcctca gagccttcca agggtgccaa gctgtgatga gagggccacc gcagagccca 2100
   ggaggcagca tctccagagc tggcccttcc catctcccct ctcccctctc ccgttcttcc 2160
   tcccatccac ctcctctcta ctctgccagt ctcagccttc aaagcacttg acatcaggga 2220
   ccctgaaccc ttcccctggg aggtgagggc ctgatcaagg cacctcctct gcccactcgg 2280
   ggcccagctg tgatttttat cagtaatggc catgcctcca cccaccttag ttaggagcta 2340
   cttccaaaaa gcatccttca gcctcttcct gtcctttaga cctgactctc taccagatgt 2400
   ttggagggaa gggctggggc tctgagccag attccacacc tcacgttcag tcacagccct 2460
   cagctatctt ccctccggcc actgggctac ctctccttca gtcccagaag acaagtctca 2520
   ccaacccagg gagtcaagga ccagcaaacc aaagtggata atggactttt tcattcctgt 2580
   ttttcttggc aggagagaag caaggccact aaaagaggag atggtggaga cggaggctca 2640
   gcagtggtct tgaggggtaa aggacttaga tgcccagatg aagagggaaa gctgacatct 2700
   gcagggaacc cactttgagg ctgaggccat ggcaggacag ctgctgtggg gtgcagaggc 2760
   agaagatgaa ggacaaaagg aagggaaaac tgatggccaa cctagagcag caaggagcag 2820
   ggcttggagc tcgggtggtg gagatgacaa ggacactgtg gggtctgggt ccccagaact 2880
   ctggagctac aggccactct aggcccaagg gctagtcctc ttccccagtc ccctcagagg 2940
   cccccgccag ccccaccttg aaagcagcat acaggggaag gcttggacca agctgggcga 3000
   ccaagcacat ggggcaggaa cacatggtaa aggggtgggg aatatgggag ggagtgtggt 3060
   gtggatgggg gtgatgcagg gactgagggg aaccctggga caggcacagg ctgggcagag 3120
   gcacagggca gtgcagggga ctctgcagtg gggtcgggaa gtgagtttct ttgcagtgag 3180
   cagtgcagtg gaagtcgggc acagaggtag cagacagatg tgaagcagtg gtgaaggcca 3240
   tgtagcaagt ggggaaatac atccaaaggg cctgggagtt ggggggtgcc caacgcaatc 3300
   cttgggggtg cagggtggag cagaaagtga aggagggaca cgtgcaagag tggtgtgcat 3360
   ggtggtgtga catgaggacc gttcctagga tgggacagtg ggtcaggcag gacaaggaga 3420
   aagcagggca gaatgatgcc tagaggacca catcaggcat ggctgacagc ttgtgcccat 3480
   gggctgtggc gtatgtcaga tcgcagggta ggaacgagtc tggcctggtg ccggcccagt 3540
   gtttcctcag ctcatccgcc ctctgttgct ccctagcatt ccaggagcca tcttggactc 3600
   tcctccccag gtttgaaagg ccatcagatt agcagggacg gggtgtaggg catcacccaa 3660
   ggttccttct cttaaactaa gggtggggga tctgaatgtt tttatgttga ctgttcttga 3720
   ctaaattttc aagagtttca gaagcaacag gacagaccag acgtttcatt ctaccctggg 3780
   gcgaacagaa cttcttcctc ccaaacaatg acttcctgcc atgtttgatg gggacagcta 3840
   ccactgtcct ctgcccccat tcccctttca gctcccatga gcatgcatag ttcaccagac 3900
   caatggccta gccattctct aagtcccatc ctggaagaag ttatttcttc aagagctgca 3960
   cctctcctcc tagcattagt ttagatcaac tcaaggagta tttattaatg gctgctgtct 4020
   ccagtttctg gggttaagca ctaaggacac aagaatcaat cagaccttct ccctgaactt 4080
   aagatagcca caatcagaaa aaggacaagg acatgagaca gtggtgatgg ccatcagaca 4140
   gagacttcaa atgctgatgg agggcagagg aagtacttag ggaggttggt gtcagaggca 4200
   ggagtggggg atcagggaag gtggattcta ggaaaaggga gtgcctgagg taggccttag 4260
   aaggggatga gtcagatttt tacagaggag gagggcaggg cttgggtcca gtggaggaag 4320
   aaggaaggag aggcttggaa agccttgtgt cttgggaaaa aaaggccttt gagcatatgg 4380
   gtccagccac tcagaagtgc aggggccatg ccttggtgtt ccaataagtg aatggaagca 4440
   gtggtggtag ctacactggg cagagttggc agggtgctgg ttcactctgc ccagccctga 4500
   atgtgtgcct taaaggcccc ctacaagggg ccccatacga cagagctttt aactggtgcc 4560
   ttccctgtac ccgcagcagc cacaagtggg cccagactat tgcagcctcc cataaacatg 4620
   tgagcatgtt ctgagtgtgc catgatgtga gtggacctgg ctggaatctt cggagagcga 4680
   ctgaggtgtt caaatcgaat ctcccaggag gcttccttcc agccccctat tctggtaact 4740
   accaggaggc ttccttccag ccccctattc tggtaactac caaaatccct cgggtgcaag 4800
   tgtaggggta gagatggaag gatgagaggt gaaattgacc ttttgaaagc aaagctctgg 4860
   ctcacaggcc ccaaactacc agccgtatct agcatatccc caccctccac ccactacctc 4920
   ctccaacaaa ggagtcaact cagttgaaaa aactggtcct ttggcctatc catgggtcaa 4980
   agtccacctc tcctgggggc ctggagagga ctgagcctac ggaaagggga taccttccca 5040
   ctcagcactg cttcacacag gccccctgcc tggggctctc caaggagcct tcttcaccca 5100
   cttccagctc cacttctgca aggttaagtc aagtgagaac gatgagaaat agggagatgg 5160
   tgtctcctta agtccttgat ctgcctgtct gtggaatggg aggttggatt agctgcgctg 5220
   aggtcccatc cacagctggt gctcagctgc ttgaagggga gactccctcc tctgtaactt 5280
   ctttctgggg gattggggtg ggcagtacct atccccagtc ccctcctagc ttgactttag 5340
   tggtttccaa tgtagaagtt aacaaagtat gccccattcc tgtgacaaaa gcacaaccat 5400
   tctgaagtta ctggagcatg ggctcagctc atcctccctc tggccccttc tcccatgggg 5460
   acatctcggc ccagcacccc tatcccattt ccagagttct tccttcccca tctgggcctt 5520
   cataaaatgc aggggaagcc agactggtct caggagcgct aaagcccttc cgtggggggt 5580
   cgtctttctg ggactagccc tgctgtttag gacctgggac cacaatgggg tacctgccga 5640
   gggggtcccc aagagatcca ggctgtcatg tgatttatgg tggcatgtgt tgtgtatttg 5700
   ttggctactt gtgtcttgaa atctagaatt atttcacgca gaattgtcac tgtttgtcag 5760
   gaagagaaaa tgggctagtg gaagcccagt cttgagttct tgtcttgtta ccatttaaaa 5820
   ttgacattta attttcaaat cactgttggt gcctaatcac ttaagttatt aatttattct 5880
   gttgtattct tttttttaaa ttgtaacata tttatccggt gggtgggaca ggagtgtgtt 5940
   caagtgggtc atgtttttgc tgtggtgaca catggtacag gcttggagct tgcaggtccc 6000
   tttctactgt ggtgttggag caggacaata aagtccacta gaaatgcacc cca 6053
<210> 60
   <211> 1393
   <212> DNA
   <213> Homo sapiens
<400> 60
   gcaggcattg tttgttcttg tcttggattt atgcctttaa atttcacctt ttattacaca 60
   gctatagcag gcctttttat gagactaacc tggcctctcc actaaaggat gtgtgacttt 120
   ctggggacag aagagtacag tccctgacat cacacactgc agagatggat aaccaaggag 180
   taatctactc agacctgaat ctgcccccaa acccaaagag gcagcaacga aaacctaaag 240
   gcaataaaag ctccatttta gcaactgaac aggaaataac ctatgcggaa ttaaaccttc 300
   aaaaagcttc tcaggatttt caagggaatg acaaaaccta tcactgcaaa gatttaccat 360
   cagctccaga gaagctcatt gttgggatcc tgggaattat ctgtcttatc ttaatggcct 420
   ctgtggtaac gatagttgtt attccctcta cattaataca gaggcacaac aattcttccc 480
   tgaatacaag aactcagaaa gcacgtcatt gtggccattg tcctgaggag tggattacat 540
   attccaacag ttgttactac attggtaagg aaagaagaac ttgggaagag agtttgctgg 600
   cctgtacttc gaagaactcc agtctgcttt ctatagataa tgaagaagaa atgaaatttc 660
   tgtccatcat ttcaccatcc tcatggattg gtgtgtttcg taacagcagt catcatccat 720
   gggtgacaat gaatggtttg gctttcaaac atgagataaa agactcagat aatgctgaac 780
   ttaactgtgc agtgctacaa gtaaatcgac ttaaatcagc ccagtgtgga tcttcaataa 840
   tatatcattg taagcataag ctttagaggt aaagcgtttg catttgcagt gcatcagata 900
   aattgtatat ttcttaaaat agaaatatat tatgattgca taaatcttaa aatgaattat 960
   gttatttgct ctaataagaa aattctaaat caattattga aacaggatac acacaattac 1020
   taaagtacag acatcctagc atttgtgtcg ggctcatttt gctcaacatg gtatttgtgg 1080
   ttttcagcct ttctaaaagt tgcatgttat gtgagtcagc ttataggaag taccaagaac 1140
   agtcaaaccc atggagacag aaagtagaat agtggttgcc aatgtctgag ggaggttgaa 1200
   ataggagatg acctctaact gatagaacgt tactttgtgt cgtgatgaaa actttctaaa 1260
   tttcagtagt ggtgatggtt gtaactctgc gaatatacta aacatcattg atttttaatc 1320
   attttaagtg catgaaatgt atgctttgta cacgacactt caataaagct atccagaaaa 1380
   aaaaaaaaaa aaa 1393
<210> 61
   <211> 1242
   <212> DNA
   <213> Homo sapiens
<400> 61
   atttcatgtt atacttaata aaacaaaaca tacctgtata cacacacatt cactcacatt      60
   gaagatgcaa gatgaagaaa gatacatgac attgaatgta cagtcaaaga aaaggagttc     120
   tgcccaaaca tctcaactta catttaaaga ttattcagtg acgttgcact ggtataaaat     180
   cttactggga atatctggaa ccgtgaatgg tattctcact ttgactttga tctccttgat     240
   cctgttggtt tctcagggag tattgctaaa atgccaaaaa ggaagttgtt caaatgccac     300
   tcagtatgag gacactggag atctaaaagt gaataatggc acaagaagaa atataagtaa     360
   taaggacctt tgtgcttcga gatctgcaga ccagacagta ctatgccaat cagaatggct     420
   caaataccaa gggaagtgtt attggttctc taatgagatg aaaagctgga gtgacagtta     480
   tgtgtattgt ttggaaagaa aatctcatct actaatcata catgaccaac ttgaaatggc     540
   ttttatacag aaaaacctaa gacaattaaa ctacgtatgg attgggctta actttacctc     600
   cttgaaaatg acatggactt gggtggatgg ttctccaata gattcaaaga tattcttcat     660
   aaagggacca gctaaagaaa acagctgtgc tgccattaag gaaagcaaaa ttttctctga     720
   aacctgcagc agtgttttca aatggatttg tcagtattag agtttgacaa aattcacagt     780
   gaaataatca atgatcacta tttttggcct attagtttct aatattaatc tccaggtgta     840
   agattttaaa gtgcaattaa atgccaaaat ctcttctccc ttctccctcc atcatcgaca     900
   ctggtctagc ctcagagtaa cccctgttaa caaactaaaa tgtacacttc aaaattttta     960
   cgtgatagta taaaccaatg tgacttcatg tgatcatatc caggattttt attcgtcgct    1020
   tattttatgc caaatgtgat caaattatgc ctgtttttct gtatcttgcg ttttaaattc    1080
   ttaataaggt cctaaacaaa atttcttata tttctaatgg ttgaattata atgtgggttt    1140
   atacattttt tacccttttg tcaaagagaa ttaactttgt ttccaggctt ttgctactct   1200
   tcactcagct acaataaaca tcctgaatgt tttcttaaaa aa                        1242
<210> 62
   <211> 4612
   <212> DNA
   <213> Homo sapiens
<400> 62
   gaggcattga ggcagccagc gcaggggctt ctgctgaggg ggcaggcgga gcttgaggaa      60
   accgcagata agtttttttc tctttgaaag atagagatta atacaactac ttaaaaaata 120
   tagtcaatag gttactaaga tattgcttag cgttaagttt ttaacgtaat tttaatagct 180
   taagatttta agagaaaata tgaagactta gaagagtagc atgagaaagg aaaagataaa 240
   aggtttctaa aacatgacgg aggttgagat gaagcttctt catggagtaa aaaatgtatt 300
   taaaagaaaa ttgagagaaa ggactacaga gccccgaatt aataccaata gaagggcaat 360
   gcttttagat taaaatgaag gtgacttaaa cagcttaaag tttagtttaa aagttgtagg 420
   tgattaaaat aatttgaagg cgatctttta aaaagagatt aaaccgaagg tgattaaaag 480
   accttgaaat ccatgacgca gggagaattg cgtcatttaa agcctagtta acgcatttac 540
   taaacgcaga cgaaaatgga aagattaatt gggagtggta ggatgaaaca atttggagaa 600
   gatagaagtt tgaagtggaa aactggaaga cagaagtacg ggaaggcgaa gaaaagaata 660
   gagaagatag ggaaattaga agataaaaac atacttttag aagaaaaaag ataaatttaa 720
   acctgaaaag taggaagcag aagaaaaaag acaagctagg aaacaaaaag ctaagggcaa 780
   aatgtacaaa cttagaagaa aattggaaga tagaaacaag atagaaaatg aaaatattgt 840
   caagagtttc agatagaaaa tgaaaaacaa gctaagacaa gtattggaga agtatagaag 900
   atagaaaaat ataaagccaa aaattggata aaatagcact gaaaaaatga ggaaattatt 960
   ggtaaccaat ttattttaaa agcccatcaa tttaatttct ggtggtgcag aagttagaag 1020
   gtaaagcttg agaagatgag ggtgtttacg tagaccagaa ccaatttaga agaatacttg 1080
   aagctagaag gggaagttgg ttaaaaatca catcaaaaag ctactaaaag gactggtgta 1140
   atttaaaaaa aactaaggca gaaggctttt ggaagagtta gaagaatttg gaaggcctta 1200
   aatatagtag cttagtttga aaaatgtgaa ggactttcgt aacggaagta attcaagatc 1260
   aagagtaatt accaacttaa tgtttttgca ttggactttg agttaagatt attttttaaa 1320
   tcctgaggac tagcattaat tgacagctga cccaggtgct acacagaagt ggattcagtg 1380
   aatctaggaa gacagcagca gacaggattc caggaaccag tgtttgatga agctaggact 1440
   gaggagcaag cgagcaagca gcagttcgtg gtgaagatag gaaaagagtc caggagccag 1500
   tgcgatttgg tgaaggaagc taggaagaag gaaggagcgc taacgatttg gtggtgaagc 1560
   taggaaaaag gattccagga aggagcgagt gcaatttggt gatgaaggta gcaggcggct 1620
   tggcttggca accacacgga ggaggcgagc aggcgttgtg cgtagaggat cctagaccag 1680
   catgccagtg tgccaaggcc acagggaaag cgagtggttg gtaaaaatcc gtgaggtcgg 1740
   caatatgttg tttttctgga acttacttat ggtaaccttt tatttatttt ctaatataat 1800
   gggggagttt cgtactgagg tgtaaaggga tttatatggg gacgtaggcc gatttccggg 1860
   tgttgtaggt ttctcttttt caggcttata ctcatgaatc ttgtctgaag cttttgaggg 1920
   cagactgcca agtcctggag aaatagtaga tggcaagttt gtgggttttt ttttttacac 1980
   gaatttgagg aaaaccaaat gaatttgata gccaaattga gacaatttca gcaaatctgt 2040
   aagcagtttg tatgtttagt tggggtaatg aagtatttca gttttgtgaa tagatgacct 2100
   gtttttactt cctcaccctg aattcgtttt gtaaatgtag agtttggatg tgtaactgag 2160
   gcggggggga gttttcagta tttttttttg tgggggtggg ggcaaaatat gttttcagtt 2220
   ctttttccct taggtctgtc tagaatccta aaggcaaatg actcaaggtg taacagaaaa 2280
   caagaaaatc caatatcagg ataatcagac caccacaggt ttacagttta tagaaactag 2340
   agcagttctc acgttgaggt ctgtggaaga gatgtccatt ggagaaatgg ctggtagtta 2400
   ctcttttttc cccccacccc cttaatcaga ctttaaaagt gcttaacccc ttaaacttgt 2460
   tattttttac ttgaagcatt ttgggatggt cttaacaggg aagagagagg gtgggggaga 2520
   aaatgttttt ttctaagatt ttccacagat gctatagtac tattgacaaa ctgggttaga 2580
   gaaggagtgt accgctgtgc tgttggcacg aacaccttca gggactggag ctgcttttat 2640
   ccttggaaga gtattcccag ttgaagctga aaagtacagc acagtgcagc tttggttcat 2700
   attcagtcat ctcaggagaa cttcagaaga gcttgagtag gccaaatgtt gaagttaagt 2760
   tttccaataa tgtgacttct taaaagtttt attaaagggg aggggcaaat attggcaatt 2820
   agttggcagt ggcctgttac ggttgggatt ggtggggtgg gtttaggtaa ttgtttagtt 2880
   tatgattgca gataaactca tgccagagaa cttaaagtct tagaatggaa aaagtaaaga 2940
   aatatcaact tccaagttgg caagtaactc ccaatgattt agtttttttc cccccagttt 3000
   gaattgggaa gctgggggaa gttaaatatg agccactggg tgtaccagtg cattaatttg 3060
   ggcaaggaaa gtgtcataat ttgatactgt atctgttttc cttcaaagta tagagctttt 3120
   ggggaaggaa agcattgaac tgggggttgg tctggcctac tgggctgaca ttaactacaa 3180
   ttatgggaaa tgcaaaagtt gtttggatat ggtagtgtgt ggttctcttt tggaattttt 3240
   ttcaggtgat ttaataataa tttaaaacta ctatagaaac tgcagagcaa aggaagtggc 3300
   ttaatgatcc tgaagggatt tcttctgatg gtagcttttg tattatcaag taagattcta 3360
   ttttcagttg tgtgtaagca agtttttttt tagtgtagga gaaatacttt tccattgttt 3420
   aactgcaaaa caagatgtta aggtatgctt caaaaatttt gtaaattgtt tattttaaac 3480
   ttatctgttt gtaaattgta actgattaag aattgtgata gttcagcttg aatgtctctt 3540
   agagggtggg cttttgttga tgagggaggg gaaacttttt tttttctata gacttttttc 3600
   agataacatc ttctgagtca taaccagcct ggcagtatga tggcctagat gcagagaaaa 3660
   cagctccttg gtgaattgat aagtaaaggc agaaaagatt atatgtcata cctccattgg 3720
   ggaataagca taaccctgag attcttacta ctgatgagaa cattatctgc atatgccaaa 3780
   aaaattttaa gcaaatgaaa gctaccaatt taaagttacg gaatctacca ttttaaagtt 3840
   aattgcttgt caagctataa ccacaaaaat aatgaattga tgagaaatac aatgaagagg 3900
   caatgtccat ctcaaaatac tgcttttaca aaagcagaat aaaagcgaaa agaaatgaaa 3960
   atgttacact acattaatcc tggaataaaa gaagccgaaa taaatgagag atgagttggg 4020
   atcaagtgga ttgaggaggc tgtgctgtgt gccaatgttt cgtttgcctc agacaggtat 4080
   ctcttcgtta tcagaagagt tgcttcattt catctgggag cagaaaacag caggcagctg 4140
   ttaacagata agtttaactt gcatctgcag tattgcatgt tagggataag tgcttatttt 4200
   taagagctgt ggagttctta aatatcaacc atggcacttt ctcctgaccc cttccctagg 4260
   ggatttcagg attgagaaat ttttccatcg agccttttta aaattgtagg acttgttcct 4320
   gtgggcttca gtgatgggat agtacacttc actcagaggc atttgcatct ttaaataatt 4380
   tcttaaaagc ctctaaagtg atcagtgcct tgatgccaac taaggaaatt tgtttagcat 4440
   tgaatctctg aaggctctat gaaaggaata gcatgatgtg ctgttagaat cagatgttac 4500
   tgctaaaatt tacatgttgt gatgtaaatt gtgtagaaaa ccattaaatc attcgaaata 4560
   ataaactatt tttattagag aatgtaaaaa aaaaaaaaaa aaaaaaaaaa aa 4612
<210> 63
   <211> 620
   <212> DNA
   <213> Homo sapiens
<400> 63
   tttttttttt ttttttatgt cagaagttta ttttattggt aaaatattaa ataatataca 60
   gaattaaaaa ctgcacttac acttctgaga agaaataaat cttggacaaa ttatatgctt 120
   gttcctgaga ttccctaaat ttattccagt agctgtgcac tcagacagta caaaccttta 180
   taatataatc cttttcacaa agtattacaa agtttctgca gcttctttag ttgtacacac 240
   atacagacac acactcacac atgcatatac aacactcata cacatataca cagtaaccac 300
   atacatgcac agaacatgta tgtgtgtgca ttaaaaacaa agaagataca agtcccacat 360
   tttagtctgc agtgataaaa ctatttagcg gaggtctcca aatcagtatg tagtagagtg 420
   acttgtcaca cagctgcaaa gactgtttca gtatttccat agtaaacctc attttctgag 480
   gtatggtatt tgagccttaa aaaaaactac attacactta gcatacctga catacaaggt 540
   atgggatttt aacttgacat atatatttat atatatatat atatatatat gtctttttta 600
   aacactaggt tttatttccc 620
<210> 64
   <211> 8648
   <212> DNA
   <213> Homo sapiens
<400> 64
   cagggaggga cgcccccgcc gacaggagaa ttggttcccg ggcccgcggc gatgcccccc 60
   cggtagctcg ggcccgtggt cgggtgtttg tgagtgtttc tatgtgggag aaggaggagg 120
   aggaggaaga agaagcaacg atttgtcttc tcggctggtc tccccccggc tctacatgtt 180
   ccccgcactg aggagacgga agaggagccg tagccacccc ccctcccggc ccggattata 240
   gtctctcgcc acagcggcct cggcctcccc ttggattcag acgccgattc gcccagtgtt 300
   tgggaaatgg gaagtaatga cagctggcac ctgaactaag tacttttata ggcaacacca 360
   ttccagaaat tcaggatgaa tggggatatg ccccatgtcc ccattactac tcttgcgggg 420
   attgctagtc tcacagacct cctgaaccag ctgcctcttc catctccttt acctgctaca 480
   actacaaaga gccttctctt taatgcacga atagcagaag aggtgaactg ccttttggct 540
   tgtagggatg acaatttggt ttcacagctt gtccatagcc tcaaccaggt atcaacagat 600
   cacatagagt tgaaagataa ccttggcagt gatgacccag aaggtgacat accagtcttg 660
   ttgcaggccg tcctggcaag gagtcctaat gttttcaggg agaaaagcat gcagaacaga 720
   tatgtacaaa gtggaatgat gatgtctcag tataaacttt ctcagaattc catgcacagt 780
   agtcctgcat cttccaatta tcaacaaacc actatctcac atagcccctc cagccggttt 840
   gtgccaccac agacaagctc tgggaacaga tttatgccac agcaaaatag cccagtgcct 900
   agtccatacg ccccacaaag ccctgcagga tacatgccat attcccatcc ttcaagttac 960
   acaacacatc cacagatgca acaagcatcg gtatcaagtc ccattgttgc aggtggtttg 1020
   agaaacatac atgataataa agtttctggt ccgttgtctg gcaattcagc taatcatcat 1080
   gctgataatc ctagacatgg ttcaagtgag gactacctac acatggtgca caggctaagt 1140
   agtgacgatg gagattcttc aacaatgagg aatgctgcat cttttccctt gagatctcca 1200
   cagccagtat gctcccctgc tggaagtgaa ggaactccta aaggctcaag accaccttta 1260
   atcctacaat ctcagtctct accttgttca tcacctcgag atgttccacc agatatcttg 1320
   ctagattctc cagaaagaaa acaaaagaag cagaagaaaa tgaaattagg caaggatgaa 1380
   aaagagcaga gtgagaaagc ggcaatgtat gatataatta gttctccatc caaggactct 1440
   actaaactta cattaagact ttctcgtgta aggtcttcag acatggacca gcaagaggat 1500
   atgatttctg gtgtggaaaa tagcaatgtt tcagaaaatg atattccttt taatgtgcag 1560
   tacccaggac agacttcaaa aacacccatt actccacaag atataaaccg cccactaaat 1620
   gctgctcaat gtttgtcgca gcaagaacaa acagcattcc ttccagcaaa tcaagtgcct 1680
   gttttacaac agaacacttc agttgctgca aaacaacccc agacttctgt ggtacagaat 1740
   caacaacaga tatcacaaca gggacctata tatgatgaag tggaattgga tgcattggct 1800
   gaaattgagc gaatagagag agaatcagct attgaaaggg agcgcttctc aaaagaagtt 1860
   caagataaag ataagccttt gaaaaaaaga aaacaagatt cttacccaca ggaggctggg 1920
   ggtgctacag gaggtaatag accagcttct caggagacgg gttctacggg aaatgggtca 1980
   aggccagcat taatggttag cattgatctt catcaggcag gaagagtgga ctctcaggct 2040
   tctataactc aggattcaga ctccataaaa aagcctgaag aaatcaaaca atgtaatgat 2100
   gcacctgttt ctgttcttca ggaagatatt gttggaagtc ttaaatctac accagaaaac 2160
   catcctgaga cacctaaaaa aaagtctgat cctgagcttt caaagagtga aatgaaacaa 2220
   agtgaaagta gattagcaga atctaaacca aatgaaaacc gattggtgga gacaaaatca 2280
   agtgaaaata agttagaaac taaagttgag acccaaacag aagaacttaa acagaatgag   2340
   agcagaacaa ctgaatgcaa acaaaacgag agcaccatag ttgagcctaa acaaaatgaa   2400
   aatagactgt ctgacacaaa accaaatgac aacaaacaaa ataatggcag atcagaaaca   2460
   acaaaatcaa ggcctgaaac cccaaagcaa aagggtgaaa gccggcctga gactccaaaa   2520
   caaaagagtg atgggcatcc tgaaacccca aaacagaagg gtgatggaag gcctgaaact   2580
   ccaaagcaaa aaggtgagag ccgccctgaa actccaaagc aaaaaaatga agggcgacct   2640
   gaaacaccaa aacacaggca tgacaatagg agggattctg gaaagccatc tacagagaaa   2700
   aaacctgaag tgtctaaaca taaacaagat actaaatctg actcacctcg gttaaaatca   2760
   gaacgagctg aagccttaaa gcagagacct gatgggcgat ctgtttctga gtcactaaga   2820
   cgtgaccatg ataataaaca aaaatcagat gacaggggtg aatcagagcg acatcgaggg   2880
   gatcagtcta gggttcgaag accagaaaca ttgagatcct ctagtagaaa tgaacatggc   2940
   attaaatctg atagttcaaa aactgataaa ctagaacgaa aacacaggca tgaatcaggg   3000
   gactcaaggg aaagaccatc ttctggggaa caaaaatcaa gacctgacag tcctcgtgtt   3060
   aaacaaggag attctaataa atcaagatct gataaacttg gttttaaatc accaactagt   3120
   aaagatgaca aaaggacaga gggtaacaag agtaaagtag acactaataa agcacaccct   3180
   gacaataagg cagaatttcc aagttatttg ttggggggca ggtctggtgc gttgaaaaat   3240
   tttgtcattc cgaaaatcaa gagggataaa gatggcaatg ttactcagga gacaaagaaa   3300
   atggaaatga aaggagagcc gaaagacaaa gtagaaaaaa taggattagt tgaagatcta   3360
   aataaaggag ctaagcctgt agttgtgcta caaaaactgt ctttggatga tgttcagaaa   3420
   cttattaaag atagagagga caaatcaaga agttccctta aacctatcaa gaataaacca   3480
   tcaaagtcaa ataaaggtag tatagatcaa tcagtgttaa aagaattacc ccctgaactc   3540
   ctggcagaaa ttgagtccac catgccactt tgtgaacgtg tgaaaatgaa caaacgcaag   3600
   cgtagcacag ttaatgaaaa gccaaaatat gctgaaatca gttcagatga agataatgat   3660
   agtgatgaag cttttgaatc ctctaggaaa cgacataaaa aagatgatga taaagcttgg   3720
   gaatatgaag agcgtgacag aagaagctct ggggatcata ggagaagtgg ccactctcat   3780
   gaaggaagaa ggagttcagg tggtggtcgt tatcgaaacc gaagtccgtc agattctgac   3840
   atggaagatt attctcctcc tcccagcctt agtgaggttg ctaggaaaat gaagaaaaaa   3900
   gaaaaacaga agaaaaggaa agcatatgaa ccaaaactaa cacctgaaga aatgatggac   3960
   tcttcaactt ttaagagatt cacagcctca atagagaata ttttggataa tttggaagat   4020
   atggatttta ctgcgtttgg tgatgatgat gaaattcctc aggaactgct cttaggaaaa   4080
   catcagctta atgaacttgg cagtgaatct gctaaaataa aagcaatggg tataatggat   4140
   aagctttcaa ctgacaaaac tgtgaaagtc ttaaatatct tggagaagaa tattcaggat   4200
   gggtcaaagc tttccacttt gttaaatcat aataacgata ctgaagaaga agaaaggtta   4260
   tggagagacc ttattatgga gagagttaca aaatcagcgg atgcttgtct tacaactatc 4320
   aacattatga catcccctaa catgccaaaa gctgtgtaca ttgaggatgt aattgaaaga 4380
   gttatacagt acactaaatt tcatttgcag aatacacttt atcctcagta tgatcctgtt 4440
   tacagattag atcctcatgg aggaggctta ttaagttcaa aagcaaaacg ggctaaatgt 4500
   tctacccata agcagagagt aatagtaatg ctttataaca aagtttgtga cattgttagc 4560
   agcttatcag aattgctaga gatacaactt cttacagaca caacaattct tcaggtttca 4620
   tctatgggaa taacaccatt ttttgtggaa aatgtcagtg aactacagtt gtgtgccatt 4680
   aagttagtca ctgcagtatt ctcaagatat gaaaaacata ggcagttaat tttggaagaa 4740
   atttttactt cacttgcaag attaccaacc agcaagagga gtttaaggaa cttcaggtta 4800
   aacagtagtg atatggatgg agaacctatg tatattcaga tggttacagc actggtttta 4860
   caacttattc agtgtgtggt acacttacca tcatcagaga aggactctaa tgcagaagaa 4920
   gattcaaata aaaaaattga ccaggatgtt gtcattacta actcttatga aacagctatg 4980
   cgaacagccc aaaacttcct ctccatcttc cttaaaaaat gtggtagtaa gcaaggtgaa 5040
   gaagattaca gaccactgtt tgaaaatttt gttcaagacc ttctttcaac agtcaataag 5100
   cctgaatggc cagctgctga actactcctt agtttgttag ggagactgtt ggttcatcag 5160
   ttcagtaaca agtcaacaga gatggcttta agagtggcat ctcttgatta ccttggaact 5220
   gttgctgcac ggctaagaaa agatgctgtt acaagcaaaa tggatcaagg atctatagaa 5280
   cgcattttaa aacaggtttc aggaggggaa gatgaaatcc aacaattaca aaaagcattg 5340
   cttgattact tggatgaaaa cactgagact gatccttcac tagtgttttc tcgtaaattc 5400
   tatatagccc agtggtttcg agacacaact ctggaaacag aaaaagcaat gaaatcacaa 5460
   aaagatgaag aatcatctga aggaacacat catgcaaagg aaattgagac aactggccaa 5520
   attatgcatc gagctgaaaa ccgaaaaaag tttcttagaa gcattatcaa aaccacacct 5580
   tctcagttta gcacattaaa gatgaactct gatactgtgg actatgatga tgcttgcttg 5640
   attgttcgat acttggcctc catgaggccg tttgcccaga gctttgatat ttatttgaca 5700
   cagatcctac gagttcttgg tgaaaatgca attgctgttc gaacaaaagc catgaagtgt 5760
   ttgtctgagg ttgttgctgt agaccccagt attctagcaa ggcttgatat gcaacgaggt 5820
   gttcatggac gattgatgga taattcgact agtgtccgag aagcagcagt agaattacta 5880
   ggtcgatttg tcctttgtcg acctcagctt gctgaacagt attatgatat gctgattgaa 5940
   agaatattgg atactggtat cagtgtcagg aaaagagtaa taaagattct cagagacatt 6000
   tgtattgaac aaccaacatt tccaaaaatc acagaaatgt gtgtaaaaat gattcgcaga 6060
   gtcaatgatg aagagggcat taagaaatta gtaaatgaaa cattccagaa actctggttt 6120
   actccaactc cacacaatga caaagaagca atgacaagga aaattttaaa cattaccgat 6180
   gtggttgcag catgcagaga tactggatat gactggtttg agcaactgct tcaaaacttg 6240
   ttgaagtccg aagaggattc ctcatataaa cctgtgaaga aagcttgtac tcaacttgtt 6300
   gataacctag ttgagcacat tcttaaatat gaggaatctc tagctgactc tgacaataaa 6360
   ggtgtgaatt ctggaagatt ggtagcttgc ataaccactt tgttcttatt cagcaaaata 6420
   agaccccagc tcatggttaa acatgcaatg actatgcaac cataccttac cactaaatgt 6480
   agtacgcaaa atgatttcat ggttatctgc aatgttgcaa aaatcctaga gctagttgta 6540
   ccactgatgg agcatccaag tgaaactttt cttgccacta ttgaggaaga tctaatgaag 6600
   ctcatcatca aatatggcat gactgtagtg caacattgtg tgagctgtct tggagctgtt 6660
   gtaaataaag tgacacaaaa ttttaaattt gtgtgggctt gtttcaatag atactatggt 6720
   gccatttcaa aattaaaaag tcaacaccaa gaggacccaa ataacacttc acttctaaca 6780
   aacaaaccag cacttcttag atcccttttc accgttggag cactatgtcg gcattttgat 6840
   tttgatctgg aagattttaa aggcaacagc aaggttaaca taaaagataa agtacttgaa 6900
   ctattgatgt attttacaaa acactcagat gaagaagtac aaacaaaagc tatcattggt 6960
   ctaggatttg cctttattca gcatccaagt ctaatgttcg agcaagaagt gaagaatcta 7020
   tataataata ttttatctga taagaactcc tcagtcaatt taaaaataca agtgttaaaa 7080
   aacctccaga cctacctaca agaagaagat acacgtatgc agcaggcaga tagagactgg 7140
   aagaaagttg caaaacagga agacttaaaa gaaatgggtg atgtttcctc agggatgagt 7200
   agttccatca tgcagcttta tctcaaacag gtgcttgagg cattttttca cacccagtca 7260
   agtgtacgcc actttgccct aaatgtcatt gcattgactc taaatcaagg tcttattcat 7320
   ccagttcagt gtgtgccata tttaattgct atgggcacag acccagaacc tgctatgcgg 7380
   aacaaggctg atcagcaact tgtggaaata gacaaaaaat atgctggatt cattcatatg 7440
   aaagcagtgg ctggtatgaa gatgtcttac caggtacaac aggcaatcaa cacatgccta 7500
   aaagatcctg taaggggttt cagacaagac gagtcctcta gcgctttgtg ttcacacctt 7560
   tactccatga tccgtggaaa ccgccaacac agacgagcct ttcttatttc tttactcaac 7620
   ctctttgatg acacagcaaa aacagacgtg actatgctct tgtatatagc agacaatcta 7680
   gcctgttttc cataccagac acaggaagag ccgttgttta taatgcatca tatagacatt 7740
   acactctcag tttctggtag taacctactg cagtcattca aggagtctat ggtaaaggac 7800
   aaaaggaaag agagaaaatc atcacctagt aaggaaaatg agtcaagcga cagtgaagaa 7860
   gaagtttcca ggcctcggaa gtcacggaaa cgtgtagatt cagattcaga ttcagattca 7920
   gaagacgata taaattcagt gatgaaatgt ttgccagaaa attcagctcc tttaatcgaa 7980
   tttgcaaatg tgtcccaggg tattttatta cttctcatgt taaaacaaca tttgaagaat 8040
   ctttgtggat tttctgatag taaaattcag aagtactctc catctgaatc tgcaaaagta 8100
   tatgataaag cgataaaccg aaaaacagga gttcattttc atccaaaaca aacactggac 8160
   ttcctgcgga gtgacatggc taattccaaa atcacagaag aggtgaaaag gagtatagta 8220
   aaacagtatc tagatttcaa acttctcatg gaacatctgg accctgatga agaagaagaa 8280
   gaaggggagg tttcagctag cacaaatgct cggaacaaag caattacctc actgcttgga 8340
   ggaggcagcc ctaaaaataa tacagcagca gagacagaag atgatgaaag tgatggggag 8400
   gatagaggag gaggcacttc aggggtgagg cggaggagga gtcaacgtat ttcgcagcgt 8460
   attacgtaaa atgattttta tgtgcttata tatgtcagtc tattaaatgt acaccaagta 8520
   atgtaatact taaaagagaa aacattttgt agatagagat tctctactta cccgtttata 8580
   catccttttg tagaaagttt aacataaaag acaataaaaa aacagaaatg agatttatcc 8640
   agcaaaaa 8648
<210> 65
   <211> 706
   <212> DNA
   <213> Homo sapiens
<400> 65
   aggcacctta caacttcaga attcgaggaa gatgatgtga atcttccaat tttatctatt 60
   tgattcaaat ggtatgtttt ttcacatttc tcataatttc cacgtcaact cccatgatga 120
   tcatgcagga aagcagcatg attgcacttc ttgtagtgca taccatcact aaaacatagt 180
   tactgtcttt ctgtggtctt aagaaaaatt cagaaacagc ataccaatgt acatataaga 240
   taaataagct tatgtggtac agtaagtgtt ctgttctgag aaataccttg attcgtatcc 300
   taatgtctga tgttattggt agttctcaaa cttccccaat tctgctttca tctaaattca 360
   tcttatctat ctattgttaa ataatctatc agtccatttt tatctatcat ctatatatct 420
   gtctttatca atcattcgtc tgtaatctat ctgtgccgtt tggcataaga gacagaaggg 480
   catagcagtg gaaactcaaa ggagttacct ccatgtgaat ggacagtggt gatggtgatc 540
   tgtgaggtgg tgatgttata gaaagaaaaa ctagaagaga gcatgcccta actcagggac 600
   cttgtccagc aggcactctg gcttgttata ctgtgctttg aatatgaccc aaatttcaat 660
   agtgctgaca tttttgcctc ctgggccgaa aaaccttcct cctttt 706
<210> 66
   <211> 265
   <212> DNA
   <213> Homo sapiens
<400> 66
   tttttttttt ggttgcagac atttaaataa tctatatttg tagggtgata tcttggagat     60
   tactttacta tcaatatata gaagtaataa cctggtagat agcattacct atgaaaggtc    120
   catcccatct gctattcttg ctgaaagctt ttcatgcacc agtaaactca gtaaatatca    180
   gaagataaag cataagccta ccacgccacg gatttactcc taatgtctcc tcaaagtgca    240
   gcccgatgca acgattacaa atgat                                          265
<210> 67
   <211> 3366
   <212> DNA
   <213> Homo sapiens
<400> 67
   ggttgcgcgt gtgccatgga ctcagccgcc cggtgatatt gacaatagga gagagaaagg 60
   ggcattgact gggacccacc gcgggtagcg aaaggtggct ctggcagcgg cggctccagc 120
   tcctgcggct cctcctcctt attctgtccc cttctcttgc tgccgctgca gatccagtct 180
   tcctccctcc cttccccccc tccccacgtc gtcgccgccg ccgccgggtc cggggcaacg 240
   agctgaggcg ccgcccgcca ggaatgtgag cgaggagcca ccggcggagc cgcaacgggg 300
   tcggtgccga tttgatggga cgggcccgcg ggggaggatc gtgaggccgc cgccgccacc 360
   ggaacgctga ggttcgggtc cggccgtgag gcctagaggc tccgccgccg cggaaccgga 420
   gggaccccgt accggacagc cgtcgcccca ggctccccgc agctgcccgg acctccccct 480
   gcacgtcccg gtcccgccgc ccgcccccgc tgcggccacc tcgcccgtct cccgcccctc 540
   caagccacag atcatctttg gattcttccc cagaagcttc aagtagggat atgtcctcag 600
   caccaactac tcctccatca gtggataaag tagacggatt ttctcggaag tccgtcagaa 660
   aagccagaca gaagaggtcg caaagttcct cacagtttag gtctcaaggc aagcctattg 720
   agttaacacc tctgccgctg ctaaaagacg ttccatcctc agagcagcct gaactgttcc 780
   taaagaaact tcagcagtgc tgtgtcattt ttgacttcat ggacacgcta tctgatctta 840
   aaatgaaaga atacaagcgc tccactctta atgaactggt ggactacatt acaataagca 900
   gaggctgttt gacagagcag acttaccctg aagtagttag aatggtatct tgcaatatat 960
   tcagaactct ccctcctagt gacagcaatg aatttgatcc agaagaagat gaacctaccc 1020
   ttgaggcatc gtggccacac ttacagcttg tatatgaatt tttcatacga tttttggaaa 1080
   gccaagaatt ccaacccagc attgccaaaa aatatataga tcagaaattt gtattacagc 1140
   ttctggagct atttgacagc gaagaccctc gggaacggga ctacttaaaa acagtcttac 1200
   acagaattta tggcaagttt cttggtctta gagcatttat ccgaaaacag attaacaata 1260
   tttttctaag gtttgtttat gaaacagaac acttcaatgg tgtagctgaa ctgctggaaa 1320
   tattaggaag tattatcaat ggctttgctt tacctcttaa ggcagaacac aaacagtttc 1380
   tggtgaaagt attgatccct ttacacactg tcaggagctt atcactcttc catgcacagc 1440
   tggcatattg tatagtacag tttctggaga aagatccttc actcacagaa ccagttatta 1500
   gggggttaat gaaattttgg cctaaaacat gtagtcaaaa agaggtcatg ttccttgggg 1560
   aactggaaga aatattggat gtgattgaac cttcacaatt tgttaaaatc caagaacctt 1620
   tgtttaaaca aatcgccaag tgtgtatcta gcccccattt tcaggtggca gaaagagcac 1680
   tctattattg gaataatgaa tacatcatga gtttgataga agaaaactct aacgtcatcc 1740
   ttcccatcat gttttccagc ctttatagga tttcaaaaga acattggaat ccggctattg 1800
   tggcgttggt gtacaatgtg ttgaaggcat ttatggaaat gaacagcacc atgtttgacg 1860
   agctgacagc cacatacaag tcagatcgtc agcgtgagaa aaagaaagaa aaggagcgtg 1920
   aagaattgtg gaaaaaattg gaggatctgg agttaaagag aggtcttaga cgtgatggaa 1980
   taattccaac ttaacaaaaa caatgacaac aacattacta acctgtggag tcacacgttt 2040
   atgtagtaga agatggagca acagttttct gtattgtgca actttacagt agatttcacc 2100
   tttgtttcat tattacagca gcactgtata tacctgtctc taagtaaagg aaaaaacaaa 2160
   ataaggactt caatccaaag tttggacagt agatggactt ctcagaactt tgcaaacata 2220
   atcattgttc tcaccctctt ttaaaaaaaa aaatcggtct tcaaagatct gttgatgaaa 2280
   ttgctatgtt aaaattccat tatcgggagt tccttattta tcactagcag agagtatgat 2340
   acaattttca aatgtgaaca atcttaaatt tagcttgtct ttctgctaag ctgttaaatg 2400
   tatttatagt aaaggaagaa aaaaagactg tcatttcctt ataagtttgt gtaacatcct 2460
   cctctggata acttgactgt aatttaacat ctttttcttt tgcacatctt cctgagttga 2520
   atgtccacgt ggaatggggt catgaattat aaaagtccct gataaaagtt ttgtttactg 2580
   gggtgaacat ctttccagta accaggtagt cctggtactc ctttagtttt aaaattagga 2640
   gttaagagag aagaggtgat aaacatagta gggaagggaa tatcggattc atgcatcagt 2700
   ttatggtgaa tccaaatcaa tgtcttgaat cctttgaaaa caggcactgg gacatcacag 2760
   gcttcagtac ctgaccagta ttagttgcat atatcattga acacacatac cagagatgtt 2820
   ttagaaatgt gagaaaaaca tccttttgga ccatttgaaa taagaaagac aaacactaaa 2880
   caatacaacc atgaaattga tcaccgggat tgcaaatcta attgggaaaa gagttgagca 2940
   aacagcttgg actgtttgga gttgttgcct tactttttaa tatgtattta taaagtattc 3000
   cagcaaaaga ggatgtagcc tctgggaaaa aacaaacatg ttacagtgtt ttttgtagat 3060
   tctcgttcta tatctcatca cagcgccagc cctgttttta gccggaaagg attcaggata 3120
   aacattatta tgcattctga attggatgca tattcctaac tactgtattt gttaccaaaa 3180
   gtggttctac aaatgctact gaaaaaaatc tggaaattcc taatgtcctg agtattaata 3240
   ataaagttta aaaatgcttt tatatcaaag gtgcatcgtg accaaattgt ttaagaaaaa 3300
   aaaacaaaaa aaacaaaatc tagggctgta ttttatatat atatatatat atatatatat 3360
   atatat 3366
<210> 68
   <211> 3192
   <212> DNA
   <213> Homo sapiens
<400> 68
   attcgctgcg gagccggagg aggaggggag aggcctggag gacaccaaca tgaacaagtt 60
   gaaatcatcg cagaaggata aagttcgtca gtttatgatc ttcacacaat ctagtgaaaa 120
   aacagcagta agttgtcttt ctcaaaatga ctggaagtta gatgttgcaa cagataattt 180
   tttccaaaat cctgaacttt atatacgaga gagtgtaaaa ggatcattgg acaggaagaa     240
   gttagaacag ctgtacaata gatacaaaga ccctcaagat gagaataaaa ttggaataga     300
   tggcatacag cagttctgtg atgacctggc actcgatcca gccagcatta gtgtgttgat     360
   tattgcgtgg aagttcagag cagcaacaca gtgcgagttc tccaaacagg agttcatgga     420
   tggcatgaca gaattaggat gtgacagcat agaaaaacta aaggcccaga tacccaagat     480
   ggaacaagaa ttgaaagaac caggacgatt taaggatttt taccagttta cttttaattt     540
   tgcaaagaat ccaggacaaa aaggattaga tctagaaatg gccattgcct actggaactt     600
   agtgcttaat ggaagattta aattcttaga cttatggaat aaatttttgt tggaacatca     660
   taaacgatca ataccaaaag acacttggaa tcttctttta gacttcagta cgatgattgc     720
   agatgacatg tctaattatg atgaagaagg agcatggcct gttcttattg atgactttgt     780
   ggaatttgca cgccctcaaa ttgctgggac aaaaagtaca acagtgtagc actaaaggaa     840
   ccttctagaa tgtacatagt ctgtacaata aatacaacag aaaattgcac agtcaatttc     900
   tgctggctgg actgaactga agatcaatcc tcacaattca gactgagggt tgagacaaaa     960
   ctttaaggat acatcttgga ccatatcgta tttcattctt ctaatggtgg tttgggcttg    1020
   tcttctagtc tgggccgctc taaacattta taattccaac attgtggatt tcatcttata    1080
   tctgtggacc atcctagttt attctcccat aagtcttaga agctttatgg tgattatttt    1140
   gaggttttca ttctcgcata aagcacaatg ctgtcttcat cagaaaacag ttggcataag    1200
   aattaaacat atgaacatca caaaacaatt tataaaaact tcttaaatat acgctttggg    1260
   ctagttgcaa agactatgct aatagcactt ccagtgagag tgatatattt aagtgtactg    1320
   gatctggaat ggtgttttgg tttgggggga attttttttt tttcctggca aatcacatgt    1380
   gttgttgatg tgagtatctg atgaaaaaac aatgtcagaa taaccgacat gaaaattttt    1440
   taggataact tggtgcctac ctgaaaaatg tattgtgttt tagactcttg atttcaaaag    1500
   gttccacaga actagtctgc gcttacctta cccatgttta tatatagctg tcctacaggg    1560
   agcttttatt tagaaaatgt ctgcataatg ttagattctt ctcctgtcta cattatgcac    1620
   tacataattg gacttcatta tgcttttgaa atgcttatct gcctgtcaca taagttaaac    1680
   tatttaattt gttttgaatg ttttggattg ctacacaata caatattcta aatttaggca    1740
   tgagggtttt tttgttttgt ttttactttt tttttgtcat cgcactatgg aacacaaatg    1800
   gaattctctt aatttataag aagatagttg cagttaaatt ttgaaaatgg ttgtaatgag    1860
   ccatgaagtt caatctttat aatataggta ctgctctttc agacaaatag tccattttcg    1920
   atgacttatt attttgttga aattgcttta actgctaatc actgtggttg ccaaatattt    1980
   acttcaggag caaagatttt caaacaagca tacacgatgc aaaataccaa tctggcttct    2040
   agtctcttta ctgttttcgt ttcactcaga ttagctcagt tttctcatca aagcagaatg    2100
   ctatcttgta tgtatttttt tcattacaag ccccatgagc tgcttttatg ctgaaaatgg    2160
   tcatttccct gttcacttac tgacatgtga agaagggttt cttgctttct taaacatttc 2220
   cgtaaggcag gctagaaatg taatacttca aatgtttgat gattatggtc ttttgatagg 2280
   aatagattct gcttgggata tatatccagg cactctctaa ggtctagggt tgatattaac 2340
   aaaggaatgt acttagaata gcagtacatt ttatgcaaat atggaaatta ttttaagaaa 2400
   caatgacata tcaaaactgc tttttacatg attttgaaat agactagaaa gctttcccta 2460
   tagacatatt aatattccaa tcataacttt aattcaagaa tgcagtttta ccaaaagaaa 2520
   aatttgaaaa tttctattca ggctactgga attggttatt aaaagaaaaa ggaaaaagaa 2580
   gaatcttgct gctttcagta tttcctgatt tttttgtaaa tataaagagg aacttcaatt 2640
   atgaaaaatt tttaaaagat atatatatct atatatctat atatatgtac tgttttgttt 2700
   cctgtcttga agattttgag ttatggttat tggtttcaga ttgattaatt cacatatgct 2760
   gtgttttgaa atgagatccc attagctttt tttttttttt ttttttttca atataaagtg 2820
   ttttctttaa aagtcatatt ggttcgtggc ctagtgcctt ggattttaca tatttttctt 2880
   tttaaatgca aaaccttttc aacaaaatag tgtttgtcat caggttggta ctaaacattt 2940
   ataattactg tgtaattata aacaaaaata cataaagctt tgaatataat tatgtagcat 3000
   aaaagttaag gttgttcact atgatggcat cttagaatta aacaaaactt ttactagggc 3060
   tgaaaagaga agactgattt aatgtggtgt gattattctg aagataaatg tctggctaca 3120
   gggaatattt tgtactaaaa aatgattaca caaaaaaaaa aaaaaaaaaa aaaaaaaaaa 3180
   aaaaaaaaaa aa 3192
<210> 69
   <211> 3782
   <212> DNA
   <213> Homo sapiens
<400> 69
   ttctgccctt cccccgctta ggggggcggg ggtaggggaa aggaaaataa tacaatttca     60
   ggggaagtcg ccttcaggtc tgctgctttt ttattttttt tttttaatta aaaaaaaaaa    120
   ggacatagaa aacatcagtc ttgaacttct cttcaagaac ccgggctgca aaggaaatct    180
   cctttgtttt tgttatttat gtgctgtcaa gttttgaagt ggtgatcttt agacagtgac    240
   tgagtatgga tcatttgaac gaggcaactc aggggaaaga acattcagaa atgtctaaca    300
   atgtgagtga tccgaagggt ccaccagcca agattgcccg cctggagcag aacgggagcc    360
   cgctaggaag aggaaggctt gggagtacag gtgcaaaaat gcagggagtg cctttaaaac    420
   actcgggcca tctgatgaaa accaacctta ggaaaggaac catgctgcca gttttctgtg    480
   tggtggaaca ttatgaaaac gccattgaat atgattgcaa ggaggagcat gcagaatttg    540
   tgctggtgag aaaggatatg cttttcaacc agctgatcga aatggcattg ctgtctctag    600
   gttattcaca tagctctgct gcccaggcca aagggctaat ccaggttgga aagtggaatc    660
   cagttccact gtcttacgtg acagatgccc ctgatgctac agtagcagat atgcttcaag 720
   atgtgtatca tgtggtcaca ttgaaaattc agttacacag ttgccccaaa ctagaagact 780
   tgcctcccga acaatggtcg cacaccacag tgaggaatgc tctgaaggac ttactgaaag 840
   atatgaatca gagttcattg gccaaggagt gccccctttc acagagtatg atttcttcca 900
   ttgtgaacag tacttactat gcaaatgtct cagcagcaaa atgtcaagaa tttggaaggt 960
   ggtacaaaca tttcaagaag acaaaagata tgatggttga aatggatagt ctttctgagc 1020
   tatcccagca aggcgccaat catgtcaatt ttggccagca accagttcca gggaacacag 1080
   ccgagcagcc tccatcccct gcgcagctct cccatggcag ccagccctct gtccggacac 1140
   ctcttccaaa cctgcaccct gggctcgtat caacacctat cagtcctcaa ttggtcaacc 1200
   agcagctggt gatggctcag ctgctgaacc agcagtatgc agtgaataga cttttagccc 1260
   agcagtcctt aaaccaacaa tacttgaacc accctccccc tgtcagtaga tctatgaata 1320
   agcctttgga gcaacaggtt tcgaccaaca cagaggtgtc ttccgaaatc taccagtggg 1380
   tacgcgatga actgaaacga gcaggaatct cccaggcggt atttgcacgt gtggctttta 1440
   acagaactca gggcttgctt tcagaaatcc tccgaaagga agaggacccc aagactgcat 1500
   cccagtcttt gctggtaaac cttcgggcta tgcagaattt cttgcagtta ccggaagctg 1560
   aaagagaccg aatataccag gacgaaaggg aaaggagctt gaatgctgcc tcggccatgg 1620
   gtcctgcccc cctcatcagc acaccaccca gccgtcctcc ccaggtgaaa acagctacta 1680
   ttgccactga aaggaatggg aaaccagaga acaataccat gaacattaat gcttccattt 1740
   atgatgagat tcagcaggaa atgaagcgtg ctaaagtgtc tcaagcactg tttgcaaagg 1800
   ttgcagcaac caaaagccag ggatggttgt gcgagctgtt acgctggaaa gaagatcctt 1860
   ctccagaaaa cagaaccctg tgggagaacc tctccatgat ccgaaggttc ctcagtcttc 1920
   ctcagccaga acgtgatgcc atttatgaac aggagagcaa cgcggtgcat caccatggcg 1980
   acaggccgcc ccacattatc catgttccag cagagcagat tcagcaacag cagcagcaac 2040
   agcaacagca gcagcagcag cagcaggcac cgccgcctcc acagccacag cagcagccac 2100
   agacaggccc tcggctcccc ccacggcaac ccacggtggc ctctccagca gagtcagatg 2160
   aggaaaaccg acagaagacc cggccacgaa caaaaatttc agtggaagcc ttgggaatcc 2220
   tccagagttt catacaagac gtgggcctgt accctgacga agaggccatc cagactctgt 2280
   ctgcccagct cgaccttccc aagtacacca tcatcaagtt ctttcagaac cagcggtact 2340
   atctcaagca ccacggcaaa ctgaaggaca attccggttt agaggtcgat gtggcagaat 2400
   ataaagaaga ggagctgctg aaggatttgg aagagagtgt ccaagataaa aatactaaca 2460
   cccttttttc agtgaaacta gaagaagagc tgtcagtgga aggaaacaca gacattaata 2520
   ctgatttgaa agactgagat aaaagtattt gtttcgttca acagtgccac tggtatttac 2580
   taacaaaatg aaaagtccac cttgtcttct ctcagaaaac ctttgttgtt cattgtttgg 2640
   ccaatgaatc ttcaaaaact tgcacaaaca gaaaagttgg aaaaggataa tacagactgc 2700
   actaaatgtt ttcctctgtt ttacaaactg cttggcagcc ccaggtgaag catcaaggat 2760
   tgtttggtat taaaatttgt gttcacggga tgcaccaaag tgtgtacccc gtaagcatga 2820
   aaccagtgtt ttttgttttt tttttagttc ttattccgga gcctcaaaca agcattatac 2880
   cttctgtgat tatgatttcc tctcctataa ttatttctgt agcactccac actgatcttt 2940
   ggaaacttgc cccttattta aaaaaaaaaa agaaaaaaaa gagtttgtta ctctattgta 3000
   tgttacaaaa gaactataga ctgtggaatg cagtttaaag atgacatatg ccaacaaatg 3060
   ccttgtatta tatggcactg ccgtaattca aatttgtttt tattttggaa ataaaagttc 3120
   actgtacttt tttttcattc tcattgttac atgatttttt aaaaaaagga aaagaaaatg 3180
   tgaaacacaa tttagtcctc attatttatt tgtagatcct gcagcatcat gttgtaatta 3240
   attttttgga agtttccgtt aaatgtaata ttgcttctct tgttaccata ctgattcttt 3300
   tctatttata aatgtatttt gatgggcagt aaaacaaagt gtcttaaaag ttttaaatag 3360
   agaaaatgtg ctttacacag ttgcctataa aaagtgctct atgttatcca agcaattcat 3420
   actataagct tcactcttat tgttgtatgc aatttttact atcatgcaaa taagcttagg 3480
   taaataaaac taatagatca ccttagaaaa ttatgcaatt aatgtgaaaa taattgatgt 3540
   ttgcaatgtg tcttcctttg gtttacaatc aattttaaag ctacatctgt ataaaatttc 3600
   tgtataaagg tgtatttctt ttttatgagt ttatggctat gaaaacagct attttgttac 3660
   agctggctgt ttttataagt gtatcacaat tttctttatg cagaaatgtt ctgactagga 3720
   gtggttattg actgtaacta cacaattaaa attgtttgta tcgtaaaaaa aaaaaaaaaa 3780
   aa 3782
<210> 70
   <211> 1621
   <212> DNA
   <213> Homo sapiens
<400> 70
   gcggcaggtc tctgcgcagc ccagcccgcc ggtccacgcc gcgcaccgct ccgagggcca      60
   gcgccacccg ctccgcagcc ggcaccatgc gcgagatcgt gcacatccag gcgggccagt     120
   gcggcaacca gatcggcgcc aagttttggg aggtcatcag cgatgagcat gggatcgacc     180
   ccacaggcag ttaccatgga gacagtgact tgcagctgga gagaatcaac gtgtactaca     240
   atgaggctgc tggtaacaaa tatgtacctc gggccatcct ggtggatctg gagcctggca     300
   ccatggactc tgtcaggtct ggacccttcg gccagatctt cagaccagac aacttcgtgt     360
   tcggccagag tggagccggg aataactggg ccaagggcca ctacacagag ggagccgagc     420
   tggtcgactc ggtcctggat gtggtgagga aggagtcaga gagctgtgac tgtctccagg     480
   gcttccagct gacccactct ctggggggcg gcacggggtc cgggatgggc accctgctca    540
   tcagcaagat ccgggaagag tacccagacc gcatcatgaa caccttcagc gtcatgccct     600
   cacccaaggt gtcagacacg gtggtggagc cctacaacgc caccctctct gtccaccagc     660
   tggtggaaaa cacagatgaa acctactcca ttgataacga ggccctgtat gacatctgct     720
   tccgcaccct gaagctgacc acccccacct acggggacct caaccacctg gtgtcggcca     780
   ccatgagcgg ggtcaccacc tgcctgcgct tcccgggcca gctgaacgca gacctgcgca     840
   agctggcggt gaacatggtg cccttccctc gcctgcactt cttcatgccc ggcttcgcgc     900
   ccctgaccag ccggggcagc cagcagtacc gggcgctcac ggtgcccgag ctcacccagc     960
   agatgttcga ctccaagaac atgatggccg cctgcgaccc gcgccacggc cgctacctga    1020
   cggtggctgc catcttccgg ggccgcatgt ccatgaagga ggtggacgag cagatgctca    1080
   acgtgcagaa caagaacagc agctacttcg tggagtggat ccccaacaac gtgaagacgg    1140
   ccgtgtgcga catcccgccc cgcggcctga agatgtcggc caccttcatc ggcaacagca    1200
   cggccatcca ggagctgttc aagcgcatct ccgagcagtt cacggccatg ttccggcgca    1260
   aggccttcct gcactggtac acgggcgagg gcatggacga gatggagttc accgaggccg    1320
   agagcaacat gaacgacctg gtgtccgagt accagcagta ccaggacgcc acggccgacg    1380
   aacaagggga gttcgaggag gaggagggcg aggacgaggc ttaaaaactt ctcagatcaa    1440
   tcgtgcatcc ttagtgaact tctgttgtcc tcaagcatgg tctttctact tgtaaactat    1500
   ggtgctcagt tttgcctctg ttagaaattc acactgttga tgtaatgatg tggaactcct    1560
   ctaaaaatta cagtattgtc tgtgaaggta tctatactaa taaaaaagca tgtgtagaaa    1620
   a                                                                    1621
<210> 71
   <211> 2529
   <212> DNA
   <213> Homo sapiens
<400> 71
   cggagttgtc gtgtgttctg gattcattcc ggcaccacca tgtcgaaggt ttcctttaag 60
   atcacgctga cgtcggaccc acggctgccg tacaaagtac tcagtgttcc tgaaagtaca 120
   cctttcacag cagtcttaaa gtttgcagca gaagaattta aagttcctgc tgcaacaagt 180
   gcaattatta ccaatgatgg aataggaata aatcctgcac agactgctgg aaatgttttt 240
   ctaaaacatg gttcagaact gcggattatt cctagagatc gtgttggaag ttgttaatat 300
   ctgctacttg gaacatacga ttgcctttca gaataaatat tggtattttt tgttgttgta 360
   aaattgaaat caggcattta acatactatg aaaacaccag gagtcaatga ttaatgaaag 420
   gtgactcatc tgtccctttt tgttgtccat actcttccta tgaagaggga atgcgtatga 480
   attaaggcta ctactgtcac agaagatcat agtctttgat gctacctcac aacacaaaca 540
   ggtagttcgt tgggggcaaa tgaattagcc aactgttaac tggaagcttt tgataatttt 600
   tttttttaga acaatttgga acattaaaat ttactgaatc gtatatattc atctgagata 660
   aaaatataaa aagaattatg gaccctggat ggcaatttgc ttgatagcat ctgatttgca 720
   gactcataat ttgattttta attaaatata taggttatga tgaagtgaat agacatatca 780
   gtgaacagtt aactatatta aatttttatc atttactttt tttaagattc agacctcagt 840
   tatataaatt tcagtttaat atcaaccaaa aaattaaaat tttaatctaa cccttatgtg 900
   tataaattgg tgtcccatac cagcttttaa tggtggacct atagaatcca gtacttttaa 960
   tggtgggaat ttacagtaga agcatccttt gctgagttat acattccttt atcaatctct 1020
   tttgatacaa catttaaaac aagtagcttc aagaaaccac tggtgttttg aggatagtat 1080
   ttctaaatag cattcaggaa cagagtatta ttgcacagat ctgaagatca aaaaaaagct 1140
   caaggaaata cagatcggaa gtgctgatga gttatattta ttgaaaaccc aacttttaag 1200
   gaagtgctaa gatcagtcac ccatgtgaat aagaagccag gaaaggaaag atggggaagc 1260
   ccagatcacc aggcttctat taaggaggaa agcaacagag gaaacagtga aggggaacag 1320
   aagggggtag caaagtgtta cagaaaagcg gactggatag acaaaactgc agaaggtgta 1380
   tgttggggag aactgaaagg gaaaacaaaa tacttgacat agtcttaagt agaagaaggc 1440
   agttagagaa aacaaagtat ctactggcct tgtcaacata cagacttcaa aatacccctt 1500
   atgagaatcc aaagaatgat gtgtgtaagg gaagatttta tttgcccttc cggaagaaat 1560
   cagtatctat gcaaatcttg aaagacgaaa tcaaagccca ttaatgattc agaatcagtg 1620
   cttgacctcc tgtattctga atggtgaact ctggaagcag ggattgtgtc tggctctttt 1680
   tagagctgga agtgtagtgg ctttcattaa atacttgctg taaagtcttt ctaagaccaa 1740
   ttattatctt agcatgtttc agtatcttct ctatcatagg ccctaagttc attgggggaa 1800
   aaaaaataag aagattcaac agaatcagca tttgaagtgt accattggta gttgtttatg 1860
   aaattaccag atattcataa atgtgacaaa tgaacagcag gattatgaat tatcaaagga 1920
   aaaagtattt gctgaggtga aaaaatctga tgtttgagga agtttttatt tttatttatt 1980
   tgttttgttt tttttttttt ttgaggcaga ctctctgtcg ccaggcttct cctgcctcag 2040
   cctcacgagt agctgggact acaggcatgc accaccacgc ccagctaatt tttgtatttt 2100
   tagcagagac agggaggaag tttttatttt tataaacaaa atctgatgtt cagaggcccc 2160
   gtttcttaca ataaatgttg agtcttagtt aagcaggaat ttatgaacac ccatttcctg 2220
   actttttgct ttaatttaga tttttctcca tcctgtttct agcacaaaaa tttgcctgct 2280
   gtgttacaaa aataaattta tgttgtcctg tgccataaag tgatatattt aatattttta 2340
   ttctttggtt ttgaacattg taagttccta aaaacatttt attaaacaag tagacatttt 2400
   gttattaaaa aaatgtgatc tgtaatttct ttgtgcagaa tgatttgaag tattgtattc 2460
   agtttacatg cattattggt ttataattaa tatctaatga aaatacatgt tgttatattg 2520
   taaaaaaaa 2529
<210> 72
   <211> 5184
   <212> DNA
   <213> Homo sapiens
<400> 72
   aaaaccctgc aggagactgc gagccctgca gaactgctag ctgcggggga gagggcaggg 60
   gtcgggcgcc tgtggcggag ccgggctggg gccagggcag ggaggctgac aagcggcggg 120
   agaagccggc ggagggcggg atcgcgcctc ctgacatgtt gggggtatcc ctggccgggc 180
   cgggccgggg ctaagagcgg cgctgcgggc cggggtcggg gtcgggtcgc ggtccgcccc 240
   cgctgtccct ccgtcctgcc ctgtcgagga cgtgcgttcc gcactcggcc gcctccagag 300
   ggagcgaggg aagcggctag aggatcgggg agaaggagca ttcgccggag gctggaggag 360
   gctgacccgc gtccccgccc agcctgctcc tatgcggtac ttgaaggatg gcgaagaggt 420
   cgcgcagtga ggatgaggat gatgaccttc agtatgccga tcatgattat gaagtaccac 480
   aacaaaaagg actgaagaaa ctctggaaca gagtaaaatg gacaagggac gaggatgata 540
   aattaaagaa gttggttgaa caacatggaa ctgatgattg gactctaatt gctagtcatc 600
   ttcaaaatcg ctctgatttt cagtgccagc atcgatggca gaaagtttta aatcctgaat 660
   tgataaaggg tccttggact aaagaagaag atcagagggt tattgaatta gttcagaaat 720
   atgggccaaa aagatggtct ttaattgcaa aacatttaaa aggaagaata ggcaagcagt 780
   gtagagaaag atggcataat catctgaatc ctgaggtaaa gaaatcttcc tggacagaag 840
   aggaggacag gatcatctat gaagcacata agcggttggg aaatcgttgg gcagaaattg 900
   ccaaactact tccaggaagg actgataatt ctatcaaaaa tcattggaat tctactatgc 960
   gaagaaaagt ggaacaggag ggctatttac aagatggaat aaaatcagaa cgatcttcat 1020
   ctaaacttca acacaaacct tgtgcagcta tggatcatat gcaaacccag aatcagtttt 1080
   acatacctgt tcagatccct gggtatcagt atgtgtcacc tgaaggcaat tgtatagaac 1140
   atgttcagcc tacttctgcc tttattcagc aacccttcat tgatgaagat cctgataagg 1200
   aaaagaaaat aaaggaactt gagatgcttc ttatgtcagc tgagaatgaa gttagaagaa 1260
   agcgaattcc atcacagcct ggaagttttt ctagctggtc tggtagtttc ctcatggatg 1320
   ataacatgtc taatactcta aatagccttg acgagcacac tagtgagttt tacagtatgg 1380
   atgaaaatca gcctgtgtct gctcagcaga attcacccac aaagttcctg gccgtggagg 1440
   caaacgctgt gttatcctct ttgcagacca tcccagaatt tgcagagact ctagaactta 1500
   ttgaatctga tcctgtagca tggagtgacg ttaccagttt tgatatttct gatgctgctg 1560
   cttctcctat caaatccacc ccagttaaat taatgagaat tcagcacaat gaaggagcca 1620
   tggaatgcca atttaacgtc agtcttgtac ttgaagggaa aaaaaacact tgtaatggtg 1680
   gcaacagtga agctgttcct ttaacatccc caaatatagc caagtttagc actccaccag 1740
   ccatcctcag aaagaagaga aaaatgcgag tgggtcattc cccaggcagc gaacttaggg 1800
   atggctcatt gaacgatggt ggtaatatgg cgctaaaaca tacaccactg aaaacactac 1860
   cattttctcc ttcacagttt ttcaacacat gtcctggtaa tgaacaactt aatatagaaa 1920
   atccttcatt tacatcaacc cctatttgtg ggcagaaagc tctcattaca actcctcttc 1980
   ataaggaaac aactcccaaa gatcaaaagg aaaatgtagg gtttagaaca cctactatta 2040
   gaagatctat actgggtacc acaccaagaa ctcctactcc ttttaagaat gcgcttgctg 2100
   ctcaggagaa aaaatatgga cctcttaaaa ttgtgtccca gccacttgct ttcttggaag 2160
   aagatattcg ggaagtttta aaagaagaaa ctggaacaga cctattcctc aaagaggaag 2220
   atgaacctgc ttacaaaagc tgcaaacaag agaataccgc ttctgggaag aaagtcagaa 2280
   aatcactagt cttagataat tgggaaaaag aagaatcagg cactcaactg ttgactgaag 2340
   acatttcaga catgcagtca gaaaatagat ttactacatc cttattaatg ataccattat 2400
   tggaaataca tgacaatagg tgcaacttga ttcctgaaaa acaagatata aattcaacca 2460
   acaaaacata tacacttact aaaaagaaac caaaccctaa cacttccaaa gttgtcaaat 2520
   tggaaaagaa tcttcagtca aattgtgaat gggaaacagt ggtttatggg aagacagaag 2580
   accaacttat tatgactgaa caagcaagaa gatatctgag tacttacaca gctaccagta 2640
   gtacttcaag agctctcata ctgtaattgt tattaaaatt gatgaaatgc cccactccct 2700
   tactgcagtc tctactaaat taggttgcag tgaaattttt ctcaattagt tgtttttaaa 2760
   gttgtaagat agccctttta atacagcatc ttttttctat tctatatagt aggcagaaag 2820
   ctagtaagtc acttaagggg tagatagttt catagtttat tttttaagag atgagatttt 2880
   taaaaattgt ttttaaagaa caagatggga aaataataga atgttcatgg atttctaaaa 2940
   gtaaattctc atatattttc ttcacaagat atatgttgct actctcttga tgctgcagtt 3000
   ttgttataga taggtgtatg agtatatatg atttctgaaa ttagtctatg tatggaaagc 3060
   acacatgatt ttatgaagta cttttgccca tgtgctgatt tacttaggct accatttaca 3120
   aagaaacaca ttgaaaagga atttaaagga aggatagaaa gttgcactac taattttttg 3180
   tttttttttt cagaagcagt aaaattaact acagtgttaa atgtatttat ttgagcatag 3240
   tactgaaaac aaaaagcatt caaaaaagag ttttttcttt attagtaaat agtattttct 3300
   taatctcaga ggagctgaga gttttgttga atgtattgta cagtatgtag gagcaggaga 3360
   actttgtaaa ttggaaagaa gtctgttttt ataatttatt tttattttta aagcttaaat 3420
   gtagatattt atacgtatac agggtgccta gaagccaatg ttgtttcctg ttattacagc 3480
   taacacagta aagaataatt ttgactttaa gtatgaaaca gtagtaagtt atagctgcaa 3540
   agaatacaat atctatactg tatgtcacat ctacctaaat gttgcactat gccctttaaa 3600
   tcatgctggt tataaagtag ttctaaaaat gtactaaata ataatttaat attttctttt 3660
   taaattatat cgggggtggt catatacatt aatctggtga tttgtatatg tgtttgaaat 3720
   ttttgcattt tgtttaaaaa ataatatggt accttggtcc ctaaaaacag tctgcactta 3780
   gaagtttata tttactcagt gtttcagaag tggagaacat tatcttttat ttataaaaat 3840
   attttgtcct tttttaaatg ttttgtgttt ctctacaggt tacaacagtt gcttcagttg 3900
   cctgttttag gtgtttgcac ttattttatt tcttcttgaa agaattttta tttgcttttg 3960
   tggtagagat tatatgtaat tttttttcag tcatataatg gtgtgctgtc aacttaaaca 4020
   ctgacaggta aatagaattg tacactgtag tttgaattat ttataattga cacactctct 4080
   ccctctccac tcctgaagta tgctgctata gaaaatagca gaatcggctt gctgctacga 4140
   gagaaggaaa gagcgaccac cacttgcact gtgtgaaaag ataaaaaaca aatgatggca 4200
   agttctcaag ttaactaaat ggaatcaacc attaccaggc aaattcttgc aaataccaaa 4260
   atactactat gccttataaa acaaaatgaa agcaggttaa gattttctgc tctgtttgta 4320
   tgttaataga aatggaaata ctaagtattt taatgcttag ctcttgaaca gtagacctaa 4380
   aagggtttta agctatttaa atctacttgc tagtttttgc atattttata tatatatata 4440
   tttatatata tatatagtga gaagtgaaga aaatgtatgg tactaagatt atgccttatt 4500
   gataaataga taaaccaatt tgaatcctct tagcatgttt aagtatgttg attgctttct 4560
   aattaatgaa cttctcacag aaatttcact tagtgaaacc aatgattgta gcaaactcat 4620
   actggatcat ttcagttacc ttgaactaat agcacataat ggttttttgt tgttgttgtt 4680
   tttaatgtag cccttacctg gatatacata gtctgcaatc accaaagtat aatatcttgt 4740
   aaggctatat tttttaaagc atattttttc ttgagcatta aattatccta aatggtaata 4800
   tattgtggat aagtctgggc ttattggaca taatacatat ttgggttggt actggttgaa 4860
   tccttcagtt aactgctttg ttgctttttg caagattttt tatcttaaac atgtcaggca 4920
   tcttaagtca cctttatact gttttgttcc tctgagtttc tttcagtatg ttatacaaat 4980
   gccagacata acatgtagca gccatacttg catggaaact gactacacat acataatact 5040
   gcattttatt gtaaggtttt cacattaata cagcaattac cctgactaaa ttgagttttg 5100
   tgatatatgg aaaacttcat tgtaagagaa tcttgcatac aatgttgaca tattaacatc 5160
   caaaataaag catctgtgta caag 5184
<210> 73
   <211> 7149
   <212> DNA
   <213> Homo sapiens
<400> 73
   atgtctggcg gcgccgcaga gaagcagagc agcactcccg gttccctgtt cctctcgccg 60
   ccggctcctg cccccaagaa tggctccagc tccgattcct ccgtggggga gaaactggga 120
   gccgcggccg ccgacgctgt gaccggcagg accgaggagt acaggcgccg ccgccacact 180
   atggacaagg acagccgtgg ggcggccgcg accactacca ccactgagca ccgcttcttc 240
   cgccggagcg tcatctgcga ctccaatgcc actgcgctgg agcttcccgg ccttcctctt 300
   tccctgcccc agcccagcat ccccgcggct gtcccgcaga gtgctccacc ggagccccac 360
   cgggaagaga ccgtgaccgc caccgccact tcccaggtag cccagcagcc tccagccgct 420
   gccgcccctg gggaacaggc cgtcgcgggc cctgccccct cgactgtccc cagcagtacc 480
   agcaaagacc gcccagtgtc ccagcctagc cttgtgggga gcaaagagga gccgccgccg 540
   gcgagaagtg gcagcggcgg cggcagcgcc aaggagccac aggaggaacg gagccagcag 600
   caggatgata tcgaagagct ggagaccaag gccgtgggaa tgtctaacga tggccgcttt 660
   ctcaagtttg acatcgaaat cggcagaggc tcctttaaga cggtctacaa aggtctggac 720
   actgaaacca ccgtggaagt cgcctggtgt gaactgcagg atcgaaaatt aacaaagtct 780
   gagaggcaga gatttaaaga agaagctgaa atgttaaaag gtcttcagca tcccaatatt 840
   gttagatttt atgattcctg ggaatccaca gtaaaaggaa agaagtgcat tgttttggtg 900
   actgaactta tgacgtctgg aacacttaaa acgtatctga aaaggtttaa agtgatgaag 960
   atcaaagttc taagaagctg gtgccgtcag atccttaaag gtcttcagtt tcttcatact 1020
   cgaactccac ctatcattca ccgcgatctt aaatgtgaca acatctttat caccggccct 1080
   actggctcag tcaagattgg agacctcggt ctggcaaccc tgaagcgggc ttcttttgcc 1140
   aagagtgtga taggtacccc agagttcatg gcccctgaga tgtatgagga gaaatatgat 1200
   gaatccgttg acgtttatgc ctttgggatg tgcatgcttg agatggctac atctgaatat 1260
   ccttactcgg agtgccaaaa tgctgcgcag atctaccgtc gcgtgaccag tggggtgaag 1320
   ccagccagtt ttgacaaagt agcaattcct gaagtgaagg aaattattga aggatgcata 1380
   cgacaaaaca aagatgaaag atattccatc aaagaccttt tgaaccatgc cttcttccaa 1440
   gaggaaacag gagtacgggt agaattagca gaggaagatg atggagaaaa aatagccata 1500
   aaattatggc tacgtattga agatattaag aaattaaagg gaaaatacaa agataatgaa 1560
   gctattgagt tttcttttga tttagagaga gatgtcccag aagatgttgc acaagaaatg 1620
   gtagagtctg ggtatgtctg tgaaggtgat cacaagacca tggctaaagc tatcaaagac 1680
   agagtatcat taattaagag gaaacgagag cagcggcagt tggtacggga ggagcaagaa 1740
   aaaaaaaagc aggaagagag cagtctcaaa cagcaggtag aacaatccag tgcttcccag 1800
   acaggaatca agcagctccc ttctgctagc accggcatac ctactgcttc taccacttca 1860
   gcttcagttt ctacacaagt agaacctgaa gaacctgagg cagatcaaca tcaacaacta 1920
   cagtaccagc aacccagtat atctgtgtta tctgatggga cggttgacag tggtcaggga 1980
   tcctctgtct tcacagaatc tcgagtgagc agccaacaga cagtttcata tggttcccaa 2040
   catgaacagg cacattctac aggcacagtc ccagggcata taccttctac tgtccaagca 2100
   cagtctcagc cccatggggt atatccaccc tcaagtgtgg cacaggggca gagccagggt 2160
   cagccatcct caagtagctt aacaggggtt tcatcttccc aacccataca acatcctcag 2220
   cagcagcagg gaatacagca gacagcccct cctcaacaga cagtgcagta ttcactttca 2280
   cagacatcaa cctccagtga ggccactact gcacagccag tgagtcagcc tcaagctcca 2340
   caagtcttgc ctcaagtatc agctggaaaa cagcttccag tttcccagcc agtaccaact 2400
   atccaaggcg aacctcagat cccagttgcg acacaaccct cggttgttcc agtccactct 2460
   ggtgctcatt tccttccagt gggacagccg ctccctactc ccttgctccc tcagtaccct 2520
   gtctctcaga ttcccatatc aactcctcat gtgtctacgg ctcagacagg tttctcatcc 2580
   cttcccatca caatggcagc tggcattact cagcctctgc tcacgttggc ttcatctgct 2640
   acaacagctg cgatcccggg ggtatcaact gtggttccta gtcagcttcc aacccttctg 2700
   cagcctgtga ctcagctgcc aagtcaggtt cacccacagc tcctacaacc agcagttcag 2760
   tccatgggaa taccagctaa ccttggacaa gctgctgagg ttccactttc ctctggagat 2820
   gttctgtacc agggcttccc acctcgactg ccaccacagt acccaggaga ttcaaatatt 2880
   gctccctctt ccaacgtggc ttctgtttgc atccattcta cagtcctatc ccctcccatg 2940
   ccgacagaag tactggctac acctgggtac tttcccacag tggtgcagcc ttatgtggaa 3000
   tcaaatcttt tagttcctat gggtggtgta ggaggacagg ttcaagtgtc ccagccagga 3060
   gggagtttag cacaagcccc cactacatcc tcccagcaag cagttttgga gagtactcag 3120
   ggagtctctc aggttgctcc tgcagagcca gttgcagtag cacagcccca agctacccag 3180
   ccgaccactt tggcttcctc tgtagacagt gcacattcag atgttgcttc aggtatgagt 3240
   gatggcaatg agaacgtccc atcttccagt ggaaggcatg aaggaagaac tacaaaacgg 3300
   cattaccgaa aatctgtaag gagtcgctct cgacatgaaa aaacttcacg cccaaaatta 3360
   agaattttga atgtttcaaa taaaggagac cgagtagtag aatgtcaatt agagactcat 3420
   aataggaaaa tggttacatt caaatttgac ctagatggtg acaaccccga ggagatagca 3480
   acaattatgg tgaacaatga ctttattcta gcaatagaga gagagtcgtt tgtggatcaa 3540
   gtgcgagaaa ttattgaaaa agctgatgaa atgctcagtg aggatgtcag tgtggaacca 3600
   gagggtgatc agggattgga gagtctacaa ggaaaggatg actatggctt ttcaggttct 3660
   cagaaattgg aaggagagtt caaacaacca attcctgcgt cttccatgcc acagcaaata 3720
   ggcattccta ccagttcttt aactcaagtt gttcattctg cgggaaggcg gtttatagtg 3780
   agtcctgtgc cagaaagccg attacgagaa tcaaaagttt tccccagtga aataacagat 3840
   acagttgctg cctctacagc tcagagccct ggaatgaact tgtctcactc tgcatcatcc 3900
   cttagtctac aacaggcctt ttctgaactt agacgtgccc aaatgacaga aggacccaac 3960
   acagcacctc caaactttag tcatacagga ccaacatttc cagtagtacc tcctttctta 4020
   agtagcattg ctggagtccc aaccacagca gcagccacag caccagtccc tgcaacaagc 4080
   agccctccta atgacatttc cacatcagta attcagtctg aggttacagt gcccactgaa 4140
   gaggggattg ctggagttgc caccagcaca ggtgtggtaa cttcaggtgg tctccccata 4200
   ccacctgtgt ctgaatcacc agtactttcc agcgtagttt caagtatcac aatacctgca 4260
   gttgtctcaa tatctactac atccccgtca cttcaagtcc ccacatccac atctgagatc 4320
   gttgtttcta gtacagcact gtatccttca gtaacagttt cagcaacttc agcctctgca 4380
   gggggcagta ctgctacccc aggtcctaag cctccagctg tagtatctca gcaggcagca 4440
   ggcagcacta ctgtgggagc cacattaaca tcagtttcta ccaccacttc attcccaagc 4500
   acagcttcac agctgtccat tcagcttagc agcagtactt ctactcctac tttagctgaa 4560
   accgtggtag ttagcgcaca ctcactagat aagacatctc atagcagtac aactggattg 4620
   gctttctccc tctctgcacc atcttcctct tcctctcctg gagcaggagt gtctagttat 4680
   atttctcagc ctggtgggct gcatcctttg gtcattccat cagtgatagc ttctactcct 4740
   attcttcccc aagcagcagg acctacttct acacctttat taccccaagt acctagtatc 4800
   ccacccttgg tacagcctgt tgccaatgtg cctgctgtac agcagacact aattcatagt 4860
   cagcctcaac cagctttgct tcccaaccag ccccatactc attgtcctga agtagattct 4920
   gatacacaac ccaaagctcc tggaattgat gacataaaga ctctagaaga aaagctgcgg 4980
   tctctgttca gtgaacacag ctcatctgga gctcagcatg cctctgtctc actggagacc 5040
   tcactagtca tagagagcac tgtcacacca ggcatcccaa ctactgctgt tgcaccaagc 5100
   aaactcctga cttctaccac aagtacttgc ttaccaccaa ccaatttacc actaggaaca 5160
   gttgctttgc cagttacacc agtggtcaca cctgggcaag tttctacccc agtcagcact 5220
   actacatcag gagtgaaacc tggaactgct ccctccaagc cacctctaac taaggctccg 5280
   gtgctgccag tgggtactga acttccagca ggtactctac ccagcgagca gctgccacct 5340
   tttccaggac cttctctaac ccagtcccag caacctctag aggatcttga tgctcaattg 5400
   agaagaacac ttagtccaga gattatcaca gtgacttctg cggttggtcc tgtgtccatg 5460
   gcggctccaa cagcaatcac agaagcagga acacagcctc agaagggtgt ttctcaagtc 5520
   aaagaaggcc ctgtcctagc aactagttca ggagctggtg tttttaagat gggacgattt 5580
   caggtttctg ttgcagcaga cggtgcccag aaagagggta aaaataagtc agaagatgca 5640
   aagtctgttc attttgaatc cagcacctca gagtcctcag tgctatcaag tagtagtcca 5700
   gagagtacct tggtgaaacc agagccgaat ggcataacca tccctggtat ctcttcagat 5760
   gtgccagaga gtgcccacaa aactactgcc tcagaggcaa agtcagacac tggcagccct 5820
   accaaggttg gacgttttca ggtgacaact acagcaaaca aagtgggtcg tttctctgta 5880
   tcaaaaactg aggacaagat cactgacaca aagaaagaag gaccagtggc atctcctcct 5940
   tttatggatt tggaacaagc tgttcttcct gctgtgatac caaagaaaga gaagcctgaa 6000
   ctgtcagagc cttcacatct aaatgggccg tcttctgacc cggaggccgc ttttttaagt 6060
   agggatgtgg atgatggttc cggtagtcca cactcgcccc atcagctgag ctcaaagagc 6120
   cttcctagcc agaatctaag tcaaagcctt agtaattcat ttaactcctc ttacatgagt 6180
   agcgacaatg agtcagatat cgaagatgaa gacttaaagt tagagctgcg acgactacga 6240
   gataaacatc tcaaagagat tcaggacctg cagagtcgcc agaagcatga aattgaatct 6300
   ttgtatacca aactgggcaa ggtgccccct gctgttatta ttcccccagc tgctcccctt    6360
   tcagggagaa gacgacgacc cactaaaagc aaaggcagca aatctagtcg aagcagttcc    6420
   ttggggaata aaagccccca gctttcaggt aacctgtctg gtcagagtgc agcttcagtc    6480
   ttgcaccccc agcagaccct ccaccctcct ggcaacatcc cagagtccgg gcagaatcag    6540
   ctgttacagc cccttaagcc atctccctcc agtgacaacc tctattcagc cttcaccagt    6600
   gatggtgcca tttcagtacc aagcctttct gctccaggtc aaggaaccag cagcacaaac    6660
   actgttgggg caacagtgaa cagccaagcc gcccaagctc agcctcctgc catgacgtcc    6720
   agcaggaagg gcacattcac agatgacttg cacaagttgg tagacaattg ggcccgagat    6780
   gccatgaatc tctcaggcag gagaggaagc aaagggcaca tgaattacga gggccctgga    6840
   atggcaagga agttctctgc acctgggcaa ctgtgcatct ccatgacctc gaacctgggt    6900
   ggctctgccc ccatctctgc agcatcagct acctctctag gtcacttcac caagtctatg    6960
   tgccccccac agcagtatgg ctttccagct accccatttg gcgctcaatg gagtgggacg    7020
   ggtggcccag caccacagcc acttggccag ttccaacctg tgggaactgc ctccttgcag    7080
   aatttcaaca tcagcaattt gcagaaatcc atcagcaacc ccccaggctc caacctgcgg    7140
   accacttag                                                            7149
<210> 74
   <211> 4204
   <212> DNA
   <213> Homo sapiens
<400> 74
   ccggggctga gctgcggcgg cgcctgacaa tgccccctcg cgccccggcc tcggcgcccg 60
   ctcccggctc cagcgcgcct gcacgtacgt agccttcacg tgtgtgtgga tacggagtgc 120
   atgtgtggag acagaggaat atgctccaac aaatttcgga tagaggcgcc gaagacagga 180
   cgtggggaag ctaaagaagt aaacaggtca tggcacgttt aacaaaaaga cgacaggcgg 240
   atacaaaagc tatccagcat ctttgggcag ccattgagat tatacggaac cagaagcaga 300
   ttgccaacat tgaccgtatt acaaagtata tgtctcgagt ccacggtatg caccctaaag 360
   agaccacccg tcagctgagc ttagctgtga aagatggtct tattgtcgaa actctaacag 420
   tgggctgcaa aggttcaaaa gctggtattg aacaagaagg atattggttg ccaggagatg 480
   agattgactg ggaaacagaa aatcatgact ggtattgttt tgaatgccat ttgcctggag 540
   aggtgttgat atgtgacctg tgttttcgtg tgtatcattc caagtgtttg tctgatgagt 600
   tcaggcttag agacagcagt agtccctggc agtgcccagt ttgcaggagc attaagaaga 660
   agaatacaaa caaacaggag atgggcacat acctcagatt cattgtctcc cgcatgaagg 720
   agagggctat agatcttaat aaaaagggga aggacaataa acacccgatg tacaggaggc 780
   tggtgcactc agctgtggac gttcccacca ttcaagagaa agtgaatgaa gggaaatacc 840
   gaagttatga agagttcaaa gctgatgccc aattgcttct ccacaatacc gtgattttct 900
   atggagcaga cagtgagcaa gctgacattg cgaggatgct atataaagac acatgtcatg 960
   agctggatga actgcagctt tgcaagaatt gcttttactt gtcaaatgct cgtcctgaca 1020
   actggttctg ttatccttgt atacctaatc atgagctggt ttgggctaaa atgaaaggtt 1080
   ttgggttttg gccagccaaa gtcatgcaga aagaagacaa tcaagtcgac gttcgcttct 1140
   ttggccacca ccaccagagg gcctggattc cttctgaaaa cattcaagat atcacagtca 1200
   acattcatcg gctgcacgtg aagcgcagta tgggttggaa aaaggcctgt gatgagctgg 1260
   agctgcatca gcgtttccta cgagaaggga gattttggaa atctaagaat gaggaccgag 1320
   gtgaggaaga ggcagaatcc agtatctcct ccaccagtaa tgagcagcta aaggtcactc 1380
   aagaaccaag agcaaagaaa ggacgacgta atcaaagtgt ggagcccaaa aaggaagaac 1440
   cagagcctga aacagaagca gtaagttcta gccaggaaat acccacgatg cctcagccca 1500
   tcgaaaaagt ctccgtgtca actcagacaa agaagttaag tgcctcttca ccaagaatgc 1560
   tgcatcggag cacccagacc acaaacgacg gcgtgtgtca gagcatgtgc catgacaaat 1620
   acaccaagat cttcaatgac ttcaaagacc ggatgaagtc ggaccacaag cgggagacag 1680
   agcgtgttgt ccgagaagct ctggagaagc tgcgttctga aatggaagaa gaaaagagac 1740
   aagctgtaaa taaagctgta gccaacatgc agggtgagat ggacagaaaa tgtaagcaag 1800
   taaaggaaaa gtgtaaggaa gaatttgtag aagaaatcaa gaagctggca acacagcaca 1860
   agcaactgat ttctcagacc aagaagaagc agtggtgcta caactgtgag gaggaggcca 1920
   tgtaccactg ctgctggaac acatcctact gctccatcaa gtgccagcag gagcactggc 1980
   acgcggagca caagcgcacc tgccgccgga aaagatgaag ctggcccttc ccggagtcac 2040
   cccgatgatt actcttttca gacacagcgg tttttgtttc caagaagcca aaattgttta 2100
   gaatttgctt cccattttgc accagccttt aaacactttt cgtgaagaaa ttttgcacag 2160
   tagtttaaat cttttgttaa tgctcctccg aagtttttca gggggtaaaa gtaacatcag 2220
   tggagggtat tattttaaat aaattttaat tgagaatttg ttgcattttc agcaaatttt 2280
   aaaacatttt taggttttac agagatttta acctttaaac aacagatctt taaaaaacag 2340
   gtgaatacaa gtgagtttaa caaagaaaca tttagaatag atctgaatgt aagaactaca 2400
   gaactgtttc agaaataaaa catactacct tgatgtgaca tttttttctt aaccttgttg 2460
   agctggtttt gttcagctta atttactgtt caaaggcatt atctgttggt cacaccagtg 2520
   ggtatatgat tgaatttagg gaacagggtt gacacagcag ggctagtcct gcatattttt 2580
   tcttaaatat ttcccaattg tgtttttcat tatttctttt caatatataa cttttataac 2640
   aaattattag ctttgatctt gtagtttaaa attgcaggga actggggtaa tcttttactg 2700
   agctggatct tagagaaaat gaatatttaa attttaaagt ttgcacattt catctttgtc 2760
   ctaacatgag tgcttgtaac aaaataaaca acaaaaacaa agccaaaaac tacctttatc 2820
   catatgtgaa attatagatg aggcatacga atttgtttaa tgcttccctt cccttcccac 2880
   atatcatctc actgcctatt atctggtgtc acctcatgta tcgtaagtta atactaaaag 2940
   aagagaaagc acttaagttt cacagaagcc gttatgtttg tagtaatggg tcattgccta 3000
   ctaatgaact ccatcactgt acacagaatg aagaataatg catgttaatt ttcttgtatt 3060
   aaagatgccg tgatttgtaa aaagtctgta ttttgcggaa tgtctggatt aagaagcatt 3120
   accaatagga atggatcgat agttgaataa tgatttttta tacatagata tataaaatac 3180
   agccaggaaa acttaaatta cttttctttt aaaatatctc acaatttatg tggtattttt 3240
   aaagactgat cttagaccaa gtcaaattcc catttatcat ttagttattt tttgaggtta 3300
   gagaataatt tgtacatatt tatttagacc tttgatattt attaaagcaa ttactcacaa 3360
   tggaagtgaa agaatcagga gaaaagcctt ttaaaaagta ttctccaggt ttgtcagggt 3420
   cactctaaag ataaaaatgt aactaagtct tctgtgaaat atcatccatc taatcttgat 3480
   gctgttgcag atggtggtga cacaagttaa ttgacaaact actgccaaat ggtgcacaat 3540
   attttgtaaa aagtacccag tagccccatt tcatacaatg tacctaaatt atgcagtaac 3600
   ttggcatcat cgttccctcc ttgttgctgt gtaattagtc agtgttgcca cagtgtgtgg 3660
   cgctgatgga gatgtcagaa ccgagaacac ttaaccttct ttgattgttt ttcaagtttt 3720
   aagacttcga tccaccccta tgagagcaag taattgtgga aatatttttg gtgtaaaatc 3780
   attccagagt atgtaatatt taactgatag ctgcatgaaa gtgagattcg tgttactttg 3840
   gcttttctgt ctctgttgac acggttgcac atttccaagt tactacagcg tgaaaactgt 3900
   gtgtttaaaa aaaaacaaaa ttaaaaaaag agattgtggc ctggttttgt aaaagtttta 3960
   ggtaaaatta caattgattg ttttaggaat cattattaaa aattttctgc aaatcataaa 4020
   gctatatcga ggtgttgagc ttagccacta tgcacattgt aattattcat tgtggctgtc 4080
   cagttctatg atgcattctg gcattttgta agctgctctg actagcaata tatagattca 4140
   tttaatgtgt taacattggt taataaatgt atttaaaaaa aactaaaaaa aaaaaaaaaa 4200
   aaaa 4204
<210> 75
   <211> 1109
   <212> DNA
   <213> Homo sapiens
<400> 75
   actggaagac caggcagccc agctgaaggc agtaagctcg gctcacagtc gcaggagagt     60
   tctggggtac acgggcaaag gggcttgaga aggcccggag gcgaagccga agagaagcaa    120
   ctgtgccccg gagaagagaa gctcgcccat tccagactgg gaaccagctt tcagtgaaga    180
   tggcagggcc agaactgttg ctcgactcca acatccgcct ctgggtggtc ctacccatcg    240
   ttatcatcac tttcttcgta ggcatgatcc gccactacgt gtccatcctg ctgcagagcg    300
   acaagaagct cacccaggaa caagtatctg acagtcaagt cctaattcga agcagagtcc    360
   tcagggaaaa tggaaaatac attcccaaac agtctttctt gacacgaaaa tattatttca    420
   acaacccaga ggatggattt ttcaaaaaaa ctaaacggaa ggtagtgcca ccttctccta    480
   tgactgatcc tactatgttg acagacatga tgaaagggaa tgtaacaaat gtcctcccta    540
   tgattcttat tggtggatgg atcaacatga cattctcagg ctttgtcaca accaaggtcc    600
   catttccact gaccctccgt tttaagccta tgttacagca aggaatcgag ctactcacat    660
   tagatgcatc ctgggtgagt tctgcatcct ggtacttcct caatgtattt gggcttcgga    720
   gcatttactc tctgattctg ggccaagata atgccgctga ccaatcacga atgatgcagg    780
   agcagatgac gggagcagcc atggccatgc ccgcagacac aaacaaagct ttcaagacag    840
   agtgggaagc tttggagctg acggatcacc agtgggcact agatgatgtc gaagaagagc    900
   tcatggccaa agacctccac ttcgaaggca tgttcaaaaa ggaattacag acctctattt    960
   tttgaagacc gagcagggat tagctgtgtc aggaacttgg agttgcactt aaccttgtaa   1020
   ctttgtttgg agctggcacc tcttgaaata aaaaggagga tgcacgagct ggcaggcatg   1080
   caaaaaaaaa aaaaaaaaaa aaaaaaaaa                                     1109
<210> 76
   <211> 5860
   <212> DNA
   <213> Homo sapiens
<400> 76
   atgcaggaga gccagaccaa gagcatgttc gtgtcccggg ccctggagaa gatcctagcc   60
   gacaaggagg tgaagcggcc ccagcactcc cagctgcgca gggcctgcca ggtggcgctc  120
   gatgaaatta aagcagaaat agaaaagcag aggcttggca ctgctgcacc accaaaggca  180
   aacttcattg aagctgacaa gtattttctt ccattcgagc tagcttgcca gtccaagtcc  240
   ccaagggtag tcagcacatc ccttgactgc ttgcagaaac tcatcgcata cgggcacatc  300
   actggcaacg cccctgacag tggagcccct gggaagcggc tgatcgacag aattgttgaa  360
   accatttgca gttgttttca gggccctcag actgatgaag gggttcagtt acaaataatt  420
   aaggctcttc tgactgcagt gacttcccca cacattgaaa ttcatgaggg tactatcctg  480
   cagacagtga gaacatgtta caatatctat ttggccagca aaaatctcat caatcaaacc  540
   actgccaagg ctacccttac tcagatgctg aacgtcattt tcacccgcat ggaaaaccaa  600
   gtgttgcagg aggccagaag actggaaaaa ccaatccagt caaaacccca gtcccctgtg  660
   atccaagctg cagcagtatc cccaaagttc gttcgtttga agcacagtca ggcacaaagc  720
   aaaccaacaa ctcccgaaaa aacagattta accaacggtg aacatgccag gagtgattct  780
   ggaaaagtaa gcacagaaaa tggagacgca cccagagaaa gaggctcatc actgtcaggg  840
   actgatgacg gagcccagga ggtggtgaag gacatcttgg aagatgtagt cacatctgcc  900
   attaaagaag cagcggaaaa gcatggtctg acagaacctg agagagttct aggtgaactg  960
   gagtgccagg aatgtgctat tcccccagga gttgatgaaa actcacagac caacgggata 1020
   gccgatgaca ggcagtcctt gtcgtcagca gataatctgg aatcggatgc acaaggacat 1080
   caagtggctg ccaggttctc ccacgttctg cagaaggatg ccttccttgt gttccgctcc 1140
   ctgtgcaagc tgtccatgaa accccttggt gaaggccctc cagacccaaa atcccatgag 1200
   ctgcgttcca aggtggtttc cctgcagctg ctcctctctg tgttgcaaaa tgctggcccc 1260
   gtattcagga ctcacgagat gttcatcaat gcaatcaagc aatatctctg tgtggccttg 1320
   tccaaaaacg gcgtctcttc agtgcctgat gtctttgagc tctctcttgc catttttctt 1380
   actcttcttt caaactttaa aatgcacttg aaaatgcaga tagaggtctt tttcaaagag 1440
   attttcctga acattttaga aacatcaaca agttcttttg agcacaggtg gatggtcatt 1500
   cagactctga cgaggatctg tgcagatgcc cagtgtgttg tggatattta tgtcaactac 1560
   gactgtgatt taaatgctgc taacattttt gagcgccttg taaatgattt atccaaaatt 1620
   gctcagggaa gaagtggaca tgagctggga atgacacctc tgcaggagct cagcctgagg 1680
   aagaaaggcc tggagtgcct cgtgtccatt ctcaagtgca tggtggagtg gagcaaagac 1740
   ctgtatgtga atcccaacca ccagaccagc ctcggtcagg agaggctcac ggatcaggaa 1800
   ataggggatg ggaaaggcct tgacatggca agacggtgta gtgtgacgtc catggagtcc 1860
   acagtgtcct cggggaccca gacaactgtt caggatgacc ctgagcaatt tgaggtcatc 1920
   aagcaacaaa aagaaatcat tgaacacggc atcgagctgt tcaacaagaa acccaagagg 1980
   gggatccagt ttctccagga gcagggcatg ctgggaacgt cagttgaaga catagcccaa 2040
   ttcctgcacc aggaggagcg cctggattcc acccaagtag gcgattttct gggagatagc 2100
   gcaaggttca acaaggaggt gatgtatgcc tacgtggacc aacttgactt ctgtgaaaaa 2160
   gaatttgtct cagccctgcg gacattccta gaaggtttcc gcctacctgg agaagcccaa 2220
   aagattgacc gattaatgga gaagtttgcc gcaagataca tagaatgcaa ccaagggcaa 2280
   actctgtttg ctagtgctga cactgcttat gtcctagcgt attcaattat tatgctgact 2340
   acagacttgc acagtcctca ggtaaaaaat aaaatgacga aagagcagta tattaaaatg 2400
   aatcggggta tcaatgatag taaagatctg ccagaagagt atctctcaag catctatgaa 2460
   gagatagaag gcaagaaaat tgcaatgaaa gaaacaaaag agctaacgat tgcaaccaaa 2520
   tctactaagc agaatgtagc tagtgaaaag cagcggcggc tgctgtacaa cttagagatg 2580
   gagcaaatgg ctaaaacagc caaagctctg atggaggctg tgagccatgc caaagccccg 2640
   tttaccagtg ccactcacct ggaccatgtc cggccaatgt tcaaactggt gtggacgcca 2700
   ctattggcag cctacagcat cggactccag aactgtgatg acactgaagt ggcctccttg 2760
   tgtttggaag gcatccgatg tgcaatccga atcgcctgca tctttggaat gcagctggaa 2820
   cgagatgcct atgttcaggc tcttgctcgc ttctccctac tcacagccag ctccagcatc 2880
   acaaaaatga agcagaaaaa catcgacacc attaagacgc ttatcacagt ggctcacacc 2940
   gatggcaact accttgggaa ttcctggcat gagatcttga aatgcatcag ccagctggag 3000
   ctcgctcagc tgataggaac cggtgtgaag acgcgctacc tgtctggatc tgggcgtgaa 3060
   agagaaggga gcctgaaggg ccacacattg gcaggagaag agttcatggg ccttggcctc 3120
   ggtaatttgg tgagtggcgg agtgaataaa agacagatgg ccagcttcca agaatcggtt 3180
   ggtgagacca gctcgcagag tgtggttgta gctgtggaca ggatttttac tgggtctacc 3240
   agactggatg gaaatgcaat agttgacttt gtccgctggc tgtgtgctgt gtccatggat 3300
   gaactggctt ccccccacca tcctcgcatg ttcagcttgc agaagattgt ggagatatca 3360
   tactacaaca tgaatcggat ccgactacag tggtctcgaa tatggcatgt gattggagat 3420
   cacttcaata aggttggctg caaccctaat gaagatgtgg ctatctttgc tgttgactca 3480
   ttaaggcaac tctccatgaa gtttcttgag aagggtgaat tagccaactt ccgtttccag 3540
   aaagattttc tgaggccctt tgagcatatt atgaagaaaa acaggtctcc caccatccgg 3600
   gacatggcga tccgctgcat tgcccagatg gtgaactccc aggcggccaa catccgctca 3660
   ggctggaaga acatctttgc cgtgttccac caggcagcct ctgatcatga tgggaacatt 3720
   gtggagctgg ccttccagac cacttgccac attgtcacaa ctattttcca gcaccatttt 3780
   cctgcagcca tcgattcctt tcaggatgct gtgaagtgct tatcagagtt cgcctgcaac 3840
   gccgctttcc ctgacacgag catggaagcg attcggctca tccgcttctg tggcaaatac 3900
   gtctctgaga ggcctcgggt gctacaagaa tacacaagtg atgacatgaa tgtagctcct 3960
   ggtgacagag tctgggtccg aggctggttc cccatcttat tcgaactctc ctgcatcatt 4020
   aatagatgca agttagatgt acgaacaagg ggactcacag tcatgtttga gatcatgaag 4080
   agctatggcc acacctttga aaagcactgg tggcaggacc tgttcagaat cgtgtttcgg 4140
   atttttgaca atatgaaact ccctgagcaa ctgtcagaga aatctgagtg gatgacaaca 4200
   acctgcaatc acgcacttta tgctatttgt gatgttttta cccagtttta tgaagctttg 4260
   aatgaagttc ttctttctga tgtatttgca caattgcagt ggtgtgtcaa acaagataat 4320
   gaacagttgg cgcgatcagg tacaaattgc ttagaaaact tagtaatatc caatggagag 4380
   aaattcagtc ctgaagtctg ggatgaaacc tgcaactgta tgttggatat tttcaaaaca 4440
   accatcccac atgttttgct gacatggaga cctgtaggaa tggaggaaga ttcatcagaa 4500
   aagcatttgg atgtggatct ggaccgccag tctttaagca gcatagataa aaatccctct 4560
   gagaggggac agagccagct ctctaaccca acagatgaca gctggaaggg tagaccatac 4620
   gcaaatcaga aactgtttgc cagcctcctc atcaagtgtg tggtccagtt ggaattgata 4680
   cagaccattg acaacattgt gttctaccct gcgacgagca aaaaggagga tgcagagcac 4740
   atggttgccg cccagcaaga cacgctggat gcagatatcc acatagagac ggaggatcag 4800
   ggcatgtata agtacatgtc ttcccagcac ctcttcaagc tgttggactg tttgcaggaa 4860
   tcccattcat tctcaaaggc cttcaactcc aattacgagc agcggactgt cctgtggcga 4920
   gcaggtttta agggcaagtc taaacccaat cttctaaaac aagaaaccag cagcctggcc 4980
   tgttgtttga ggatcctgtt tcgaatgtat gttgatgaga accgcaggga ttcctgggaa 5040
   gaaatacagc agagactttt aactgtttgc agtgaagctc ttgcctattt catcactgtg 5100
   aattctgaga gccatcggga ggcctggaca agtctcttgt tgttacttct aactaaaacc 5160
   ctcaaaataa atgatgaaaa gttcaaagca catgcttcaa tgtactaccc ctacttgtgt 5220
   gaaattatgc agtttgacct gatccctgag ctccgagcag ttctgcggaa gttcttccta 5280
   cggataagtg ttgtgtataa gatatggata ccagaagagc catcacaggt accagcagca 5340
   ctgtcaccag tgtggtagcc ctggctgccc aggccagtgc tgcagctctg cagaatgttc 5400
   agcatgccat ttctgactgg cacatctcgt gaagtttcat agaaacaagg agttggcatc 5460
   ttggatctca gaatggcctg gaaacggatg gcctctacgc tgttccatca cagtctccaa 5520
   ctaaggctta tggtatttca ttaaactgtt gcatacccag ttagcacagt aggtggggag 5580
   tctgcttcat ttctatcatt ccatttttct gattaaactg tcaaatctgt cattgcatat 5640
   gccatcgttt tctagcaaaa tcccatgatt ggctataaac gttttgtaag aagtcactct 5700
   ccttgaaaat actgaacata gctgtatagg tttgtgatta ttagagaata tgttaataaa 5760
   acttcttgta acggctaact gccacctaaa atatgctggg ttttctgttg tttgagtgtg 5820
   ttagagaaat ttgaatgttt ttgtcagtta cgagtcagcc 5860
<210> 77
   <211> 2034
   <212> DNA
   <213> Homo sapiens
<400> 77
   gtaagagtta gggtttgacc ggagctagtg aggatccagc ccaggctcca gttgtccgcg 60
   gttcctcctc ctgtcgctgc ccgtgtgatt actgagactc agatggacca cacatccccg 120
   acctacatgc ttgctaactt aacccacttg cattctgaac aacttctgca gggcttgaat 180
   cttcttcgcc aacatcacga actctgtgac atcattcttc gagtaggtga tgttaaaatt 240
   catgctcaca aagtggtact tgccagcgtc agcccgtatt tcaaagctat gttcactgga 300
   aacctttctg aaaaagagaa cagtgaggtt gagtttcaat gcattgatga aactgctctc 360
   caggccattg tggagtatgc ctatacaggg actgttttta tttctcagga cacagttgaa 420
   tctctcctgc cagcagcaaa cctactccag ataaaacttg tcctgaaaga atgttgtgca 480
   tttcttgaaa gccaacttga tcctggtaat tgtattggaa tttctcgttt tgcagaaaca 540
   tatggttgcc gtgaccttta tttggcagcc actaaataca tatgccagaa ttttgaagct 600
   gtttgccaga ctgaagagtt ttttgagctt acacatgctg acttggatga aattgtttcc 660
   aatgactgtt tgaatgtagc taccgaagag actgtttttt atgcattaga gtcttggatc 720
   aagtatgatg tacaagaacg ccagaaatac ttagcacagt tactaaacag tgtacgatta 780
   ccattgttga gtgttaagtt tctcactaga ctatatgaag caaatcatct tattcgtgat 840
   gatcgcactt gtaaacatct tttgaatgaa gccctaaagt accactttat gcctgaacat 900
   agactctctc atcagacagt cttgatgaca cgacctcgct gtgctcccaa agtactttgt 960
   gcagtaggag ggaaatctgg actctttgcc tgtttggata gtgtggagat gtactttcct 1020
   cagaatgact cttggattgg tttggcaccc ctaaacattc ctcgctatga atttggaata 1080
   tgcgttttag accaaaaagt atatgttata ggtggtattg caactaatgt gcgtcctggc 1140
   gtcactatca gaaaacatga aaattcagtg gaatgctgga atcctgatac aaatacttgg 1200
   acttctctag agagaatgaa tgaaagccga agtactcttg gagtagtagt acttgcagga 1260
   gaactttatg ccttaggtgg ttatgatgga caatcttatt tacaatctgt agagaagtac 1320
   attcccaaaa taagaaaatg gcaacctgtg gcaccaatga cgacaacaag aagttgtttt 1380
   gctgcagcgg tattggatgg aatgatatat gccattggtg ggtatggtcc tgcccacatg 1440
   aacagtgtgg agcgttatga tccaagtaag gactcctggg agatggttgc atccatggca 1500
   gataaaagga ttcactttgg cgtgggtgtc atgctaggct ttatttttgt ggtgggtgga 1560
   cataatggag tctcacattt gtccagcatt gaaagatacg atcctcatca aaatcagtgg 1620
   actgtgtgta gaccaatgaa agaacctaga acaggagttg gtgctgctgt aatcgataac 1680
   tacctttatg tcgtcggtgg tcactcaggg tcttcctatc tgaatacagt gcagaaatat 1740
   gatcctatct cagatacgtg gctggattca gctggcatga tatactgtcg ctgcaacttt 1800
   gggttaactg cactttgaca aatgtgaact ctcggaaata gtatggtggt gaaacttgta 1860
   ctgcatgaac atccggatgg cccagttttc tgaaacccac aagctgcatt gctttctttt 1920
   taacttgaag tagcatgaag gctcaaaagt tttgttgggt acttttaatt gagaagtagt 1980
   tttggttgct cttgattaca cagtaaatca ataatcaaaa agaaaaaaaa aaaa 2034
<210> 78
   <211> 2943
   <212> DNA
   <213> Homo sapiens
<400> 78
   ccgaaagaga aaacaggccg cgcgggcggc agaggagccg ggcgccgcaa tggacgtgcg 60
   ggcgctgccg tggctgccgt ggctgctgtg gctgctgtgc cggggcggcg gcgatgcgga 120
   ctcccgcgcc cccttcaccc cgacctggcc gcggagccgc gagcgtgaag ccgccgcctt 180
   ccgggaaagt cttaatagac atcgatactt gaattcttta tttcccagtg aaaactccac 240
   cgccttctat ggaataaatc agttttccta tttgtttcct gaagagttta aagccattta 300
   tttaagaagc aaaccttcca agtttcccag atactcagca gaagtacata tgtccatccc 360
   caatgtgtct ttgccgttaa gatttgactg gagggacaag caggttgtga cacaagtgag 420
   aaaccagcag atgtgtggag gatgctgggc cttcagcgtg gtgggggcag tggaatctgc 480
   ttatgcaata aaggggaagc ccctggaaga cctaagtgtc cagcaggtca ttgactgttc 540
   gtataataat tatggctgca atggaggctc tactctcaat gctttgaact ggttaaacaa 600
   gatgcaagta aaactggtga aagattcaga atatcctttt aaagcacaaa atggtctgtg 660
   ccattacttt tctggttcac attctggatt ttcaatcaaa ggttattctg catatgactt 720
   cagtgaccaa gaagatgaaa tggcaaaagc acttcttacc tttggccctt tggtagtcat 780
   agtagatgca gtgagctggc aagattatct gggaggcatt atacagcatc actgctctag 840
   tggagaagca aatcatgcag ttctcataac tgggtttgat aaaacaggaa gcactccata 900
   ttggattgtg cggaattcct ggggaagttc ttggggagta gatggttatg cccatgtcaa 960
   aatgggaagt aatgtttgtg gtattgcaga ttccgtttct tctatatttg tgtgacatgt 1020
   tgggcagatc aagagacagc tacaaaaatg aaggttttca taatgcaatg taacatagta 1080
   cttcaaagta ttattcaact tcaagtttca gcaactacct acaaaagatt ctaaggccta 1140
   gtagtattta aactaagttt cagaatgttc ccttcttgta gagagatgga caaccaaagt 1200
   cagtgggaca aactccagca cagaagcctg cgaggaagcc tatggaatag tttcctgtcc 1260
   tgagacgaaa ttcagattag gagatatttt aggcccctgc aactggggaa ggctactgtt 1320
   tgtttttgtt tgcttattat ttatttgttt gtttattgtg agatatttca ggtgggatca 1380
   aagaggtcat aagaatttat tttcttttgt ggggtgtaac tactagcttt agattacccc 1440
   tatacacaag aatggccaac ctaaaattat gtgtgtcttg tacagttagt tatattagca 1500
   gccctctgag atggcgtatc tatcggaagg atttcaaaca ccaattgctt tacctgaaca 1560
   aatggtgctt accctttgaa cagcagagtg accacgtaga aggaaggaaa agggcaaaat 1620
   cgcttcagtt aaactgaaat taaatgaaca ataaggcaac tatataagta acttctagta 1680
   gcattgcctg agagacaaat tattgtttga taattttcat tgtgaatagg aatccaatag 1740
   atcatattgc ttactttgtt ctttttatac tatagaataa tattttgttc tctagtatat 1800
   caaaatacca aaatattatc tcatattttc tccctctttc tcttactctt taccaagttt 1860
   tcctggtggc ttggcttccc tgactaaaga attaagtctc atttttactt tccatttcta 1920
   ttttcttacc acttggttgg ctccctttgt ctctgtacct ttaccaacat taggatctca 1980
   cctctttctt cctcccttaa ttcataagca ccactcctat caaagtccca tctcttaacc 2040
   ctgggtatca aacaaactgt gagttttcca gaatctgttt cccagttttc ccctcagctt 2100
   tcctggtctc ccatccgaac tgcttctttg tgcacctctt gttctttctc ttggctccca 2160
   gtcttgattc ctgtgatcac tcttgcatca ctaattgcac aagtgatttc aggtgcaatt 2220
   ctgattagcc tgcgtccaca cagtgatcga tgatcctatg tgcctagaaa ggacactgtg 2280
   tgctgctcat gacctgcaac aggaaaaaag ccatttcttg ttagcagtgt aagaacctta 2340
   gagcaaagga gttgaccttc tgattgaata taagcacaac catattaaat gaatcaatac 2400
   aagaaaatta tttctgatac tatgtatgta catatttctt ctctaaaatg tatcattctt 2460
   ttctaatgta tatgatctaa caaaaatgaa acatgaaatg cagtagcaac cactaaaaaa 2520
   aaaaattcaa ggacatctaa ctttttctct cacttttgcc ctttgtttat ccttccctgt 2580
   gattagataa acaaaataaa aaacaaaatg ctgtatttct cttcttacgc cagtcagaac 2640
   caatccgaaa agaatgtgtg ttgactcagg tttggagtta tttcaggaag acagatattg 2700
   accttttaat tgataaatat tcttattatc ctggaatgcc agaaaagaac tatgtcctgc 2760
   ttgtctagtt tgtattcgct gactttctat gtgatataga tgcatttgta atactctttt 2820
   tcaagtgcta aaggatttct aaaatttcaa actgattaat atgtttctgc tgttctggat 2880
   tttgatgaca tttacaataa aacaacctac atttgacttt ggtttaaaaa gaaaaaaaaa 2940
   aaa 2943
<210> 79
   <211> 1087
   <212> DNA
   <213> Homo sapiens
<400> 79
   agtgtacacg tcggttgcct aacaaccggc agcggactcc tttggctatg gcctccaact 60
   ttaagaaggc aaacatggca tcaagttctc agcgaaaaag aatgagccct aagcctgagc 120
   ttactgaaga gcaaaagcag gagatccggg aagcttttga tcttttcgat gcggatggaa 180
   ctggcaccat agatgttaaa gaactgaagg tggcaatgag ggccctgggc tttgaaccca 240
   agaaagaaga aattaagaaa atgataagtg aaattgataa ggaagggaca ggaaaaatga 300
   actttggtga ctttttaact gtgatgaccc agaaaatgtc tgagaaagat actaaagaag 360
   aaatcctgaa agctttcaag ctctttgatg atgatgaaac tgggaagatt tcgttcaaaa 420
   atctgaaacg cgtggccaag gagttgggtg agaacctgac tgatgaggag ctgcaggaaa 480
   tgattgatga agctgatcga gatggagatg gagaggtcag tgagcaagag ttcctgcgca 540
   tcatgaaaaa gaccagcctc tattaagatc agtgtcttct ttttctactg caagcacatg 600
   taactagatt tagtgcctgc catggtgtga aatctggctt ttgagaacac aaacttttcc 660
   cccacggacc tccctttatc actttaatag tgaccttgag cctattttag ccgtttggaa 720
   gtgttctttg atattacagt tctttgtaaa atgacctgcg aattacccta attctcaaaa 780
   gcaaaacaag agcacacaag cgtgaagaaa aggatcttaa agctttgagc acctgccatt 840
   ttgccttgca tcgtttccct cgtcatgcat ttccacatat ccacaaacac agaacgactt 900
   tagacaagca catgttacac ctgtgttgcc acaagcagtc attcttgacg gctccagttt 960
   ttatttgaca cttgagttta gttttctctt ttataaaccc agtgaactcc tgcactggca 1020
   tttggatgtg tgttaatgct atttgttttg tcttaaaagt aaaacctttc tcagtttgaa 1080
   aaaaaaa 1087
<210> 80
   <211> 3202
   <212> DNA
   <213> Homo sapiens
<400> 80
   ttcccatccg caccagcaag agggcccctg tgccccaaac gcgaaccgta cggctccaga      60
   cagcaccgcg gaactcggtg gcttccagaa ggccccgcgc ctgcgcattc cgctgcctgc     120
   gcctgcgcct gcgcctgcgc cgttctcccg gccgccgcct tagcacctcc tccggacggt     180
   gtcgccgaag tctcgcgagc ccggagcgtg gcacgtgggc cccctccgcc tccggctccg     240
   tcctcctctg gccccctccg cccccggccc cggccccacg gcgggatgcg ggacaacacc     300
   agccccatca gcgtgattct ggtgagctcg gggagcaggg gcaataagct gctgttcagg     360
   taccccttcc agagaagcca ggagcacccg gcgtcccaga caagtaagcc gcgtagcaga     420
   tacgctgcca gcaacacggg cgaccatgct gatgagcagg acggcgattc caggttttca     480
   gatgttattc tggcaacaat tttggcaacc aagtctgaaa tgtgtggcca aaaatttgaa     540
   ctgaagattg ataatgtgcg atttgttggg cacccaacac tgctacagca tgctctgggg     600
   cagatctcca aaacagatcc ttccccgaag agggaagcac ctactatgat tctttttaat     660
   gtggtgtttg cactgagggc caacgcagac ccgtcagtga taaactgtct gcataacctg     720
   tcccgtcgta tcgccaccgt gctgcagcac gaggagcgcc gctgccagta cctcacccgg     780
   gaggccaagc tgatcctggc gctccaggat gaggtgtccg ccatggctga tggaaatgaa     840
   ggtcctcagt ccccattcca tcacatcctg cccaagtgca agctggccag ggacctcaag     900
   gaagcttatg acagcctgtg cacgtcgggc gtagttcggc ttcacatcaa cagctggctg     960
   gaggtgagct tctgcctgcc ccacaagatc cactatgcgg cctccagtct gatcccccca    1020
   gaggccatcg aacggagcct gaaagccatc cgcccctacc atgccctgct gctgctcagt    1080
   gatgagaagt ccttgctggg tgagcttcct attgactgct cccctgccct agtgcgggtg    1140
   atcaagacca catctgctgt gaagaacctg cagcagctag cccaagatgc ggacctggcc    1200
   ttgctgcagg ttttccagct tgcagctcat ctggtgtact ggggcaaggc catcatcatc    1260
   tacccgctgt gtgagaacaa cgtctacatg ctgtctccca atgccagcgt atgtctgtac    1320
   tccccgctgg ccgagcagtt ctcccaccag ttcccatctc atgacctgcc gtccgttctt    1380
   gccaagttct ccttgccggt ctccttgtca gaatttagga atcccctggc ccccgctgtg     1440
   caggagaccc agctcatcca gatggtggtg tggatgctgc agcgccggct tctcatccag    1500
   ctgcacacct atgtctgcct gatggcctca cccagcgagg aggagccccg tccgcgagag    1560
   gacgacgtcc ccttcactgc ccgggtcggc ggtcgcagcc tcagcacgcc caacgccctc    1620
   agctttggct ccccaaccag cagcgatgac atgaccctca ccagccccag catggacaac    1680
   tccagcgcag agctacttcc cagcggggac tcgccactga accagaggat gacggagaac    1740
   ctgctggcca gcctgtcgga gcatgaacgc gcagccatcc tcagtgtacc cgcagcccag    1800
   aaccctgagg acctccgcat gtttgccagg ctccttcact acttccgcgg ccgccaccac    1860
   ctggaggaga ttatgtacaa cgagaacacg cggcgctccc agctgctcat gctgtttgac    1920
   aagttccgca gcgtgctggt ggtgaccacc cacgaggacc ctgtcattgc cgtcttccag    1980
   gctctgctcc cctgagccca ggcggagggc ggaaggctgc tggggtgcgc aggtgggcgc 2040
   tcgcgtctcc ccaccccagg gctccccccg tgctgaggct gagccctctt ggccctgagg 2100
   cctggcattg ggtggatgcg ggctggccgt ggcccagtga agcctgcaga gccccgctgt 2160
   ccttgcccct ggtggttccg ctgtggggct gctgccctct gtgttcctac gcttccctcc 2220
   agtccttgcc gcacgcgtag gcatctccac gctggagggg ggcctgccag gactcctgtc 2280
   tctgggtgag gccgcatcct tcaaggccca tgtgggctgt gcgtttctca gaccctcctt 2340
   ctgtccctac acctgctcct tggacccccc agtctgtggc caccctgaag aatgtgcaga 2400
   aacacttgtg tggcctgtcc ctgtctctct gacagccttc catttgtgaa gtgccctgtg 2460
   gccccctccc cagcacctct gtctgccatg cgcttcttcc tcccaggcta ccctgagcct 2520
   ttcctggccc agtcctcacc acagtccaca gaagccacaa acaggctcat agccaagctg 2580
   tgacctggtc ctgaccatct ggggcacgag ggcctgggct ggccctgcta ggctggagaa 2640
   gccctgtcac ctgtgcacat cttgctggtg gaggcatggc ccactgtgca ggaccccacc 2700
   ctcgggggct ttcggctccc acactgatga ttctccccag catccacacc gggcctggcg 2760
   ctacgtactc aggcccccag ctctcgtgtc ctggaggaag agctagctcc agacatgggt 2820
   tgatcaccta gaggagctct ggctaaggca cagttttcta gaaataaaac atttattcgg 2880
   gtttggaaag gccccttgga ttcctggctg gggacaatga tgacctggac cctggccaga 2940
   agagccctgg ccctccagca aggcagcacc tgctctgatg cacctgtgca cccctggccc 3000
   tcccagccac ctgggagccc gaagcttagc ctgtaggtgg ccaagccagt ccctactctt 3060
   gggctgcggc cacgtgaggc tccgcatcag cagccagggg cgagcactag tggacaaagc 3120
   cagcaaatgc ggcgttcctg tgagcagata ccctcaacca gccagctgtc aaaagtattt 3180
   caagataaaa tccaggccaa gc 3202
<210> 81
   <211> 2032
   <212> DNA
   <213> Homo sapiens
<400> 81
   gttacttttt aaatggacaa aagtggctct caatccagca catgcacatt gaagcaagtt 60
   aaaggattta atatgaagca cagaagcaga tagtgccaaa tagcaagcag tagttgttac 120
   acatttctgg aaaagcagtg tccgtgttgt tatgtacacc tccaaaaaaa gttcaaatgt 180
   gtaggggaat tgttgtgtaa agtaaactga actgcagggt agcagtattt taatagctat 240
   tatagacctg tatttactgt atttaaatga gtaaaggaac aagcagtgac acatcactag 300
   gaagggtgag cagggcagca tttccttctc ccactgctgc tgagatggca gaaattagtc 360
   gaattcagta cgaaatggaa tatactgaag gcattagtca gcgaatgagg gtcccagaaa 420
   agttaaaagt agcaccgcca aacgctgacc tggaacaagg attccaagaa ggagttccaa 480
   atgctagtgt gataatgcaa gttccggaga ggattgttgt agcaggaaat aatgaagatg 540
   tttcattttc aagaccagca gatcttgacc ttattcagtc aactcccttt aaacccctgg 600
   cactgaaaac accacctcgt gtacttacgc tgagtgaaag accactagat tttctggatt 660
   tagaaagacc tcctacaacc cctcaaaatg aagaaatccg agcagttggc agactaaaaa 720
   gagagcggtc tatgagtgaa aatgctgttc gccaaaatgg acagctggtc agaaatgatt 780
   ctctgtggca cagatcagat tctgccccaa gaaataaaat ttcaaggttc caggcaccga 840
   tttctgcacc ggagtacact gtgacaccat cgccacaaca ggctcgggtc tgtcctcccc 900
   atatgttacc tgaagatgga gctaatcttt cctctgctcg tggcattttg tcgcttatcc 960
   agtcttctac tcgtagggca taccagcaga tcttggatgt gctggatgaa aatcgcagac 1020
   ctgtgttgcg tggtgggtct gctgccgcca cttctaatcc tcatcatgac aacgtcaggt 1080
   atggcatttc aaatatagat acaaccattg aaggaacgtc agatgacctg actgttgtag 1140
   atgcagcttc actaagacga cagataatca aactaaatag acgtctacaa cttctggaag 1200
   aggagaacaa agaacgtgct aaaagagaaa tggtcatgta ttcaattact gtagctttct 1260
   ggctgcttaa tagctggctc tggtttcgcc gctagaggta acatcagccc tcaaaaatac 1320
   tgtctcaaca gctggaaata taaaagattt gcaaacttct ttgtttctgt ctctgcattg 1380
   tatgccattt tatagtccac accctgaaaa tgtatttctt ccagaaagtc tggaggaagg 1440
   acctatattt gtagaagtaa aggtatattc tgtcactcag ctgtattcac gtctgagcag 1500
   ttctgcagta acacctgctt aaaattctcc ctttgcatgt tttgtaaata ggctccagtt 1560
   ttgtttttta aaaggaattt attttttgcc tcatcagtcc acccaactga ttctgaatgg 1620
   gagagagtct gtagagaatt gattcagaaa agtgtctgtg aaagaaaaac aattattttg 1680
   tcctgtttct caaacagtgt taagcagttt tgttaataga catttttgca tcgacacttc 1740
   aacattaaca ctttgaaagt catggtctgg tgccagattt aagaaactcg aaccacctaa 1800
   tatttcataa ccttcttcat taggtacttg tacagattaa tttctaacat tgcagcagtt 1860
   tcatatgtgt gcaatatgtg cattctttca ttttagtttt gcacttggtt ttctataaag 1920
   tacgttttta ctcagttcat gcgtgaacaa tttaaaaaac gacagaataa ggtacaaatg 1980
   tagtgtattt aataaactgt caaccaaaga aaaaaaaaaa aaaaaaaaaa aa 2032
<210> 82
   <211> 3426
   <212> DNA
   <213> Homo sapiens
<400> 82
   aattccgggc ggcggcggcc gaggctgaag gaagatggcg gacggcgtgg accacataaa 60
   catttacgcg gatgtcggcg aagagttcaa ccaggaagct gaatatggtg ggcatgatca 120
   gatagatttg tatgacgatg tcatatctcc atctgcaaat aatggagatg ccccagaaga 180
   ccgagattac atggatactc tcccaccaac tgttggtgat gatgtgggta aaggagcagc 240
   accaaatgtt gtctatacat atactggaaa gagaattgca ttatatattg gaaatctaac    300
   atggtggaca acagatgaag acttaactga agcagttcat tctttgggag taaatgatat    360
   tttggagata aaattttttg aaaatcgagc aaatggccag tcaaaggggt ttgcccttgt    420
   tggtgttgga tctgaagcat cttcaaaaaa gttaatggat ctgttaccta aaagagaact    480
   tcatggtcag aatcctgttg taactccatg caataaacag ttcctgagtc aatttgaaat    540
   gcagtccagg aaaactacac aatcaggaca aatgtctggg gaaggtaaag ctggtcctcc    600
   aggaggcagt tcccgtgcag catttccaca aggtggtaga ggacggggcc gttttccagg    660
   ggctgttcct ggtggggaca gatttcctgg gccagcagga ccaggagggc cacccccacc    720
   ttttccagct ggacagactc caccacgtcc acccttaggt cctccaggcc cacctggtcc    780
   accaggtcct ccacctcctg gtcaggttct gcctcctcct ctagctgggc ctcctaatcg    840
   aggagatcgc cctccaccac cagttctttt tcctggacaa ccttttgggc agcctccatt    900
   gggtccactt cctcctggcc ctccacctcc agttccaggc tacggccccc ctcctggccc    960
   accacctcca caacagggac cacctccacc tccaggcccc tttccacctc gtccacccgg   1020
   tccacttggg ccacccctta cactagctcc tcctccgcat cttcctggac cacctccagg   1080
   tgccccaccg ccagctccgc atgtgaaccc agctttcttt cctccaccaa ctaacagtgg   1140
   catgcctaca tcagatagcc gaggtccacc accaacagat ccatatgggc gacctccacc   1200
   atatgatagg ggtgactatg gcccccctgg aagggaaatg gatactgcaa gaacgccatt   1260
   gagtgaagct gaatttgaag aaatcatgaa tagaaatagg gcaatctcaa gcagtgctat   1320
   ttcgagagct gtgtctgatg ccagtgctgg tgattatggg agtgctattg agacactggt   1380
   aactgcaatt tctttaatta aacaatccaa agtatctgct gatgatcgtt gcaaagttct   1440
   tattagttct ttgcaagatt gccttcatgg aattgagtcc aagtcttatg gttctggatc   1500
   aagacgtgaa cgatcaagag agagggacca tagtagatca cgagaaaaga gtcgacgtca   1560
   taaatcccgt agtagagacc gtcatgacga ttattacaga gagagaagca gagaacgaga   1620
   gaggcaccgg gatcgtgacc gagaccgtga ccgagagcgt gaccgagagc gcgaatatcg   1680
   tcatcgttag aagctgaagg aagaggatca ccttccaaga caaaacagtc ttcatgggcc   1740
   aaaaatgacg cttgtccagc agtttgcttc ttgtgattga actgaacctg taaggattca   1800
   tggataaaat gaacaggaat agatctgaat aaagcaaatc tgcataaatg gtaaccagta   1860
   gctctacttt tattttttat gttgcttaac tgttttattt gaaggaaacc tgtgtgattt   1920
   aaaaagttat agcttttgca actttattac tggttatata catttggcca ttatgatgtg   1980
   caagcaattg gaaaaaaagt caagtaaatg cttgtttttg tagtagtttg ttcttgttaa   2040
   aaatgtttat atgataatgt ctgtaaacag catcactttg attacaatag atgtagtgtt   2100
   gtaataaact gtttaatggg gctgatgtgt aaagctgttc aagttatttg atgtttacac   2160
   ctcagggaaa gtcttgtgtt cagcaatatc taaagataat gttactatga caacattttt   2220
   actgtccttt aaagcattgc aatagcgttt ttggatatgc ctcaatctaa tcttgcgttc 2280
   agtgaattaa acatagtaat taagtgtctt ttgcccttga ttttgatatt agaataggtg 2340
   attacatgga tatttaatat ttctatattc tgcttttcta gctgttttta cctagttagc 2400
   ttgtgacttt gctgaatggt atgtaaactt gtaaaaatag agatttgaca gacatagcaa 2460
   tctagtcaat gtgtaagggg tcaaaaaaaa cagaggtttt aacacataag taaaaacccg 2520
   tacatatttg atgtgtaatg caggttaatt acaacacaga tgtaccgaaa cacttaattg 2580
   tgaaccgcta acattgaaga aattttgaca attccgattt gatgctgcaa ttacttgctg 2640
   tttttattga tcttatggtt tatttcttaa gccatagtca gtgtaaatac agccctgcag 2700
   caggtaaatg tgagtaaaga gagccttata ttttccaatt ggtataaaat ttttgaagga 2760
   tgtgatgttc attaacattc ggttgtattc cccagtattt gtaatgggaa attacagata 2820
   aaccgtgtct gcacagttta aggaatacta tgtatattca tgcaccgtat tgattcatgc 2880
   tatagttact taatcaaaga tttttttcaa acctgcctta catataggcc cactttaaaa 2940
   gcacctgact agcatgtgtt cttgattgca aaattggcag aggcagggtg tcaacttgat 3000
   taggtgtttt tatgggaatg taatttgaaa tcactacttc agaaatttga cttaaaattc 3060
   ttgagcacgt taatatgttt ttaagatctg attatctttg agagatcttc tgttaataca 3120
   cattggttgt taaagagtac ccaaattcta ggacaatgct taaagtgtta aaatacccta 3180
   gatactgtgt tatgtgcaac tgtagaaacc ctccagaaat ttccactgct gttcttcact 3240
   ttcatcttgt ctgctatcaa accacttctg acaaaattag ctgttttgaa ttacccatat 3300
   cactgccagt tttattttaa aatattttgt gtttgaagta tctgtgcatg ggatcgttga 3360
   tgtttatcag aactgttcac tttcagaaat gattttttaa agcattttgt tgaaatgcgg 3420
   ttgctt 3426
<210> 83
   <211> 5601
   <212> DNA
   <213> Homo sapiens
<400> 83
<210> 84
   <211> 2271
   <212> DNA
   <213> Homo sapiens
<400> 84
   gcctcgcggc aggagcggga tttccggggt cacgggaacc ggcaggggaa cgggataaag      60
   ttcccggaga aaggaaagga gagcgtggga tagtaaaaga gaagacgcgg agaagaggag     120
   aggacctaca agaacggagg acaggggcgc acgatggtcc cggggggagc ggaaacaaag     180
   gcacgcaaaa cggaaaagcg tgtgtagggg agcggaaaag gaagtcacca ccgtggcctg     240
   cgacgaaatg gcgaaaagtc ttttgaagac agcctctctg tctggaagga caaaattgct     300
   acatcaaaca ggattgtcac tttatagtac atcccatgga ttttatgagg aagaagtgaa     360
   aaaaacactt cagcagtttc ctggtggatc cattgacctt cagaaggaag acaatggcat     420
   tggcattctt actctgaaca atccaagtag aatgaatgcc ttttcaggtg ttatgatgct     480
   acaacttctg gaaaaagtaa ttgaattgga aaattggaca gaggggaaag gcctcattgt     540
   ccgtggggca aaaaatactt tctcttcagg atctgatctg aatgctgtga aatcactagg     600
   aactccagag acttccttta ataagtgttg cgctggttca aggttgggca ttgggtggag 660
   gagcagaatt tactacagca tgtgatttca ggttaatgac tccagagagt aagatcagat 720
   tcgtccacaa agagatgggc ataataccaa gctggggtgg caccacccgg ctagttgaaa 780
   taatcggaag tagacaagct ctcaaagtgt tgagtggggc ccttaaactg gattcaaaaa 840
   atgctctaaa cataggaatg gttgaagagg tcttgcagtc ttcagatgaa actaaatctc 900
   tagaagaggc acaagaatgg ctaaagcaat tcatccaagg gccaccggaa gtaattagag 960
   ctttgaaaaa atctgtttgt tcaggcagag agctatattt ggaggaagca ttacagaacg 1020
   aaagagatct tttaggaaca gtttggggtg ggcctgcaaa tttagaggct attgctaaga 1080
   aaggaaaatt taataaataa ttggtttttc gtgtggatgt actccaagta aagctccagt 1140
   gactaatatg tataaatgtt aaatgatatt aaatatgaac atcagaatta ctttgaaggc 1200
   tactattaat atgcagactt acttttaatc atttgaatat ctgaactcat ttacctcatt 1260
   tcttgccaat tactcacttg ggtatttact gcgtaatctg gaacatttag ctaaaatata 1320
   cacttttggc ttaaaaatta ttgctgtcaa ttccaataat aattcttagc ttataaccaa 1380
   agagcagtgt ttaaaaggag agcttctata caaaacctat tcctggcgtt acttttcata 1440
   caatttttgt tctgttttac ctggaaataa tttaccaaaa taactgagtg ttgctgctaa 1500
   agaacaaaag tggggaggta tcagggaaca agaaaacaag aaagggtatg atcaatcatt 1560
   ttcttctgct ccaaacagct ggagtaaaat tcatgggaaa tggcccttca tttaaaaaaa 1620
   gatgtacctc actacccact acaaatttgg aactttgttc ttttcaataa ttagttttct 1680
   attgtaaatt acctactaaa cagtggtagc catgacatgg aaagtcaact gattctacaa 1740
   ttggacattc atttgtgtgc cctggaattt ccaactagta ataaacaact actgttgatg 1800
   tagttttaaa ccacttgaag ggactcatga agcatcctgc aacataaatt tgcattttta 1860
   catcagattt cttttttttc ctgaaaaaca actaaccttc taacaactat ctttcaaaag 1920
   taaatgtaat aaaaatgcac aacataaaat gtttatgatc ccagcaatac actttttaaa 1980
   aaatgtgaaa gtcaaagaat taagttctag ttctgactca tcacaagagg tcaaaagtat 2040
   ttgctactgt aacattcaat tcacatttga gaatcatggt aaaaataact tgcatttgcc 2100
   ttaccatcat gatcctactg ttgagttagg aaaatatggt tagacagact cacattactt 2160
   tttttcagag gtaaactcta gattactgtg tcaacccaat actatttggc catagatgta 2220
   aaaactacca aataaaagtg gattttgtgg tctacaaaaa aaaaaaaaaa a 2271
<210> 85
   <211> 2697
   <212> DNA
   <213> Homo sapiens
<400> 85
   tggatatggg actcctgttt gtggcctgcc tctcttccga gtcttccagt gcagtccaga 60
   taacgatctc tgagagtctc ttgagaccac tgaagcaaga ggcttcaagg tttgttctag 120
   gcttctctag ttggcaggtc ttccccgatc ccaccccctc ccccagggct gccaggcgta 180
   ttcctgcctg ggcctcctgg cacaagagat aaaatgaaca gggttactcc cagtaactgg 240
   tccaggagat accagcagag agggagtagg agagaagaaa catgtcaggg tgctcacagg 300
   agtagtgggg ggaggttttg ctatttccag attcttaagc caacaaaagt gccttcatat 360
   tttctgtctg gaagacagaa agcccagaag gagcccagaa gcaacagttt gagagaggcg 420
   cttctgcggc caagtggata agaggagcgg cctgcaacca tgggacaggg tgagccaagc 480
   cagcgctcga cagggcttgc tggactgtat gcagcccccg cagcatcacc tgttttcatt 540
   aaaggaagtg ggatggatgc cctgggtatc aagagctgtg actttcaggc agcaagaaac 600
   aatgaggagc accacaccaa ggccctcagc tcccggcgcc tctttgtgag gagggggcag 660
   cccttcacca tcatcctgta cttccgcgct ccagtccgtg catttctgcc tgccctgaag 720
   aaggtggccc tcactgcaca aactggagag cagccttcca agatcaacag gacccaagcc 780
   acattcccaa tttccagtct gggggaccga aagtggtgga gtgcagtggt ggaggagaga 840
   gatgcccagt cctggaccat ctctgtgacc acacctgcgg acgctgtcat tggccactac 900
   tcgcttctgc tgcaggtctc aggcaggaag caactcctct tgggtcagtt cacactgctt 960
   tttaacccct ggaatagaga ggatgctgtt ttcctgaaga atgaggctca gcgcatggag 1020
   tacttgttga accagaatgg tctcatctac ctgggtacag ctgactgcat ccaggcagag 1080
   tcctgggact ttggccagtt cgagggggat gtcattgacc tcagcctgcg cttgctgagc 1140
   aaggacaagc aggtagagaa gtggagccag ccggtgcacg tggcccgtgt gttgggtgcc 1200
   ttgctgcatt ttctcaagga gcagagggtc ctgcccaccc cgcagaccca ggccacccag 1260
   gaaggggcct tgctgaacaa gcgccggggc agcgtgccca tcctgcggca gtggctcacc 1320
   ggccgaggcc gacctgtgta tgatggccag gcctgggtgt tggctgctgt tgcttgcaca 1380
   gtgctgcgat gcctgggaat ccctgcccgc gtggtgacca cgtttgcctc agcacagggc 1440
   accggtgggc gtcttctcat agatgaatac tataatgagg agggacttca gaacggagaa 1500
   ggccagagag gcagaatctg gatcttccag acttccacag agtgctggat gacgcggcct 1560
   gccttgcccc agggttatga tggatggcag attctcgacc caagtgctcc taatggaggt 1620
   ggagtcctgg ggtcctgtga tctggtgccg gtcagagcag tcaaggaggg gaccgtgggg 1680
   ctgaccccag cagtgtcaga cctttttgct gccataaatg cctcatgtgt ggtctggaag 1740
   tgctgtgagg atgggacact ggagttgact gactccaaca caaagtatgt tggcaacaac 1800
   atcagcacca agggtgtggg cagtgaccgc tgcgaggaca tcactcagaa ctacaagtat 1860
   cctgaagggt ctcttcagga aaaagaggtg ctggagagag tcgagaaaga gaaaatggaa 1920
   cgtgagaaag acaacggcat ccgtcctccc agtctcgaga ctgccagtcc tctgtacctg 1980
   ctcttgaaag cacccagctc cctacccctg agaggggatg cccagatctc agtgacgctg 2040
   gttaatcaca gtgagcagga gaaggcagtg cagctggcaa ttggggtcca ggctgtacac 2100
   tacaacggtg tccttgctgc caagctctgg aggaagaagc tgcacctcac gctcagtgcc 2160
   aacctggaaa agataataac catcggcctg ttcttctcca attttgagcg aaacccaccc 2220
   gagaacacct tccttagact caccgccatg gcaacacact ctgaatccaa ccttagctgc 2280
   tttgctcagg aagacattgc catttgtaga ccacaccttg ccatcaagat gccagagaaa 2340
   gcagagcagt atcaacccct cacagcctca gtcagcctcc agaactccct agatgccccc 2400
   atggaggact gtgtgatctc catcctggga agggggctca ttcacagaga gaggagctac 2460
   agattccgtt cagtgtggcc tgaaaacacc atgtgtgcca agttccagtt cacgccaaca 2520
   catgtggggc tccagagact cactgtggaa gtggactgca acatgttcca gaacctaacc 2580
   aactataaaa gcgtcaccgt ggtagcccct gaactatcag cttaaacttc cagctctatc 2640
   accactctcc tgccaaccct tgttctacaa tctaaaccaa acatgtgcta ggaagag 2697
<210> 86
   <211> 4034
   <212> DNA
   <213> Homo sapiens
<400> 86
   atgcaggcga cgccgagtga ggctgaggct gggggcgaat cgccgcagag ctgcgtgtcg      60
   gcggcgcatt ctgattggac agcggggaag cccgtcagcc tgttggcccc gctgatcccg     120
   ccccgtagcg cagggcagcc tttaaccttt agccccagtg ggcgccagcc actcagatcg     180
   cttcttgttg gtatgtgtag cggcagtggc cgccggcgga gcagtctgag cccgacgatg     240
   aggccgggga cgggagctga gcgtggaggc ctcatggtga gtgaaatgga gagccatcct     300
   ccctcgcagg gtcctgggga cggggagcgg agattgtccg gctcaagcct ctgctccggc     360
   tcttgggtct ctgctgacgg cttcctgagg agacggccct cgatggggca ccctggcatg     420
   cattatgccc caatgggaat gcaccctatg ggtcagagag cgaatatgcc tcctgtacct     480
   catggaatga tgccgcagat gatgccccct atgggagggc caccaatggg acaaatgcct     540
   ggaatgatgt cgtcagtaat gcctggaatg atgatgtctc atatgtctca ggcttccatg     600
   cagcctgcct taccgccagg agtaaatagt atggatgtag cagcaggtac agcatctggt     660
   gcaaaatcaa tgtggactga acataaatca cctgatggaa ggacttacta ctacaacact     720
   gaaaccaaac agtctacctg ggagaaacca gatgatctta aaacacctgc tgagcaactc     780
   ttatctaaat gcccctggaa ggaatacaaa tcagattctg gaaagcctta ctattataat     840
   tctcaaacaa aagaatctcg ctgggccaaa cctaaagaac ttgaggatct tgaagcaatg     900
   atcaaagctg aagaaagcag taagcaagaa gagtgcacca caacatcaac agccccagtc     960
   cctacaacag aaattccgac cacaatgagc accatggctg ctgccgaagc agcagctgct    1020
   gttgttgcag cagcagcagc ggcagcagca gcagcagctg cagccaatgc taatgcttcc    1080
   acttctgctt ctaatactgt cagtggaact gttccagttg ttcctgagcc tgaagttact    1140
   tccattgttg ctactgttgt agataatgag aatacagtaa ctatttcaac tgaggaacaa 1200
   gcacaactta ctagtacccc tgctattcag gatcaaagtg tggaagtatc cagtaatact 1260
   ggagaagaaa catctaagca agaaactgta gctgatttta ctcccaaaaa agaagaggag 1320
   gagagccaac cagcaaagaa aacatacact tggaatacaa aggaagaggc aaagcaagct 1380
   tttaaagaat tattgaaaga aaagcgggta ccatcgaatg cttcatggga gcaggctatg 1440
   aaaatgatta ttaatgatcc acgatacagt gctttggcaa agttaagtga aaaaaagcaa 1500
   gcctttaatg cctataaagt ccagacagaa aaagaagaaa aagaagaagc aagatcaaag 1560
   tacaaagagg ctaaggaatc ctttcagcgt tttcttgaaa atcatgagaa aatgacttct 1620
   acaaccagat acaaaaaagc agagcaaatg tttggagaga tggaagtttg gaatgcaata 1680
   tcagaacgtg atcgtcttga aatctatgaa gatgttttgt tctttctttc aaaaaaagaa 1740
   aaggaacaag caaagcagtt gcgaaagaga aattgggaag ccttaaaaaa catacttgac 1800
   aacatggcta atgtaacata ctctaccact tggtctgaag cccagcagta tctgatggat 1860
   aatccaactt ttgcagaaga tgaggagtta caaaatatgg acaaagaaga tgcattaatt 1920
   tgctttgaag aacacattcg ggctttagaa aaggaggaag aagaagaaaa acagaagagt 1980
   ttgctgagag aaaggagacg acagcgaaaa aatagggaat ctttccagat atttttagat 2040
   gaattacatg aacatggaca actgcattct atgtcatctt ggatggaatt gtatccaact 2100
   attagttctg atattagatt cactaatatg cttggtcagc ctggatcaac tgcacttgat 2160
   cttttcaagt tttatgttga ggatcttaaa gcacgttatc atgacgagaa gaagataata 2220
   aaagacattc taaaggataa aggatttgta gttgaagtaa acactacttt tgaagatttt 2280
   gtggcgataa tcagttcaac taaaagatca actacattag atgctggaaa tatcaaattg 2340
   gctttcaata gtttactaga aaaggcagaa gcccgtgaac gtgaaagaga aaaagaagag 2400
   gctcggaaga tgaaacgaaa agaatctgca tttaagagta tgttaaaaca agctgctcct 2460
   ccgatagaat tggatgctgt ctgggaagat atccgtgaga gatttgtaaa agagccagca 2520
   tttgaggaca taactctaga atctgaaaga aaacgaatat ttaaagattt tatgcatgtg 2580
   cttgagcatg aatgtcagca tcatcattca aagaacaaga aacattctaa gaaatctaaa 2640
   aaacatcata ggaaacgttc ccgctctcga tcggggtcag attcagatga tgatgatagc 2700
   cattcaaaga aaaaaagaca gcgatcagag tctcgttctg cttcagaaca ttcttctagt 2760
   gcagagtctg agagaagtta taaaaagtca aaaaagcata agaagaaaag taagaagagg 2820
   agacataaat ctgactctcc agaatccgat gctgagcgag agaaggataa aaaagaaaaa 2880
   gatcgggaaa gtgaaaaaga cagaactaga caaagatcag aatcaaaaca caaatcgcct 2940
   aagaaaaaga ctggaaagga ttctggtaat tgggatactt ctggcagcga actgagtgaa 3000
   ggggaattgg aaaagcgcag aagaaccctt ttggagcaac tggatgatga tcaataaatt 3060
   ataccaaata tatgtttaca gtatgattta aagtctgatt cagaccaggg actctatttt 3120
   aagttcaact gaaataacac tgggttttaa ttatatcaca ggaaaaaaaa agtgcattta   3180
   agtattgtta tcgtggactt tataaaagca aaggaaattg aaagtaactt ttgattctgt   3240
   atcaagaatc atattttcat acagtcataa ctgtctttct gtgacccttt cacagggcac   3300
   tgtaggatgg attaaaggtg gcaatttact gataactgca gatgtctcta ctttgttcta   3360
   aaatctaagt catgaggtga tttgatttac tttatagaag ctggattttg aagatctaat   3420
   gaaaaatttt ttgataatat agtagtacaa aaaaagcacc agcaactgat aaaaattgct   3480
   tttttgtgcg ctacccaact ggttaaagcc aatgtgatct tttatggtga aactcctaag   3540
   aaacaggtgg ttttgctgga aacttggtag acccttaatt atagtggtgc taatgagcac   3600
   tactgtaata taaagccacc attatttttt atcaaacatc tgaatacatt ttacaaaggc   3660
   tattgtgagg gcattatttt gagcatctat tttgaggtga tgtttaaaaa aactttaaca   3720
   tcaaatcaaa ttgtaaatta atttaaatat attgccttaa ggacctacta aagaatgtgc   3780
   caccagactt taagtgatag ttgcaatatc cttgtctaaa aaaaaaaaaa aagttgactt   3840
   aaacattttc tttaacagtt gtcttttttt tctaaattca gtctttctct tgcttttttt   3900
   tccctgctat tgaggaagta ttttgccttc cctactcact gagaagtatt gacttcgtgg   3960
   tacacattct aaagcatttc tgatttgaat atttttgtac atttttatca attattaaac   4020
   cttctcttct agtg                                                      4034
<210> 87
   <211> 5138
   <212> DNA
   <213> Homo sapiens
<400> 87
   gatccgcaga ggagcccact tgagagcgcc tcctgtcgtc tgtaaggttg ccttgccatc      60
   cctcggcacc ccaacttccc ccgccccccc atcgcctcct cctccatcct ccagttcaaa     120
   atggcgacgg cggcggcagc ggcggcggtg atggctcctc cgggctgccc gggttcgtgc     180
   cccaacttcg ccgtagtctg ctccttcttg gagcgctacg ggccgctgct agacctgcct     240
   gagttgccgt tccctgagct ggagcgggtg ctgcaggcgc cgccgccgga cgtcggcaac     300
   ggagaagtac caaaagaatt ggtggagctc catttgaagc tgatgaggaa aattggcaaa     360
   tctgttactg cagacagatg ggaaaaatat ttgatcaaga tatgccaaga gtttaacagt     420
   acctgggcat gggagatgga gaagaagggc tatcttgaaa tgagtgttga atgcaaacta     480
   gcactcttaa agtacctctg tgagtgtcag tttgatgaca atctcaaatt caagaatatt     540
   attaatgagg aggatgccga tactatgcgt ctccagccaa ttggtcgaga caaagatggc     600
   ctcatgtact ggtaccaatt ggatcaagat cacaatgtca gaatgtacat agaagaacaa     660
   gatgatcaag atggctcttc atggaaatgc attgtcagaa atcgaaacga gttggctgag     720
   actcttgcac tcctgaaagc acaaattgat cctgtactat tgaaaaactc tagccaacaa     780
   gacaactctt ctcgggaaag tcccagctta gaggatgagg agactaaaaa agaggaagaa 840
   acacctaaac aagaggaaca gaaagaaagt gaaaagatga aaagtgagga gcagcctatg 900
   gatttagaaa accgttctac agccaatgtt ctagaagaga ctactgtgaa aaaagaaaaa 960
   gaagatgaaa aggaacttgt gaaactgcca gtcatagtga agctagaaaa acctttgcca 1020
   gaaaatgaag aaaaaaagat tatcaaagaa gaaagtgatt ccttcaagga aaatgtcaaa 1080
   cccattaaag ttgaggtgaa ggaatgtaga gcagatccta aagataccaa aagtagcatg 1140
   gagaagccag tggcacagga gcctgaaagg atcgaatttg gtggcaatat taaatcttct 1200
   cacgaaatta ctgagaaatc tactgaagaa actgagaaac ttaaaaatga ccagcaggcc 1260
   aagataccac taaaaaaacg agaaattaaa ctgagtgatg attttgacag tccagtcaag 1320
   ggacctttgt gtaaatcagt tactccaaca aaagagtttt tgaaagatga aataaaacaa 1380
   gaggaagaga cttgtaaaag gatctctaca atcactgctt tgggtcatga agggaaacag 1440
   ctggtaaatg gagaagttag tgatgaaagg gtagctccaa attttaagac agaaccaata 1500
   gagacaaagt tttatgagac aaaggaagag agctatagcc cctctaagga cagaaatatc 1560
   atcacggagg gaaatggaac agagtcctta aattctgtca taacaagtat gaaaacaggt 1620
   gagcttgaga aagaaacagc ccctttgagg aaagatgcag atagttcaat atcagtctta 1680
   gagatccata gtcaaaaagc acaaatagag gaacccgatc ctccagaaat ggaaacttct 1740
   cttgattctt ctgagatggc aaaagatctc tcttcaaaaa ctgctttatc ttccaccgag 1800
   tcgtgtacca tgaaaggtga agagaagtct cccaaaacta agaaggataa gcgcccacca 1860
   atcctagaat gtcttgaaaa gttagagaag tccaaaaaga cttttcttga taaggacgca 1920
   caaagattga gtccaatacc agaagaagtt ccaaagagta ctctagagtc agaaaagcct 1980
   ggctctcctg aggcagctga aacttctcca ccatctaata tcattgacca ctgtgagaaa 2040
   ctagcctcag aaaaagaagt ggtagaatgc cagagtacaa gtactgttgg tggccagtct 2100
   gtgaaaaaag tagacctaga aaccctaaaa gaggattctg agttcacaaa ggtagaaatg 2160
   gataatctgg acaatgccca gacctctggc atagaggagc cttctgagac aaagggttct 2220
   atgcaaaaaa gcaaattcaa atataagttg gttcctgaag aagaaaccac tgcctcagaa 2280
   aatacagaga taacctctga aaggcagaaa gagggcatca aattaacaat caggatatca 2340
   agtcggaaaa agaagcccga ttctcccccc aaagttctag aaccagaaaa caagcaagag 2400
   aagacagaaa aggaagagga gaaaacaaat gtgggtcgta ctttaagaag atctccaaga 2460
   atatctagac ccactgcaaa agtggctgag atcagagatc agaaagctga taaaaaaaga 2520
   ggggaaggag aagatgaggt ggaagaagag tcaacagctt tgcaaaaaac tgacaaaaag 2580
   gaaattttga aaaaatcaga gaaagataca aattctaaag taagcaaggt aaaacccaaa 2640
   ggcaaagttc gatggactgg ttctcggaca cgtggcagat ggaaatattc cagcaatgat 2700
   gaaagtgaag ggtctggcag tgaaaaatca tctgcagctt cagaagagga ggaagaaaag 2760
   gaaagtgaag aagccatcct agcagatgat gatgaaccat gcaaaaaatg tggccttcca 2820
   aaccatcctg agctaattct tctgtgtgac tcttgcgata gtggatacca tactgcctgc 2880
   cttcgccctc ctctgatgat catcccagat ggagaatggt tctgcccacc ttgccaacat 2940
   aaactgctct gtgaaaaatt agaggaacag ttgcaggatt tggatgttgc cttaaagaag 3000
   aaagagcgtg ccgaacgaag aaaagaacgc ttggtgtatg ttggtatcag tattgaaaac 3060
   atcattcctc cacaagagcc agacttttct gaagatcaag aagaaaagaa aaaagattca 3120
   aaaaaatcca aagcaaactt gcttgaaagg aggtcaacaa gaacaaggaa atgtataagc 3180
   tacagatttg atgagtttga tgaagcaatt gatgaagcta ttgaagatga catcaaagaa 3240
   gccgatggag gaggagttgg ccgaggaaaa gatatctcca ccatcacagg tcatcgtggg 3300
   aaagacatct ctactatttt ggatgaagaa agaaaagaaa ataaacgacc ccagagggca 3360
   gctgctgctc gaaggaagaa acgccggcga ttaaatgatc tggacagtga tagcaacctg 3420
   gatgaagaag agagcgagga tgaattcaag atcagtgatg gatctcaaga tgagtttgtt 3480
   gtgtctgatg aaaacccaga tgaaagtgaa gaagatccgc catctaatga tgacagtgac 3540
   actgactttt gtagccgtag actgaggcga cacccctctc ggccaatgag gcagagcagg 3600
   cgtttgcgaa gaaagacccc aaagaaaaaa tattccgatg atgatgaaga ggaggaatct 3660
   gaggagaata gtagagactc tgaaagtgac ttcagtgatg attttagtga tgattttgta 3720
   gaaactcggc gaaggcggtc aaggagaaat cagaaaagac aaattaacta caaagaagac 3780
   tcagaaagtg acggttccca gaagagtttg cgacgtggta aagaaataag gcgagtacac 3840
   aagcgaagac tttccagctc agagagtgaa gagagctatt tgtccaagaa ctctgaagat 3900
   gatgagctag ctaaagaatc aaagcggtca gttcgaaagc ggggccgaag cacagacgag 3960
   tattcagaag cagatgagga ggaggaggaa gaggaaggca aaccatcccg caaacggcta 4020
   caccggattg agacggatga ggaggagagt tgtgacaatg ctcatggaga tgcaaatcag 4080
   cctgcccgtg acagccagcc tagggtcctg ccctcagaac aagagagcac caagaagccc 4140
   taccggatag aaagtgatga ggaagaggac tttgaaaatg taggcaaagt ggggagccca 4200
   ttggactata gcttagtgga cttaccttca accaatggac agagccctgg caaagccatt 4260
   gagaacttga ttggcaagcc tactgagaag tctcagaccc ccaaggacaa cagcacagcc 4320
   agtgcaagcc tagcctccaa tgggacaagt ggtgggcagg aggcaggagc accagaagag 4380
   gaggaagatg agcttttgag agtgactgac cttgttgatt atgtctgtaa cagtgaacag 4440
   ttataagact ttttttccat ttttgtgcta atttattcca cggtagctct cacaccagcg 4500
   ggccagttat taaaagctgt ttaatttttc ctagaaaact ccactacaga atgactttta 4560
   gaagaaaaat ttcaacaaat cctgaagtct ttctgtgaag tgaccagttc tgaactttga 4620
   agataaataa ttgctgtaaa ttccttttga ttttcttttt ccaggttcat ggtccttggt 4680
   aatttcattc atggaaaaaa atcttattat aataacaaca aagatttgta tatttttgac 4740
   tttatatttc ctgagctctc ctgactttgt gaaaaagggt ggatgaaaat gcattccgaa   4800
   tctgtgaggg cccaaaacag aatttagggg tgggtgaaag cacttgtgct ttagcttttt   4860
   catattaaat atatattata tttaaacatt catggcatag atgatgattt acagacaatt   4920
   taaaagttca agtctgtact gttacagttt gagaattgta gataacatca tacataagtc   4980
   atttagtaac agcctttgtg aaatgaactt gtttactatt ggagataacc acacttaata   5040
   aagaagagac agtgaaagta ccatcataat taacctaaat ttttgttata gcagagtttc   5100
   ttgtttaaaa aaaaataaaa tcatctgaaa agcaaaaa                           5138
<210> 88
   <211> 3041
   <212> DNA
   <213> Homo sapiens
<400> 88
   ggcagcaggt cggtaggcgg gaaatggcga ctggctgaag gagctggttc tgttgctgct     60
   gcggggtaag cgggaaagac accacacatt gcgcagtcgg gaccatcgcc ggagcctgag    120
   gacacttctc tgtcgtcaca gttagtttta catgtggcac ccataaaaga tgaggctgag    180
   aacacggaaa gcttctcagc agtcaaatca aatccaaaca caacgcactg ccagagcaaa    240
   gaggaaatat tcagaggttg atgatagtct gccttcagga ggagaaaaac catcgaagaa    300
   tgaaaccggc ttgttgtctt caattaaaaa atttattaaa ggaagcacac ctaaggaaga    360
   gagagaaaat ccttcaaaac ggagtagaat tgaacgtgat atagataaca atttgatcac    420
   gtcaacacca agagcaggag aaaaacctaa caaacagata tctcgagtaa gacggaaaag    480
   tcaagtaatg gagaagcttg gtagttatga aatgacaaat caacatgtaa aacaaaatgg    540
   aaaattagaa gataatcctt cctctggcag tcctccaagg actactttgt tggggaccat    600
   attttcacct gtcttcaact ttttttcacc agcaaataaa aatggaacgt caggatcaga    660
   ttctccagga caggctgtgg aagctgaaga aatagtaaaa caacttgata tggaacaggt    720
   ggatgagatc actaccagta ctactacatc aactaatgga gcagcttact caaatcaagc    780
   agttcaagtg agaccatcac taaacaatgg tttagaagaa gcagaagaaa cagttaatcg    840
   tgatatccca ccccttacag caccagtaac tccagatagt ggttattcat cagcccacgc    900
   ggaggccacc tatgaagaag actgggaagt atttgacccc tattatttca tcaaacatgt    960
   cccgccactg acagaagaac aactaaatag gaaacctgct cttccgttga aaacaagaag   1020
   cacaccggaa ttctccctag ttttagactt ggatgaaaca ctagtgcatt gtagtctaaa   1080
   tgagctagaa gatgcagcac ttacttttcc agtccttttc caagatgtca tttatcaggt   1140
   ttatgtgaga ttaagaccat tttcagggaa attcctggaa cgaatgtctc agatgtatga   1200
   gatcattctt tttactgctt ctaagaaggt gtatgcagac aagttactga acatactaga   1260
   ccctaaaaag caactggtca ggcaccggct tttccgtgaa cattgtgttt gtgtacaagg   1320
   aaactatata aaggacttaa atattcttgg aagagatctt tcaaaaacta taataattga   1380
   caactcacca caagcctttg catatcagct ttctaatgga atccctatag aaagttggtt 1440
   tatggataaa aatgacaatg aactcctaaa attgattcca ttcctggaga agcttgtaga 1500
   actgaatgaa gatgttcgac cacacatcag agacagattt cgcttgcatg atttgctgcc 1560
   cccagattaa gtaccaagac ttgtcaaatc actgaagggg gagagaatgc tggacccttt 1620
   tgactaagac aaaacattgc catactggtg aaatttgcat tttgtacccc gtcttgcttt 1680
   ttcttatctt ttggtgccca ttattaatta agggtacaga aaggagactt tatctatctc 1740
   agatcgaata catatagtag gtaggcttaa acagaagagt ctttagaact aggcaactcc 1800
   agtgaaattt tttatgacag gacatctgca gtttataaag aattctgttt ctgccaccag 1860
   cagtttgacc tgtagtcaca caggatttta tgataataac agagatgaaa atggactttt 1920
   attttctctc tgtttggtgc tctaagtggg tttgtagcca cttttctgtt actttatgat 1980
   tctcatttca acaggaatgg ccagtaaaag ttgttttatt tttcctgtta aggtttataa 2040
   cctttttaca tttttgacct gtattagatt agaatttcat tatgcattcc ttttagttga 2100
   ggcgcttcat agttttctgg agatagtcaa tttttagttt tttattatac atttgaatgg 2160
   ccgttttcct gctttgtctg cctgcacatt gtatatttgt ttaaaaatat tctctacttt 2220
   tagtctatgt aagtttcatt tagaaagaca tgcatttata ttttgtttct gtaatattct 2280
   actgtaggtg aaatcttttc aaaaatcaag agactgggta gataagaata tatgaatgac 2340
   taatatacaa aagatttaaa ccattgtgat ggcgtatatc tcaaatggta atatcttgca 2400
   atggcacaca gatttaatgg ataaaggtat acctcagact tcactgtgct cacaatcttt 2460
   gaggagaaag tttggtcacc tgtttggctt gatttagtta ctgctgcctt tggttttctc 2520
   caggaatgaa gtattcagca cagtgactca gtattttcta gttattttgc atgggctggt 2580
   agtacctctg ttatgctctc agttacaatc aatttaaaac tgtataacag tcttggtacc 2640
   taacagtagc tatgcataat cctgtggcgc agtacactcc caagccacca atgcagttaa 2700
   tatgctctca tagtggtttt tctatgctat atgaaaatag tcttcaagcc ttggttctct 2760
   aatgcagcta ccacaagagg ttgatatttt tatagtggcg tgtaatctcc ttttcgggag 2820
   gctttttatg gaaggtagaa tttgtaaaag ttcgtatgct ttgcctctca actgcattaa 2880
   ccagtcacag gctcagactg tttttgtgta aaggatgtca aagaacggca ctttttctaa 2940
   agagaagttt gatattttgt atgcttgtta agaaagtaca gtattggaaa ttaaaggtgg 3000
   acaactgata aaaaaaaaaa aaaaaaaaaa aaaaaaaaaa a 3041
<210> 89
   <211> 2309
   <212> DNA
   <213> Homo sapiens
<400> 89
   tttggcctta tagcagttat ccatggccgt actgtagtaa agttccttag aactttgcca 60
   ggagtgaact agaaaaaagt gcttactagg gcctaagagt tgctttgtgc cgtgtagtct 120
   ggcctttgca ctagtagatc attgctgaca taggtcagtt tagagacctt tctgtgttaa 180
   tgcctcctgg tactgtctta agatacgtac agtgtctgtt tttagatcta tgcatatgtc 240
   atgaagctcc ttgtgggctc tgcatgaagc tgctgctttg tttttgggtt aacagatgtg 300
   cctgtcaact agcatgtgta ttgtccaaat tccataaact taaggttttt aagggctgtg 360
   tggtttctga gctctatgtg tctttcctat ccttgtacct tcaaagggtg agaaatgaga 420
   tttatacatc caaagttagt ctgataaata tggctttttg tttctccatg taacctagac 480
   tgtcaaaaat aagtgatggt gataagtagg cctggagcct cagcttctgt aaatctcatt 540
   cctaaaattt tgctagactc gtgttggcaa aaacaaatac ctgtggattg tccttaaggc 600
   ttttaatcag atacctgtgt tgctgttagc tgaactgtag tgaagcatcg atccaaatcg 660
   gtcttctgaa gtatcagtta tgcttttgag tttagaaaat acttaggtgt tagtctagtc 720
   ttcccattca tgaatcagtg tatgtccata tcagagagcc tcaacttctt ttttcttcct 780
   ttttaaaaat gattttagtg ttttgattta gtgtatacta catagttcag tattattggc 840
   tttaccagtg ttgacagaaa aattttaaat ctccagttgc aaacagcaat ggattaggat 900
   atggaaataa aatcatggtg acatcactgc tgagttatct taaacctctg ctacttaatt 960
   ctccatattg aaatgcatac tcctccacat acatggcttc caagtaaagg caattgtaga 1020
   ggggccctgt ctatcccagt atggttggat tttaaacata tctgtgtttc cgttattttg 1080
   ggaactgatt aatatttaca attttttttg tttatgagtt attttgatac taagaaaaga 1140
   gagaatctag aacatcttga agttgaaata caaattttat tcttttggtc ttgggagaat 1200
   ttaagcagtc tatgcaactc atcaaatggt gagaaatagc cctccgaggt tccagtaagc 1260
   tttcagtgac tttgatacct ccccaagttt cttgagttgc tgcttgtcaa cacccagctt 1320
   ttaactgagt gtttgctcct gatggtttag gagattttca tgttgtatca cactgtcaag 1380
   ttttattttg tctttttatc cctccgtgga tgtgagtttg aaacaagcac ggtacagtaa 1440
   tcctgcctga tagagtagtc tggaatgaga attacttttt gggtgagaga gttctccatt 1500
   ttaatgtttc taaagttttt catatgaact tggcattgga aaagggaggt aaagaaaaag 1560
   gacgtttact aaaagcagtg tctactcttc ccctttgtga gtgtttattc atggctaatg 1620
   aaaaaaagag aaggactctt gggttttgtg ttgccatgtt aagcatggag agggatgctt 1680
   gacagcatgc taattgaagc cagagcaagt atgtccttca tcaggtaatc aggaactctt 1740
   cagttgaagc tgaggaacta actgattagt tgttgatcat aatataattg gttacaaagt 1800
   ggaagtgcca gctggcttaa gtacccaaag aaaagaatgc agcagcctaa cttagtgtta 1860
   ccatatgtta ctgaatttga aactgacctt ttttcccacc ctacttcaca cacctaaaac 1920
   tcttttcttg tcagaccaaa gagcgaaaag aaaaaaaaaa agtaaaacac tttaccaatc 1980
   tgtcactcag gtacaatttt gtggtgagat ttttgtctgt tctctttgta ttgctcttaa 2040
   gagtcctttc tcagcatatt attctgccat tgcctctgtc ttccttgggg cacctcagct 2100
   ctggatgcta cccctgggat atctactgct gttatgtgaa tgataggagg taagtgacca 2160
   ttatagtaag ggctctttgt aaaaaaattc aaaaaattta aaaaggatgt atacatttta 2220
   tagtctggct atcagtttga tatcttgctg tcaagtatgt ttctcaatct gtatttatcc 2280
   atcccatcaa taaatgttaa tggtaaaac 2309
<210> 90
   <211> 2670
   <212> DNA
   <213> Homo sapiens
<400> 90
   ggccgagccg gaagaccctc tctttcgctg tttgagagtc tctcggctca aggaccggga 60
   ggtaagaggt ttgggactgc cccggcaact ccagggtgtc tggtccacga cctatcctag 120
   gcgccatggg tgtgataggt atacagctgg ttgttaccat ggtgatggcc agtgtcatgc 180
   agaagattat acctcactat tctcttgctc gatggctact ctgtaatggc agtttgaggt 240
   ggtatcaaca tcctacagaa gaagaattaa gaattcttgc agggaaacaa caaaaaggga 300
   aaaccaaaaa agataggaaa tataatggtc acattgaaag taagccatta accattccaa 360
   aggatattga ccttcatcta gaaacaaagt cagttacaga agtggatact ttagcattgc 420
   attactttcc agaataccag tggctggtgg atttcacagt ggctgctaca gttgtgtatc 480
   tagtaactga agtctactac aattttatga agcctacaca ggaaatgaat atcagcttag 540
   tctggtgcct acttgttttg tcttttgcaa tcaaagttct attttcatta actacacact 600
   attttaaagt agaagatggt ggtgaaagat ctgtttgtgt cacctttgga ttttttttct 660
   ttgtcaaagc aatggcagtg ttgattgtaa cagaaaatta tctggaattt ggacttgaaa 720
   cagggtttac aaatttttca gacagtgcga tgcagtttct tgaaaagcaa ggtttagaat 780
   ctcagagtcc tgtttcaaaa cttactttca aatttttcct ggctattttc tgttcattca 840
   ttggggcttt tttgacattt cctggattac gactggctca aatgcatctg gatgccctga 900
   atttggcaac agaaaaaatt acacaaactt tacttcatat caacttcttg gcacctttat 960
   ttatggtttt gctctgggta aaaccaatca ccaaagacta cattatgaac ccaccactgg 1020
   gcaaagaaag tatcccttta atgacagaag ccacattcga tactctgcga ctctggttaa 1080
   taatcctgct gtgtgctttg cggttggcca tgatgcgtag tcacctgcaa gcttatttaa 1140
   atttagccca aaaatgtgtg gatcagatga agaaagaagc ggggcgaata agcacggttg 1200
   agctacagaa aatggtggct cgagtctttt attatctttg tgtcattgca ctgcagtatg 1260
   tggcgcctct ggtaatgctg cttcacacaa ctctgctttt gaaaacacta ggtaatcatt 1320
   cctggggtat ttatccagaa tctatctcta ccttaccagt ggataatagt ctactgtcca 1380
   attctgttta ctctgaatta ccatcagctg aagggaaaat gaaggtaact gttacacaaa 1440
   taacagtggc actgagcagc ttaaaaaata tttttactcc tcttcttttt cgaggacttc 1500
   tgtcttttct gacctggtgg attgctgctt gcctcttttc tacaagcctt tttgggcttt 1560
   tctatcacca gtatctgact gtggcatgaa tctcagttaa caaaaaagca tatccaaatc 1620
   accctttaaa ttaaaatatc tgtgccctta aagggctgat gaaaaccaga agaaagcaaa 1680
   tacaatggga aaaaaaaaca tatcagaatg tcttgtatta aatgtttcct ctgtattctc 1740
   agggtgaatt aatgtagtaa tatttaaaat tacaaaatag attgttaact gttacactgt 1800
   ggcattggaa ttttaactct ttgtatttac tggtatgaga gggctatcta caagggtaat 1860
   atttctgatt accctggttt acagaaacct ccagcagtct ttgaaacatc tcacatgact 1920
   ctagttattg attgctttta atggttttat ggtactgttg atagtcatag tggctgccta 1980
   tagaacaatc ttcaaactga gccatgcttt aggggaggga aaggggctaa agtctcttct 2040
   gttggtaatt tattagttac tcttgaaaca gtaaaatcca acagaaagga agagatagct 2100
   actgtatatt acagtaaaga aagctgcata gttattttaa atttaatgga gatgaatatg 2160
   gttaaaatat ataactactg ctgcttgaga atagcaagag tattgtttta aaacatattc 2220
   cacccaactt gagagttctt ttaaaatgat tggccatatg aacatttgta atcttgccat 2280
   taggtttgga cctgccatat tttgttttat tctgtgatcc taactagttc cttttaatag 2340
   gctaaaatat ttatcaatac tgatcagact ttaaagaaat tactttgtaa acctgctgac 2400
   tacctgtatg tattgtatat atattatata ttaaatatat aatatattga gattataaaa 2460
   gatgaaaata ttgaatcctt ataatatttt aagttgcaga atgtatgtta aaaagtgact 2520
   tgaatgagat gtatttgtat ctagaaattt tatttctttt tggaatgaga ttaaaataca 2580
   ttttgaaagt taaaaaaaaa aaaaaaaaaa aaaaaaaaaa aaaaaaaaaa aaaaaaaaaa 2640
   aaaaaaaaaa aaaccaaaaa aaaaaaaaaa 2670
<210> 91
   <211> 2336
   <212> DNA
   <213> Homo sapiens
<400> 91
   cttttcttcc gcacggttgg aggaggtcgg ctggttatcg ggagttggag ggctgaggtc 60
   gggagggtgg tgtgtacaga gctctaggac tcacgcacca ggccagtcgc ggattttggg 120
   ccgaggcctg ggttacaagc agcaagtgcg cggttggggc cactgcgagg ccgttttaga 180
   aaactgttta aaacaaagag caattgatgg ataaatcagg aatagattct cttgaccatg 240
   tgacatctga tgctgtggaa cttgcaaatc gaagtgataa ctcttctgat agcagcttat 300
   ttaaaactca gtgtatccct tactcaccta aaggggagaa aagaaacccc attcgaaaat 360
   ttgttcgtac acctgaaagt gttcacgcaa gtgattcatc aagtgactca tcttttgaac 420
   caataccatt gactataaaa gctatttttg aaagattcaa gaacaggaaa aagagatata 480
   aaaaaaagaa aaagaggagg taccagccaa caggaagacc acggggaaga ccagaaggaa 540
   ggagaaatcc tatatactca ctaatagata agaagaaaca atttagaagc agaggatctg 600
   gcttcccatt tttagaatca gagaatgaaa aaaacgcacc ttggagaaaa attttaacgt 660
   ttgagcaagc tgttgcaaga ggatttttta actatattga aaagctgaag tatgaacacc 720
   acctgaaaga atcattgaag caaatgaatg ttggtgaaga tttagaaaat gaagattttg 780
   acagtcgtag atacaaattt ttggatgatg atggatccat ttctcctatt gaggagtcaa 840
   cagcagagga tgaggatgca acacatcttg aagataacga atgtgatatc aaattggcag 900
   gggatagttt catagtaagt tctgaattcc ctgtaagact gagtgtatac ttagaagaag 960
   aggatattac tgaagaagct gctttgtcta aaaagagagc tacaaaagcc aaaaatactg 1020
   gacagagagg cctgaaaatg tgacaggatc atgaatgtca aaggctttta tcttgagaac 1080
   atggtgtctg gagttaaagg tattggcata ctccacacat ctgtaccatt cttgagtgat 1140
   cgcttaggaa tgaatgtgat ttgaactcat tcatgttgag agggtgtcaa attgagaacc 1200
   aggtagatcc ccaccaccta cagtaaaaag gaccctaaag taaattggtt gaagaaatta 1260
   gatcccaaag attcttggtg aattttgaag tcttcatcag tatatccata ttaaaacgag 1320
   atgacagaag ccaaagtaat tatggcaagt aatggttttt atcttaacta taagttattt 1380
   gctcaagggt gtaatggtca ttaccaaggc ttttagaatg cagtttctca tttgctgtgg 1440
   acatgaccat aaaaaaaaat ttcccagtag gttttctatc tgctacgttg ctagcaatca 1500
   gcttattggg aacagttgat taactgtaat agaaatgcaa tacaaataaa atgtgaacca 1560
   catgtgattt ttctttaaaa tcagtgagat ttgaaaattc tcctagatct cttgaatcat 1620
   gcaaatttgc tttgccttta tattgtaacc cttgtgggtt gctaataacc aagcagtttg 1680
   tagtagagtt aactcaggct cgttctaggg actcattcat gttcactcac tgtacactca 1740
   tctctggaaa tgtaaaattt acttttatac tattgttatg tagggctgac aggacaactg 1800
   gatcagtttc attaaaaagg tatgtatgca ttagaaaaga catttgtatg ggtcatttca 1860
   aagagggctt atgaggctgt gaaacccaga gctcttaacg ctgtgaccaa agatggaagt 1920
   tctctatagg aagccatagc actcctaatg tttggtgcta tgttttcctg aggagatata 1980
   aaacgtaata atccatgatt gttgccatgt gagagtttta aaggttaatc aaaatttctc 2040
   ttcttcaggg caaacttgaa gataaatctt ttgactccag ctctttagag gatctaaagt 2100
   gaccttgatg gacagtggaa gaaatcacaa catggaattc ctcgaataac aatttattga 2160
   ctttaaataa ttttgtctaa tgctacatat acacaattaa aaaaccttta cactatttct 2220
   agaaagtcag catgtatttt tggctcgaag tttctctagt gttttctgtg gaaggaataa 2280
   aaatttgagt ttcaaaaaaa aaaaaaaaaa aaaaaaaaaa aaaaaaaaaa aaaaaa 2336
<210> 92
   <211> 5662
   <212> DNA
   <213> Homo sapiens
<400> 92
   agcgggttgt gactgaaacc cgtcaatatg gcggcgatcg gccgcggccg ctctctgaag 60
   aacctccgag tacgagggcg gaatgacagc ggcgaggaga acgtcccgct ggatctgacc 120
   cgagaacctt ctgataactt aagagagatt ctccaaaatg tggccagatt gcagggagta 180
   tcaaatatga gaaagctagg ccatctgaat aactttacta agcttctttg tgatattggc 240
   cacagtgaag aaaaactggg ctttcactat gaggatatca taatttgttt gcggttagct 300
   ttattaaatg aagcaaaaga agtgcgagca gcagggctac gagcgcttcg atatctcatc 360
   caagactcca gtattctcca gaaggtgcta aaattgaaag tggactattt aatagctagg 420
   tgcattgaca tacaacagag caacgaggta gagaggacac aagcacttcg attagtcaga 480
   aagatgatta ctgtgaatgc ttccttgttt cctagttctg tgaccaactc attaattgca 540
   gttggaaatg atggacttca agaaagagac agaatggtcc gagcatgcat tgccattatc 600
   tgtgaactag cacttcagaa tccagaggtg gtggcccttc gaggaggact aaacaccata 660
   ttgaaaaatg tgattgattg ccaattaagt cgaataaatg aggccctaat tactacaatt 720
   ttgcaccttc ttaatcatcc aaagactcgg cagtatgtgc gagctgatgt agaattagag 780
   agaattttag caccctatac tgattttcac tacagacata gtccagatac agctgaagga 840
   cagctcaaag aagacagaga agcacgattt ctagccagta aaatgggaat catagcaaca 900
   ttccgatcat gggcaggtat tattaattta tgtaaacctg gaaattctgg gatccagtct 960
   ctaataggag tactttgcat accaaatatg gaaataaggc gaggtctact tgaagtgctt 1020
   tatgatatat ttcgtcttcc tctacctgtt gtgactgagg agttcataga agcactactc 1080
   agtgtagatc cagggaggtt ccaagacagt tggaggcttt cagatggctt tgtggcagct 1140
   gaggcaaaaa ctattcttcc tcatcgtgcc agatccaggc cagacctcat ggataattat 1200
   ttggcactga tactctctgc atttattcgt aatggacttt tagagggtct agttgaagtg 1260
   ataacaaaca gtgatgatca tatctcagtt agagctacca tccttttagg agagctttta 1320
   catatggcaa acacaattct tcctcattca catagccatc atttacactg cttgccaacc 1380
   ctaatgaata tggctgcatc ctttgatatc cccaaggaaa agagactgcg agccagtgca 1440
   gccttgaact gtttaaaacg cttccatgaa atgaagaaac gaggacctaa gccttatagt 1500
   cttcatttag accacattat tcagaaagca attgcaacac accagaaacg ggatcagtat 1560
   ctccgagttc agaaagatat atttatcctt aaggatacag aggaagctct tttaattaac 1620
   cttagagata gccaagtcct tcaacataaa gagaatcttg aatggaattg gaatcttata 1680
   gggaccattc ttaagtggcc aaatgtaaat ctaagaaact ataaagatga acagttacac 1740
   aggtttgtac gaagactact ttatttttac aagcccagca gtaaattata tgccaacctg 1800
   gatctggatt ttgccaaggc caaacagctc acggttgtag gttgccagtt tacagaattt 1860
   cttcttgaat ctgaagagga tgggcaaggc tacttagaag atctagtaaa ggatattgtt 1920
   cagtggctca atgcttcatc tggaatgaaa cccgaaagaa gtcttcaaaa taatggttta 1980
   ttgaccaccc ttagtcaaca ctacttttta tttattggaa cactttcttg ccaccctcat 2040
   ggagttaaaa tgctggaaaa atgcagtgta tttcagtgtc tccttaatct ttgctccttg 2100
   aaaaaccaag atcacttgct aaaacttact gtttctagct tggactatag cagagatgga 2160
   ttggctagag tcatcctttc caaaatttta actgcagcta ctgatgcctg cagactctat 2220
   gcaacaaaac atttaagggt attattgaga gctaatgttg aattctttaa taattgggga 2280
   attgagttgt tagtgaccca gctacatgat aaaaacaaaa cgatttcctc tgaagctctt 2340
   gatatcctcg atgaagcatg tgaagacaag gccaatcttc atgctctcat tcagatgaaa 2400
   ccagcgttat cccaccttgg agacaagggt ttgcttctcc tgctgagatt tctctccatt 2460
   ccaaaaggat tttcctatct gaatgaaaga ggttatgtag caaaacaatt ggaaaagtgg 2520
   cacagggaat acaactccaa atatgttgac ttgattgagg aacaactcaa tgaagcactt 2580
   actacttacc ggaagcctgt tgatggtgat aactatgttc gtcggagtaa ccaaagatta 2640
   cagcgtcctc acgtctacct gcctatacac ctttatggac aactagtaca ccataaaaca 2700
   ggctgccatt tgttggaagt acagaatatt attacagaac tctgtcgtaa tgttcgtaca 2760
   ccagatttgg ataagtggga agaaattaaa aaactgaaag catctctttg ggccttggga 2820
   aatatcggct catcaaattg gggtctcaat ttgctacagg aagaaaacgt gattccagat 2880
   atactaaaac ttgcaaaaca gtgtgaagtt ctttccatca gagggacctg tgtatatgta 2940
   cttgggctca tagctaaaac caaacaaggc tgtgatattc taaaatgtca caactgggat 3000
   gctgtgaggc atagtcgcaa acatctgtgg ccagtggttc cagatgatgt ggaacaactc 3060
   tgtaatgaac tttcatctat cccaagcact ctaagtttga actcggagtc aaccagctct 3120
   agacataata gtgaaagtga atctgtgcca tcgagtatgt tcatattgga ggatgaccgg 3180
   tttggcagca gctctactag tacatttttc cttgatatca atgaagatac agagccaaca 3240
   ttttatgacc gatctggacc cataaaggat aaaaattcat tccctttctt tgcttctagt 3300
   aaacttgtga agaatcgtat cttaaattcg cttactttgc ctaacaaaaa acatcgtagt 3360
   agcagtgatc caaaaggagg gaaattatca tctgaaagta agacaagcaa caggcgaatc 3420
   agaacactta cggagcccag tgttgatttt aatcatagtg atgattttac acccatatcc 3480
   actgtacaga aaacattaca attagagact tcatttatgg ggaataagca cattgaagac 3540
   actggtagta caccaagcat tggagaaaat gacttaaaat tcaccaagaa ttttggtaca 3600
   gagaatcaca gagaaaatac aagccgagag aggttagtag tagaaagttc aacgagctca 3660
   catatgaaga tacgtagcca aagtttcaat acagacacta caacaagtgg cataagttca 3720
   atgagctcaa gtccttcacg agagacagta ggtgtagatg ctacaactat ggacacagac 3780
   tgtggaagca tgagtactgt ggtaagtact aaaactatta agacaagcca ctatttgacg 3840
   ccacagtcta accatctgtc tctctccaaa tcaaattcgg tgtccctggt gcctccaggt 3900
   tcttctcata cgcttcctag aagagcacag tcccttaaag caccctctat tgctacaatt 3960
   aaaagtctag cagattgtaa ctttagttac acaagttcta gagatgcttt tggctatgct 4020
   acactgaaaa gactacagca acaaagaatg catccatcct tatctcactc tgaagctttg 4080
   gcatctccag caaaagatgt gctatttact gataccatca ccatgaaggc caacagtttt 4140
   gagtccagat taacaccaag caggttcatg aaagccttaa gttatgcatc attagataaa 4200
   gaagatttat tgagtcctat taatcaaaat accctgcaac gatcttcctc agtgcggtcc 4260
   atggtgtcca gtgccacata tgggggttca gatgattaca ttggtcttgc tctcccggtg 4320
   gatataaatg atatattcca ggtaaaggat attccctatt ttcagacaaa aaacatacca 4380
   ccacatgatg atcgaggtgc aagagcattt gcccatgatg caggaggtct tccatctgga 4440
   actggaggtc ttgtaaaaaa ttcttttcac ttgctacgac agcagatgag tcttacggaa 4500
   ataatgaatt caatccattc agatgcctct ctgtttttag aaagtacaga agacactgga 4560
   ctacaggaac atacagatga taactgcctt tattgtgtct gtattgaaat tctgggtttc 4620
   cagcccagca accaactgag tgcaatatgt agtcattcag actttcaaga tattccatat 4680
   tctgattggt gtgagcagac tatccataat cctttagaag tggttccctc taagttttcg 4740
   gggatttctg gatgcagtga tggggtgtct caagaaggct cagctagcag caccaaaagc 4800
   acagaattgt tactaggtgt taaaacaatt ccagatgata caccaatgtg ccgtatactc 4860
   cttcgcaaag aagttctaag attagtcatt aatttgagta gttcagtttc aactaaatgt 4920
   catgagactg ggcttttaac aattaaggag aagtatcctc aaacatttga tgacatatgc 4980
   ctttactctg aggtttccca tttgctgtca cactgcacat tcagacttcc gtgtcggagg 5040
   ttcatacaag aattatttca agatgtacag tttctacaaa tgcatgaaga agcagaggct 5100
   gtgttggcaa caccaccaaa gcaacctata gttgatacat ctgctgaatc ctgacctcat 5160
   atttatgatg gatatagata catactatat atattcatat ttgtggattt cctaaaagcc 5220
   tcagaaaata cgactgacta ggcagcaaag acaggagtat cttctgtaca ctgttccgca 5280
   gttactggta catgaacagt tggaactgct gactttccta accaaaacaa cttccttctc 5340
   tcctttgttg agccttttga ggggttcatg attcattacc acagttttaa gagtttcagt 5400
   taccattgta tgcaagagcc aagcactgaa tacctacata ggttttctat tttctttcat 5460
   tttaaaagcg taatgacagt ggaacaataa tgggatatgc agaagcaccc ttcacaagtt 5520
   atttctgaat gattttaggg taaataatac agatgccttg tatgttaact aacttgtgga 5580
   aagcaggaat cagtgtctct aaggctgcat cctattacca caatggggtt gtgctataac 5640
   tggctggtat tagagaggga ac 5662
<210> 93
   <211> 5128
   <212> DNA
   <213> Homo sapiens
<400> 93
   gcgggcggtg ctggagcgcc aagtggcgct ggagaaccgg cgcttccttt cgccgcttcc 60
   gccgccatct ccgcgtttgt ggggcgggaa agagggaggg ggctagcggc tgcagctgga 120
   gcgggcttct ctccggggac ggtcctttcc tccctgctct ccttttcctt ctttccgcgt 180
   tgccgccgcc cgccccctgc gcctccccgc ggagcctggg tccgggaggg ggaaggtagg 240
   ggcggcgggg ggcgggagag tctggcgagc ggacgctagc tctgaggaaa ctcatcaatc 300
   cgtgagcccc ggagtccggg gtgcacatcg gcccagccgc agcgtcggcg gcggcggcgg 360
   cagcagcacg aaggggctca gtcggggtag gcgggggcgg tgccggtgcc gcgggggcgg 420
   gcccgaccgt cccgcccaga agttgtaggg cttggctcct cgcgatcttg ttcctttccc 480
   ctccgcttct ctgacctagc tgcgcggccc cggcccggga gctgccgaac ccgcgcctcc 540
   cctgggtgag gaggacacgc ctgccctcgt cgagaaaact tttcctgccg actcagttgg 600
   ggcggcggtg gcaggaagtg cgggcagcga cctctcctcc gcctgccccg cgcgccctgc 660
   cggaggtcgg cgctgagctt gcgatcaagt ttgtgggggc ccccttcccc agttgccggc 720
   gagtctcgcc tcgagagggg cgcccgaccc cggggagggc ggcaggccag ggcgaaggcc 780
   aagggcgtgt ggtggcgccg gagactaggt gcggagcaag gcggggactc gcacccgcat 840
   ccgagagcgc ggaggtcgcg cagcccggga gaagggagcc tccggcggct gcttcctaga 900
   gtccacagtg cgctgtctcc tttggctgag gagagtgtcc tggccccgag tctatcgagg 960
   aaaatgaagt taagccgcca gttcaccgtg ttcggcagtg cgatcttctg tgtggtgatt 1020
   ttctcgctct acctgatgct ggaccggggt cacttagact accccaggaa cccgcgccgc 1080
   gagggctcct tccctcaggg ccagctctca atgttgcaag aaaaaataga ccatttggag 1140
   cgtttgctag ctgagaataa tgagatcatc tcaaatatta gagactcagt catcaatttg 1200
   agtgagtctg tggaggatgg tccgaaaagt tcacaaagca atttcagcca aggtgctggc 1260
   tcacatcttc tgccctcaca attatccctc tcagttgaca ctgcagactg tctgtttgct 1320
   tcacaaagtg gaagtcacaa ttcagatgtg cagatgttgg atgtttacag tctaatttct 1380
   tttgacaatc cagatggtgg agtttggaag caaggatttg acattactta tgaatctaat 1440
   gaatgggaca ctgaacccct tcaagtcttt gtggtgcctc attcccataa cgacccaggt 1500
   tggttgaaga ctttcaatga ctactttaga gacaagactc agtatatttt taataacatg 1560
   gtcctaaagc tgaaagaaga ctcacggagg aagtttattt ggtctgagat ctcttacctt 1620
   tcaaagtggt gggatattat agatattcag aagaaggatg ctgttaaaag tttaatagaa 1680
   aatggtcagc ttgaaattgt gacaggtggc tgggttatgc ctgatgaagc tactccacat 1740
   tattttgcct taattgatca actaattgaa ggacatcagt ggctggaaaa taatatagga 1800
   gtgaaacctc ggtccggctg ggctattgat ccctttggac actcaccaac aatggcttat 1860
   cttctaaacc gtgctggact ttctcacatg cttatccaga gagttcatta tgcagttaaa 1920
   aaacactttg cactgcataa aacattggag tttttttgga gacagaattg ggatctggga 1980
   tctgtcacag atattttatg ccacatgatg cccttctaca gctatgacat ccctcacact 2040
   tgtggacctg atcctaaaat atgctgccag tttgatttta aacgtcttcc tggaggcaga 2100
   tttggttgtc cctggggagt ccccccagaa acaatacatc ctggaaatgt ccaaagcagg 2160
   gctcggatgc tactagatca gtaccgaaag aagtcaaagc tttttcgtac caaagttctc 2220
   ctggctccac taggagatga tttccgctac tgtgaataca cggaatggga tttacagttt 2280
   aagaattatc agcagctttt tgattatatg aattctcagt ccaagtttaa agttaagata 2340
   cagtttggaa ctttatcaga tttttttgat gcgctggata aagcagatga aactcagaga 2400
   gacaagggcc aatcgatgtt ccctgtttta agtggagatt ttttcactta tgccgatcga 2460
   gatgatcatt actggagtgg ctattttaca tccagaccct tttacaaacg aatggacaga 2520
   atcatggaat ctcatttaag ggctgctgaa attctttact atttcgccct gagacaagct 2580
   cacaaataca agataaataa atttctctca tcatcacttt acacggcact gacagaagcc 2640
   agaaggaatt tgggactgtt tcaacatcat gatgctatca caggaactgc aaaagactgg 2700
   gtggttgtgg attatggtac cagacttttt cattcgttaa tggttttgga gaagataatt 2760
   ggaaattctg catttcttct tattttgaag gacaaactca catacgactc ttactctcct 2820
   gataccttcc tggagatgga tttgaaacaa aaatcacaag attctctgcc acaaaaaaat 2880
   ataataaggc tgagtgcgga gccaaggtac cttgtggtct ataatccttt agaacaagac 2940
   cgaatctcgt tggtctcagt ctatgtgagt tccccgacag tgcaagtgtt ctctgcttca 3000
   ggaaaacctg tggaagttca agtcagcgca gtttgggata cagcaaatac tatttcagaa 3060
   acagcctatg agatctcttt tcgagcacat ataccgccat tgggactgaa agtgtataag 3120
   attttggaat cagcaagttc aaattcacat ttagctgatt atgtcttgta taagaataaa 3180
   gtagaagata gcggaatttt caccataaag aatatgataa atactgaaga aggtataaca 3240
   ctagagaact cctttgtttt acttcggttt gatcaaactg gacttatgaa gcaaatgatg 3300
   actaaagaag atggtaaaca ccatgaagta aatgtgcaat tttcatggta tggaaccaca 3360
   attaaaagag acaaaagtgg tgcctacctc ttcttacctg atggtaatgc caagccttat 3420
   gtttacacaa caccgccctt tgtcagagtg acacatggaa ggatttattc ggaagtgact 3480
   tgcttttttg accatgttac tcatagagtc cgactatacc acatacaggg aatagaagga 3540
   cagtctgtgg aagtttccaa tattgtggac atccgaaaag tatataaccg tgagattgca 3600
   atgaaaattt cttctgatat aaaaagccaa aatagatttt atactgacct aaatgggtac 3660
   cagattcaac ctagaatgac actgagcaaa ttgcctcttc aagcaaatgt ctatcccatg 3720
   accacaatgg cctatatcca ggatgccaaa catcgtttga cactgctctc tgctcagtca 3780
   ttaggggttt cgagtttgaa tagtggtcag attgaagtta tcatggatcg aagactcatg 3840
   caagatgata atcgtggcct tgagcaaggt atccaggata acaagattac agctaatcta 3900
   tttcgaatac tactagaaaa aagaagtgct gttaatacgg aagaagaaaa gaagtcggtc 3960
   agttatcctt ctctccttag ccacataact tcttctctca tgaatcatcc agtcattcca 4020
   atggcaaata agttctcctc acctaccctt gagctgcaag gtgaattctc tccattacag 4080
   tcatctttgc cttgtgacat tcatctggtt aatttgagaa caatacagtc aaaggtgggc 4140
   aatgggcact ccaatgaggc agccttgatc ctccacagaa aagggtttga ttgtcggttc 4200
   tctagcaaag gcacagggct gttttgttct actactcagg gaaagatatt ggtacagaaa 4260
   cttttaaaca agtttattgt cgaaagtctc acaccttcat cactatcctt gatgcattca 4320
   cctcccggca ctcagaatat aagtgagatc aacttgagtc caatggaaat cagcacattc 4380
   cgaatccagt tgaggtgaac ctgactttca catttggatt gagaatcatt ggcttttata 4440
   cctttcttgg tttgacgtgc aataaagaag cacattattt tagcttctgg ctactgtgag 4500
   aacatgaatt ctgtgattct gtgggttttt tcttttttct tttaccagta cagtaagaaa 4560
   aaaaaaaaaa aaaaaaaagc catgctatca atcaagattc ttttttttta aactttctcc 4620
   catgaactac caccatcagt atgaattgat gcaacaaatg aagaaatatt taaagacagc 4680
   ctctcaacag attgtatctc aggttaaatg ctaactaatt atgtctgtgt tgggggttgc 4740
   gaagagattc ttaaaagtat ctgtgtgttg atcatcagtt ttacaaaaac acctatttgg 4800
   ctgaaatgga ataaaatgtt tgtgggtaaa agctaatggc caaaatggtt gcaatcattc 4860
   atactagtta gaaaaattat gtgttgaaat aagtggaaaa gtgcaatcca tccaccctta 4920
   tgattaacgt agatgatttt tatacctttt tctgatgtac ctcttgacct tctccttccc 4980
   ttcctaccct ttctaagtat ttccagaaat acctgatttt gaatcattca acagtagaaa 5040
   aagaggcata ttttcattac ttgacaatgt gggatgggtg caatttattc catcttcact 5100
   aaaatagaag caattccata ggtaccat 5128
<210> 94
   <211> 2192
   <212> DNA
   <213> Homo sapiens
<400> 94
   cggcacgagc taggatctgt gactgccacc cctcccccca cccgggctcg gcgggggagc 60
   gactcatgga gctgccgtaa gttttaccaa cagactgcag tttcttcact accaaaatga 120
   catcattttc cacctctgct cagtgttcaa catctgacag tgcttgcagg atctctcctg 180
   gacaaatcaa tcaggtacga ccaaaactgc cgcttttgaa gattttgcat gcagcaggtg 240
   cgcaaggtga aatgttcact gttaaagagg tcatgcacta tttaggtcag tacataatgg 300
   tgaagcaact ttatgatcag caggagcagc atatggtata ttgtggtgga gatcttttgg 360
   gagaactact gggacgtcag agcttctccg taaagaaccc aagccctctc tatgatatgc 420
   taagaaagaa tcttgtcact ttagccactg ctactacaga tgctgctcag actctcgctc 480
   tcgcacagga tcacagtatg gatattccaa gtcaagacca actgaagcaa agtgcagagg 540
   aaagttccac ttccagaaaa agaactacag aagacgatat ccccacactg cctacctcag 600
   agcataaatg catacattct agagaagatg aagacttaat tgaaaattta gcccaagatg 660
   aaacatctag gctggacctt ggatttgagg agtgggatgt agctggcctg ccttggtggt 720
   ttttaggaaa cttgagaagc aactatacac ctagaagtaa tggctcaact gatttacaga 780
   caaatcagga tgtgggtact gccattgttt cagatactac agatgacttg tggtttttga 840
   atgagtcagt atcagagcag ttaggtgttg gaataaaagt tgaagctgct gatactgaac 900
   aaacaagtga agaagtaggg aaagtaagtg acaaaaaggt gattgaagtg ggaaaaaatg 960
   atgacctgga ggactctaag tccttaagtg atgataccga tgtagaggtt acctctgagg 1020
   atgagtggca gtgtactgaa tgcaagaaat ttaactctcc aagcaagagg tactgttttc 1080
   gttgttgggc cttgaggaag gattggtatt cagattgttc aaagttaacc cattctctct 1140
   ccacgtctga tatcactgcc atacctgaaa aggaaaatga aggaaatgat gtccctgatt 1200
   gtcgaagaac catttcggct cctgtcgtta gacctaaaga tgcgtatata aagaaagaaa 1260
   actccaaact ttttgatccc tgcaactcag tggaattctt ggatttggct cacagttctg 1320
   aaagccaaga gaccatctca agcatgggag aacagttaga taacctttct gaacagagaa 1380
   cagatacaga aaacatggag gattgccaga atctcttgaa gccatgtagc ttatgtgaga 1440
   aaagaccacg agacgggaac attattcatg gaaggacggg ccatcttgtc acttgttttc 1500
   actgtgccag aagactaaag aaggctgggg cttcatgccc tatttgcaag aaagagattc 1560
   agctggttat taaggttttt atagcataat ggtagtacga acataaaaat gcatttattc 1620
   agttcactta ccacattatt tgaaaatcaa tcctttattt aattttattt ccaacctgtc 1680
   agagaatgtt cttaggcatc aaaatccaag gtagctgtaa gaaaaatact ggagctaaca 1740
   atgaagaaca gaagtaatct gattagtcaa attattaagt gccatggatt actttatgca 1800
   gcagtcaggt acatagttag gtgaacccaa aagaaaaact cttgaaaaca agagatttct 1860
   tccatgcaca tttacaatat tgaggtataa ttaacatgat aaagtgtttc cttctaacga 1920
   gttgtagaaa tctgagtaac cacccaaaaa agcaatagaa tgtttgtgtc accccaaaac 1980
   actcccttct gcccctcttc agacagtcct tcagctattt catggctctc accctagttt 2040
   tttttttttt ttgcactttt ttttttccgg gggtataggg gaggtgtggg gcgacagggt 2100
   ctgtcttgtt ctgtctccca ggctgaagtg cagtgagtca agattgagcc actgcactcc 2160
   agcctgggtg acagcgcgag actccatctc ag 2192
<210> 95
   <211> 2302
   <212> DNA
   <213> Homo sapiens
<400> 95
   ggagagacca cactgccatg cctccctctg ggccccgagg aaccctcctt ctgttgccgc 60
   tgctgctgct gctcctgctt cgcgccgtgc tggctgtccc cctggagcga ggggcgccca 120
   acaaggagga gacccctgcg actgagagtc ccgacacagg cctgtactac caccggtacc 180
   tccaggaggt catcgatgta ctggagacgg atgggcattt ccgagagaag ctgcaggctg 240
   ccaatgcgga ggacatcaag agcgggaagc tgagccgaga gctggacttt gtcagccacc 300
   acgtccgcac caagctggat gagctcaagc gacaggaggt gtcacggctg cggatgctgc 360
   tcaaggccaa gatggacgcc gagcaggatc ccaatgtaca ggtggatcat ctgaatctcc 420
   tgaaacagtt tgaacacctg gaccctcaga accagcatac attcgaggcc cgcgacctgg 480
   agctgctgat ccagacggcc acccgggacc ttgcccagta cgacgcagcc catcatgaag 540
   agttcaagcg ctacgagatg cttaaggaac acgagagacg gcgttatctg gagtcactgg 600
   gagaggagca gagaaaggag gcggagagga agctggaaga gcaacagcgc cggcaccgcg 660
   agcaccctaa agtcaacgtg cctggcagcc aagcccagtt gaaggaggtg tgggaggagc 720
   tggatggact ggaccccaac aggtttaacc ccaagacctt cttcatactg catgatatca 780
   acagtgatgg tgtcctggat gagcaggagc tggaggcact cttcaccaag gagctggaga 840
   aagtgtacga cccaaagaat gaggaggacg acatgcggga gatggaggag gagcgactgc 900
   gcatgcggga gcatgtgatg aagaatgtgg acaccaacca ggaccgcctc gtgaccctgg 960
   aggagttcct cgcatccact cagaggaagg agtttgggga caccggggag ggctgggaga 1020
   cagtggagat gcaccctgcc tacaccgagg aagagctgag gcgctttgaa gaggagctgg 1080
   ctgcccggga ggcagagctg aatgccaagg cccagcgcct cagccaggag acagaggctc 1140
   tagggcggtc ccagggccgc ctggaggccc agaagagaga gctgcagcag gctgtgctgc 1200
   acatggagca gcggaagcag cagcagcagc agcagcaagg ccacaaggcc ccggctgccc 1260
   accctgaggg gcagctcaag ttccacccag acacagacga tgtacctgtc ccagctccag 1320
   ccggtgacca gaaggaggtg gacacttcag aaaagaaact tctcgagcgg ctccctgagg 1380
   ttgaggtgcc ccagcatctg tgatcctccg ggaccccagc cctcaggatt cctgatgctc 1440
   caaggcgact gatgggcgct ggatgaagtg gcacagtcag cttccctggg ggccggtgtc 1500
   atgttgggct cctggggcgg gggcacggcc tggcatttca cgcattgctg ccaccccagg 1560
   tccacctgtc tccactttca cagcctccaa gtctgtggct cttcccttct gtcctccgag 1620
   gggcttgcct tctctcgtgt ccagtgaggt gctcagtgat cggcttaact tagagaagcc 1680
   cgccccctcc ccttctccgt ctgtcccaag agggtctgct ctgagcctgc gttcctaggt 1740
   ggctcggcct cagctgcctg ggttgtggcc gccctagcat cctgtatgcc cacagctact 1800
   ggaatccccg ctgctgctcc gggccaagct tctggttgat taatgagggc atggggtggt 1860
   ccctcaagac cttcccctac cttttgtgga accagtgatg cctcaaagac agtgtcccct 1920
   ccacagctgg gtgccagggg caggggatcc tcagtatagc cggtgaaccc tgataccagg 1980
   agcctgggcc tccctgaacc cctggcttcc agccatctca tcgccagcct cctcctggac 2040
   ctcttggccc ccagcccctt ccccacacag ccccagaagg gtcccagagc tgaccccact 2100
   ccaggaccta ggcccagccc ctcagcctca tctggagccc ctgaagacca gtcccaccca 2160
   cctttctggc ctcatctgac actgctccgc atcctgctgt gtgtcctgtt ccatgttccg 2220
   gttccatcca aatacacttt ctggaacaaa aaaaaaaaaa aaaaaaaaaa aaaaaaaaaa 2280
   aaaaaaaaaa aaaaaaaaaa aa 2302
<210> 96
   <211> 4079
   <212> DNA
   <213> Homo sapiens
<400> 96
   gccgctcggc cgcgcgcggg tcggccggct ctatggggaa agcggcggct ccgagccgag 60
   gcggcggctg tggcggccgc tcccgcgggc tctcgtcgct gttcacggtt gtcccctgcc 120
   tgtcgtgcca cacggcggcg ccgggcatga gcgcttccac gtccggctcc gggccggagc 180
   ccaagcccca gccccagccc gtgcccgaac cggagcgggg accgctgtca gaacaggtgt 240
   cggaggcagt ttcggaggca gtgccaagat cggaacctgt gtccgagacg acgtctgagc 300
   cggagccagg ggctgggcag ccatcggaac tgctgcaggg gtcgcggccg gggtcagagt 360
   caagctcagg tgtaggggct gggcccttca ctaaggccgc atcggagccg ctctcccggg 420
   cggtggggag cgcgaccttt ctcagacccg agtcaggatc gctgccagcg ttaaagcccc 480
   tgcctcttct gcgaccagga caggcgaaga ctcctcttgg ggttccaatg tcggggactg 540
   gcacgacctc cagtgcccca ctggccttac tgcctctgga cagcttcgag ggctggcttc 600
   tcaagtggac caactatctg aagggctacc agcgccgctg gttcgtgctg ggcaatggtt 660
   tgctctctta ctacagaaat cagggtgaaa tggcccacac gtgccgtgga accatcaacc 720
   tgtccaccgc gcacattgac acggaggact cttgtggtat cttgctgacc agtggggcca 780
   ggagctacca cctcaaggcc agctcagagg tggaccggca gcagtggatc accgccctgg 840
   agctggccaa ggccaaggct gtccgcgtga tgaacactca ttcagatgac tctggggacg 900
   acgacgaggc taccacccca gccgacaaga gcgagctgca ccacaccctg aagaatcttt 960
   ccctgaagtt agatgacctc agcacgtgca atgacctcat cgccaagcac ggcgctgcac 1020
   tccagcgctc cctgacagag ctggacggcc tcaagatccc atctgagagt ggggagaagc 1080
   tgaaggtggt gaatgagcgg gccaccctct tccgcatcac atccaatgct atgatcaacg 1140
   cctgcaggga cttcttggaa ctagcagaga tacacagtcg gaaatggcag cgggcactgc 1200
   agtatgagca ggagcagcgc gtgcacttgg aggaaaccat tgagcagctg gcgaagcagc 1260
   acaacagcct cgagcgggcc ttccacagtg cccctggccg gccggccaac ccctccaaga 1320
   gcttcattga gggaagcctc ttgactccca aaggagagga cagtgaggaa gatgaagata 1380
   ccgagtactt tgatgccatg gaagactcca catccttcat caccgtgatc accgaggcca 1440
   aggaagacag cagaaaagct gaaggtagca ccgggacaag ttccgtggac tggagctcag 1500
   cagacaatgt actagatggt gcctcgctcg tgcccaaggg ttcatccaaa gtcaagaggc 1560
   gagtccgcat tcccaacaag cccaactaca gccttaacct ctggagcatc atgaagaact 1620
   gcatcggccg ggagctctcc aggatcccca tgccggtgaa cttcaatgag cccctgtcca 1680
   tgctccagcg gctgacagag gacctggagt accaccacct gctggacaag gcagtgcact 1740
   gcaccagctc agtggagcag atgtgcctgg tggccgcctt ctctgtgtcc tcctactcca 1800
   ccacagtgca ccgcatcgcc aagcccttca accccatgct gggggagacc ttcgagctgg 1860
   accgcctcga cgacatgggc ctgcgctccc tctgtgagca ggtgagccac caccccccct 1920
   cagctgcgca ctacgtgttc tccaagcatg gctggagcct ctggcaggag atcaccatct 1980
   ccagcaagtt ccggggaaaa tacatctcca tcatgccgct aggtgccatc cacttagaat 2040
   tccaggccag tgggaatcac tacgtgtgga ggaagagcac ctcaactgtt cacaacatca 2100
   tcgtgggcaa gctctggatc gaccagtcag gggacatcga gattgtgaac cataagacca 2160
   atgaccggtg ccagctgaag ttcctgccct acagctactt ctccaaagag gcagcccgga 2220
   agggagaacg cggagaacat gtactacttc tcagagctgg ccctgaccct caacgagcac 2280
   gaggagggcg tagcgccaac cgacagccgc ctgcggcccg accagcggct gatggagaag 2340
   ggccgttggg acgaggccaa taccgagaag cagcggctgg aggagaagca gcgcctgtcg 2400
   cggcgccggc ggctggaggc ctgcgggccg ggcagcagct gcagctcgga ggaagagaag 2460
   gaggcggatg cctacacgcc actgtggttt gagaagaggc tggatccgct gaccggggag 2520
   atggcctgtg tgtacaaggg cggctactgg gaggccaagg agaagcaaga ctggcatatg 2580
   tgccccaaca tcttctgagc gccacccttg caacaaatac aggcgcctgc acagcctggc 2640
   ccacctgttc attaatgcac tcaatttagt actgaatggt ctttctccca gcccattccc 2700
   agcccttcct atttcctttc ctattttttt tttctcccca cactttcttg ggactcccac 2760
   cttggaagga ggaagggctg acctgggttc tctccagccc ccaggtgcgc cgggtcaccc 2820
   gtgccccttc attatggacc tgggccctac cggaacccct gccccagtta ccacaactca 2880
   ggccggctgg cccgggccat gggctgcgca aatcaccagc cccaacccca gggaggaact 2940
   ggcccctcct agggagcctc ttcgactttt ttagaaaaat gatctccatt tctttccagc 3000
   catgatgttt agtaaatatt tttagtaccg cacttagcag acagctttcc aagtgtgctt 3060
   tcttgccaca aaagtgtcct ggcaagagcc ccttattttt aagacatcag gaagccagac 3120
   cgctttgagt tgggagaatt ttgtagctca acatatcaag tcctcgatgg tatctgagct 3180
   gcccacaccc ccacctgcca aggccccaca gagcccaaaa cagaaggggg ctgccccagc 3240
   ccagcagagc acagagtttc tggagctccc atccacagat gcaggagggg gtactgatgg 3300
   taacccccat gtggatttga gggcagcagt ccctggcctc accctagcca gcctgggtgg 3360
   ctccctagcc ccaagaggcc aggaagggct ggaaggcagg gcctgcaggt gctccccgcc 3420
   ctgagaccca ggccccaaat cagcaataat gaacaaaccc ttggcccagc ctgggctggt 3480
   gacctgggca ccagagacct tgcatccctc ctcatcctag gaggccccta ggggtgcccc 3540
   atctcagtgt cccctgaact ctttatttgc ctaatttata tatatatata tgagatatat 3600
   aaatatatat aaaatagcta ttttgcttaa atttctacag tatgtaaaag tgaaaaaatg 3660
   atgaagacgg gtgcacctgt ctgagtttgg ccctcatgtg agctgtgccc ttccctctcc 3720
   tcatgccccc ttccagcggc ttctgccaac catggggggc tggaccacca tggccactga 3780
   cccagcccct cagaatccca cactccaatc ctttccattt cagtttagtc ctaaaagttc 3840
   atcacagggt ctttctttct actccaggac tggttttgtt tttatatata taaaaaaaaa 3900
   aagtgaaaac accaatgtgt gaaatgcctt acaatgccca ctggagaggc ggggcggggt 3960
   ggggcaggat ggccccactg gggctcctac agagctgtgg aatgtacctc tccccaacac 4020
   tgttttgtta gcgagcacct tttgaccagt aataaaaaac cttggctttg gagttttcc 4079
<210> 97
   <211> 3724
   <212> DNA
   <213> Homo sapiens
<400> 97
   tctccctcgg cgccgccgcc gccgcccgcg gggctgggac ccgatgcggt tagagccgcg   60
   gagcctggaa gagccccgag cgtttctgct ttgggacaac catacatcta attccttaaa  120
   gtagttttat atgtaaaact tgcaaagaat cagaacaatg cctccacgac catcatcagg  180
   tgaactgtgg ggcatccact tgatgccccc aagaatccta gtagaatgtt tactaccaaa  240
   tggaatgata gtgactttag aatgcctccg tgaggctaca ttaataacca taaagcatga  300
   actatttaaa gaagcaagaa aataccccct ccatcaactt cttcaagatg aatcttctta  360
   cattttcgta agtgttactc aagaagcaga aagggaagaa ttttttgatg aaacaagacg   420
   actttgtgac cttcggcttt ttcaaccctt tttaaaagta attgaaccag taggcaaccg  480
   tgaagaaaag atcctcaatc gagaaattgg ttttgctatc ggcatgccag tgtgtgaatt  540
   tgatatggtt aaagatccag aagtacagga cttccgaaga aatattctga acgtttgtaa  600
   agaagctgtg gatcttaggg acctcaattc acctcatagt agagcaatgt atgtctatcc  660
   tccaaatgta gaatcttcac cagaattgcc aaagcacata tataataaat tagataaagg  720
   gcaaataata gtggtgatct gggtaatagt ttctccaaat aatgacaagc agaagtatac  780
   tctgaaaatc aaccatgact gtgtaccaga acaagtaatt gctgaagcaa tcaggaaaaa  840
   aactcgaagt atgttgctat cctctgaaca actaaaactc tgtgttttag aatatcaggg  900
   caagtatatt ttaaaagtgt gtggatgtga tgaatacttc ctagaaaaat atcctctgag  960
   tcagtataag tatataagaa gctgtataat gcttgggagg atgcccaatt tgatgttgat 1020
   ggctaaagaa agcctttatt ctcaactgcc aatggactgt tttacaatgc catcttattc 1080
   cagacgcatt tccacagcta caccatatat gaatggagaa acatctacaa aatccctttg 1140
   ggttataaat agtgcactca gaataaaaat tctttgtgca acctacgtga atgtaaatat 1200
   tcgagacatt gataagatct atgttcgaac aggtatctac catggaggag aacccttatg 1260
   tgacaatgtg aacactcaaa gagtaccttg ttccaatccc aggtggaatg aatggctgaa 1320
   ttatgatata tacattcctg atcttcctcg tgctgctcga ctttgccttt ccatttgctc 1380
   tgttaaaggc cgaaagggtg ctaaagagga acactgtcca ttggcatggg gaaatataaa 1440
   cttgtttgat tacacagaca ctctagtatc tggaaaaatg gctttgaatc tttggccagt 1500
   acctcatgga ttagaagatt tgctgaaccc tattggtgtt actggatcaa atccaaataa 1560
   agaaactcca tgcttagagt tggagtttga ctggttcagc agtgtggtaa agttcccaga 1620
   tatgtcagtg attgaagagc atgccaattg gtctgtatcc cgagaagcag gatttagcta 1680
   ttcccacgca ggactgagta acagactagc tagagacaat gaattaaggg aaaatgacaa 1740
   agaacagctc aaagcaattt ctacacgaga tcctctctct gaaatcactg agcaggagaa 1800
   agatttctta tggagtcaca gacactattg tgtaactatc cccgaaattc tacccaaatt 1860
   gcttctgtct gttaaatgga attctagaga tgaagtagcc cagatgtatt gcttggtaaa 1920
   agattggcct ccaatcaaac ctgaacaggc tatggaactt ctggactgta attacccaga 1980
   tcctatggtt cgaggttttg ctgttcggtg cttggaaaaa tatttaacag atgacaaact 2040
   ttctcagtat ttaattcagc tagtacaggt cctaaaatat gaacaatatt tggataactt 2100
   gcttgtgaga tttttactga agaaagcatt gactaatcaa aggattgggc actttttctt 2160
   ttggcattta aaatctgaga tgcacaataa aacagttagc cagaggtttg gcctgctttt 2220
   ggagtcctat tgtcgtgcat gtgggatgta tttgaagcac ctgaataggc aagtcgaggc 2280
   aatggaaaag ctcattaact taactgacat tctcaaacag gagaagaagg atgaaacaca 2340
   aaaggtacag atgaagtttt tagttgagca aatgaggcga ccagatttca tggatgctct 2400
   acagggcttt ctgtctcctc taaaccctgc tcatcaacta ggaaacctca ggcttgaaga 2460
   gtgtcgaatt atgtcctctg caaaaaggcc actgtggttg aattgggaga acccagacat 2520
   catgtcagag ttactgtttc agaacaatga gatcatcttt aaaaatgggg atgatttacg 2580
   gcaagatatg ctaacacttc aaattattcg tattatggaa aatatctggc aaaatcaagg 2640
   tcttgatctt cgaatgttac cttatggttg tctgtcaatc ggtgactgtg tgggacttat 2700
   tgaggtggtg cgaaattctc acactattat gcaaattcag tgcaaaggcg gcttgaaagg 2760
   tgcactgcag ttcaacagcc acacactaca tcagtggctc aaagacaaga acaaaggaga 2820
   aatatatgat gcagccattg acctgtttac acgttcatgt gctggatact gtgtagctac 2880
   cttcattttg ggaattggag atcgtcacaa tagtaacatc atggtgaaag acgatggaca 2940
   actgtttcat atagattttg gacacttttt ggatcacaag aagaaaaaat ttggttataa 3000
   acgagaacgt gtgccatttg ttttgacaca ggatttctta atagtgatta gtaaaggagc 3060
   ccaagaatgc acaaagacaa gagaatttga gaggtttcag gagatgtgtt acaaggctta 3120
   tctagctatt cgacagcatg ccaatctctt cataaatctt ttctcaatga tgcttggctc 3180
   tggaatgcca gaactacaat cttttgatga cattgcatac attcgaaaga ccctagcctt 3240
   agataaaact gagcaagagg ctttggagta tttcatgaaa caaatgaatg atgcacatca 3300
   tggtggctgg acaacaaaaa tggattggat cttccacaca attaaacagc atgcattgaa 3360
   ctgaaaagat aactgagaaa atgaaagctc actctggatt ccacactgca ctgttaataa 3420
   ctctcagcag gcaaagaccg attgcatagg aattgcacaa tccatgaaca gcattagaat 3480
   ttacagcaag aacagaaata aaatactata taatttaaat aatgtaaacg caaacagggt 3540
   ttgatagcac ttaaactagt tcatttcaaa attaagcttt agaataatgc gcaatttcat 3600
   gttatgcctt aagtccaaaa aggtaaactt tgaagattgt ttgtatcttt ttttaaaaaa 3660
   caaaacaaaa caaaaatccc caaaatatat agaaatgatg gagaaggaaa aaaaaaaaaa 3720
   aaaa 3724
<210> 98
   <211> 3241
   <212> DNA
   <213> Homo sapiens
<400> 98
   ggcttccccg ccccgccccg tccccgggcg tctccatttt ggtctcaggt gtggactcgg 60
   caagaaccag cgcaagaggg aagcggagtt atagctaccc cggccgcgga gccggctcac 120
   tgcactaccc ccgccccctt ctttcctcca gacgccgaag tcgcgggcgc tcatggcggg 180
   cctggaggta ctgttcgcat cggcagcgcc ggccatcacc tgcaggcagg acgcgctcgt 240
   ctgcttcttg cattgggaag tggtgacaca cggttactgc ggcttgggtg tcggtgacca 300
   gccgggtccc aatgataaga agtcagaact gctgccagct gggtggaaca acaataaaga 360
   cctgtatgtc ctccggtatg agtataagga tgggtccaga aagctccttg tgaaagccat 420
   caccgtggag agcagcatga tcctcaatgt gctggaatat ggctcacagc aagtggcaga 480
   cttgaccctg aacttggatg attatatcga tgcagaacac ctgggtgact tccacaggac 540
   ctacaagaac agtgaggagc ttcggtctcg tattgtgtct ggaatcatca cacctatcca 600
   tgagcagtgg gaaaaggcta atgtaagcag tccccaccgg gagttccccc ctgctaccgc 660
   cagagaggtg gacccactcc ggattcctcc acaccaccca cacaccagtc ggcagcctcc 720
   ctggtgtgat cccctgggcc cgtttgttgt cgggggagaa gacttagacc cttttgggcc 780
   tcggagaggt ggcatgattg tggatcccct gagatctggc ttcccaagag cacttattga 840
   cccttcctca ggcctcccga accgacttcc tccaggcgct gtgcccccag gagctcgctt 900
   tgaccccttt ggacccattg ggaccagccc acccggacct aacccagacc atctcccccc 960
   gccgggctac gatgacatgt acctgtgaag gcctcaagaa tgtaacatcc caggcttccc 1020
   tccattctcc tggagctgcc accgctgtcc ccatcagcaa ccatgttctt gcaggctggg 1080
   ggcaagggat tctgctcatg tgtttgcaga ccggctggga tagcctcccc accccttatc 1140
   agagccaaga cacctgctgc agctctccac ctagctgcag atagctccca aagagaaatc 1200
   agtgtgtctc tttcaccatc agctcctccc ctttcaccac cagctcctct ccacttccca 1260
   agggagactc cggcaacctt cagcaacata tatcctcgac cagatgcagt gctataagaa 1320
   cagaacgcat tttggatgtt attattaaga accaaatgtc aatacagaat tcatgttgcc 1380
   ggtttcccac ttttcttttt acattaatgc atagctgctt ccatttatga gactttagag 1440
   tttgagtttc tgtagggctg aatgactctt tttcctgccc agggcccatt cttgcttctc 1500
   aggcaccttc cgtttattaa ttgccattgc tcctgacatc actaagatgg gtccccttct 1560
   ggctgcatga atggaaatga gtgactggaa atcccatagg ccacaagaat gactttcaca 1620
   agggcaggaa cattgtggaa agactgcatc attctgatga ggcaaaatcc tccagctatt 1680
   cctgtctggg ccagttttgt aggtccatct gtgcatgggc agcagtagtc aaaaagccaa 1740
   ggaaaaaaca gagcagacct gaaggctaat cttatttttg ccactaactt agtgaatgac 1800
   cctaagcaag ttccttctcc tcttagggcc ttgtgccaag cctatgaaat tggaggtggc 1860
   tttcctgctc taaagcattt tgatgtctca ttctgtgttt ggtaacccct ataaactggg 1920
   gcagaggaaa agaatgatgg ttcaaggcca tacttccctt gaaccttgtg tggttcttgc 1980
   ctaactctgt ggtttttgga ccccatgggg cccagacaga gcacaggagc atgggctgcc 2040
   tctgagtgtg gtgttgaact tcgggaggag cagggagccc tgcaccttgt gtcctggccc 2100
   acctgacctt tggtgttctc cggatccttt tcagcccgag gcctgacaga cgcgggcagt 2160
   gatgagccct gttctggagt ggaaagagca cgatagagca ccaggctaag aggcacgaga 2220
   tcaaggcggt agtcacttcc gctctgcagc tagcatttca accatatgtg gatcctttca 2280
   tttctcagct ccctggattc cttcccctaa attaggacct attatttacc tgtaggtaag 2340
   caagctactg tagctcttct gaggtatctg ccaggctgtt ttctgtagcc tcagattgcc 2400
   tatctgctta gcctgagaac aggtagatga aaactaaact gatgcctagg cccagggtca 2460
   gtctcagatg gaagctgggc ctgggtgggg aggctagcat gcgtggctcc ctgggtattt 2520
   ctgtcagtcc ccatggcaag cagtgattta gtaaaacacc ccagagtcag ggaagccaac 2580
   caccttgaaa cctttagaac atctctgctt tggagaaaga cccagagatc aggcagaggt 2640
   gcagattcaa tcattactca taacctttga gagctggcaa atgggaggag tgttagtctt 2700
   tgttttggaa ttggaccatt cttgcaacca aaaggactta gagcagtttg ctcataaaga 2760
   catcctttat tataaaagga agtatttaaa ggatgataga gaccatcaga tagaagcagg 2820
   gaaggtagat aacttttagg accttgatgt gaggaaaaga taaaatttaa acaataaatt 2880
   tgccacttag attttctagc agcaaagtcc gaaaagatag ttatatacaa aattctgttt 2940
   tctgaaaaca aaattgtgat tcaccttcag aattggccat tttttttgtg agtttccttg 3000
   catcaaggac actgagaaac acagtcattg tcttaggtgt tctatgggag gaagtgaata 3060
   gagcctttag gaacttcctg gtcaagctta tggtgcttat tttgatctgg gccacttccc 3120
   tccttccagt catgagtaat catcaaggag caagttggag tgtttcaggt gtatattttg 3180
   tagaacccaa aagattggag ccttaacaat aaacatcagc actaagtgac ctgttaaaaa 3240
   a 3241
<210> 99
   <211> 3925
   <212> DNA
   <213> Homo sapiens
<400> 99
   agcggggctg cgcgaagtca tcgctgttcc agacagcgat gactcgagag cggtgggggt 60
   ggcggcgcga tcggccgggc tgtaaccgtc gtctgtccgg gagcggctgg agcggcagcg 120
   gcggccgggc acggcgcgag gtgacgccac agggcagcgg cggcagcgga ggcagcggcg 180
   gcagcaggag acgcagcggc ggccgcagca gcagcagcaa gacggactcg tggagacgcg 240
   ccgccgccgc cgccgccggg ccgggccggg tgtcgcgcgc cgaggctggg ggggagtcgt 300
   cgccgccgcc gccaccgcta ccgccgccgc cgccgccgcc gaggtgactg aggagagagg 360
   cgcctcctcg ctcccgccac cgccggactt caatgcccag tccccagctc gccagcgttt 420
   ttcgttggaa tatacgttgc acatttatgg cgattctgag tgtgagggca gacttctgcc 480
   aggctcagca cagcattttc gctgacaagt gagcttggag gttctatgtg ccataattaa 540
   cattgccttg aagactcctg gacaccgaga ctggcctcag aaatagttgg cttttttttt 600
   tttttaattg caagcatatt tcttttaatg actccagtaa aattaagcat caagtaaaca 660
   agtggaaagt gacctacact tttaacttgt ctcactagtg cctaaatgta gtaaaggctg 720
   cttaagtttt gtatgtagtt ggattttttg gagtccgaat atttccatct gcagaaattg 780
   aggcccaaat tgaatttgga ttcaagtgga ttctaaatac tttgcttatc ttgaagagag 840
   aagcttcata aggaataaac aagttgaata gagaaaacac tgattgataa taggcatttt 900
   agtggtcttt ttaatgtttt ctgctgtgaa acatttcaag atttattgat tttttttttt 960
   cactttcccc atcacactca cacgcacgct cacacttttt atttgccata atgaaccgtc 1020
   cagcccctgt ggagatctcc tatgagaaca tgcgttttct gataactcac aaccctacca 1080
   atgctactct caacaagttc acagaggaac ttaagaagta tggagtgacg actttggttc 1140
   gagtttgtga tgctacatat gataaagctc cagttgaaaa agaaggaatc cacgttctag 1200
   attggccatt tgatgatgga gctccacccc ctaatcagat agtagatgat tggttaaacc 1260
   tgttaaaaac caaatttcgt gaagagccag gttgctgtgt tgcagtgcat tgtgttgcag 1320
   gattgggaag ggcacctgtg ctggttgcac ttgctttgat tgaatgtgga atgaagtacg 1380
   aagatgcagt tcagtttata agacaaaaaa gaaggggagc gttcaattcc aaacagctgc 1440
   tttatttgga gaaataccga cctaagatgc gattacgctt cagagatacc aatgggcatt 1500
   gctgtgttca gtagaaggaa atgtaaacga aggctgactt gattgtgcca tttagaggga 1560
   actcttggta cctggaaatg tgaatctgga atattacctg tgtcatcaaa gtagtgatgg 1620
   attcagtact cctcaaccac tctcctaatg attggaacaa aagcaaacaa aaaagaaatc 1680
   tctctataaa atgaataaaa tgtttaagaa aagagaaaga gaaaaggaat taattcagtg 1740
   aaggatgatt ttgctcctag ttttggagtt tgaatttctg ccaggattga attattttga 1800
   aatctcctgt ctttttaaac tttttcaaaa taggtctcta aggaaaacca gcagaacatt 1860
   aggcctgtgc aaaaccatct gtttggggag cacactcttc cattatgctt ggcacataga 1920
   tctccctgtg gtgggatttt ttttttccct ttttttgtgg gggagggttg gtggtatatt 1980
   tttcccctct tttttccttc ctctcctaca tctccctttt cccccgatcc aagttgtaga 2040
   tggaatagaa gcccttgttg ctgtagatgt gcgtgcagtc tggcagcctt aagcccacct 2100
   gggcactttt agataaaaaa aaaaaaaaac aaaaaacaac accaaaaaaa cagcagtgat 2160
   atatatattc caggtggttt ttagtcttta ctgatgaaag ggtgttcatg ttagtttctt 2220
   caaaacccta tctaatacta ggcaaagtag ccaagagcct tttgttttgt ttttattttg 2280
   ataaattagt ggagaaatgg cattttaaga ggagtctctt ctcaacttac ctgagagtcg 2340
   aattcttctc ttccctaacc aatgaagcta agtggttatc ccagaaactt gtcttctaaa 2400
   agggaggact ccaggccatc aataaagatg tccaggcagt gagcgtactt tttacaccct 2460
   gtagaattgt gggctgtagc gttactctga ttttctgtct agtatcagag aatgctggta 2520
   gcttaaaatt tttattttag gacttgtact ctgaattttc aggaaccgtc aaaggagcag 2580
   cagcaaattc acatattttc gacttgagaa atgcttgtgg tatgtgtttt ccaaactgcc 2640
   ccctatatgt aaagttcagt ttaaccactg attgccttgt tattactagg ttttttgaga 2700
   ttaaaaaaaa aaaatccctg gtttaaaacc aacaatgatg cctagtgagt atgtgtccac 2760
   aggccataac agggtagaag agagacatcg tgcaacccaa tgagtagtga agggactgtg 2820
   ttgcttgtga agcggtgtag tagcattttt gcagattctt ggctgggttt agtgtactga 2880
   tctagaaaag ctgtttttct gctcctttgt ggaaggcagt tatgatcagg ctgcatggac 2940
   aaagcaggta gaggggcacc atcaggggct cttgcactat tttcacctct aaatattacg 3000
   tactcagtag tgccctgctt ctagggctct gaatacgggc ttaaagtcat cttgtcctgc 3060
   tggaatttgc tgtgcagagc cataagcctc ccattttgtt agcgtcagct aggccaatag 3120
   gaacagaccg ggaccttgtc tcacactgat gatacctcac atgttgaccg gctatgtgaa 3180
   ctgcctattt cctatgctgg agttttgatt tttaactaaa cgcaaatctg tagattctct 3240
   cctctcccat cccagaaaac aaaacaaaat aatgcttttc gaaattgttt ctaggacttt 3300
   aaaacataat ggtatatcca aaattcttta tttcagaatg caacaataga ttccattaat 3360
   atagactcaa gatcaaaaca gcatacctgc taagctaaga tagatggtgt tgattccact 3420
   gggttttgat caatacaata acaaaccttt ttcctttgac atactctgaa ttttgttgtt 3480
   tggggggagg gggtgtgtgt gtgtgtgtgt gtgtgtgtgt gtattgtgtg tgtgtgtgtg 3540
   tgcacgcgca gtgtccatca gtatcagtgc ctgcctgagt taggaaaatt acattcctgg 3600
   ttctgtattg aggagaagga tgtataaagc aacatgaaac attagccctc cttttatttt   3660
   aaagactaat gttaattgtt cttaaaactg gatttttttt ccttaaagca atttttttct   3720
   tttcgattta atgaagtatt gctagctgaa gccagtttga catagagaga tgtcagattg   3780
   atttgaaagg tgtgcagcct gatttaaaac caaaccctga acccttttaa agaacaataa   3840
   aacatatttt acacgctcaa aaaaaaaaaa aaaaaaaaaa aaaaaaaaaa aaaaaaaaaa   3900
   aaaaaaaaaa aaaaaaaaaa aaaaa                                         3925
<210> 100
   <211> 2709
   <212> DNA
   <213> Homo sapiens
<400> 100
   gctggtggaa cccctgcaca gagacggaca caggatgagc tctaagtacc cgcggtctgt 60
   ccggcgctgc ctgcccctct gggccctaac actggaagca gctctcattc tcctcttcta 120
   tttttttacc cactatgacg cttccttaga ggatcaaaag gggctcgtgg catcctatca 180
   agttggccaa gatctgaccg tgatggcggc cattggcttg ggcttcctca cctcgagttt 240
   ccggagacac agctggagca gtgtggcctt caacctcttc atgctggcgc ttggtgtgca 300
   gtgggcaatc ctgctggacg gcttcctgag ccagttccct tctgggaagg tggtcatcac 360
   actgttcagt attcggctgg ccaccatgag tgctttgtcg gtgctgatct cagtggatgc 420
   tgtcttgggg aaggtcaact tggcgcagtt ggtggtgatg gtgctggtgg aggtgacagc 480
   tttaggcaac ctgaggatgg tcatcagtaa tatcttcaac acagactacc acatgaacat 540
   gatgcacatc tacgtgttcg cagcctattt tgggctgtct gtggcctggt gcctgccaaa 600
   gcctctaccc gagggaacgg aggataaaga tcagacagca acgataccca gtttgtctgc 660
   catgctgggc gccctcttct tgtggatgtt ctggccaagt ttcaactctg ctctgctgag 720
   aagtccaatc gaaaggaaga atgccgtgtt caacacctac tatgctgtag cagtcagcgt 780
   ggtgacagcc atctcagggt catccttggc tcacccccaa gggaagatca gcaagactta 840
   tgtgcacagt gcggtgttgg caggaggcgt ggctgtgggt acctcgtgtc acctgatccc 900
   ttctccgtgg cttgccatgg tgctgggtct tgtggctggg ctgatctccg tcgggggagc 960
   caagtacctg ccggggtgtt gtaaccgagt gctggggatt ccccacagct ccatcatggg 1020
   ctacaacttc agcttgctgg gtctgcttgg agagatcatc tacattgtgc tgctggtgct 1080
   tgataccgtc ggagccggca atggcatgat tggcttccag gtcctcctca gcattgggga 1140
   actcagcttg gccatcgtga tagctctcac gtctggtctc ctgacaggtt tgctcctaaa 1200
   tcttaaaata tggaaagcac ctcatgaggc taaatatttt gatgaccaag ttttctggaa 1260
   gtttcctcat ttggctgttg gattttaagc aaaagcatcc aagaaaaaca aggcctgttc 1320
   aaaaacaaga caacttcctc tcactgttgc ctgcatttgt acgtgagaaa cgctcatgac 1380
   agcaaagtct ccaacaataa aatacagcca tgtaccacat aacaacatct tggtaaacaa 1440
   cagactgcat atatgatggt ggtcatccag taagctaagg ttaatttatt attattcctt 1500
   gttttttttt tttttttttt ttttttgaga tgtagtctta ctctgtcacc caggctagag 1560
   tgcaatggca ccatcttggc tcactgcaac ctctacctcc tgggttcaag caaatctcct 1620
   gcctcagcct ccaaagtagc tgggattaca ggcacccacc acatctggct aattttttgt 1680
   atttttagta aagatggggt ttcaccatgt tggccaggct gatctcaaac tcctgacctc 1740
   aagtgatctg cccgcctcgg cctcccaaag tgctggaacc acaggcctga gccactgtgc 1800
   ccagccttgt ttgctttttt aacagataac agtgtgctca tagaaactgc tttgacatga 1860
   ctgcaatcat gtgcttcata gaaacttaat tagattatac cactagagtc ttcagatttt 1920
   tatacttttt ttttttgaaa cggagtctca ctctgtcacc aggctggagt gcagtgccgc 1980
   aatctcggct cactgcaacc tccgcctccc aggttcaagc aattctcctg cctcagcctc 2040
   ccgagtagct gggattacaa gtgcgcacta ccacacccag ctaatttttg catttttact 2100
   tgacagggtt tcaccatgtt ggctaggata gtttcaccag gatctcttgg cctcatgatc 2160
   agcctgcctc ggcctcccaa agtgctggga ttacaggtgt gagccaccgt gcccagccta 2220
   tacttccctt tttgaatacc atttggtgtt ttgaagaatt aacagctttg tgaacgtggc 2280
   agtgcttgtg attcaggctt ccattgagac caaggggaga acctggttgc aggacaaaca 2340
   gacggacagc gtgtggcagt gtttaaatgc tcttctgaag gctgatacga cagctctctg 2400
   tgcactgatt gcatatgcat cccaagatta tattattgtt ttctactgct atgtgtcaca 2460
   ctttgccaaa caggatgtgg aaaatgaata agcggttttc ttaggcactt cttaacagac 2520
   aattggtcaa aatgaactcc attgcttaag aaacacataa acaccattta gtcactgaac 2580
   atagctatat gtatggttgt tactatggga aatcttgttt tgccaatttt ctttgaaaat 2640
   tctggcagac caaggttctt tttgtttaca taatacttga aaaataaaaa tgaacaagct 2700
   aacaaacta 2709
<210> 101
   <211> 4272
   <212> DNA
   <213> Homo sapiens
<400> 101
   aagaggttgg gcggatgttg tgagccgggt cgctgcggcc gaggctccgg cccccaggac 60
   cactggctgc ccatgagaga tgaaggatgg catccaaggg ggccggcatg tctttctccc 120
   gcaagagcta taggctgacc tcagatgctg agaaatccag ggtcacaggc attgtgcagg 180
   agaagctgct gaatgactac ctgaaccgca tcttttcctc ttctgaacat gcacccccag 240
   cagccaccag caggaaaccc ctgaacttcc agaacctgcc agaacatttg gaccagttgc 300
   tacaggtgga caatgaggag gaggaaagcc agggacaggt tgaagggcgg cttggcccat 360
   ccactgtggt cctggaccac acaggcggct ttgaggggct tctcctggtg gatgatgacc 420
   tgctgggggt gattggacac agcaactttg gcaccatccg ctctaccaca tgcgtgtaca 480
   aagggaaatg gctctacgag gtcctcatct cctcccaggg gctcatgcag atcggctggt 540
   gcaccatcag ctgccgcttc aaccaggagg agggggttgg agatacacac aactcctatg 600
   cctatgatgg caaccgcgtg cgcaagtgga atgtgaccac aacgaattat ggcaaggcgt 660
   gggcagcggg ggacatcgtg agctgcctga ttgacctgga tgatggcact ctgtccttct 720
   gcctgaacgg tgtatcactg ggcactgcct ttgagaacct gtccaggggc ctgggtatgg 780
   cctacttccc agccatcagc ctctctttca aggagtccgt ggccttcaac tttggcagcc 840
   gtcctctgcg ctacccagtg gcaggctacc ggcccctgca ggacccaccg agtgctgacc 900
   tggtgcgggc acagaggttg ctgggctgct tccgggcagt gctgagtgtg gagctggacc 960
   ctgtggaggg gcggctgttg gacaaggaga gctccaagtg gcggttgcgg ggccagccca 1020
   ccgtcctcct cacactggcc cacatcttcc atcactttgc accgcttctg cgcaaggtgt 1080
   atctggtgga ggctgtgctc atgagcttct tgctgggcat cgtggagaag ggcacaccca 1140
   cacaggcaca gtccgtggtg caccaggtcc tggacctctt gtggctcttc atggaggact 1200
   acgaggtaca agattgcctc aagcagttga tgatgtctct gcttcggctg taccgattct 1260
   cacccattgt cccagacctg ggcctacaga tccattacct gcggctcact atcgccatcc 1320
   tgaggcatga gaagtcccgc aagtttctgc ttagcaatgt cctcttcgac gtgctccgct 1380
   ccgtcgtctt cttttacatc aagagccccc tgcgtgtgga ggaggccggc ctgcaggagc 1440
   tcattcccac cacctggtgg ccccactgct ccagtaggga gggcaaagag agcacggaga 1500
   tgaaggagga gaccgcagag gagcggctgc ggcggcgagc ctacgaacgg ggctgtcagc 1560
   ggctcaggaa gcgcatcgaa gtggtggaag aactacaggt ccagatcctg aagctgctgc 1620
   tggacaataa agatgacaat gggggtgaag cttctaggta tatcttcctg accaagtttc 1680
   gcaagtttct gcaggagaac gccagtggcc gggggaacat gcccatgctc tgcccccctg 1740
   agtacatggt ctgcttctta caccggctga tctctgccct gcgctactat tgggatgaat 1800
   acaaggcttc caatcctcat gcttccttca gtgaggaggc ctacatcccg ccccaggtct 1860
   tctataatgg caaggtggac tactttgacc tgcagcgcct ggggggcctc ctctcgcacc 1920
   tgcggaagac cctcaaagat gaccttgctt ccaaagccaa cattgtgatc gacccactgg 1980
   agctccagtc aaccgccatg gatgacctag atgaggatga ggagccagcc ccagctatgg 2040
   cccagcgccc catgcaggcc ctggctgttg gggggccact gcccctgccc cggcccggct 2100
   ggctcagttc tccaactttg ggccgagcca accgcttcct cagcacagcg gctgtgagcc 2160
   tcatgacccc acggcggcct ctgagcacct cggagaaagt gaaggtccgc acgctgagcg 2220
   tggagcagag gacccgtgag gacattgaag gcagccactg gaatgagggc ttgctgctgg 2280
   ggcggccccc cgaggagcct gagcagcccc tcaccgagaa ctcgctgctg gaagtcctgg 2340
   atggggcggt catgatgtac aacctcagcg tacaccagca gctgggcaag atggtgggtg 2400
   tctccgatga tgtcaatgaa tacgctatgg ctctgaggga cacagaggac aagctccgcc 2460
   ggtgccccaa gaggaggaag gacatccttg cagagttgac caagagccag aaggttttct 2520
   cagaaaagct ggaccacctg agccgccgtc ttgcctgggt ccatgccact gtctactccc 2580
   aggagaagat gctggacatc tactggctgc tgcgcgtctg cctgcggacc attgagcacg 2640
   gtgatcgcac agggtctctc tttgccttca tgcccgagtt ctacctgagc gtggccatca 2700
   acagctacag tgctctcaag aattactttg gtcccgtgca cagcatggag gagctcccag 2760
   gctatgaaga gaccctgacc cgcctggctg ccattctcgc caaacacttt gccgacgcac 2820
   gcattgtggg cactgacatc cgagactcac tgatgcaggc cctggccagc tacgtgtgct 2880
   acccacactc cctgcgggct gtggagcgaa tccccgagga gcagcgtatc gccatggtga 2940
   ggaacctcct ggcgccctat gagcagcggc cctgggccca gaccaactgg atcctggtgc 3000
   ggctctggag gggctgtggc ttcgggtacc gctatacacg gctgccacat ctgctgaaaa 3060
   ccaaacttga ggacgccaat ttgcccagcc tccagaagcc ctgcccttcc accctgctgc 3120
   agcagcacat ggcggacctc ctacagcagg gtcctgatgt ggcacccagc ttcctcaaca 3180
   gcgtcctcaa tcagctcaac tgggccttct ctgaattcat tggcatgatc caagagatcc 3240
   agcaggctgc tgagcgcctg gagcggaact ttgtggacag ccggcagctc aaggtatgtg 3300
   ccacctgctt tgacctctcg gtcagcctgc tgcgtgtctt ggagatgact atcacactgg 3360
   tgcctgagat attccttgac tggacccggc ctacctctga gatgctgctg cggcgtcttg 3420
   cacagctgct aaaccaggtg ctgaaccggg tgacagctga gaggaacctg tttgatcgtg 3480
   tggtcaccct acggctgcct ggcctagaga gcgtggacca ctatcccatt ctggtggcag 3540
   tgacgggcat cctggtgcag ctcctggtgc gtggcccagc ctcagagaga gagcaagcca 3600
   catcagtgct cctggcagat ccctgcttcc agctacgctc aatatgctat ctcctgggac 3660
   agccagagcc cccagcacct ggcactgctc tgccagcccc tgaccggaag cgcttctccc 3720
   tgcagagcta tgcggattat atcagtgccg atgagctggc ccaagtggaa cagatgctgg 3780
   cgcacctgac ctctgcatct gcccaggcag cagctgcctc cctgcccacc agtgaggagg 3840
   acctctgccc catctgctat gcccacccca tctctgctgt gttccagccc tgtggccaca 3900
   agtcctgcaa agcctgtatc aaccagcacc tgatgaacaa caaggactgc ttcttctgca 3960
   aaaccaccat cgtgtctgta gaggactggg agaagggagc caatacgagt actacctcct 4020
   cagctgccta gccctcacag cctgtgccat cctggaacct ccacctttga acccagagcc 4080
   aggctgggcc ctatttatga gctccctttg cccttctcct gtatcccaca ccaccacatc 4140
   caacctcctt gcctgcctgt atcctcattg gtgggagccc agccatggcc ctaattgtgc 4200
   ctgagcttga ctttcagtca gggccacagt gagcattaaa ttattattcc atacaaaaaa 4260
   aaaaaaaaaa aa 4272
<210> 102
   <211> 2553
   <212> DNA
   <213> Homo sapiens
<400> 102
   ctagaggaag aggtagctcc acaggaggta cagctgctta cacatctctc ctcagagctg 60
   tcccttgact tgggggtgaa tttcaggcca acagggcttc ctgggataca agagcgttct 120
   ccatggatct gccttactac catggacgtc tgaccaagca agactgtgag accttgctgc 180
   tcaaggaagg ggtggatggc aactttcttt taagagacag cgagtcgata ccaggagtcc 240
   tgtgcctctg tgtctcgttt aaaaatattg tctacacata ccgaatcttc agagagaaac 300
   acgggtatta caggatacag actgcagaag gttctccaaa acaggtcttt ccaagcctaa 360
   aggaactgat ctccaaattt gaaaaaccaa atcaggggat ggtggttcac cttttaaagc 420
   caataaagag aaccagcccc agcttgagat ggagaggatt gaaattagag ttggaaacat 480
   ttgtgaacag taacagcgat tatgtggatg tcttgccttg aagataaggc tgccggacaa 540
   agcaagttga agagatgagt aacagttctc actgatgacc cacttctgca ggcataggtc 600
   cagagcacca aactctagtg gacaattcag actctcctgg ttgtgtaact gaagatgttc 660
   tgcccaccag caccagaggt cactctccac atccccgcct cccagacata taccaggagc 720
   aatttcaaaa ccctctccag tttcactctt ctttcttgga atgggacagc ctgaacattt 780
   tccctttgac ttgttaaagg taccacccta catcatggtt gacaccctcc tttggaccat 840
   gcagtcagag gggcagcttt atacagagga ggggcacact tgtctgtgag tttgaagccc 900
   tgagttccag tcctgtggct gtgtgacttt gaacacgttt ctgcccatct ccaggtctta 960
   gtttctttgt ctctgtggtt gggtgggatg ataaacattg ccccagtctc tctgttgagc 1020
   ctgcttgtgt caggtgaagg tgagggatcg ggagtgatgg agatgcatac agatcagcac 1080
   cttctctgta cctgctggac ccccatgtgc acccttctcc ctccagcctg ggctactctc 1140
   ctcctggttc tttctaatct taaacttcct ggtgagcatt gccccagcca cactttctcc 1200
   ctggttttgc ttaattccac ttatgcatca gtcatttcaa cagcatgaat gtggttctgt 1260
   acagtgatgt atgtggagat ttctaaaata ttctatgtgt gatttctgtt cccaactagg 1320
   ttctaagtgc catcaaagca cggttgggtc tcccttgtat cttccacagt gtgtagaaat 1380
   ttgtgctgcc cagagctgtg cctagttcat agaaactgac tggaagcaca ttgctgaggg 1440
   aggtttgttg aattggtttt taaggtttat tgaaattgat ttgctgaatt tttctgctag 1500
   ttcaaaatgt gaattaggac ctggtcagtt tgaaatatac caaattctat gccctttctc 1560
   ttacctatca tcaaattgta gtaatgtatt tcaccccact ggacttatct tcagagtttt 1620
   aaaagagagg agctctcgac ttagaggtaa tatgaacaga tgaacagaca ctgtggctgg 1680
   agccccaaag tgtggagcat tgtgagattt ggggtcacag caatttatgg ctactattcc 1740
   ctgggtctgg taggtaggac aaatgtcctc ttttactttt caaaactggc ttaggtattc 1800
   atggaccttg attcttctat atacatttta taataaatac gtgaaattgc ttaaaacctc 1860
   tttttggaat ttttaatgaa cttgcaacaa atttgtaaat aggagagaac tgacgttctt   1920
   gtgatgtttt cccatgcata aacatgccac atccatgttc ttgggtcatc ttttttgccc   1980
   tttcatggag tcataaattt tcccaactga agtcttgtat attctgttag attaattcct   2040
   atttctagtt gctgtaaatg atatcttatg ttttattaca tttctaatag gatatggtgt   2100
   tggtgaaatg ttaaccctct cattagatct actagtttac ctgttgattc tattgtgttt   2160
   tctatgtaaa tgattttgtc agctataaat aataacattt tattttctct ttccttgcaa   2220
   tacctatgct catttatttt ttatgtttac ccattggtta gggcctcctg tatacattaa   2280
   acagttcata gtcatgatag tgagtattct tacactgttc ccagtattac agggaatgct   2340
   tagaaatttt ctttatttaa acattatgtt tgttgtagcc ttgttaaagg ctattttata   2400
   atttttacta gaaatatttt gacatttatt gtgatttttt tctatctcta atctattgag   2460
   atagtcacat tccttttgtc ttcactccat tataaaggta agttacctta ataaaattgt   2520
   tgatatcatt tcatcaaaaa aaaaaaaaaa aaa                                2553
<210> 103
   <211> 2481
   <212> DNA
   <213> Homo sapiens
<400> 103
   atggcggagc cctcggcccc ggagagcaag cacaagtcgt ccctcaactc gtccccgtgg 60
   agtggcctca tggccctggg aaacagccgg cacggccacc acgggcccgg ggcccagtgc 120
   gcgcacaagg cggcgggcgg cgcggcgccg ccgaagccgg ccccggcggg gctgtccggg 180
   gggctgtcgc agccggctgg gtggcagtcg cttctctcct tcaccatcct cttcctggcc 240
   tggcttgccg gcttcagctc gcgcctcttc gccgtcatcc gcttcgaaag catcatccac 300
   gagttcgacc cgtggtttaa ctatagatca acacatcatc ttgcatctca tgggttctat 360
   gaatttttaa attggtttga tgaaagagca tggtatccac taggaagaat agtaggtggt 420
   actgtttacc cagggttgat gataaccgct ggccttattc attggatttt aaatacattg 480
   aacataactg ttcacataag agacgtatgt gtgttccttg caccaacttt tagcggcctt 540
   acatctatat ctactttcct gcttacaaga gaactttgga accaaggagc aggactttta 600
   gctgcttgtt ttattgctat tgtaccaggc tacatatctc ggtcagtagc tggatccttt 660
   gataatgaag gcattgctat ttttgcactt cagttcacat actatttatg ggtaaaatct 720
   gtaaaaactg ggtcagtttt ttggacaatg tgctgctgct tatcctattt ctatatggtc 780
   tctgcttggg gtggttatgt atttatcatc aatcttattc cactgcatgt atttgtgttg 840
   ttactgatgc agagatacag caaaagagtc tacatagcat atagcacttt ctacattgtg 900
   ggtttaatat tatcaatgca gatacctttt gtgggattcc agccaatcag aacaagtgaa 960
   cacatggcag ctgcaggtgt ctttgcattg ctgcaagctt atgctttctt gcagtatctg 1020
   agagaccgat taacaaaaca agagttccag acccttttct ttttgggtgt atcactagct 1080
   gcaggtgctg tgttccttag tgtcatctat ttgacttata caggttacat tgcaccatgg 1140
   agtggcaggt tttattcatt gtgggatact gggtatgcaa aaatacacat tccaattatt 1200
   gcatcagtgt ctgagcatca acctacgact tgggtgtctt tcttctttga tctacatatt 1260
   cttgtatgta ccttcccagc aggcctttgg ttctgcatca aaaatatcaa cgatgaaaga 1320
   gtatttgttg ctctatatgc aatcagtgct gtctactttg ctggagtgat ggtgcgactg 1380
   atgttgactt tgactccagt cgtgtgtatg ctgtctgcaa ttgccttttc aaatgttttt 1440
   gagcactatt tgggggatga catgaaaagg gaaaatccac ctgtggagga cagcagtgat 1500
   gaggatgaca aaagaaacca aggaaatttg tatgataagg caggtaaagt gaggaaacat 1560
   gcaactgaac aggaaaaaac tgaagaggga ttaggcccta atataaaaag cattgtcacc 1620
   atgttgatgc tgatgctatt gatgatgttt gctgtccact gtacctgggt cacaagcaat 1680
   gcctactcta gtccaagtgt agtcctggcc tcatacaatc atgatggcac caggaatatc 1740
   ttagatgatt ttagagaagc ttacttttgg ctaaggcaaa atacagatga acatgcacga 1800
   gtaatgtctt ggtgggatta tggctatcag atagctggaa tggctaatag aactacgttg 1860
   gtggataata acacctggaa taacagccac atagcactgg tgggaaaagc tatgtcttct 1920
   aatgaaacag cagcctataa aatcatgagg actctagatg tagattatgt tttggttatt 1980
   tttggagggg ttattggcta ttctggtgat gatatcaaca aatttctctg gatggttagg 2040
   atagctgaag gagaacatcc caaagacatt cgggaaagtg actattttac cccacaggga 2100
   gaattccgtg tagacaaagc aggatcccct actttgttga attgccttat gtataaaatg 2160
   tcatactaca gatttggaga aatgcagctg gattttcgta cacccccagg ttttgaccga 2220
   acacgtaatg ctgagattgg aaataaggac attaaattca aacatttgga agaagccttt 2280
   acatcagaac actggcttgt taggatatat aaagtaaaag cacctgataa cagggagaca 2340
   ttagatcaca aacctcgagt caccaacatt ttcccaaaac agaagtattt gtcaaagaag 2400
   actaccaaaa ggaagcgtgg ctacattaaa aataagctgg tttttaagaa aggcaagaaa 2460
   atatctaaga agactgttta a 2481
<210> 104
   <211> 509
   <212> DNA
   <213> Homo sapiens
<400> 104
   attctttccc caagtctcta tggtagcgtc agcgtcggag gcggtagtga cggtggcgtt 60
   tccttgagga agagtgaggg ttccaacttt tctgcttatc tgggaggtgt tgggcgcgga 120
   cagtcgagat gtcagagaaa aagcagccgg tagacttagg tctgttagag gaagacgacg 180
   agtttgaaga gttccctgcc gaagactggg ctggcttaga tgaagatgaa gatgcacatg 240
   tctgggagga taattgggat gatgacaatg tagaggatga cttctctaat cagttacgag 300
   ctgaactaga gaaacatggt tataagatgg agacttcata gcatccagaa gaagtgttga 360
   agtaacctaa acttgacctg cttaatacat tctagggcag agaacccagg atgggacact 420
   aaaaaaatgt gtttatttca ttatctgctt ggatttattt gtgtttttgt aacacaaaaa 480
   ataaatgttt tgatataaaa aaaaaaaaa 509
<210> 105
   <211> 7389
   <212> DNA
   <213> Homo sapiens
<400> 105
   gtttctctct ctggtcggaa gcggcggtaa tggcggatgg tgggttgtgg cgccggcggc 60
   ggctgctgtg agggacgatg agtgcctcct tcgtgccgaa cggggccagc ctggaagatt 120
   gtcactgtaa cctcttctgc ctggctgact tgacaggaat taagtggaaa aaatatgtat 180
   ggcaaggccc aacttctgcc cctattctgt ttcctgtgac agaagaagac cccattttga 240
   gcagttttag tcgctgcctt aaggcagatg tacttggtgt ttggcggcga gatcaaagac 300
   ctggaagaag agaattgtgg atattttggt ggggtgaaga cccagttttg ctgaccttat 360
   tcaccatgac ttatcagaag aagaagatgg aatgtgggag aatggacttt cctatgaatg 420
   ccgtactctg cttttccaaa gcagttcaca atctattgga acggtgttta atgaacagga 480
   attttgtacg tattggcaag tggtttgtaa agccttatga aaaagatgaa aaacctataa 540
   ataaaagtga acacttgtcc tgctccttca ccttttcctt gcatggagac agcaatgttt 600
   gtaccagtgt ggaaattaac caacatcaac ctgtatacct tctcagtgaa gagcatatca 660
   cccttgctca acagtctaat agcccatttc aagttatctt atgcccattt ggactaaatg 720
   gcactctcac aggacaggca ttcaagatgt ctgattcagc tacaaaaaaa ttaattggtg 780
   aatggaaaca gttctatcct atctcatgtt gcttgaagga gatgtctgaa gaaaaacagg 840
   aagatatgga ttgggaagat gattctttag ctgcagtaga agttcttgtt gctggtgtcc 900
   gaatgatcta cccagcatgc tttgttctag tccctcagtc agacattcct actcctagcc 960
   ctgtgggatc cactcactgt tcatcttctt gcttgggtgt ccaccaagtg cctgcttcca 1020
   caagagatcc tgctatgtct tcggttacgc ttacaccacc tacgtctcct gaggaagtcc 1080
   aaacagttga tcctcagtct gtccagaagt gggtcaaatt ttcttcagta tctgatggct 1140
   tcaactccga tagtactagc caccatggtg ggaaaatacc cagaaaatta gcaaatcatg 1200
   tggtggatag agtttggcaa gaatgcaata tgaacagagc acagaacaag aagaagtatt 1260
   ctgcttcatc aggtggtcta tgcgaagaag cgacagctgc taaagtggca tcctgggatt 1320
   ttgttgaagc cacacaaaga acaaattgca gttgtttgag gcacaaaaat ctcaagtcaa 1380
   gaaatgctgg acaacaagga caggcaccat ctttaggtca gcaacaacaa atacttccta 1440
   agcacaagac caatgagaag caagaaaaga gtgaagagcc acagaaacgc cccttgactc 1500
   cttttcacca tcgtgtgtct gttagtgatg atgttggcat ggacgcagat tcagccagcc 1560
   aaagacttgt gatctctgct ccagacagtc aagtgagatt ttcaaatatc cgaactaatg 1620
   atgtagcaaa gactcctcag atgcatggca ccgaaatggc aaattcacct caaccacccc 1680
   cacttagtcc tcacccttgt gatgtggttg atgaaggagt gactaaaaca ccttcaactc 1740
   ctcagagtca acatttttat caaatgccaa caccagatcc cttggttcct tctaaaccaa 1800
   tggaagatag gatagacagt ttgtcccagt ctttcccacc tcaatatcag gaagctgtag 1860
   aacctacagt atatgttggt acagcagtaa acttggaaga agatgaagcc aatatagcct 1920
   ggaagtatta caagttccca aagaaaaaag atgtagagtt tttaccacct caacttccaa 1980
   gtgataaatt caaggatgat ccagttggac cttttggaca ggaaagtgta acatcagtta 2040
   cagagttaat ggtgcaatgt aagaaacctt taaaagtttc tgatgaatta gtgcagcaat 2100
   atcaaattaa aaaccagtgt ctttcagcaa tagcatctga tgcagaacaa gaacctaaaa 2160
   ttgatccata tgcatttgtt gaaggagatg aggaattcct ttttcctgat aaaaaagata 2220
   gacaaaatag tgagagagaa gctggaaaaa aacacaaggt agaagatggg acatctagtg 2280
   taacagtgtt atcacatgaa gaagatgcta tgtcattatt tagtccctct atcaagcaag 2340
   atgctccacg ccctactagt catgcccgtc ctccatcaac aagtttgatt tatgactcag 2400
   acctggctgt ctcttatact gaccttgata atctcttcaa ttctgatgaa gatgaactaa 2460
   cacctggatc taaaagatca gcaaatggat cagatgataa agccagctgc aaggaatcaa 2520
   agacaggaaa tctggacccg ttatcttgca taagcactgc agatcttcat aaaatgtatc 2580
   ctacaccacc atcattggaa caacatatta tgggattttc cccaatgaat atgaataata 2640
   aagaatatgg tagtatggat acaacacctg gaggaactgt tctagaagga aatagttcta 2700
   gtataggagc gcagttcaaa attgaggttg atgagggatt ctgtagcccc aaaccttctg 2760
   aaattaaaga tttttcttat gtctataagc ctgaaaattg tcaaattcta gtgggatgtt 2820
   ccatgtttgc acctctaaaa actctaccaa gccaatatct gccccttatc aaattgccag 2880
   aagagtgtat ttaccgtcag agttggactg ttggaaaatt ggaattgctt tcttcagggc 2940
   cttcaatgcc attcatcaaa gagggtgatg gaagtaatat ggatcaagaa tatggcactg 3000
   cttatacacc tcaaactcat acttcttgtg ggatgcctcc tagcagtgca cctcctagta 3060
   acagcggagc aggaattctt ccttctccat ccacccctcg gtttccaact ccaaggactc 3120
   caaggactcc tcggactcct cgtggagctg gtggacctgc tagtgctcaa ggttcagtca 3180
   aatatgaaaa ttcagacttg tattcaccag cttctacccc atctacatgc agacccctta 3240
   attctgttga acctgcaact gtcccttcca tccctgaagc acacagtctt tatgtaaacc 3300
   tcatcctttc agaatcagtt atgaatttgt ttaaagactg taactctgat agttgttgca 3360
   tctgtgtttg caacatgaac atcaagggtg ccgatgttgg agtttacatt ccagatccaa 3420
   cgcaggaagc acaatatagg tgtacctgtg gcttcagtgc tgtcatgaac agaaaatttg 3480
   gaaacaattc aggattattt cttgaagatg aactagatat cataggacgc aatacagact 3540
   gtggcaaaga agcagaaaaa cgttttgaag ctctcagggc tacctctgct gaacatgtta 3600
   atggaggact aaaggaatct gaaaaattat ctgatgattt gatattattg ctacaagatc 3660
   agtgcactaa tttattttca ccctttggag cagcagacca agatcctttt cctaaaagtg 3720
   gtgtaattag caattgggta cgtgttgaag agcgtgactg ttgcaatgac tgctaccttg 3780
   cattagaaca tgggcgtcag ttcatggata acatgtcagg aggaaaagtt gatgaagcac 3840
   ttgtgaaaag ttcatgctta cacccctggt ccaaaagaaa cgatgtgagt atgcagtgct 3900
   cacaggatat acttcgaatg ctcctctctc ttcagccagt tcttcaggat gccattcaga 3960
   aaaaaagaac agtaagacct tggggtgttc agggtcctct cacttggcaa caatttcata 4020
   aaatggctgg ccgaggctct tatggaactg atgaatcccc agaaccactg ccaatcccca 4080
   catttttgtt gggttatgat tatgattatc tggtgctttc tccatttgct cttccttatt 4140
   gggagagact tatgctggaa ccctatggat ctcaaagaga tatagcctat gttgtactgt 4200
   gtccagaaaa tgaagccttg ttaaatggag caaaaagctt ttttagagat cttactgcaa 4260
   tatatgagtc ctgtcgatta ggtcaacata gacctgtttc tcgactgtta acagatggga 4320
   tcatgagagt tggatctact gcatcaaaga aactatcaga aaagttggta gcagaatggt 4380
   tttctcaggc agctgatggt aacaatgaag cattttctaa actcaagctt tatgcacaag 4440
   tctgcagata tgacctaggt ccttatcttg cttccctgcc attggacagc tctctacttt 4500
   cccagccaaa tttagttgcc cctacaagtc agtctttgat tactccacct cagatgacaa 4560
   atactggaaa tgctaatact ccatctgcca ccttagcatc tgcagcgagc agcactatga 4620
   cagtgacttc aggtgttgcc atatctactt cagttgccac agctaattca actttgacca 4680
   cagcttcaac ttcatcttca tcatcctcca acttgaatag tggagtatca tcaaataaac 4740
   taccttcgtt tccacccttt ggcagtatga acagtaatgc tgcaggatcc atgtctacac 4800
   aagcaaatac agttcagagt ggtcagctag gagggcaaca gacatcagct ctacagacag 4860
   ctgggatttc tggagaatca tcttcacttc ccactcagcc gcatcctgat gtgtctgaaa 4920
   gcacgatgga tcgggataaa gtgggaatcc ccacagatgg tgattcacat gcagtcacgt 4980
   atccacctgc aattgttgtt tatataattg atccttttac atacgaaaat acagacgaga 5040
   gcactaactc ttctagtgtg tggacattgg ggctacttcg atgctttcta gaaatggtcc 5100
   agactcttcc tcctcatatc aagagtactg tttctgtaca gattattcct tgtcagtacc 5160
   tgttgcaacc tgtgaagcat gaagatagag aaatctatcc ccagcattta aaatccctgg 5220
   ctttttcggc ctttacccag tgtcggaggc cacttccaac atcaaccaat gtgaaaacat 5280
   tgactggctt tggtccaggt ttagccatgg aaactgccct tagaagtcct gatagaccag 5340
   agtgtattcg actttatgca cctcctttta ttctggctcc agtgaaggac aaacagacag 5400
   agctaggaga aacatttgga gaagctggac agaaatataa tgttcttttt gtgggatact 5460
   gtttatcaca tgatcaaagg tggattcttg catcttgcac agatctatat ggagaacttt 5520
   tagaaacttg tatcattaac atcgatgttc caaatagggc tcgtcggaaa aaaagttctg 5580
   ctagaaaatt tggtctacag aaactttggg agtggtgctt aggacttgta caaatgagtt 5640
   cattgccatg gagagttgta attggtcgtc taggaaggat tggtcatgga gaattgaaag 5700
   attggagctg tttgctgagt cgtcgaaact tgcagtctct aagtaaaagg ctcaaagaca 5760
   tgtgtagaat gtgtggtata tctgctgcag actcccctag cattctcagt gcttgcttgg 5820
   tggcaatgga gccgcaaggc tcttttgtta ttatgccaga ttctgtgtca actggttctg 5880
   tatttggaag aagcacgact ctaaatatgc agacatctca gctaaatacc ccacaggata 5940
   catcatgtac tcatatactt gtgtttccta cttctgcttc tgtgcaagta gcttcagcta 6000
   cttataccac tgaaaatttg gatttagctt tcaatcccaa caatgatgga gcagatggaa 6060
   tgggtatctt tgatttgtta gacacaggag atgatcttga ccctgatatc attaatatcc 6120
   ttcctgcttc tccaactggt tctcctgtac attctccagg atctcattac ccccatggag 6180
   gtgatgcggg caagggtcag agtactgatc ggctactatc aacagaacct catgaggaag 6240
   tacctaatat tcttcagcaa ccattggccc ttggttactt tgtatcaact gccaaagcag 6300
   gtccattacc tgactggttc tggtcagcat gtcctcaagc acaatatcag tgtccccttt 6360
   ttcttaaggc ctctttgcac ctccacgtgc cttcagtgca atctgacgag ctgcttcaca 6420
   gtaaacactc ccacccactt gactcaaatc agacttcaga tgtcctcagg tttgttttgg 6480
   aacagtacaa tgcactctcc tggctaacct gtgaccctgc aacccaggac agacgctcat 6540
   gtctcccaat tcattttgtg gtgctgaatc agttatataa ctttattatg aatatgctgt 6600
   gatcttcatt tgatggaact gtgcaagaaa agaacaagga aaaatggatg tttcgctgca 6660
   ggattaagtt acaattatct tctcagtgaa ggtcatttgt gatggggtct aattcttatt 6720
   acttcaacaa atattgtttt gacttggggg gaggggctat aaccctgcta tttttcattg 6780
   actctattga actctttagg atgatgactg atcatacaaa acgtattata acattttcgt 6840
   agcaaaatta accttttttt tttccagtca cagtatttgt gaaaagtaat gagccatagt 6900
   acccagtcat gttaaatgaa tattaaaagc atggagagga aacatgagga acaatgaatt 6960
   tcaacatatg gcttcagaac atgaagatgt tcttgtatgg attatagtat ctagtattca 7020
   aaaatgcctg catctcttct cttatttatt gtaagttttt aaatgtataa attgtcttat 7080
   atttcttaac ctcttttata aaaattttcc tagaaggttt atactgcctt cttgctttaa 7140
   agcaattggt ctaaaatata tgtaatcgtc ttaattaaaa agttgcagta gggttgcttt 7200
   tagagtatta tttttttgta agggggtggg tgggacagta aatttgtatt gtctcgatgt 7260
   acagtttaac ggggatagag ggggaataat gtccatacca ttgtgtgtgg aggatttaca 7320
   gctaagctgt agttgcagag tacatgtaca gtaatgaagt tcactgtgtt tataaattga 7380
   aaaggtacc 7389
<210> 106
   <211> 6428
   <212> DNA
   <213> Homo sapiens
<400> 106
   gctagtggaa gttactgccg cgccaccgag tccggaccgg agactttggg gcctaactag      60
   tgaatggtag tgtctagaaa gggtatgtcc cttcaagaga gaggtgccaa tgtccaaccg     120
   gcctaataac aatccagggg ggtcactgcg acgttcacag aggaacactg ccggggccca     180
   accacaagac gactcaatag gaggaagaag ctgcagttca tcatctgctg tgatagttcc     240
   acaaccagag gatccagaca gagccaatac ttcagaaaga caaaaaacgg ggcaggtgcc     300
   taagaaagac aattctcgag gagtgaagcg cagtgctagt ccagactaca acaggaccaa     360
   ttctcctagc tctgcaaaaa aaccaaaagc acttcagcat actgaatctc cctcagaaac     420
   aaataagcca catagtaagt caaagaagag acatttagac caggagcaac aactgaaatc     480
   tgcacaatca ccatcaacaa gcaaggctca taccaggaag agtggggcca ctggcggttc     540
   acggagtcag aaaagaaaaa ggacagagag ttcttgtgta aagagtggct ccgggtctga     600
   atcaactggt gcagaagaga gatctgcgaa acctaccaag ctggcttcaa aatcagccac     660
   ctcagccaaa gctgggtgta gcaccatcac tgattcttct tctgctgcct ctacttcctc     720
   ctcgtcttct gctgtagcct cggcctcctc cactgtacca ccaggtgcca gagtgaaaca     780
   aggaaaagat cagaacaagg ccaggcgttc ccgttcagcg tccagtccca gccccagaag     840
   aagtagcagg gaaaaggaac agagtaaaac tggtggctct tcaaaatttg attgggctgc     900
   tcgtttcagc cctaaagtta gccttcctaa aacaaaactg tctcttccag ggtcttctaa     960
   gtcagagaca tcaaaacctg gaccttctgg attacaggcc aaattagcaa gtttaagaaa    1020
   atctacgaag aaacgcagtg agtctccacc tgctgagctc cccagtttga ggcggagcac    1080
   acgccaaaag accacgggct cctgtgctag taccagtcgg cgaggctctg gcctgggcaa    1140
   aagaggagca gctgaagctc gtcgacagga gaaaatggca gaccctgaaa gcaaccagga    1200
   ggcagtaaat tcttcagctg ctcggacaga tgaagctccc caaggagctg caggggctgt    1260
   tggcatgacc acctctgggg agagtgaatc agatgattcc gagatgggac gtttgcaagc    1320
   tttgttagag gcaaggggtc ttccccctca cctatttggt cctcttggtc ctcggatgtc    1380
   acagcttttc catagaacaa ttggaagtgg agctagttct aaggcccagc agctactaca    1440
   aggattgcaa gccagtgatg aaagtcaaca gcttcaggca gttattgaga tgtgtcagtt    1500
   actggtcatg ggaaatgagg agacactggg agggtttcct gtcaagagtg ttgttccagc    1560
   tttgattacg ttacttcaga tggagcacaa ttttgatatt atgaaccatg cttgtcgagc    1620
   cttaacatac atgatggaag cacttcctcg atcttctgct gttgtagtag atgctattcc    1680
   tgtcttttta gaaaagctgc aagttattca gtgtattgat gtggcagagc aggccttgac    1740
   tgccttggag atgttgtcac ggagacatag taaagccatt ctacaggcgg gtggtttggc    1800
   agactgcttg ctgtacctag aattcttcag cataaatgcc caaagaaatg cattagcaat 1860
   tgcagctaat tgctgccaga gtatcacgcc agatgaattt cattttgtgg cagattcact 1920
   cccattgcta acccaaaggc taacacatca ggataaaaag tcagtagaaa gcacttgcct 1980
   ttgttttgca cgcctagtgg acaacttcca gcatgaggag aatttactcc agcaggttgc 2040
   ttccaaagat ctgcttacaa atgttcaaca gctgttggta gtgactccac ccattttaag 2100
   ttctgggatg tttataatgg tggttcgcat gttttctctg atgtgttcca actgtccaac 2160
   tttagctgtt caacttatga aacaaaacat tgcagaaacg cttcactttc tcctgtgtgg 2220
   tgcctccaat ggaagttgtc aggaacagat tgatcttgtt ccacgaagcc ctcaagagtt 2280
   gtatgaactg acatctctga tttgtgaact tatgccatgt ttaccaaaag aaggcatttt 2340
   tgcagttgat accatgttga agaagggaaa tgcacagaac acagatggtg cgatatggca 2400
   gtggcgtgat gatcggggcc tctggcatcc atataacagg attgacagcc ggatcattga 2460
   gcaaatcaat gaggacacgg gaacagcacg tgccattcag agaaaaccta acccgttagc 2520
   caatagtaac actagtggat attcagagtc aaagaaggat gatgctcgag cacagcttat 2580
   gaaagaggat ccggaactgg ctaagtcttt tattaagaca ttatttggtg ttctttatga 2640
   agtgtatagt tcctcagcag gacctgcggt cagacataag tgccttagag caattcttag 2700
   gataatttat tttgcggatg ctgaacttct gaaggatgtt ctgaaaaatc atgctgtttc 2760
   aagtcacatt gcttccatgc tgtcaagcca agacctgaag atagtagtgg gagcacttca 2820
   gatggcagaa attttaatgc agaagttacc tgatattttt agtgtttact tcagaagaga 2880
   aggtgtaatg catcaagtaa aacacttagc agaatcagag tctttgttga caagtccacc 2940
   aaaggcatgt acgaatggat cgggatccat gggatccaca acttcagtca gcagtgggac 3000
   agccacagct gccactcatg ctgcagctga cttgggatca cccagcttgc agcacagcag 3060
   ggatgattct ttagatctca gccctcaagg tcgattaagt gatgttctaa agagaaaacg 3120
   actgccaaaa cgagggccaa gaaggccaaa gtactcacct ccaagagatg atgacaaagt 3180
   agacaatcaa gctaaaagcc ccaccactac tcagtcacct aaatcttctt tcctggcaag 3240
   cttgaatcca aaaacatggg gaaggttaag tacacagtcc aacagcaaca acattgagcc 3300
   agcacggact gcgggaggta gtggccttgc cagggctgcc tcaaaggata ccatctccaa 3360
   taatagagaa aaaattaaag gttggattaa ggagcaggca cataaatttg tagaacgtta 3420
   tttcagttct gagaatatgg atggaagcaa ccctgcattg aatgtccttc agagactttg 3480
   tgctgcaacc gaacaactca acctccaggt ggatggtgga gctgagtgcc ttgtagaaat 3540
   ccgtagcata gtctcagagt cagatgtttc atcatttgaa atccaacata gtggatttgt 3600
   gaagcagctg ttgctttatt tgacatctaa aagtgaaaag gatgctgtga gcagagagat 3660
   cagattaaag cgatttcttc atgtattttt ttcttctcca cttcctggag aagagcccat 3720
   tggaagagtg gaaccagtgg gtaatgcacc tttgttggca ttagttcaca agatgaacaa 3780
   ctgcctcagc cagatggaac aatttccagt caaagtacat gatttcccta gtggaaatgg 3840
   gacaggaggc agcttttctc tcaacagagg atcacaggct ttaaaatttt tcaacacaca 3900
   tcaattaaaa tgccagttac aaaggcatcc agactgtgca aatgtgaagc agtggaaggg 3960
   tggacctgtc aagattgacc ctctggcttt ggtacaagcc atcgagagat accttgtagt 4020
   tagagggtat ggaagagtaa gagaagatga tgaagacagc gatgacgatg gatcagatga 4080
   ggaaatagat gagtctctgg ctgctcagtt cctaaattca ggaaatgtaa gacacaggct 4140
   gcagttttat attggagaac atttgctgcc gtataacatg actgtgtatc aggcagtacg 4200
   gcagtttagt atacaggctg aagatgaaag agaatccaca gatgatgaga gcaatcctct 4260
   aggcagagct ggtatttgga caaagactca tacaatatgg tataaacctg tgagagagga 4320
   tgaagaaagt aataaagatt gtgttggtgg taaaagagga agagcccaaa cagctccaac 4380
   gaaaacttcc cctagaaatg caaaaaagca tgatgagtta tggcacgatg gagtgtgccc 4440
   atcagtatca aatcctttag aagtttacct cattcccaca ccacctgaaa atataacatt 4500
   tgaagacccg tcattagatg tgatccttct tttaagagtt ttacatgcta tcagtcgata 4560
   ctggtattac ttgtatgata atgcaatgtg caaggaaatt attccaacta gtgaatttat 4620
   taacagtaag ttaacagcaa aagcaaatag gcaacttcaa gatcctttag taatcatgac 4680
   aggaaacatc ccaacatggc ttactgagct aggaaaaacc tgcccatttt tctttccttt 4740
   tgatacccgg caaatgcttt tttatgtaac tgcatttgat cgggaccgag caatgcaaag 4800
   attacttgat accaacccag aaatcaacca gtctgattct caagatagca gagttgcacc 4860
   tagattggat agaaaaaaac gtactgtgaa ccgagaggag ctgctgaaac aggcggagtc 4920
   tgtgatgcag gacctcggca gctcacgggc catgttagaa atccagtatg aaaatgaggt 4980
   tggtacaggt cttgggccta cactggagtt ttatgcgctt gtatctcagg aactacagag 5040
   agctgacttg ggtctttgga gaggtgaaga agtaactctt agcaatccaa aagggagcca 5100
   agaagggacc aagtatattc aaaacctcca gggcctgttt gcgcttccct ttggtaggac 5160
   agcaaagcca gctcatatcg caaaggttaa gatgaagttt cgcttcttag gaaaattaat 5220
   ggccaaggct atcatggatt tcagattggt ggaccttccc cttggcttac ccttttataa 5280
   atggatgcta cggcaagaaa cttcactgac atcacacgat ttgtttgaca tcgacccagt 5340
   tgtagccaga tcagtttatc acctagaaga cattgtcaga cagaagaaaa gacttgaaca 5400
   agataaatcc cagaccaaag agagtctaca gtatgcatta gaaaccttga ctatgaatgg 5460
   ctgctcagtt gaagatctag gactggattt cactctgcca gggtttccca atatcgaact 5520
   gaagaaagga gggaaggata taccagtcac tatccacaat ttagaggagt atctaagact 5580
   ggttatattc tgggcactaa atgaaggcgt ttctaggcaa tttgattcgt tcagagatgg 5640
   atttgaatca gtcttcccac tcagtcatct tcagtacttc tacccggagg aactggatca 5700
   gctcctttgt ggcagtaaag cagacacttg ggatgcaaag acactgatgg aatgctgtag 5760
   gcctgatcat ggttatactc atgacagtcg ggctgtgaag tttttgtttg agattctcag   5820
   tagttttgat aatgagcagc agaggttatt tctccagttt gtgactggta gcccaagatt   5880
   gcctgttgga ggattccgga gtttgaatcc acctttgaca attgtccgaa agacgtttga   5940
   atcaacagaa aacccagatg acttcttgcc ctctgtaatg acttgtgtga actatcttaa   6000
   gttgccggac tattcaagca ttgagataat gcgtgaaaaa ctgttgatag cagcaagaga   6060
   agggcagcag tcgttccatc tttcctgatt atagcaagaa atgcagtgtc tgcctgttac   6120
   agcaaaagaa acaaatcatg atttcttttc taatgttatc acctgagtca aggaaacatg   6180
   ttacgccttc ttgttgtagg aaaaacggct tgcagattat aaagagacat ttggttgata   6240
   ttcattaatg gccccatgga cttaaagtga tcaggcccta aaacgttgtt gtgatgaggt   6300
   ttctttagca agttcttgtt taaattatca tttatttgat gagtgaagtt tttaacatgc   6360
   tttgctgtgt gaaatttaaa aaagggatgt ttttccaggc tggaacaata aatgtggctg   6420
   tgcagttt                                                            6428
<210> 107
   <211> 497
   <212> DNA
   <213> Homo sapiens
<220>
   <221> misc_feature
   <222> (72)..(72)
   <223> n is a, c, g, t or u
<220>
   <221> misc_feature
   <222> (94)..(94)
   <223> n is a, c, g, t or u
<220>
   <221> misc_feature
   <222> (253)..(253)
   <223> n is a, c, g, t or u
<220>
   <221> misc_feature
   <222> (370)..(370)
   <223> n is a, c, g, t or u
<220>
   <221> misc_feature
   <222> (404)..(404)
   <223> n is a, c, g, t or u
<220>
   <221> misc_feature
   <222> (414)..(414)
   <223> n is a, c, g, t or u
<220>
   <221> misc_feature
   <222> (472)..(472)
   <223> n is a, c, g, t or u
<400> 107
   tttttttttt tttttttttt gttttaaact acaaaggatg ttgcatacac tgatgaatct      60
   gtatctgact anaagtgctc aggggttgga aggngacaga aaacaaaggc aaaatttgca     120
   ttgcctttcc atctgaaatg aaaaactgca caactattaa ccaacattta ataggataat     180
   ttttaaacag ctcatgcgtc aacaagataa atttctttct agacaagaga caagactgaa     240
   tccaataaaa agngtcatag ctccagttgg gagagaaaaa aaaaaaacta cagaatattt     300
   atgatagcat tacaaacagg atcctacgca actatgcaaa agaggttgcc cacatttgta     360
   ttgaacaaan gctttgaatt cttgcccttc cactcttctc ttgngacttt ctgngctgtc     420
   agccctagta ttcgctgtca accacaatac taagctgtag gacaaatcca gnggtccaat     480
   gaggctgatt acacctt                                                    497
<210> 108
   <211> 5911
   <212> DNA
   <213> Homo sapiens
<400> 108
   aattgcctct caccccccgg aggggttatt ttgggggtgg ttggaggcgg tggcggcggc 60
   ggcgaggagg ggaatttcct tgtgcctcca ttcccgggag gggggagcgg cgttggaggc 120
   caccgtttcc agcatcaaca acagcaactt gtgattggcg gtgaccggat attcagttgc 180
   acatccccac atcaatgcac tgccaatggg ttatatcctg tgttgtgacc tcatggttta 240
   agtgggaata aagatgagta taagcagtga tgaggtcaac ttcttggtat atagatactt 300
   gcaagagtca ggattttctc attcagcatt tacctttggt atagaaagcc atatcagtca 360
   gtccaatata aatggtgccc tcgtcccacc cgctgcattg atttctatca tccagaaagg 420
   tctacagtat gtagaagcag aagttagtat taatgaggat ggtaccttgt ttgatggtcg 480
   accaatagag tctctgtccc tgatagatgc cgtaatgcct gatgtagtac aaacaagaca 540
   acaagcttat agagataagc ttgcacagca acaggcagca gctgctgcag ctgccgcagc 600
   tgcagccagc caacaaggat ctgcaaaaaa tggagaaaac acagcaaatg gggaggagaa 660
   tggagcacat actatagcaa ataatcatac tgatatgatg gaagtggatg gggatgttga 720
   aatccctcct aataaagctg ttgtgttgcg gggccatgaa tctgaagttt ttatctgtgc 780
   ctggaaccct gttagtgatc tcctagcatc agggtctgga gactcaacag caagaatatg 840
   gaatcttagt gagaacagca ccagtggctc tacacagtta gtacttagac attgtatacg 900
   agaaggaggg caagatgttc caagcaacaa ggatgtcaca tctctagatt ggaatagtga 960
   aggtacactt ctagcaactg gttcctatga tgggtttgcc agaatatgga ctaaagatgg 1020
   taaccttgct agcaccttag ggcagcataa aggccctata tttgcattaa aatggaataa 1080
   gaaaggaaat ttcatcctaa gtgctggagt agacaagact acaattattt gggacgcaca 1140
   tactggtgaa gccaagcaac agtttccttt tcattcagca ccagcattgg atgttgattg 1200
   gcagagcaac aacacctttg cttcttgtag tacagatatg tgcattcatg tctgtaaatt 1260
   aggacaagac agacctatta aaacattcca aggacatacg aatgaagtaa atgctatcaa 1320
   atgggaccca actggcaatc tcttggcctc ctgttctgac gacatgactt taaagatatg 1380
   gagtatgaaa caagacaatt gtgtccatga tttgcaagca cataataaag aaatttatac 1440
   tatcaaatgg agtccaacag gaccagggac taataatcca aatgccaacc ttatgttagc 1500
   aagtgcatcc tttgattcta ctgttaggtt atgggatgta gaccgaggga tatgcatcca 1560
   taccttgaca aaacaccaag agcctgtgta cagtgtagct ttcagtcctg atggcaggta 1620
   tctggcaagt ggttcttttg acaaatgtgt acacatctgg aacacgcaga caggtgctct 1680
   agttcacagc tataggggaa caggtggaat atttgaagtt tgctggaatg cagcaggaga 1740
   caaagttgga gccagtgcat cagatggttc agtttgtgta ttagaccttc ggaaatagcg 1800
   ctactagttg gaagccatgg accgactatg aatgtgtaca tagccaaaat gactgtccct 1860
   gacccatgta ctgctatagt cccacttgaa ccatggccag tccactacag ccaaatctaa 1920
   aagaaatata tacatacagt gtatataaac aaaattgcac cctgaagatg acagagtttt 1980
   gtcacagctt gtgaattctg ttcaccaagt gctggaatct aatctgctgt gcccctaaaa 2040
   tagcatttag aagttttgga tatgaaaaac agaagagaga aaaatataca ttataaaagc 2100
   agaacataca tgtaccagtt tttggatact aaatgacagc cttgtttctc ccctttgaat 2160
   cagcagacac catggattat attctttttt tcccttcagt agtgagcagt ttgtatgtac 2220
   agagaaaatg gacttacaaa aacttgcagc agtagtttgt tcttgcttta aaatttcgtt 2280
   tttggtttag attatggatg catgaagtaa gggagtgaat cagtttcttg tttatatttt 2340
   tttcaccttt taaacaaaaa attctttaaa atattttaat gcattctttt gaagaggtag 2400
   atgtttggta cattttatgg ctcccagagc atatattcag ttggtgcatg ttgtggaagg 2460
   gggaattgga aattaaatga aaacctatga ctttggtcat gtcaatctgt aagacacatc 2520
   agtaaaaggg tattatgctc tgttggtttt gtttttttgt tttgcttttt tttttttttt 2580
   ttcttttttg ttttttggtg atgtggctta aatgcaatag tttctttttt gggacatatt 2640
   tctgccaatt aaagactaga agggcacaac ttttttttta attaccatag agaagataca 2700
   ttaaaaaaaa tcttctgatg ttttgtagcc ataactaaat tatggtaaaa atgtgcacta 2760
   ttgtgaaaag gagcaacgta gttttgggtt ttttgttgtt tgtttgtttt gctttgtttt 2820
   ttaagagatt aaaatgtttc tggataagga ttagcttctc gaagtgtcca tcattctgtg 2880
   tagaagctta aatatgtaat gtaaccaaac tccagtatta aaaatctctc atgttgtttt 2940
   ctttatacaa agcaagataa cggcatataa cactgccatt acatggcaaa atgtttgcta 3000
   ccttagttta aaaaacaatc tcaaacaaaa gacttgcttc aaggtgtttt taaatagcag 3060
   tgattcagaa ttttttttaa tgaaagtata attgcactaa ccttcttcct gctgctctga 3120
   ttctgcattt gtggtacttg tgactacgtt ttttcaaata tagatagatt taagctgcta 3180
   attttttttt ttttagtaat cactactata tcatgtcttt tactctgttt ataatatcaa 3240
   gtattttctt aaagatatag atattaaacc ttgtgctcat gcaacttaga gtaacatata 3300
   cagacaaatg attgcatgag gccatgttta tatgtgtgac taataaggct tgtcatgatt 3360
   aacataatcc aggtatgtca tttctgaaga gaatagtcat caaatttata tctcgaagat 3420
   tttaattaag gaattgctta ttgttgagct tagcaaatta ataacactat ttctgtcact 3480
   aattattttg aggcctttta gtactaaaat tttaacctgt gttctaagta gaaactgatt 3540
   taacccaagt aatgcagctt tgattgattt cagcattcgt tgctttgcta tttttacaaa 3600
   acagcattga ttgaagcaag tcttggtttt actaaggtag ggtagcattt gctattggta 3660
   aagagaataa atacacttaa tttcacaata cattgttata tgtaccccag ttgttgttag 3720
   tggggactat gatactgtaa taatattttt aaaaatttac atcaagagag gcagtcattc 3780
   acgatggttt tgtgccagct ctttttaggg ttttggatca cattagagat atttagaaca 3840
   tattaccctg tgacttacgt aggaaaccta atatgctgag tatctggcac ttgaattcct 3900
   gcttttattg ctggaggtcc acatctgtgg ttgacctctg ttattgttta aaaaaaataa 3960
   ataaaaatta aaaaaatctg tgcaataatt ttaaaatgtg ctcccaggaa tagacacaaa 4020
   tgttttgagt atcttttaag ctgcattttc ctttagtgat gcatttgtca attgcactga 4080
   atttaaatct gaaagtcaga ggtgattatt gatagtactt ttgtattttg atatggacag 4140
   tttattcatt tgcatacagt tattgacttt ttcccagctg attaaaagat agtcaagaaa 4200
   ttctgcaata tagctgccaa aatagacagc tacattttta tgatattgtc atcttttctg 4260
   tttttttttt cttttttttc tttagctatt ttacttaagc ataatagcca caataggaca 4320
   tataaaagat tataaataca gagctttatt atcctgacgt cttgggtctt ttaagtatat 4380
   acttttctga aaggtatcca ttttgtaggc ttgggttctt catgagcata cgattgttta 4440
   tttttgctgc tgttctcaac atcatcattg cctgctgatg tgccacgatg ctgctccaat 4500
   agacagcaat aagattgtct ctaatttgag cagtaacatg attgcaagag accaagtttc 4560
   acagcttgta aagttctgta tttgggattc ttgcttattt ttccgcctgt gtttttctga 4620
   gaacttattc ctgatgatca attgaatcca gtagtttttc tatgctattt gttgttgtat 4680
   aagctactgt aagaaactta tcataaggaa aaatagaaag gaaaacttga atcaatactc 4740
   attgattaaa atggaataaa gaaagagcag ctgccacttt taaacaacat aaaggaatat 4800
   ctttttttgt ctccgtgtag gaaatcccat aagttcttat atttgttcca gttcccattt 4860
   cctgccattg accagataac atcattgact ttcaaatgac ttttagaagt gataactctt 4920
   aatttcctaa tagatactag attgtattga attctgtttt aattattctc taggtaagta 4980
   tgttttagga ttaaatacct tttacagata ctgaaagtgc ctccttttgt ggtgtaaaaa 5040
   acaaattatg gtgcaaaaag taatcactag attgaaatac atgaaggttt tttgcttttt 5100
   gacatacgaa aatgtcaaga gaaaggccaa agatttgtac tttttcactt acaaagcact 5160
   cctttttccc ttaaacttct ttctgtcaaa ttagatttaa tgagagagta ctatttttaa 5220
   ggagctatct gtttatgtag aatgattttg ttaagagtaa tgtaaactat tattgagtag 5280
   aggcctaaag aggactgtgc atttttgcta tttaaaggaa tcacaaatga tcatacttaa 5340
   gtgagcaaaa atgacaagtt ttactagcta agtagagaaa taaatctcaa atgcagcgct 5400
   acaattttca ttatcttaag cacattgtac atttctacag aacctgtgat tattctcgca 5460
   tgataaggat ggtacttgca tatggtgaat tactactgtt gacagtttcc gcagaaatcc 5520
   tatttcagtg gaccaacatt gtggcatggc agcaaatgcc aacattttgt ggaatagcag 5580
   caaatctaca agagaccctg gttggttttt cgttttgttt tctttgtttt ttcccccttc 5640
   tcctgaatca gcagggatgg aaggagggta gggaagttat gaattactcc ttccagtagt 5700
   agctctgaag tgtcacattt aatatcagtt ttttttaaac atgattctag ttaaatgtag 5760
   aagagagaag aaagaggaag tgttcacttt tttaatacac tgatttagaa atttgatgtc 5820
   ttatatcagt agttctgagg tattgatagc ttgctttatt tctgccttta cgttgacagt 5880
   gttgaagcag ggtgaataac tagggcatat a 5911
<210> 109
   <211> 1736
   <212> DNA
   <213> Homo sapiens
<400> 109
   cgaccctcag cagccagccc ggcccgcccg cgcccgtccg cagccgcccg ccagacgcgc     60
   ccagtatgag ggagatcgtg cacatccagg ccggccagtg cggcaaccag atcggggcca    120
   agttctggga agtcatcagt gatgagcatg gcatcgaccc cagcggcaac tacgtgggcg    180
   actcggactt gcagctggag cggatcagcg tctactacaa cgaggcctct tctcacaagt    240
   acgtgcctcg agccattctg gtggacctgg aacccggaac catggacagt gtccgctcag    300
   gggcctttgg acatctcttc aggcctgaca atttcatctt tggtcagagt ggggccggca    360
   acaactgggc caagggtcac tacacggagg gggcggagct ggtggattcg gtcctggatg    420
   tggtgcggaa ggagtgtgaa aactgcgact gcctgcaggg cttccagctg acccactcgc    480
   tggggggcgg cacgggctcc ggcatgggca cgttgctcat cagcaaggtg cgtgaggagt     540
   atcccgaccg catcatgaac accttcagcg tcgtgccctc acccaaggtg tcagacacgg    600
   tggtggagcc ctacaacgcc acgctgtcca tccaccagct ggtggagaac acggatgaga    660
   cctactgcat cgacaacgag gcgctctacg acatctgctt ccgcaccctc aagctggcca    720
   cgcccaccta cggggacctc aaccacctgg tatcggccac catgagcgga gtcaccacct    780
   ccttgcgctt cccgggccag ctcaacgctg acctgcgcaa gctggccgtc aacatggtgc    840
   ccttcccgcg cctgcacttc ttcatgcccg gcttcgcccc cctcacagcc cggggcagcc    900
   agcagtaccg ggccctgacc gtgcccgagc tcacccagca gatgttcgat gccaagaaca    960
   tgatggccgc ctgcgacccg cgccacggcc gctacctgac ggtggccacc gtgttccggg   1020
   gccgcatgtc catgaaggag gtggacgagc agatgctggc catccagagc aagaacagca   1080
   gctacttcgt ggagtggatc cccaacaacg tgaaggtggc cgtgtgtgac atcccgcccc   1140
   gcggcctcaa gatgtcctcc accttcatcg ggaacagcac ggccatccag gagctgttca   1200
   agcgcatctc cgagcagttc acggccatgt tccggcgcaa ggccttcctg cactggtaca   1260
   cgggcgaggg catggacgag atggagttca ccgaggccga gagcaacatg aacgacctgg   1320
   tgtccgagta ccagcagtac caggacgcca cggccgagga agagggcgag atgtacgaag   1380
   acgacgagga ggagtcggag gcccagggcc ccaagtgaag ctgctcgcag ctggagtgag   1440
   aggcaggtgg cggccggggc cgaagccagc agtgtctaaa cccccggagc catcttgctg   1500
   ccgacaccct gctttcccct cgccctaggg ctcccttgcc gccctcctgc agtatttatg   1560
   gcctcgtcct ccccacctag gccacgtgtg agctgctcct gtctctgtct tattgcagct   1620
   ccaggcctga cgttttacgg ttttgttttt tactggtttg tgtttatatt ttcggggata   1680
   cttaataaat ctattgctgt cagataccct taaaaaaaaa aaaaaaaaaa aaaaaa       1736
<210> 110
   <211> 6138
   <212> DNA
   <213> Homo sapiens
<400> 110
   acttccggtt gctgtgctga gtcggaagtg ggaacccttc ggccgctgag attctgtcgt     60
   gtcgtcgctg ctggcacttc aggctctgcc tctcccacta ggtctggatg gaggatacct    120
   taaagtgaaa tgacagacca ggagaataac aacaacatct caagtaaccc ctttgctgct    180
   ctttttggct ccctggctga tgccaaacag tttgcggcaa tccaaaaaga gcagctgaag    240
   caacaatctg atgaactccc agctagccca gatgactcgg ataatagcgt gtcagagagc    300
   ctggatgaat tcgattactc tgtggctgag attagccgct cattccgatc acagcaggaa    360
   atatgtgagc aactcaacat caatcacatg atccaaagga tcttccttat tactctggac    420
   aacagtgatc ccagcttgaa aagcgggaat ggcatcccta gccgttgtgt gtatttggaa    480
   gaaatggcag tagagctaga agatcaagac tggcttgata tgagcaatgt tgagcaggcc    540
   ctcttcgctc gcttattact tcaagatcca ggcaaccact taattaacat gacttcttct    600
   acaacgctaa atctctctgc tgatcgagat gcaggagaga ggcacatttt ttgttacctt    660
   tactcctgct tccagagagc caaggaagag attaccaaag ttccagagaa cctgctaccc    720
   tttgcagtgc agtgcagaaa cctcactgtg tccaataccc gaacagttct tctcacccca    780
   gagatctatg ttgaccaaaa catccatgag caactggtag atttgatgtt agaagccatc    840
   cagggagccc gtgagtacat gaacaagatc tattttgaag atgtaactga gtttctggaa    900
   gaggtcattg aagccttgat attggatgag gaagttagaa catttccaga agtcatgatt    960
   ccagtgtttg atattttatt gggccgaata aaagatctag agctctgtca gatccttttg   1020
   tatgcatatc tggatattct tctctatttc actaggcaaa aagatatggc aaaggttttt 1080
   gtagaataca ttcagcccaa ggaccctacc aatgggcaaa tgtaccagaa gaccttgctg 1140
   ggagtaattc tgagtatctc ctgcttatta aagactccgg gtgttgtaga aaatcatggc 1200
   tactttttga atccatctcg ttccagcccc caggagatca aagtacagga ggccaacatc 1260
   catcagttca tggctcagtt ccacgaaaag atctaccaga tgctgaagaa cttactccag 1320
   ctctctccag aaaccaaaca ctgtatcttg tcctggcttg gaaactgttt gcatgcaaat 1380
   gcaggccgca ccaagatttg ggccaatcag atgccagaaa tctttttcca aatgtatgcc 1440
   tcagatgctt tctttctgaa tctgggtgct gctctcctga agctatgcca gccattttgc 1500
   aaacccagat cctctcggct cctcaccttt aatcccacat actgtgccct caaggagttg 1560
   aatgatgaag aacgaaaaat taaaaatgta cacatgagag gtttggacaa agaaacctgt 1620
   ttgatcccag ctgtgcagga gccgaagttt ccacagaact acaaccttgt aacagagaac 1680
   cttgctctga cagagtacac cttgtacttg ggatttcaca ggttgcatga tcagatggta 1740
   aaaatcaacc aaaatctgca tcggctgcag gttgcctggc gggatgctca gcaaagttct 1800
   agccctgctg ctgacaatct tcgtgagcag tttgaacgac tgatgaccat ctatctttct 1860
   accaagactg ccatgacaga gccacaaatg ctacaaaact gcctaaactt gcaggtgtcc 1920
   atggctgttc tactggttca actggccata ggcaatgagg gctcacagcc aatagagcta 1980
   acctttcctt tgccagatgg ctacagctct ttggcttatg tgccagaatt ttttgcagat 2040
   aacctgggtg attttctcat ttttctccgc cgctttgccg atgacatttt ggagacatca 2100
   gcagattccc tggagcatgt ccttcacttt atcaccattt tcactggaag catagaaaga 2160
   atgaagaatc cccacctgag ggccaaacta gcagaggtgt tggaagcagt gatgccccac 2220
   ctggatcaga ccccaaatcc cttggtatcc agtgtgttcc accggaaacg tgtgttctgc 2280
   aactttcagt atgcacccca acttgcagag gctctaatca aggtttttgt ggacatcgaa 2340
   tttacaggag acccccatca atttgaacag aagtttaatt accgccgtcc catgtatcct 2400
   atcctaagat acatgtgggg gacagatacc tatcgggaga gcattaagga tttggctgac 2460
   tatgcctcta agaatttaga agccatgaat cccccacttt tcctccgctt tcttaacctg 2520
   ctaatgaatg atgccatctt ccttttggat gaagccatac agtatttgag caagataaag 2580
   attcagcaaa ttgagaagga tcgaggtgaa tgggatagtc tgactccaga agcccgccga 2640
   gaaaaggagg ctggcctaca gatgtttgga cagctggcac gtttccataa catcatgtcc 2700
   aatgaaacaa tcggtaccct tgcctttctc acatcagaga tcaagtcact ctttgtgcat 2760
   cccttcctgg ctgagcgcat catctccatg ttgaactact tcctgcaaca cctggttggc 2820
   cccaagatgg gtgccttaaa agtcaaggac ttcagcgaat ttgacttcaa accccagcag 2880
   cttgtatcag atatctgcac tatctactta aatcttgggg atgaggagaa tttctgtgcc 2940
   actgtgccca aggatggacg ttcctattcc ccaactctct ttgcacagac agttcgagtc 3000
   ttgaagaaaa taaataagcc tgggaatatg attatggctt tcagcaactt ggcagagaga 3060
   atcaagtctc ttgcagacct ccaacaacag gaagaggaaa cctatgcaga tgcctgtgat 3120
   gagttcctgg atcccattat gagcacactg atgtgtgacc ctgtggtgct gccatcttcc 3180
   agagtcactg tggatagatc caccattgca agacatttgc tcagtgacca aacagatccc 3240
   tttaaccgta gtcccctcac catggaccag atccggccaa acacagaact aaaagaaaaa 3300
   atccaacggt ggcttgcaga gaggaaacaa caaaaggagc aacttgaata gatactgtga 3360
   actaaccaaa ccaaaaccaa ccccagagtg cagataaaca attgtttgtg gtttctctct 3420
   ttctggttct gttccttttc tttcttcttt tctttttctt tttttttttt ttttttacta 3480
   aattagagaa ctgctcttgc tgaaattatg atagttaaga ttcctaagaa cttgacaatg 3540
   ctcccctggc ttgcaggaaa ttatacttat tatagcatca ccaagtttag aaatcatcac 3600
   taattttcac cctctgttgt ctcttcatat tcttcttcct tttcctaaat gaaattatat 3660
   tatttcaact actaaaactt cccgccaccc tgctatttct cttccaatta ctgttcaaac 3720
   atttttggtg agtgctgctt ttataaatat gtgatgtcac atatttcagt gacagctgat 3780
   gttatcagaa aaatcctgtt atcctgtgta tttactagtc ctcatgatct agacttaacc 3840
   ccttttctgt gttacagagt aacttcacat aaaagcagat acctttaagt ttgtgtagac 3900
   ttctgaataa gatgatagac gagaattttt tttaaaaaag gaatttaaag gaatcttgtg 3960
   caacttctgt tgatagctct taattttgtt atacagtctt tttaatgagg attggtaggg 4020
   caatcctacc aattgatggg ggtgaggaga ctgttgggcc cttattctct tatgaaaatc 4080
   tgtttctaca aggactaggc ctaagtagat ttaggcaacg ggtatcatat ccatgaaaaa 4140
   tgtcatttta agacactaat atcaacagta tatctcagtg tgtgagatat ataaatacac 4200
   atacacccca tgtgcttttt tgttggttta gtgtaaactg agtaccactt tatattttct 4260
   cttccatagt gagatgtatg cagtatgtgg ccatgtttac taacatactc attcttcaca 4320
   aaattctgag attataaaat gtatatagtt aatatttgtt tgagcttgtg acctgacttc 4380
   taagagctac ttctaacaca gccttaggtc ttcaattaac aaagtaggaa gttgagcaca 4440
   accttgtcca cagccatttt aaatatccac ttagccctgt gtagttggta ggtggacacc 4500
   actttcataa gtgaacttga gctcactact caattgtgca gcttaatatg ctgactaggg 4560
   acattcccct atggattatc tttatatcac tgtctttcta agcccagaga tgtcataagt 4620
   gacaagaaaa gtgataggat caagaaatgg gtgtcactgc ttcatttgga acatgttatt 4680
   aactatggtc tctttcaaat aaatagtagt tttatatttc aacacaagtt gctataagca 4740
   gtccttgatg ggtttttgat tgcatcagct ggaaagcctc aaatctaaga gggcttccca 4800
   tcctagatat aaaataggtg ttgcctattg ctgtgcttat aaaatgaaaa aggaaattga 4860
   ggacactttt gcaaatgcca gaatgtaaga ttcattcagt gtgctccctg ggcctttatg 4920
   gcatgggttg acaggatttg tttattttct aaaattagct tcattcaata tttatcatcc 4980
   tcctttccct ctctgagaat gaactatgta taaaataagc ttctgcctat ttgcatttat 5040
   cttccaaacc caatctagta ggatgttctc attttaaaaa cgaggggaaa agaccagagt 5100
   ttttcaggag aaaactggag gaaaatgggc acaaaaactc agaaggcagc tattcccagc 5160
   agcttcctag ttaacaaccc ccatgctgcc tccagtcttt gtctgtattc ttctgtattt 5220
   aaccttcaga ttgtaagcct tttctggcaa gcttttcttc tttttttaaa ctcttttcct 5280
   gaaacttttt atgaatggct atggcaccat taatgctgct gaatatcttt aaactctgca 5340
   caagcaagtg tgtagcttaa ggccactact ggtaaggaaa ccaagtgtcc tctgtgcctt 5400
   ttttctttct gtgaagtaat ttaagaatat ccaaaaaaat tagactttaa aaagttatct 5460
   tggtacaaca ccgtgtgtat atacacttgg aagcttaaaa aggtgttttg tctggaactt 5520
   agaagcagct ctaaatctag tagagcagac tttctaacat acctagtttt gtgtattggc 5580
   tttgctggag tatgatagca aaatgaagac tcttttactc agctctggta ttgctcataa 5640
   cttaccaaga ggctaatact aaacttggaa aattgtttaa gtatgtttta tcaagcagtc 5700
   tgggttttgt tttttaatat actttttaat ggatatgtga aaactgaagg aaatgttaaa 5760
   ggttttttaa tggtgcaagt gaaggtgcca gttgctattt gatatcacac tctacaaaag 5820
   cttcattact ttatttgatg gtggttgcta agcagccatt gcacagagca taagtctact 5880
   gggtgccttt acatgccaga ggctgatgct gcactgttga tgtcatgtga ggaaataatg 5940
   cacatgctct aactgctcaa caggaaatga acctagaaac agaaaatgaa aaggttgatt 6000
   gaaataaaac ttgatcaacg cgactgtatt ttgaaacatt ccaggaaggt tacttcttgt 6060
   caaacttgcc tggcagtgtt tgttcaaaac ttgtatttaa taaatgaaca tctgacttac 6120
   aaaaaaaaaa aaaaaaaa 6138
<210> 111
   <211> 3032
   <212> DNA
   <213> Homo sapiens
<400> 111
   gaattccgga gaaaggcgca ggggtgggag ctgttgccga agctgccaca gcaaaagttc 60
   tcccccctcc ccccttcccc tcctctcaag gcccctagaa aggttggagc tgccggccct 120
   gcagtcggtg accgctgacg acttcggccg cgcccgcgga tagcgggagg aatcagcagc 180
   ttggaaattc aagcacgtga tctggcggga tgggcgtttg cctaacgtat ttaatggagg 240
   aatcggatgg cataagtgat taaggtggta ttgaggattt ctgaagccta tgaaaggtag 300
   aaactcaacc atgatttctt tttcaactct acagcattcc tttccttgaa gtcttcgttt 360
   ttaccttagt ctcgggcagt tatacttaag catgaacatt gacgacaaac tggaaggatt 420
   gtttcttaaa tgtggcggca tagacgaaat gcagtcttcc aggacaatgg ttgtaatggg 480
   tggagtgtct ggccagtcta ctgtgtctgg agagctacag gattcagtac ttcaagatcg 540
   aagtatgcct caccaggaga tccttgctgc agatgaagtg ttacaagaaa gtgaaatgag 600
   acaacaggat atgatatcac atgatgaact catggtccat gaggagacag tgaaaaatga 660
   tgaagagcag atggaaacac atgaaagact tcctcaagga ctacagtatg cacttaatgt 720
   ccctataagc gtaaagcagg aaattacttt tactgatgta tctgagcaac tgatgagaga 780
   caaaaaacaa atcagagagc cagtagactt acagaaaaag aagaagcgga aacaacgttc 840
   tcccgcaaaa atccttacaa taaatgagga tggatcactt ggtttgaaaa cccctaaatc 900
   tcacgtttgt gagcactgca atgctgcctt tagaacgaac tatcacttac agagacatgt 960
   cttcattcat acaggtgaaa aaccatttca atgtagtcaa tgtgacatgc gtttcataca 1020
   gaagtacctg cttcagagac atgagaagat tcatactggt gaaaaaccat ttcgctgtga 1080
   tgaatgtggt atgagattca tacaaaaata tcatatggaa aggcataaga gaactcatag 1140
   tggagaaaaa ccttaccagt gtgaatactg tttacagtat ttttccagaa cagatcgtgt 1200
   attgaaacat aaacgtatgt gccatgaaaa tcatgacaaa aaactaaata catgtgccat 1260
   caaaggtggc cttctgacat ctgaggaaga ttctggcttt tctacatcac caaaagacaa 1320
   ctcactgcca aaaaagaaaa ggcagaaaac ggagaaaaaa tcatctggaa tggacaaaga 1380
   gagtgctttg gacaaatctg acctgaaaaa agacaaaaat gattacttgc ctgtttattc 1440
   ttcaagtact aaagtaaaag atgagtatat ggttgcagaa tatgctgttg aaatgccaca 1500
   ttcgtcagtt gggggctcgc atttagaaga tgcgtcagga gaaatacacc cacctaagtt 1560
   agttctcaaa aaaattaata gtaagagaag tctgaaacag ccactggagc aaaatcaaac 1620
   aatttcacct ttatccacat atgaagagag caaagtttca aagtatgctt ttgaacttgt 1680
   ggataaacag gctttactgg actcagaagg caatgctgac attgatcagg ttgataattt 1740
   gcaggagggg cccagtaaac ctgtgcatag tagtactaat tatgatgatg ccatgcagtt 1800
   tttgaagaag aagcggtatc ttcaagcagc aagtaacaac agcagggaat atgcgctgaa 1860
   tgtgggtacc atacgttctc agccttctgt aacacaagca gctgtggcaa gtgtcattga 1920
   tgaaagtacc acggcatcca tattagagtc acaggcactg aatgtggaga ttaagagtaa 1980
   tcatgacaaa aatgttattc cagatgaggt actgcagact ctgttggatc attattccca 2040
   caaagctaat ggacagcatg agatatcctt cagtgttgca gatactgaag tgacttctag 2100
   catatcaata aattcttcag aagtaccaga ggtcacccca tcagagaatg ttggatcaag 2160
   ctcccaagca tcctcatcag ataaagccaa catgttgcag gaatactcca agtttctgca 2220
   gcaggctttg gacagaacta gccaaaatga tgcctatttg aatagcccga gccttaactt 2280
   tgtgactgat aaccagaccc tcccaaatca gccagcattc tcttccatag acaagcaggt 2340
   ctatgccacc atgcccatca atagctttcg atcaggaatg aattctccac taagaacaac 2400
   tccagataag tcccactttg gactaatagt tggtgattca cagcactcat ttcccttttc 2460
   aggtgatgag acaaaccatg cttctgccac atcaacacag gactttctgg atcaagtcac 2520
   ttctcagaag aaagctgagg cccagcctgt ccaccaagct taccaaatga gctcctttga 2580
   acagcccttc cgtgctccct atcatggatc aagagctgga atagctactc aatttagcac 2640
   tgccaatgga caggtgaacc ttcggggacc agggacaagt gctgaatttt cagaatttcc 2700
   cttggtgaat gtaaatgata atagagctgg gatgacatct tcacctgatg ccacaactgg 2760
   ccagactttt ggctaaaaaa aaaaaaaaag tgaaataata ctggcacttt agaacagatt 2820
   aatcaagagt ggggttacta tgtgtaaatg gagtgctgta cagatttaag agcaatgcgt 2880
   aataacaagt taagctgata tacgatgata ataactgcat ttcgtttggt tagtcagcat 2940
   tctttgaact gccttacatg ttgtcacctt tatagaagca atgcattact tgttttagat 3000
   cagaaacttg ctattccacc cacaccaagt aa 3032
<210> 112
   <211> 1224
   <212> DNA
   <213> Homo sapiens
<400> 112
   gcccattgtt tttgtaatct ctgaggagaa gcagcagcaa acatttgcta gtcagacaag      60
   tgacagggaa tggattccaa acaccagtgt gtaaagctaa atgatggcca cttcatgcct     120
   gtattgggat ttggcaccta tgcacctcca gaggttccga gaagtaaagc tttggaggtc     180
   acaaaattag caatagaagc tgggttccgc catatagatt ctgctcattt atacaataat     240
   gaggagcagg ttggactggc catccgaagc aagattgcag atggcagtgt gaagagagaa     300
   gacatattct acacttcaaa gctttggtcc acttttcatc gaccagagtt ggtccgacca     360
   gccttggaaa actcactgaa gaaagctcaa ttggactatg ttgacctcta tcttattcat     420
   tctccaatgt ctctaaagcc aggtgaggaa ctttcaccaa cagatgaaaa tggaaaagta     480
   atatttgaca tagtggatct ctgtaccacc tgggaggcca tggagaagtg taaggatgca     540
   ggattggcca agtccattgg ggtgtcaaac ttcaaccgca ggcagctgga gatgatcctc     600
   aacaagccag gactcaagta caagcctgtc tgcaaccagg tagaatgtca tccgtatttc     660
   aaccggagta aattgctaga tttctgcaag tcgaaagata ttgttctggt tgcctatagt     720
   gctctgggat ctcaacgaga caaacgatgg gtggacccga actccccggt gctcttggag     780
   gacccagtcc tttgtgcctt ggcaaaaaag cacaagcgaa ccccagccct gattgccctg     840
   cgctaccagc tgcagcgtgg ggttgtggtc ctggccaaga gctacaatga gcagcgcatc     900
   agacagaacg tgcaggtttt tgagttccag ttgactgcag aggacatgaa agccatagat     960
   ggcctagaca gaaatctcca ctattttaac agtgatagtt ttgctagcca ccctaattat    1020
   ccatattcag atgaatatta acatggaggg ctttgcctga tgtctaccag aagccctgtg    1080
   tgtggatggt gacgcagagg acgtctctat gccggtgact ggacatatca cctctactta    1140
   aatccgtcct gtttagcgac ttcagtcaac tacagctgag tccataggcc agaaagacaa    1200
   taaattttta tcattttgaa ataa                                           1224
<210> 113
   <211> 2279
   <212> DNA
   <213> Homo sapiens
<400> 113
   aggtggagcc ttttttgctc acggcagcaa gttcccttct cctttctctc ccccggcggc 60
   gtgtgcattg gctcttcaag ctgcctgtgc tgctccgtgg agtgaaaaag gcagggtgtg 120
   ctcgcagact gtgctataaa ctgcaatttc tatttggggt cctcacggag aagaacacca 180
   ggaaagacag acaggaccag tgccatgggc cagctttgct gctttccttt ctcaagagat 240
   gaaggaaaaa tcagtgaaaa gaacggaggg gagcccgatg acgctgaact agtaaggctc 300
   agtaagaggc tggtggagaa cgcggtgctc aaggctgtcc agcagtatct ggaggaaaca 360
   cagaataaaa acaagccggg ggaggggagc tctgtgaaaa ccgaagcagc tgatcagaat 420
   ggcaatgaca atgagaacaa caggaaatga gcccggaacg caggccccca tgtctctgtg 480
   caaagcctcc ctgcttccct ctgctgagtc tagggactga cttgcagcgt gctgtttaag 540
   ttaagtttct ctggtgcaat ctgtgaagat tgcctaatac ttttcatgat cgatgtgttc 600
   gcattgctga aacacaacag aagaaaaatg gagtgctggg actggcagag gaaattaatt 660
   gatgaaagaa gaatggccca agtttcattc gccctcagcc acgcacaagg gaaagggaac 720
   tttgggttat gcctcctgga cgcaaattaa aggccgagaa agaggccttg ccatcaatgg 780
   aatactgcca tttatattgc ttagcagggc atttgactac tttatctgag gccagaactc 840
   tcacacacag ctatcaagtg ctaagtttaa aataatcact gttggaattg tcatctgtac 900
   aattagtcca taatgtttca tgtttgtcct aagtgtgctg ttgctatgca gtgtgatctt 960
   tatttatagt aaattatgtt tcatgtaaat gatatatttt tggtgaaatg caaccttttc 1020
   tataaaatgt gggcaacatt ttaaagtttt tttaaaatcc tattttgata agtcagtatg 1080
   ccatatttaa tgaaatgtta ttatataatt tttttttctt aggcaagaaa cctattggaa 1140
   ttcgagactt aattaatgaa gctttgcatc gagaaacgat gggtctgaag tccaaagtga 1200
   aacagataaa ggaactttta ttaaagcctg agactcaggc cagaattagg agggagcttt 1260
   ttgaaggaag acttattaac aacagtaatt cagcaaatga cgttgatttc agcacaactt 1320
   tgacataagc tctacattgc gattgtgaca acatagctta tgaaatcttt tcagcttatt 1380
   aagtagctct ttggtaaaca ccaaagaagt ttctgatagt gtctgcacaa cagcaaacca 1440
   acatttggtg aggaattagc aatttcttgc caaagaaaat tgattctgcc caattatttt 1500
   ttgagctaca cttgtgtttt agaatatctg tttctgtaat attgagagtt attttataga 1560
   aatgatttct taattagctg ttgtgagata tttctcgggt ccttgcagaa aaaaacatac 1620
   agactgtgaa caaatcattc acaaacagaa taaaacagag ccaacaacag tattttaagg 1680
   gtcacttgcc tcctgttgac acaattgttg ctaaatcaaa agaagcgttg tccaggtgtg 1740
   tctacatcta gtgttacttt taatgagaat ttgaatgttt attgaacaat agtacttgaa   1800
   tgaacattta taaatgtaat tattgcgatc actggttaag aatgttttat atatccttat   1860
   aatatttttc actgatcaaa atgttgttct gctttttcat ttcttaagga atacatgttt   1920
   gggattttta ttttttacgt gtccgaagat aagctccagg tcttatcgta tcccttgcca   1980
   tctgaacttg tttgcactgc ttctgtttga aagagcatct tgaaaaactt ccccggtatg   2040
   atgattgttg gtaacaactt tttctatagt cattgatgga gtagatcatg atggagggga   2100
   aatcactgga gatcaaatat gtaaaatcat ttcaaatata aaatccagtt tactcatgga   2160
   ttttagctat tttttcactg ggtaaattat actacattta tttacaaatg agtttatgca   2220
   ttttcatggc tcttaataaa catattgttt tcccttgaaa aaaaaaaaaa aaaaaaaaa    2279
<210> 114
   <211> 1937
   <212> DNA
   <213> Homo sapiens
<400> 114
   cacctgtcat tcgttcgtcc tcagtgcagg gcaacaggac tttaggttca agatggtgac      60
   tgcagccatg ctgctacagt gctgcccagt gcttgcccgg ggccccacaa gcctcctagg     120
   caaggtggtt aagactcacc agttcctgtt tggtattgga cgctgtccca tcctggctac     180
   ccaaggacca aactgttctc aaatccacct taaggcaaca aaggctggag gagattctcc     240
   atcttgggcg aagggccact gtcccttcat gctgtcggaa ctccaggatg ggaagagcaa     300
   gattgtgcag aaggcagccc cagaagtcca ggaagatgtg aaggctttca agacagatct     360
   gcctagctcc ctggtctcag tcagcctaag gaagccattt tccggtcccc aggagcagga     420
   gcagatctct gggaaggtca cacacctgat tcagaacaat atgcctggaa actatgtctt     480
   cagttatgac cagtttttca gggacaagat catggagaag aaacaggatc acacctaccg     540
   tgtgttcaag actgtgaacc gctgggctga tgcatatccc tttgcccaac atttctttga     600
   ggcatctgtg gcctcaaagg atgtgtccgt ctggtgtagt aatgattacc tgggcatgag     660
   ccgacaccct caggtcttgc aagccacaca ggagaccctg cagcgtcatg gtgctggagc     720
   tggtggcacc cgcaacatct caggcaccag taagtttcat gtggagcttg agcaggagct     780
   ggctgagctg caccagaagg actcagccct gctcttctcc tcctgctttg ttgccaatga     840
   ctctactctc ttcaccttgg ccaagatcct gccagggtgc gagatttact cagacgcagg     900
   caaccatgct tccatgatcc aaggtatccg taacagtgga gcagccaagt ttgtcttcag     960
   gcacaatgac cctgaccacc taaagaaact tctagagaag tctaacccta agatacccaa    1020
   aattgtggcc tttgagactg tccactccat ggatggtgcc atctgtcccc tcgaggagtt    1080
   gtgtgatgtg tcccaccagt atggggccct gaccttcgtg gatgaggtcc atgctgtagg    1140
   actgtatggg tcccggggcg ctgggattgg ggagcgtgat ggaattatgc ataagattga    1200
   catcatctct ggaactcttg gcaaggcctt tggctgtgtg ggcggctaca ttgccagcac    1260
   ccgtgacttg gtggacatgg tgcgctccta tgctgcaggc ttcatcttta ccacttctct 1320
   gccccccatg gtgctctctg gagctctaga atctgtgcgg ctgctcaagg gagaggaggg 1380
   ccaagccctg aggcgagccc accagcgcaa tgtcaagcac atgcgccagc tactcatgga 1440
   caggggcctt cctgtcatcc cctgccccag ccacatcatc cccatccggg tgggcaatgc 1500
   agcactcaac agcaagctct gtgatctcct gctctccaag catggcatct atgtgcaggc 1560
   catcaactac ccaactgtcc cccggggtga agagctcctg cgcttggcac cctcccccca 1620
   ccacagccct cagatgatgg aagattttgt ggagaagctg ctgctggctt ggactgcggt 1680
   ggggctgccc ctccaggatg tgtctgtggc tgcctgcaat ttctgtcgcc gtcctgtaca 1740
   ctttgagctc atgagtgagt gggaacgttc ctacttcggg aacatggggc cccagtatgt 1800
   caccacctat gcctgagaag ccagctgcct aggattcaca ccccacctgc gcttcacttg 1860
   ggtccaggcc tactcctgtc ttctgctttg ttgtgtgcct ctagctgaat tgagcctaaa 1920
   aataaagcac aaaccac 1937
<210> 115
   <211> 8280
   <212> DNA
   <213> Homo sapiens
<400> 115
   agaggctgcg gtgagtccgc cagccccagc tgctcctcct caagccccca aggcccttcg     60
   gcgggcccct gctgaaagac cggcatgccc tattctgtgg gcttccgcga agccgatgct    120
   gctaccagct ttctgagagc agcaagatca ggtaacttgg acaaagcttt ggatcacctg    180
   cggaatgggg tagatattaa cacctgtaac cagaatgggt tgaatggctt gcatctggct    240
   tctaaggaag gccatgtgaa aatggtggtt gaacttctgc acaaagaaat cattctagaa    300
   acgacaacca agaaggggaa cacggccctg cacatcgctg ctctagccgg gcaggatgag    360
   gtggtccggg agcttgtcaa ctatggagcc aacgtcaatg cccagtcaca gaaaggtttt    420
   acacccctgt acatggcagc acaagagaac cacttggaag tggttaagtt tttactggaa    480
   aatggagcta accagaatgt agccacagaa gacggcttca cgcctctggc ggtagccctg    540
   cagcagggcc atgagaacgt cgtcgcgcac ctcatcaact acggcaccaa ggggaaggtg    600
   cgcctcccgg ccctgcacat cgcggcccgc aacgacgaca cgcgcacggc tgcggtgctg    660
   ctgcagaacg accccaaccc ggacgtgctt tccaagacgg gattcacgcc cctgcacatt    720
   gcggctcact acgagaacct caacgtggcc cagttgctcc tcaacagagg agccagcgtc    780
   aatttcacac cacagaacgg catcacgcca ctgcacatcg cctcccgcag gggcaacgtg    840
   atcatggtgc ggctgctgct ggatcgggga gcccagatag aaaccaagac caaggacgaa    900
   ttgacacctc tccactgtgc agctcgaaat gggcacgtgc gaatctcaga gatcctgctg    960
   gaccacgggg caccaatcca agccaaaacc aagaacggcc tgtccccaat tcacatggcg   1020
   gctcagggag accacctcga ctgtgtccgg ctcctgttgc aatacgacgc agagatagac 1080
   gacatcaccc tggaccacct gaccccactc cacgtggctg cccactgtgg acaccacagg 1140
   gtggctaagg tccttctgga taaaggggcc aaacccaact ccagagccct gaatggcttt 1200
   acccccttac acatcgcctg caaaaagaac cacgtccgtg tcatggagct gctgctgaag 1260
   acgggagcct cgatcgacgc ggtcaccgag tctggcctga cacctctcca cgtggcctcc 1320
   ttcatggggc accttcccat cgtgaagaac ctcctgcagc ggggggcgtc gcccaacgtc 1380
   tccaacgtga aagtggagac cccgctacac atggcagcca gagccgggca cacggaagtg 1440
   gccaaatatt tactccagaa caaagccaaa gtcaatgcca aggccaagga tgaccagacc 1500
   ccacttcact gtgcagctcg catcggccac acaaacatgg tgaagctcct gctggaaaat 1560
   aacgccaacc ccaacctggc caccaccgcc gggcacaccc ccctgcacat tgcagcccgt 1620
   gagggccatg tggaaacagt cctggccctt ctggaaaagg aagcatccca ggcctgcatg 1680
   accaagaaag gatttacccc tctgcacgtg gcggccaagt acgggaaggt gcgggtggca 1740
   gagctgctgc tggagcggga cgcacacccg aatgctgccg gaaaaaatgg cctgaccccc 1800
   ctgcacgtgg ccgtccatca caacaacctg gacatcgtca agctgctgct tccccggggc 1860
   ggctccccgc acagccctgc ctggaatggc tacacccctt tgcacatcgc tgccaagcag 1920
   aaccaggtgg aggtggcccg tagtctgctg cagtatgggg gctcagcaaa cgccgagtcg 1980
   gtgcaaggtg tgacgcccct tcacctggcc gcccaggagg gccacgcaga gatggtggct 2040
   ctgctgctct cgaaacaagc caatggcaac ctggggaaca agagcggact cactcccctc 2100
   catctggtag cacaagaagg ccacgttcca gtggcagatg tgctgatcaa acacggtgtc 2160
   atggtggacg ccaccacccg gatgggctac actcccctcc atgtggccag tcactatgga 2220
   aacatcaagc tggtgaagtt tctgctgcag caccaggcag atgtcaatgc caagaccaag 2280
   ctaggataca gccccctgca ccaggcagcc cagcagggac acacagacat cgtgactctg 2340
   cttctgaaaa acggtgcttc cccaaacgag gtcagctcgg atggaaccac acctctggcc 2400
   atagccaagc gcttgggcta catttctgtc accgacgtgc tcaaggtcgt cacggatgaa 2460
   accagtttcg tgttagtcag tgataagcat cgaatgagtt tccctgagac agttgatgag 2520
   atcctggatg tctcggaaga tgaaggggaa gaactcatca gcttcaaggc tgagaggcgg 2580
   gattccaggg atgttgatga agagaaggag ctgctggatt ttgtgccgaa gctagaccaa 2640
   gtggtggaat ctccagccat ccccaggatt ccctgtgcca tgcctgagac agtggtgatc 2700
   aggtcagaag agcaggagca ggcatctaaa gagtatgatg aggactccct catccccagc 2760
   agcccggcca ccgagacctc agacaacatc agcccggtgg ccagcccggt gcatacaggg 2820
   tttctggtga gcttcatggt tgacgcccgg ggtggttcca tgagaggaag tcgccacaac 2880
   ggcctgcgag tggtgatccc gccacggacg tgcgcagcgc ccacccgcat cacctgccgc 2940
   ctggtcaagc cccagaagct cagcacgccg cccccactgg ccgaggagga gggcctggcc 3000
   agcaggatca tagcactggg gcccacgggg gcacagttcc tgagccctgt aatcgtggag 3060
   atcccgcact ttgcctccca tggccgtgga gaccgcgagc tcgtggttct gaggagcgaa 3120
   aacggctccg tgtggaagga gcacaggagc cgctatggag agagctacct ggatcagatc 3180
   ctcaacggga tggacgaaga gctggggagc ctggaggagc tagagaagaa gagggtgtgc 3240
   cgaatcatca ccaccgactt cccgctgtac ttcgtgatca tgtcacggct ctgccaggac 3300
   tacgacatca tcggtcccga agggggctcc ctgaagagca agctggtgcc cctggtacag 3360
   gcaacgttcc cggagaatgc cgtcaccaag agagtgaagc tggctctgca ggcccagcct 3420
   gtcccggatg agcttgtcac taagctcctg ggcaaccagg ccacattcag ccccattgtc 3480
   accgtggagc cccggcgccg gaagttccac cgccccattg ggcttcggat cccactacct 3540
   ccttcctgga ccgacaaccc gagggacagt ggggagggag acaccaccag cctgcgcctg 3600
   ctttgcagcg tcattggagg aacagaccaa gcccagtggg aagacataac aggaaccacc 3660
   aaacttgtat atgccaacga gtgcgccaac ttcaccacca atgtctctgc caggttttgg 3720
   ctgtcggact gtcctcggac tgctgaggct gtgaactttg ccaccctgct gtacaaagag 3780
   ctcactgcag tgccctacat ggccaaattc gtcatctttg ccaagatgaa tgacccccga 3840
   gaggggcgcc tgcgctgcta ctgcatgaca gatgataaag tggacaagac cctggagcag 3900
   catgagaact tcgtggaggt ggcccggagc agggacatag aggtgttgga aggaatgtcc 3960
   ctgtttgcag aactctctgg gaacctggtg cctgtgaaga aagctgccca gcagcggagc 4020
   ttccacttcc agtcatttcg ggagaaccgt ctggccatgc ctgtaaaggt gagggacagc 4080
   agtcgagagc cgggagggtc cctgtcgttt ctgcgcaagg cgatgaagta cgaggacacc 4140
   cagcacattc tctgccacct gaacatcacc atgcccccct gcgctaaggg aagtggagcc 4200
   gaagatagga gaaggacccc gacgcccctg gccctgcgat acagcattct cagtgagtcc 4260
   acaccaggtt ctctcagtgg gacagagcag gcagagatga agatggctgt tatctcagag 4320
   cacctcggtc tcagctgggc agagttggcc cgggagctgc agttcagtgt ggaagacatc 4380
   aacaggatcc gagtggaaaa tcccaactcc ctgttggagc agagtgtggc cttgctgaac 4440
   ctctgggtca tccgtgaagg ccaaaacgca aacatggaga atctgtacac agccctgcag 4500
   agcattgacc gtggcgagat cgtgaacatg ctggagggtt ccggccgaca gagccgcaac 4560
   ttgaagccag acaggcggca caccgaccgc gactactcgc tgtcaccctc ccagatgaat 4620
   ggttactcct cactgcagga cgagctgctg tcccctgcct ccctgggctg tgcactttcc 4680
   tctccgctac gtgcagacca gtactggaat gaggtggccg tcctagacgc catccccttg 4740
   gcggccacgg agcatgacac catgctggag atgtctgaca tgcaggtgtg gtctgcgggc 4800
   ctcacgcctt ctctggtcac tgctgaggac tcctctctgg agtgtagcaa ggctgaggac 4860
   tctgatgcca caggtcacga gtggaagttg gagggggcac tctcagagga accgcggggc 4920
   cccgagttgg gctctctgga acttgtggag gacgacacag tggattcaga tgccacaaat 4980
   ggccttatcg atttgcttga acaggaggaa ggtcagaggt cagaagagaa gctgccaggt 5040
   tctaagaggc aggatgacgc gacaggtgca gggcaggact cagagaatga agtgtctctt 5100
   gtttcaggcc atcagagggg gcaagcccga atcacacatt cccccaccgt gagtcaggtg 5160
   acggagagga gtcaggacag actgcaggac tgggatgcag acggctcgat tgtctcatac 5220
   ctgcaagatg ctgcacaagg ttcctggcaa gaggaggtca cgcaaggtcc acactcattc 5280
   cagggaacaa gtaccatgac tgaagggcta gagcccggtg gatctcagga gtacgagaag 5340
   gtcctggtgt ctgtaagtga gcacacgtgg acagaacagc ccgaggctga gagctcccag 5400
   gccgacaggg accggaggca gcaaggccaa gaagagcagg tgcaggaggc caagaacacc 5460
   ttcacccaag tggtgcaggg gaatgagttt cagaatattc caggggagca ggtgacagag 5520
   gagcaattca cggatgagca gggcaacatt gtcaccaaga agatcattcg caaggtggtt 5580
   cgacagatag acttgtccag cgccgatgcc gcccaggagc acgaggaggt ggagctgaga 5640
   gggagtggcc tacagccgga cctgatagag ggcaggaagg gggcgcagat agtgaagcgg 5700
   gccagcctga aaagggggaa acagtgaccc cgagccgctc tccttggagt agcctctcgg 5760
   gaggatcaca cctcgacacc caacccctga accccacaca ctctgccatg cacacaggag 5820
   gagagctgga cctgagggcc accgcagcgg tgcacacatt cctctgggct gacggcatga 5880
   cctctgtaag ggactcctgc tagtcccctc ttggcatgaa tgactgactg tagacgcatg 5940
   acctccaggc ttcaatcctg cctcttgcaa tgacagctga tctgtcggaa ccaggacaca 6000
   aaagcagcaa gaagcgggga gagagaggga tagaaaacaa gcgcaggaga gcctgcgaac 6060
   gcaaaagtga atgagggctt tttgtggctg gggatgggtt ttggttttgg ggtttttttt 6120
   ttaaattgtt ttgacttcgt acagggtact ttttcccaac ctcatctgtc agaaatccat 6180
   gtgggcttcc tggaaagaaa aaaaaaaaaa agaaaactag gcatgaaatc agtttaacac 6240
   cttaatctta agccatgtcc tcatctgccc accctcccca acccacatac ctccattcca 6300
   cttgtgacac cccctcgact ccctggtgac gctcctccca gatcgctcta catgacattc 6360
   aggacacaca cacacacaca cacacacaca ccactcgcct ccactgaatc tacacacaga 6420
   ttttactgtg acttctgaag ctgtggataa attggaattt tttatgttgt ctctctctct 6480
   gccaatttca atacgaatca tcttccaatg gaaaatcatt accttgaaga gtgcattcgg 6540
   ggttccttgt gttagggact taagaatctg aggcgcgagg acccccaggc ttacctgtag 6600
   gactcaaagg gagccaggac cccatctgaa agggtctcct ctctcagttg ggggacaggc 6660
   cggtggctga cccaggattg caccagcatg tccatagaga agaggttttc tatgtcttca 6720
   agcactatat catagtccgt gttcaaagtg taaactgtac agtaatcagc cttgtgtata 6780
   tgaaaaacaa taaatactat gcaaaccaat agaaacattt agcagtacgt acagagcctg 6840
   acagcgcagc tcctgaggac ttctgcggct gcaggagaag gagctgtggc ctgtctttca 6900
   gtgaaagagg aaggaaagga gtagggctag tgaatgtatc tgccaggtct tagaagtcag 6960
   gaccacccag ggtctcccag agtgaacttg gtgctctaag tccaggcagc ctgcaggctg 7020
   gggaccctgg gtgcaggtgc caggctcgag gctgttccct gtcctgcctc agtgcctccc 7080
   ctctgcccag cccctgtggc ctcttggaga ggcgagccag ggggctcccc ggaccgcatg 7140
   gccggtgaga gtagagtcgg aggcggagag cctggacgcc cagaggccgg ggccaggctc 7200
   tgggtcaagg caggaaggag acagacagct ggcagttcct cgcctcggtg ccatcaggga 7260
   ggagccttaa gttccactag agcctccggc ccagggaggc cagaatcagc acaatcccgg 7320
   ccccgccctc gcggcgggcg cccccagggc caggagagag agacagggct ggctcctctc 7380
   gcccctggag ccgcggtgtg gacggcccgg ccccgtccgc cccgccgctc tgcgggagtg 7440
   tccttcggga cacacttaaa ccttccaagt agcacagaag ccccctccac gtcagagccc 7500
   cctggccgcg gggcctgtga gcggagaggg gccctgcttc ccacacaaat ccgccccggt 7560
   gcacccccgg ccaggcaggc cccggggcgg cctaggcgcc ttgtcggccg ggggtggggc 7620
   tgcttttgct tcctttagag gggcgggcag ggagaggaag ctctagctcc gggcctgagt 7680
   ttccccgagg ctgcgtcgga ggagctgcag ggcgagcccg gacggctgcc gtctccgcca 7740
   gaggccaccc cggcccggcc tccaggcaga ggcgcccccc tccatcctcc cccatcccgg 7800
   gggccacggc aggggggccg cacagcgcgg ctgggaccga ttcggtgacg gcccaaggaa 7860
   gctgggcgca gggctcgcct ctcccgcctc gatcctgcac cttcctctcc caggtcgctg 7920
   ctgcagccaa cccagtagcc cccttagcgc ccccgcggac cccgcagctc cagactccgc 7980
   tttggcacct ctgctccggc tggcatggct gcacgctccg gctgctagta ggagccgtta 8040
   ggggaggagt ttggggtctc cacacgatgc ctagagaatg ctgcagtctg cacattagac 8100
   gctttttaga agttttgaaa ttaccttgat ttttttaatt gttatgaaaa tggatctttt 8160
   cttgactctc ccacatgctc tgttatggga gagaatcccc taccctactc tgatgtatag 8220
   accattctcc cttcaccagc cgaaccaatg tcaaaattaa taaagaaatg gactaaggca 8280
<210> 116
   <211> 7207
   <212> DNA
   <213> Homo sapiens
<400> 116
   ggccgcgtcg accatgaaaa agcggcggcg gcgggagaga ggaggaggcg gcggcggggc 60
   agtgaaacta cggtagctgc cccctgagct ggtggtgtgg ctttgtgggg agggcgtagt 120
   tcctaatccc ctttccgggc agccgccggg gctcggggct gtgagcggcc gtgaggctgc 180
   ctccccgggc cccctgcctc cgccatgttc cgcagggcac gccttagcgt gaagccgaat 240
   gtcaggcctg gtgtaggcgc caggggctcc acagcttcca atccccagcg tggacgggag 300
   tctcccaggc cgccggagcc tgccacggac tctgcttcca agcccgcgga gcccacagat 360
   gtgcccacag tcgatttcgg tggagcggag ccccaagaaa aggctcctag gagcagtact 420
   gaaaagactg gtggtgacaa tgatgttgaa gaatccagta gatcttcctc tactgtttca 480
   cagagaagaa agcgaatatc aagtacttct agcctggtta agtctagtgt cagtgttcct 540
   tcagaatctc atcccttatc tacaattaat caagaggctc cacagccaac tgccacttca 600
   acaaaagaga aacagccatg ctcagacaga taccgaatat acaaagccca gaaactgagg 660
   gaaatgttaa aagaagaatt gagaaaagag aagaaacaat ggaaaaacaa atatgctata 720
   aatgaaagtc agaggccacc agatcgttca aaaatgacta tgagagactt catatattat 780
   ctaccagata ataatccaat gacttcttca ctggaacaag aaaagaaaac tgaaaagcca 840
   tcgactccag tccagacaag agagcaagaa ggtaagagta ctcctaatgc tgaagataat 900
   gaaatggaag aagagacaga tgatgggcca ttactggttc ctcgagtaaa agtggcagaa 960
   gatggttcca ttattttgga tgaagaaagt ttaactgtag aagttttaag aacaaaaggc 1020
   ccttgtgttg ttgaagaaaa tgaccccata tttgagcgcg gttctacaac tacatactcc 1080
   agctttagga aaaactatta ctctaaacca tggtcaaata aagaaacaga tatgtttttt 1140
   ttagccatca gcatggtagg aactgacttt tctatgatcg gacaactttt tcctcacaga 1200
   gcaaggatag aaattaagaa taaatttaaa cgggaagaga aaacaaatgg atggagaata 1260
   gacaaagcat tccaggaaaa gcgccctttt gacttcgatt tttttgctca tttgcttcag 1320
   aaagttcttg ctgaagaaga gaaaagaaaa caaaaatctg ttaaaaatca cagtttaaag 1380
   gagaagaaat ccaccaaacc acggaaaaat gtaaaagtga aaaaagttgc ctgtgaagga 1440
   gtgaataatg atccagatga gtctatgagt tctagaattt cagacacgga aagatctcag 1500
   aaggatgctc agacagttga agaagagtct ctgaccttat caagggagga tgcagagcag 1560
   gttgcattag aagtagacct aaatcaaaag aaaagaagga ggaagaagca agatggagct 1620
   aatgaactgg gagtaaacaa tcttttagaa aatgccactg ttcaggcggg tccttctaaa 1680
   ggagaaaaac acaagaataa atgtcaggct ataaggcctg agctaaagga aggtgaatgc 1740
   agtaaggagc agatgctttc ctgcacacaa aacatagatg gcattgtggg ttttgcctcc 1800
   actgaaaaag ttgagaaaag aactgacccc atcctttcat taagtaatca acaagatgcc 1860
   acatcagtag caactgagtc ttcagaatca agcacttcag atttgccttc attcgaagtt 1920
   ggaattagag cattgtgtga ggtgaataat gctgagggta gttgtataga agaaagaaat 1980
   gttgacctaa aaaataattc actggaaatt gatcaaacag aaaatgttaa accaatgttg 2040
   agaggtcgct tccaaagacc taaacccaat ttgtcaaggg ctgggaagaa atcagttctt 2100
   tcacaaggca aaacagagtc agagagcaag aattcacatt caaaaacttc agttgaaaag 2160
   aaccacgtgg aaaaagataa aatgaataca ttggacattt tgagaatgga gactacagag 2220
   agagagaatc cagaagctga aactgtatct gttttgggtg aaaaaaattg tctgcaggaa 2280
   gggagtcaac taaaggcttt aagacctgta caagtgaggg gccgattgca aaagccaaag 2340
   ccaaatgcag gtaaagctgc tgaaagaaaa gaaattctca tatcacagga agaaattggg 2400
   gccaatgtag agaagaatga aaatgaatcc tgtgctgata gagatactcc tcaacacatg 2460
   gaagatcaat cgcgtaaaga ttttgaagag gaagatgtca tattacagcc tgagaaaaat 2520
   gattcttttc aaaatgtgca gccagatgag cccaaggttc ttaatgaatg tctaagcgtt 2580
   caagagaata ataaggcaaa taaacttaac caagtcccaa ttctaaggac tcgatttcag 2640
   aaaccaaagc caaatatagg aagaggaact ggaaggagag aaatttcctc aaaggaagag 2700
   gtactagaga agattcttgt ctctggggaa atggcggcag cattgagaga aactgtaaga 2760
   ctagacacct caccaaagga gatggtacca gcagagatta atactaaaga aatgcagtca 2820
   gatttaaaag aaactggaag aagagccatt tctcccaggg agaagattct agatgtgatt 2880
   gatgacacca tagaaatgga gacaggtctg aaagcaatgg gaagagagat ttgtctaagg 2940
   gagaagacgc cagaggtgat tgatgccact gaggaaatag acaaagattt ggaagaagct 3000
   ggaagaagag aaatatcccc acagaaaaat ggcccagagg aggttaagcc tctaggtgaa 3060
   gtggagacag atttgaaagc aactggaaat gagagttccc caagggagaa gacaccagag 3120
   ggtactgatg ccactgagga aatagacaaa aatttggaag aaactggaag aagaaaaata 3180
   tccccaaggg aaaatggccc agaggaggtc aagcctgtag atgaaatgga gacagatttg 3240
   aacgcaactg gaagagagag ttctccaagg gagaagacac cagaggtgat tgatgctact 3300
   gaggaaatag atttggaaga aactgaaaga gaagtatccc cacaggaaaa tggactagag 3360
   gaggtcaagc ctctaggtga aatggagacg gatttgaaag caactggaag agacagtttc 3420
   ccaaggggga agacaccaga ggtgattgat gccattgagg aaatagagat agatttggaa 3480
   gaaactgaaa gagaaatatc cccacaggaa aatggcctag aggaggttaa gcctctaggt 3540
   gaaatgcaaa cagatttgaa agcaaccgga agggagattt ccccaaggga gaagacacca 3600
   gaggtgattg atgccactga ggaaatagac aaagatctgg aagaaactgg aagaagagaa 3660
   atatccccag aggaaaatgg cccagaggag gtcaagcctg tagatgaaat ggagacagac 3720
   ttgaaaacaa ctggaagaga gggttcctca agggagaaga cacgagaggt gattgatgct 3780
   gctgaggtaa tagagacaga tttggaagaa actgaaagag aaatatcgcc acaggaaaat 3840
   ggcccagagg aggtcaagcc tgtaggtaaa atggagacag atttgaaaga aattagagaa 3900
   gaaatttccc aaagggaaaa ggtgctagca gagttcagtg ctataaggga aaaggagatt 3960
   gatttgaaag aaactggaaa aagagacatt cccatcatgg agaaagtatc aggaaagatg 4020
   gctgttgttg aagaaatgga ggcagatttg aaagaaactg gaaaagaaaa ttttagagag 4080
   agaggatctg aagagatctg tgttactgag gaaaaggtgg cagaattgaa acaaactgga 4140
   aaaacagaca tttctccaag ggaaaacgag ctagaggaga ccagtacctc aagacaaact 4200
   gacacacatt taatgcagag cggtagcaat gacttcagtg ctatgccttc actagatatt 4260
   cagaacatta gcagtgaagt actgtcgatg atgcatacac ctgtagaaga aaaaagaaat 4320
   tctgaaaaag aagtatcaag tcacttcagt catttcaaga tttcttcaca gactcatgaa 4380
   tctgataaaa cagaagtcca ggggattcaa tctccagatg ttccagagca gttttcagat 4440
   attaatttaa gcaaatctct tcctcaagaa cagaagccac ttgaaattaa accagcacct 4500
   tttgtgagga gccgattcaa aagaccaaaa ccaaacttag caagagcagc tttgaagaga 4560
   gagactacag aatcagaaaa atatatatat gagaagaaat cagaaaccaa gaaaatggag 4620
   actattgtga tgcaagaaaa taatgaacaa actgatactc tcccttctca acatgatgaa 4680
   gcttccctaa tgatatcaag agaaaaagac acattaggtc acaggaatga ggaggctgtg 4740
   atattgccat gtacacagac tgaaaggaac ctttcacctt caaattcttg tgaacctaaa 4800
   gaggagtctc agtcagcacc agtccagaaa aatgactcag ttgtttctgt ggggactaat 4860
   aatgtaaaca ctttccagca agaaatgaag gaaagtgtta tccaaactgc tcgacaagta 4920
   aggggccgac ttcagagacc gagaccaaat ataagaaaga caggacagag gcaaatagta 4980
   gacaaaggtg aagccaaagg cataattaag gaaggaagaa cgatattacc aaaagatgaa 5040
   actgaaaaga aagtcttaac tgtgtcaaat tctcaaattg aaactgaaat tgaagttcca 5100
   tcgtccgcag ttccagaaca cagaatgtat gaaaatcaaa gtcaggtggt tcttgtagaa 5160
   aaccttcatg ttaacaaaac aaatgaaaca atcagacatg aaaataaacc gtatgttcct 5220
   agttcagcac aaatgacaag aaggaaattc caaaaggcta agccaaattt gggaagagca 5280
   cacagtaaga aagaggaacc agttttagaa aaagtcacaa cagatcagag caaggaaggc 5340
   aagccagaag atcatttgct gcagaaagga gcttccaaca cccagctcct tctaaaagaa 5400
   aaagctgagc ttctgacatc tctggaggtt tcagcaagaa aagattgtgt aggttccaaa 5460
   gagtctgctt tggcaaaaat agatgcggaa ttagaagaag ttggaccatc aagaagggtt 5520
   ggagaggaaa ctgtaggaga taattcacca tcttcagttg ttgaagagca atatctcaat 5580
   aaactaacaa gctgtccaca accgttaaac gaaacaagtt actctaaaat tgccctggat 5640
   gggaaaacaa ctatctcttc tacatctgag tatgagagaa atcgtggtga aaggagaagt 5700
   cataaaaagt tcaaaccaaa tgtcaccaga ggtcgtggat caaaacgagt tcggggtaag 5760
   acctctaaga aggaacctag agcttccaag gccatgctgg tgactcttcg ggcttcccag 5820
   gaagaagatg atgatgctga cgattttgag tctgactatg aggaagaaag ctatcatctt 5880
   gctcccgaag aagtaaacaa agctccagta tttgtacctg ttggtctcag atctcctgaa 5940
   cctgtttctg ctcagattga ggaaacaatg gaagagcttg aaataactgt gaatgtccca 6000
   gatgtaggat gcatagctgt tgttgaacat gagctaccaa acacagatgt gactactgaa 6060
   gaaatgaaac aagaagaaaa tttgagtgta ccgtttgaaa tgaccacaag tgaacatatc 6120
   caagatgaac caggtaccaa tgatggaagc accgaagctg caataacttt acttacaatg 6180
   ggagatctag tattgcagtc agagatcagt agtgaacagg gtgatgtagg agtatgtata 6240
   cttcctcatg ttcattcaaa ggataaaagc catattcctt ctagcctaga taatgtaaat 6300
   cacaaaattg ttcatgaatg tcaggaactt tcttcacctg tcattactac atctcctgca 6360
   tcatttgaag aaaacaagat tgtattggag gaacaaagtt ccagagaaga aataagttta 6420
   atggagaaag taaaggagaa tgctacacca accaggaata caatttctaa agtgaccagt   6480
   aatttgagaa taagaagtag gcttgctaag cctaaaccaa atcttgagaa gactttaggg   6540
   accaacaggc ttgatgatta tcaggaagtt tccagtttgt gtgtaaccaa aggggcagaa   6600
   atggaaactc aaagagaaac agagaaaaat gcttccaaag caacagaatt ggaaaataaa   6660
   aaccttggac cagttacaac agcagagaat aaggatcaga gcaaattggc atgtgtacat   6720
   ggtatcaaag ggaccagtat ttcttcagaa gtaaacctaa ctgaaagaaa cgaaaatcaa   6780
   gaagagagct ctcaggaggt tcacatgttg tcagttgctc cagttgcttc ctctgagaca   6840
   gggccctgca cacttggttt ggataggggt cttggtgaaa attctgttga agagccccag   6900
   ataaaggact ctaaaggaga cagtgtgctt acacttcctg tgccagaggt aaaagaatgt   6960
   acagtataat aagggatagc aaggtgtttt cctccattta aaaaaaaaaa aggtataatt   7020
   tacatacaat aaacttcacc tcttttatct tcatttttct ttatttcttc tttttttttt   7080
   ggagacagtc ttgttctgtt gcccaggcaa gagtgcagtg gtatgatctc agctcactgc   7140
   agcctctgcc acccagattc aacagattct cctgcctcgg cctgccgggt agctgggact   7200
   acaggca                                                             7207
<210> 117
   <211> 1264
   <212> DNA
   <213> Homo sapiens
<400> 117
   gtggtaccca gtcctcaggt gcaaccccct gcgtggtcct ctgtggcagc cttctctcat     60
   tcagagctgt tttccacaga ggtagtgaaa agaactggat tttcaagttc actttgcaag    120
   agaaaaagaa aactcagtag aagataatgg caagtccaga ctggggatat gatgacaaaa    180
   atggtcctga acaatggagc aagctgtatc ccattgccaa tggaaataac caatcccctg    240
   ttgatattaa aaccagtgaa accaaacatg acacctctct gaaacctatt agtgtctcct    300
   acaacccagc cacagccaaa gaaattatca atgtggggca ttctttccat gtaaattttg    360
   aggacaacga taaccgatca gtgctgaaag gtggtccttt ctctgacagc tacaggctct    420
   ttcagtttca ttttcactgg ggcagtacaa atgagcatgg ttcagaacat acagtggatg    480
   gagtcaaata ttctgccgag cttcacgtag ctcactggaa ttctgcaaag tactccagcc    540
   ttgctgaagc tgcctcaaag gctgatggtt tggcagttat tggtgttttg atgaaggttg    600
   gtgaggccaa cccaaagctg cagaaagtac ttgatgccct ccaagcaatt aaaaccaagg    660
   gcaaacgagc cccattcaca aattttgacc cctctactct ccttccttca tccctggatt    720
   tctggaccta ccctggctct ctgactcatc ctcctcttta tgagagtgta acttggatca    780
   tctgtaagga gagcatcagt gtcagctcag agcagctggc acaattccgc agccttctat    840
   caaatgttga aggtgataac gctgtcccca tgcagcacaa caaccgccca acccaacctc    900
   tgaagggcag aacagtgaga gcttcatttt gatgattctg agaagaaact tgtccttcct    960
   caagaacaca gccctgcttc tgacataatc cagttaaaat aataattttt aagaaataaa   1020
   tttatttcaa tattagcaag acagcatgcc ttcaaatcaa tctgtaaaac taagaaactt   1080
   aaattttagt tcttactgct taattcaaat aataattagt aagctagcaa atagtaatct   1140
   gtaagcataa gcttatctta aattcaagtt tagtttgagg aattctttaa aattacaact   1200
   aagtgatttg tatgtctatt tttttcagtt tatttgaacc aataaaataa ttttatctct   1260
   ttct                                                                1264
<210> 118
   <211> 2295
   <212> DNA
   <213> Homo sapiens
<400> 118
   acacgccgcg ctgaggcccg cgggcccgtc atggaggcgc ccaccgtgga gacgcccccc 60
   gacccctcgc ccccttcggc cccggcccct gccctggttc cgttgcgcgc cccggatgtg 120
   gcgcggctgc gcgaggagca ggaaaaggtg gtcactaact gccaagagag aatccagcat 180
   tggaagaagg tagataatga ctataatgcc cttcgagaaa gactcagcac cttgcctgat 240
   aaattgtctt ataatataat ggtaccattt ggcccttttg ccttcatgcc aggaaaactt 300
   gtccatacta atgaagtcac tgttttactg ggggacaact ggtttgcaaa gtgctcagca 360
   aagcaggctg taggtttagt tgagcaccgg aaagaacatg taagaaaaac aatagatgac 420
   ttaaaaaaag tgatgaaaaa ttttgaatcc agagttgaat tcacagaaga tttgcagaaa 480
   atgagcgatg ctgcaggtga tattgttgac atacgagaag aaattaaatg tgacttcgaa 540
   tttaaagcaa aacaccgaat tgctcataaa ccgcattcca aaccaaaaac ttcagatatt 600
   tttgaagcag atattgcaaa tgatgtgaaa tccaaggatt tgctagctga taaagaactg 660
   tgggctcgac ttgaagaact agagagacag gaagaattgc tgggtgaact tgatagtaag 720
   cctgatactg tgattgcaaa tggagaagat acgacatctt ctgaagagga aaaggaagat 780
   cgtaacacaa atgtgaatgc gatgcatcaa gtaacagact ctcatactcc ttgtcataag 840
   gatgttgcaa gttcagaacc attcagtggt caagtgaata gtcagttgaa ctgttcagtg 900
   aatggttcca gttcttacca cagtgatgat gatgatgatg atgatgatga cgacgacgac 960
   gacaacattg acgacgatga tggtgataac gaccatgagg ctttaggggt tggagataat 1020
   tctataccaa caatatattt ttcacatact gttgagccta agagggtccg aataaatact 1080
   ggaaagaata ccactttaaa attcagtgaa aagaaagaag aagccaaacg taaacgaaag 1140
   aacagcactg gcagtggcca ctctgcccag gagctgccga ccatcaggac gcctgcagac 1200
   atttacagag cctttgttga tgttgtgaat ggagaatatg tccctcgcaa atccatcctg 1260
   aagtctcgaa gtagagagaa tagtgtgtgt agcgacacta gtgaaagcag tgctgctgaa 1320
   tttgatgata ggcggggagt tttgaggagt atcagctgcg aagaagccac ttgcagtgac 1380
   accagtgaga gcattttgga agaggaacca caagaaaatc aaaagaaact tttgccctta 1440
   tcagtaacac ctgaggcttt ttctggaact gttatagaaa aagaatttgt atcaccttcc 1500
   ttaacaccac ccccagccat tgctcatccc gcactaccca ctattccaga acgaaaggaa 1560
   gttctgttgg aagcatctga agaaactgga aagagggttt caaagtttaa agctgccaga 1620
   ttgcaacaga aagactaggc cctgtctagg aaatgggaat ttacatccta aaacctagtt 1680
   gttcatttgt ttagagtatc tatagcaaaa taggttacat gtagtttgaa ataaggtatc 1740
   ctgagttact ttggcaacaa gttcttttac ccttacccgt ggtatttgaa aaaaatcaag 1800
   gtaactgtct gaatacttta atatcagctt gttttgtgaa ttctctgaat actgtcaaca 1860
   ctcttatcta agtttgcctt tatgatgcag tggcagcatt ttgaattact tttcaaagaa 1920
   tactgttcat atgcattgtt tttgtgtttc aaactaaata caggcagttt tgtgccagct 1980
   gtgatattgt gcataccata tggaccattt taaagaaaat tttaaaattt caaatagatt 2040
   caacaattac attacttgct ttcacatttt aaaggcactt taaaaaaatc tacttctctt 2100
   gtaggttttg cggctagttg gctattcaag aaacctcgcc cctctgaatg tcatactgta 2160
   atctttaagg aagaaagcta catgaattaa ttgtactcta tgggaaaatt tctttggaaa 2220
   gatattttgt aaaacttttt tttccaagta aaaactttat gaaacttggt ctcaaaaaaa 2280
   aaaaaaaaaa aaaaa 2295
<210> 119
   <211> 2745
   <212> DNA
   <213> Homo sapiens
<400> 119
   gcgcatgcgc gagtgtacgc gttgccggcg aagaggggag cctgacgact cggaaatttg 60
   aataccacag tagcatggag tgtgacctca tggagactga catcttggag tcgttggaag 120
   atctaggtta caagggccca ttgttggaag atggagcgct ctctcaggca gtctctgctg 180
   gagccagttc ccccgagttt accaaactct gtgcttggct ggtgtctgaa ttaagagtgc 240
   tctgtaaact agaggaaaac gtgcaagcaa ctaacagtcc gagtgaagct gaagaattcc 300
   agcttgaggt gagtgggcta ctaggggaga tgaactgccc gtatctttca ctgacatctg 360
   gggatgtgac caagcgcctt ctcattcaga agaactgcct cctcttgctc acatacctca 420
   tctcagaact agaagctgcc agaatgctct gtgtgaatgc tcctccaaaa aaagctcaag 480
   aaggaggcgg tagtgaggtc tttcaagagt tgaaaggcat atgtattgct ctaggaatgt 540
   ccaaacctcc agccaatata actatgttcc aattcttcag cgggattgaa aaaaaattaa 600
   aggaaacatt agcaaaagtt ccacctaatc atgtgggaaa gcctttactg aagaagccaa 660
   tgggaccagc ccactgggaa aagatagaag caattaacca agccatagcc aatgaatatg 720
   aagtccggag aaagctgcta ataaaacgtt tggatgtcac tgtacaatcc tttggctggt 780
   ctgacagagc taagagccag acagaaaaat tagccaaggt ttaccagccg aaacgttcag 840
   tcttatcccc taaaactact atttctgttg cccatctttt ggctgcaagg caggacttgt 900
   caaagatttt aaggacaagc agcggctcta taagagaaaa gactgcctgt gccatcaata 960
   aggtgttgat gggcagggtg cctgacagag gtggtagacc caatgaaatc gaacctccac 1020
   ccccagagat gccaccgtgg cagaagaggc aagatggccc ccagcagcaa acaggaggcc 1080
   gaggaggagg gagaggtggc tatgaacatt cctcatacgg aggacgagga ggtcatgaac 1140
   aaggaggcgg gagaggtgga cgtggtggct atgaccatgg tggccgaggg ggaggaagag 1200
   gaaataagca tcaaggaggc tggacagatg gagggagtgg tggaggaggt ggctaccaag 1260
   atggtggtta tcgagattca ggtttccagc caggtggcta tcatggtggc cacagcagtg 1320
   gtggctatca aggcggaggt tatggtggct tccaaacatc ttcttcatat acaggaagtg 1380
   gataccaggg tggtggctac cagcaggaca atagatacca agatggcggg caccatggtg 1440
   atcgtggtgg tggtcgtggt gggcgaggtg gtcgtggagg ccgaggtggt cgtgcaggcc 1500
   agggaggagg ctggggagga agagggagcc agaattatca ccaagggggt caatttgaac 1560
   agcatttcca gcatggaggt tatcagtata atcattctgg atttggacag ggaagacatt 1620
   acactagttg aggctaccga accttacatt ttgctagagc tcaagtaata gaaacttagt 1680
   ttcagaatcc tgaattcagc acctattttg aattaatgtg agaccacagg tggcaggcag 1740
   attcctgctt ggcataagca tttgtaggtc ttcattcaat tctgttagat ttttttattg 1800
   gacttacata atgccgttta tttgagaaac acataacatc tctcctttct atgaaaaatt 1860
   ttttaaaagg tggttaaaat tgcctttaat tgcccagtag actaattcca cagtcagaac 1920
   atgcaaactt ttttgaagaa attacttgaa taagtagttt tcatgttttc aatatgcagt 1980
   tttgaaaatg aggattcacc tagacttttt tagatttact actaggaaac cttcctcata 2040
   tgaataacca tttatatgtg ttttgcttaa agtattccaa tgcctatttt ccaagcacag 2100
   ttctgccccc cggttgactt ttatgccacg tgtgcttcat gatggaactt ttaggtcagt 2160
   tcctattaaa tgagctcttc tgcagatagc acattcagta gccttatttt gttgatggaa 2220
   tactgtatca tatgctcaac tctgaaaacc ttgaacacgg ccaaaatcca taaagattat 2280
   aaaagcaaac taagttgtga agctatagta catgtaggca tttagttaag tatagcaatt 2340
   caaactgacc tgcatccatc caaaacaaat tcctccttca accttatttt tacttgaaat 2400
   ttgctagaag aaatagcaaa ccgaaatttg ttttatgcat gagttaatac cactggctca 2460
   gcaaatacaa gttagtttgc tttaagcagg taactttttt tgtaatggaa gaaatgcact 2520
   acaaagttaa gacagatttt tgctaagtgc aggaggccct ttattattgc tgcagaaaac 2580
   aaaagcctgg ctgagttgat gttttacatt ctcccttact gaaatctaca tgacatgatg 2640
   cttcttgctg ggtttttgta catgtaaaca ttgtcaagct gtgaaagaaa atggctggag 2700
   gtgtgctttg tgtgaaaggt gagcaataaa gtatctgtta agttc 2745
<210> 120
   <211> 2727
   <212> DNA
   <213> Homo sapiens
<400> 120
   cacgagaaga caggaggaag aaagggagag agggccaggc agtcgcactg tgaacagaac      60
   aggagaaggc gaagcggggc aaagttccct gcccaccgac gccagcctgc ttggatgact     120
   tgcctcgttt cataattcac ttactgtctg caccagccgg cctcagcctg gctggaccct     180
   gctgcctgtg tggcccggag ccagaggccc ccacactccc agctgctctt ctacagatgc     240
   catcaacgag caggactctg ggtggctcca ctgtctaagc ctggagagtc accgccgagg     300
   gatgaggacg cgccagcccg ggggaacgcg ccagctgctt tcgcggcccc aagcgcgcag     360
   tgcccagcag ccgcgccgag cctgacacgc tgtcctctcc cctcgcgcac agggctctgc     420
   gagtgacccg gcgggcgagc tccgtgctgc atggaacggc tgcagaagca accacttacc     480
   tcccccggga gcgtgagccc ctcccgagat tccagtgtgc ctggctctcc ctccagcatc     540
   gtggccaaga tggacaatca ggtgctgggc tacaaggacc tggctgccat ccccaaggac     600
   aaggccatcc tggacatcga gcggcccgac ctcatgatct acgagcctca cttcacttat     660
   tccctcctgg aacacgtgga gctgcctcgc cagcgcgagc gctcgctgtc acccaaatcc     720
   acatcccccc caccatcccc agaggtgtgg gcggacagcc ggtcgcctgg aatcatctct     780
   caggcctcgg cccccagaac cactggaacc ccccggacca gcctgcccca tttccaccac     840
   cctgagacct cccgcccaga ttccaacatc tacaagaagc ctcccatcta taagcagaga     900
   gagtccgtgg gaggcagccc tcagaccaag cacctcatcg aggatctcat catcgagtca     960
   tccaagtttc ctgcagccca gcccccagac cccaaccagc cagccaaaat cgaaaccgac    1020
   tactggccat gccccccgtc tctggctgtt gtggagacag aatggaggaa gcggaaggcg    1080
   tctcggaggg gagcagagga agaggaggag gaggaagatg acgactctgg agaggagatg    1140
   aaggctctca gggagcgtca gagagaggaa ctcagtaagg ttacttccaa cttgggaaag    1200
   atgatcttga aagaagagat ggaaaagtca ttgccgatcc gaaggaaaac ccgctctctg    1260
   cctgaccgga cacccttcca tacctccttg caccagggaa cgtctaaatc ttcctctctc    1320
   ccccgctatg gcaggaccac cctgagccgg ctacagtcca cagagttcag cccatcaggg    1380
   agtgagactg gaagcccagg cctgcagaac ggagagggcc agagggggag gatggaccgg    1440
   gggaactccc tgccctgtgt gctggagcag aagatctatc cctatgaaat gctagtggtg    1500
   accaacaagg ggcgaaccaa gctgccaccg ggggtggatc ggatgcggct tgagaggcat    1560
   ctgtctgccg aggacttctc aagggtattt gccatgtccc ctgaagagtt tggcaagctg    1620
   gctctgtgga agcggaatga gctcaagaag aaggcctctc tcttctgatg gcccccacct    1680
   gctccgggac ggccccctta cccctgctgc ttcagggttt ttccccggcg ggttgggagg    1740
   ggcaggaggt ggggtggaaa tagggtgggc tcctttcctc aggtagagtg gggggccaaa    1800
   acctctgcag tccccggcag tgagctatgg actttcttcc ccctcacgag gctgggggcc 1860
   tcctgctctc gtccctggcc ctccctgtac agggcaaagc cagtctgggc tctggcacac 1920
   agagttcatg tttgccgccc tctccctgcc cctcacccca gaggtgagag gaatgagggg 1980
   cattggtggt taggccggtt ggctgtcttg aacagctgga gggaagatgc aggggtggga 2040
   agcggccagg cagaaagagc tccaggctct tgtgtcgccc acccagccct cccatactca 2100
   ctcctgacag ctttcctgca ctgcagcttc ctgctcctct gactctagtg ggaacaggcc 2160
   ccagctcagc ctccgcgagg gaggtcaccc ctccacttca gcttgccctg acctccgctc 2220
   gcaaaccccg agcttccaag ccttttgctc cagccctgcg gcttccccag aagcctgggc 2280
   ttagggtgga gatgccgcct acccgatcct ggccctccac ctgcctccag gccacgaaat 2340
   gggaattcca gcactaagcc aggcaccggg cagaagctgg gccttccgcc tcccttggat 2400
   ggggtcaaga ggccaggcct ggcacatttt ggagtgtcct ggctaccagc tctcacacct 2460
   acacccacgc accccctcac acactatgct ctctcaagaa tgtaatttat tggggccccc 2520
   ccagctgctt tcctcacctg cccctgccct accttacacc cccagcttga cttctttcca 2580
   gtccacgtgg atataatgat atctatattt ttgcccaggt ctgggtattg ctcctgccca 2640
   gaccctgaca tccctttcca ctgtgtgtgt gaccatgctg ggggaggggg actctgcttg 2700
   gaattaaaag gttgctttgg gtcccta 2727
<210> 121
   <211> 5695
   <212> DNA
   <213> Homo sapiens
<400> 121
   gcggctcagg ggcggggacg ggggcgtggt cccgccgtat aagaacggag ggggcggcgg     60
   cgggacggct cactcggtgc cgctgcctag gggctgtaga ggtcgcgccg ctcctgctgg    120
   ggcctgccca cgccaaggac ctgcctctgt cgcctcctct tctattgccc agtttcccca    180
   gccagaacat cccctgaaga tggcagagga gagcagctgt accagggatt gcatgtcctt    240
   cagcgtgctc aactgggatc aggttagccg gctgcatgag gtcctcactg aagttgtacc    300
   tatccacgga cgaggcaact ttccaacctt ggagataact ctgaaggaca tcgtccagac    360
   cgtccgcagt cggctggagg aggcaggcat caaagtgcac gacgtccggc tgaatggctc    420
   cgcagctggc cacgttttgg tcaaagacaa tggcttgggc tgcaaagacc tggacctaat    480
   cttccatgtg gctcttccaa cagaggcaga atttcagctg gttagagatg tggttctgtg    540
   ttcccttctg aacttcctgc cagagggtgt gaacaagctc aaaatcagtc cagtcactct    600
   gaaggaggca tatgtgcaga agctagtgaa ggtttgcacg gacactgacc gctggagcct    660
   gatctccctc tccaacaaga acgggaagaa cgtggagctg aagtttgtcg actccattcg    720
   gcgtcagttt gagttcagtg tggactcttt ccaaatcatc ctggattctt tgcttttctt    780
   ctatgactgt tccaataatc ccatctctga gcacttccac cccaccgtga ttggggagag 840
   catgtacggg gactttgagg aagcttttga ccatctgcag aacagactga tcgccaccaa 900
   gaacccagaa gaaatcagag gcgggggact tctcaagtac agcaaccttc ttgtgcggga 960
   cttcaggccc acagaccagg aagaaatcaa aactctagag cgctacatgt gctccaggtt 1020
   cttcatcgac ttcccggaca tccttgaaca gcagaggaag ttggagactt accttcaaaa 1080
   ccacttcgct gaagaagaga gaagcaagta cgactacctc atgatccttc gcagggtggt 1140
   gaacgagagc accgtgtgtc tcatggggca tgaacgcagg cagactctga acctcatctc 1200
   cctcctggcc ttgcgtgtgc tggcggaaca aaacatcatc cccagtgcca ccaacgtcac 1260
   ctgttactac cagccggccc cttacgtcag tgatggcaac ttcagcaact actacgttgc 1320
   ccatcctcca gtcacctaca gccagcctta ccctacctgg ctgccctgta actaaccttg 1380
   agacctgagg gtttccacag tgggaacccc aatagggcta gggctctcag gtaggggagc 1440
   ctccttctag atgtaggcat ttggctttta aaggggaact cagctctgat tctgcttttt 1500
   tttttttttt tcctttgtgt acccattgga atgggtctac agtgtatcat gagccaaccc 1560
   tcaaaggacc cgtattacag tgccacgttg gaaaacgcta caggaagcat gacctatcca 1620
   catctttcca agatagacac taacatgtca tgtcccaaac attagcacgt gggggttgag 1680
   ctctgtgcag taatcgagat tgggagaatt tgggcagcgc gtgagaagtg ctaagctact 1740
   tgttttctca cttgagcccg ggtaggctgt gttggccctc acttgggatt ctcagcagtt 1800
   acatgaaagt tgtgctgata atctcttctc ttgtaccaat tttagtcagg cagaaaatgg 1860
   taaacatgag ggtgctcttg tgacttaatt tttgttcaag ggactaaatt gcttatgttt 1920
   attccctgtc agcggagtgg agaatgtcat tcatcaataa accaaagcca atagctggag 1980
   aattgagatc tggttgaaag tggtttatgg tttacatgct gtactatcct gaggaattgc 2040
   gagatattgc tgaggggaaa aaaaaatgac cttttcttga aatgtaactt gaaaacaaaa 2100
   taaaatgtgg aacataatgt ttaattagaa ttgtggtggt ggtagtggaa ggggataatt 2160
   gtaaatagga aacatgaatg ttcatttttt tctttaaaga attcttatta aatggctccc 2220
   tgcctttttt ttcttttttc ctcatcagct ctttcatggc tgaattttgt tttattcttc 2280
   ctaagactga ggattgtgct gagtccagag tcattgtggt aactgacatg agggtcttcc 2340
   catgttttaa ctggaaaccc actttggtca cattccaagt atgacacagc tgttcttctg 2400
   gagtacttta gctatttttt gtttttgttc ttttcgtttt tttttttccc aaaaatagtg 2460
   acttccttct ccaggtgtgt ttgacagcaa ctcaattcag gaatttcggt agaactgagt 2520
   gacctgtgga actgctttag aatctaacct gctgtcttcg tgctctgtgt gaaggggaag 2580
   ctggggggtt agcatgaagt ctggccttgt gtgcattgga gcttccaagg cactttgaaa 2640
   tcattccagt atatttggga agaattgagt gaatgagaat gctcttcctt attctggtag 2700
   atttgacttg tttataattc tgcactttag aagaaaaaca gtgttaatct gtagttgaaa 2760
   gaaagcttag tagatgagag agttctaggc tactgtggct ttttccagta gatttagatg 2820
   agattatgtg ttttgaaatg ttttgtggga tcccttagaa agcatcactt cagggcagag 2880
   acactcaata ttgccagcca gcttgggttc taaagtgatt taatcaaatt catgctcctg 2940
   atcttttttt tcccccttcc tttggctatg aaaacccaaa gcccggagtg attgttttct 3000
   ccttgcttta agcagtgaag ttatcctaat gcaaaagagc ttagtagaaa atgagtggtt 3060
   tacctttttt tctaaaagta tattttcaag tttattctgg aatgtgatgt cttggtcctc 3120
   ttaaaagcag atcagccatg actgaaactc aaggcttagc tggtatctat gttgtgctac 3180
   attaggtgac tagaagccac ttcttagtgt aatcagctcc tgtttccctg tgagccttag 3240
   ttatatttta attcagtggc tttgagtcaa ggccggttct aattgagggg acccagtgtg 3300
   cttcagtgtt aagagtgggg caatgaagag tgaaccccaa tgaagagtga tcccaacttt 3360
   ggaaactatc tggtcattca tgaccttaaa aagctgccat ggtggtcaaa tggcatgtgt 3420
   ttgcacaaaa atgaccgatg tgtttaacca aagctttgaa atgtgatgaa gccaccaaca 3480
   taagcacttg cctaacagaa atcagtattt cttctactta gaaggcttgg ggcccagggt 3540
   aatgaggcac cagatgaaga taagatctgc atcaaggaat taaatttcca gtttgtcctt 3600
   ggggttcttt ctgcttatgt tttttccccc ccatctggta atagtcctct tctgagaatg 3660
   aaaggaggca cagaagttgc gagaaagacc cagtccccag tctttgccac cattgcacca 3720
   tggttgtctc ggagtccctt cacctgacct ttttagtcag ctcagatctt tttgtagttt 3780
   gagaatagac ctattcaaga gctatcaggg ttaaaagctg cactgaccaa agctgtactt 3840
   ttaaaacaca acccctgggc tagtgatgtc tctacttgct ctatgagcct tttgccagcc 3900
   tcagaatagg cgaggtaacc ttgaatttca gggccctgga aactctagct gaaacctgcc 3960
   tttcctaatg ctcacacaag caccaggtac cctgagctta tactgagtcc agtggttcct 4020
   attttatgtg tgtgtgtatg tgtgtgtgtt gagtaacatg aaacttggca gtagcagaga 4080
   gcactgaagt tcacatgcaa gttctaatct aaagttaagc agtctcttat ttgtttcggg 4140
   actctgattt gactcttttt ctgatgcttt cggcatgtct gcagcctgtt ctcctagcct 4200
   gctgcttgga gtcaggttga gcatcagtga gatgaagaca gtaccttttc ctctcacatt 4260
   ggcagaatag cacgcactag atgcctgacc ttgagctcta gtctcccctt taaatcttac 4320
   cttggcagta acacattatt cctcattcaa taatttcaat gctgaaactg aactctatta 4380
   ctaatgcctt ccaatcagag ttcctgatgg ggatgcctgt gggatggccc acacctgggg 4440
   gacctggcag atggggtgag ttgggtaagg aagatgatgc ttagttcctg atagatgcta 4500
   cagatgtagt ttggcatttc agtttttgtc cagtttgatt ttcactgggg tttgagtcac 4560
   agcaagctgt gtatgaactt gtgttttgtg gtgacacatt aataatcaaa ttgctaaaac 4620
   cctgaatctg cttattcttc agcttcacct ctgcactaac cccttaccct tatggtacgt 4680
   caggatttta actagtactg ctttgttgac tttgggaaat ggtgccactc aaaagcaact 4740
   tcctaacttg aggaataact cctttgtagt ttactttctg gtactggttg gtgccttgta 4800
   ttgggataca gctatattct tagctcaaat gtcctctttt tgagagcaaa gtagttatcc 4860
   aatggtgaga cgagaccctg acgctcatag aggccattcc ttcctgggtg tcggaccagg 4920
   gctctgtgtc agggaaaacc ttctgggtgg actttgtaag aatccagttt ccaaggttag 4980
   attcacatcc ttaatcttgc agagactaga atttcacagc tcgctttgga acatattcca 5040
   attcaaccag ttaaaatcag aggaccaagt cgtgggggag gggggcggtg ttgaggagag 5100
   gtatttttaa agatctggca acttttcagg attatttgtg gagaactcta aggttaagat 5160
   caggaaataa aagactgtgt gtgtgtgtgt gtgtgcgtgt gtgtgttcaa gtgcctaaat 5220
   cttgtttacc tatcacttta aaaaaataat tgaagtgtaa gctaaataaa atgcttggag 5280
   ttttgcctgg gctagtgaga gttggtgcaa attcttgtgt gtgtttgcat aggaaggtga 5340
   gatgaccatc tactaaagag gaagtagcta aatacagatc tgtgggtgtt tttaaaaaaa 5400
   ctcaacctat ctggtgtttt attttaatgg ataaaaatgt aatttttcta aggtagcaac 5460
   ttatttccaa attaatatag atgaaaaata gataccaatt agactaaatt gaaagctttt 5520
   tgttctatat ttgcatagcc tttgaaatat ttcttagtgc ctaggaggtc tggggattcc 5580
   tctttcgtgg tggtcactaa ccttacttga tgcagataaa atcacttgtc aatgcaaaat 5640
   gtgttagaac ttgataaagc tttgagtttg agaaataaag gtatatttaa aattt 5695
<210> 122
   <211> 1531
   <212> DNA
   <213> Homo sapiens
<400> 122
   ccggcccacg ccagctcccg gccgcggcac agcagccccg gcgctccccg cgccgccccg     60
   cgcaggcgcc cccgccccgc cgtcgccgcc gccgcagcca ggagccgctg caccatgccc    120
   cgcatagatg cggacctcaa gctcgacttc aaggatgtcc tgctccgacc taagcggagc    180
   agcctcaaga gccgagccga ggtggatctt gaacgcacct tcacgtttcg aaattcaaag    240
   cagacctact cagggattcc catcatcgtg gccaacatgg acactgtggg cacgtttgag    300
   atggcagccg tgatgtcaca gcactccatg tttacagcaa ttcataagca ttactccctg    360
   gatgactgga agctctttgc cacaaatcac ccagaatgcc tgcagaatgt agccgtgagt    420
   tcaggcagtg ggcagaatga tctggaaaag atgaccagca tcctggaagc tgtgccacag    480
   gttaagttta tttgcctgga tgtggccaat gggtattcag aacattttgt ggaattcgtg    540
   aaacttgtcc gtgccaaatt tcctgaacac accattatgg cagggaacgt ggtgacagga    600
   gaaatggtag aagagcttat tctttccgga gcagatatca tcaaagtggg agttggacca    660
   ggttctgtgt gcaccacccg caccaagacg ggagtggggt acccccagct gagtgccgtc    720
   attgagtgtg ccgactctgc ccatggcctg aagggccaca tcatctctga tggaggctgt    780
   acgtgtccag gggatgtcgc caaagccttt ggagctggag cagattttgt catgctggga     840
   ggaatgtttt cgggtcatac ggagtgtgct ggagaagtga ttgagaggaa cggacggaag    900
   ctcaagctct tctacgggat gagctctgac accgccatga acaagcacgc aggaggagtt    960
   gctgagtaca gagcctctga gggtaagact gtggaagttc cttacaaagg agatgtggaa   1020
   aacactatcc tggatattct cgggggactg aggtccacgt gcacctacgt gggggccgcc   1080
   aaactcaagg agctcagcag gagggcaaca ttcatccggg tgacccagca gcacaacacc   1140
   gtgttcagct aaccctgggg acaaagcagc gtctggctcg agtggaagcg tccaaacctg   1200
   cttttcccat ctccccccaa gtctgttccg tcagagcttc tggctgctcc tgaatggtgg   1260
   aatgctgtgt cctctcttct gtctcctgcc gcctggaggc ttcggggctc tcccgcctgc   1320
   cttgtcgggg cccagacgca aggcaccgat tgggccaaca tcagagccct gctgcccaga   1380
   actcataacc tcattgttca aaccaacact tgcacctttc tctttttctc tttctctctc   1440
   cctttctttg tttttctttc ttttttaaaa gaagatggtt tcactttaat ataatgctat   1500
   tatcttaaga cttaaaaaaa aaaaaaaaaa a                                  1531
<210> 123
   <211> 3088
   <212> DNA
   <213> Homo sapiens
<400> 123
   attgcaatta ctcattttga taaagtttat tcttaggcgt taaccatttc taaaggatcc     60
   ccaaatcatc acttacttag gtgaaattat ataaaatgac acatttctga gaaatgttta    120
   ggtccagtga accgtagttg gtttatggga gtgatttcga gatagccaaa tgaactttga    180
   tttttgtttt gaatgtggtg gagtcaagag attgtagatg cctagtttga tttaaacact    240
   attgtaaacc tatcttgcct attgtgtgga caccaaaaga gaccaatgag cctgtttatt    300
   tttcagaggt ctaggaaaat tgcatccgtg tgagtagatg tacagaacta atctaaaagt    360
   gactgctagt aatattttag aatataataa tttcaagaat tattctgagt gttttaaatg    420
   tgccctgaaa tgttggcatg tcatttcagc atttccattt gaattgctct tgtaatattt    480
   ttgcacaaaa aggactgaga aaaagactac tttggttaaa ggaaaaaaat agtataattt    540
   ggtctttaat tttaatgtct cctgtggaaa cactggggat gaattttgtt ggcacagtta    600
   ttcaggatat ttaaaacagt gttgaacagt tcacaagaaa ttaagcaaac ttggatttta    660
   aaaattaaaa tactttcttt ccatgtgact gttttgcata gtttttcccc cctatgtcat    720
   aacactacat tccttgtttt tcttaaataa aatactctgc aggttttgtt gtgagtgttt    780
   ataacagtat tttgtaagct gcctgggttt cctcagggaa acattattta gtggaatagg    840
   atatagttta tggagagaca actaagtgaa atcccatcac caccaccagc aagcactgag    900
   gtgctgcatt aagtgacaat caaactagta agtatttatg aaagaattta gaaaggggtt    960
   caaagaagca attgctttta aagcagttct ctggggttct tgaataaaaa tggctaaatg   1020
   tatttattta tattttaaaa tacctgtgtg ttctgttttc tcatcaaagt gatttcggtg 1080
   tcatgtattt aatgtgtggt ttgttttgtt ttgcaccata ttcttcttag ggaactggct 1140
   ctgatttggg gtgcagtcac caactctcat gataatcttt acggacagtc tgaatctact 1200
   ttacttcagg acggcagtgg aggggccaac tccagatgcc cctgcacttc cctcctcaag 1260
   tcatctaaga aagaatttta gaaataactc ctttcaaatc tgattcatac agttccaacc 1320
   tttttctagt ttcacttgct gccttctttg ttttatgggc actttttgtg tgattacttc 1380
   cccaacacag tcaaaggcca gtatgacaga accaatggca catgatgcct ctattttgcc 1440
   catttgggga aatgttagta aaagaaatag catcttttaa tggctgtcat tatcatatcc 1500
   atattaaaat ccatttaaag ttcttaaatt tggcggaata atgtatacag gctgtaaact 1560
   tctacaaaaa atgtttgctg tctttaatat ggtgtcaagt tacttggtcc tttgaaaaca 1620
   aaacaaaact tccatacaca gcactgccca gtaaagcagt gttggaatat tggaaaccta 1680
   gacattcgag gatccttctt gctcacaagc attggaacag aatcctccac tttaggactc 1740
   agagaagcta tggagtccca caggctaggt atagcacaca acttagagaa cggtagttaa 1800
   agcacgtcaa ccacaattga atttaatgat ctaaaaaata tttcttaact ctgagggtta 1860
   aactgaagat acgttttcaa ataaaagctt ttaaatttct taggaaacaa actgtgacat 1920
   cgcttttaaa aatgacattc aaatgtttat aatgtgtgtt tttgagttgg cttggactag 1980
   tgcaagttca ctgctggcaa tggttatgcc agtagttaag ttaaaataag ttgttacctt 2040
   cagattgatg tcttgtataa ccacaatcat cttcaagcaa aaaaattggc caggaaatgt 2100
   ctttttaaaa aaaaaaaaaa aaaaagatgt gtcagccaga acagttacca ttcagaccat 2160
   actggaatga ttttgaaatc tttactatgg tttaagttgg ggaaggcctc agtatggcat 2220
   tttcatattc actctatgta aagattgaag cccaaagatg agttaaattc agtgctgtat 2280
   tgaaaccagt cagtgcaaaa aaaaaaaaaa gttttacctt tgtaaatttt tcattgttaa 2340
   catcagttgg tagttgttaa aacatgcacc aaattcttta taatgttctg tgtggttctc 2400
   aatgtgatat tcttgacatt gctgtcctta ctgcttggtt cttggtttta aattaaaaag 2460
   tagatgtagc tcatcatttt tacttccaaa acggtggagc taaaaacggc taattgatct 2520
   tgagataaga atacccttta ctaaggcaaa gatttaattg aaatccaaaa tgatttatag 2580
   aaacgaaagg cattttccat ttgttgtctt ctgccgaaat tctgaattgc atacacgcat 2640
   gaaggataat gaatggtttc aacggtgcta tattaggttc tctgattttc atgacctatg 2700
   tatgtttgga aatacaatat tgtataaaat gtggatcgtt ttgttaccga tttaatattg 2760
   ccattttaat ttggtgtaac aattggttac acttgttttt caaagctatc ctttgaggag 2820
   aaagtcaaaa tgtgttgtta gcctacctcc actcaggcct cttgctgtgt aatcattatt 2880
   ccaggttatg atgtgcatag cttttaaaac tgcgtgtact attattctgc tctttcccat 2940
   actcttgtca tgtattcaga acctaaactt atagcaaaag agccaagcag tagcagtttt 3000
   tgtaaatgta aaaataatta gatccagact ttgtttcctt caattttaaa aataaaaaat 3060
   acctcagtgc taaaaaaaaa aaaaaaaa 3088
<210> 124
   <211> 550
   <212> DNA
   <213> Homo sapiens
<400> 124
   ggatattact ttccttgaaa tctaaaagtc tgagaatcat gtaatgaaca ggtaaaagaa 60
   aatgtaagaa cattatttaa gaagaccatg tttttgtaga ggaaaaaaga acatccaccc 120
   tttacagtta tttatttgtc cccaactttt ggaattaaaa gacctgaact tgacatttat 180
   gtatttatat ttggctaaac atcttgaaac ctttgcaaaa aaaacaaaag ccagaaaaaa 240
   taaaacaaat attaacaata acaatttttg ggtgtgaaaa ttcatgggaa atgtattttt 300
   tttctgtcca ctttagtata ttctcaggat tttctatgaa atgtatacac aacttattca 360
   aaatgaattt taaaatgtaa aataactttt aatatcacta cgttttaagt attattttaa 420
   acataataat tctgaaacaa aatagagaat tcctgacaac caatctcacc tctgaactgc 480
   tcttttttaa ggcattggta agacaatctt tgtttttgtc ccaaccactt ctatctatcc 540
   tcctatacta 550
<210> 125
   <211> 996
   <212> DNA
   <213> Homo sapiens
<400> 125
   agcgtgtggg aagaaaactg ggttgatccc tatcttcttt ttaaggagcc acatttgcca 60
   tttattattg ctcttttgcc tatttaaaaa aaataacagt cagaaaaaat aatgattatt 120
   aaaatgatag tcattattta cttagtgaat gtcaggatag gtatttatta ctcttagctt 180
   cttattggta tcagatgtag atcacggtct tactgttgat taactcttca gttatatata 240
   catactttga ttctttagtt acataattga taacaaagtg tgcttctgaa gaattattct 300
   ggctgtatgg ctcttgtttc ctccccttgg taaaaatgga ggcatgaatc tcagttttgt 360
   gatctgcttt taatttcata tatgacatta tttgatatac ataattacca acatccaaaa 420
   gcgtttcttc ccctttctac gtgtgcacta aattctaaaa caaaaattgg aatacaaaat 480
   gcctaataaa gaaaaaattc atgtacagaa aagcaaaaga taatctactt tgttaagata 540
   atttgtcact agctggttga ataatgcaca gtttttgttt aaggttacac atttatgcct 600
   ggattaaata acaatagtaa aacttgacaa gtcttttgaa aaacagctgc taaacatatt 660
   gctttctgga atgtaatcat tagcaacaaa tattctctta ttctatacaa ttgcagaatg 720
   ctagtggaac tttcttgtag gttatttgac aatgaatgtt aattgattta ttttttaaaa 780
   atgtaaaatc ctcttatccc tttaaggccg gtttaggctt tcatgtccct gtggtagaaa 840
   atttcaaatg ctggcttcta atttctactc actgaagtgt cttccagaca taccagatgg 900
   ttggagctcc tttgatagta tataagggag ctacataaat gtatttgaac taactcgtga 960
   gcaaaatttc tgttttcaga aaaaaaaaaa aaaaaa 996
<210> 126
   <211> 3334
   <212> DNA
   <213> Homo sapiens
<400> 126
   attcctgcct gggaggttgt ggaagaagga agatggccag agctttgtgt ccactgcaag     60
   ccctctggct tctggagtgg gtgctgctgc tcttgggacc ttgtgctgcc cctccagcct    120
   gggccttgaa cctggaccca gtgcagctca ccttctatgc aggccccaat ggcagccagt    180
   ttggattttc actggacttc cacaaggaca gccatgggag agtggccatc gtggtgggcg    240
   ccccgcggac cctgggcccc agccaggagg agacgggcgg cgtgttcctg tgcccctgga    300
   gggccgaggg cggccagtgc ccctcgctgc tctttgacct ccgtgatgag acccgaaatg    360
   taggctccca aactttacaa accttcaagg cccgccaagg actgggggcg tcggtcgtca    420
   gctggagcga cgtcattgtg gcctgcgccc cctggcagca ctggaacgtc ctagaaaaga    480
   ctgaggaggc tgagaagacg cccgtaggta gctgcttttt ggctcagcca gagagcggcc    540
   gccgcgccga gtactccccc tgtcgcggga acaccctgag ccgcatttac gtggaaaatg    600
   attttagctg ggacaagcgt tactgtgaag cgggcttcag ctccgtggtc actcaggccg    660
   gagagctggt gcttggggct cctggcggct attatttctt aggtctcctg gcccaggctc    720
   cagttgcgga tattttctcg agttaccgcc caggcatcct tttgtggcac gtgtcctccc    780
   agagcctctc ctttgactcc agcaacccag agtacttcga cggctactgg gggtactcgg    840
   tggccgtggg cgagttcgac ggggatctca acactacaga atatgtcgtc ggtgccccca    900
   cttggagctg gaccctggga gcggtggaaa ttttggattc ctactaccag aggctgcatc    960
   ggctgcgcgc agagcagatg gcgtcgtatt ttgggcattc agtggctgtc actgacgtca   1020
   acggggatgg gaggcatgat ctgctggtgg gcgctccact gtatatggag agccgggcag   1080
   accgaaaact ggccgaagtg gggcgtgtgt atttgttcct gcagccgcga ggcccccacg   1140
   cgctgggtgc ccccagcctc ctgctgactg gcacacagct ctatgggcga ttcggctctg   1200
   ccatcgcacc cctgggcgac ctcgaccggg atggctacaa tgacattgca gtggctgccc   1260
   cctacggggg tcccagtggc cggggccaag tgctggtgtt cctgggtcag agtgaggggc   1320
   tgaggtcacg tccctcccag gtcctggaca gccccttccc cacaggctct gcctttggct   1380
   tctcccttcg aggtgccgta gacatcgatg acaacggata cccagacctg atcgtgggag   1440
   cttacggggc caaccaggtg gctgtgtaca gagctcagcc agtggtgaag gcctctgtcc   1500
   agctactggt gcaagattca ctgaatcctg ctgtgaagag ctgtgtccta cctcagacca   1560
   agacacccgt gagctgcttc aacatccaga tgtgtgttgg agccactggg cacaacattc   1620
   ctcagaagct atccctaaat gccgagctgc agctggaccg gcagaagccc cgccagggcc 1680
   ggcgggtgct gctgctgggc tctcaacagg caggcaccac cctgaacctg gatctgggcg 1740
   gaaagcacag ccccatctgc cacaccacca tggccttcct tcgagatgag gcagacttcc 1800
   gggacaagct gagccccatt gtgctcagcc tcaatgtgtc cctaccgccc acggaggctg 1860
   gaatggcccc tgctgtcgtg ctgcatggag acacccatgt gcaggagcag acacgaatcg 1920
   tcctggactc tggggaagat gacgtatgtg tgccccagct tcagctcact gccagcgtga 1980
   cgggctcccc gctcctagtt ggggcagata atgtcctgga gctgcagatg gacgcagcca 2040
   acgagggcga gggggcctat gaagcagagc tggccgtgca cctgccccag ggcgcccact 2100
   acatgcgggc cctaagcaat gtcgagggct ttgagagact catctgtaat cagaagaagg 2160
   agaatgagac cagggtggtg ctgtgtgagc tgggcaaccc catgaagaag aacgcccaga 2220
   taggaatcgc gatgttggtg agcgtgggga atctggaaga ggctggggag tctgtgtcct 2280
   tccagctgca gatacggagc aagaacagcc agaatccaaa cagcaagatt gtgctgctgg 2340
   acgtgccggt ccgggcagag gcccaagtgg agctgcgagg gaactccttt ccagcctccc 2400
   tggtggtggc agcagaagaa ggtgagaggg agcagaacag cttggacagc tggggaccca 2460
   aagtggagca cacctatgag ctccacaaca atggccctgg gactgtgaat ggtcttcacc 2520
   tcagcatcca ccttccggga cagtcccagc cctccgacct gctctacatc ctggatatac 2580
   agccccaggg gggccttcag tgcttcccac agcctcctgt caaccctctc aaggtggact 2640
   gggggctgcc catccccagc ccctccccca ttcacccggc ccatcacaag cgggatcgca 2700
   gacagatctt cctgccagag cccgagcagc cctcgaggct tcaggatcca gttctcgtaa 2760
   gctgcgactc ggcgccctgt actgtggtgc agtgtgacct gcaggagatg gcgcgcgggc 2820
   agcgggccat ggtcacggtg ctggccttcc tgtggctgcc cagcctctac cagaggcctc 2880
   tggatcagtt tgtgctgcag tcgcacgcat ggttcaacgt gtcctccctc ccctatgcgg 2940
   tgcccccgct cagcctgccc cgaggggaag ctcaggtgtg gacacagctg ctccgggcct 3000
   tggaggagag ggccattcca atctggtggg tgctggtggg tgtgctgggt ggcctgctgc 3060
   tgctcaccat cctggtcctg gccatgtgga aggtcggctt cttcaagcgg aaccggccac 3120
   ccctggaaga agatgatgaa gagggggagt gatggtgcag cctacactat tctagcagga 3180
   gggttgggcg tgctacctgc accgcccctt ctccaacaag ttgcctccaa gctttgggtt 3240
   ggagctgttc cattgggtcc tcttggtgtc gtttccctcc caacagagct gggctacccc 3300
   ccctcctgct gcctaataaa gagactgagc cctg 3334
<210> 127
   <211> 1666
   <212> DNA
   <213> Homo sapiens
<400> 127
   ctccataagg cacaaacttt cagagacagc agagcacaca agcttctagg acaagagcca      60
   ggaagaaacc accggaagga accatctcac tgtgtgtaaa catgacttcc aagctggccg     120
   tggctctctt ggcagccttc ctgatttctg cagctctgtg tgaaggtgca gttttgccaa     180
   ggagtgctaa agaacttaga tgtcagtgca taaagacata ctccaaacct ttccacccca     240
   aatttatcaa agaactgaga gtgattgaga gtggaccaca ctgcgccaac acagaaatta     300
   ttgtaaagct ttctgatgga agagagctct gtctggaccc caaggaaaac tgggtgcaga     360
   gggttgtgga gaagtttttg aagagggctg agaattcata aaaaaattca ttctctgtgg     420
   tatccaagaa tcagtgaaga tgccagtgaa acttcaagca aatctacttc aacacttcat     480
   gtattgtgtg ggtctgttgt agggttgcca gatgcaatac aagattcctg gttaaatttg     540
   aatttcagta aacaatgaat agtttttcat tgtaccatga aatatccaga acatacttat     600
   atgtaaagta ttatttattt gaatctacaa aaaacaacaa ataattttta aatataagga     660
   ttttcctaga tattgcacgg gagaatatac aaatagcaaa attgaggcca agggccaaga     720
   gaatatccga actttaattt caggaattga atgggtttgc tagaatgtga tatttgaagc     780
   atcacataaa aatgatggga caataaattt tgccataaag tcaaatttag ctggaaatcc     840
   tggatttttt tctgttaaat ctggcaaccc tagtctgcta gccaggatcc acaagtcctt     900
   gttccactgt gccttggttt ctcctttatt tctaagtgga aaaagtatta gccaccatct     960
   tacctcacag tgatgttgtg aggacatgtg gaagcacttt aagttttttc atcataacat    1020
   aaattatttt caagtgtaac ttattaacct atttattatt tatgtattta tttaagcatc    1080
   aaatatttgt gcaagaattt ggaaaaatag aagatgaatc attgattgaa tagttataaa    1140
   gatgttatag taaatttatt ttattttaga tattaaatga tgttttatta gataaatttc    1200
   aatcagggtt tttagattaa acaaacaaac aattgggtac ccagttaaat tttcatttca    1260
   gataaacaac aaataatttt ttagtataag tacattattg tttatctgaa attttaattg    1320
   aactaacaat cctagtttga tactcccagt cttgtcattg ccagctgtgt tggtagtgct    1380
   gtgttgaatt acggaataat gagttagaac tattaaaaca gccaaaactc cacagtcaat    1440
   attagtaatt tcttgctggt tgaaacttgt ttattatgta caaatagatt cttataatat    1500
   tatttaaatg actgcatttt taaatacaag gctttatatt tttaacttta agatgttttt    1560
   atgtgctctc caaatttttt ttactgtttc tgattgtatg gaaatataaa agtaaatatg    1620
   aaacatttaa aatataattt gttgtcaaag taaaaaaaaa aaaaaa                    1666
<210> 128
   <211> 3389
   <212> DNA
   <213> Homo sapiens
<400> 128
   gtcggtgtga caactgatcg ggtgaacgat gcaccactaa ccaccatgga aacaaggaaa 60
   aataaagcaa gctcacagga tctctcttca ctggattgag agcctcagcc tgccgactga 120
   gaaaaagagt tccaggaaaa agaaggaatc ccggctgcag cctcctgcct tcctttatat 180
   tttaaaatag agagataaga ttgcgtgcat gtgtgcatat ctatagtata tattttgtac 240
   actttgttac acagacacac aaatgcacct atttataccg ggcaagaaca caaccatgtg 300
   attatctcaa ccaaggaact gaggaatcca gcacgcaagg acatcggagg tgggctagca 360
   ctgaaactgc ttttcaagca tcatgctgct attcctgcaa atactgaaga agcatgggat 420
   ttaaatattt tacttctaaa taaatgaatt actcaatctc ctatgaccat ctatacatac 480
   tccaccttca aaaagtacat caatattata tcattaagga aatagtaacc ttctcttctc 540
   caatatgcat gacatttttg gacaatgcaa ttgtggcact ggcacttgtt tcagtgaaga 600
   aaaactttgt ggttctatgg cattcatcat ttgacaaatg caagcatctt ccttatcaat 660
   cagctcctat tgaacttact agcactgact gtggaatcct taagggccca ttacatttct 720
   gaagaagaaa gctaagatga aggacatgcc actccgaatt catgtgctac ttggcctagc 780
   tatcactaca ctagtacaag ctgtagataa aaaagtggat tgtccacggt tatgtacgtg 840
   tgaaatcagg ccttggttta cacccagatc catttatatg gaagcatcta cagtggattg 900
   taatgattta ggtcttttaa ctttcccagc cagattgcca gctaacacac agattcttct 960
   cctacagact aacaatattg caaaaattga atactccaca gactttccag taaaccttac 1020
   tagcctggat ttatctcaaa acaatttatc ttcagtcacc aatattaatg taaaaaagat 1080
   gcctcagctc ctttctgtgt acctagagga aaacaaactt actgaactgc ctgaaaaatg 1140
   tctgtccgaa ctgagcaact tacaagaact ctatattaat cacaacttgc tttctacaat 1200
   ttcacctgga gcctttattg gcctacataa tcttcttcga cttcatctca attcaaatag 1260
   attgcagatg atcaacagta agtggtttga tgctcttcca aatctagaga ttctgatgat 1320
   tggggaaaat ccaattatca gaatcaaaga catgaacttt aagcctctta tcaatcttcg 1380
   cagcctggtt atagctggta taaacctcac agaaatacca gataacgcct tggttggact 1440
   ggaaaactta gaaagcatct ctttttacga taacaggctt attaaagtac cccatgttgc 1500
   tcttcaaaaa gttgtaaatc tcaaattttt ggatctaaat aaaaatccta ttaatagaat 1560
   acgaaggggt gattttagca atatgctaca cttaaaagag ttggggataa ataatatgcc 1620
   tgagctgatt tccatcgata gtcttgctgt ggataacctg ccagatttaa gaaaaataga 1680
   agctactaac aaccctagat tgtcttacat tcaccccaat gcatttttca gactccccaa 1740
   gctggaatca ctcatgctga acagcaatgc tctcagtgcc ctgtaccatg gtaccattga 1800
   gtctctgcca aacctcaagg aaatcagcat acacagtaac cccatcaggt gtgactgtgt 1860
   catccgttgg atgaacatga acaaaaccaa cattcgattc atggagccag attcactgtt 1920
   ttgcgtggac ccacctgaat tccaaggtca gaatgttcgg caagtgcatt tcagggacat 1980
   gatggaaatt tgtctccctc ttatagctcc tgagagcttt ccttctaatc taaatgtaga 2040
   agctgggagc tatgtttcct ttcactgtag agctactgca gaaccacagc ctgaaatcta 2100
   ctggataaca ccttctggtc aaaaactctt gcctaatacc ctgacagaca agttctatgt   2160
   ccattctgag ggaacactag atataaatgg cgtaactccc aaagaagggg gtttatatac   2220
   ttgtatagca actaacctag ttggcgctga cttgaagtct gttatgatca aagtggatgg   2280
   atcttttcca caagataaca atggctcttt gaatattaaa ataagagata ttcatgccaa   2340
   ttcagttttg gtgtcctgga aagcaagttc taaaattctc aaatctagtg ttaaatggac   2400
   agcctttgtc aagactgaaa attctcatgc tgcgcaaagt gctcgaatac catctgatgt   2460
   caaggtatat aatcttactc atctgaatcc atcaactgag tataaaattt gtattgatat   2520
   tcccaccatc tatcagaaaa acagaaaaaa atgtgtaaat gtcaccacca aaggtttgca   2580
   ccctgatcaa aaagagtatg aaaagaataa taccacaaca cttatggcct gtcttggagg   2640
   ccttctgggg attattggtg tgatatgtct tatcagctgc ctctctccag aaatgaactg   2700
   tgatggtgga cacagctatg tgaggaatta cttacagaaa ccaacctttg cattaggtga   2760
   gctttatcct cctctgataa atctctggga agcaggaaaa gaaaaaagta catcactgaa   2820
   agtaaaagca actgttatag gtttaccaac aaatatgtcc taaaaaccac caaggaaacc   2880
   tactccaaaa atgaacaaaa aaaaaaaaag cgaaagactg cagttgtgct aaaaacaaaa   2940
   caaaacaaac aaacaaacaa aaaagtaaaa aaagattact ttcgagagag aagtttaagc   3000
   ttcaccaatg ctgctcctga ccaatggaaa tatgtacaac ttcagcattt taagtaactg   3060
   gcttcaaggg gtactgtggc aaccaaataa aataactcca ttttctaaaa ctttcatgta   3120
   acttttatgt ctggactaca gttcaagtgg acaaaaacat ttctgtattt tttttaagta   3180
   aataagagta gttgaactga gcaatacctc ctcctgtgtt gtattacaca tattagccac   3240
   gagtttttgc agtgaccaga taaacttgaa ttgacacgtg gtgtaataaa atggacaaat   3300
   tctgtagagt agacacagtg agtatgtgga cctcttttat aaggaaaaat acattttgga   3360
   ttaaaatcaa aaaaaaaaaa aaaaaaaaa                                      3389
<210> 129
   <211> 984
   <212> DNA
   <213> Homo sapiens
<400> 129
   cccacgcgtc cgcccacgcg tccgcaggca ctgaagtact tgatactaat tggatattat 60
   gaccactttt cagcaattgt attactgtaa tggtgggatc cataaaggaa atggtaaacc 120
   tgtcttatat gggtagttgt gtgaattgca aaacttacat caaataattt ttttgcctcg 180
   tcttttgaac aagtcatgac atcattctag ttcctttttc aacttagtga gatgtctgct 240
   ctgtgttaat attataatgt gtataatcta caacagaatt gtcctccaaa ttgcaagttt 300
   tcctttttga atctaatata tcaggctcat ttttagatga cttagatgca taaggatttt 360
   atcgtagctt gatgttagct ctctgtaacc tttcaggtat attaatcaaa aaagccaaac 420
   ttaataaagc atctaattta aaatggattg atctcaaata ttttatagcc actatctttt 480
   cacaatagta aaacataaaa gagtttaagc tatgccaatt tagagagtgc ttaacaatgt 540
   gtaattactt tattttcact taatagattt ttacctgtgc cttgtttcag atctgaatag 600
   gtttcctttg acctcaaaat cactaatgca gggaaaatta ccatatcatg agagtatttt 660
   tcctctttct tttttttgcc tgatgcttag aataataaat actcccaagg cttaggagta 720
   gacatggcct gagagaaagc ctattaaatg ctattttcta atggttttaa aaaacaactt 780
   aaacaaatca tgctactcaa actgattttc ttgctaaaac agaactagtt ttagagtata 840
   ttttagacat ctttttttct caattactag ttaaacttga agttgggctc tatctggatt 900
   tgctttatag atctgcttca tttcatgcca gatttgggca aaccatgaac aacccccaac 960
   tccaggaaaa aaaaaaaaaa aaaa 984
<210> 130
   <211> 1542
   <212> DNA
   <213> Homo sapiens
<400> 130
   acaattactc tacagctcag aacaccaact gctgaggctg ccttgggaag aggatgatcc      60
   taaacaaagc tctgctgctg ggggccctcg ctctgaccac cgtgatgagc ccctgtggag     120
   gtgaagacat tgtggctgac cacgttgcct cttgtggtgt aaacttgtac cagttttacg     180
   gtccctctgg ccagtacacc catgaatttg atggagatga gcagttctac gtggacctgg     240
   agaggaagga gactgcctgg cggtggcctg agttcagcaa atttggaggt tttgacccgc     300
   agggtgcact gagaaacatg gctgtggcaa aacacaactt gaacatcatg attaaacgct     360
   acaactctac cgctgctacc aatgaggttc ctgaggtcac agtgttttcc aagtctcccg     420
   tgacactggg tcagcccaac accctcattt gtcttgtgga caacatcttt cctcctgtgg     480
   tcaacatcac atggctgagc aatgggcagt cagtcacaga aggtgtttct gagaccagct     540
   tcctctccaa gagtgatcat tccttcttca agatcagtta cctcaccttc ctcccttctg     600
   ctgatgagat ttatgactgc aaggtggagc actggggcct ggaccagcct cttctgaaac     660
   actgggagcc tgagattcca gcccctatgt cagagctcac agagactgtg gtctgtgccc     720
   tggggttgtc tgtgggcctc atgggcattg tggtgggcac tgtcttcatc atccaaggcc     780
   tgcgttcagt tggtgcttcc agacaccaag ggccattgtg aatcccatcc tggaagggaa     840
   ggtgcatcgc catctacagg agcagaagaa tggacttgct aaatgaccta gcactattct     900
   ctggcccgat ttatcatatc ccttttctcc tccaaatatt tctcctctca ccttttctct     960
   gggacttaag ctgctatatc ccctcagagc tcacaaatgc ctttacattc tttccctgac    1020
   ctcctgattt tttttttctt ttctcaaatg ttacctacaa agacatgcct ggggtaagcc    1080
   acccggctac ctaattcctc agtaacctcc atctaaaatc tccaaggaag caataaattc    1140
   cttttatgag atctatgtca aatttttcca tctttcatcc agggctgact gaaactatgg    1200
   ctaataattg gggtactctt atgtttcaat ccaatttaac ctcatttccc agatcatttt    1260
   tcatgtccag taacacagaa gccaccaagt acagtatagc ctgataatat gttgatttct    1320
   tagctgacat taatatttct tgcttccttg tgttcccacc cttggcactg ccacccaccc    1380
   ctcaattcag gcaacaatga aattaatgga taccgtctgc ccttggccca gaattgttat    1440
   agcaaaaatt ttagaaccaa aaaataagtc tgtactaatt tcaatgtggc ttttaaaagt    1500
   atgacagaga aataagttag gataaaggaa atttgaatct ca                       1542
<210> 131
   <211> 1897
   <212> DNA
   <213> Homo sapiens
<400> 131
   gcgggaagtc tgaagccggt aaacatggcc gtcaccgaca gcctcagccg ggctgcgact 60
   gtcttggcaa ctgtgttgct cttgtccttc ggcagcgtgg ccgctagtca tatcgaggat 120
   caagcagaac aattctttag aagtggccat acaaacaact gggctgttct ggtgtgtaca 180
   tcccgattct ggtttaatta tcgacatgtt gcaaataccc tttctgttta tagaagtgtc 240
   aagaggctag gtattcctga cagtcacatt gtcctaatgc ttgcagatga tatggcctgt 300
   aatcctagaa atcccaaacc agctacagtg tttagtcaca agaatatgga actaaatgtg 360
   tatggagatg atgtggaagt ggattataga agttatgagg taactgtgga gaatttttta 420
   cgggtattaa ctgggaggat cccacctagt actcctcggt caaaacgtct tctttctgat 480
   gacagaagca atattctaat ttatatgaca gggcatggtg gaaatggttt cttaaaattt 540
   caagattctg aagaaattac caacatagaa ctcgcggatg cttttgaaca aatgtggcag 600
   aaaagacgct acaatgagct actgtttatt attgatactt gccaaggagc atccatgtat 660
   gaacgatttt attctcctaa cataatggct ctagctagta gtcaagtggg agaagattca 720
   ctctcgcatc aacctgatcc tgcaattgga gtccatctta tggatagata cacattttat 780
   gtcttggaat ttttggaaga aattaaccca gctagccaaa ctaatatgaa tgaccttttt 840
   caggtatgtc ccaaaagtct gtgtgtgtct actcctggac atcgcactga tctttttcag 900
   agggatccta aaaatgtact gataactgat ttctttggaa gtgtacggaa agtggaaatt 960
   acaacagaga ctattaaatt gcaacaggat tcagaaatca tggaaagcag ctataaggaa 1020
   gaccagatgg atgagaaact aatggaacct ctgaaatatg ctgaacaact tcctgtagct 1080
   cagataatac accagaaacc gaagctgaaa gactggcatc ctcctggggg ctttattctg 1140
   ggattatggg cacttattat catggttttc ttcaaaactt atggaattaa gcatatgaag 1200
   ttcatttttt agacttgatg atgaatgaag aatgcatgga ggactgcaaa cttggataat 1260
   aatttatgtc attatatatt tttaaaaatg tgtttctctt gtatgaattg gaaataagta 1320
   taaggaaact aaatttgaat caactattaa ttttataact taaagaaaaa taattgttaa 1380
   tgcaactgct taatggcact aaatatattc cagttttgta ttttgtgtat tataaaagcg 1440
   aatgagacag agatcagaat acattgactg tttttgaaaa tagtaatttc cccttatccc 1500
   cttttcattt ggaaaagaaa caattgtgaa gacattaaat tctcactaac agaagtaact 1560
   ttggttaatt attttttgta tatcctccca atcttttgac ttatgcacat attttttccc 1620
   aatatggaga tcatatggaa tgtactattt tgtaatgtct tttttcattt tacaatgtat 1680
   tatcaacctt ttccctctca aaaatacatt gtgaatgact gcatagtatt cactttatga 1740
   atatttaatt catttcacag tcttctattg ttggaccact tacattgtac caaatgtttt 1800
   cctttggttt attctttaat gtattaatat tttactgctg gtcactcatg gaatcctgca 1860
   gctttaatta aaagcaaaga tgaaaaaaaa aaaaaaa 1897
<210> 132
   <211> 1721
   <212> DNA
   <213> Homo sapiens
<400> 132
   caccagcaca gcaaacccgc cgggatcaaa gtgtaccagt cggcagcatg gctacgaaat      60
   gtgggaattg tggacccggc tactccaccc ctctggaggc catgaaagga cccagggaag     120
   agatcgtcta cctgccctgc atttaccgaa acacaggcac tgaggcccca gattatctgg     180
   ccactgtgga tgttgacccc aagtctcccc agtattgcca ggtcatccac cggctgccca     240
   tgcccaacct gaaggacgag ctgcatcact caggatggaa cacctgcagc agctgcttcg     300
   gtgatagcac caagtcgcgc accaagctgg tgctgcccag tctcatctcc tctcgcatct     360
   atgtggtgga cgtgggctct gagccccggg ccccaaagct gcacaaggtc attgagccca     420
   aggacatcca tgccaagtgc gaactggcct ttctccacac cagccactgc ctggccagcg     480
   gggaagtgat gatcagctcc ctgggagacg tcaagggcaa tggcaaaggg ggttttgtgc     540
   tgctggatgg ggagacgttc gaggtgaagg ggacatggga gagacctggg ggtgctgcac     600
   cgttgggcta tgacttctgg taccagcctc gacacaatgt catgatcagc actgagtggg     660
   cagctcccaa tgtcttacga gatggcttca accccgctga tgtggaggct ggactgtacg     720
   ggagccactt atatgtatgg gactggcagc gccatgagat tgtgcagacc ctgtctctaa     780
   aagatgggct tattcccttg gagatccgct tcctgcacaa cccagacgct gcccaaggct     840
   ttgtgggctg cgcactcagc tccaccatcc agcgcttcta caagaacgag ggaggtacat     900
   ggtcagtgga gaaggtgatc caggtgcccc ccaagaaagt gaagggctgg ctgctgcccg     960
   aaatgccagg cctgatcacc gacatcctgc tctccctgga cgaccgcttc ctctacttca    1020
   gcaactggct gcatggggac ctgaggcagt atgacatctc tgacccacag agaccccgcc    1080
   tcacaggaca gctcttcctc ggaggcagca ttgttaaggg aggccctgtg caagtgctgg    1140
   aggacgagga actaaagtcc cagccagagc ccctagtggt caagggaaaa cgggtggctg    1200
   gaggccctca gatgatccag ctcagcctgg atgggaagcg cctctacatc accacgtcgc    1260
   tgtacagtgc ctgggacaag cagttttacc ctgatctcat cagggaaggc tctgtgatgc    1320
   tgcaggttga tgtagacaca gtaaaaggag ggctgaagtt gaaccccaac ttcctggtgg    1380
   acttcgggaa ggagcccctt ggcccagccc ttgcccatga gctccgctac cctgggggcg    1440
   attgtagctc tgacatctgg atttgaactc caccctcatc acccacactc cctattttgg    1500
   gccctcactt ccttggggac ctggcttcat tctgctctct cttggcaccc gacccttggc    1560
   agcatgtacc acacagccaa gctgagactg tggcaatgtg ttgagtcata tacatttact    1620
   gaccactgtt gcttgttgct cactgtgctg cttttccatg agctcttgga ggcaccaaga    1680
   aataaactcg taaccctgtc cttcaaaaaa aaaaaaaaaa a                        1721
<210> 133
   <211> 3592
   <212> DNA
   <213> Homo sapiens
<400> 133
   gaggaagagg ctgaggaggc gcggggggcg ggggaggctc aggagcgggc ggtgacggcg      60
   acggcggcgg cagaggaggc agcggctggg ccgggccccg tgcgtctgtc cgcgccccgt    120
   ggatgcgaat cggccgcggc ggaggcggcg gcggcggagg aggcggcggc gggaggagga    180
   gtcggtgagc cggctccggg ccggaggggc gcggaggatg aggccgctgc ctggcgctct    240
   tggcgtggcg gcggccgccg cgctgtggct gctgctgctg ctgctgcccc ggacccgggc    300
   ggacgagcac gaacacacgt atcaagataa agaggaagtt gtcttatgga tgaatactgt    360
   tgggccctac cataatcgtc aagaaacata taagtacttt tcacttccat tctgtgtggg    420
   gtcaaaaaaa agtatcagtc attaccatga aactctggga gaagcacttc aaggggttga    480
   attggaattt agtggtctgg atattaaatt taaagatgat gtgatgccag ccacttactg    540
   tgaaattgat ttagataaag aaaagagaga tgcatttgta tatgccataa aaaatcatta    600
   ctggtaccag atgtacatag atgatttacc aatatggggt attgttggtg aggctgatga    660
   aaatggagaa gattactatc tttggaccta taaaaaactt gaaataggtt ttaatggaaa    720
   tcgaattgtt gatgttaatc taactagtga aggaaaggtg aaactggttc caaatactaa    780
   aatccagatt tcatattcag taaaatggaa aaagtcagat gtgaaatttg aagatcgatt    840
   tgacaaatat cttgatccgt ccttttttca acatcggatt cattggtttt caattttcaa     900
   ctccttcatg atggtgatct tcttggtggg cttagtttca atgattttaa tgagaacatt    960
   aagaaaagat tatgctcggt acagtaaaga ggaagaaatg gatgatatgg atagagacct   1020
   aggagatgaa tatggatgga aacaggtgca tggagatgta tttagaccat caagtcaccc   1080
   actgatattt tcctctctga ttggttctgg atgtcagata tttgctgtgt ctctcatcgt   1140
   tattattgtt gcaatgatag aagatttata tactgagagg ggatcaatgc tcagtacagc   1200
   catatttgtc tatgctgcta cgtctccagt gaatggttat tttggaggaa gtctgtatgc   1260
   tagacaagga ggaaggagat ggataaagca gatgtttatt ggggcattcc ttatcccagc   1320
   tatggtgtgt ggcactgcct tcttcatcaa tttcatagcc atttattacc atgcttcaag   1380
   agccattcct tttggaacaa tggtggccgt ttgttgcatc tgtttttttg ttattcttcc    1440
   tctaaatctt gttggtacaa tacttggccg aaatctgtca ggtcagccca actttccttg   1500
   tcgtgtcaat gctgtgcctc gtcctatacc ggagaaaaaa tggttcatgg agcctgcggt   1560
   tattgtttgc ctgggtggaa ttttaccttt tggttcaatc tttattgaaa tgtatttcat   1620
   cttcacgtct ttctgggcat ataagatcta ttatgtctat ggcttcatga tgctggtgct   1680
   ggttatcctg tgcattgtga ctgtctgtgt gactattgtg tgcacatatt ttctactaaa   1740
   tgcagaagat taccggtggc aatggacaag ttttctctct gctgcatcaa ctgcaatcta   1800
   tgtttacatg tattcctttt actactattt tttcaaaaca aagatgtatg gcttatttca   1860
   aacatcattt tactttggat atatggcggt atttagcaca gccttgggga taatgtgtgg   1920
   agcgattggt tacatgggaa caagtgcctt tgtccgaaaa atctatacta atgtgaaaat   1980
   tgactagaga cccaagaaaa cctggaactt tggatcaatt tctttttcat aggggtggaa   2040
   cttgcacagc aaaaacaaac aaacgcaaga agagatttgg gctttaacac actgggtact   2100
   ttgtgggtct ctctttcgtc ggtggcttaa agtaacatct atttccattg atcctaggtt   2160
   cttcctgact gctttctcca actgttcaca gcaaatgctt ggattttatg cagtaggcat   2220
   tactacagta catggctaat cttcccaaaa actagctcat taaagatgaa atagaccagc   2280
   tctcttcagt gaagaggaca aatagtttat ttaaagcatt tgttccaata aaataaatag   2340
   agggaaactt ggatgctaaa attacatgaa taggaatctt cctggcactt agtgtttcta   2400
   tgttattgaa aaatgatgtt ccagaaagat tacttttttc ctcttatttt tactgccatt    2460
   gtcgacctat tgtgggacat ttttatatat tgaatctggg ttcttttttg actttttttt   2520
   ttcccaatcc aacagcatcc ttttttttaa aagagagaat tagaaaatat taaatcctgc   2580
   atgtaatata tctgctgtca tcttagttgg accaacttcc catttattta tcttaaaact   2640
   atacagttac atcttaattc catccaaaga agatacagtt tgaagacaga agtgtactct   2700
   ctacaatgca atttactgta cagttagaaa gcaaagtgtt aaatggagaa gatacttgtt   2760
   tttattaaac attttgagat ttagataaac tacattttaa ctgaatgtct aaagtgatta   2820
   tcttttttcc ccccaagtta gtcttaaatc ttttgggttt gaatgaaggt tttacataag    2880
   aaattattaa aaacaagggg ggtgggtaat aaatgtatat aacattaaat aatgtaacgt   2940
   aggtgtagat tcccaaatgc atttggatgt acagatcgac tacagagtac ttttttctta   3000
   tgatgattgg tgtagaaatg tgtgatttgg gtgggctttt acatcttgcc taccattgca   3060
   tgaaacattg gggtttcttc aaaatgtgtg tgtcatactt cttttgggag gggggttgtt    3120
   ttcttctgtt tattttctga gactcctaca ggagccaaat ttgtaattta gagacactta   3180
   attttgttaa tcctgtctgg gacacttaag taacatctaa agcattattg ctttagaatg   3240
   ttcaaataaa atttcctgac caaattgttt tgtggaaata gatgtgtttg caatttgaag   3300
   atatctttct gtccagaagg caaaattacc gaatgccatt tttaaaagta tgctataaac   3360
   tatgctactc tcatacaggg gacccgtatt ttaaaatctc cagacttgct tacatctaga   3420
   ttatccagca caatcataaa gtgaatgaca aaccctttga atgaaattgt ggcacaaaat    3480
   ctgttcaggt tggtgtaccg tgtaaagtgg ggatggggta aaagtggtta acgtactgtt   3540
   ggatcaacaa ataaaggtta cagttttgta agagaaaaaa aaaaaaaaaa aa           3592
<210> 134
   <211> 3917
   <212> DNA
   <213> Homo sapiens
<400> 134
   tagtcggagc gaggtggcga gtcgctgagc ccgccgcggc cccgagagcg gctgcagccg     60
   ccgccgccgg gaaggagagg gcgaggcgcg cccgagccgc cgccgccgcc gccaccgccg    120
   ccgccgccac caccgccacc ggagtcgcgg gccagccggg cagcctccgc gggccccggc    180
   cggggcgggg ggcgcgggcc acaggcccct gctccggccg tcgtttgcag accgcgggcg    240
   ccgatgtcgc ccgcgccccg ttaggatgag tctcgggtcg ggcgaggagc cgccgcagcc    300
   gccgccgccc gagccgcggg caggagcctc gggagccgcc gccgccgccg ccgccgcccg    360
   gccgggcccc gacgccgccc gcgcgccccc gggcccccga cacacatgag attcttcagg    420
   ctcactttca agtgcttcgt ggactgcttc tgactgcgcc gcccgcgccc cgcaccccgc    480
   cgtccgcccg ccgccccgtc ccccggcccg gccgcccccc ggcccccggc cggcccgcgc    540
   cctcggggcc ctccccggtg ccgccggtgc cccccgcctg accgccgccc cccgtgaggc    600
   gccgcgaccc cggcccggcc gtgcggcccg ccggggccat ggcgaagaag agcgccgaga    660
   acggcatcta tagcgtgtcc ggcgacgaga agaagggccc cctcatcgcg cccgggcccg    720
   acggggcccc ggccaagggc gacggccccg tgggcctggg gacacccggc ggccgcctgg    780
   ccgtgccgcc gcgcgagacc tggacgcgcc agatggactt catcatgtcg tgcgtgggct    840
   tcgccgtggg cttgggcaac gtgtggcgct tcccctacct gtgctacaag aacggcggag    900
   gtgtgttcct tattccctac gtcctgatcg ccctggttgg aggaatcccc attttcttct    960
   tagagatctc gctgggccag ttcatgaagg ccggcagcat caatgtctgg aacatctgtc   1020
   ccctgttcaa aggcctgggc tacgcctcca tggtgatcgt cttctactgc aacacctact   1080
   acatcatggt gctggcctgg ggcttctatt acctggtcaa gtcctttacc accacgctgc   1140
   cctgggccac atgtggccac acctggaaca ctcccgactg cgtggagatc ttccgccatg   1200
   aagactgtgc caatgccagc ctggccaacc tcacctgtga ccagcttgct gaccgccggt    1260
   cccctgtcat cgagttctgg gagaacaaag tcttgaggct gtctggggga ctggaggtgc    1320
   caggggccct caactgggag gtgacccttt gtctgctggc ctgctgggtg ctggtctact   1380
   tctgtgtctg gaagggggtc aaatccacgg gaaagatcgt gtacttcact gctacattcc   1440
   cctacgtggt cctggtcgtg ctgctggtgc gtggagtgct gctgcctggc gccctggatg   1500
   gcatcattta ctatctcaag cctgactggt caaagctggg gtcccctcag gtgtggatag   1560
   atgcggggac ccagattttc ttttcttacg ccattggcct gggggccctc acagccctgg   1620
   gcagctacaa ccgcttcaac aacaactgct acaaggacgc catcatcctg gctctcatca   1680
   acagtgggac cagcttcttt gctggcttcg tggtcttctc catcctgggc ttcatggctg   1740
   cagagcaggg cgtgcacatc tccaaggtgg cagagtcagg gccgggcctg gccttcatcg   1800
   cctacccgcg ggctgtcacg ctgatgccag tggccccact ctgggctgcc ctgttcttct   1860
   tcatgctgtt gctgcttggt ctcgacagcc agtttgtagg tgtggagggc ttcatcaccg   1920
   gcctcctcga cctcctcccg gcctcctact acttccgttt ccaaagggag atctctgtgg   1980
   ccctctgttg tgccctctgc tttgtcatcg atctctccat ggtgactgat ggcgggatgt   2040
   acgtcttcca gctgtttgac tactactcgg ccagcggcac caccctgctc tggcaggcct   2100
   tttgggagtg cgtggtggtg gcctgggtgt acggagctga ccgcttcatg gacgacattg   2160
   cctgtatgat cgggtaccga ccttgcccct ggatgaaatg gtgctggtcc ttcttcaccc   2220
   cgctggtctg catgggcatc ttcatcttca acgttgtgta ctacgagccg ctggtctaca   2280
   acaacaccta cgtgtacccg tggtggggtg aggccatggg ctgggccttc gccctgtcct   2340
   ccatgctgtg cgtgccgctg cacctcctgg gctgcctcct cagggccaag ggcaccatgg   2400
   ctgagcgctg gcagcacctg acccagccca tctggggcct ccaccacttg gagtaccgag   2460
   ctcaggacgc agatgtcagg ggcctgacca ccctgacccc agtgtccgag agcagcaagg   2520
   tcgtcgtggt ggagagtgtc atgtgacaac tcagctcaca tcaccagctc acctctggta   2580
   gccatagcag cccctgcttc agccccaccg cacccctcca gggggcctgc ctttccctga   2640
   cacttttggg gtctgcctgg gggaggaggg gagaaagcac catgagtgct cactaaaaca   2700
   actttttcca tttttaataa aacgccaaaa atatcacaac ccaccaaaaa tagatgcctc   2760
   tccccctcca gccctagccg agctggtcct aggccccgcc tagtgcccca cccccaccca   2820
   cagtgctgca ctcctcctgc ccctgccacg cccaccccct gcccacctct ccaggctctg   2880
   ctctgcagca cacccgtggg tgacccctca ccccagaagc agcagtggca gcttgggaaa   2940
   tgtgaggaag ggaaggaggg agagacggga gggaggagag agaggagaag ggaggcaggg   3000
   gaggggcagc agaaccaagg caaatatttc agctgggcta tacccctctc cccatccctg   3060
   ttatagaagc ttagagagcc agccagcaat ggaaccttct ggttcctgcg ccaatcgcca   3120
   ccagtatcaa ttgtgtgagc ttgggtgcga gtgcacgcgt gcgtgagtac ggagagtata   3180
   tatagatctc tatctcttag caaaggtgaa tgccagatgt aaatggcgcc tctgggcaaa   3240
   ggaggcttgt attttgcaca ttttataaaa acttgagaga atgagatttc tgcttgtata   3300
   tttctaaaaa gaggaaggag cccaaaccat cctctcctta ccactcccat ccctgtgagc   3360
   cctaccttac ccctctgccc ctagccaagg agtgtgaatt tatagatcta actttcatag   3420
   gcaaaacaaa agcttcgagc tgttgcgtgt gtgagtctgt tgtgtggatg tgcgtgtgtg 3480
   gtccccagcc ccagactgga ttggaaaagt gcatggtggg ggcctcgggg ctgtccccac 3540
   gctgtccctt tgccacaagt ctgtggggca agaggctgca atattccgtc ctgggtgtct 3600
   gggctgctaa cctggcctgc tcaggcttcc caccctgtgc ggggcacacc cccaggaagg 3660
   gaccctggac acggctccca cgtccaggct taaggtggat gcacttcccg cacctccagt 3720
   cttctgtgta gcagctttaa cccacgtttg tctgtcacgt ccagtcccga gacggctgag 3780
   tgaccccaag aaaggcttcc ccgacaccca gacagaggct gcagggctgg ggctgggtga 3840
   gggtggcggg cctgcgggga cattctactg tgctaaaaag ccactgcaga catagcaata 3900
   aaaacatgtc attttcc 3917
<210> 135
   <211> 5922
   <212> DNA
   <213> Homo sapiens
<400> 135
   gcggccgcgg ggaccctcgg cgtggtcctc tgaccctgca aacccgcgac ggaggaaggg 60
   gaggtcctgc ccgaggcgcc agcccgagga ggaggatgcc catttaaccc gccctcgcct 120
   gccgggcgct tgcctcggtg cccgccgccg gagcctccga gccgcgcccg tggaagtgct 180
   gcatggggca gggctgctga agcgcggagt tcggggtcgc gccgctccca ggcaggcgcg 240
   ggagcccggt gcggcagttg gcacagtttc ggcggcgcct tctgcgcggg agtggggggc 300
   gcggtgcgcc cggccggcct ccgcggtgcc ctggtgaggc gagagttatg gagccgccca 360
   gctgcattca ggatgagccg ttcccgcacc ccctggagcc cgagccgggc gtctcagctc 420
   agcccggccc cgggaagcca agcgataagc ggttccggct gtggtacgtt ggggggtcgt 480
   gcctggacca caggaccacg ctgcctatgc tgccctggct catggccgag atccgcaggc 540
   gcagccagaa gcccgaggcg ggcggctgcg gggcgccggc ggcccgagag gtgatcctgg 600
   tgctcagcgc gcccttcctg cgttgcgtcc ccgcgccggg cgctggggcc tcggggggca 660
   ctagtccgtc ggccacgcag cccaacccgg cggtattcat cttcgagcac aaggcgcagc 720
   atatctcgcg cttcatccac aacagccacg acctcaccta ctttgcctac ctgatcaagg 780
   cgcagcccga cgaccccgag tcgcagatgg cctgccacgt tttccgcgcc acagacccca 840
   gccaggttcc tgatgttatt agcagcataa ggcaattatc taaagcggcc atgaaagagg 900
   atgccaaacc cagcaaagat aatgaggacg ccttttacaa ctctcagaag ttcgaagtcc 960
   tgtactgtgg aaaggtgacc gtgacccaca agaaggcccc ctcaagcctc atcgatgact 1020
   gcatggagaa gttcagcctg cacgaacagc agcgcctgaa gatccaaggc gagcagcgcg 1080
   gtccggaccc aggagaggac ctggctgact tggaggtggt ggtgcccggg tcccccggag 1140
   actgcctgcc ggaggaggct gacggcaccg acacccacct tggcttacct gccggggcca 1200
   gccagcctgc cctgaccagc tctcgggtct gcttccctga gcggattttg gaagattctg 1260
   gctttgatga gcagcaggag tttcggtctc ggtgcagcag tgtcaccggc gtgcaacgga 1320
   gagttcacga gggcagccag aaatcccagc cgcgacggag acacgcgagc gcacccagtc 1380
   acgtccagcc ctcggactcg gagaagaaca ggaccatgct cttccaggtt gggcgatttg 1440
   agattaacct tatcagtcca gacactaaat cagttgtgct agaaaagaat tttaaagata 1500
   tctcctcttg ttctcagggt ataaagcatg tggatcactt tggctttatc tgccgggagt 1560
   ctccagagcc tggacttagc cagtatattt gttatgtatt ccagtgtgcc agcgaatctc 1620
   tggttgatga ggtaatgctg actctgaaac aggccttcag tacggcggct gccctgcaga 1680
   gtgccaagac gcagattaaa ctgtgtgagg cctgcccgat gcactctttg cataagctct 1740
   gtgaaaggat tgaaggtctc tacccaccaa gagccaagct ggtgatacag aggcatctct 1800
   catcactgac agataatgag caagctgaca tctttgaaag agttcagaaa atgaagccag 1860
   tcagtgacca ggaagaaaat gaacttgtga ttttacacct gaggcagctg tgtgaagcca 1920
   agcagaagac acacgtgcac atcggggaag gcccttctac tatttcaaat agtacaatcc 1980
   cagaaaatgc aacaagcagt ggaaggttca aacttgacat tctgaaaaat aaagctaaga 2040
   gatccttaac tagctccctg gaaaatatct tctcaagggg agctaacaga atgagaggtc 2100
   ggcttggaag tgtggacagt tttgaacggt ccaacagtct tgcttcagag aaggactact 2160
   caccagggga ttctccacca gggacaccgc cagcgtcccc accgtcctca gcttggcaaa 2220
   cgtttcccga agaggattcc gactccccgc agtttcgaag acgggcacac acgttcagcc 2280
   acccaccttc aagcacaaag agaaagctga atttgcagga tgggagggct cagggtgtgc 2340
   gttcccctct gctgaggcag agctccagtg aacagtgcag caatctttcg tcagttcgac 2400
   gcatgtacaa ggagagtaat tcttcctcca gtcttccaag tcttcacact tccttctctg 2460
   ccccttcctt cactgccccc tctttcctga aaagctttta ccagaattca ggtagactgt 2520
   ccccacagta tgaaaatgaa atcagacaag acactgcttc agaatcaagt gatggagaag 2580
   ggagaaaaag gacctcatct acctgcagca atgagtccct aagtgtggga ggaacctctg 2640
   tcactcctcg ccggatctcc tggcggcagc gcattttcct cagggttgct tctcccatga 2700
   acaaatctcc ctcagcaatg caacagcaag atggattgga caggaacgag ctgctgccac 2760
   tgtcccccct ctctccaacc atggaggagg aaccgctggt tatattcctg tctggggagg 2820
   atgacccaga aaagattgaa gaaagaaaga aatcaaaaga actgaggagc ttgtggagaa 2880
   aagctataca ccaacaaatc ttgttacttc gaatggaaaa agaaaaccag aaacttgaag 2940
   gagcaagcag agatgaactc cagtccagaa aagttaaatt agactatgaa gaagttggtg 3000
   catgtcagaa agaggtctta ataacttggg ataagaagtt gttaaactgc agagctaaaa 3060
   tcagatgtga tatggaagat attcatactc ttcttaaaga aggagttccc aaaagtcgac 3120
   gaggagaaat ttggcagttt ctggctttac agtaccgact cagacacaga ttgcctaata 3180
   aacaacagcc tcctgacata tcctataagg aacttttgaa gcagctcact gctcagcagc 3240
   atgcgattct cgtggattta ggaaggacgt ttcctactca cccttacttt tcagtacagc 3300
   ttgggccagg acagctgtca ctgtttaacc tcctgaaagc ctattctttg ctggacaaag 3360
   aagtgggata ctgtcagggg atcagctttg tggctggagt cctgcttctg cacatgagtg 3420
   aagagcaagc ctttgaaatg ctgaaattcc tcatgtatga cctcggcttc cgcaagcagt 3480
   acagacctga catgatgtcg ctgcagattc aaatgtacca gctgtccagg ctccttcatg 3540
   actatcacag agatctctac aatcaccttg aagaaaatga aatcagcccc agtctttatg 3600
   ctgccccctg gttcctcaca ttgtttgcct ctcagttttc attaggattt gtagccagag 3660
   tttttgatat tatttttctt cagggaactg aagttatatt caaggttgca ctcagcctac 3720
   tgagcagcca agagacactt ataatggaat gtgagagctt tgaaaatatt gttgagtttc 3780
   ttaaaaacac gctacctgat atgaatacct ctgaaatgga aaaaattatt acccaggttt 3840
   ttgagatgga tatttctaag cagttgcatg cctatgaggt ggaatatcat gtgctacagg 3900
   atgagcttca ggaatcttca tattcctgtg aggatagtga aactttggag aagctggaga 3960
   gggccaatag ccaactgaaa agacaaaaca tggacctcct agaaaaatta caggtagctc 4020
   atactaaaat ccaggccttg gaatcaaacc tggaaaatct tttgacgaga gagaccaaaa 4080
   tgaagtcttt aatccggacc ctggaacaag aaaaaatggc ttatcaaaag acagtggagc 4140
   aactccggaa gctgctgccc gcggatgctc tagccaattg tgacctgttg ctgagagacc 4200
   taaactgcaa ccctaacaac aaagccaaga taggaaataa gccataattg aagaggcacg 4260
   gcctcagcag aaagtgctcc ttagaatact acagagagga agagcctgca tgtcgctggc 4320
   ccaaggctgg accctgaagc tgatggaacc acctaatact ggtgctgagc tcctagtcac 4380
   agcaggtgga cctcgtgctc atcagagcat gccaatccta agccattgga catatgtaga 4440
   ctggtttttg ttgttgctat gtacatataa atatatatat aaaatgaaca tagttcatgc 4500
   tttcagataa aatgagtaga tgtatattta gattaatttt tttagtcaga acttcatgaa 4560
   atccacacca aaggaaaggt aaactgaaat ttcccttgga catatgtgaa atctttttgt 4620
   ctttatagtg aaacaaagcc agagcatctt tgtatattgc aatatacttg aaaaaaatga 4680
   atgtattttt ttctccaaag aacagcatgt ttcactcaat ggtgaaaagg tggaaacatt 4740
   tatgtaactt tatgtgtatc tgtcttgata tctactgaca ttgtctatat gaggaaaatg 4800
   attactggtc atgctcctgt gagttttttg ggaaggtagg gtcatttctc cctgcctgct 4860
   ttgtgccaac tagcatgttg catctacatg cattatgagt ctggttaggc attactttaa 4920
   acatacataa agagacagta ggacattgtg gctgagtcta cccagctcaa ggtaaaggag 4980
   aatgttgcta attttttagc aaactagacc agcattatta ctcaaactaa aaatatcaca 5040
   cctgaaaaat ttaatttagg acctaaaatg tctagattag ctttctgctt tttttatttg 5100
   aataactcat tcagttgtga atgaattcct ctttatttgg tgccacagtc accaaatgac 5160
   aaggatttgc cactttccca ccaaattgtg agtgcttgta atttaggtct ctctacctta   5220
   aattcagtat aaggaaacgt aattatgatt gattttttcc aaagatgaca agctgtgttg   5280
   aaatacattt tttcttttga ccaattgaca gaatctaata agctttaata atcttcccct   5340
   tttatgtgaa aagttttgag aactgtgaaa tgtttaggaa caaactgttg aaatccattg   5400
   gaagggaaaa aagaaagtgg taccagtgtt accagctcaa ctaaaacctg caattctgca   5460
   tttcaactct tcacttcctc agcctacaaa tagctcatta gatgacattc acgcatgctg   5520
   ggtataggca aggaaagtaa ttttcaaagt acatttgcag ttctcttttt cagagatgat   5580
   tctatgatag tgcctctgaa agttgatgca gcatttttgc ctttccaaaa agtatttatc   5640
   ctcactgctt tttgcagtac ttgtattttc acagatggat tatctggggt aattttcttc   5700
   aaagggagtt tgttatacac agtgaaaatg tattatagag tagaatagta aagctctagg   5760
   ggtttcagaa agctttgatg aacagatgac aaacatctga aaccccctcc gcactgttac   5820
   ccagtgtgta tataatgact tgttatagct cagtgtgccc ttgaatccat acagtttctt   5880
   aaaagacaat aaaatcttat taataaagtt aatgtaactt ct                       5922
<210> 136
   <211> 7261
   <212> DNA
   <213> Homo sapiens
<400> 136
   ctgctccttg caccccgctc cctgcctgga cacaggctca ctcgctgcct tcttctgggg 60
   gaaaccagct tcttgccagc cacagctgct gcctccgcca ctggccaccg cccctgtcct 120
   gggagtccct tggcccagac acccacctga cttagtggct cctctgcagg aaaggtggct 180
   gccccctgcg ttcctccatc caaccatgag ctggtgccca tcaccactga gaatgcacca 240
   aagaatgtag tggacaaggg agaaggagcc tcccggggtg gaaacacacg gaaaagcctc 300
   gaggacaacg gctccaccag ggtcaccccg agtgtccagc cccacctcca gcccatcaga 360
   aacatgagtg tgagccggac catggaggac agctgtgagc tggacctggt gtacgtcaca 420
   gagaggatca tcgctgtctc cttccccagc acagccaatg aggagaactt ccggagcaac 480
   ctccgtgagg tggcgcagat gctcaagtcc aaacatggag gcaactacct gctgttcaac 540
   ctctctgagc ggagacctga catcacgaag ctccatgcca aggtactgga atttggctgg 600
   cccgacctcc acaccccagc cctggagaag atctgcagca tctgtaaggc catggacaca 660
   tggctcaatg cagaccctca caatgtcgtt gttctacaca acaagggaaa ccgaggcagg 720
   ataggagttg tcatcgcggc ttacatgcac tacagcaaca tttctgccag tgcggaccag 780
   gctctggacc ggtttgcaat gaagcggttc tatgaggata agattgtgcc cattggccag 840
   ccatcccaaa gaaggtacgt gcattacttc agtggcctgc tctccggctc catcaaaatg 900
   aacaacaagc ccttgtttct gcaccacgtg atcatgcacg gcatccccaa ctttgagtct 960
   aaaggaggat gtcggccatt tctccgcatc taccaggcca tgcaacctgt gtacacatct 1020
   ggcatctaca acatcccagg agacagccag actagcgtct gcatcaccat cgagccagga 1080
   ctgctcttga agggagacat cttgctgaag tgctaccaca agaagttccg aagcccagcc 1140
   cgagacgtca tcttccgtgt gcagttccac acctgtgcca tccatgacct gggggttgtc 1200
   tttgggaagg aggaccttga tgatgctttc aaagatgatc gatttccaga gtatggcaaa 1260
   gtggagtttg tattttctta tgggccagag aaaattcaag gcatggagca cctggagaac 1320
   gggccgagcg tgtctgtgga ctataacacc tccgaccccc tcatccgctg ggactcctac 1380
   gacaacttca gtgggcatcg agatgacggc atggaggagg tggtgggaca cacgcagggg 1440
   ccactagatg ggagcctgta tgctaaggtg aagaagaaag actccctgca cggcagcacc 1500
   ggggctgtta atgccacacg tcctacactg tcggccaccc ccaaccacgt ggaacacacg 1560
   ctttctgtga gcagcgactc gggcaactcc acagcctcca ccaagaccga caagaccgac 1620
   gagcctgtcc ccggggcctc cagtgccact gctgccttga gtccccagga gaagcgggag 1680
   ctggaccgcc tgctgagtgg ctttggctta gagcgagaga agcaaggcgc catgtaccac 1740
   acccagcacc tcaggtcccg cccagcaggg ggctcggctg tgccctcctc tggacgccac 1800
   gttgtcccag cccaggttca tgtcaatggt ggggcgttag catctgagcg ggagacagac 1860
   atcctggacg atgaattgcc aaaccaggat ggtcacagtg cgggcagcat gggcacactc 1920
   tcttctctgg acggggtcac caacaccagt gaggggggct acccagaggc cctgtcccca 1980
   ctgaccaacg gtctggacaa gtcctacccc atggagccta tggtcaatgg aggaggctac 2040
   ccctacgagt ctgccagccg ggcggggcct gcccatgctg gccacacggc ccccatgcgg 2100
   ccctcctact ctgcacagga gggtttagct ggctaccaga gggaggggcc ccacccagcc 2160
   tggccacagc cagtgaccac ctcccactat gcccatgacc ccagcggtat gttccgctct 2220
   caatcctttt cggaagctga accccagctg cccccagctc cggtccgagg gggaagcagc 2280
   cgggaggctg tgcaaagggg actgaattcg tggcagcagc agcagcagca gcagcagcag 2340
   cctcgcccac ctccacgcca gcaggaaaga gcccacttgg agagtcttgt agccagcagg 2400
   cccagccctc agccattggc agagaccccc atccccagtc tccctgagtt cccgcgagca 2460
   gcctcccagc aggagattga acagtccatc gaaacactca atatgctgat gctggacctg 2520
   gagccagcct ccgctgctgc cccactacac aagtcccaga gtgtccccgg ggcctggcca 2580
   ggggcttctc cactctcctc ccagcccctc tctggatcct cccgtcagtc ccatccactg 2640
   acccagtcca gatctggcta tatccccagt gggcattcgt tgggaacccc tgagccagcc 2700
   ccacgggcct ctctggagtc tgtccctcct ggcaggtctt actcacctta tgactatcag 2760
   ccatgtttgg ctgggcctaa ccaggatttc cattcaaaga gcccagcctc ttcctccttg 2820
   cctgccttcc ttccgaccac ccacagccct ccagggcctc agcaaccccc agcctctctc 2880
   cctggcctca ctgctcagcc tctgctctca ccaaaggaag cgacttcaga cccctcccgg 2940
   actccagagg aggagccatt gaatttagaa gggctggtgg cccacagggt agcaggggta 3000
   caggctcggg agaagcagcc tgcagagccc ccagcccctc tgcggaggcg ggcggccagt 3060
   gatggacagt atgagaacca gtctccagaa gccacatccc ctcgtagccc tggggttcgc 3120
   tcccctgtcc agtgtgtctc cccggagctg gctcttacca tcgctctcaa tcctggaggg 3180
   cggcccaaag agccccattt gcacagctac aaggaggcct tcgaggagat ggagggaacc 3240
   tccccgagca gcccaccacc cagtggggtg cggtcccccc cgggtctggc caagacaccc 3300
   ctgtctgctc tgggcctgaa acctcacaac ccagcggaca tcctgttgca ccccacagga 3360
   gttaccagaa gacggatcca gccagaggaa gatgagggga aggtggtggt caggctgtca 3420
   gaagagcccc ggagctatgt ggagtctgtg gcacggacag cggtggctgg accccgagct 3480
   caggactctg agcccaagag ctttagtgct ccagccaccc aggcctatgg ccatgagata 3540
   cccctgagga acgggaccct gggtggctcc tttgtctccc ccagccccct ctccaccagc 3600
   agccccatcc tcagtgctga cagcacttca gtggggagtt tcccgtcggg agagagcagt 3660
   gaccagggtc cccggacgcc cacccagcct ctgttggagt ctggcttccg ctcaggcagc 3720
   ctgggacagc ccagcccgtc tgcccagaga aactaccaga gctcttctcc tctcccgact 3780
   gtgggcagta gctacagcag ccccgactac tcacttcagc atttcagctc ctctccggaa 3840
   agccaggctc gagctcagtt cagtgtggct ggcgtccaca cggtgcctgg gagccctcag 3900
   gcgcgccaca gaacagtggg caccaacact ccccctagtc ctggcttcgg ctggcgggcc 3960
   atcaatccca gcatggctgc ccccagcagt cccagtttga gccatcacca gatgatgggt 4020
   ccaccaggca ctggcttcca tggtagcact gtctccagcc cccagagcag tgcagcgacc 4080
   accccgggga gccccagcct gtgtcggcac ccagcagggg tctaccaggt ttctggcctc 4140
   cacaacaaag tggccaccac cccggggagt cccagcctgg gccggcaccc tggggctcac 4200
   caaggcaacc tggcctccgg tcttcatagc aatgcaatag ccagccctgg aagccccagc 4260
   ctgggccgtc acctcggagg gtctggatct gtggttcccg gcagcccctg cttggaccgg 4320
   catgtggcct atggtggcta ttctaccccg gaggatcgga gacccacact gtcccggcag 4380
   agcagtgcct ctggctacca ggctccttcc acgccctcct tccctgtctc ccctgcctac 4440
   taccctggcc tgagcagccc tgccacctcc ccgtcaccag actccgcagc cttccggcaa 4500
   gggagcccaa caccagcctt gccagagaag cgaaggatgt cagtgggaga ccgggcaggc 4560
   agcctcccca actatgccac catcaatggg aaggtgtctt cgcctgtcgc cagcggcatg 4620
   tccagtccca gtgggggcag caccgtctcc ttctcccaca ctctgcccga cttctccaag 4680
   tactccatgc cagacaacag cccggagacg cgggctaaag tgaagtttgt ccaggacact 4740
   tctaagtatt ggtacaagcc tgagatctcc agggagcagg ccatcgcgct cctcaaggac 4800
   caggagccgg gggccttcat catccgcgac agtcactcct tccgaggcgc gtacgggctg 4860
   gccatgaagg tgtcttcgcc acctccaacc atcatgcagc agaataaaaa aggagacatg 4920
   acccatgagc tggtcaggca ttttctgata gagactggcc ccagaggagt caagctcaag 4980
   ggctgcccca atgagccaaa cttcggatcg ctgtctgccc tggtctacca gcactccatc 5040
   atcccattgg ccctgccttg caagctggtc attccaaacc gagaccccac agatgaatcg 5100
   aaagatagct ccggccctgc caactcaact gcagacctgc tgaaacaagg ggcagcctgc 5160
   aatgtgctct tcgtcaactc tgtggacatg gagtcactca ctgggccaca ggccatctct 5220
   aaagccacat ctgagacgtt ggctgcagac cccacgccag ctgccaccat cgttcacttc 5280
   aaagtctctg cccagggaat cactctgact gacaaccaga gaaagctctt tttcagacgc 5340
   cactaccctc tcaacactgt caccttctgt gacctggatc cacaggaaag aaagtggatg 5400
   aaaacagagg gtggtgcccc tgctaagctc ttcggcttcg tggcccggaa gcagggcagc 5460
   accacggaca acgcctgcca cctctttgct gagcttgacc ccaaccagcc ggcctctgcc 5520
   atcgtcaact tcgtctccaa ggtcatgctg aatgccggcc aaaagagatg aaccctgccc 5580
   cttgcccagg gccagtgcca tggggaaggg gcttgtgggg aggggaccca tgaatcctga 5640
   ccactcttga acccagaagg aggactttgg gccaatttcg gaggagagaa gaaagtgcaa 5700
   cgtggggaga gggaagtgaa ttgcagaggg gagggggaaa agagagagag agagagagag 5760
   agagagagag agaaagatgg aggagaagaa cttggattcc cctgggtaga tggaaactgc 5820
   aaaaacccaa agcctccaaa actaaccagg tccacctaac accccctccc tcccctaaga 5880
   agatggatgt cctcaaaaga gaaggaacaa acctccttgg gaatccacat tttttggggg 5940
   aatggaaaag ctctgtctcc ctaactcaac tgctttgcaa ggggaaatca agctgggaga 6000
   atctttttct ggccacctgt ggggtaggtt gtcaaaccaa acagagccac cgtgggacat 6060
   caagtggaag aacttgtttg cttgaaagta tctcagaccc aaggcacctc aggtctcttt 6120
   gctgtgcctc cactatattg tcgtgtgggt gtgtgtctgc acccacatcc tcacacattg 6180
   atctagatct gcctttatcc actcgaatta taaacagctc ggcttgtcct tgtcccatgt 6240
   gtttgtagac acacatgcat actgtccaaa gattagggtt ggtggtggca gtgcagcagg 6300
   ggagggacaa acaaccaagc tatgggtgac agaggctctc tcctggtgcc tgcacctgca 6360
   ctctagtgac cctgggtgcc gccagaccct tctcttctac aaagacccca gcaggagtgg 6420
   gagggtctgc aatggcatcg ccctgtcctg ccttggccag aagcctggag ctttggtttg 6480
   aggaggtaga gatatgtgta tccataggaa gagatctgtc agaacaggca gctgttgagc 6540
   tcggggtgtc ttccccaagg catgtggctc agcagcaaga aaggcaagtt gctcctgctg 6600
   gggccctgga ctctgcctta gctcccacct ctcagccttg ttattgggtt tcatgcccct 6660
   ggaccagcct tatctcagac ctgcttacct gcatgatgcc tttttggggg ctggggattg 6720
   agtcttgctg ctctgcccag ccctgttcta ttctgcaggg tccctgtgtt ggaattctcc 6780
   ctggggaacc tactttctgc tcagtgaggc tccggccaga aacctggagt ccttatcctc 6840
   ccctctgtaa gtgttttagg gtctggcttt tgcaggcacc ctctgacctc agcagagctc 6900
   ctgggcctgc tgcctgcaca ccacatcgcc tacctacaat gccaaagcct cactgtcacc 6960
   ctttctgcct tggtttccct agctgagcca cgctgcccat gcagcagagg gcagaaggct 7020
   tgcacttggg ccaaagggcc taaggtccac tggacagttg ggaaaacacc tgaccaccat 7080
   ttaaggactc taagccagaa tggaaaattc accaggactc cattcttaag cctatgcgag 7140
   tcccctagag agaggcattg tactgatata taaatattat ataatatata catgagacat 7200
   actgacagaa tctgtaagct aataaaatgt aagaaaaggt taaaaaaaga aaaaaaaaaa 7260
   a 7261
<210> 137
   <211> 2357
   <212> DNA
   <213> Homo sapiens
<400> 137
   attggggttt cattgttccc gctgggccgg gcggtttagt gtaattgccg ccggaggagg 60
   aggcggagta acctctggtc agccgagaaa ccccactatc ctgtagccat aaccgcttaa 120
   acgatttggg aggtagtgaa gggcagggag ctggacctgg aggcgccgcc gcgacagcag 180
   cagccatgga ggacgagatg cccaagactc tatacgtcgg taacctttcc agagatgtga 240
   cagaagctct aattctgcaa ctctttagcc agattggacc ttgtaaaaac tgcaaaatga 300
   ttatggatac agctggaaat gatccctatt gttttgtgga gtttcatgag catcgtcatg 360
   cagctgcagc attagctgct atgaatggac ggaagataat gggtaaggaa gtcaaagtga 420
   attgggcaac aacccctagc agtcaaaaga aagatacaag caatcatttc catgtctttg 480
   ttggtgatct cagcccacaa attacaactg aagatataaa agctgctttt gcaccatttg 540
   gaagaatatc agatgcccga gtggtaaaag acatggcaac aggaaagtct aagggatatg 600
   gctttgtctc ctttttcaac aaatgggatg ctgaaaacgc cattcaacag atgggtggcc 660
   agtggcttgg tggaagacaa atcagaacta actgggcaac ccgaaagcct cccgctccaa 720
   agagtacata tgagtcaaat accaaacagc tatcatatga tgaggttgta aatcagtcta 780
   gtccaagcaa ctgtactgta tactgtggag gtgttacttc tgggctaaca gaacaactaa 840
   tgcgtcagac tttttcacca tttggacaaa taatggaaat tcgagtcttt ccagataaag 900
   gatattcatt tgttcggttc aattcccatg aaagtgcagc acatgcaatt gtttctgtta 960
   atggtactac cattgaaggt catgttgtga aatgctattg gggcaaagaa actcttgata 1020
   tgataaatcc cgtgcaacag cagaatcaaa ttggatatcc ccaaccttat ggccagtggg 1080
   gccagtggta tggaaatgca caacaaattg gccagtatat gcctaatggt tggcaagttc 1140
   ctgcatatgg aatgtatggc caggcatgga accagcaagg atttaatcag acacagtctt 1200
   ctgcaccatg gatgggacca aattatggag tgcaaccgcc tcaagggcaa aatggcagca 1260
   tgttgcccaa tcagccttct gggtatcgag tggcagggta tgaaacccag tgaataagga 1320
   ctccagaatc taaagccagt ggcttgaggc tacagggagt gtagtaaagc cgttgtttac 1380
   ttaaagattt atcaaatcag tcagtgcaaa tgtcagatac aatgtattca tttaaaagat 1440
   tcatttttaa tcatgaaatt acttatcatc cacattgttt taaaaagaaa caagatgctg 1500
   gatgtctgcc aatttttgcc ttcattacct tttttgataa agtttctcag atccttgttt 1560
   caaacacaaa tgcagggatt gctgccactt tttaactatt aagaggcaga aaattgcaca 1620
   atattgaact tttttccact gaagtagtgt gcagttctag tttgcattcc tgatatgatt 1680
   taaaacatgt aatataaaga tgttaaaaaa aaaaaccaaa actgtgcaga gtctagaagt 1740
   tgtttgtcat cttcagcttg tgcacaattc tgttttaggt taaaaaaagg cattgtttga 1800
   gctgtcccat ctccactgtt atccctttgg gttttttaat ataaattatt agtttacatc 1860
   atttttgtat ctacatcttt tttcacaaat ttgtcttgcc ttattaaagt tctgtaaaat 1920
   atacttaaat ggaaaaaatg atgttcattt agattgaaaa cttttctcag atggattgat 1980
   aattgcattc atcttgtgtt ttatatgaga aggtgcctca agaatttcct gttggatttg 2040
   tttaaaagga tttttatctt tcgtgataaa ctttgctgtg taccaggaac tataaaaaca 2100
   aaaacttgtt actaaagaaa atatctgaaa tgtgataagt tcttatgcca tgttaatttc 2160
   atgtgtcaac ttcaacattt acatgtatta tttcattatg taaaatgttt tagcaattta 2220
   atattttgca cagttagcaa actttgtatg tcatttcctt caaggcatca tgcagagttg 2280
   acatgagatt tataaggttt taagttgttt gcatgtgaaa atcaaataca tactttggta 2340
   gtctttgaaa aaaaaaa 2357
<210> 138
   <211> 461
   <212> DNA
   <213> Homo sapiens
<400> 138
   aaaaaataaa aatcatttat ttaaacaaaa atatacacac agtgtacaat gaatatggtt 60
   tacacagaac aataaaacaa aaatgatatc tcttaaatgc caacataaaa tgatctattt 120
   atgcctagaa aaaatttcta ggaatacaca ttattgattc atctaaaata catatataac 180
   ataaaaagaa aacaaatgcc aacctttcag atgagtacca caaaaaccta aaagttaaat 240
   gttagattaa ataaaattat aaaatatcca cacgagaagt atgtgtatac aaagtattgt 300
   atgtatgcag tgaaatgaga gtgcccatac agttatatgc aaacttttga aagtttcata 360
   gttgtttaac aaaatatttt taatgttgat agtgacataa aaacatcaca agaattatct 420
   agataatata gcaatattgc caaaatttga gtcaatgtta c 461
<210> 139
   <211> 2594
   <212> DNA
   <213> Homo sapiens
<400> 139
   gggagacggt gcgggcggcc gggagcgcag ccctccggga ggcgggtcat ggcctcggcg 60
   ccgcccgggg agcggctgcg cgaggatgcg cggtgcccgg tgtgcctgga tttcctgcag 120
   gagccggtca gcgtggactg cggccacagc ttctgcctca ggtgcatctc cgagttctgc 180
   gagaagtcgg acggcgcgca gggcggcgtc tacgcctgtc cgcagtgccg gggccccttc 240
   cggccctcgg gctttcgccc caaccggcag ctggcgggcc tggtggagag cgtgcggcgg 300
   ctggggttgg gcgcggggcc cggggcgcgg cgatgcgcgc ggcacggcga ggacctgagc 360
   cgcttctgcg aggaggacga ggcggcgctg tgctgggtgt gcgacgccgg ccccgagcac 420
   aggacgcacc gcacggcgcc gctgcaggag gccgccggca gctaccaggt aaagctccag 480
   atggctctgg aacttatgag gaaagagttg gaggacgcct tgactcagga ggccaacgtg 540
   gggaaaaaga ctgtcatttg gaaggagaaa gtggaaatgc agaggcagcg cttcagattg 600
   gagtttgaga agcatcgtgg ctttctggcc caggaggagc aacggcagct gaggcggctg 660
   gaggcggagg agcgagcgac gctgcagaga ctgcgggaga gcaagagccg gctggtccag 720
   cagagcaagg ccctgaagga gctggcggat gagctgcagg agaggtgcca gcgcccggcc 780
   ctgggtctgc tggagggtgt gagaggagtc ctgagcagaa gtaaggctgt cacaaggctg 840
   gaagcagaga acatccccat ggaactgaag acagcatgct gcatccctgg gaggagggag 900
   ctcttaagga agttccaagt ggatgtaaag ctggatcccg ccacggcgca cccgagtctg 960
   ctcttgaccg ccgacctgcg cagtgtgcag gatggagaac catggaggga tgtccccaac 1020
   aaccctgagc gatttgacac atggccctgc atcctgggtt tgcagagctt ctcatcaggg 1080
   aggcattact gggaggttct ggtgggagaa ggagcagagt ggggtttagg ggtctgtcaa 1140
   gacacactgc caagaaaggg ggaaaccacg ccatctcctg agaatggggt ctgggccctg 1200
   tggctgctga aagggaatga gtacatggtc cttgcctccc catcagtgcc tcttctccaa 1260
   ctggaaagtc ctcgctgcat tgggattttc ttggactatg aagccggtga aatttcattc 1320
   tacaatgtca cagatggatc ttatatctac acattcaacc aactcttctc tggtcttctt 1380
   cggccttact ttttcatctg tgatgcaact cctcttatct tgccacccac aacaatagca 1440
   gggtcaggaa attgggcatc cagggatcat ttagatcctg cttctgatgt aagagatgat 1500
   catctctaaa attctgttcc caagatgcag tcctagcgta gcgaacgttc ctggagtggg 1560
   gtgaaggata tcaatatact aagttttaac agatacccca tttaggtcag cacttgattc 1620
   gttgttgctg tgaaatatgt ccatgggaca aaagagggaa tatgaaatat ttgcatatgg 1680
   gaagattata gagcataata attttgtaaa tggagcaatc tcaacctcta tttctagatc 1740
   acattttctt gatgtcttcc ttcaaattaa tgaccttgga ttacataagg atttctatgc 1800
   attcattata atttgttatt cctttcaata tccttgtatt tcaaatcttc catataagaa 1860
   ttagacatgg caattcttaa attgattcag aatggtctga tactattcca gtatcacctc 1920
   cttaattctg tttctcctcg ttttcctgat tttccttctc attctctcct tccccgctct 1980
   gtctctctct ccctgtcact ctctctctct cgttccttat tttttgtttc ttacctctta 2040
   ctgtttaacc tgttgcttcc ttctggatta atacatttag agccattcct ttatatggtc   2100
   acatttccta tgactttact caattacttt taaaatcctt tctattctga gactaatttt   2160
   taagaattac aaagctcatt cttctgaatc taatatcact aactcctaga ctttttccgt   2220
   tttctttgga tacactttaa gtaggaattt atcagaattt tcattcaact cgttctttaa   2280
   tgcagatatt tactagttat aagaccttaa ggctgggtgc agtggctcac gcctgtaatc   2340
   ccagcacttt gggaggctga ggcgggtgga tcacaagctc aggagttcaa gaccagcctg   2400
   gccaacatgg tgaaaccctg tctctactaa aaaaaaaaaa aaaatagaaa aattagctgg   2460
   gcatggtggc aggagcctgt aatcccagct attctggagg tggagacagg agaattgctt   2520
   gaaccctgga ggcggaggtt gcagtgagcc aatatctcac cactgtactc cagcccagtg   2580
   cgagactcca tctc                                                     2594

## Description

La présente invention concerne le domaine de la cancérologie. Plus particulièrement, l'invention concerne un procédé pour le diagnostic d'un cancer du sein.
Chez la femme, le cancer du sein est la première cause de mortalité par cancer dans les pays industrialisés. L'âge est le facteur de risque le plus important. Ainsi, le risque augmente de 0,5% par année d'âge dans les pays occidentaux. D'autres facteurs de risques sont connus tels que le nombre des grossesses et l'âge de la première grossesse, l'allaitement, l'âge de la puberté et de la ménopause, les traitements oestrogéniques après la survenue de la ménopause, le stress et la nutrition.
Le diagnostic du cancer du sein est généralement effectué par mammographie. Toutefois, il est estimé que la taille minimale d'une tumeur repérable par mammographie est de 1 cm, ce qui présente un passé évolutif de 8 ans en moyenne lors du diagnostic. De plus, la détection precoce d'une tumeur est d'autant plus importante parce que les petites tumeurs sont beaucoup moins malignes que ce que l'on peut extrapoler de leurs tailles : l'aggressivité des grandes tumeurs ne vient pas seulemennt de leur taille, mais aussi de leur « aggressivité inherente », qui augmente avec l'age d'une tumeur (Bucchi et al., Br J Cancer 2005, p. 156-161 ; Norden T, Eu J Cancer 1997, p. 624-628). L'analyse de l'expression d'un panel de gènes cibles est également relevante dans la lutte contre le cancer du sein, et on peut citer notamment l'analyse d'un panel de 176 gènes qui est exprimé différentiellement entre des patientes exprimant le récepteur ER et des patients n'exprimant pas le récepteur ER (Bertucci et al, Human Molecular Genetics, 2000 ; 9 : 2981-2991). On peut citer également l'analyse d'un panel de 37 gènes qui permet de diagnostiquer un cancer du sein d'une façon précoce (Sharma et al, , Breast cancer Research 2005, 7 :R634-R644). Toutefois, les patientes avaient toutes des soupçons d'une maladie mammaire (« suspect initial mammogram « ). Ce panel de gènes pourrait être maladapté pour un test de routine de diagnostic du cancer du sein préalablement à toute mammographie.
WO-A-02059271 décrit l'analyse d'un panel de plusieurs gènes permettant le diagnostic d'un cancer du sein.
Il est donc très utile de disposer d'un outil permettant de diagnostiquer d'une façon très précoce un cancer du sein.
La présente invention est définie par les revendications 1-6 ci-dessous. Elle propose un nouveau procédé pour le diagnostic du cancer du sein, qui permet de discriminer les patients atteints d'un cancer du sein d'une façon très précoce, mais également lors d'un stade avancé. L'analyse de l'expression de ces 8 gènes cibles peut être réalisée directement à partir d'un échantillon sanguin, ce qui permet d'éviter des interventions graves ou nuisante, et les incertitudes liées au prélèvement pendant ces interventions. Le sang péripherique étant le compartiment intérieur le plus accessible en clinique, l'utilisation de cette source permet notamment un diagnostic en routine, moins contraignant qu'une mammographie.
Avant d'aller plus avant dans l'exposé de l'invention, il convient de donner les définitions suivantes, qui s'appliquent pour toutes les variantes de l'invention.
Au sens de la présente invention, on entend par échantillon biologique, tout échantillon prélevé chez un patient, et susceptible de contenir un matériel biologique tel que défini ci après. Cet échantillon biologique peut être notamment un échantillon de sang, de sérum, de salive, tissu, de tumeur, de moelle osseuse, de cellules circulantes du patient. On dispose de cet échantillon biologique par tout type de prélèvement connu de l'homme du métier. Au sens de la présente invention, on entend par matériel biologique, tout matériel permettant de détecter l'expression d'un gène cible. Le matériel biologique peut comprendre notamment des protéines, ou des acides nucléiques tels que notamment les acides desoxyribonucléiques (ADN) ou les acides ribonucléiques (ARN). L'acide nucléique peut être notamment un ARN (acide ribonucléique). Selon un mode préféré de réalisation de l'invention, le matériel biologique comprend des acides nucléiques, préférentiellement, des ARN, et encore plus préférentiellement des ARN totaux. Les ARN totaux comprennent les ARN de transfert, les ARN messagers (ARNm), tel que les ARNm transcrits du gène cible, mais également transcrits de tout autre gène et les ARN ribosomaux. Ce matériel biologique comprend du matériel spécifique d'un gène cible, tel que notamment les ARNm transcrits du gène cible ou les protéines issues de ces ARNm mais peut comprendre également du matériel non spécifique d'un gène cible, tel que notamment les ARNm transcrits d'un gène autre que le gène cible, les ARNt, les ARNr issus d'autres gènes que le gène cible.
Lors de l'étape a) du procédé selon l'invention, on extrait le matériel biologique de l'échantillon biologique par tous les protocoles d'extraction et de purification d'acides nucléiques bien connus de l'homme du métier. A titre indicatif, l'extraction d'acides nucléiques peut être réalisée par :
- une étape de lyse des cellules présentes dans l'échantillon biologique, afin de libérer les acides nucléiques contenus dans les cellules du patient. A titre d'exemple, on peut utiliser les méthodes de lyse telles que décrites dans les demandes de brevet WO 00/05338 (lyse mixte magnétique et mécanique), WO 99/53304 (lyse électrique) et WO 99/15321 (lyse mécanique). L'homme du métier pourra utiliser d'autres méthodes de lyse bien connues, telles que les chocs thermiques ou osmotiques ou les lyses chimiques par des agents chaotropiques tels que les sels de guanidium (US 5,234,809).
- une étape de purification, permettant la séparation entre les acides nucléiques et les autres constituants cellulaires relargués dans l'étape de lyse. Cette étape permet généralement de concentrer les acides nucléiques, et peut être adaptée à la purification d'ADN ou d'ARN. A titre d'exemple, on peut utiliser des particules magnétiques éventuellement revêtues d'oligonucléotides, par adsorption ou covalence (voir à ce sujet les brevets US 4,672,040 et US 5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet: WO-A-97/45202 et WO-A-99/35500. Un autre exemple intéressant de méthode de purification des acides nucléiques est l'utilisation de silice soit sous forme de colonne, soit sous forme de particules inertes (Boom R. et al., J. Clin. Microbiol., 1990, n°28(3), p. 495-503) ou magnétiques (Merck: MagPrep^{®} Silica, Promega: MagneSil^{™} Paramagnetic particles). D'autres méthodes très répandues reposent sur des résines échangeuses d'ions en colonne ou en format particulaire paramagnétique (Whatman: DEAE-Magarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-Bind^{™}).
   Lorsque l'on souhaite extraire spécifiquement l'ADN d'un échantillon biologique, on peut notamment réaliser une extraction par du phénol, du chloroforme et de l'alcool pour éliminer les protéines et précipiter l'ADN avec de l'éthanol 100%.
   L'ADN peut alors être culoté par centrifugation, lavé et remis en solution.
   Lorsque l'on souhaite extraire spécifiquement les ARN d'un échantillon biologique, on peut notamment réaliser une extraction par du phénol, du chloroforme et de l'alcool pour éliminer les protéines et précipiter les ARN avec de l'éthanol 100%.

Les ARN peuvent alors être culotés par centrifugation, lavés et remis en solution. Lors de l'étape b, et au sens de la présente invention, on entend par réactif spécifique, un réactif qui, lorsqu'il est mis en contact avec du matériel biologique tel que défini précédemment, se lie avec le matériel spécifique dudit gène cible. A titre indicatif, lorsque le réactif spécifique et le matériel biologique sont d'origine nucléique, la mise en contact du réactif spécifique et du matériel biologique permet l'hybridation du réactif spécifique avec le matériel spécifique du gène cible. Par hybridation, on entend le processus au cours duquel, dans des conditions appropriées, deux fragments nucléotidiques se lient avec des liaisons hydrogènes stables et spécifiques pour former un complexe double brin. Ces liaisons hydrogènes se forment entre les bases complémentaires Adénine (A) et thymine (T) (ou uracile (U)) (on parle de liaison A-T) ou entre les bases complémentaires Guanine (G) et cytosine (C) (on parle de liaison G-C). L'hybridation de deux fragments nucléotidiques peut être totale (on parle alors de fragments nucléotidiques ou de séquences complémentaires), c'est à dire que le complexe double brin obtenu lors de cette hybridation comprend uniquement des liaisons A-T et des liaisons C-G. Cette hybridation peut être partielle (on parle alors de fragments nucléotidiques ou de séquences suffisamment complémentaires), c'est à dire que le complexe double brin obtenu comprend des liaisons A-T et des liaisons C-G permettant de former le complexe double brin, mais également des bases non liées à une base complémentaire. L'hybridation entre deux fragments nucléotidiques dépend des conditions opératoires qui sont utilisées, et notamment de la stringence. La stringence est définie notamment en fonction de la composition en bases des deux fragments nucléotidiques, ainsi que par le degré de mésappariement entre deux fragments nucléotidiques. La stringence peut également être fonction des paramètres de la réaction, tels que la concentration et le type d'espèces ioniques présentes dans la solution d'hybridation, la nature et la concentration d'agents dénaturants et/ou la température d'hybridation. Toutes ces données sont bien connues et les conditions appropriées peuvent être déterminées par l'homme du métier. En général, selon la longueur des fragments nucléotidiques que l'on souhaite hybrider, la température d'hybridation est comprise entre environ 20 et 70°C, en particulier entre 35 et 65°C dans une solution saline à une concentration d'environ 0,5 à 1 M. Une séquence, ou fragment nucléotidique, ou oligonucléotide, ou polynucléotide, est un enchaînement de motifs nucléotidiques assemblés entre eux par des liaisons ester phosphoriques, caractérisé par la séquence informationnelle des acides nucléiques naturels, susceptibles de s'hybrider à un fragment nucléotidique, l'enchaînement pouvant contenir des monomères de structures différentes et être obtenu à partir d'une molécule d'acide nucléique naturelle et/ou par recombinaison génétique et/ou par synthèse chimique. Un motif est dérivé d'un monomère qui peut être un nucléotide naturel d'acide nucléique dont les éléments constitutifs sont un sucre, un groupement phosphate et une base azotée ; dans l'ADN le sucre est le désoxy - 2 - ribose, dans l'ARN le sucre est le ribose ; selon qu'il s'agisse de l'ADN ou l'ARN, la base azotée est choisie parmi l'adénine, la guanine, l'uracile, la cytosine, la thymine ; ou bien le monomère est un nucléotide modifié dans l'un au moins des trois éléments constitutifs ; à titre d'exemple, la modification peut intervenir soit au niveau des bases, avec des bases modifiées telles que l'inosine, la méthyl - 5désoxycytidine, la désoxyuridine, la diméthylamino-5 désoxyuridine, la diamino - 2,6 - purine, la bromo - 5 désoxyuridine ou toute autre base modifiée capable d'hybridation, soit au niveau du sucre, par exemple le remplacement d'au moins un désoxyribose par un polyamide (P.E. Nielsen et al, Science, 254, 1497-1500 (1991), soit encore au niveau du groupement phosphate, par exemple son remplacement par des esters notamment choisis parmi les diphosphates, alkyl- et aryl-phosphonates et phosphorothioates.
Au sens de la présente invention, le réactif spécifique peut être une amorce d'amplification. Au sens de la présente invention, on entend par amorce d'amplification, un fragment nucléotidique comprenant de 5 à 100 motifs nucléiques, préférentiellement de 15 à 30 motifs nucléiques permettant l'initiation d'une polymérisation enzymatique, telle que notamment une réaction d'amplification enzymatique. Par réaction d'amplification enzymatique, on entend un processus générant de multiples copies d'un fragment nucléotidique par l'action d'au moins une enzyme. De telles réactions d'amplification sont bien connues de l'homme du métier et on peut citer notamment les techniques suivantes :
- PCR (Polymerase Chain Reaction), telle que décrite dans les brevets US 4,683,195, US 4,683,202 et US 4,800,159,
- LCR (Ligase Chain Reaction), exposée par exemple dans la demande de brevet EP 0 201 184,
- RCR (Repair Chain Reaction), décrite dans la demande de brevet WO 90/01069,
- 3SR (Self Sustained Sequence Replication) avec la demande de brevet WO 90/06995,
- NASBA (Nucleic Acid Sequence-Based Amplification) avec la demande de brevet WO 91/02818, et
- TMA (Transcription Mediated Amplification) avec le brevet US 5,399,491.
Lorsque l'amplification enzymatique est une PCR, le réactif spécifique comprend au moins 2 amorces d'amplification, spécifiques d'un gène cible, permettant l'amplification du matériel spécifique du gène cible. Le matériel spécifique du gène cible comprend alors préférentiellement un ADN complémentaire obtenu par transcription inverse d'ARN messager issu du gène cible (on parle alors d'ADNc spécifique du gène cible) ou un ARN complémentaire obtenu par transcription des ADNc spécifiques d'un gène cible (on parle alors d'ARNc spécifique du gène cible). Lorsque l'amplification enzymatique est une PCR réalisée après une réaction de transcription inverse, on parle de RT-PCR.
Le réactif spécifique peut être également une sonde d'hybridation. Par sonde d'hybridation, on entend un fragment nucléotidique comprenant au moins 5 motifs nucléotiques, préférentiellement de 5 à 100 motifs nucléiques, encore plus préférentiellement de 10 à 35 motifs nucléiques, possédant une spécificité d'hybridation dans des conditions déterminées pour former un complexe d'hybridation avec le matériel spécifique d'un gène cible. Dans la présente invention, le matériel spécifique du gène cible peut être une séquence nucléotidique comprise dans un ARN messager issu du gène cible (on parle alors d'ARNm spécifique du gène cible), une séquence nucléotidique comprise dans un ADN complémentaire obtenu par transcription inverse dudit ARN messager (on parle alors d'ADNc spécifique du gène cible), ou encore une séquence nucléotidique comprise dans un ARN complémentaire obtenu par transcription dudit ADNc tel que décrit précédemment (on parlera alors d'ARNc spécifique du gène cible). La sonde d'hybridation peut comprendre un marqueur permettant sa détection. Par détection on entend soit une détection directe par une méthode physique, soit une détection indirecte par une méthode de détection à l'aide d'un marqueur. De nombreuses méthodes de détection existent pour la détection des acides nucléiques. [Voir par exemple Kricka et al., Clinical Chemistry, 1999, n° 45(4), p.453-458 ou Keller G.H. et al., DNA Probes, 2nd Ed., Stockton Press, 1993, sections 5 et 6, p.173-249]. Par marqueur, on entend un traceur capable d'engendrer un signal que l'on peut détecter. Une liste non limitative de ces traceurs comprend les enzymes qui produisent un signal détectable par exemple par colorimétrie, fluorescence ou luminescence, comme la peroxydase de raifort, la phosphatase alcaline, la beta galactosidase, la glucose-6-phosphate déshydrogénase; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I.
Au sens de la présente invention, la sonde d'hybridation peut être une sonde dite de détection. Dans ce cas, la sonde dite de détection est marquée au moyen d'un marqueur tel que défini précédemment. La sonde de détection peut être notamment une sonde de détection « molecular beacons » telle que décrite par Tyagi & Kramer (Nature Biotechnol., 1996, 14:303-308). Ces "molecular beacons" deviennent fluorescentes lors de l'hybridation. Elles possèdent une structure de type tige-boucle et contiennent un fluorophore et un groupe "quencher". La fixation de la séquence de boucle spécifique avec sa séquence complémentaire d'acide nucléique cible provoque un déroulement de la tige et l'émission d'un signal fluorescent lors de l'excitation à la longueur d'onde qui convient. Pour la détection de la réaction d'hybridation, on peut utiliser des séquences cibles marquées, directement (notamment par l'incorporation d'un marqueur au sein de la séquence cible) ou indirectement (notamment par l'utilisation d'une sonde de détection telle que définie précédemment) la séquence cible. On peut notamment réaliser avant l'étape d'hybridation une étape de marquage et/ou de clivage de la séquence cible, par exemple en utilisant un désoxyribonucléotide triphosphate marqué lors de la réaction d'amplification enzymatique. Le clivage peut être réalisé notamment par l'action de l'imidazole et de chlorure de manganèse. La séquence cible peut aussi être marqué après l'étape d'amplification, par exemple en hybridant une sonde de détection selon la technique d'hybridation sandwich décrite dans le document WO 91/19812. Un autre mode particulier préférentiel de marquage d'acides nucléiques est décrit dans la demande FR2 780 059.

La sonde d'hybridation peut être également une sonde dite de capture. Dans ce cas, la sonde dite de capture est immobilisée ou immobilisable sur un support solide par tout moyen approprié, c'est-à-dire directement ou indirectement, par exemple par covalence ou adsorption. Comme support solide, on peut utiliser des matériaux de synthèse ou des matériaux naturels, éventuellement modifiés chimiquement, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels etc. Le support solide peut être sous la forme d'une plaque de microtitration, d'une membrane comme décrit dans la demande WO-A-94/12670, d'une particule. On peut également immobiliser sur le support plusieurs sondes de capture différentes, chacune étant spécifique d'un gène cible. En particulier, on peut utiliser comme support une biopuce sur laquelle peuvent être immobilisées un grand nombre de sondes. Par biopuce, on entend un support solide de dimension réduite où est fixée une multitude de sondes de capture à des positions prédéterminées. Le concept de biopuce, ou puce à ADN, date du début des années 90. Il repose sur une technologie pluridisciplinaire intégrant la micro-électronique, la chimie des acides nucléiques, l'analyse d'images et l'informatique. Le principe de fonctionnement repose sur un fondement de la biologie moléculaire: le phénomène d'hybridation, c'est-à-dire l'appariement par complémentarité des bases de deux séquences d'ADN et/ou d'ARN. La méthode des biopuces repose sur l'emploi de sondes de capture fixées sur un support solide sur lesquelles on fait agir un échantillon de fragments nucléotidiques cibles marqués directement ou indirectement avec des fluorochromes. Les sondes de capture sont positionnées de manière spécifique sur le support ou puce et chaque hybridation donne une information particulière, en relation avec le fragment nucléotidique cible. Les informations obtenues sont cumulatives, et permettent par exemple de quantifier le niveau d'expression d'un gène ou de plusieurs gènes cibles. Pour analyser l'expression d'un gène cible, on peut alors réaliser une biopuce portant de très nombreuses sondes qui correspondent à tout ou partie du gène cible, qui est transcrit en ARNm. On hybride alors par exemple les ADNc ou les ARNc spécifiques d'un gène cible que l'on souhaite analyser sur des sondes de capture spécifique. Après hybridation, le support ou puce est lavé(e), et les complexes ADNc ou ARNc marquées / sondes de capture sont révélés par un ligand de forte affinité lié par exemple à un marqueur de type fluorochrome. La fluorescence est lue par exemple par un scanner et l'analyse de la fluorescence est traitée par informatique. On peut citer à titre indicatif, les puces à ADN mises au point par la société Affymetrix ("Accessing Genetic Information with High-Density DNA arrays", M. Chee et al., Science, 1996, 274, 610-614. "Light-generated oligonucleotide arrays for rapide DNA séquence analysis", A. Caviani Pease et al., Proc. Natl. Acad. Sci. USA, 1994, 91, 5022-5026), pour les diagnostics moléculaires. Dans cette technologie, les sondes de capture sont généralement de tailles réduites, autour de 25 nucléotides. D'autres exemples de biopuces sont donnés dans les publications de G. Ramsay, Nature Biotechnology, 1998, n° 16, p. 40-44 ; F. Ginot, Human Mutation, 1997, n°10, p.1-10; J. Cheng et al, Molecular diagnosis, 1996, n°1(3), p.183-200 ; T. Livache et al, Nucleic Acids Research, 1994, n° 22(15), p. 2915-2921 ; J. Cheng et al, Nature Biotechnology, 1998, n° 16, p. 541-546 ou dans les brevets
US-A-4,981,783, US-A-5,700,637, US-A-5,445,934, US-A-5,744,305 et US-A-5,807,522. La caractéristique principale du support solide doit être de conserver les caractéristiques d'hybridation des sondes de capture sur les fragments nucléotidiques cibles tout en générant un bruit de fond minimum pour la méthode de détection.
Pour l'immobilisation des sondes sur le support, on distingue trois grands types de fabrication.

Il y a, tout d'abord, une première technique qui consiste en un dépôt de sondes pré-synthétisées. La fixation des sondes se fait par transfert direct, au moyen de micropipettes, de micro-pointes ou par un dispositif de type jet d'encre. Cette technique permet la fixation de sondes de taille allant de quelques bases (5 à 10) jusqu'à des tailles relativement importantes de 60 bases (impression) à quelques centaines de bases (micro-déposition) :

L'impression est une adaptation du procédé utilisé par les imprimantes à jet d'encre. Elle repose sur la propulsion de très petites sphères de fluide (volume <1 n1) et à un rythme pouvant atteindre 4000 gouttes/secondes. L'impression n'implique aucun contact entre le système libérant le fluide et la surface sur laquelle il est déposé.

La micro-déposition consiste à fixer des sondes longues de quelques dizaines à plusieurs centaines de bases à la surface d'une lame de verre. Ces sondes sont généralement extraites de bases de données et se présentent sous forme de produits amplifiés et purifiés. Cette technique permet de réaliser des puces dénommées microarrays portant environ dix mille spots, dit zones de reconnaissance, d'ADN sur une surface d'un peu moins de 4 cm2. Il ne faut toutefois pas oublier l'emploi de membranes de Nylon, dites « macroarrays », qui portent des produits amplifiés, généralement par PCR, avec un diamètre de 0,5 à 1 mm et dont la densité maximale est de 25 spots/cm2. Cette technique très flexible est utilisée par de nombreux laboratoires. Dans la présente invention, cette dernière technique est considérée comme faisant partie des biopuces. On peut toutefois déposer en fond de plaque de microtitration un certain volume d'échantillon dans chaque puits, comme c'est le cas dans les demandes de brevet WO-A-00/71750 et FR 00/14896, ou déposer au fond d'une même boîte de Pétri un certain nombre de gouttes séparées les unes des autres, selon une autre demande de brevet FR00/14691.

La deuxième technique de fixation des sondes sur le support ou puce est appelée la synthèse in situ. Cette technique aboutit à l'élaboration de sondes courtes directement à la surface de la puce. Elle repose sur la synthèse d'oligonucléotides in situ (voir notamment les demandes de brevet WO 89/10977 et WO 90/03382), et est fondée sur le procédé des synthétiseurs d'oligonucléotides. Elle consiste à déplacer une chambre de réactions, où se déroule la réaction d'élongation d'oligonucléotides, le long de la surface de verre.

Enfin, la troisième technique est appelée la photolithographie, qui est un procédé à l'origine des biopuces développées par Affymetrix. Il s'agit également d'une synthèse in situ. La photolithographie est dérivée des techniques des microprocesseurs. La surface de la puce est modifiée par la fixation de groupements chimiques photolabiles pouvant être activés par la lumière. Une fois illuminés, ces groupes sont susceptibles de réagir avec l'extrémité 3' d'un oligonucléotide. En protégeant cette surface par des masques de formes définies, on peut illuminer et donc activer sélectivement des zones de la puce où l'on souhaite fixer l'un ou l'autre des quatre nucléotides. L'utilisation successive de masques différents permet d'alterner des cycles de protection/réaction et donc de réaliser les sondes d'oligonucléotides sur des spots d'environ quelques dizaines de micromètre carré (µm2). Cette résolution permet de créer jusqu'à plusieurs centaines de milliers de spots sur une surface de quelques centimètres carré (cm2). La photolithographie présente des avantages : massivement parallèle, elle permet de créer une puce de N-mères en seulement 4 x N cycles. Toutes ces techniques sont utilisables avec la présente invention.
Au sens de la présente invention, la détermination de l'expression (étape c) du procédé selon l'invention) d'un gène cible peut être réalisée par tous les protocoles connus de l'homme du métier.
D'une manière générale, l'expression d'un gène cible peut être analysée par la détection des ARNm (ARN messagers) qui sont transcrits du gène cible à un instant donné ou par la détection des protéines issues de ces ARNm.
Lorsque le réactif spécifique est une amorce d'amplification, on peut déterminer l'expression d'un gène cible de la manière suivante:
1) après avoir extrait comme matériel biologique, les ARN totaux (comprenant les ARN de transfert (ARNt), les ARN ribosomaux (ARNr) et les ARN messagers (ARNm)) d'un échantillon biologique tel que présenté précédemment, on réalise une étape de transcription inverse afin d'obtenir les ADN complémentaires (ou ADNc) desdits ARNm. A titre indicatif, cette réaction de transcription inverse peut être réalisée à l'aide d'une enzyme reverse transcriptase qui permet d'obtenir, à partir d'un fragment d'ARN, un fragment d'ADN complémentaire. On peut utiliser notamment l'enzyme reverse transcriptase provenant de l'AMV (Avian Myoblastosis Virus) ou de MMLV (Moloney Murine Leukaemia Virus). Lorsque l'on souhaite plus particulièrement obtenir uniquement les ADNc des ARNm, on réalise cette étape de transcription inverse en présence de fragments nucléotidiques comprenant uniquement des bases thymine (polyT), qui s'hybrident par complémentarité sur la séquence polyA des ARNm afin de former un complexe polyT-polyA qui sert alors de point de départ à la réaction de transcription inverse réalisée par l'enzyme reverse transcriptase. On obtient alors des ADNc complémentaires des ARNm issus d'un gène cible (ADNc spécifique du gène cible) et des ADNc complémentaires des ARNm issus d'autres gènes que le gène cible (ADNc non spécifique du gène cible).
2) on met en contact la ou les amorces d'amplification spécifiques d'un gène cible avec les ADNc spécifique du gène cible et les ADNc non spécifique du gène cible. La ou les amorces d'amplification spécifiques d'un gène cible s'hybrident avec les ADNc spécifique du gène cible et on amplifie spécifiquement une région prédéterminée, de longueur connue, des ADNc provenant des ARNm issus du gène cible. Les ADNc non spécifiques du gène cible ne sont pas amplifiés, alors qu'on obtient alors une grande quantité d'ADNc spécifiques du gène cible. Au sens de la présente invention, on parle indifféremment d' « ADNc spécifiques du gène cible » où d' « ADNc provenant des ARNm issus du gène cible ». Cette étape peut être réalisée notamment par une réaction d'amplification de type PCR ou par toute autre technique d'amplification telle que définie précédemment. En PCR, on peut également amplifier simultanément plusieurs ADNc différents, chacun étant spécifique de différents gènes cibles par l'utilisation de plusieurs couples d'amorces d'amplification différents, chacune étant spécifique d'un gène cible: on parle alors d'amplification en multiplex.
3) on détermine l'expression du gène cible en détectant et quantifiant les ADNc spécifiques du gène cible obtenus lors de l'étape 2) ci dessus. Cette détection peut être réalisée après migration par électrophorèse des ADNc spécifiques du gène cible en fonction de leur taille. Le gel et le milieu de migration peuvent comprendre du bromure d'éthydium afin de permettre la détection directe des ADNc spécifiques du gène cible lorsque le gel est placé, après un temps de migration donné, sur une table lumineuse à rayons UV (ultra violet) par l'émission d'un signal lumineux. Ce signal est d'autant plus lumineux que la quantité des ADNc spécifiques du gène cible est importante. Ces techniques d'électrophorèse sont bien connues de l'homme du métier. Les ADNc spécifiques du gène cible peuvent également être détectés et quantifiés par l'utilisation d'une gamme de quantification obtenue par une réaction d'amplification conduite jusqu'à saturation. Afin de tenir compte de la variabilité d'efficacité enzymatique qui peut être observée lors des différentes étapes (transcription inverse, PCR...), on peut normaliser l'expression d'un gène cible de différents groupes de patients, par la détermination simultanée de l'expression d'un gène dit de ménage, dont l'expression est similaire chez les différents groupes de patients. En réalisant un rapport entre l'expression du gène cible et l'expression du gène de ménage, c'est à dire en réalisant un rapport entre la quantité d'ADNc spécifiques du gène cible, et la quantité d'ADNc spécifiques du gène de ménage, on corrige ainsi toute variabilité entre les différentes expérimentations. L'homme du métier pourra se référer notamment aux publications suivantes : Bustin SA, J Mol Endocrinol , 2002, 29 : 23-39 ; Giulietti A Methods, 2001, 25 : 386-401.
Lorsque le réactif spécifique est une sonde d'hybridation, on peut déterminer l'expression d'un gène cible de la manière suivante:
1) après avoir extrait, comme matériel biologique, les ARN totaux d'un échantillon biologique tel que présenté précédemment, on réalise une étape de transcription inverse, telle que décrite précédemment afin d'obtenir des ADNc complémentaires des ARNm issus d'un gène cible (ADNc spécifique du gène cible) et des ADNc complémentaires des ARNm issus d'autres gènes que le gène cible (ADNc non spécifique du gène cible).
2) on met en contact tous les ADNc avec un support, sur lequel sont immobilisées des sondes de capture spécifiques du gène cible dont on souhaite analyser l'expression, afin de réaliser une réaction d'hybridation entre les ADNc spécifiques du gène cible et les sondes de capture, les ADNc non spécifiques du gène cible ne s'hybridant pas sur les sondes de capture. La réaction d'hybridation peut être réalisée sur un support solide qui inclut tous les matériaux tels qu'indiqués précédemment. Selon un mode préféré de réalisation, la sonde d'hybridation est immobilisée sur un support. Préférentiellement, le support est une biopuce. La réaction d'hybridation peut être précédée d'une étape d'amplification enzymatique des ADNc spécifique du gène cible telle que décrite précédemment pour obtenir une grande quantité d'ADNc spécifiques du gène cible et augmenter la probabilité qu'un ADNc spécifiques d'un gène cible s'hybride sur une sonde de capture spécifique du gène cible. La réaction d'hybridation peut également être précédée d'une étape de marquage et/ou de clivage des ADNc spécifiques du gène cible telle que décrite précédemment, par exemple en utilisant un désoxyribonucléotide triphosphate marqué pour la réaction d'amplification. Le clivage peut être réalisé notamment par l'action de l'imidazole et de chlorure de manganèse. L'ADNc spécifique du gène cible peut aussi être marqué après l'étape d'amplification, par exemple en hybridant une sonde marquée selon la technique d'hybridation sandwich décrite dans le document WO-A-91/19812. D'autres modes particuliers préférentiels de marquage et/ou clivage d'acides nucléiques sont décrit dans les demandes WO 99/65926, WO 01/44507, WO 01/44506, WO 02/090584, WO 02/090319.
3) on réalise ensuite une étape de détection de la réaction d'hybridation. La détection peut être réalisée par la mise en contact du support sur lequel sont hybridés les sondes de capture spécifiques du gène cible avec les ADNc spécifiques du gène cible avec une sonde dite de détection, marquée par un marqueur, et on détecte le signal émis par le marqueur. Lorsque l'ADNc spécifique du gène cible a été préalablement marqué par un marqueur, on détecte directement le signal émis par le marqueur.

Lorsque le réactif spécifique est une sonde d'hybridation, on peut également déterminer l'expression d'un gène cible de la manière suivante:
1) après avoir extrait, comme matériel biologique, les ARN totaux d'un échantillon biologique tel que présenté précédemment, on réalise une étape de transcription inverse, telle que décrite précédemment afin d'obtenir les ADNc des ARNm du matériel biologique.
On réalise ensuite la polymérisation de l'ARN complémentaire du ADNc par l'utilisation d'une enzyme polymerase de type T7 polymérase qui fonctionne sous la dépendance d'un promoteur et qui permet d'obtenir, à partir d'une matrice d'ADN, l'ARN complémentaire.
On obtient alors les ARNc des ADNc des ARNm spécifiques du gène cible (on parle alors d'ARNc spécifique du gène cible) et les ARNc des ADNc des ARNm non spécifiques du gène cible.
2) on met en contact tous les ARNc avec un support, sur lequel sont immobilisées des sondes de capture spécifiques du gène cible dont on souhaite analyser l'expression, afin de réaliser une réaction d'hybridation entre les ARNc spécifiques du gène cible et les sondes de capture, les ARNc non spécifiques du gène cible ne s'hybridant pas sur les sondes de capture. Lorsque l'on souhaite analyser simultanément l'expression de plusieurs gènes cibles, on peut immobiliser sur le support plusieurs sondes de capture différentes, chacune étant spécifique d'un gène cible. La réaction d'hybridation peut également être précédée d'une étape de marquage et/ou de clivage des ARNc spécifiques du gène cible telles que décrites précédemment.
3) on réalise ensuite une étape de détection de la réaction d'hybridation. La détection peut être réalisée par la mise en contact du support sur lequel sont hybridées les sondes de capture spécifiques du gène cible avec l'ARNc spécifique du gène cible avec une sonde dite de détection, marquée par un marqueur, et on détecte le signal émis par le marqueur. Lorsque l'ARNc spécifiques du gène cible a été préalablement marqué par un marqueur, on détecte directement le signal émis par le marqueur. L'utilisation d'ARNc est particulièrement avantageux lorsqu'on utilise un support de type biopuce sur lequel est hybride un grand nombre de sondes.
Selon un mode particulier de réalisation de l'invention, les étapes B et C sont effectuées en même temps. Ce mode préféré peut être notamment mise en oeuvre par « NASBA en temps réel » qui regroupe en une étape unique la technique d'amplification NASBA et la détection en temps réel qui fait appel à des "molecular beacons". La réaction NASBA intervient dans le tube, produisant de l'ARN simple brin avec lequel les "molecular beacons" spécifiques peuvent s'hybrider simultanément pour donner un signal fluorescent. La formation des nouvelles molécules d'ARN est mesurée en temps réel par contrôle continu du signal dans un lecteur fluorescent. Contrairement à une amplification par RT-PCR, l'amplification en NASBA peut se faire en présence de contamination en ADN dans l'échantillon. Il n'est donc pas nécessaire de vérifier que l'ADN a bien été complètement éliminé lors de l'extraction des ARN.
D'une manière surprenante, les inventeurs ont mis en évidence que l'analyse de l'expression des an moins 8 gènes cibles ayant les séquences SEQ ID NO: 1 - 8 sélectionnés parmi les 74 gènes tel que présenté dans le tableau 1 ci après, est très pertinente pour le diagnostic précoce du cancer du sein.

**Tableau 1 - Liste des 74 gènes exprimés différentiellement lors du développement d'un cancer du sein stade I / II**

| **SEQ ID N°** | **Description de la séquence** | **N° Genbank** |
|---|---|---|
| 1 | Centrosome-associated protein 350 [CAP350] | NM_014810 |
| 2 | Hypothetical protein MGC23401 | NM_144982 |
| 3 | Trophoblast-derived noncoding RNA [TncRNA] | AF001893 (Hs.523789) |
| 4 | Vacuolar protein sorting 35 (yeast) [PUM2] | NM_015317 |
| 5 | Ribosomal protein L36a-like [RPL36AL] | NM_001001 |
| 6 | Mitochondrial ribosomal protein L51 [MRPL51] | NM_016497 |
| 7 | KIAA0794 protein [KIAA0794] | XM_087353 |
| 8 | Transcribed locus | CA775887 (Hs. 388575) |
| 9 | Hypothetical protein MGC14817 [MGC14817] | NM_032338 |
| 10 | Hypothetical protein FLJ11046 | NM_018309 |
| 11 | Pleckstrin homology, Sec7 and coiled-coil domains 4 [PSCD4] | NM_013385 |
| 12 | Lactate dehydrogenase B [LDHB] | NM_002300 |
| 13 | NADH dehydrogenase (ubiquinone) alpha subcomplex 1 [NDUFA1] | NM_004541 |
| 14 | Muscleblind-like (Drosophila) [MBNL1] | NM_021038 |
| 15 | Ubiquitin specific protease 25 [USP25] | NM_013396 |
| 16 | TATA element modulatory factor 1 [TMF1] | NM_007114 |
| 17 | Ring finger protein 19 [RNF19] | NM_015435 |
| 18 | Signal peptidase complex subunit 3 homolog (S. cerevisiae) [SPCS3] | NM_021928 |
| 19 | Enhancer of polycomb homolog 1 (Drosophila) [EPC1] | NM_025209 |
| 20 | Zinc finger, matrin type 2 [ZMAT2] | NM_144723 |
| 21 | Image clone 3069209 | BF512254 |
| 22 | ORMI-like 3 (S. cerevisiae) [ORMDL3] | NM_139280 |
| 23 | CDNA FLJ11397 fis, clone HEMBA1000622 | AW962458 (Hs. 470871) |
| 24 | Tankyrase, TRF1-interacting ankyrin-related ADP-ribose polymerase [TNKS] | NM_003747 |
| 25 | Ribosomal protein S23 [RPS23] | NM_001025 |
| 26 | CDNA clone IMAGE:5263531 | AK025902 (Hs.399763) |
| 27 | PABP1-dependent poly A-specific ribonuclease subunit [PAN3] | NM_175854 |
| 28 | Hypothetical protein FLJ21924 | NM_024774 |
| 29 | CDNA FLJ42313 fis, clone TRACH2019425 | AK124306 (Hs.386042) |
| 30 | Family with sequence similarity 49, member B [FAM49B] | NM_016623 |
| 31 | Dicer1, Dcr-1 homolog (Drosophila) [D1CER1] | NM_030621 |
| 32 | Ribosomal protein L37 [RPL37] | NM_000997 |
| 33 | UDP-glucose ceramide glucosyltransferase [UGCG] | NM_003358 |
| 34 | Complement component (3b/4b) receptor 1 [CR1] | NM_000573 |
| 35 | KIAA1702 protein | AB051489 (Hs.485628) |
| 36 | Hypothetical protein FLJ10618 | NM_018155 |
| 37 | Hypothetical protein LOC146174 | NM_173501 |
| 38 | MRNA; cDNA DKFZp686D22106 (from clone DKFZp686D22106) | CR933609 (Hs. 445036) |
| 39 | Anterior pharynx defective 1 homolog A (C. elegans) [APH1A] | NM_016022 |
| 40 | U2-associated SR140 protein [SR140] | XM_031553 |
| 41 | Androgen-induced proliferation inhibitor [APRIN] | NM_015032 |
| 42 | Peptidylprolyl isomerase D (cyclophilin D) [PPID] | NM_005038 |
| 43 | Mitochondrial ribosomal protein S17 [MRPS17] | NM_015969 |
| 44 | Adaptor-related protein complex 1, sigma 2 subunit [AP1S2] | NM_003916 |
| 45 | Heat shock 90kDa protein 1, alpha [HSPCA] | NM_005348 |
| 46 | GNAS complex locus [GNAS] | NM_000516 |
| 47 | 5-azacytidine induced 2 [AZI2] | NM_022461 |
| 48 | BCL2-like 1 [BCL2L1] | NM_001191 |
| 49 | Bobby sox homolog (Drosophila) [BBX] | NM_020235 |
| 50 | Calcium-transporting ATPase, type 2C, member 1 [ATP2C1] | NM_001001485 |
| 51 | Cathepsin Z [CTSZ] | NM_001336 |
| 52 | CDNA FLJ26120 fis, clone SYN00419 | AK129631 (Hs.433995) |
| 53 | COMM domain containing 6 [COMMD6] | NM_203495 |
| 54 | Cytochrome c oxidase subunit VIIb [COX7B] | NM_001866 |
| 55 | Cytoplasmic polyadenylation élément binding protein 2 [CPEB2] | NM_182485 |
| 56 | Endoplasmic reticulum-golgi intermediate compartment 32 kDa protein [HIAA1181] | NM_020462 |
| 57 | Ewing sarcoma breakpoint region 1 [EWSR1] | NM_005243 |
| 58 | Glyceraldehyde-3-phosphate dehydrogenase [GAPD] | NM_002046 |
| 59 | GRB2-associated binding protein 2 [GAB2] | NM_012296 |
| 60 | Killer cell lectin-like receptor subfamily C, member 1 or 2 [KLRC1/KLRC2] | NM_002259 |
| 61 | Killer cell lectin-like receptor subfamily F1 [KLRF1] | NM_016523 |
| 62 | Metastasis associated lung adenocarcinoma transcript 1 [MALAT1] | BX538238 (Hs.187199) |
| 63 | MRNA; cDNA DKFZp58600724 | BU676985 (Hs.159115) |
| 64 | Nipped-B homolog (Drosophila) [NIPBL] | NM_015384 |
| 65 | Prader-Willi/Angelman region-1 [PAR1] | BE783065 (Hs.546847) |
| 66 | PRO1550 | AF086013 (Hs.371588) |
| 67 | Protein phosphatase 2, regulatory subunit B (BS6), epsilon isoform [PPP2R5E] | NM_006246 |
| 68 | RP42 homolog [RP42] | NM_020640 |
| 69 | Special AT-rich sequence binding protein 1 [SATB1] | NM_002971 |
| 70 | Tubulin beta 2 [TUBB2] | NM_001069 |
| 71 | Ubiquitin-fold modifier 1 [Ufm1] | NM_016617 |
| 72 | v-myb myeloblastosis viral oncogene homolog [MYBL1] | XM_034274 |
| 73 | WNK lysine deficient protein kinase 1 [WNK1] | NM_018979 |
| 74 | Zinc finger, MYND domain containing 11 [ZMYND11] | NM_006624 |

Parmi ces gènes, on peut distinguer des gènes dont la fonction est connue mais qui n'ont jamais été mis en relation avec le cancer du sein (SEQ ID N°3 à 6 ; 11 ; 13 à 15 ; 17 ; 25 ; 31 à 34 ; 39 ; 42 à 46 ; 48 ; 50 ; 51 ; 54 ; 60 ; 62 ; 64 ; 67 ; 69 ; 70 ; 72 à 74) ainsi que des gènes dont la fonction est inconnue (SEQ ID N°1 ; 2 ; 7 à 10 ; 16 ; 18 à 23 ; 26 à 30 ; 35 à 38 ; 40 ; 47 ; 49 ; 52 ; 53 ; 55 à 57 ; 61 ; 63 ; 65 ; 66 ; 68; 1).
Toutes les isoformes des gènes présentant les SEQ ID NO:1-8 selon l'invention sont relevantes pour la présente invention. Il convient notamment de noter qu'il existe plusieur variants pour le gène cible de SEQ ID N°14, seul le premier variant est présenté dans le tableau ci dessus, mais les variants, ayant pour numéro d'accession Genbank ; NM_207292; NM_207293; NM_207294; NM_207295; NM_207296; NM_207297 sont tout aussi pertinents au sens de la présente description.
De même, pour la SEQ ID N°17, seul le premier variant est présenté dans le tableau ci dessus, mais le variant, ayant pour numéro d'accession Genbank NM_183419 est tout aussi pertinent au sens de la présente description.
De même, pour la SEQ ID N°31, seul le premier variant est présenté dans le tableau ci dessus, mais le variant, ayant pour numéro d'accession Genbank NM_177438 est tout aussi pertinent au sens de la présente description.
De même, pour la SEQ ID N°34, seul le premier variant est présenté dans le tableau ci dessus, mais le variant, ayant pour numéro d'accession Genbank NM_000651 est tout aussi pertinent au sens de la présente description.
De même, pour la SEQ ID N°41, seul le premier variant est présenté dans le tableau ci dessus, mais le variant, ayant pour numéro d'accession Genbank ; NM_015928 est tout aussi pertinent au sens de la présente description.
Pour le gène cible de SEQ ID N°46, seul le premier variant est présenté dans le tableau ci dessus, mais les variants, ayant pour numéro d'accession Genbank; NM_016592; NM_080425; NM_080426 sont tout aussi pertinents au sens de la présente description.
Pour le gène cible de SEQ ID N°47, seul le premier variant est présenté dans le tableau ci dessus, mais le variants, ayant pour numéro d'accession Genbank; NM_203326 est tout aussi pertinents au sens de la présente description.
Pour le gène cible de SEQ ID N°48, seul le premier variant est présenté dans le tableau ci dessus, mais le variants, ayant pour numéro d'accession Genbank; NM_138578 est tout aussi pertinents au sens de la présente description.
Pour le gène cible de SEQ ID N°50, seul le premier variant est présenté dans le tableau ci dessus, mais les variants, ayant pour numéro d'accession Genbank; NM_001001485 ; NM_001001486 ; NM_001001487 ; NM_014382 sont tout aussi pertinents au sens de la présente description.
Pour le gène cible de SEQ ID N°53, seul le premier variant est présenté dans le tableau ci dessus, mais le variants, ayant pour numéro d'accession Genbank NM_203497 est tout aussi pertinents au sens de la présente description.
Pour le gène cible de SEQ ID N°55, seul le premier variant est présenté dans le tableau ci dessus, mais le variants, ayant pour numéro d'accession Genbank NM_182646 est tout aussi pertinents au sens de la présente description.
Pour le gène cible de SEQ ID N°57, seul le premier variant est présenté dans le tableau ci dessus, mais le variants, ayant pour numéro d'accession Genbank NM_013986 est tout aussi pertinents au sens de la présente description.
Pour le gène cible de SEQ ID N°59, seul le premier variant est présenté dans le tableau ci dessus, mais le variants, ayant pour numéro d'accession Genbank NM_080491 est tout aussi pertinents au sens de la présente description.
Pour le gène cible de SEQ ID N°60, seul le premier variant est présenté dans le tableau ci dessus, mais les variants, ayant pour numéro d'accession Genbank; NM_002259 ; NM_002260 ; NM_007328 ; NM_213657 ; NM_213658 sont tout aussi pertinents au sens de la présente description.
Pour le gène cible de SEQ ID N°64, seul le premier variant est présenté dans le tableau ci dessus, mais le variants, ayant pour numéro d'accession Genbank NM_133433 est tout aussi pertinents au sens de la présente description.
Pour le gène cible de SEQ ID N°74, seul le premier variant est présenté dans le tableau ci dessus, mais le variants, ayant pour numéro d'accession Genbank NM_212479 est tout aussi pertinents au sens de la présente description.
De plus, on décrit ici que l'analyse de gènes cibles sélectionnés parmis les 95 gènes présentés dans le tableau 2 ci après est très pertinente pour le diagnostic d'un cancer du sein avancé.

**Tableau 2 - Liste des 95 gènes exprimés différentiellement lors du développement d'un cancer du sein stade III / IV**

| **SEQ ID N°** | **Description de la séquence** | **N° Genbank** |
|---|---|---|
| 1 | Centrosome-associated protein 350 [CAP350] | NM_014810 |
| 2 | Hypothetical protein MGC23401 | NM_144982 |
| 3 | Trophoblast-derived noncoding RNA [TncRNA] | AF001893 (Hs.523789) |
| 4 | Vacuolar protein sorting 35 (yeast) [PUM2] | NM_015317 |
| 5 | Ribosomal protein L36a-like [RPL36AL] | NM_001001 |
| 6 | Mitochondrial ribosomal protein L51 [MRPL51] | NM_016497 |
| 13 | NADH dehydrogenase (ubiquinone) alpha subcomplex 1 [NDUFA1] | NM_004541 |
| 14 | Muscleblind-like (Drosophila) [MBNL1] | NM_021038 |
| 20 | Zinc finger, matrin type 2 [ZMAT2] | NM_144723 |
| 26 | CDNA clone IMAGE:5263531, partial cds | AK025902 (Hs.399763) |
| 28 | Hypothetical protein FLJ21924 | NM_024774 |
| 36 | Hypothetical protein FLJ10618 | NM_018155 |
| 37 | Hypothetical protein LOC283666 | BC048264 (Hs.512943) |
| 39 | Anterior pharynx defective 1 homolog A (C. elegans) [APH1A] | NM 016022 |
| 40 | U2-associated SR140 protein [SR140] | XM_031553 |
| 41 | Androgen-induced proliferation inhibitor [APR1N] | NM_015032 |
| 46 | GNAS complex locus [GNAS] | NM_000516 |
| 48 | BCL2-like 1 [BCL2L1] | NM_001191 |
| 59 | GRB2-associated binding protein 2 [GAB2] | NM_012296 |
| 60 | Killer cell lectin-like receptor subfamily C, member 1 or 2 [KLRC1/KLRC2] | NM_002259 |
| 61 | Killer cell lectin-like receptor subfamily F1 [KLRF1] | NM_016523 |
| 63 | mRNA; cDNA DKFZp58600724 (from clone DKFZp58600724) | BU676985 (Hs.159115) |
| 65 | Prader-Willi/Angelman région-1 [PAR1] | BE783065 (Hs.546847) |
| 67 | Protein phosphatase 2, regulatory subunit B (B56), epsilon isoform [PPP2R5E] | NM_006246 |
| 69 | Special AT-rich sequence binding protein 1 [SATB1] | NM_002971 |
| 70 | Tubulin beta 2 [TUBB2] | NM_001069 |
| 71 | Ubiquitin-fold modifier 1 [Ufm1] | NM_016617 |
| 72 | v-myb myeloblastosis viral oncogene homolog [MYBL1] | XM_034274 |
| 73 | WNK lysine deficient protein kinase 1 [WNK1] | NM_018979 |
| 74 | Zinc finger, MYND domain containing 11 [ZMYND11] | NM_006624 |
| 75 | 30 kDa protein LOC55831 | NM_018447 |
| 76 | ADP-ribosylation factor guanine nucleotide-exchange factor 2 [ARFGEF2] | NM_006420 |
| 77 | BTB (POZ) domain containing 5 [BTBD5] | NM_017658 |
| 78 | Cathepsin O [CTSO] | NM_001334 |
| 79 | Centrin, EF-hand protein 2 [CETN2] | NM_004344 |
| 80 | Chromosome 16 open reading frame 35 [C16orf35] | NM_012075 |
| 81 | Chromosome 2 open reading frame 33 [C2orf33] | NM_020194 |
| 82 | Cleavage and polyadenylation specific factor 6, 68kDa [CPSF6] | NM_007007 |
| 83 | Cysteine-rich motor neuron 1 [CRIM1] | NM_016441 |
| 84 | Enoyl Coenzyme A hydratase domain containing 1 [ECHDC1] | NM_018479 |
| 85 | Erythrocyte membrane protein band 4.2 [EPB42] | NM_000119 |
| 86 | Formin binding protein 3 [FNBP3] | XM_371575 |
| 87 | Hepatitis B virus x associated protein [HBXAP] | NM_016578 |
| 88 | Hypothetical protein HSPC129 | NM_016396 |
| 89 | Hypothetical protein LOC144438 | AK002085 (Hs.92308) |
| 90 | Hypothetical protein MGC33214 | NM_153354 |
| 91 | Hypothetical protein MGC5306 | NM_024116 |
| 92 | Likely ortholog of mouse TORC2-specific protein AVO3 (S. cerevisiae) [AV03] | NM_152756 |
| 93 | Mannosidase, alpha, class 2A, member 1 [MAN2A1] | NM_002372 |
| 94 | Mdm4, p53 binding protein (mouse) [MDM4] | NM_002393 |
| 95 | Nucleobindin 1 [NUCB1] | NM_006184 |
| 96 | Oxysterol binding protein 2 [OSBP2] | NM_001003812 |
| 97 | Phosphoinositide-3-kinase, catalytic, alpha polypeptide [PIK3CA] | NM_006218 |
| 98 | Proteasome (prosome, macropain) inhibitor subunit 1 (PI31) [PSMF1] | NM_006814 |
| 99 | Protein tyrosine phosphatase type IVA, member 2 [PTP4A2] | NM_003479 |
| 100 | Rhesus blood group, D antigen [RHD] | NM_016124 |
| 101 | Ring finger protein 123 [RNF123] | NM_022064 |
| 102 | SH2 domain-containing molecule EAT2 [EAT2] | NM_053282 |
| 103 | Source of immunodominant MHC-associated peptides [SIMP] | NM_178862 |
| 104 | Split hand/foot malformation (ectrodactyly) type 1 [SHFM1] | NM_006304 |
| 105 | Thyroid hormone receptor associated protein 1 [THRAP1] | NM_005121 |
| 106 | Thyroid hormone receptor interactor 12 [TRIP12] | NM_004238 |
| 107 | Transcribed locus | AL037805 (Hs. 445247) |
| 108 | Transducin (beta)-like 1X-linked receptor 1 [TBL1XR1] | NM_024665 |
| 109 | Tubulin, beta 3 [TUBB3] | NM_006086 |
| 110 | Ubiquitination factor E4A (UFD2 homolog, yeast) [UBE4A] | NM_004788 |
| 111 | Zinc finger protein 148 (pHZ-52) [ZNF148] | NM_021964 |
| 112 | 3-alpha hydroxysteroid dehydrogenase, type II [AKRI C3] | NM_003739 |
| 113 | A kinase (PRKA) anchor protein 7 [AKAP7] | NM_004842 |
| 114 | Aminolevulinate, delta-, synthase 2 [ALAS2] | NM_000032 |
| 115 | Ankyrin 1, erythrocytic [ANK1] | NM_000037 |
| 116 | B double prime 1, subunit of RNA polymerase III transcription initiation factor IIIB [BDP1] | NM_018429 |
| 117 | Carbonic anhydrase 1 [CA1] | NM_001738 |
| 118 | Chromosome 19 open reading frame 2 [C19orf2] | NM_003796 |
| 119 | DKFZP564F0522 protein | NM_015475 |
| 120 | Erythrocyte membrane protein band 4.9 (dematin) [EPB49] | NM_001978 |
| 121 | Family with sequence similarity 46, member C [FAM46C] | NM_017709 |
| 122 | guanosine monophosphate reductase [GMPR] | NM_006877 |
| 123 | Homo sapiens, clone IMAGE:5267398, mRNA cDNA DKFZp686I23208 | BX538337 (Hs.40289) |
| 124 | Image clone 3481554 | BF062399 |
| 125 | IMAGE clone 5259272 | BC032890 (Hs.184430) |
| 126 | Integrin, alpha 2b [ITGA2B] | NM_000419 |
| 127 | Interleukin 8 [IL8] | NM_000584 |
| 128 | Leucine rich repeat neuronal 3 [LRRN3] | NM_018334 |
| 129 | Leukocyte receptor cluster (LRC) member 10 [LENG10] | AF211977 |
| 130 | Major histocompatibility complex, class II, DQ alpha 1 [HLA-DQA1] | NM_002122 |
| 131 | Phosphatidylinositol glycan, class K [PIGK] | NM_005482 |
| 132 | Selenium binding protein 1 [SELENBP1] | NM_003944 |
| 133 | SM-11044 binding protein [SMBP] | NM_020123 |
| 134 | Solute carrier family 6 (neurotransmitter transporter, creatine), member 8 [SLC6A8] | NM_005629 |
| 135 | TBC1 domain family, member 4 [TBC1D4] | NM_014832 |
| 136 | Tensin [TNS] | NM_022648 |
| 137 | TIA1 cytotoxic granule-associated RNA binding protein [TIA1] | NM_022037 |
| 138 | Transcribed locus | AA456099 (Hs.176376) |
| 139 | Tripartite motif-containing 58 [TRIM58] | NM_015431 |

Parmi ces gènes, on peut distinguer des gènes dont la fonction est connue mais qui n'ont jamais été mis en relation avec le cancer du sein (SEQ ID N°3 à 6 ; 13 ; 14 ; 39 ; 46 ; 48 ; 60 ; 67 ; 69 ; 70 ; 72 à 74 ; 76 ; 78 ; 79 ; 82 ; 83 ; 85 ; 87 ; 92 à 94 ; 97 ; 99 à 101 ; 103 ; 105 ; 110 ; 111 ; 113 à 116 ; 118 ; 120 ; 122 ; 126 ; 130 à 132 ; 134 ; 136 ; 137) ainsi que des gènes dont la fonction est inconnue (SEQ ID N°1 ; 2 ; 20 ; 26 ; 28 ; 36 ; 37 ; 40 ; 61 ; 63 ; 65 ; 71 ; 75 ; 77 ; 80 ; 81 ; 84 ; 86 ; 88 à 91 ; 95 ; 96 ; 102 ; 104 ; 106 à 109 ; 119 ; 121 ; 123 à 125 ; 128 ; 129 ; 133 ; 135 ; 138 ; 139).
Toutes les isoformes des gènes selon l'invention sont relevantes pour la présente description. A ce titre, il convient notamment de noter qu'il existe plusieur variants pour le gène cible de SEQ ID N°14, seul le premier variant est présenté dans le tableau ci dessus, mais les variants, ayant pour numéro d'accession Genbank NM_207292; NM_207293; NM_207294; NM_207295; NM_207296; NM_207297 sont tout aussi pertinents au sens de la présente description.
De même, pour le gène cible de SEQ ID N°41, seul le premier variant est présenté dans le tableau ci dessus, mais le variant ayant pour numéro d'accession Genbank NM_015928 est tout aussi pertinents au sens de la présente description.
De même, pour le gène cible de SEQ ID N°74, seul le premier variant est présenté dans le tableau ci dessus, mais le variant ayant pour numéro d'accession Genbank NM_212479 est tout aussi pertinents au sens de la présente description.
De même, pour le gène cible de SEQ ID N°96, seul le premier variant est présenté dans le tableau ci dessus, mais le variant ayant pour numéro d'accession Genbank NM_030758 est tout aussi pertinents au sens de la présente description.
Pour le gène cible de SEQ ID N°98, seul le premier variant est présenté dans le tableau ci dessus, mais les variants, ayant pour numéro d'accession Genbank NM_178578; NM_178579sont tout aussi pertinents au sens de la présente description.
Pour le gène cible de SEQ ID N°99, seul le premier variant est présenté dans le tableau ci dessus, mais les variants, ayant pour numéro d'accession Genbank NM_080391; NM_080392 sont tout aussi pertinents au sens de la présente description.
De même, pour le gène cible de SEQ ID N°100, seul le premier variant est présenté dans le tableau ci dessus, mais le variant ayant pour numéro d'accession Genbank NM_016225 est tout aussi pertinents au sens de la présente description.
Pour le gène cible de SEQ ID N°112, seul le premier variant est présenté dans le tableau ci dessus, mais les variants, ayant pour numéro d'accession Genbank NM_016377; NM_138633 sont tout aussi pertinents au sens de la présente description.
Pour le gène cible de SEQ ID N°115, seul le premier variant est présenté dans le tableau ci dessus, mais les variants, ayant pour numéro d'accession Genbank NM_020475; NM_020476; NM_020477; NM_020478; NM_020479; NM_020480; NM_020481 sont tout aussi pertinents au sens de la présente description.
De même, pour le gène cible de SEQ ID N°137, seul le premier variant est présenté dans le tableau ci dessus, mais le variant ayant pour numéro d'accession Genbank NM_022173 est tout aussi pertinent pour la présente description.
A ce titre, l'invention concerne procédé pour le diagnostic *in vitro* du cancer du sein chez un patient susceptible d'être atteint d'un cancer du sein **caractérisé en ce qu**'il comprend les étapes suivantes :
a. on extrait du matériel biologique d'un échantillon biologique prélevé chez le patient,
b. on met en contact le matériel biologique avec au moins un réactif spécifique choisi parmi les réactifs spécifiques des gènes cibles présentant une séquence nucléique ayant l'une quelconque des SEQ ID N° 1 à 8
c. on détermine l'expression desdits gènes cibles.
L'analyse de l'expression des 8 gènes cibles présentant les sequences SEQ ID N°1 à 8 permet alors de disposer d'un outil pour le diagnostic du cancer du sein, et est très adapté pour un diagnostic précoce. On peut par exemple analyser l'expression d'un gène cible chez un patient dont on ne connaît pas le diagnostic, et comparer avec des valeurs d'expression moyenne connues du gène cible de patients sains et des valeurs d'expression moyenne connues du gène cible de patients atteint d'un cancer du sein en stade précoce. Lors de l'étape b) on peut mettre en contact le matériel biologique avec au moins 10, au moins 15, au moins 20, au moins 25, au moins 30, au moins 35, au moins 40, au moins 45, au moins 50, au moins 55, au moins 60, au moins 65, au moins 70, au moins 74 réactifs spécifiques choisis parmi les réactifs spécifiques des gènes cibles présentant une séquence nucléique ayant l'une quelconque des SEQ ID N° 1 à 74 et on détermine, lors de l'étape c, l'expression d'au moins au moins au moins 5, au moins 10, au moins 15, au moins 20, au moins 25, au moins 30, au moins 35, au moins 40, au moins 45, au moins 50, au moins 55, au moins 60, au moins 65, au moins 70, au moins 74 desdits gènes cibles.
Selon un mode particulier de réalisation de la méthode décrite un procédé *in vitro* pour le diagnostic, préférentiellement précoce, du cancer du sein chez un patient susceptible d'être atteint d'un cancer du sein est **caractérisé en ce qu**'il comprend les étapes suivantes :
a. on extrait du matériel biologique d'un échantillon biologique prélevé chez le patient,
b. on met en contact le matériel biologique avec au moins 46 réactifs spécifiques choisi parmi les réactifs spécifiques des gènes cibles présentant une séquence nucléique ayant l'une quelconque des SEQ ID N° 1 à 46
c. on détermine l'expression desdits gènes cibles.
Selon un autre mode préféré de ladite méthode, lors de l'étape b) on met en contact le matériel biologique avec au moins 23 ou avec 23 réactifs spécifiques choisis parmi les réactifs spécifiques des gènes cibles présentant une séquence nucléique ayant l'une quelconque des SEQ ID N° 1 à 23 et on détermine, lors de l'étape c, l'expression d'au moins 23 ou 23 desdits gènes cibles.
Selon un mode particulièrement préféré de ladite méthode un procédé in vitro pour le diagnostic, préférentiellement précoce, du cancer du sein chez un patient susceptible d'être atteint d'un cancer du sein **caractérisé en ce qu**'il comprend les étapes suivantes :
a. on extrait du matériel biologique d'un échantillon biologique prélevé chez le patient,
b. on met en contact le matériel biologique avec au moins 23 réactifs spécifiques choisi parmi les réactifs spécifiques des gènes cibles présentant une séquence nucléique ayant l'une quelconque des SEQ ID N° 1 à 23
c. on détermine l'expression desdits gènes cibles.
Selon l'invention, lors de l'étape b) on met en contact le matériel biologique avec au moins 8 ou avec 8 réactifs spécifiques choisis parmi les réactifs spécifiques des gènes cibles présentant une séquence nucléique ayant l'une quelconque des SEQ ID N°1 à 8 et on détermine, lors de l'étape c, l'expression d'au moins 8 ou 8 desdits gènes cibles.
L'invention concerne un procédé *in vitro* pour le diagnostic, préférentiellement précoce, du cancer du sein chez un patient susceptible d'être atteint d'un cancer du sein **caractérisé en ce qu**'il comprend les étapes suivantes :
a. on extrait du matériel biologique d'un échantillon biologique prélevé chez le patient,
b. on met en contact le matériel biologique avec au moins 8 réactifs spécifiques choisi parmi les réactifs spécifiques des gènes cibles présentant une séquence nucléique ayant l'une quelconque des SEQ ID N° 1 à 8
c. on détermine l'expression desdits gènes cibles.
L'utilisation de panel de gènes restreint est particulièrement adapté pour obtenir un outil de pronostic. En effet, l'analyse de l'expression d'une dizaine de gènes ne nécessite pas la fabrication à façon de puce à ADN, et peut être mise en oeuvre directement par des techniques de PCR ou de NASBA, ou puce de basse densité, ce qui présente un atout économique important et une mise en oeuvre simplifiée.
Aussi décrit est un procédé pour le diagnostic *in vitro* du cancer du sein chez un patient susceptible d'être atteint d'un cancer du sein **caractérisé en ce qu**'il comprend les étapes suivantes :
a. on extrait du matériel biologique d'un échantillon biologique prélevé chez le patient,
b. on met en contact le matériel biologique avec au moins un réactif spécifique choisi parmi les réactifs spécifiques des gènes cibles présentant une séquence nucléique ayant l'une quelconque des SEQ ID N° 1 à 6 ; 13 ;14 ;20 ;26 ;28 ; 36 à 41 ; 46; 48; 59 à 61 ; 63 ; 65 ; 67 ; 69 à 139
c. on détermine l'expression desdits gènes cibles
L'analyse de l'expression d'un gène cible choisi parmi l'une quelconque des SEQ ID N°1 à SEQ -ID N° 1 à 6 ; 13 ;14 ;20 ;26 ;28 ; 36 à 41 ; 46; 48; 59 à 61 ; 63 ; 65 ; 67 ; 69 à 139 permet alors de disposer d'un outil pour le diagnostic du cancer du sein, qui est très adapté pour le diagnostic d'un cancer en stade tardif. On peut par exemple analyser l'expression d'un gène cible chez un patient dont on ne connaît pas le diagnostic, et comparer avec des valeurs d'expression moyenne connues du gène cible de patients sains et des valeurs d'expression moyenne connues du gène cible de patients atteint d'un cancer du sein en stade tardif. Cet outil permet également le suivi par exemple d'un traitement prescrit à un patient atteint d'un cancer du sein avancé
Lors de l'étape b) on peut mettre en contact le matériel biologique avec au moins au moins 5, au moins 10, au moins 15, au moins 20, au moins 25, au moins 30, au moins 35, au moins 40, au moins 45, au moins 50, au moins 55, au moins 60, au moins 65, au moins 70, au moins 75, au moins 80, au moins 85, au moins 90, au moins 95 ou au moins 97 réactifs spécifiques choisis parmi les réactifs spécifiques des gènes cibles présentant une séquence nucléique ayant l'une quelconque des SEQ ID N° 1 à 6 ; 13 ;14 ;20 ;26 ;28 ; 36 à 41 ; 46; 48; 59 à 61 ; 63 ; 65 ; 67 ; 69 à 139 et on détermine, lors de l'étape c, l'expression d'au moins au moins au moins 5, au moins 10, au moins 15, au moins 20, au moins 25, au moins 30, au moins 35, au moins 40, au moins 45, au moins 50, au moins 55, au moins 60, au moins 65, au moins 70, au moins 75, au moins 80, au moins 85, au moins 90, au moins 95 ou au moins 97 desdits gènes cibles.
Aussi décrit est un procédé pour le diagnostic tardif in vitro du cancer du sein chez un patient susceptible d'être atteint d'un cancer du sein **caractérisé en ce qu**'il comprend les étapes suivantes :
a. on extrait du matériel biologique d'un échantillon biologique prélevé chez le patient,
b. on met en contact le matériel biologique avec au moins 54 ou 54 réactifs spécifiques choisi parmi les réactifs spécifiques des gènes cibles présentant une séquence nucléique ayant l'une quelconque des SEQ ID N° 1 à 6 ; 13 ;14 ;20 ;26 ;28 ;38 à 41 ;69 ;74 à 110
c. on détermine l'expression desdits gènes cibles.
Lors de l'étape b) on peut mettre en contact le matériel biologique avec au moins 29 ou avec 29 réactifs spécifiques choisis parmi les réactifs spécifiques des gènes cibles présentant une séquence nucléique ayant l'une quelconque des SEQ ID N° 1 ;2 ;4 à 6 ;13 ;14 ;20 ;26 ;38 ; 39 ;41 ;69 ;75 ;79 à 81 ;87 ;89 ;93 ;95 à 96 ;101 ;103 à 106 ;108 ;110 et on détermine, lors de l'étape c, l'expression d'au moins 29 ou 29 desdits gènes cibles.
Aussi décrit est un procédé pour le diagnostic précoce in vitro du cancer du sein chez un patient susceptible d'être atteint d'un cancer du sein **caractérisé en ce qu**'il comprend les étapes suivantes :
a. on extrait du matériel biologique d'un échantillon biologique prélevé chez le patient,
b. on met en contact le matériel biologique avec au moins 29 ou 29 réactifs spécifiques choisi parmi les réactifs spécifiques des gènes cibles présentant une séquence nucléique ayant l'une quelconque des SEQ ID N° 1 ;2 ;4 à 6 ;13 ;14 ;20 ;26 ;38 ; 39 ;41 ;69 ;75 ;79 à 81 ;87 ;89 ;93 ;95 à 96 ;101 ;103 à 106 ;108 ;110
c. on détermine l'expression desdits gènes cibles
Lors de l'étape b) on peut mettre en contact le matériel biologique avec au moins 10 ou avec 10 réactifs spécifiques choisis parmi les réactifs spécifiques des gènes cibles présentant une séquence nucléique ayant l'une quelconque des SEQ ID N° 1 ; 2 ; 4; 6 ; 13 ; 14 ; 26 ; 69 ; 81 ; 105 et on détermine, lors de l'étape c, l'expression d'au moins 10 ou 10 desdits gènes cibles.
Aussi décrit est un procédé in vitro pour le diagnostic, préférentiellement tardif du cancer du sein chez un patient susceptible d'être atteint d'un cancer du sein **caractérisé en ce qu**'il comprend les étapes suivantes :
a. on extrait du matériel biologique d'un échantillon biologique prélevé chez le patient,
b. on met en contact le matériel biologique avec au moins 10 ou 10 réactifs spécifiques choisi parmi les réactifs spécifiques des gènes cibles présentant une séquence nucléique ayant l'une quelconque des SEQ ID N° SEQ ID N° 1 ; 2 ; 4; 6 ; 13 ; 14 ; 26 ; 69 ; 81 ; 105
c. on détermine l'expression desdits gènes cibles.
L'utilisation de panel de gènes restreint est particulièrement adapté pour obtenir un outil de pronostic. En effet, l'analyse de l'expression d'une dizaine de gènes ne nécessite pas la fabrication à façon de puce à ADN, et peut être mise en oeuvre directement par des techniques de PCR ou de NASBA, ou puce de basse densité, ce qui présente un atout économique important et une mise en oeuvre simplifiée.
L'échantillon biologique prélevé chez le patient est préférentiellement un échantillon sanguin. Ceci permet d'obtenir un procédé de diagnostic facile à mettre en oeuvre et peu douloureux pour le patient. Le matériel biologique extrait lors de l'étape a) comprend préférentiellement des acides nucléiques, ce qui permet une analyse aisée et rapide de l'expression du ou des gènes cibles lors de l'étape c). Dans ce cas, lesdits réactifs spécifiques de l'étape b) sont préférentiellement des sondes d'hybridation. Préférentiellement, ces sonde d'hybridation sont immobilisées sur un support, qui est préférentiellement une biopuce. Cette biopuce permet alors l'analyse simultanée de l'ensemble des gènes cibles selon l'invention.
Aussi décrit est un support, tel que défini précédemment, comprenant au moins 8 sondes d'hybridation spécifique de gènes cibles présentant une séquence nucléique ayant l'une quelconque des SEQ ID N° 1 à 8. L'invention concerne également un support, tel que défini précédemment, constitué de 8 sondes d'hybridation spécifique de gènes cibles présentant une séquence nucléique ayant l'une quelconque des SEQ ID N° 1 à 8.
Aussi décrit est un support, tel que défini précedemment, comprenant au moins 23 sondes d'hybridation spécifique de gènes cibles présentant une séquence nucléique ayant l'une quelconque des SEQ ID N° 1 à 23, et un support, tel que défini précédemment, constitutué de 23 sondes d'hybridation spécifique de gènes cibles présentant une séquence nucléique ayant l'une quelconque des SEQ ID N° 1 à 23.
Aussi décrit est support tel que défini précédemment, comprenant au moins 46 sondes d'hybridation spécifique de gènes cibles présentant une séquence nucléique ayant l'une quelconque des SEQ ID N° 1 à 46. Préférentiellement, ce support comprend en outre au moins 28 sondes d'hybridation spécifique de gènes cibles présentant une séquence nucléique ayant l'une quelconque des SEQ ID N°47 à 74. Un support, tel que défini précedemment, peut être consititué 46 sondes d'hybridation spécifique de gènes cibles présentant une séquence nucléique ayant l'une quelconque des SEQ ID N° 1 à 46. Préférentiellement, ce support est constitué, en outre, 28 sondes d'hybridation spécifique de gènes cibles présentant une séquence nucléique ayant l'une quelconque des SEQ ID N°47 à 74.
Aussi décrits sont les supports suivants:
- support comprenant au moins ou constitué de 10 sondes d'hybridation spécifique de gènes cibles présentant une séquence nucléique ayant l'une quelconque des SEQ ID N° 1 ; 2 ; 4; 6 ; 13 ; 14 ; 26 ; 69 ; 81 ; 105.
- un support comprenant au moins ou constitué de 29 sondes d'hybridation spécifique de gènes cibles présentant une séquence nucléique ayant l'une quelconque des SEQ ID N°1 ;2 ;4 à 6 ;13 ;14 ;20 ;26 ;38 ; 39 ;41 ;69 ;75 ;79 à 81 ;87 ;89 ;93 ;95 à 96 ;101 ;103 à 106 ;108 ;110.
- un support comprenant au moins ou constitué de 54 sondes d'hybridation spécifique de gènes cibles présentant une séquence nucléique ayant l'une quelconque des SEQ ID N° 1 à 6 ; 13 ;14 ;20 ;26 ;28 ; 38 à 41 ;69 ;74 à 111. Préférentiellement, ce support comprend en outre des sondes d'hybridation spécifique de gènes cibles présentant une séquence nucléique ayant l'une quelconque des SEQ ID N°36; 37; 46; 48; 59 à 61 ; 63 ; 65 ; 67 ; 70 à 72 ; 73 ; 112 à 139.
Les figures ci-jointes sont données à titre d'exemples explicatifs et n'ont aucun caractère limitatif. Elles permettront de mieux comprendre l'invention.

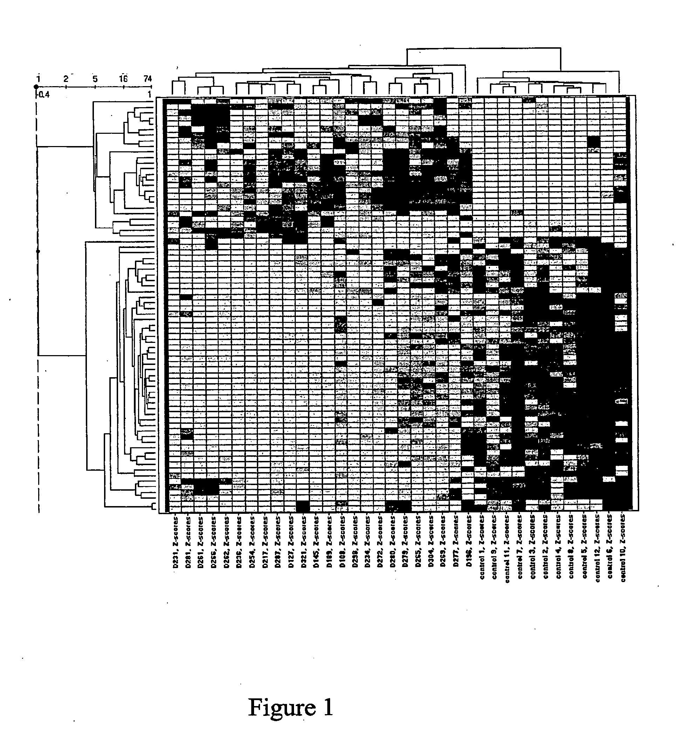

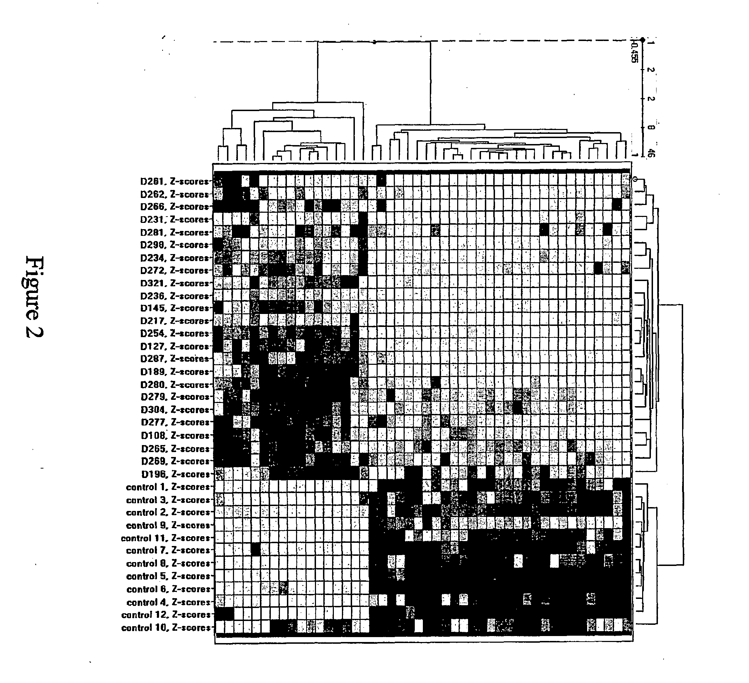

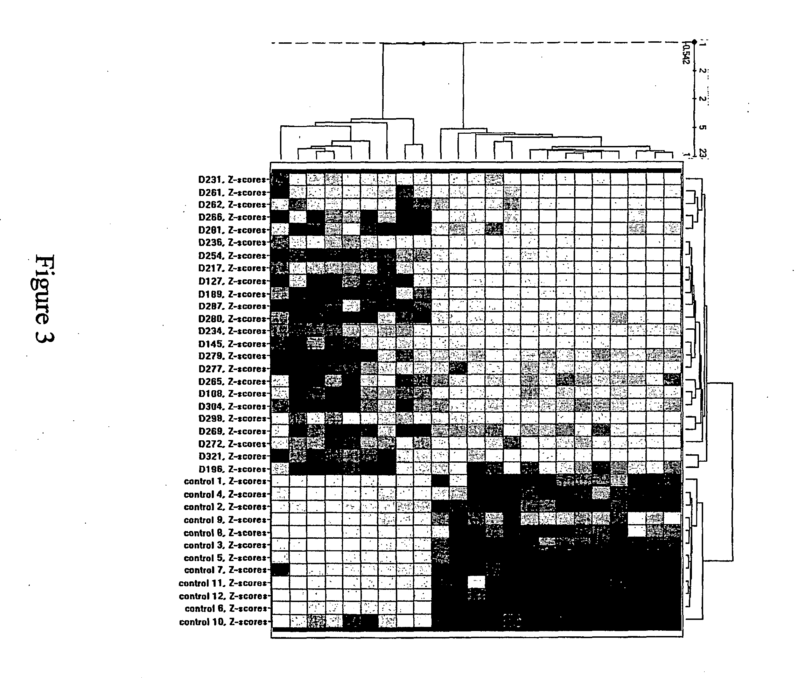

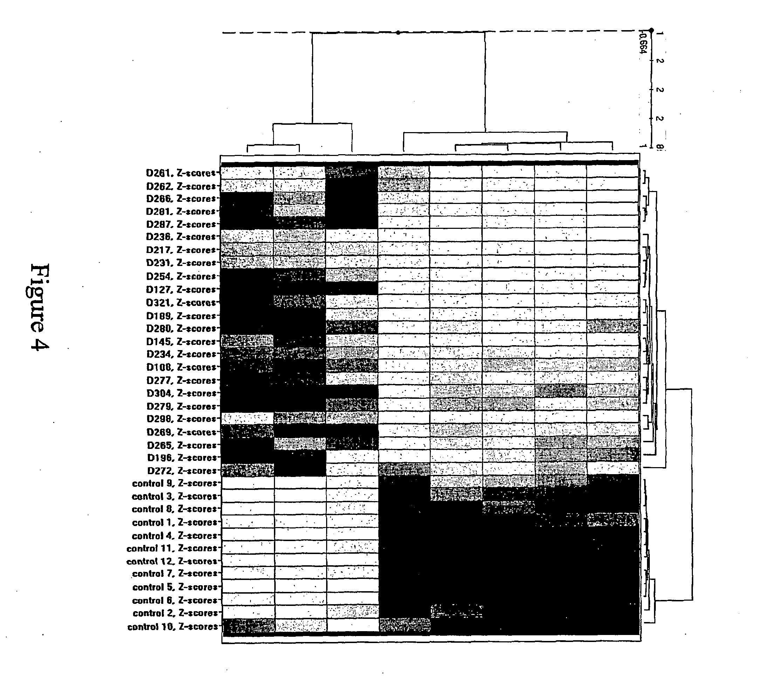

Les figures 1 à 4 représentent l'analyse de clustering hiérarchique d' échantillons de sang obtenu à partir de 24 patients atteintes d'un cancer en stade précoce (C I/II, appelé également D) et 12 patients Controles (donneuses saines) en utilisant l'expression de 74 (figure 1), 46 (figure 2), 23 (figure 3) ou 8 (figure 4) gènes identifiés par l'analyse algorithmique. La fonction de clustering hiérarchique du logiciel Spotfire organise les patients C I/II et contrôles en colonnes, et les gènes en lignes de manière à obtenir en position adjacente les patients ou les gènes présentant des profils d'expression comparables. Le coefficient de corrélation de Pearson a été utilisé comme indice de similarité pour les gènes et les patients. Les résultats correspondent au niveau de fluorescence Affymetrix normalisé par le l'outil « bioconductor ». Afin de tenir compte des différences constitutives d'expression entre les gènes, les niveaux d'expression de chaque gène ont été normalisés en calculant une variable centré réduite. Le blanc représente les faibles niveaux d'expression, le gris les niveaux intermédiaires et le noir les niveaux forts. La hauteur des branches du dendrogramme indique l'indice de similarité entre les profils ) d'expression.

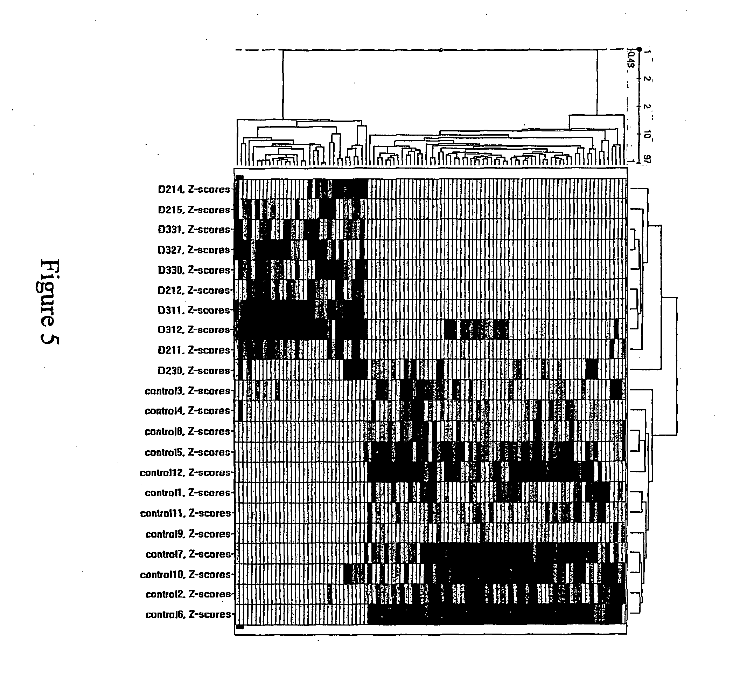

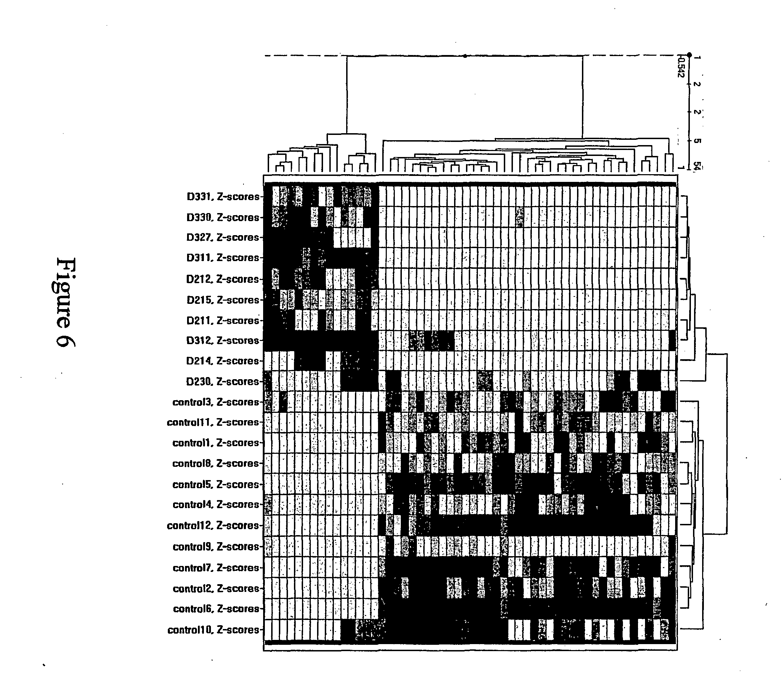

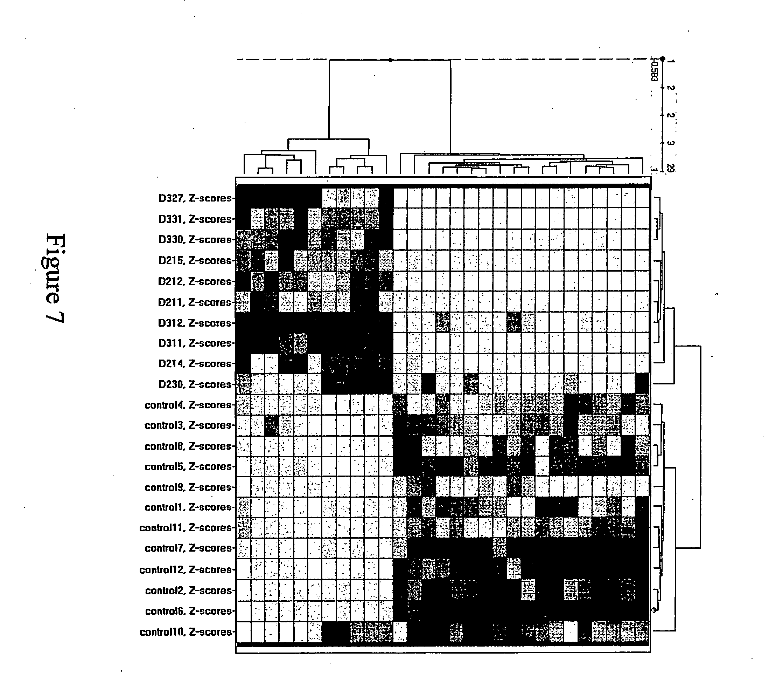

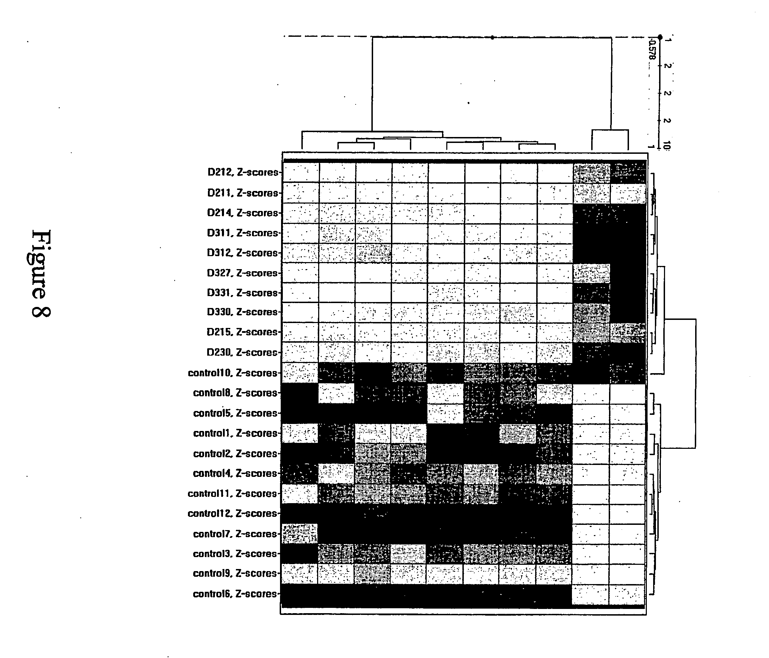

Les figures 5 à 8 représentent l'analyse de clustering hiérarchique d' échantillons de sang obtenu à partir de 10 patients atteintes d'un cancer en stade avancé (C III/IV, référencé D) et 12 patients Controles (donneuses saines) en utilisant l'expression de 97 (figure 5), 54 (figure 6), 29 (figure 7), ou 10 (figure 8) gènes identifiés par l'analyse algorithmique. La fonction de clustering hiérarchique du logiciel Spotfire organise les patients C III/IV et contrôles en colonnes, et les gènes en lignes de manière à obtenir en position adjacente les patients ou les gènes présentant des profils d'expression comparables. Le coefficient de corrélation de Pearson a été utilisé comme indice de similarité pour les gènes et les patients. Les résultats correspondent au niveau de fluorescence Affymetrix normalisé par le 1' outil « bioconductor » Afin de tenir compte des différences constitutives d'expression entre les gènes, les niveaux d'expression de chaque gène ont été normalisés en calculant une variable centré réduite. Le blanc représente les faibles niveaux d'expression, le gris les niveaux intermédiaires et le noir les niveaux forts. La hauteur des branches du dendrogramme indique l'indice de similarité entre les profils d'expression.
Les exemples suivants sont donnés à titre illustratif et n'ont aucun caractère limitatif. Ils permettront de mieux comprendre l'invention. Seulement l'exemple 1a trait à l'invention.

### Exemple 1: Mise en évidence d'un profil d'expression pour le diagnostic du cancer du sein à partir d'un échantillon sanguin

*Caractéristiques des échantillons biologiques :* L'exemple présenté ci après a été réalisé à partir de 46 échantillons sanguins (5 ml de sang total, prélevé dans deux tubes PaxGene). Ces échantillons regroupaient 12 échantillons sanguins provenant de patientes contrôles saines (S, obtenus de l'Etablissement Français du Sang) et 24 échantillons de patientes atteintes d'un cancer du sein en phase I/II (CI/II).

*Extraction du matériel biologique (ARN totaux) de l'échantillon biologique :* Les prélèvements sanguins ont été collectés directement dans des tubes PAXGene^{™} Blood RNA (PreAnalytix, Frankin Lakes, USA). Après l'étape de prélèvement de l'échantillon sanguin et afin d'obtenir une lyse totale des cellules, les tubes ont été laissés à température ambiante pendant 4 h puis conservés à -20°C jusqu'à l'extraction du matériel biologique. Plus précisément, dans ce protocole, les ARN totaux ont été extraits à l'aide des kits PAXGene Blood RNA® (PreAnalytix) en respectant les recommandations du fabriquant. Brièvement, les tubes ont été centrifugés (10 min, 3000 g) afin d'obtenir un culot d'acides nucléiques. Ce culot a été lavé et repris dans un tampon contenant de la protéinase K nécessaire à la digestion des protéines (10 min à 55°C). Une nouvelle centrifugation (5 min 19 000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column et, avant l'élution de ceux-ci, une digestion de l'ADN contaminant a été effectuée à l'aide du RNAse free DNAse set (Qiagen Ltd, Crawley, UK). La qualité des ARN totaux a été analysée par le bio analyseur AGILENT 2100 (Agilent Technologies, Waldbronn, Germany). Les ARN totaux comprennent les ARN de transfert, les ARN messagers (ARNm) et les ARN ribosomaux.

*Synthèse d'ADNc, obtention des ARNc et marquage des ARNc et quantification* : Afin d'analyser l'expression des gènes cibles selon l'invention, les ADN complémentaires (ADNc) des ARNm contenus dans les ARN totaux tels que purifiés ci dessus, ont été obtenus à partir de 10 µg d'ARN totaux par l'utilisation de 400 unités de l'enzyme de transcription reverse SuperScriptII (Invitrogen) et 100 pmol d'amorce poly-T contenant le promoteur de la T7 promotor (T7-oligo(dT)24-primer, Proligo, Paris, France).
Les ADNc ainsi obtenus ont ensuite été extraits avec du phénol/chloroforme, et précipités tel que décrit précédemment par de l'acétate d'ammonium et de l'éthanol, et remis en solution dans 24 µl d'eau DEPC. Un volume de 20 µl de cette solution purifiée d'ADNc a fait l'objet ensuite d'une transcription *in vitro* par l'utilisation d'une ARN polymérase T7 qui reconnaît spécifiquement le promoteur de la T7 polymérase tel que mentionné ci dessus. Cette transcription permet d'obtenir l'ARNc de l'ADNc. Cette transcription permet d'obtenir l'ARNc de l'ADNc. Cette transcription a été réalisée par l'utilisation d'un kit IVT labelling kit (Affymetrix, Santa Clara, CA), qui permet non seulement d'obtenir l'ARNc mais également l'incorporation de bases pseudouridine biotinylées lors de la synthèse de l'ARNc . Les ARNc purifiés ont ensuite été quantifiés par spectrophotométrie, et la solution d'ARNc a été ajustée à une concentration de 1 µg/µl d'ARNc. L'étape de clivage de ces ARNc a ensuite été réalisée à 94°C pendant 35 min, par l'utilisation d'un tampon de fragmentation (40 mM de Tris acétate, pH 8,1, 100 mM d'acétate de potassium, 30 mM d'acétate de magnesium) afin de provoquer l'hydrolyse des ARNc et obtenir des fragments de 35 à 200 bp. Le succès d'une telle fragmentation a été vérifié par une électrophorèse sur gel d'agarose 1,5%.

### Mise en évidence d'un profil d'expression des gènes permettant de discriminer les patientes Controles (S) des patientes atteintes d'un cancer en stade IlII

L'expression d'environ 30 000 gènes a été analysée et comparée entre des patientes S et C I/II. Pour cela, 10 µg d'ARNc fragmentés issus de chaque échantillon ont été ajoutés à un tampon d'hybridation (Affymetrix) et 200 µl de cette solution ont été mis en contact pendant 16 h à 45°C sur une puce d'expression (Human Genome U133P1us2 GeneChip® (Affymetrix), qui comporte 54.000 groupes de sondes représentant environ 30.000 gènes selon le protocole d'Affymetrix.
Afin d'enregistrer les meilleures performances d'hybridation et de lavage, des ARN qualifiés de « contrôle » biotinylés (bioB, bioC, bioD et cre) et des oligonucléotides (oligo B2) ont également été inclus dans le tampon d'hybridation. Après l'étape d'hybridation, les ARNc biotinylés et hybridés sur la puce, ont été révélés par l'utilisation d'une solution de streptavidine-phycoerythrine et le signal a été amplifié par l'utilisation d'anticorps anti-streptavidine. L'hybridation a été réalisée dans une étuve d'hybridation «GeneChip Hybridisation oven» (Affymetrix), et le protocole Euk GE-WS2 du protocole d'Affymetrix a été suivi. Les étapes de lavage et de révélation ont été réalisées sur une station « Fluidics Station 450 » (Affymetrix). Chaque puce U133_Plus_2 a ensuite été analysée sur un scanner Agilent G3000 GeneArray Scanner à une résolution de 1,5 microns afin de repérer les zones hybridées sur la puce. Ce scanner permet la détection du signal émis par les molécules fluorescentes après excitation par un laser argon en utilisant la technique du microscope à épifluorescence. On obtient ainsi pour chaque position, un signal proportionnel à la quantité de ARNc fixés. Le signal a ensuite été analysé par le logiciel GeneChip Operating Software (GCOS 1.2, Affymetrix).
Afin de prévenir les variations obtenues par l'utilisation de différentes puces, il a été réalisé une approche de normalisation utilisant l'outil « Bioconductor », qui permet d'harmoniser la distribution moyenne des données brutes obtenues pour chaque puce. Les résultats obtenus sur une puce peuvent alors être comparés aux résultats obtenus sur une autre puce. Le logiciel GCOS 1.2 permettait aussi d'inclure un algorithme statistique pour considérer si un gène était exprimé ou non. Chaque gène représenté sur la puce U133_Plus_2 était couvert par 11 à 16 couples de sondes de 25 nucléotides. Par couple de sondes, on entend une première sonde qui s'hybridait parfaitement (on parle alors de sondes PM ou perfect match) avec un des ARNc issus d'un gène cible, et une deuxième sonde, identique à la première sonde à l'exception d'un mésappariement (on parle alors de sonde MM ou mismatched) au centre de la sonde. Chaque sonde MM servait à estimer le bruit de fond correspondant à une hybridation entre deux fragments nucléotidiques de séquence non complémentaire. (Affymetrix technical note "Statistical Algorithms Reference Guide" ; Lipshutz, et al (1999) Nat. Genet. 1 Suppl., 20-24). Les 46 échantillons restants montraient une moyenne de 42,1 % de gènes exprimés.
A partir des 54.000 groupes de sondes, représentant environ 30 000 gènes, de la puce, les inventeurs ont sélectionné les gènes pertinents qui étaient corrélés au developpement d'un cancer du sein.
Les gènes qui ont un niveau d'expression trop faible sur la majorité des puces ainsi que les gènes qui ne présentent pas de variation importante entre les différentes puces ont été exclus (Li et al, 2001, Bioinformatics, 17 : 1131-1142). La recherche d'un panel de gènes discriminant les groupes de patientes EFS et CI/II a été réalisée par une technique de Data Mining (http://ligarto.org/rdiaz/Papers/jornadas.bioinfo.randomForest.pdf).
Cette analyse a permis de mettre en évidence un premier panel de gènes, comprenant 46 gènes pertinents selon l'invention (SEQ ID N°1 à 46). Une analyse complémentaire (SAM, Significance Analysis of Microarrays) a également permis de mettre en évidence des gènes supplémentaires qui s'avéraient également très pertinents (SEQ ID N°47 à 74). L'augmentation ou la diminution d'expression de chacun de ces gènes, observée chez les patientes S par rapport aux patientes C I/II est présentée dans le tableau 3.

**Tableau 3 - liste des 74 gènes exprimés différentiellement lors du développement d'un cancer du sein**

| **SEQ ID N°** | **Description de la séquence** | **N° Genbank** | **C I/II vs. Sains** |
|---|---|---|---|
| 1 | Centrosome-associated protein 350 [CAP350] | NM _14810 | 0,6 |
| 2 | Hypothetical protein MGC23401 | NM _14982 | 0,6 |
| 3 | Trophoblast-derived noncoding RNA [TncRNA] | AF001893 (Hs.523789) | 0,4 |
| 4 | Vacuolar protein sorting 35 (yeast) [PUM2] | NM_015317 | 0,6 |
| 5 | Ribosomal protein L36a-like [RPL36AL] | NM_001001 | 2,8 |
| 6 | Mitochondrial ribosomal protein L51 [MRPL51] | NM_016497 | 2,1 |
| 7 | KIAA0794 protein [KIAA0794] | XM_087353 | 1,7 |
| 8 | Transcribed locus | CA775887 (Hs. 388575) | 0,6 |
| 9 | Hypothetical protein MGC14817 [MGC14817] | NM_032338 | 2,1 |
| 10 | Hypothetical protein FLJ11046 | NM_018309 | 0,7 |
| 11 | Pleckstrin homology, Sec7 and coiled-coil domains 4 [PSCD4] | NM_013385 | 1,5 |
| 12 | Lactate dehydrogenase B [LDHB] | NM_002300 | 2,3 |
| 13 | NADH dehydrogenase (ubiquinone) alpha subcomplex 1 [NDUFA1] | NM_004541 | 1,7 |
| 14 | Muscleblind-like (Drosophila) [MBNL1] | NM_021038 | 0,5 |
| 15 | Ubiquitin specific protease 25 [USP25] | NM_013396 | 0,6 |
| 16 | TATA element modulatory factor 1 [TMF1] | NM_007114 | 0,7 |
| 17 | Ring finger protein 19 [RNF19] | NM_015435 | 0,6 |
| 18 | Signal peptidase complex subunit 3 homolog (S. cerevisiae) [SPCS3] | NM_021928 | 0,6 |
| 19 | Enhancer of polycomb homolog 1 (Drosophila) [EPC1] | NM_025209 | 0,6 |
| 20 | Zinc finger, martin type 2 [ZMAT2] | NM_144723 | 1,7 |
| 21 | Image clone 3069209 | BF512254 | 0,6 |
| 22 | ORM1-like 3 (S. cerevisiae) [ORMDL3] | NM_139280 | 1,5 |
| 23 | CDNA FLJ11397 fis, clone HEMBA1000622 | AW962458 (Hs. 470871) | 0,6 |
| 24 | Tankyrase, TRF1-interacting ankyrin-related ADP-ribose polymerase [TNKS] | NM_003747 | 0,6 |
| 25 | Ribosomal protein S23 [RPS23] | NM_001025 | 1,8 |
| 26 | CDNA clone IMAGE:5263531 | AK025902 (Hs.399763) | 0,7 |
| 27 | PABP 1-dependent poly A-specific ribonuclease subunit [PAN3] | NM_175854 | 0,6 |
| 28 | Hypothetical protein FLJ21924 | NM_024774 | 0,6 |
| 29 | CDNA FLJ42313 fis, clone TRACH2019425 | AK124306 (Hs.386042) | 2,1 |
| 30 | Family with sequence similarity 49, member B [FAM49B] | NM_016623 | 1,5 |
| 31 | Dicerl, Dcr-1 homolog (Drosophila) [DICER1] | NM_030621 | 0,7 |
| 32 | Ribosomal protein L37 [RPL37] | NM_000997 | 1,6 |
| 33 | UDP-glucose ceramide glucosyltransferase [UGCG] | NM_003358 | 0,7 |
| 34 | Complement component (3b/4b) receptor 1 [CRI] | NM_000573 | 1,6 |
| 35 | KIAA1702 protein | AB051489 (Hs.485628) | 0,7 |
| 36 | Hypothetical protein FLJ10618 | NM_018155 | 0,5 |
| 37 | Hypothetical protein LOC146174 | NM_173501 | 0,7 |
| 38 | MRNA; cDNA DKFZp686D22106 (from clone DKFZp686D22106) | CR933609 (Hs. 445036) | 0,7 |
| 39 | Anterior pharynx defective 1 homolog A (C. elegans) [APH1A] | NM_016022 | 0,7 |
| 40 | U2-associated SR140 protein [SR140] | XM_031553 | 0,5 |
| 41 | Androgen-induced proliferation inhibitor [APRIN] | NM_015032; NM 015928 | 0,6 |
| 42 | Peptidylprolyl isomerase D (cyclophilin D) [PPID] | NM_005038 | 1,4 |
| 43 | Mitochondrial ribosomal protein S17 [MRPS17] | NM_015969 | 1,8 |
| 44 | Adaptor-related protein complex 1, sigma 2 subunit [AP1S2] | NM_003916 | 0,6 |
| 45 | Heat shock 90kDa protein 1, alpha [HSPCA] | NM_005348 | 1,8 |
| 46 | GNAS complex locus [GNAS] | NM_000516; NM_016592; NM_080425; NM_080426 | 0,5 |
| 47 | 5-azacytidine induced 2 [AZI2] | NM_022461 | 0,6 |
| 48 | BCL2-like 1 [BCL2L1] | NM_001191 | 1,9 |
| 49 | Bobby sox homolog (Drosophila) [BBX] | NM_020235 | 0,6 |
| 50 | Calcium-transporting ATPase, type 2C, member 1 [ATP2C1] | NM_001001485 | 0,6 |
| 51 | Cathepsin Z [CTSZ] | NM_001336 | 0,6 |
| 52 | CDNA FLJ26120 fis, clone SYN00419 | AK129631 (Hs.433995) | 2,2 |
| 53 | COMM domain containing 6 [COMMD6] | NM_203495 | 0,6 |
| 54 | Cytochrome c oxidase subunit VIIb [COX7B] | NM_001866 | 0,5 |
| 55 | Cytoplasmic polyadenylation element binding protein 2 [CPEB2] | NM_182485 | 0,6 |
| 56 | Endoplasmic reticulum-golgi intermediate compartment 32 kDa protein [KIAA1181] | NM_020462 | 0,5 |
| 57 | Ewing sarcoma breakpoint region 1 [EWSR1] | NM_005243 | 1,9 |
| 58 | Glyceraldehyde-3-phosphate dehydrogenase [GAPD] | NM_002046 | 2,3 |
| 59 | GRB2-associated binding protein 2 [GAB2] | NM_012296 | 2,2 |
| 60 | Killer cell lectin-like receptor subfamily C, member 1 or 2 [KLRC1/KLRC2] | NM_002259 | 0,4 |
| 61 | Killer cell lectin-like receptor subfamily F1 [KLRF1] | NM_016523 | 0,6 |
| 62 | Metastasis associated lung adenocarcinoma transcript 1 [MALAT1] | BX538238 (Hs.187199) | 1,9 |
| 63 | MRNA; cDNA DKFZp586O0724 | BU676985 (Hs.159115) | 0,5 |
| 64 | Nipped-B homolog (Drosophila) [NIPBL] | NM_015384 | 0,5 |
| 65 | Prader-Willi/Angelman region-1 [PAR1] | BE783065 (Hs.546847) | 0,5 |
| 66 | PRO1550 | AF086013 (Hs.371588) | 0,6 |
| 67 | Protein phosphatase 2, regulatory subunit B (B56), epsilon isoform [PPP2R5E] | NM_006246 | 0,6 |
| 68 | RP42 homolog [RP42] | NM_020640 | 0,5 |
| 69 | Special AT-rich sequence binding protein 1 [SATB 1] | NM_002971 | 0,5 |
| 70 | Tubulin beta 2 [TUBB2] | NM_001069 | 0,5 |
| 71 | Ubiquitin-fold modifier 1 [Ufm1] | NM_016617 | 0,5 |
| 72 | v-myb myeloblastosis viral oncogene homolog [MYBL1] | XM_034274 | 0,6 |
| 73 | WNK lysine deficient protein kinase 1 [WNK1] | NM_018979 | 2,0 |
| 74 | Zinc finger, MYND domain containing 11 [ZMYND11] | NM_006624 | 0,6 |

Les inventeurs ont également étudié l'expression simultanée de 74 gènes de séquence nucléotidique choisi parmi les SEQ ID N°1 à 74 pour obtenir un profil d'expression. Les résultants sont présentés dans la figure 1. 100 % des patients étaient correctement classifiés. Les inventeurs ont également étudié l'expression simultanée de 46 gènes de séquence nucléotidique choisi parmi les SEQ ID N°1 à 46 pour obtenir un profil d'expression. Les résultats sont présentés dans la figure 2. 100 % des patients étaient correctement classifiés. Les inventeurs ont également étudié l'expression simultanée de 23 gènes de séquence nucléotidique choisi parmi les SEQ ID N°1 à 23 pour obtenir un profil d'expression. Les résultats sont présentés dans la figure 3. 100 % des patients étaient correctement classifiés. Les inventeurs ont également étudié l'expression simultanée de 8 gènes de séquence nucléotidique choisi parmi les SEQ ID N°1 à 8 pour obtenir un profil d'expression. Les résultats sont présentés dans la figure 4. 100 % des patients étaient correctement classifiés.

### Exemple 2 : Mise en évidence d'un profil d'expression pour le diagnostic du cancer du sein à partir d'un échantillon sanguin

*Caractéristiques des échantillons biologiques :* L'exemple présenté ci après a été réalisé à partir de 46 échantillons sanguins (5 ml de sang total, prélevé dans deux tubes PaxGene). Ces échantillons regroupaient 12 échantillons sanguins provenant de patientes contrôles saines (S, obtenus de l'Etablissement Français du Sang) et 24 échantillons de patientes atteintes d'un cancer du sein en phase III/IV (CIII/IV), c'est à dire un stade avancé du cancer du sein.

*Extraction du matériel biologique (ARN totaux) de l'échantillon biologique :* cette étape a été réalisée comparablement à l'exemple 1.

### Synthèse d'ADNc, obtention des ARNc et marquage des ARNc et quantification : cette étape a été réalisée comparablement à l'exemple 1.

*Mise en évidence d'un profil d'expression des gènes permettant de discriminer les patientes Controles (S) des patientes atteintes d'un cancer en stade III*/*IV* L'expression d'environ 30 000 gènes a été analysée et comparée entre des patientes S et C III/IV. Pour cela, 10 µg d'ARNc fragmentés issus de chaque échantillon ont été ajoutés à un tampon d'hybridation (Affymetrix) et 200 µl de cette solution ont été mis en contact pendant 16 h à 45°C sur une puce d'expression (Human Genôme U133P1us2 GeneChip® (Affymetrix), qui comporte 54.000 groupes de sondes représentant environ 30.000 gènes selon le protocole d'Affymetrix.
Afin d'enregistrer les meilleures performances d'hybridation et de lavage, des ARN qualifiés de « contrôle » biotinylés (bioB, bioC, bioD et cre) et des oligonucléotides (oligo B2) ont également été inclus dans le tampon d'hybridation. Après l'étape d'hybridation, les ARNc biotinylés et hybridés sur la puce, ont été révélés par l'utilisation d'une solution de streptavidine-phycoerythrine et le signal a été amplifié par l'utilisation d'anticorps antistreptavidine. L'hybridation a été réalisée dans une étuve d'hybridation «GeneChip Hybridisation oven» (Affymetrix), et le protocole Euk GE-WS2 du protocole d'Affymetrix a été suivi. Les étapes de lavage et de révélation ont été réalisées sur une station « Fluidics Station 450 » (Affymetrix). Chaque puce U133_Plus_2 a ensuite été analysée sur un scanner Agilent G3000 GeneArray Scanner à une résolution de 1,5 microns afin de repérer les zones hybridées sur la puce. Ce scanner permet la détection du signal émis par les molécules fluorescentes après excitation par un laser argon en utilisant la technique du microscope à épifluorescence. On obtient ainsi pour chaque position, un signal proportionnel à la quantité de ARNc fixés. Le signal a ensuite été analysé par le logiciel GeneChip Operating Software (GCOS 1.2, Affymetrix).
Afin de prévenir les variations obtenues par l'utilisation de différentes puces, il a été réalisé une approche de normalisation utilisant le l'outil « biocondutor » qui permet d'harmoniser la distribution moyenne des données brutes obtenues pour chaque puce. Les résultats obtenus sur une puce peuvent alors être comparés aux résultats obtenus sur une autre puce. Le logiciel GCOS 1.2 permettait aussi d'inclure un algorithme statistique pour considérer si un gène était exprimé ou non. Chaque gène représenté sur la puce U133_Plus_2 était couvert par 11 à 16 couples de sondes de 25 nucléotides. Par couple de sondes, on entend une première sonde qui s'hybridait parfaitement (on parle alors de sondes PM ou perfect match) avec un des ARNc issus d'un gène cible, et une deuxième sonde, identique à la première sonde à l'exception d'un mésappariement (on parle alors de sonde MM ou mismatched) au centre de la sonde. Chaque sonde MM servait à estimer le bruit de fond correspondant à une hybridation entre deux fragments nucléotidiques de séquence non complémentaire. (Affymetrix technical note "Statistical Algorithms Référence Guide" ; Lipshutz, et al (1999) Nat. Genet. 1 Suppl., 20-24). Les 46 échantillons restants montraient une moyenne de 42,1 % de gènes exprimés.
A partir des 54.000 groupes de sondes, représentant environ 30 000 gènes, de la puce, les inventeurs ont sélectionné les gènes pertinents qui étaient corrélés au developpement d'un cancer du sein.
Les gènes qui ont un niveau d'expression trop faible sur la majorité des puces ainsi que les gènes qui ne présentent pas de variation importante entre les différentes puces ont été exclus (Li et al, 2001, Bioinformatics, 17 : 1131-1142). La recherche d'un panel de gènes discriminant les groupes de patientes EFS et CI/II a été réalisée par une technique de Data Mining (http://ligarto.org/rdiaz/Papers/jornadas.bioinfo.randomForest.pdf).
Cette analyse a permis de mettre en évidence un premier panel de gènes, comprenant 54 gènes pertinents selon l'invention (SEQ ID N° SEQ ID N° 1 à 6 ; 13 ;14 ;20 ;26 ;28 ;38 à 41 ;69 ;74 à 111). Une analyse complémentaire (SAM, Significance Analysis of Microarrays) a également permis de mettre en évidence des gènes supplémentaires qui s'avéraient également très pertinents (SEQ ID N° SEQ ID N°36; 37; 46; 48; 59 à 61 ; 63 ; 65 ; 67 ; 70 à 72 ; 73 ; 112 à 139).
L'augmentation ou la diminution d'expression de chacun de ces gènes, observée chez les patientes S par rapport aux patientes C I/II est présentée dans le tableau 3.

**Tableau 4-liste des 96 gènes exprimés différentiellement lors du développement d'un cancer du sein**

| **SEQ ID N°** | **Description de la séquence** | **N° Genbank** | **C III/IV vs. Sains** |
|---|---|---|---|
| 1 | Centrosome-associated protein 350 [CAP350] | NM_014810 | 0,5 |
| 2 | Hypothetical protein MGC23401 | NM_144982 | 0,6 |
| 3 | Trophoblast-derived noncoding RNA [TncRNA] | AF001893 (Hs.523789) | 0,4 |
| 4 | Vacuolar protein sorting 35 (yeast) [PUM2] | NM_015317 | 0,5 |
| 5 | Ribosomal protein L36a-like [RPL36AL] | NM_001001 | 2,5 |
| 6 | Mitochondrial ribosomal protein L51 [MRPL51] | NM_016497 | 1,5 |
| 13 | NADH dehydrogenase (ubiquinone) alpha subcomplex 1 [NDUFA1] | NM_004541 | 1,7 |
| 14 | Muscleblind-like (Drosophila) [MBNL1] | NM_021038 | 0,5 |
| 20 | Zinc finger, matrin type 2 [ZMAT2] | NM_144723 | 1,9 |
| 26 | CDNA clone IMAGE:526353 1, partial cds | AK025902 (Hs.399763) | 0,7 |
| 28 | Hypothetical protein FLJ21924 | NM_024774 | 0,6 |
| 36 | Hypothetical protein FLJ10618 | NM_018155 | 0,5 |
| 37 | Hypothetical protein LOC283666 | BC048264 (Hs.512943) | 0,5 |
| 39 | Anterior pharynx defective 1 homolog A (C. elegans) [APH1A] | NM_016022 | 0,6 |
| 40 | U2-associated SR140 protein [SR1 40] | XM_031553 | 0,5 |
| 41 | Androgen-induced proliferation inhibitor [APRIN] | NM_015032 | 0,6 |
| 46 | GNAS complex locus [GNAS] | NM_000516 | 0,5 |
| 48 | BCL2-like 1 [BCL2L1] | NM_001191 | 2,5 |
| 59 | GRB2-associated binding protein 2 [GAB2] | NM_012296 | 2,1 |
| 60 | Killer cell lectin-like receptor subfamily C, member 1 or 2 [KLRC1/KLRC2] | NM_002259 | 0,3 |
| 61 | Killer cell lectin-like receptor subfamily F1 [KLRF1] | NM_016523 | 0,3 |
| 63 | mRNA; cDNA DKFZp58600724 (from clone DKFZp586O0724) | BU676985 (Hs.159115) | 0,4 |
| 65 | Prader-Willi/Angelman region-1 [PARI] | BE783065 (Hs.546847) | 0,4 |
| 67 | Protein phosphatase 2, regulatory subunit B (B56), epsilon isoform [PPP2R5E] | NM_006246 | 0,5 |
| 69 | Special AT-rich sequence binding protein 1 [SATB1] | NM_002971 | 0,5 |
| 70 | Tubulin beta 2 [TUBB2] | NM_001069 | 3,1 |
| 71 | Ubiquitin-fold modifier 1 [Ufm1] | NM_016617 | 0,5 |
| 72 | v-myb myeloblastosis viral oncogene homolog [MYBL1] | XM_034274 | 0,5 |
| 73 | WNK lysine deficient protein kinase 1 [WNK1] | NM_018979 | 2,2 |
| 74 | Zinc finger, MYND domain containing 11 [ZMYND11] | NM_006624 | 0,6 |
| 75 | 30 kDa protein LOC55831 | NM_018447 | 1,7 |
| 76 | ADP-ribosylation factor guanine nucleotide-exchange factor 2 [ARFGEF2] | NM_006420 | 0,6 |
| 77 | BTB (POZ) domain containing 5 [BTBD5] | NM_017658 | 0,7 |
| 78 | Cathepsin O [CTSO] | NM_001334 | 0,7 |
| 79 | Centrin, EF-hand protein 2 [CETN2] | NM_004344 | 2,0 |
| 80 | Chromosome 16 open reading frame 35 [C16orf35] | NM_012075 | 2,0 |
| 81 | Chromosome 2 open reading frame 33 [C2orf33] | NM_020194 | 0,6 |
| 82 | Cleavage and polyadenylation specific factor 6, 68kDa[CPSF6] | NM_007007 | 0,7 |
| 83 | Cysteine-rich motor neuron 1 [CRIM1] | NM_016441 | 0,6 |
| 84 | Enoyl Coenzyme A hydratase domain containing 1 [ECHDC1] | NM_018479 | 0,6 |
| 85 | Erythrocyte membrane protein band 4.2 [EPB42] | NM_000119 | 2,4 |
| 86 | Formin binding protein 3 [FNBP3] | XM_371575 | 0,6 |
| 87 | Hepatitis B virus x associated protein [HBXAP] | NM_016578 | 0,6 |
| 88 | Hypothetical protein HSPC129 | NM_016396 | 0,5 |
| 89 | Hypothetical protein LOC144438 | AK002085 (Hs.92308) | 0,6 |
| 90 | Hypothetical protein MGC33214 | NM_153354 | 0,7 |
| 91 | Hypothetical protein MGC5306 | NM_024116 | 0,7 |
| 92 | Likely ortholog of mouse TORC2-specific protein AV03 (S. cerevisiae) [AV03] | NM_152756 | 0,6 |
| 93 | Mannosidase, alpha, class 2A, member 1 [MAN2A1] | NM_002372 | 0,7 |
| 94 | Mdm4, p53 binding protein (mouse) [MDM4] | NM_002393 | 0,7 |
| 95 | Nucleobindin 1 [NUCB1] | NM_006184 | 1,6 |
| 96 | Oxysterol binding protein 2 [OSBP2] | NM_001003812 | 2,0 |
| 97 | Phosphoinositide-3-kinase, catalytic, alpha polypeptide [PIK3CA] | NM_006218 | 0,6 |
| 98 | Proteasome (prosome, macropain) inhibitor subunit 1 (PI31) [PSMF1] | NM_006814 | 1,5 |
| 99 | Protein tyrosine phosphatase type IVA, member 2 [PTP4A2] | NM_003479 | 0,7 |
| 100 | Rhesus blood group, D antigen [RHD] | NM_016124 | 2,4 |
| 101 | Ring finger protein 123 [RNF123] | NM_022064 | 1,8 |
| 102 | SH2 domain-containing molecule EAT2 [EAT2] | NM_053282 | 0,4 |
| 103 | Source of immunodominant MHC-associated peptides [SIMP] | NM_178862 | 0,5 |
| 104 | Split hand/foot malformation (ectrodactyly) type 1 [SHFM1] | NM_006304 | 1,5 |
| 105 | Thyroid hormone receptor associated protein [THRAP1] | NM_005121 | 0,6 |
| 106 | Thyroid hormone receptor interactor 12 [TRIP12] | NM_004238 | 0,7 |
| 107 | Transcribed locus | AL037805 (Hs. 445247) | 0,6 |
| 108 | Transducin (beta)-like 1X-linked receptor 1 [TBL1XR1] | NM_024665 | 0,5 |
| 109 | Tubulin, beta 3 [TUBB3] | NM_006086 | 1,6 |
| 110 | Ubiquitination factor E4A (UFD2 homolog, yeast) [UBE4A] | NM_004788 | 0,7 |
| 111 | Zinc finger protein 148 (pHZ-52) [ZNF148] | NM_021964 | 0,7 |
| 112 | 3-alpha hydroxysteroid dehydrogenase, type II [AKR1C3] | NM_003739 | 0,4 |
| 113 | A kinase (PRKA) anchor protein 7 [AKAP7] | NM_004842 | 0,4 |
| 114 | Aminolevulinate, delta-, synthase 2 [ALAS2] | NM_000032 | 2,8 |
| 115 | Ankyrin 1, erythrocytic [ANK1] | NM_000037 | 2,4 |
| 116 | B double prime 1, subunit of RNA polymerase III transcription initiation factor IIIB [BDP1] | NM_018429 | 0,5 |
| 117 | Carbonic anhydrase I [CA1] | NM_001738 | 5,6 |
| 118 | Chromosome 19 open reading frame 2 [C19orf2] | NM_003796 | 0,5 |
| 119 | DKFZP564F0522 protein | NM_015475 | 0,4 |
| 120 | Erythrocyte membrane protein band 4.9 (dematin) [EPB49] | NM_001978 | 2,1 |
| 121 | Family with sequence similarity 46, member C [FAM46C] | NM_017709 | 2,8 |
| 122 | guanosine monophosphate reductase [GMPR] | NM_006877 | 2,4 |
| 123 | Homo sapiens, clone IMAGE:5267398, mRNA cDNA DKFZp686I23208 | BX538337 (Hs.40289) | 0,5 |
| 124 | Image clone 3481554 | BF062399 | 0,5 |
| 125 | IMAGE clone 5259272 | BC032890 (Hs.184430) | 0,5 |
| 126 | Integrin, alpha 2b [ITGA2B] | NM_000419 | 2,7 |
| 127 | Interleukin 8 [IL8] | NM_000584 | 0,4 |
| 128 | Leucine rich repeat neuronal 3 [LRRN3] | NM_018334 | 0,4 |
| 129 | Leukocyte receptor cluster (LRC) member 10 [LENG10] | AF211977 | 0,5 |
| 130 | Major histocompatibility complex, class II, DQ alpha 1 [HLA-DQA1] | NM_002122 | 2,2 |
| 131 | Phosphatidylinositol glycan, class K [PIGK] | NM_005482 | 0,5 |
| 132 | Selenium binding protein 1 [SELENBP1] | NM_003944 | 2,6 |
| 133 | SM-11044 binding protein [SMBP] | NM_020123 | 0,5 |
| 134 | Solute carrier family 6 (neurotransmitter transporter, creatine), member 8 [SLC6A8] | NM_005629 | 2,3 |
| 135 | TBC1 domain family, member 4 [TBC1D4] | NM_014832 | 0,5 |
| 136 | Tensin [TNS] | NM_022648 | 2,8 |
| 137 | TIA1 cytotoxic granule-associated RNA binding protein [TIA1] | NM_022037 | 0,5 |
| 138 | Transcribed locus | AA456099 (Hs.176376) | 0,4 |
| 139 | Tripartite motif-containing 58 [TRIM58] | NM_015431 | 2,3 |

Les inventeurs ont également étudié l'expression simultanée des 96 gènes présentés ci dessus pour obtenir un profil d'expression. Les résultats sont présentés dans la figure 5. 100% des patients étaient correctement classés.
Les inventeurs ont également étudié l'expression simultanée de 54 gènes de séquence nucléotidique choisi parmi les SEQ ID N° 1 à 6 ; 13 ;14 ;20 ;26 ;28 ;38 à 41 ;69 ;74 à 111 pour obtenir un profil d'expression. Les résultats sont présentés dans la figure 6. 100 % des patients étaient correctement classés.
Les inventeurs ont également étudié l'expression simultanée de 29 gènes de séquence nucléotidique choisi parmi les SEQ ID N ° 1 ;2 ;4 à 6 ;13 ;14 ;20 ;26 ;38 ; 39 ;41 ;69 ;75 ;79 à 81 ;87 ;89 ;93 ;95 à 96 ;101 ;103 à 106 ;108 ;110 pour obtenir un profil d'expression. Les résultats sont présentés dans la figure 7. 100 % des patients étaient correctement classés.
Les inventeurs ont également étudié l'expression simultanée de 10 gènes de séquence nucléotidique choisi parmi les SEQ ID N° 1 ; 2 ; 4; 6 ; 13 ; 14 ; 26 ; 69 ; 81 ; 105 pour obtenir un profil d'expression. Les résultats sont présentés dans la figure 8. 100 % des patients étaient correctement classés.
*Ceci confirme que l'analyse de l'expression de tout ou partie des gènes de SEQ ID N° 1 à 139 est un bon outil pour discriminer les patientes atteintes d'un cancer ou non, et, si la patiente est atteinte d'un cancer, de connaître le stade d'évolution de son cancer*.

## Revendications

1. Procédé pour le diagnostic *in vitro* du cancer du sein chez un patient susceptible d'être atteint d'un cancer du sein **caractérisé en ce qu'**il comprend les étapes suivantes :
a) on extrait du matériel biologique d'un échantillon biologique prélevé chez le patient,
b) on met en contact le matériel biologique avec 8 réactifs spécifiques choisi parmi les réactifs spécifiques des 8 gènes cibles présentant une séquence nucléique ayant l'une quelconque des SEQ ID N° 1 à 8
c) on détermine l'expression desdits gènes cibles.

2. Procédé selon la revendication 1 **caractérisé en ce que** l'échantillon biologique prélevé chez le patient est un échantillon sanguin.

3. Procédé selon l'une quelconque des revendications 1 à 2 **caractérisé en ce que** le matériel biologique extrait lors de l'étape a) comprend des acides nucléiques.

4. Procédé selon la revendication 3 **caractérisé en ce que** lesdits réactifs spécifiques de l'étape b) sont des sondes d'hybridation

5. Procédé selon la revendication 4 **caractérisé en ce que** lesdites sonde d'hybridation sont immobilisées sur un support.

6. Procédé selon la revendication 5 **caractérisé en ce que** le support est une biopuce.

## Claims

1. Method for the *in vitro* diagnosis of breast cancer in a patient who may be suffering from a breast cancer, **characterized in that** it comprises the following steps:
a) biological material is extracted from a biological sample taken from the patient,
b) the biological material is brought into contact with 8 specific reagents chosen from the specific reagents for the 8 target genes with a nucleic sequence having any one of SEQ ID Nos. 1 to 8,
c) the expression of said target genes is determined.

2. Method according to Claim 1, **characterized in that** the biological sample taken from the patient is a blood sample.

3. Method according to either of Claims 1 and 2, **characterized in that** the biological material extracted in step a) comprises nucleic acids.

4. Method according to Claim 3, **characterized in that** said specific reagents of step b) are hybridization probes.

5. Method according to Claim 4, **characterized in that** said hybridization probes are immobilized on a support.

6. Method according to Claim 5, **characterized in that** the support is a biochip.

## Patentansprüche

1. Verfahren für die *in-vitro*-Diagnose von Brustkrebs bei einem Patienten mit Verdacht auf Brustkrebs, **dadurch gekennzeichnet, dass** es die folgenden Schritte umfasst:
a) Herauspräparieren von biologischem Material aus einer biologischen Probe, die von dem Patienten entnommen wurde,
b) Inkontaktbringen des biologischen Materials mit 8 spezifischen Reaktanten aus der Reihe der spezifischen Reaktanten der 8 Zielgene, die eine Nukleinsäuresequenz gemäß einer der Sequenzen SEQ ID Nr.: 1 bis 8 aufweisen,
c) Bestimmen der Expression dieser Zielgene.

2. Verfahren nach Anspruch 1, **dadurch gekennzeichnet, dass** es sich bei der von dem Patienten entnommenen biologischen Probe um eine Blutprobe handelt.

3. Verfahren nach einem der Ansprüche 1 bis 2, **dadurch gekennzeichnet, dass** das in Schritt a) herauspräparierte biologische Material Nukleinsäuren umfasst.

4. Verfahren nach Anspruch 3, **dadurch gekennzeichnet, dass** es sich bei den spezifischen Reaktanten von Schritt b) um Hybridisierungssonden handelt.

5. Verfahren nach Anspruch 4, **dadurch gekennzeichnet, dass** die Hybridisierungssonden auf einem Träger immobilisiert sind.

6. Verfahren nach Anspruch 5, **dadurch gekennzeichnet, dass** es sich bei dem Träger um einen Biochip handelt.
